# EUROPEAN PATENT APPLICATION

(11) **EP 4 745 138 A1**
(43) Date of publication of application: **20.05.2026**
(21) Application number: 24842341.0
(22) Date of filing: 15.07.2024
(51) Int. Cl.: C07D 413/14, A61K 31/454, A61K 31/395, A61P 35/00, A61P 29/00

(54) **THIO-GLUTARIMIDO ISOINDOLINONE DERIVATIVE, BIFUNCTIONAL PROTEIN DEGRADER CONTAINING SAME, AND USES THEREOF**

(30) Priority: 14.07.2023 CN 202310868335
(71) Applicant: Gluetacs Therapeutics (Shanghai) Co., Ltd., Shanghai 201306 (CN)
(72) Inventor: YANG, Xiaobao, Shanghai 201306 (CN); REN, Chaowei, Shanghai 201306 (CN); SUN, Renhong, Shanghai 201306 (CN); LI, Yan, Shanghai 201306 (CN); ZHAO, Baoyin, Shanghai 201306 (CN); ZHOU, Hailong, Shanghai 201306 (CN); DENG, Meigui, Shanghai 201306 (CN); REN, Yinbo, Shanghai 201306 (CN); YAO, Shun, Shanghai 201306 (CN)
(74) Representative: J A Kemp LLP
(86) International application number: PCT/CN2024/105404
(87) International publication number: WO 2025/016350

(57) **Abstract**

The present disclosure relates to compounds of Formula (I) and Formula (II) or salts, enantiomers, stereoisomers, solvates, isotopically enriched analogs, prodrugs, or polymorphs thereof, and applications thereof. The present disclosure also relates to pharmaceutical compositions comprising, as an active ingredient, a compound of Formula (I) or a salt, enantiomer, stereoisomer, solvate, isotopically enriched analog, prodrug, or polymorph thereof, and applications thereof.

PBM-LIN-ULM (I)

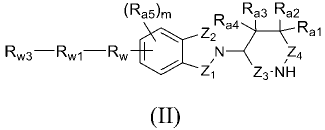

## Description

### Technical Field

The present disclosure relates to relates to compounds of formula (I), thio-glutarimido isoindolinone derivatives of Formula (II), and use thereof. The compounds of Formula (I) are effective in treating or preventing tumors or cancer. The thio-glutarimido isoindolinone derivatives of Formula (II) are capable of binding to the Cereblon (CRBN) E3 ligase and are used in the preparation of the compounds of Formula (I).

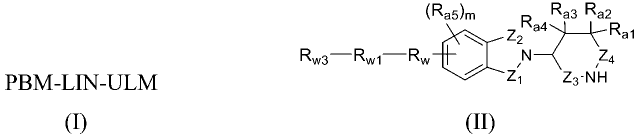

### Background

Bifunctional proteolysis-targeting drugs consist of three moieties, one end of which is a ligand warhead that can bind to a specific target protein, and the other end is a small molecule ligand of E3 ubiquitin ligase, both of which are connected together through linkers of different lengths and categories located in the middle. The bifunctional proteolysis-targeting small molecules can utilize the binding effects of the ligands at both ends to simultaneously bind to a specific target prot1ein and E3 ubiquitin ligase, thereby recruiting E3 ubiquitin ligase to the vicinity of the specific target protein and enabling E3 ubiquitin ligase to ubiquitinate the target protein. The polyubiquitinated target protein will be recognized and degraded by proteasomes in the body.

Over 600 distinct E3 ubiquitin ligases are involved in the design of proteolysis-targeting drugs; however, only a few have been successfully utilized in preclinical research on degraders. Among them, Cereblon (CRBN) E3 ubiquitin ligase is one of the most widely used E3 ubiquitin ligases. The known ligands for CRBN-type E3 ubiquitin ligase (CRBN ligands) include thalidomide and its analogs pomalidomide and lenalidomide, all of which possess a phthalimide backbone. Currently, several bifunctional protein degraders in clinical stages, such as ARV-110 targeting AR, ARV-471 targeting ER, NX-2127 targeting BTK, and FHD-609 targeting BRD9, are designed using CRBN ligands of the immunomodulatory imide drug (IMiD) type containing the phthalimide scaffold. Therefore, there is a pressing need to develop a new series of novel CRBN ligands possessing high CRBN binding affinity and drug-like properties. These ligands are intended for the design and synthesis of corresponding bifunctional protein degraders targeting various proteins, for the treatment and/or prevention of target protein-mediated or associated diseases or disorders.

Currently, molecular glue degraders directly target and eliminate specific proteins and offer potential advantages such as the ability to target "undruggable" proteins. Moreover, they typically exhibit low molecular weight and good drug-like properties, making them a major focus in drug development. In addition to the already marketed IMiD drugs, a range of molecular glue compounds based on the CRBN E3 ubiquitin ligase have been developed. Examples include CC-122, CC-220, CC-90009, CC-99282, and CC-92480 from Bristol Myers Squibb (BMS); DKY709 from Novartis; and CFT7455 from C4 Therapeutics, all of which are currently in clinical trials. The substrate spectrum of these molecular glues has also expanded from initially identified transcription factors IKZF1/3 to include casein kinase 1α (CK1α), zinc finger protein 91 (ZFP91), and translation termination factor GSPT1, among others. Degradation of these substrate proteins enables molecular glues to exert various pharmacological activities, such as immunomodulatory, anti-inflammatory, and antitumor effects, across different indications. Given this, the design and development of novel CRBN E3 ubiquitin ligase ligands for molecular glue degraders have become a prominent research focus.

In summary, the series of novel CRBN E3 ubiquitin ligase ligands we have designed can be utilized not only for developing bifunctional protein degraders but also for molecular glue degraders.

### Summary of Invention

In view of the above, an object of the present disclosure is to provide novel small molecule ligands for E3 ubiquitin ligases, bifunctional protein degraders designed based on such novel E3 ubiquitin ligase ligands and various target protein ligands, as well as their applications and usage methods. The E3 ligands provided herein exhibit stronger CRBN binding affinity and improved drug-like properties, and themselves demonstrate enhanced activity and/or selectivity as molecular glues for inducing substrate protein degradation. Furthermore, bifunctional protein degraders designed using the E3 ligands provided herein, targeting different proteins, show stronger degradation/inhibition effects against various classes or families of pathogenic proteins, favorable pharmacokinetic properties, and consequently elicit broad pharmacological activities.

To achieve the above objectives and other related purposes, in one aspect, the present disclosure provides a novel small-molecule ligand (thio-glutarimido isoindolinone derivative) for the CRBN E3 ubiquitin ligase. This ligand exhibits high CRBN-binding affinity and favorable drug-like properties, and can be used to treat various diseases or disorders, including tumors and autoimmune diseases.

In some embodiments, the thio-glutarimido isoindolinone derivativeof the present disclosure may be the compound of Formula (II): 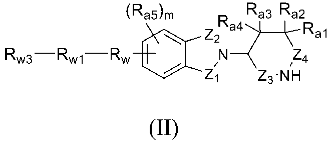 or salts, stereoisomers (including enantiomers), solvates, isotopically enriched analogs, prodrugs, or polymorphs thereof,
wherein Z₁ represents C(O), C(S), CH₂ or CD₂, and Z₂, Z₃, and Z₄ each independently represent C(O) or C(S), wherein at least one of Z₁, Z₂, Z₃, and Z₄ represents C(S);
Rₐ₁, Rₐ₂, Rₐ₃, and Rₐ₄ are identical or different and each independently represents hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy or halogenated C₁₋₆ alkoxy;
(Rₐ₅)ₘ indicates that the isoindoline ring to which it is attached is optionally substituted with m Rₐ₅, with each Rₐ₅ being identical or different and each independently representing halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2 or 3;
R_{w} represents O, S, S(O), S(O)₂, alkenylene, alkynylene or N(Rₐ₆), wherein Rₐ₆ represents H or C₁₋₃ alkyl, or R_{w} represents a bond; or
R_{w} represents: 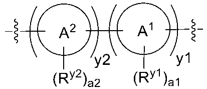
   wherein each ring A¹ is identical or different and each independently represents nitrogen-containing heterocyclylene, arylene or heteroarylene, y1 represents an integer of 1 or 2, and (R^{y1})ₐ₁ indicates that each ring A¹ is independently optionally substituted with a1 R^{y1} groups, with each R^{y1} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a1 represents an integer of 0-20;
   each ring A² is identical or different and each independently represents heterocyclylene, cycloalkylene, arylene or heteroarylene, y2 represents an integer of 0 or 1, and (R^{y2})ₐ₂ indicates that each ring A² is independently optionally substituted with a2 R^{y2} groups, with each R^{y2} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a2 represents an integer of 0-20; and
R_{w1} represents a bond or an optionally substituted linear or branched C₁₋₂₀alkylene, wherein any one or more methylene groups in the main carbon chain skeleton of the linear or branched C₁₋₂₀ alkylene are optionally replaced by one or more groups selected from Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when one or more adjacent methylene in the main carbon chain skeleton of the linear or branched C₁₋₂₀ alkylene are replaced by one or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof; and
R_{w3} represents hydrogen, deuterium, leaving group, hydroxy, halogen, COOH, NH₂, N₃, CHO, methanesulfonyloxy, trifluoromethanesulfonyloxy or p-toluenesulfonyloxy.

In another aspect, the bifunctional protein degraders designed based on such novel E3 ubiquitin ligase ligands and various target protein ligands may be the compound of Formula (I):

PBM-LIN-ULM (I)

or salts, stereoisomers (including enantiomers), solvates, isotopically enriched analogs, prodrugs, or polymorphs thereof,
wherein PBM represents a protein-binding moiety capable of binding to a target protein, and is covalently linked to ULM through LIN;
ULM represents a ligand moiety capable of binding to an E3 ubiquitin ligase, and represents the structure of the following Formula (ULM): 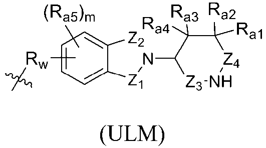
wherein Z₁ represents C(O), C(S), CH₂ or CD₂,; Z₂, Z₃, and Z₄ each independently represent C(O) or C(S), wherein at least one of Z₁, Z₂, Z₃, and Z₄ represents C(S);
Rₐ₁, Rₐ₂, Rₐ₃, and Rₐ₄ are identical or different and each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy or halogenated C₁₋₆ alkoxy;
(Rₐ₅)ₘ indicates that the isoindoline ring to which it is attached is optionally substituted with m Rₐ₅, with each Rₐ₅ being identical or different and each independently representing halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2 or 3;
R_{w} represents O, S, S(O), S(O)₂, alkenylene, alkynylene or N(Rₐ₆), wherein Rₐ₆ represents H or C₁₋₃ alkyl, or R_{w} represents a bond; or
R_{w} represents: 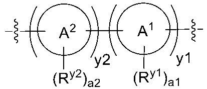
   wherein each ring A¹ is identical or different and each independently represents nitrogen-containing heterocyclylene, arylene or heteroarylene, y1 represents an integer of 1 or 2, and (R^{y1})ₐ₁ indicates that each ring A¹ is independently optionally substituted with a1 R^{y1} groups, with each R^{y1} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a1 represents an integer of 0-20;
   each ring A² is identical or different and each independently represents heterocyclylene, cycloalkylene, arylene or heteroarylene, y2 represents an integer of 0 or 1, and (R^{y2})ₐ₂ indicates that each ring A² is independently optionally substituted with a2 R^{y2} groups, with each R^{y2} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a2 represents an integer of 0-20; and
LIN represents: 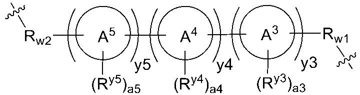
wherein R_{w2} is linked to PBM, and R_{w1} is linked to R_{w};
R_{w2} represents a bond, C(O), C(O)NH or NHC(O);
each ring A³ is identical or different and each independently represents heterocyclylene, cycloalkylene, arylene, or heteroarylene, y3 represents an integer of 0, 1, 2 or 3, and (R^{y3})ₐ₃ indicates that each ring A³ is independently optionally substituted with a3 R^{y3} substituents, wherein each R^{y3} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a3 represents an integer of 0-10;
each ring A⁴ is identical or different and each independently represents heterocyclylene, cycloalkylene, arylene, or heteroarylene, y4 represents an integer of 0, 1, 2 or 3, and (R^{y4})ₐ₄ indicates that each ring A⁴ is independently optionally substituted with a4 R^{y4} substituents, wherein each R^{y4} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a4 represents an integer of 0-10;
each ring A⁵ is identical or different and each independently represents heterocyclylene, cycloalkylene, arylene, or heteroarylene, y5 represents an integer of 0, 1, 2 or 3, and (R^{y5})ₐ₅ indicates that each ring A⁵ is independently optionally substituted with a5 R^{y5} substituents, wherein each R^{y5} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a5 represents an integer of 0-10;
R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when one or more methylene in the main carbon chain skeleton of the linear or branched C₁₋₂₀ alkylene are replaced by one or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof.

In a further aspect, the present disclosure provides a pharmaceutical composition comprising the compound of Formula (I) or salts, stereoisomers (including enantiomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, or the pharmaceutical composition comprising the same.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient, for use as a medicament.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient for use in the treatment and/or prevention of a disease or disorder selected from the group consisting of: tumors, cancers, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

In a further aspect, the present disclosure provides the use of the compound of Formula (I) or salts, stereoisomers (including enantiomers), solvates, isotopically enriched analogs, prodrugs, or polymorphs thereof, or the pharmaceutical composition as described herein, for the manufacture of a medicament for the prevention or treatment of a disease or disorder selected from the group consisting of: tumors, cancers, autoinflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

In a further aspect, the present disclosure provides a method for treating or preventing a disease or disorder in a subject, comprising administering to the subject a therapeutically effective amount of the compound of Formula (I) or salts, enantiomers, stereoisomers, solvates, isotopically enriched analogs, prodrugs, or polymorphs thereof, or the pharmaceutical composition comprising the same, wherein the disease or disorder comprises tumors, cancers, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

In a further aspect, the present disclosure also provides a use of the compound of Formula (II) or salts, stereoisomers (including enantiomers), solvates, isotopically enriched analogs, prodrugs, or polymorphs thereof for the preparation of the compound of Formula (I) or salts, enantiomers, stereoisomers, solvates, isotopically enriched analogs, prodrugs, or polymorphs thereof of the present disclosure.

In a further aspect, the present disclosure also provides a method for preparing the compound of Formula (I) or salts, enantiomers, stereoisomers, solvates, isotopically enriched analogs, prodrugs, or polymorphs thereof of the present disclosure by using the compound of Formula (II) or salts, stereoisomers (including enantiomers), solvates, isotopically enriched analogs, prodrugs, or polymorphs thereof.

In a further aspect, the present disclosure provides a pharmaceutical composition comprising the compound of Formula (II) or salts, stereoisomers (including enantiomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (II) or a pharmaceutically acceptable salt, or the pharmaceutical composition comprising the same.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient, for use as a medicament.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient for use in the prevention and/or treatment of a disease or disorder. The disease or disorder comprises: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

In a further aspect, the present disclosure provides the use of the compound of Formula (II) or pharmaceutically acceptable salts, stereoisomers (including enantiomers, diastereomers), solvates, isotopically enriched analogs, prodrugs, or polymorphs thereof, or the pharmaceutical composition as described herein, for the manufacture of a medicament for the prevention or treatment of a disease or disorder. The disease or disorder comprises: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

In a further aspect, the present disclosure provides a method for treating or preventing a disease or disorder in a subject, comprising administering to the subject a therapeutically effective amount of the compound of Formula (II) or salts, enantiomers, stereoisomers, solvates, isotopically enriched analogs, prodrugs, or polymorphs thereof, or the pharmaceutical composition comprising the same, wherein the disease or disorder comprises: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

### Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

### I. Compounds

### Compounds of Formula (I)

The compound of Formula (I) of the present disclosure, or salts (including pharmaceutically acceptable salts), stereoisomers (including enantiomers), solvates, isotopically enriched analogs, prodrugs, or polymorphs thereof, can bind to the target protein, recruit the target protein to an E3 ubiquitin ligase for ubiquitination, and subsequently lead to its degradation. The compound of Formula (I) of the present disclosure can specifically target particular classes or families of target proteins, and exhibit a broad spectrum of pharmacological activities by degrading/inhibiting diverse classes or families of target proteins.

PBM-LIN-ULM (I)

wherein PBM, LIN, and ULM are as defined in the compounds of Formula (I) above.

In some embodiments, the PBM may be a small molecule ligand that binds to specific target proteins, including but not limited to: epidermal growth factor receptor (EGFR), Cyclin-dependent kinase 4/6 (CDK4/6), anaplastic lymphoma kinase (ALK), Focal adhesion kinase (FAK), breakpoint cluster region (BCR)-Abelson leukemia virus (ABL) (BCR-ABL) tyrosine kinase, Bruton tyrosine kinase (BTK), Androgen receptor (AR), Estrogen receptor (ER), Bromodomain and extra-terminal domain protein (BET), Interleukin-1 receptor-associated kinase 4 (IRAK4), Cyclin-dependent kinase 9 (CDK9), Histone-lysine N-methyltransferase EZH2 (EZH2), B cell lymphoma-2 (BCL-2) family proteins, Cyclin-dependent kinase 2 (CDK2), serine/threonine protein kinase (AKT), microtubule-associated protein tau, SWI/SNF related, matrix associated, actin dependent regulator of chromatin, subfamily a, member 2/4 (SMARCA2/4), and ribosomal protein S6 kinase (RSK).

In some embodiments, said PBM comprises a structure selected from the following formula: 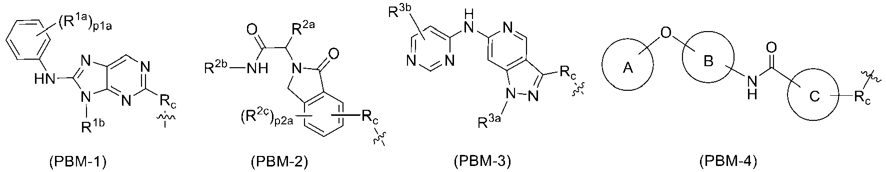 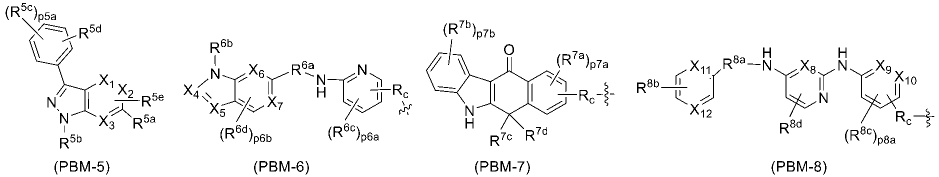 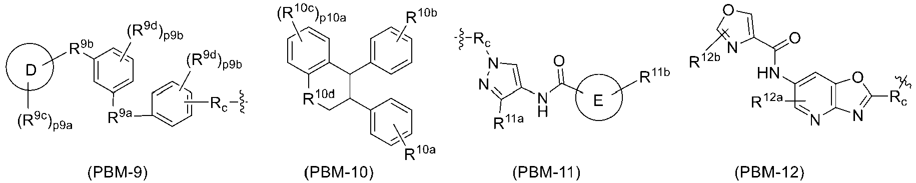 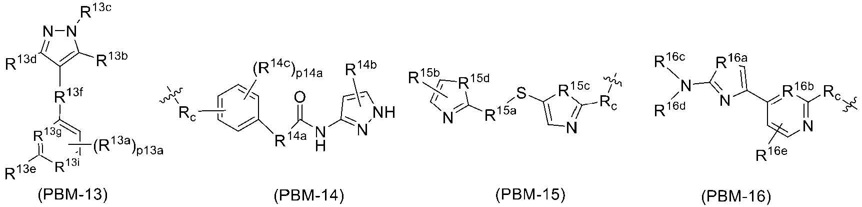 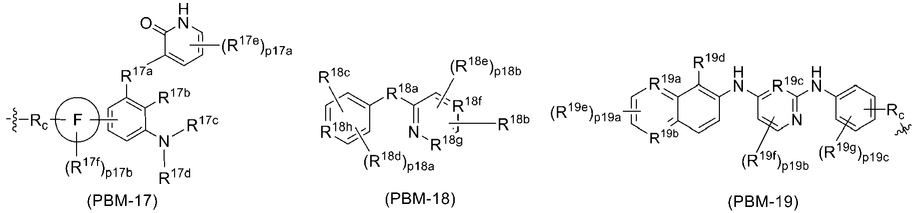 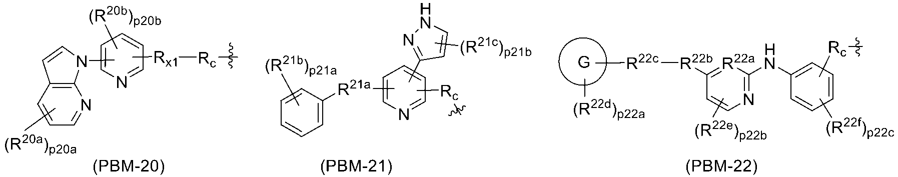 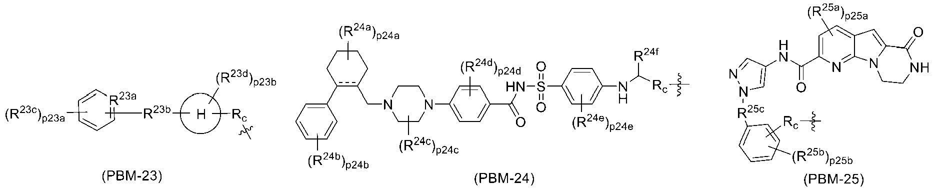 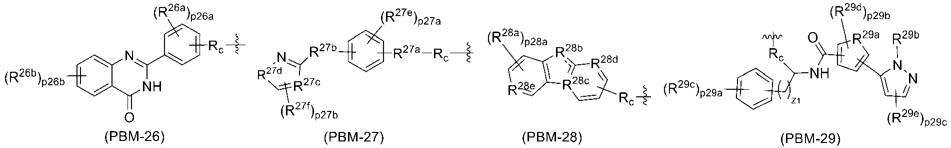 or 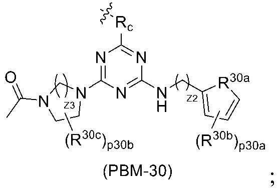
wherein (R^{1a})ₚ₁ₐ in Formula (PBM-1) indicates that the benzene ring to which it is attached is substituted with p1a R^{1a} groups, with each R^{1a} being identical or different and each independently representing deuterium, halogen, hydroxy, C₁₋₆ alkyl, deuterated C₁₋₆ alkyl, halogenated C₁₋₆ alkyl, C₁₋₆ alkoxy, halogenated C₁₋₆ alkoxy, NO₂, NH₂, or cyano;
p1a represents an integer of 0, 1, 2, 3, 4 or 5;
R^{1b} represents substituted or unsubstituted C₃₋₁₅ cycloalkyl, such as cyclopentyl;
R^{2a} in Formula (PBM-2) represents C₆₋₁₀ aryl or 5- to 20-membered heteroaryl, wherein the C₆₋₁₀ aryl and 5- to 20-membered heteroaryl are each independently optionally substituted with one or more (e.g., 1-10, 1-8, 2-6, 1-5, 1-4, 1-3, or 2-5) substituents independently selected from the group consisting of deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, and any combination thereof;
R^{2b} represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and 5- to 20-membered heteroaryl are each independently optionally substituted with one or more (e.g., 1-10, 1-8, 2-6, 1-5, 1-4, 1-3, or 2-5) R^{2d}, with each R^{2d} being identical or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R²⁰)ₚ₂ₐ indicates that the isoindoline ring in Formula (PBM-2) is optionally substituted with p2a R^{2c}, with each R^{2c} being identical or different and each independently representing deuterium, halogen, hydroxy, C₁₋₆ alkyl, halogenated C₁₋₆ alkyl, deuterated C₁₋₆ alkyl, NO₂, NH₂, C₁₋₆ alkoxy, halogenated C₁₋₆ alkoxy, or cyano;
p2a represents an integer of 0, 1, 2 or 3;
R^{3a} in Formula (PBM-3) represents hydrogen, C₁₋₁₀ alkyl (e.g., 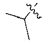 ), deuterated C₁₋₁₀ alkyl or halogenated C₁₋₁₀ alkyl;
R^{3b} represents 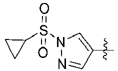 or 4- to 12-membered nitrogen-containing heterocyclyl, wherein the 4-to 12-membered nitrogen-containing heterocyclyl is optionally substituted with one or more (e.g., 1-10, 1-8, 2-6, 1-5, 1-4, 1-3, or 2-5) substituents independently selected from the group consisting of deuterium, halogen, hydroxy, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, NO₂, NH₂, cyano, and any combination thereof;
ring A in Formula (PBM-4) represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), and the ring A is optionally substituted with one or more (e.g., 1-10, 1-8, 2-6, 1-5, 1-4, 1-3, or 2-5) R^{4a}, with each R^{4a} being identical or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, or C₂₋₆ alkynyl;
ring B in Formula (PBM-4) represents C₃₋₁₅ cycloalkyl or 4- to 20-membered heterocyclyl (e.g., 4- to 20-membered heterocyclyl containing 1 to 4 heteroatoms selected from N, O and S), wherein the ring B is optionally substituted with one or more (e.g., 1-10, 1-8, 2-6, 1-5, 1-4, 1-3, or 2-5) R^{4b}, with each R^{4b} being identical or different and each independently representing deuterium, halogen, C₁₋₆ alkyl, halogenated C₁₋₆ alkyl, deuterated C₁₋₆ alkyl, C₁₋₆ alkoxy, or halogenated C₁₋₆ alkoxy;
ring C in Formula (PBM-4) represents 5- to 20-membered heteroaryl (e.g., 5- to 20-membered monocyclic or bicyclic heteroaryl containing 1 to 4 heteroatoms selected from N, O and S), wherein the ring C is optionally substituted with one or more (e.g., 1-10, 1-8, 2-6, 1-5, 1-4, 1-3, or 2-5) R^{4c}, with each R^{4c} being identical or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
X₁ in Formula (PBM-5) represents N or CR^{5f}, where R^{5f} represents hydrogen, deuterium, or amino, X₂ represents N or CR^{5g}, where R^{5g} represents hydrogen or represents R_{c}, and X₃ represents CH or N;
R^{5a} and R^{5b} each independently represent hydrogen or R_{c};
(R^{5c})ₚ₅ₐ in Formula (PBM-5) indicates that the benzene ring to which it is attached is substituted with p5a R^{5c}, with each R^{5c} being identical or different and each independently representing -O-aryl, -O-heteroaryl, 4- to 20-membered heterocyclyl, -C₁₋₃ alkylene-NHC(O)-heteroaryl, -C₁₋₃ alkylene-C(O)NH-heteroaryl, C₁₋₆ alkyl, deuterium, deuterated C₁₋₆ alkyl, halogen, or halogenated C₁₋₆ alkyl, wherein the 4-to 20-membered heterocyclyl, aryl, and heteroaryl are each independently optionally substituted with one or more (e.g., 1-10, 1-8, 2-6, 1-5, 1-4, 1-3, or 2-5) R^{5h}, with each R^{5h} being identical or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p5a represents an integer of 1, 2, 3 or 4;
R^{5d} represents R_{c}, hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
R^{5e} represents hydrogen, halogen, cyano, C₁₋₆ alkyl, halogenated C₁₋₆ alkyl, or amino;
wherein R^{5g}, R^{5a}, R^{5b}, and R^{5d} are not simultaneously hydrogen, and only one of R^{5g}, R^{5a}, R^{5b}, and R^{5d} represents R_{c}, wherein
   when R^{5a} represents R_{c}, X₂ represents CH or N, and R^{5b} is hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
   when X₂ represents CR^{5g}, where R^{5g} represents R_{c}, R^{5a} and R^{5b} are hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
   when R^{5b} represents R_{c}, X₂ represents N or CH, and R^{5a} is hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and
   when R^{5d} represents R_{c}, X₂ represents N or CH, and R^{5a} and R^{5b} are hydrogen;
X₄ in Formula (PBM-6) represents CR^{6e} or a fragment 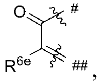 wherein symbol # indicates the point of attachment to N atom adjacent to X₄, symbol ## indicates the point of attachment to X₅, and each R^{6e} independently represents deuterium, hydrogen, halogen, C₁₋₆ alkyl, halogenated C₁₋₆ alkyl, deuterated C₁₋₆ alkyl, -C(O)-C₁₋₆ alkyl, -C(O)-deuterated C₁₋₆ alkyl or -C(O)NR^{6f}R^{6g}, where R^{6f} and R^{6g} are identical or different and each independently represent hydrogen, C₁₋₆ alkyl or deuterated C₁₋₆ alkyl;
X₅ in Formula (PBM-6) represents N or CR^{6h}, where R^{6h} represents hydrogen, deuterium, halogen, C₁₋₆ alkyl, deuterated C₁₋₆ alkyl or halogenated C₁₋₆ alkyl;
X₆ and X₇ each independently represent CH or N;
R^{6a} represents a bond or optionally substituted heteroarylene (e.g., heteroarylene substituted with halogen or deuterium);
R^{6b} represents hydrogen, C₁₋₆ alkyl, halogenated C₁₋₆ alkyl, deuterated C₁₋₆ alkyl, or optionally substituted C₃₋₁₂ cycloalkyl;
(R^{6c})ₚ₆ₐ in Formula (PBM-6) indicates that the pyridine ring to which it is attached is optionally substituted with p6a R^{6c}, with each R^{6c} being identical or different and each independently representing deuterium, halogen, C₁₋₆ alkyl, C₁₋₆ alkoxy, halogenated C₁₋₆ alkoxy, deuterated C₁₋₆ alkyl or halogenated C₁₋₆ alkyl, and p6a represents an integer of 0, 1, 2 or 3;
(R^{6d})_{p6b} in Formula (PBM-6) indicates that the nitrogen-containing bicyclic heteroaryl ring to which it is attached is optionally substituted with p6b R^{6d}, with each R^{6d} being identical or different and each independently representing deuterium, halogen, C₁₋₆ alkyl, C₁₋₆ alkoxy, halogenated C₁₋₆ alkoxy, deuterated C₁₋₆ alkyl or halogenated C₁₋₆ alkyl, and p6b represents an integer of 0 or 1;
(R^{7a})ₚ₇ₐ in Formula (PBM-7) indicates that the benzene ring to which it is attached is optionally substituted with p7a R^{7a}, with each R^{7a} being identical or different and each independently representing deuterium, cyano, halogen, C₁₋₆ alkyl, C₁₋₆ alkoxy, halogenated C₁₋₆ alkoxy, halogenated C₁₋₆ alkyl, or deuterated C₁₋₆ alkyl, and p7a represents an integer of 0, 1, 2 or 3;
(R^{7b})_{p7b} indicates that the benzene ring to which it is attached is optionally substituted with p7b R^{7b}, with each R^{7b} being identical or different and each independently representing deuterium, cyano, halogen, C₁₋₆ alkyl, C₁₋₆ alkoxy, halogenated C₁₋₆ alkoxy, halogenated C₁₋₆ alkyl, or deuterated C₁₋₆ alkyl, and p7b represents an integer of 0, 1, 2, 3 or 4;
R^{7c} and R^{7d} are identical or different and each independently represent deuterium, C₁₋₆ alkyl, halogen, halogenated C₁₋₆ alkyl, deuterated C₁₋₆ alkyl, C₁₋₆ alkoxy, or halogenated C₁₋₆ alkoxy;
X₈, X₉, X₁₀, X₁₁, and X₁₂ in Formula (PBM-8) are identical or different and each independently represent N or CH;
R^{8a} represents a bond or optionally substituted methylene (e.g., halogenated methylene);
R^{8b} represents -C(O)N(R^{8f})R^{8e}, -N(R^{8f})C(O)R^{8e}, -S(O)₂N(R^{8f})R^{8e}, -N(R^{8f})S(O)₂R^{8e}, -S(O)₂R^{8e} or - P(O)(R^{8e})₂, wherein each R^{8e} is identical or different and each independently represents C₁₋₆ alkyl or deuterated C₁₋₆ alkyl, and R^{8f} represents hydrogen or C₁₋₆ alkyl;
(R^{8c})ₚ₈ₐ in Formula (PBM-8) indicates that the 6-membered ring containing X₉ and X₁₀ to which it is attached is optionally substituted with p8a R^{8c}, with each R^{8c} being identical or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl or halogenated C₁₋₆ alkyl;
p8a represents an integer of 0, 1, 2, 3 or 4;
R^{8d} represents deuterium, halogen, hydroxy, amino, cyano, C₁₋₆ alkoxy, halogenated C₁₋₆ alkoxy, C₁₋₆ alkyl, deuterated C₁₋₆ alkyl or halogenated C₁₋₆ alkyl;
R^{9a} in Formula (PBM-9) represents -NHC(O)- or -C(O)NH-;
R^{9b} represents -NH-, -N(C₁₋₆ alkyl)-, or ethynylene;
ring D in Formula (PBM-9) represents a 5- to 20-membered monocyclic, bicyclic, or polycyclic heteroaryl group containing 1 to 4 heteroatoms selected from N, O, and S, and (R^{9c})ₚ₉ₐ indicates that the ring D is optionally substituted with p9a R^{9c}, with each R^{9c} being identical or different and each independently representing optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p9a represents an integer of 0, 1, 2, or 3;
each (R^{9d})_{p9b} in Formula (PBM-9) independently indicates that the benzene ring to which it is attached is independently optionally substituted with p9b R^{9d}, with each R^{9d} being identical or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl or halogenated C₁₋₆ alkyl;
each p9b is identical or different and each independently represents an integer of 0, 1, 2, 3 or 4 ;
R^{10d} in Formula (PBM-10) represents O, S or CH₂;
(R^{10c})ₚ₁₀ₐ indicates that the benzo-fused six-membered ring containing R^{10d} in Formula (PBM-10) is optionally substituted with p10a R^{10c}, with each R^{10c} being identical or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl or halogenated C₁₋₆ alkyl;
p10a represents an integer of 0, 1, 2, 3 or 4;
only one of R^{10a} and R^{10b} represents R_{c}, and the other represents hydrogen, halogen, hydroxy, cyano, amino, C₁₋₆ alkoxy, halogenated C₁₋₆ alkoxy, C₁₋₆ alkyl, deuterated C₁₋₆ alkyl or halogenated C₁₋₆ alkyl;
ring E in Formula (PBM-11) represents a 5- to 20-membered monocyclic, bicyclic, or polycyclic heteroarylene group containing 1 to 4 heteroatoms selected from N, O, and S;
R^{11b} represents optionally substituted 5- to 15-membered heteroaryl, optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{11a} represents hydrogen, halogen, C₁₋₆ alkyl, halogenated C₁₋₆ alkyl, deuterated C₁₋₆ alkyl, C₁₋₆ alkoxy, or halogenated C₁₋₆ alkoxy;
R^{12a} in Formula (PBM-12) represents optionally substituted 5- to 15-membered heterocyclyl, optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{12b} represents a 5- to 20-membered monocyclic, bicyclic, or polycyclic heteroaryl group containing 1 to 4 heteroatoms selected from N, O, and S, and the heteroaryl is optionally substituted with one or more (e.g., 1-10, 1-8, 2-6, 1-5, 1-4, 1-3, or 2-5) substituents selected from the group consisting of deuterium, halogen, hydroxy, cyano, amino, C₁₋₆ alkyl, halogenated C₁₋₆ alkyl, deuterated C₁₋₆ alkyl, C₁₋₆ alkoxy, halogenated C₁₋₆ alkoxy, and any combination thereof;
R^{13g} in Formula (PBM-13) represents N or CR^{13h}, where R^{13h} represents hydrogen or halogen,; R¹³ⁱ represents N or CR^{13j}, where R^{13j} represents hydrogen or halogen;
R^{13f} represents a bond, -C(O)NH-, -NHC(O)- , -S(O)₂NH- or -NHS(O)₂-;
(R^{13a})ₚ₁₃ₐ indicates that the 6-membered ring containing R^{13g} and R¹³ⁱ in Formula (PBM-13) is optionally substituted with p13a R^{13a}, with each R^{13a} being identical or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl or halogenated C₁₋₆ alkyl;
p13a represents an integer of 0, 1 or 2 ;
R¹³⁶ represents hydrogen, halogen, C₁₋₆ alkyl, deuterated C₁₋₆ alkyl, halogenated C₁₋₆ alkyl or -C₁₋₃alkylene-C₃₋₆cycloalkyl;
R^{13c} represents hydrogen, C₁₋₆ alkyl, deuterated C₁₋₆ alkyl or halogenated C₁₋₆ alkyl; or
R^{13b} and R^{13c} together with their respective attached nitrogen and carbon atoms form an optionally substituted 5- to 7-membered nitrogen-containing heterocycle;
R^{13d} represents hydrogen or R_{c}, and R^{13c} represents hydrogen or R_{c}, wherein R^{13d} and R^{13e} are not simultaneously hydrogen, and among R^{13d} and R^{13e}, there is one and only one that represents R_{c}, while the other represents hydrogen;
R^{14a} in Formula (PBM-14) represents a bond, C₁₋₃ alkylene or halogenated C₁₋₃ alkylene;
R^{14b} represents optionally substituted halogen, C₁₋₆ alkyl, C₁₋₆ alkoxy, halogenated C₁₋₆ alkoxy, deuterated C₁₋₆ alkyl, halogenated C₁₋₆ alkyl, optionally substituted C₃₋₆cycloalkyl, or optionally substituted C₁₋₆ alkyl (e.g., C₁₋₆ alkyl which is optionally substituted with aryl);
(R^{14c})ₚ₁₄ₐ indicates that the benzene ring in Formula (PBM-14) is optionally substituted with p14a R^{14c}, with each R^{14c} being identical or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl or halogenated C₁₋₆ alkyl;
p14a represents an integer of 0, 1, 2, 3 or 4;
R^{15a} in Formula (PBM-15) represents C₁₋₃ alkylene or halogenated C₁₋₃ alkylene;
R^{15b} represents deuterium, C₁₋₆ alkyl, deuterated C₁₋₆ alkyl, halogenated C₁₋₆ alkyl, or optionally substituted C₃₋₆ cycloalkyl;
R^{15c} and R^{15d} each independently represent NH, S or O;
R^{16a} in Formula (PBM-16) represents -S- or a fragment 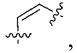 wherein when R^{16a} represents -S-, the heteroaryl containing R^{16a} and nitrogen atom in Formula (PBM-16) is thiazolyl, and when R^{16a} represents the fragment 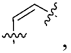 the heteroaryl containing R^{16a} and nitrogen atom in Formula (PBM-16) is pyridyl;
R^{16b} represents N or CH;
R^{16c} represents hydrogen or optionally substituted C₁₋₁₀ alkyl;
R^{16d} represents hydrogen, -C₁₋₃alkylene-optionally substituted 4- to 10-membered heterocyclyl, or optionally substituted 4- to 10-membered heterocyclyl;
R^{16e} represents hydroxy, halogen, C₁₋₆ alkyl, C₁₋₆ alkoxy, halogenated C₁₋₆ alkoxy, deuterated C₁₋₆ alkyl or halogenated C₁₋₆ alkyl;
R^{17a} in Formula (PBM-17) represents C(O)NH, NHC(O), (CH₂)₁₋₂C(O)NH, (CH₂)₁₋₂NHC(O), C(O)NH(CH₂)₁₋₂, NHC(O)(CH₂)₁₋₂, S(O)₂NH or NHS(O)₂;
R^{17b} represents hydrogen, hydroxy, halogen, cyano, C₁₋₆ alkoxy, halogenated C₁₋₆ alkoxy, C₁₋₆ alkyl, deuterated C₁₋₆ alkyl or halogenated C₁₋₆ alkyl;
R^{17c} represents hydrogen, C₁₋₆ alkyl, deuterated C₁₋₆ alkyl or halogenated C₁₋₆ alkyl; or
R^{17b} and R^{17c}, together with their respective attached carbon and nitrogen atoms and the carbon atom on the benzene ring attached to the nitrogen atom, form an optionally substituted 4- to 6-membered heteroaromatic ring or an optionally substituted 4- to 6-membered heterocycle ring;
R^{17d} represents optionally substituted C₃₋₆ cycloalkyl, C₁₋₆ alkyl, deuterated C₁₋₆ alkyl, halogenated C₁₋₆ alkyl or optionally substituted 4- to 20-membered heterocyclyl;
(R^{17e})ₚ₁₇ₐ indicates that pyridin-2(1H)-one ring to which it is attached is substituted with p17a R^{17e}, with each R^{17e} being identical or different and each independently representing hydroxy, halogen, cyano, C₁₋₆ alkyl, C₁₋₆ alkoxy, halogenated C₁₋₆ alkoxy, deuterated C₁₋₆ alkyl or halogenated C₁₋₆ alkyl;
p17a represents an integer of 0, 1, 2 or 3;
ring F in Formula (PBM-17) represents arylene or 5- to 20-membered monocyclic or bicyclic heteroarylene containing from 1 to 4 heteroatoms selected from the group consisting of oxygen, nitrogen, and sulfur; and (R^{17f})_{p17b} indicates that the ring F is optionally substituted with p17b R^{17f}, with each R^{17f} being identical or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p17b represents an integer of 0, 1, 2, 3 or 4;
R^{18f}, R^{18g}, and R^{18h} in Formula (PBM-18) each independently represent CH or N;
R^{18a} represents a bond, S or NH;
R^{18b} represents R_{c}, optionally substituted cycloalkyl or optionally substituted 4- to 15-membered nitrogen-containing heterocyclyl;
R^{18c} represents R_{c}, halogen, amino, hydroxy, cyano, C₁₋₆ alkyl, C₁₋₆ alkoxy, halogenated C₁₋₆ alkoxy, deuterated C₁₋₆ alkyl, halogenated C₁₋₆ alkyl, optionally substituted cycloalkyl or optionally substituted 4-to 15-membered nitrogen-containing heterocyclyl;
wherein R^{18b} and R^{13c} do not simultaneously represent R_{c}, and one and only one of R^{18b} and R^{18c} represents represents R_{c};
(R^{18d})ₚ₁₈ₐ indicates that the 6-membered ring to which it is attached is optionally substituted with p18a R^{18d}, with each R^{18d} being identical or different and each independently representing hydroxy, amino, cyano, halogen, C₁₋₆ alkyl, C₁₋₆ alkoxy, halogenated C₁₋₆ alkoxy, deuterated C₁₋₆ alkyl or halogenated C₁₋₆ alkyl;
p18a represents an integer of 0, 1, 2, 3 or 4;
(R^{18e})_{p18b} indicates that the 6-membered nitrogen-containing heteroaryl ring to which it is attached is optionally substituted with p18b R^{18e}, with each R^{18c} being identical or different and each independently representing hydroxy, amino, cyano, halogen, C₁₋₆ alkyl, C₁₋₆ alkoxy, halogenated C₁₋₆ alkoxy, deuterated C₁₋₆ alkyl or halogenated C₁₋₆ alkyl;
p18b represents an integer of 0, 1 or 2;
R^{19a}, R^{19b}, and R^{19c} in Formula (PBM-19) each independently represent CH or N;
R^{19d} represents -C(O)NHR^{19h} -NHC(O)R^{19h}, -S(O)₂NHR^{19h}, -NHS(O)₂R^{19h}, -S(O)₂R^{19h} or - P(O)(R^{19h})₂, wherein each R^{19h} is identical or different and each independently represents C₁₋₆ alkyl, deuterated C₁₋₆ alkyl or halogenated C₁₋₆ alkyl;
(R^{19e})ₚ₁₉ₐ represents p19a R^{19e} groups, wherein each R^{19e} is identical or different and each independently represents halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p19a represents an integer of 0, 1, 2, 3 or 4;
(R^{19f})_{p19b} represents p19b R^{19f} groups, wherein each R^{19f} is identical or different and each independently represents halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p19b represents an integer of 0, 1 or 2;
(R^{19g})_{p19c} indicates that the benzene ring to which it is attached is optionally substituted with p19c R^{19g}, with each R^{19g} each independently representing halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p19c represents an integer of 0, 1, 2, 3 or 4;
R_{c1} in Formula (PBM-20) represents a bond, or R_{c1} represents -NH-R_{c2}-C(O)-***, wherein symbol *** indicates the point of attachment to R_{c}, and R_{c2} represents optionally substituted C₃₋₁₅cycloalkyl;
(R^{20a})ₚ₂₀ₐ indicates that the 1H-pyrrolo[2,3-b]pyridine ring to which it is attached is optionally substituted with p20a R^{20a}, wherein each R^{20a} is independently cyano, halogen, hydroxy, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p20a represents an integer of 0, 1, 2, 3, 4 or 5;
(R^{20b})_{p20b} indicates that the pyridine ring to which it is attached is optionally substituted with p20b R^{20b}, wherein each R^{20b} is independently cyano, halogen, hydroxy, nitro, C₁₋₆ alkyl, halogenated C₁₋₆ alkyl, deuterated C₁₋₆ alkyl, C₁₋₆ alkoxy, or halogenated C₁₋₆ alkoxy or NR^{20c}R^{20d}, where R^{20c} and R^{20d} are identical or different and each independently represent hydrogen, C₁₋₆ alkyl, deuterated C₁₋₆ alkyl, halogenated C₁₋₆ alkyl, optionally substituted C₃₋₁₅cycloalkyl or optionally substituted 4- to 15-membered heterocyclyl, and p20b represents an integer of 0, 1, 2 or 3;
R^{21a} in Formula (PBM-21) represents NHC(O), C(O)NH, NHS(O)₂ or S(O)₂NH;
(R^{21b})ₚ₂₁ₐ indicates that the benzene ring to which it is attached is optionally substituted with p21a R^{21b} , wherein each R^{21b} is independently halogen, hydroxy, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p21a represents an integer of 0, 1, 2, 3, 4 or 5;
(R^{21c})_{p21b} indicates that the 1H-pyrazole ring to which it is attached is optionally substituted with p21b R^{21c} , wherein each R^{21c} is independently halogen, hydroxy, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p21b represents an integer of 0, 1 or 2;
R^{22a} in Formula (PBM-22) represents N or CH, and R^{22b} represents O, S, NH, CH₂, CH(F) or C(F)₂;
R^{22c} represents a bond or optionally substituted methylene (e.g., halogenated methylene);
ring G represents C₆₋₁₅ aryl, 4- to 15-membered heterocyclyl, or 5- to 15-membered heteroaryl, and (R^{22d})ₚ₂₂ₐ indicates that the ring G to which it is attached is optionally substituted with p22a R^{22d} , wherein each R^{22d} is identical or different and each independently represents deuterium, halogen, hydroxy, cyano, amino, nitro, oxo, C₁₋₆ alkyl, halogenated C₁₋₆ alkyl, deuterated C₁₋₆ alkyl, C₁₋₆ alkoxy, or halogenated C₁₋₆ alkoxy, and p22a represents an integer of 0, 1, 2, 3 or 4;
(R^{22e})_{p22b} indicates that the 6-membered nitrogen-containing heteroaromatic ring containing R^{22a} to which it is attached is optionally substituted with p22b R^{22c} , wherein each R^{22c} independently represents deuterium, halogen, hydroxy, cyano, amino, nitro, C₁₋₆ alkoxy, halogenated C₁₋₆ alkoxy, C₁₋₆ alkyl, deuterated C₁₋₆ alkyl or halogenated C₁₋₆ alkyl, and p22b represents an integer of 0, 1 or 2;
(R^{22f})_{p22c} indicates that the benzene ring to which it is attached is optionally substituted with p22c R^{22f} , wherein each R^{22f} is identical or different and each independently represents deuterium, halogen, hydroxy, cyano, amino, nitro, C₁₋₆ alkoxy, halogenated C₁₋₆ alkoxy, C₁₋₆ alkyl, deuterated C₁₋₆ alkyl or halogenated C₁₋₆ alkyl, and p22c represents an integer of 0, 1, 2, 3 or 4;
R^{23a} in Formula (PBM-23) represents N or CH;
R^{23b} represents NHC(O), C(O)NH, NHS(O)₂ or S(O)₂NH;
(R^{23c})ₚ₂₃ₐ indicates that the 6-membered ring containing R^{23a} is substituted with p23a R^{23c} , wherein each R^{23c} is identical or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p23a represents an integer of 0, 1, 2, 3 or 4;
ring H represents 5- to 15-membered heteroarylene, and (R^{23d})_{p23b} indicates that the ring H to which it is attached is optionally substituted with p23b R^{23d} , wherein each R^{23d} is identical or different and each independently represents halogen, hydroxy, mercapto, cyano, amino, nitro, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, halogenated C₁₋₆ alkoxy or optionally substituted C₃₋₁₅cycloalkyl, and p23b represents an integer of 0, 1, 2 or 3;
the dashed line --- in Formula (PBM-24) indicates the optional presence of a double bond;
(R^{24a})ₚ₂₄ₐ indicates that the 6-membered cycloalkyl ring to which it is attached is optionally substituted with p24a R^{24a} , wherein each R^{24a} is identical or different and each independently represents halogen, hydroxy, mercapto, cyano, amino, nitro, oxo, C₁₋₆ alkyl, halogenated C₁₋₆ alkyl, deuterated C₁₋₆ alkyl, C₁₋₆ alkoxy or halogenated C₁₋₆ alkoxy, and p24a represents an integer of 0, 1, 2, 3, 4, 5, 6, 7 or 8;
(R^{24b})_{p24b} indicates that the benzene ring to which it is attached is optionally substituted with p24b R^{24b} , wherein each R^{24b} is identical or different and each independently represents deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p24b represents an integer of 0, 1, 2, 3, 4 or 5;
(R^{24c})_{p24c} indicates that the 6-membered cycloalkyl to which it is attached is optionally substituted with p24c R^{24c} , wherein each R^{24c} is identical or different and each independently represents halogen, cyano, oxo, C₁₋₆ alkyl, halogenated C₁₋₆ alkyl, deuterated C₁₋₆ alkyl, C₁₋₆ alkoxy or halogenated C₁₋₆ alkoxy, and p24c represents an integer of 0, 1, 2, 3, 4, 5, 6, 7 or 8;
(R^{24d})_{p24d} indicates that the benzene ring to which it is attached is optionally substituted with p24d R^{24d} , wherein each R^{24d} is identical or different and each independently represents halogen, hydroxy, mercapto, C₁₋₆ alkyl, halogenated C₁₋₆ alkyl, deuterated C₁₋₆ alkyl, C₁₋₆ alkoxy, halogenated C₁₋₆ alkoxy or 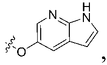 and p24d represents an integer of 0, 1 or 2;
(R^{24e})ₚ₂₄ₑ indicates that the benzene ring to which it is attached is optionally substituted with p24e R^{24e} , wherein each R^{24e} is identical or different and each independently represents deuterium, halogen, hydroxy, mercapto, cyano, amino, nitro, -SO₂CF₃, C₁₋₆ alkyl, halogenated C₁₋₆ alkyl, deuterated C₁₋₆ alkyl, C₁₋₆ alkoxy or halogenated C₁₋₆ alkoxy, and p24e represents an integer of 0, 1, 2, 3 or 4;
R^{24f} represents hydrogen, deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy or 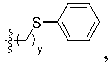 wherein y represents an integer of 1, 2 or 3;
(R^{25a})ₚ₂₅ₐ in Formula (PBM-25) represents p25a R^{25a} groups, wherein each R^{25a} is identical or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p25a represents an integer of 0, 1, 2, 3, 4 or 5;
(R^{25b})_{p25b} indicates that the benzene ring to which it is attached is optionally substituted with p25b R^{25b}, wherein each R^{25b} is identical or different and each independently represents deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p25b represents an integer of 0, 1, 2, 3 or 4; and
R^{25c} represents C₁₋₆ alkylene or halogenated C₁₋₆ alkylene;
(R^{26a})ₚ₂₆ₐ in Formula (PBM-26) indicates that the benzene ring to which it is attached is optionally substituted with p26a R^{26a} , wherein each R^{26a} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p26a represents an integer of 0, 1, 2, 3 or 4;
(R^{26b})_{p26b} indicates that the 4-oxo-3,4-dihydroquinazoline ring to which it is attached is substituted with p26b R^{26b} , wherein each R^{26b} is identical or different and each independently represents halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p26b represents an integer of 1, 2, 3 or 4;
R^{27a} represents S(O)₂ or a bond;
R^{27b} represents NH, NHC(O) or C(O)NH;
R^{27c} represents CH or N;
R^{27d} represents N(R^{27h}), wherein R^{27h} represents H, -C(O)-optionally substituted aryl or -C(O)-optionally substituted heteroaryl; or
R^{27d} represents a fragment 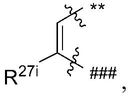 wherein symbol ** indicates the point of attachment to the adjacent nitrogen atom, symbol ### indicates the point of attachment to the adjacent carbon atom, and R²⁷ⁱ represents deuterium, hydrogen, halogen, C₁₋₆ alkyl, halogenated C₁₋₆ alkyl or deuterated C₁₋₆ alkyl;
(R^{27e})ₚ₂₇ₐ indicates that the benzene ring to which it is attached is optionally substituted with p27a R^{27e}, wherein each R^{27e} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p27a represents an integer of 0, 1, 2, 3 or 4;
(R^{27f})_{p27b} indicates that the nitrogen-containing ring to which it is attached is substituted by p27b R^{27f}, wherein each R^{27f} is identical or different and each independently represents deuterium, amino, halogen, hydroxy, cyano, C₁₋₆ alkoxy, halogenated C₁₋₆ alkoxy, C₁₋₆ alkyl, deuterated C₁₋₆ alkyl, halogenated C₁₋₆ alkyl, -NH-optionally substituted aryl, or -NH-optionally substituted heteroaryl;
p27b represents an integer of 1 or 2;
(R^{28a})ₚ₂₈ₐ in Formula (PBM-28) indicates that the fused tricyclic ring to which it is attached is optionally substituted by p28a R^{28a} substituents, wherein each R^{28a} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl; p28a represents an integer of 0, 1, 2, 3, 4, 5 or 6;
-̅ -̅ -̅ in Formula (PBM-28) represents a single bond or a double bond;
a fragment 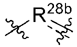 of the fused tricyclic ring in Formula (PBM-28) represents 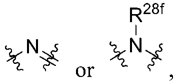 wherein R^{28f} represents hydrogen, C₁₋₆ alkyl, deuterated C₁₋₆ alkyl, or halogenated C₁₋₆ alkyl;
wherein when the fragment 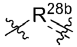 represents 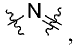 the fragment 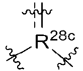 represents 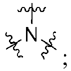
when the fragment 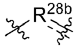 represents 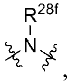 the fragment 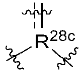 represents 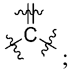
R^{28d}, and R^{28e} are identical or different and each independently represents N or CH;
R^{29a} in Formula (PBM-29) represents O, S or NH;
R^{29b} represents hydrogen, C₁₋₆ alkyl, deuterated C₁₋₆ alkyl, or halogenated C₁₋₆ alkyl;
Z1 represents an integer of 1, 2 or 3;
(R^{29c})ₚ₂₉ₐ indicates that the benzene ring to which it is attached is optionally substituted with p29a R^{29c} substituents, wherein each R^{29c} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p29a represents an integer of 0, 1, 2, 3, 4 or 5;
(R^{29d})_{p29b} indicates that the 5-membered ring containing R^{29a} to which it is attached is optionally substituted with p29b R^{29d} , wherein each R^{29d} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p29b represents an integer of 0, 1 or 2;
(R^{29e})_{p29c} indicates that the pyrazole ring to which it is attached is optionally substituted with p29c R^{29e} , wherein each R^{29e} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p29c represents an integer of 0, 1 or 2;
R^{30a} in Formula (PBM-30) represents O, S or NH;
(R^{30b})ₚ₃₀ₐ indicates that the 5-membered ring containing R^{30a} to which it is attached is optionally substituted with p30a R^{30b} substituents, wherein each R^{30b} is identical or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p30a represents an integer of 0, 1, 2 or 3;
(R^{30c})_{p30b} indicates that the diazaheterocyclyl to which it is attached is optionally substituted by p30b R^{30c} substituents, wherein each R^{30c} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p30b represents an integer of 0, 1, 2, 3, 4, 5, 6, 7, 8, 9 or 10;
Z2 represents an integer of 1, 2 or 3;
Z3 represents an integer of 1, 2, 3, 4, 5 or 6; and
R_{c} in each general formula independently represents a bond, -O-, -N(R_{d})-, -NHC(O)-*, -C(O)NH-*, - N(R_{d})-R_{f}-N(Rₑ)-*, -R_{f}-C(O)NH-*, -R_{f}-NHC(O)-*, -R_{f}-C(O)O-*, -R_{f}-OC(O)-*, -C(O)O-*, -OC(O)-*, -R_{f}-, -R_{f}-N(R_{d})-*, -O-R_{f}-N(R_{d})-*, 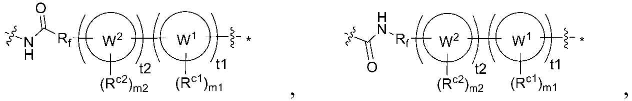 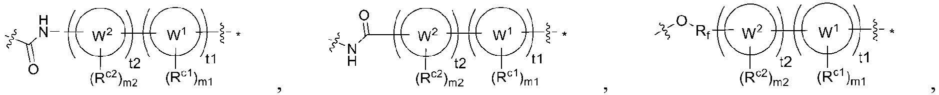 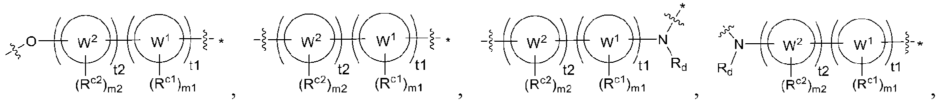 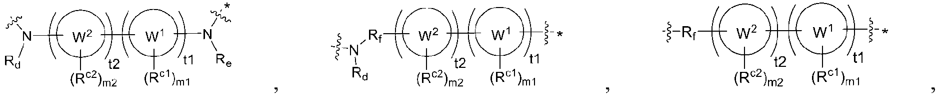 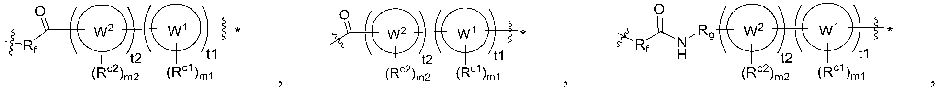 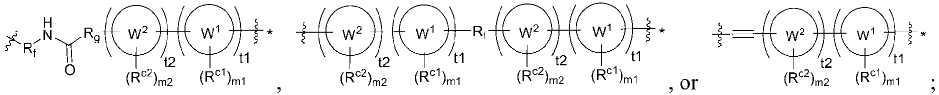
   wherein each ring W¹ is identical or different and each independently represents nitrogen-containing heterocyclylene, t1 represents an integer of 1 or 2, and (R^{c1})ₘ₁ indicates that each ring W¹ is independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} represents optionally substituted C₁₋₆alkylene or optionally substituted C₂₋₆alkenylene, and R^{c4} represents halogen, R^{c5}R^{c6}N-, hydroxy, carboxyl, ethynyl, or vinyl, wherein R^{c5} and R^{c6} are identical or different and each independently represent hydrogen or C₁₋₃ alkyl, and m1 represents an integer of 0-20;
   each ring W² is identical or different and each independently represents heterocyclylene, cycloalkylene, arylene, or heteroarylene, t2 represents an integer of 0 or 1, and (R^{c2})ₘ₂ indicates that each ring W² is independently optionally substituted with m2 R^{c2} groups, wherein each R^{c2} is independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, or R^{c8}-R^{c7}-, wherein R^{c7} represents optionally substituted C₁₋₆ alkylene or optionally substituted C₂₋₆ alkenylene, R^{c8} represents halogen, R^{c9}R^{c10}N-, hydroxy, carboxyl, ethynyl, or vinyl, wherein R^{c9} and R^{c10} are identical or different and each independently represent hydrogen or C₁₋₃ alkyl, and m2 represents an integer of 0-20; and
R_{d} and Rₑ each independently represent hydrogen or C₁₋₆ alkyl; R_{f} and R_{g} each independently represent optionally substituted C₁₋₆ alkylene or optionally substituted C₂₋₆ alkenylene; and symbol * indicates the point of attachment to LIN.

In the embodiments of the present disclosure, the number of substituents is not theoretically limited in any way, or is automatically limited by the size of the building units (i.e., the total number of replaceable hydrogen atoms in the building units), or as clearly defined herein.

In some embodiments, PBM represents a small molecule ligand of EGFR.

In some embodiments, PBM represents a structure of Formula (PBM-1): 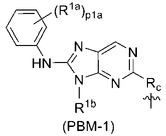
wherein (R^{1a})ₚ₁ₐ indicates that the benzene ring to which it is attached is substituted with p1a R^{1a} groups, with each R^{1a} being identical or different and each independently representing deuterium, halogen (e.g., fluorine, chlorine, bromine, or iodine), hydroxy, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), NO₂, NH₂, or cyano;
p1a represents an integer of 0, 1, 2, 3, 4 or 5;
R^{1b} represents substituted or unsubstituted C₃₋₁₅ cycloalkyl (e.g., substituted or unsubstituted C₃₋₁₀ cycloalkyl or C₃₋₆ cycloalkyl, such as cyclopropyl, cyclobutyl, cyclopentyl, or cyclohexyl). In some embodiments, the C₃₋₁₅ cycloalkyl is optionally substituted with one or more (e.g., 1-3, 1, 2 or 3) substituents selected from the group consisting of deuterium, hydroxy, amino, mercapto, nitro, halogen (e.g., fluorine, chlorine, bromine, or iodine), cyano, C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally halogenated C₃₋₆cycloalkyl, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl-NH-, NH₂-C₁₋₆alkylene, C₁₋₆ alkyl-NHC(O)-, C₁₋₆ alkyl-C(O)NH-, and any combination thereof.

In some embodiments, PBM represents the structure of Formula (PBM-1-1):

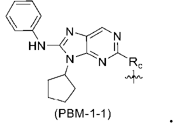

In some embodiments, PBM represents a structure of Formula (PBM-2-1A), Formula (PBM-2-1B), Formula (PBM-2-1C), or Formula (PBM-2-1D) (which are non-limiting examples of the structure of Formula (PBM-2)): 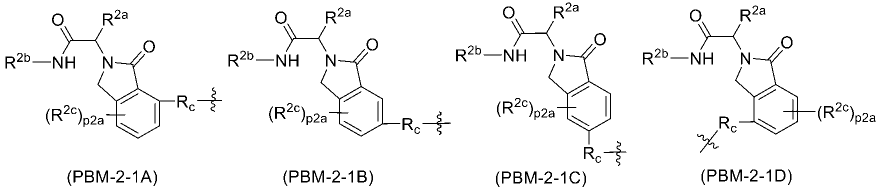 wherein in each of Formula (PBM-2-1A), Formula (PBM-2-1B), Formula (PBM-2-1C), and Formula (PBM-2-1D),
R^{2a} represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and 5- to 20-membered heteroaryl are each independently optionally substituted with one or more (e.g., 1-6, such as 1, 2, 3, 4, 5 or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₆ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof;
R^{2b} represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and 5- to 20-membered heteroaryl are each independently optionally substituted with one or more (e.g., 1-6, such as 1, 2, 3, 4, 5 or 6) substituents R^{2d} independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
(R^{2c})ₚ₂ₐ indicates that the isoindoline ring to which it is attached is optionally substituted with p2a R^{2c}, with each R^{2c} being identical or different and each independently representing deuterium, halogen (e.g., fluorine, chlorine, bromine, or iodine), hydroxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), NO₂, NH₂, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or cyano; and
p2a represents an integer of 0, 1, 2 or 3.

In some embodiments, R^{2a} represents C₆₋₁₀ aryl, such as but not limited to phenyl or naphthyl, each of which is optionally substituted with one or more (e.g., 1-6, such as 1, 2, 3, 4, 5, or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, R^{2a} represents 5- to 20-membered heteroaryl, e.g., 5- to 20-membered heteroaryl containing one or two 5- to 7-membered rings and 1 to 4 heteroatoms selected from N, O, and S. In a sub-embodiment, R^{2a} represents 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, imidazo[1,2-b]pyridazinyl, or 6,7-dihydro-5H-pyrrolo[1,2-c]imidazolyl. The heteroaryl group is optionally substituted with one or more (e.g., 1-6, such as 1, 2, 3, 4, 5, or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, R^{2b} represents C₆₋₁₀ aryl, such as but not limited to phenyl or naphthyl, each of which is optionally substituted with one or more (e.g., 1-6, such as 1, 2, 3, 4, 5, or 6) substituents R2d as defined above.

In some embodiments, R^{2b} represents heteroaryl containing one or two 5- to 7-membered rings and 1 to 4 heteroatoms selected from N, O, and S. In a sub-embodiment, R^{2b} represents 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl group is optionally substituted with one or more (e.g., 1-6, such as 1, 2, 3, 4, 5, or 6) substituents R2d as defined above.

In some embodiments, PBM represents a structure of Formula (PBM-2-1), Formula (PBM-2-2), Formula (PBM-2-3), Formula (PBM-2-4), or Formula (PBM-2-5):

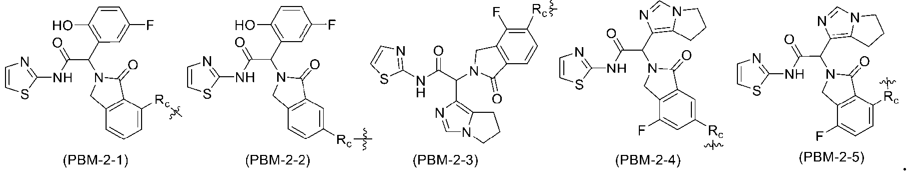

In some embodiments, PBM represents a structure of Formula (PBM-3): 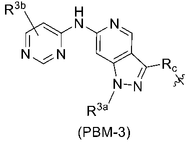
wherein R^{3a} represents hydrogen, C₁₋₁₀ alkyl (e.g., C₁₋₅ alkyl, C₁₋₅ alkyl or C₁₋₄ alkyl, such as methyl, ethyl, propyl, isopropyl ( 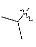 ), butyl, sec-butyl, tert-butyl, pentyl, hexyl, heptyl, or octyl), deuterated C₁₋₁₀ alkyl (e.g., perdeuterated C₁₋₅ alkyl, perdeuterated C₁₋₆ alkyl or perdeuterated C₁₋₄ alkyl, such as CD₃, CD₃CD₂-, CD₃CD₂CD₂- etc.), or halogenated C₁₋₁₀ alkyl (e.g., halogenated C₁₋₅ alkyl or halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
R^{3b} represents 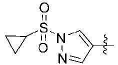 or 4- to 12-membered nitrogen-containing heterocyclyl, wherein the 4-to 12-membered nitrogen-containing heterocyclyl is optionally substituted with one or more substituents independently selected from the group consisting of deuterium, halogen (e.g., fluorine, chlorine, bromine, or iodine), hydroxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy (e.g., perdeuterated C₁₋₄ alkoxy, such as CD₃-O-, CD₃CD₂-O-, or CD₃CD₂CD₂-O-), NO₂, NH₂, cyano, and any combination thereof. In some sub-embodiments, examples of 4- to 12-membered nitrogen-containing heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl).

In some embodiments, PBM represents a structure of Formula (PBM-3-1) or Formula (PBM-3-2):

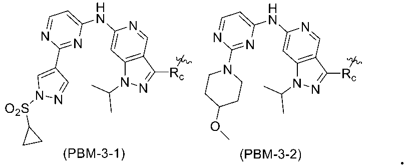

In some embodiments, PBM represents a structure of Formula (PBM-19): 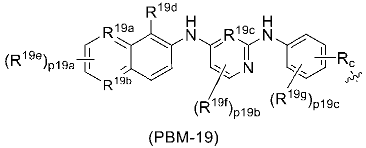
wherein R^{19a}, R^{19b}, and R^{19c} each independently represent CH or N;
R^{19d} represents -C(O)NHR^{19h}, -NHC(O)R^{19h}, -S(O)₂NHR^{19h}, -NHS(O)₂R^{19h}, -S(O)₂R^{19h} or - P(O)(R^{19h})₂, wherein each R^{19h} is identical or different and each independently represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.) or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
(R^{19e})ₚ₁₉ₐ represents p19a R^{19e} groups, wherein each R^{19e} is identical or different and each independently represents halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as trifluoromethoxy);
p19a represents an integer of 0, 1, 2, 3 or 4;
(R^{19f})_{p19b} represents p19b R^{19f} groups, wherein each R^{19f} is identical or different and each independently represents halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as trifluoromethoxy);
p19b represents an integer of 0, 1 or 2;
(R^{19g})_{p19c} indicates that the benzene ring to which it is attached is optionally substituted with p19c R^{19g} , with each R^{19g} independently representing halogen (e.g., fluorine, chlorine, bromine, or iodine), hydroxy, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.), C₁₋₆ alkoxy (e.g., C₁₋₅ alkoxy, C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as trifluoromethoxy); and
p19c represents an integer of 0, 1, 2, 3 or 4.

In some embodiments, R^{19a} and R^{19b} each independently represent CH or N. In some sub-embodiments, R^{19a} represents CH, and R^{19b} represents N. In some sub-embodiments, R^{19a} represents CH, and R^{19b} represents CH. In some sub-embodiments, R^{19a} represents N, and R^{19b} represents CH. In some sub-embodiments, R^{19a} represents N, and R^{19b} represents N.

In some embodiments, R^{19e} represents CH or N. In some sub-embodiments, R^{19e} represents N. In some sub-embodiments, R^{19e} represents CH.

In some embodiments, R^{19d} represents -C(O)NHR^{19h}, -NHC(O)R^{19h}, -S(O)₂NHR^{19h}, -NHS(O)₂R^{19h}, -S(O)₂R^{19h} or -P(O)(R^{19h})₂, wherein each R^{19h} is identical or different and each independently represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.) or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-). In some sub-embodiments, R^{19d} represents -P(O)(R^{19h})₂, wherein each R^{19h} is identical or different and each independently represents C₁₋₆ alkyl (e.g., C₁₋₄ alkyl, such as methyl, ethyl, propyl, isopropyl, butyl, sec-butyl, tert-butyl, pentyl, or hexyl), deuterated C₁₋₆ alkyl (e.g., perdeuterated C₁₋₅ alkyl, perdeuterated C₁₋₄ alkyl or perdeuterated C₁₋₃ alkyl, such as CD₃, CD₃CD₂-, CD₃CD₂CD₂- etc.) or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-). In some sub-embodiments, R^{19d} represents -P(O)(CH₃)₂.

In some embodiments, p19c represents an integer of 0, 1, 2, 3 or 4. In some sub-embodiments, p19c represents an integer of 2.

In some embodiments, PBM represents a structure of Formula (PBM-19-1), Formula (PBM-19-2), Formula (PBM-19-3), Formula (PBM-19-4), or Formula (PBM-19-5):

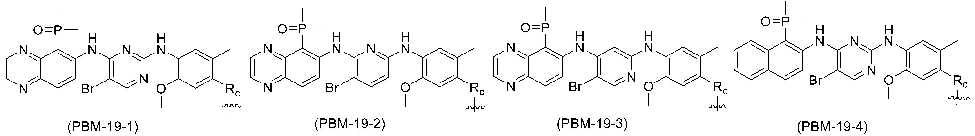

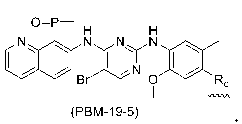

In some embodiments, PBM represents a small molecule ligand of AR.

In some embodiments, PBM represents the structure of Formula (PBM-4): 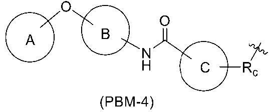
wherein ring A represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), and the ring A is optionally substituted with one or more (e.g., 1-10, 1-8 or 1-6, such as 1, 2, 3, 4, 5, 6, 7, 8, 9 or 10) R^{4a}, with each R^{4a} being identical or different and each independently representing deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₂₋₆ alkenyl (e.g., vinyl, propenyl, or butenyl), or C₂₋₆ alkynyl (e.g., ethynyl, propynyl or butynyl);
ring B represents C₃₋₁₅cycloalkyl or 4- to 20-membered heterocyclyl (e.g., 4- to 20-membered heterocyclyl containing 1 to 4 heteroatoms selected from N, O and S), wherein the ring B is optionally substituted with one or more (e.g., 1-10, 1-8 or 1-6, such as 1, 2, 3, 4, 5, 6, 7, 8, 9 or 10) R^{4b}, with each R^{4b} being identical or different and each independently representing deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₅ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
ring C represents 5- to 20-membered heteroaryl (e.g., 5- to 20-membered monocyclic or bicyclic heteroaryl containing 1 to 4 heteroatoms selected from N, O and S), wherein the ring C is optionally substituted with one or more (e.g., 1-10, 1-8 or 1-6, such as 1, 2, 3, 4, 5, 6, 7, 8, 9 or 10) R^{4c}, with each R^{4c} being identical or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents the structure of Formula (PBM-4-1A): 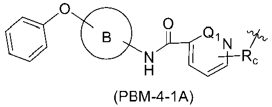
wherein the benzene ring in Formula (PBM-4-1A) is optionally substituted with one or more (e.g., 1-10, 1-8 or 1-6, such as 1, 2, 3, 4, 5, 6, 7, 8, 9 or 10) R^{4a}, and each R^{4a} is identical or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₂₋₆ alkenyl (e.g., vinyl, propenyl, or butenyl), or C₂₋₆ alkynyl (e.g., ethynyl, propynyl or butynyl);
ring B represents C₃₋₁₅ cycloalkyl or 4- to 20-membered heterocyclyl (e.g., 4- to 20-membered heterocyclyl containing 1 to 4 heteroatoms selected from N, O and S), wherein the ring B is optionally substituted with one or more (e.g., 1-10, 1-8 or 1-6, such as 1, 2, 3, 4, 5, 6, 7, 8, 9 or 10) R^{4b}, with each R^{4b} being identical or different and each independently representing deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₅ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
Q₁ represents N or CH; and
the heteroaryl group containing Q₁ and a nitrogen atom in Formula (PBM-4-1A) is optionally substituted with one or more (e.g., 1-3, such as 1, 2, or 3) R^{4c} , and each R^{4c} is identical or different and each independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₅ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, ring B in each of Formula (PBM-4) and Formula (PBM-4-1A) each independently represents C₃₋₁₅ cycloalkyl or 4- to 20-membered heterocyclyl (e.g., 4- to 20-membered heterocyclyl containing 1 to 4 heteroatoms selected from N, O and S). Examples of C₃₋₁₅ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅₋₁₅ spiro-cycloalkyl), adamantanyl, noradamantanyl, and norbornyl. The optional substituents of C₃₋₁₅ cycloalkyl are optionally selected from the group consisting of halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). Examples of 4- to 20-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). The optional substituents of 4- to 20-membered heterocyclyl are optionally selected from the group consisting of halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents a structure of Formula (PBM-4-1), Formula (PBM-4-2), Formula (PBM-4-3), or Formula (PBM-4-4):

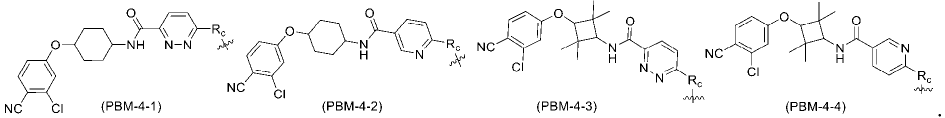

In some embodiments, PBM represents a small molecule ligand of BTK.

In some embodiments, PBM represents a structure of Formula (PBM-5): 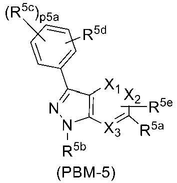
wherein X₁ in Formula (PBM-5) represents N or CR^{5f}, where R^{5f} represents hydrogen, deuterium, or amino, and X₂ represents N or CR^{5g}, where R^{5g} represents hydrogen or R_{c}, and X₃ represents CH or N;
R^{5a} and R^{5b} each independently represent hydrogen or R_{c};
(R^{5c})ₚ₅ₐ indicates that the benzene ring to which it is attached is substituted with p5a R^{5c}, wherein each R^{5c} is identical or different and each independently represents -O-aryl, -O-heteroaryl, 4- to 20-membered heterocyclyl, -C₁₋₃ alkylene-NHC(O)-heteroaryl, -C₁₋₃ alkylene-C(O)NH-heteroaryl, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterium, deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogen (e.g., fluorine, chlorine, bromine, or iodine), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), wherein the 4- to 20-membered heterocyclyl, aryl, and heteroaryl are each independently optionally substituted with one or more (e.g., 1-5, such as 1, 2, 3, 4 or 5) R^{5h}, wherein each R^{5h} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p5a represents an integer of 1, 2, 3 or 4;
R^{5d} represents R_{c}, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy);
R^{5e} represents hydrogen, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or amino;
wherein R^{5g}, R^{5a}, R^{5b}, and R^{5d} are not simultaneously hydrogen, and only one of R^{5g}, R^{5a}, R^{5b}, and R^{5a} represents R_{c}, wherein
   when R^{5a} represents R_{c}, X₂ represents CH or N, and R^{5b} is hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
   when X₂ represents CR^{5g}, where R^{5g} represents R_{c}, R^{5a} and R^{5b} are hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
   when R^{5b} represents R_{c}, X₂ represents N or CH, and R^{5a} is hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; or
   when R^{5d} represents R_{c}, X₂ represents N or CH, and R^{5a} and R^{5b} are hydrogen.

In some embodiments, R^{5c} in Formula (PBM-5) represents -O-aryl, -O-heteroaryl, -C₁₋₃ alkylene-NHC(O)-heteroaryl, or -C₁₋₃ alkylene-C(O)NH-heteroaryl, wherein the aryl may be optionally substituted C₆₋₁₀ aryl, including but not limited to e.g., phenyl or naphthyl, and the heteroaryl may be optionally substituted 5- to 14-membered monocyclic, bicyclic, or polycyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R^{5c} represents -O-phenyl or -O-naphthyl, wherein the phenyl and naphthyl are each optionally substituted with one or more (e.g., 1-5, such as 1, 2, 3, 4 or 5) R^{5h}, and each R^{5h} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R^{5c} represents -O-heteroaryl, -C₁₋₃ alkylene-NHC(O)-heteroaryl, or -C₁₋₃ alkylene-C(O)NH-heteroaryl, wherein the heteroaryl is optionally substituted with one or more (e.g., 1-5, such as 1, 2, 3, 4 or 5) R^{5h}, and each R^{5h} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, or imidazo[2,1-b]thiazolyl.

In some embodiments, R^{5c} in Formula (PBM-5) represents 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl, wherein the 4- to 20-membered monocyclic, bicyclic, or polycyclic heterocyclyl may be optionally substituted. In a sub-embodiment, R^{5c} represents 4- to 20-membered monocyclic, bicyclic, or polycyclic heterocyclyl, which is optionally substituted with one or more (e.g., 1-5, such as 1, 2, 3, 4, or 5) R^{5h} , and each R^{5h} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of 4- to 20-membered monocyclic, bicyclic, or polycyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl).

In some embodiments, R^{5b} represents R_{c}; R^{5a} is hydrogen; X₂ represents N or CH; and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy.

In some embodiments, R^{5b} represents R_{c}; R^{5a} is hydrogen; R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and X₂ represents N; X₁ represents CR^{5f}, wherein R^{5f} is hydrogen or amino; and X₃ represents N.

In some embodiments, R^{5b} represents R_{c}; R^{5a} is hydrogen; R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and X₂ represents N; X₁ represents CR^{5f}, wherein R^{5f} is hydrogen or amino; and X₃ represents CH.

In some embodiments, R^{5b} represents R_{c}; R^{5a} is hydrogen; R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and X₂ represents N; X₁ represents N; and X₃ represents CH.

In some embodiments, R^{5b} represents R_{c}; R^{5a} is hydrogen; R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and X₂ represents N; X₁ represents N; and X₃ represents N.

In some embodiments, R^{5b} represents R_{c}; R^{5a} is hydrogen; R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and X₂ represents CH; X₁ represents CR^{5f}, wherein R^{5f} is hydrogen or amino, and X₃ represents N.

In some embodiments, R^{5b} represents R_{c}; R^{5a} is hydrogen; R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and X₂ represents CH; X₁ represents CR^{5f}, wherein R^{5f} is hydrogen or amino; and X₃ represents CH.

In some embodiments, R^{5b} represents R_{c}; R^{5a} is hydrogen; R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and X₂ represents CH; X₁ represents N; and X₃ represents CH.

In some embodiments, R^{5b} represents R_{c}; R^{5a} is hydrogen; R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and X₂ represents CH; X₁ represents N; and X₃ represents N.

In some embodiments, PBM represents a structure of Formula (PBM-5-1A): 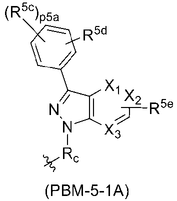
wherein X₁ in Formula (PBM-5-1A) represents N or CR^{5f}, where R^{5f} represents hydrogen or amino, and X₂ represents N or CH, and X₃ represents CH or N;
(R^{5c})ₚ₅ₐ in Formula (PBM-5-1A) indicates that the benzene ring to which it is attached is substituted with p5a R^{5c}, wherein each R^{5c} is identical or different and each independently represents -O-aryl, -O-heteroaryl, 4- to 20-membered heterocyclyl, -C₁₋₃ alkylene-NHC(O)-heteroaryl, or -C₁₋₃ alkylene-C(O)NH-heteroaryl, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterium, deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogen (e.g., fluorine, chlorine, bromine, or iodine), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), wherein the 4- to 20-membered heterocyclyl, aryl, and heteroaryl are each independently optionally substituted with one or more (e.g., 1-5, such as 1, 2, 3, 4 or 5) R^{5h}, wherein each R^{5h} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p5a represents an integer of 1, 2, 3 or 4;
R^{5d} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), or C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy); e.g., R^{5d} represents hydrogen or methyl; and
R^{5e} represents hydrogen, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or amino.

In some embodiments, R^{5c} in Formula (PBM-5-1A) represents -O-aryl, -O-heteroaryl, -C₁₋₃alkylene-NHC(O)-heteroaryl, or -C₁₋₃alkylene-C(O)NH-heteroaryl, wherein the aryl may be optionally substituted C₆₋₁₀ aryl, including but not limited to e.g., phenyl or naphthyl, and the heteroaryl may be optionally substituted 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, R^{5c} represents -O-phenyl or -O-naphthyl, wherein the phenyl and naphthyl are each optionally substituted with one or more (e.g., 1-5, such as 1, 2, 3, 4 or 5) R^{5h}, and each R^{5h} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, R^{5c} represents -O-heteroaryl, -C₁₋₃ alkylene-NHC(O)-heteroaryl, or -C₁₋₃ alkylene-C(O)NH-heteroaryl, wherein the heteroaryl is optionally substituted with one or more (e.g., 1-5, such as 1, 2, 3, 4 or 5) R^{5h}, and each R^{5h} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, and imidazo[2,1-b]thiazolyl.

In some embodiments, R^{5c} in Formula (PBM-5-1A) represents 4- to 20-membered (e.g., 4- to 15-membered, 4- to 12-membered, 4- to 10-membered, 4- to 7-membered, or 4- to 6-membered) monocyclic, bicyclic, or polycyclic heterocyclyl, wherein the 4- to 20-membered monocyclic, bicyclic, or polycyclic heterocyclyl may be optionally substituted . In a sub-embodiment, R^{5c} represents 4- to 20-membered monocyclic, bicyclic, or polycyclic heterocyclyl, which is optionally substituted with one or more (e.g., 1-5, such as 1, 2, 3, 4 or 5) R^{5h}, and each R^{5h} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In a sub-embodiment, examples of 4- to 20-membered monocyclic, bicyclic, or polycyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl).

In some embodiments, X₁ in Formula (PBM-5-1A) represents N, X₂ represents N, and X₃ represents CH.

In some embodiments, X₁ in Formula (PBM-5-1A) represents N, X₂ represents N, and X₃ represents N.

In some embodiments, X₁ in Formula (PBM-5-1A) represents N, X₂ represents CH, and X₃ represents CH.

In some embodiments, X₁ in Formula (PBM-5-1A) represents N, X₂ represents CH, and X₃ represents N.

In some embodiments, X₁ in Formula (PBM-5-1A) represents CR^{5f}, wherein R^{5f} is hydrogen or amino, X₂ represents N, and X₃ represents N.

In some embodiments, X₁ in Formula (PBM-5-1A) represents CR^{5f}, wherein R^{5f} is hydrogen or amino, X₂ represents N, and X₃ represents CH.

In some embodiments, X₁ in Formula (PBM-5-1A) represents CR^{5f}, wherein R^{5f} is hydrogen or amino, X₂ represents CH, and X₃ represents N.

In some embodiments, X₁ in Formula (PBM-5-1A) represents CR^{5f}, wherein R^{5f} is hydrogen or amino, X₂ represents CH, and X₃ represents CH.

In some embodiments, PBM represents the structure of Formula (PBM-5-1):

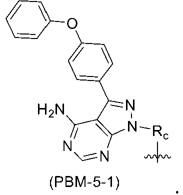

In some embodiments, PBM represents a small molecule ligand of CDK4/6.

In some embodiments, PBM represents a structure of Formula (PBM-6-1A), Formula (PBM-6-1B), Formula (PBM-6-1C), or Formula (PBM-6-1D): 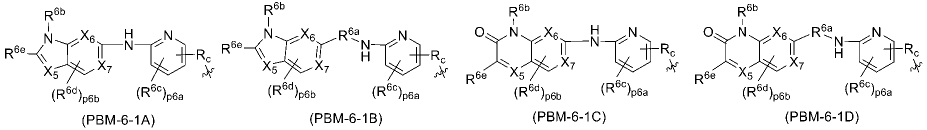
wherein each R^{6e} independently represents deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), -C(O)-C₁₋₆ alkyl (e.g., -C(O)-C₁₋₅ alkyl or -C(O)-C₁₋₃ alkyl, such as -C(O)-CH₃, -C(O)-CH₂CH₃), -C(O)-deuterated C₁₋₆ alkyl (e.g., -C(O)-deuterated C₁₋₅ alkyl or -C(O)-deuterated C₁₋₃ alkyl, such as -C(O)-CD₃) or - C(O)NR^{6f}R^{6g}, where R^{6f} and R^{6g} are identical or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{6f} and R^{6g} are not simultaneously hydrogen;
each X₅ independently represents N or CR^{6h}, where R^{6h} represents hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
X₆ and X₇ each independently represent CH or N;
R^{6a} in each of Formula (PBM-6-1B) and Formula (PBM-6-1D) each independently represents optionally substituted heteroarylene (e.g., optionally substituted 5- to 20-membered monocyclic, bicyclic, or polycyclic aromatic ring group containing one or more (e.g., from 1 to 6, or from 1 to 5, or from 1 to 4, or from 1 to 3) heteroatoms independently selected from the group consisting of oxygen, nitrogen, and sulfur), wherein the heteroarylene is optionally substituted with 1-2 substituents each independently selected from the group consisting of halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof;
each R^{6b} independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or optionally substituted C₃₋₁₂cycloalkyl (e.g., cyclopropyl, cyclobutyl, cyclopentyl, cyclopentenyl, cyclohexyl, cyclohexenyl, or cycloheptyl, which are optionally substituted with a substituent selected from the group consisting of e.g., halogen, C₁₋₆ alkyl, halogenated C₁₋₆ alkyl, and deuterated C₁₋₆ alkyl);
each (R^{6c})ₚ₆ₐ independently indicates that the pyridine ring to which it is attached is optionally substituted with p6a R^{6c}, wherein each R^{6c} is identical or different and each independently represents deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p6a represents an integer of 0, **1, 2** or 3; and
each (R^{6d})_{p6b} independently indicates that the nitrogen-containing bicyclic heteroaryl ring to which it is attached is optionally substituted with p6b R^{6d}, wherein each R^{6d} is identical or different and each independently represents deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p6b represents an integer of 0 or 1.

In some embodiments, R^{6a} in each of Formula (PBM-6-1B) and Formula (PBM-6-1D) independently represents optionally substituted heteroarylene, such as optionally substituted 5- to 14-membered monocyclic, bicyclic, or polycyclic heteroarylene group containing one or more (e.g., from 1 to 6, or from 1 to 5, or from 1 to 4, or from 1 to 3) heteroatoms independently selected from the group consisting of oxygen, nitrogen, and sulfur. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-furo[3,2-b]pyrrolylene, pyrrolo[2,1-b]thiazolylene, and imidazo[2,1-b]thiazolylene. Heteroarylene is optionally substituted with one or more (e.g., 1-4, such as 1, 2, 3, or 4) substituents selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents the structure of Formula (PBM-6-1), Formula (PBM-6-2), or Formula (PBM-6-3):

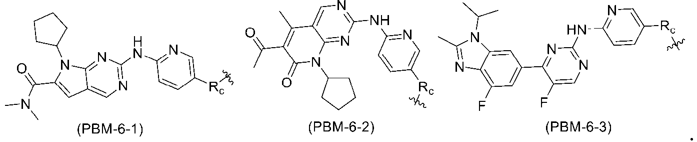

In some embodiments, PBM represents a small molecule ligand of ALK.

In some embodiments, PBM represents the structure of Formula (PBM-7): 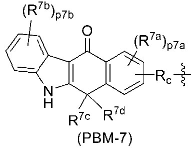
wherein (R^{7a})ₚ₇ₐ in Formula (PBM-7) indicates that the benzene ring to which it is attached is optionally substituted with p7a R^{7a}, with each R^{7a} independently representing deuterium, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and p7a represents an integer of 0, 1, 2 or 3; and
(R^{7b})_{p7b} indicates that the benzene ring to which it is attached is optionally substituted with p7b R^{7b}, with each R^{7b} independently representing deuterium, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and p7b represents an integer of 0, 1, 2, 3 or 4; and
R^{7c} and R^{7d} are identical or different and each independently represent deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogen (e.g., fluorine, chlorine, bromine, or iodine), halogenated C₁₋₆ alkyl (e.g., fluorine, chlorine, bromine, or iodine), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents the structure of Formula (PBM-7-1):

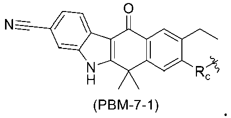

In some embodiments, PBM represents the structure of Formula (PBM-8): 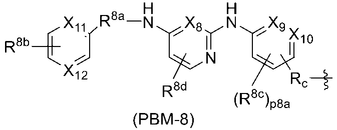
wherein X₈, X₉, X₁₀, X₁₁, and X₁₂ in Formula (PBM-8) are identical or different and each independently represent N or CH;
R^{8a} represents a bond or optionally substituted methylene (e.g., halogenated methylene);
R^{8b} represents -C(O)N(R^{8f})R^{8e}, -N(R^{8f})C(O)R^{8e}, -S(O)₂N(R^{8f})R^{8e}, -N(R^{8f})S(O)₂R^{8e}, -S(O)₂R^{8e} or - P(O)(R^{8e})₂, wherein each R^{8e} is identical or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), R^{8f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{8c})ₚ₈ₐ in Formula (PBM-8) indicates that the 6-membered ring containing X₉ and X₁₀ to which it is attached is optionally substituted with p8a R^{8c}, with each R^{8c} being identical or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p8a represents an integer of 0, 1, 2, 3 or 4; and
R^{8d} represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{8a} in Formula (PBM-8) represents a bond. In other words, the 6-membered ring containing X₁₁ and X₁₂ in Formula (PBM-8) is directly bonded to the -NH- group.

In some embodiments, R^{8a} in Formula (PBM-8) represents optionally substituted methylene (e.g., halogenated methylene).

In some embodiments, PBM represents the structure of Formula (PBM-8-1A): 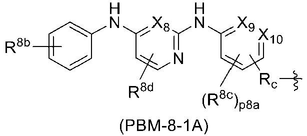
wherein X₈, X₉, and X₁₀ are identical or different and each independently represents N or CH;
R^{8b} represents -C(O)N(R^{8f})R^{8e}, -N(R^{8f})C(O)R^{8e}, -S(O)₂N(R^{8f})R^{8e}, -N(R^{8f})S(O)₂R^{8e}, -S(O)₂R^{8e} or - P(O)(R^{8e})₂, wherein each R^{8e} is identical or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{8f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{8c})ₚ₈ₐ indicates that the 6-membered ring containing X₉ and X₁₀ to which it is attached is optionally substituted with p8a R^{8c}, with each R^{8c} being identical or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p8a represents an integer of 0, 1, 2, 3 or 4; and
R^{8d} represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of Formula (PBM-8-1B): 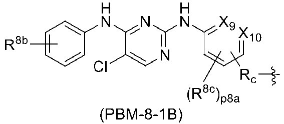
wherein X₉, and X₁₀ are identical or different and each independently represents N or CH;
R^{8b} represents -C(O)N(R^{8f})R^{8e}, -N(R^{8f})C(O)R^{8e}, -S(O)₂N(R^{8f})R^{8e}, -N(R^{8f})S(O)₂R^{8e}, -S(O)₂R^{8e} or - P(O)(R^{8e})₂, wherein each R^{8e} is identical or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{8f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{8c})ₚ₈ₐ indicates that the 6-membered ring containing X₉ and X₁₀ to which it is attached is optionally substituted with p8a R^{8c}, with each R^{8c} being identical or different and each independently representing deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p8a represents an integer of 0, 1, 2, 3 or 4.

In some embodiments, PBM represents the structure of Formula (PBM-8-1) or Formula (PBM-8-2):

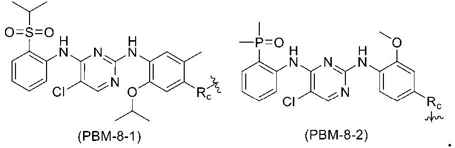

In some embodiments, PBM represents a small molecule ligand of BCR-ABL.

In some embodiments, PBM represents the structure of Formula (PBM-9): 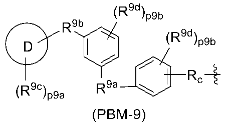
wherein R^{9a} represents -NHC(O)- or -C(O)NH-;
R^{9b} represents -NH-, -N(C₁₋₆ alkyl)- or ethynylene;
ring D represents a 5- to 20-membered monocyclic, bicyclic, or polycyclic heteroaryl group containing 1 to 4 heteroatoms selected from N, O, and S, and (R^{9c})ₚ₉ₐ indicates that the ring D is optionally substituted with p9a R^{9c}, with each R^{9c} being identical or different and each independently representing optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p9a represents an integer of 0, 1, 2, or 3;
each (R^{9d})_{p9b} in Formula (PBM-9) independently indicates that the benzene ring to which it is attached is independently optionally substituted with p9b R^{9d}, with each R^{9d} being identical or different and each independently representing deuterium, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each p9b is identical or different and each independently represents an integer of 0, 1, 2, 3 or 4.

In some embodiments, ring D in Formula (PBM-9) represents a 5- to 20-membered monocyclic, bicyclic, or polycyclic heteroaryl group containing 1 to 4 heteroatoms selected from N, O, and S. In a sub-embodiment, examples of 5- to 20-membered monocyclic, bicyclic, or polycyclic heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl group is optionally substituted with p9a R^{9c} , wherein p9a represents an integer of 0, 1, 2, or 3, and each R^{9c} is identical or different and each independently represents optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R^{9c} in Formula (PBM-9) represents optionally substituted 5- to 15-membered heteroaryl. In some embodiments, the 5- to 15-membered heteroaryl is 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, or imidazo[1,2-b]pyridazinyl. The heteroaryl group is optionally substituted with one or more (e.g., 1-6, such as 1, 2, 3, 4, 5, or 6) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, R^{9b} in Formula (PBM-9) represents -NH-.

In some embodiments, R^{9b} in Formula (PBM-9) represents ethynylene.

In some embodiments, PBM represents the structure of Formula (PBM-9-1A): 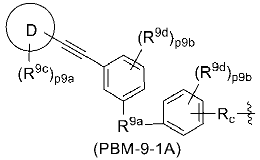
wherein R^{9a} represents -NHC(O)- or -C(O)NH-;
ring D represents a 5- to 20-membered monocyclic, bicyclic, or polycyclic heteroaryl group containing 1 to 4 heteroatoms selected from N, O, and S, and (R^{9c})ₚ₉ₐ indicates that the ring D is optionally substituted with p9a R^{9c}, with each R^{9c} being identical or different and each independently representing optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p9a represents an integer of 0, 1, 2, or 3;
each (R^{9d})_{p9b} in Formula (PBM-9-1A) independently indicates that the benzene ring to which it is attached is independently optionally substituted with p9b R^{9d}, with each R^{9d} being identical or different and each independently representing deuterium, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
each p9b is identical or different and each independently represents an integer of 0, 1, 2, 3 or 4.

In some embodiments, ring D in Formula (PBM-9-1A) represents a 5- to 20-membered monocyclic, bicyclic, or polycyclic heteroaryl group containing 1 to 4 heteroatoms selected from N, O, and S. In a sub-embodiment, examples of 5- to 20-membered monocyclic, bicyclic, or polycyclic heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl group is optionally substituted with p9a R^{9c} , wherein p9a represents an integer of 0, 1, 2, or 3, and each R^{9c} is identical or different and each independently represents: optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R^{9c} in Formula (PBM-9-1A) represents optionally substituted 5- to 15-membered heteroaryl. In some embodiments, the 5- to 15-membered heteroaryl is 5- to 14-membered monocyclic, bicyclic, or polycyclic aromatic ring group containing one or more (e.g., from 1 to 6, or from 1 to 5, or from 1 to 4, or from 1 to 3) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl group is optionally substituted with one or more (e.g., 1-6, such as 1, 2, 3, 4, 5, or 6) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, PBM represents the structure of Formula (PBM-9-1):

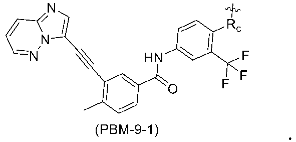

In some embodiments, PBM represents the structure of Formula (PBM-21): 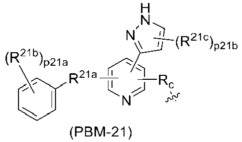
wherein R^{21a} in Formula (PBM-21) represents NHC(O), C(O)NH, NHS(O)₂ or S(O)₂NH;
(R^{21b})ₚ₂₁ₐ indicates that the benzene ring to which it is attached is optionally substituted with p21a R^{21b} , wherein each R^{21b} is independently halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy) or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p21a represents an integer of 0, 1, 2, 3, 4 or 5; and
(R^{21c})_{p21b} indicates that the 1H-pyrazole ring to which it is attached is optionally substituted with p21b R^{21c} , wherein each R^{21c} is independently halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy) or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p21b represents an integer of 0, 1 or 2.

In some embodiments, p21a in Formula (PBM-21) represents an integer of 1, and R^{21b} is halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy) or halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-). In some embodiments, R^{21b} is F₂ClC-O-.

In some embodiments, R^{21a} represents NHC(O). In some embodiments, R^{21a} represents C(O)NH.

In some embodiments, p21b represents an integer of 0.

In some embodiments, PBM represents the structure of Formula (PBM-21-1):

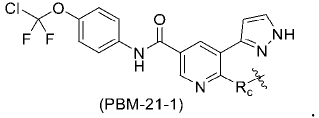

In some embodiments, PBM represents a small molecule ligand of FAK.

In some embodiments, PBM represents the structure of Formula (PBM-8-1C): 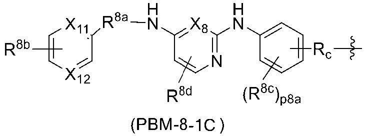
wherein X₈, X₁₁, and X₁₂ in Formula (PBM-8-1C) are identical or different and each independently represent N or CH;
R^{8a} represents a bond or optionally substituted methylene (e.g., halogenated methylene);
R^{8b} represents -C(O)N(R^{8f})R^{8e}, -N(R^{8f})C(O)R^{8e}, -S(O)₂N(R^{8f})R^{8e}, -N(R^{8f})S(O)₂R^{8e}, -S(O)₂R^{8e} or - P(O)(R^{8e})₂, wherein each R^{8e} is identical or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{8f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{8c})ₚ₈ₐ in Formula (PBM-8-1C) indicates that the benzene ring to which it is attached is optionally substituted with p8a R^{8c}, wherein each R^{8c} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p8a represents an integer of 0, 1, 2, 3 or 4; and
R^{8d} represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{8a} in Formula (PBM-8-1C) represents a bond. In other words, the 6-membered ring containing X₁₁ and X₁₂ in Formula (PBM-8-1C) is directly bonded to the -NH- group.

In some embodiments, R^{8a} in Formula (PBM-8-1C) represents optionally substituted methylene (e.g., halogenated methylene).

In some embodiments, PBM represents the structure of Formula (PBM-8-1D) or Formula (PBM-8-1E): 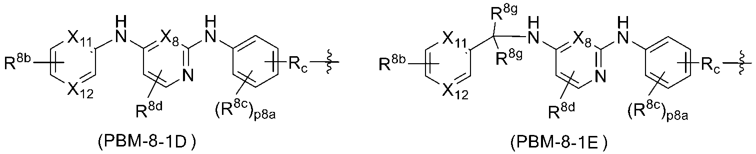
wherein X₈, X₁₁, and X₁₂ are identical or different and each independently represent N or CH;
each R^{8g} is identical or different and each independently represents hydrogen or halogen;
R^{8b} represents -C(O)N(R^{8f})R^{8e}, -N(R^{8f})C(O)R^{8e}, -S(O)₂N(R^{8f})R^{8e}, -N(R^{8f})S(O)₂R^{8e}, -S(O)₂R^{8e} or - P(O)(R^{8e})₂, wherein each R^{8e} is identical or different and each independently represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), and R^{8f} represents hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl);
(R^{8c})ₚ₈ₐ indicates that the benzene ring to which it is attached is optionally substituted with p8a R^{8c}, wherein each R^{8c} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p8a represents an integer of 0, 1, 2, 3 or 4; and
R^{8d} represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents the structure of Formula (PBM-8-3), Formula (PBM-8-4), Formula (PBM-8-5), Formula (PBM-8-6), Formula (PBM-8-7), or Formula (PBM-8-8):

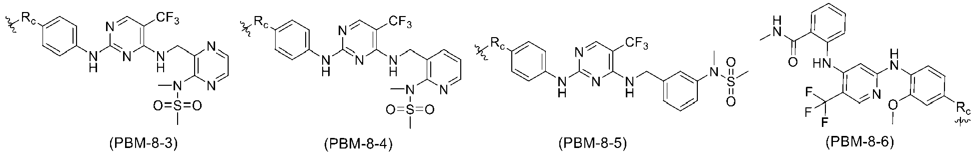

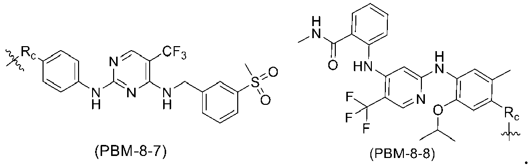

In some embodiments, PBM represents the structure of Formula (PBM-22): 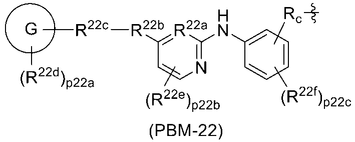
wherein R^{22a} represents N or CH;
R^{22b} represents O, S, NH, CH₂, CH(F) or C(F)₂;
R^{22c} represents a bond or optionally substituted methylene (e.g., halogenated methylene);
ring G represents C₆₋₁₅ aryl, 4- to 15-membered heterocyclyl, or 5- to 15-membered heteroaryl, and (R^{22d})ₚ₂₂ₐ indicates that the ring G to which it is attached is optionally substituted with p22a R^{22d} , wherein each R^{22d} is identical or different and each independently represents deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p22a represents an integer of 0, 1, 2, 3 or 4;
(R^{22e})_{p22b} indicates that the 6-membered nitrogen-containing heteroaromatic ring containing R^{22a} to which it is attached is optionally substituted with p22b R^{22e} , and each R^{22e} represents deuterium, halogen (e.g., fluorine, chlorine, bromine, or iodine), hydroxy, cyano, amino, nitro, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CHO-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p22b represents an integer of 0, 1 or 2;
(R^{22f})_{p22c} indicates that the benzene ring to which it is attached is optionally substituted with p22c R^{22f} , wherein each R^{22f} is identical or different and each independently represents deuterium, halogen (e.g., fluorine, chlorine, bromine, or iodine), hydroxy, cyano, amino, nitro, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, F₂ClC-O-, ClCH₂-O-, Cl₂CHO-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p22c represents an integer of 0, 1, 2, 3 or 4.

In some embodiments, R^{22a} represents N.

In some embodiments, R^{22a} represents CH.

In some embodiments, R^{22c} represents a bond.

In some embodiments, R^{22c} represents optionally substituted methylene (e.g., methylene optionally substituted with halogen (e.g., fluorine, chlorine, bromine, or iodine)).

In some embodiments, ring G represents C₆₋₁₅ aryl, 4- to 15-membered heterocyclyl, or 5- to 15-membered heteroaryl. Examples of C₆₋₁₅ aryl include, but are not limited to, phenyl and naphthyl. Examples of 4- to 15-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). Examples of 5- to 15-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-furo[3,2-b]pyrrolyl, pyrrolo[2,1-b]thiazolyl, and imidazo[2,1-b]thiazolyl.

In some embodiments, (R^{22d})ₚ₂₂ₐ in Formula (PBM-22) indicates that the ring G to which it is attached is optionally substituted with p22a R^{22d} , wherein each R^{22d} is identical or different and each independently represents deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p22a represents an integer of 0, 1, 2, 3 or 4.

In some embodiments, ring G represents isoindolinyl, which is optionally substituted with p22a R^{22d} , and each R^{22d} is identical or different and each independently represents deuterium, halogen (e.g., fluorine, chlorine, bromine, or iodine), hydroxy, cyano, amino, nitro, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p22a represents an integer of 0, 1, 2, 3 or 4. In some embodiments, p22a represents an integer of 0, 1, 2, 3 or 4. In some embodiments, ring G represents isoindolinyl, which is optionally substituted with halogen (e.g., fluorine, chlorine, bromine, or iodine), oxo, and C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

In some embodiments, (R^{22e})_{p22b} indicates that the 6-membered nitrogen-containing heteroaromatic ring containing R^{22a} to which it is attached is optionally substituted with p22b R^{22e} , and each R^{22e} represents deuterium, halogen (e.g., fluorine, chlorine, bromine, or iodine), hydroxy, cyano, amino, nitro, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p22b represents an integer of 0, 1 or 2. In some embodiments, each R^{22e} independently represents deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p22b represents an integer of 0, 1 or 2.

In some embodiments, (R^{22f})_{p22c} indicates that the benzene ring to which it is attached is optionally substituted with p22c R^{22f} , wherein each R^{22f} is identical or different and each independently represents deuterium, halogen (e.g., fluorine, chlorine, bromine, or iodine), hydroxy, cyano, amino, nitro, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p22c represents an integer of 0, 1, 2, 3 or 4. In some embodiments, each R^{22f} independently represents halogen (e.g., fluorine, chlorine, bromine, or iodine) or C₁₋₆ alkoxy (e.g., C₁₋₄ alkoxy, such as methoxy, ethoxy, propoxy, isopropoxy, n-butoxy, sec-butoxy, or tert-butoxy), and p22c represents an integer of 0, 1, 2, 3 or 4.

In some embodiments, PBM represents the structure of Formula (PBM-22-1A), Formula (PBM-22-1B), Formula (PBM-22-1C), Formula (PBM-22-2A), Formula (PBM-22-2B), or Formula (PBM-22-2C): 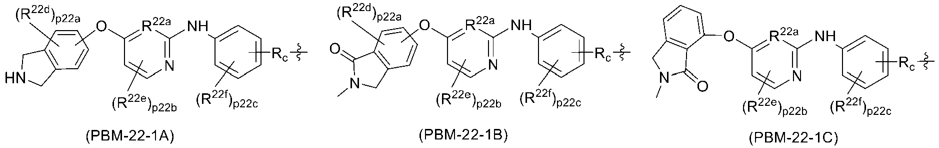 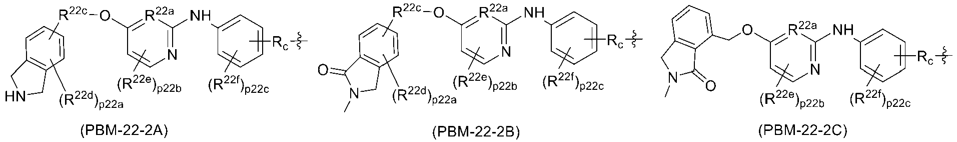 wherein R^{22a}, R^{22c}, (R^{22d})ₚ₂₂ₐ, (R^{22e})_{p22b}, and (R^{22f})_{p22c} are as defined in various embodiments of Formula (PBM-22) and each sub-embodiments thereof.

In some embodiments, PBM represents a structure of Formula (PBM-22-1), Formula (PBM-22-2), Formula (PBM-22-3), or Formula (PBM-22-4):

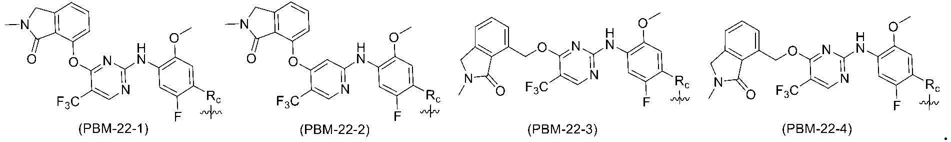

In some embodiments, PBM represents a small molecule ligand of ER.

In some embodiments, PBM represents a structure of Formula (PBM-10-1A), or Formula (PBM-10-1B) (which are non-limiting embodiments of the structure of Formula (PBM-10)): 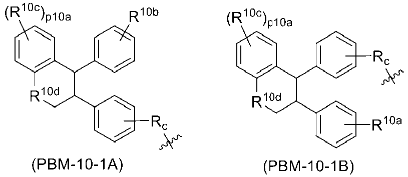
wherein each R^{10d} independently represents O, S or CH₂;
(R^{10c})ₚ₁₀ₐ indicates that the benzo-fused six-membered ring containing R^{10d} is optionally substituted with p10a R^{10c}, with each R^{10c} being identical or different and each independently representing hydrogen, hydroxy, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p10a represents an integer of 0, 1, 2, 3 or 4 ; and
R^{10a} and R^{10b} each independently represent hydrogen, halogen (e.g., fluorine, chlorine, bromine, or iodine), hydroxy, cyano, amino, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, PBM represents a structure of Formula (PBM-10-1), Formula (PBM-10-2), Formula (PBM-10-3), Formula (PBM-10-4), Formula (PBM-10-5), Formula (PBM-10-6), Formula (PBM-10-7) , Formula (PBM-10-8), Formula (PBM-10-9), Formula (PBM-10-10), Formula (PBM-10-11), or Formula (PBM-10-12):

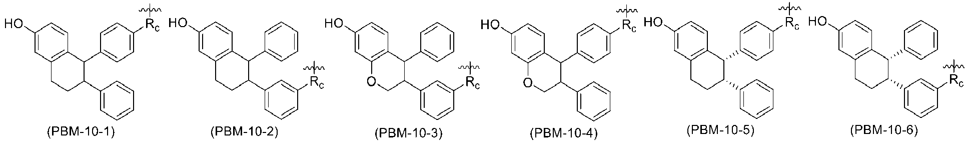

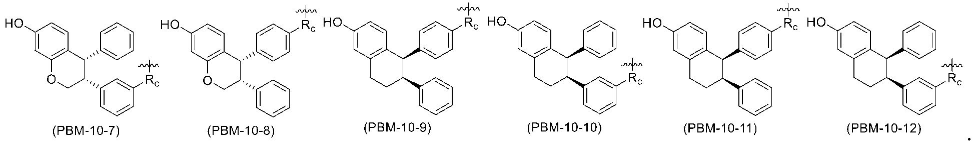

In some embodiments, PBM represents a small molecule ligand of IRAK4.

In some embodiments, PBM represents a structure of Formula (PBM-11): 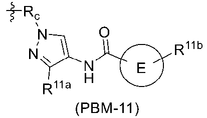
wherein ring E in Formula (PBM-11) represents a 5- to 20-membered monocyclic, bicyclic, or polycyclic heteroarylene group containing 1 to 4 heteroatoms selected from N, O, and S;
R^{11b} represents optionally substituted 5- to 15-membered heteroaryl, optionally substituted 5- to 15-membered heterocyclyl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
R^{11a} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, ring E in Formula (PBM-11) represents a 5- to 20-membered monocyclic, bicyclic, or polycyclic heteroarylene group containing 1 to 4 heteroatoms selected from N, O, and S. In some embodiments, the 5- to 20-membered monocyclic, bicyclic, or polycyclic heteroarylene is 5- to 14-membered divalent monocyclic, bicyclic, or polycyclic aromatic ring group containing from 1 to 4 (e.g., from 1 to 4, or from 1 to 3, or from 1 to 2, or 1) heteroatoms selected from the group consisting of oxygen, nitrogen, and sulfur. In some sub-embodiments, examples of 5- to 20-membered monocyclic, bicyclic, or polycyclic heteroarylene include, but are not limited to, furanylene, oxazolylene, isoxazolylene, oxadiazolylene, thienylene, thiazolylene, isothiazolylene, thiadiazolylene,pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, and imidazo[1,2-b]pyridazinylene. The 5- to 20-membered monocyclic, bicyclic, or polycyclic heteroarylene is optionally substituted with one or more (e.g., 1-3, such as 1, 2, or 3) R^{11b} substituents, wherein each R^{11b} independently represents optionally substituted 5- to 15-membered heteroaryl, optionally substituted 5- to 15-membered heterocyclyl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, R^{11b} in Formula (PBM-11) represents optionally substituted 5- to 15-membered heteroaryl. In some embodiments, the 5- to 15-membered heteroaryl is 5- to 15-membered monocyclic, bicyclic, or polycyclic aromatic ring group containing from 1 to 4 (e.g., from 1 to 4, or from 1 to 3, or from 1 to 2, or 1) heteroatoms selected from the group consisting of oxygen, nitrogen, and sulfur. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. Heteroaryl represented by R^{11b} is optionally substituted with one or more (e.g., 1-3, such as 1, 2, or 3) substituents which are optionally selected from the group consisting of: -N(R^{11c})-C₁₋₆ alkylene-optionally substituted C₃₋₈ cycloalkyl, - N(R^{11c})-optionally halogenated C₁₋₆ alkyl, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof, wherein each R^{11c} independently represents hydrogen or C₁₋₃ alkyl. In some embodiments, the substituent of heteroaryl represented by R^{11b} is -N(R^{11c})-C₁₋₆ alkylene-optionally substituted C₃₋₈ cycloalkyl, wherein R^{11c} represents hydrogen or C₁₋₃ alkyl (e.g., methyl). In some embodiments, examples of C₁₋₆ alkylene include, but are not limited to, C₁₋₃ alkylene. In some embodiments, examples of C₃₋₈ cycloalkyl include, but are not limited to, C₃₋₆ cycloalkyl. In some embodiments, representative examples of C₃₋₈ cycloalkyl include, but are not limited to, cyclopropyl, cyclobutyl, cyclopentyl,, cyclopentenyl, cyclohexyl, cyclohexenyl, cycloheptyl, and cyclooctyl. C₃₋₈ cycloalkyl is optionally substituted with one or more (e.g., 1-3, such as 1, 2, or 3) substituents selected from the group consisting of C₁-C₃ alkyl, C₃₋₆cycloalkyl, hydroxy, amino, mercapto, halogen, C₁-C₃ alkoxy, C₁-C₃ alkylamino, halogenated C₁-C₃ alkyl, amino-substituted C₁₋₃ alkylene, cyano, and any combination thereof. In some embodiments, the substituent of heteroaryl represented by R^{11b} is - N(R^{11c})-optionally halogenated C₁₋₆ alkyl, wherein R^{11c} represents hydrogen or C₁₋₃ alkyl (e.g., methyl). In some embodiments, examples of optionally halogenated C₁₋₆ alkyl include, but are not limited to, optionally halogenated C₁₋₄ alkyl, optionally halogenated C₁₋₃ alkyl, and optionally halogenated C₁₋₂ alkyl. In some embodiments, representative examples of optionally halogenated C₁₋₆ alkyl include, but are not limited to, - CH₂-CF₃.

In some embodiments, R^{11b} in Formula (PBM-11) represents optionally substituted 5- to 15-membered heterocyclyl. In some sub-embodiments, "5- to 15-membered heterocyclyl"refers to a 5- to 15-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some sub-embodiments, examples of heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. The heterocyclyl is optionally substituted with one or more (e.g., 1 to 5, 1 to 4, 1 to 3, 1 to 2, or 1) substituents selected from the group consisting of deuterium, hydroxy, amino, mercapto, nitro, halogen (e.g., fluorine, chlorine, bromine, or iodine), cyano, C₁₋₆ alkyl, halogenated C₁₋₆ alkyl, deuterated C₁₋₆ alkyl, C₃₋₆cycloalkyl, C₁₋₆ alkoxy, halogenated C₁₋₆ alkoxy, and any combination thereof.

In some embodiments, PBM represents the structure of Formula (PBM-11-1), Formula (PBM-11-2), Formula (PBM-11-3), or Formula (PBM-11-4):

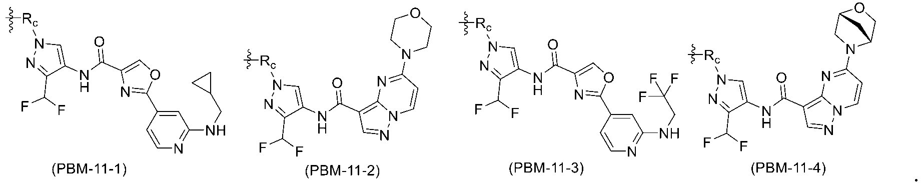

In some embodiments, PBM represents the structure of Formula (PBM-12): 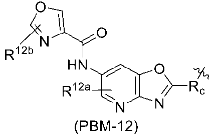
wherein R^{12a} in Formula (PBM-12) represents optionally substituted 5- to 15-membered heterocyclyl, optionally substituted 5- to 15-membered heteroaryl, deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
R^{12b} represents a 5- to 20-membered monocyclic, bicyclic, or polycyclic heteroaryl group containing 1 to 4 heteroatoms selected from N, O, and S, and the heteroaryl is optionally substituted with one or more (e.g., 1-6, such as 1, 2, 3, 4, 5 or 6) substituents selected from the group consisting of deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, R^{12a} in Formula (PBM-12) represents optionally substituted 5- to 15-membered heterocyclyl. In some sub-embodiments, "5- to 15-membered heterocyclyl"refers to a 5- to 15-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some sub-embodiments, examples of heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. The heterocyclyl is optionally substituted with one or more (e.g., 1 to 5, 1 to 4, 1 to 3, 1 to 2, or 1) substituents selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₃₋₆cycloalkyl, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, R^{12b} in Formula (PBM-12) represents an optionally substituted 5- to 20-membered (e.g., 5- to 15-membered, 5- to 10-membered or 5- to 6-membered) monocyclic, bicyclic, or polycyclic heteroaryl group containing 1 to 4 heteroatoms selected from N, O, and S. In some sub-embodiments, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, and imidazo[1,2-b]pyridazinyl. The heteroaryl is optionally substituted with one or more (e.g., 1-3, such as 1, 2, or 3) substituents optionally selected from the group consisting of: halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, PBM represents the structure of Formula (PBM-12-1), Formula (PBM-12-2), or Formula (PBM-12-3):

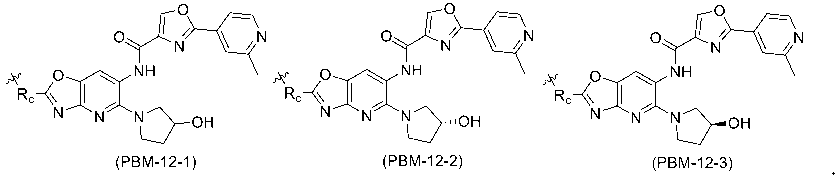

In some embodiments, PBM represents the structure of Formula (PBM-20): 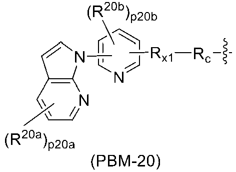
wherein Rₓ₁ in Formula (PBM-20) represents a bond or C(O), or Rₓ₁ represents -C(O)NH-Rₓ₂-C(O)-***, wherein symbol *** indicates the point of attachment to R_{c}, and Rₓ₂ represents optionally substituted C₃₋₁₅ cycloalkyl;
(R^{20a})ₚ₂₀ₐ indicates that the 1H-pyrrolo[2,3-b]pyridine ring to which it is attached is optionally substituted with p20a R^{20a}, wherein each R^{20a} is independently cyano, halogen (e.g., fluorine, chlorine, bromine, or iodine), hydroxy, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p20a represents an integer of 0, 1, 2, 3, 4 or 5;
(R^{20b})_{p20b} indicates that the pyridine ring to which it is attached is optionally substituted with p20b R^{20b}, wherein each R^{20b} is independently cyano, halogen (e.g., fluorine, chlorine, bromine, or iodine), hydroxy, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or NR^{20c}R^{20d}, where R^{20c} and R^{20d} are identical or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₁₅cycloalkyl or optionally substituted 4- to 15-membered heterocyclyl, and p20b represents an integer of 0, 1, 2 or 3.

In some embodiments, Rₓ₁ in Formula (PBM-20) represents a bond.

In some embodiments, Rₓ₁ in Formula (PBM-20) represents C(O).

In some embodiments, Rₓ₁ in Formula (PBM-20) represents -C(O)NH-Rₓ₂-C(O)-***, wherein symbol *** indicates the point of attachment to R_{c}, and Rₓ₂ represents optionally substituted C₃₋₁₅cycloalkyl. In some sub-embodiments, examples of the optionally substituted C₃₋₁₅ cycloalkyl include, but are not limited to, optionally substituted C₃₋₁₁ cycloalkyl, and optionally substituted C₃₋₈ cycloalkyl, such as optionally substituted cyclopropyl, optionally substituted cyclobutyl, optionally substituted cyclopentyl,, optionally substituted cyclohexyl, optionally substituted cycloheptyl, or optionally substituted cyclooctyl. In some embodiments, C₃₋₁₅ cycloalkyl is optionally substituted with one or more (e.g., 1-3, 1, 2 or 3) substituents selected from the group consisting of: deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, (R^{20a})ₚ₂₀ₐ indicates that the 1H-pyrrolo[2,3-b]pyridine ring in Formula (PBM-20) to which it is attached is optionally substituted with p20a R^{20a} , wherein each R^{20a} is independently cyano, halogen (e.g., fluorine, chlorine, bromine, or iodine), hydroxy, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p20a represents an integer of 0, 1, 2, 3, 4 or 5.

In some embodiments, (R^{20a})ₚ₂₀ₐ indicates that the 1H-pyrrolo[2,3-b]pyridine ring in Formula (PBM-20) to which it is attached is substituted with p20a R^{20a} , wherein p20a represents an integer of 1, and R^{20a} is cyano.

In some embodiments, (R^{20b})_{p20b} indicates that the pyridine ring to which it is attached is optionally substituted with p20b R^{20b}, wherein each R^{20b} is independently cyano, halogen (e.g., fluorine, chlorine, bromine, or iodine), hydroxy, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or NR^{20e}R^{20d}, where R^{20e} and R^{20d} are identical or different and each independently represent hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted C₃₋₁₅cycloalkyl (e.g., optionally substituted C₃₋₁₂cycloalkyl, and optionally substituted C₃₋₈cycloalkyl) or optionally substituted 4- to 15-membered heterocyclyl (e.g., optionally substituted 4- to 12-membered heterocyclyl or optionally substituted 4- to 10-membered heterocyclyl), and p20b represents an integer of 0, 1, 2 or 3. In some sub-embodiments, examples of the optionally substituted C₃₋₁₅ cycloalkyl include, but are not limited to, optionally substituted cyclopropyl, optionally substituted cyclobutyl, optionally substituted cyclopentyl,, optionally substituted cyclohexyl, optionally substituted cycloheptyl, or optionally substituted cyclooctyl. In some sub-embodiments, examples of the optionally substituted 4- to 15-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl, azacyclooctyl, and diazacyclooctyl. In some sub-embodiments, substituents of the optionally substituted C₃₋₁₅ cycloalkyl and optionally substituted 4- to 15-membered heterocyclyl are optionally selected from the group consisting of: deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, (R^{21b})_{p20b} in Formula (PBM-20) indicates that the pyridine ring to which it is attached is substituted with p20b R^{20b} , wherein p20b represents an integer of 1, and R^{20b} is NR^{20c}R^{20d}, wherein R^{20c} represents hydrogen, and R^{20d} represents C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

In some embodiments, PBM represents the structure of Formula (PBM-20-1), Formula (PBM-20-2), Formula (PBM-20-3), or Formula (PBM-20-4):

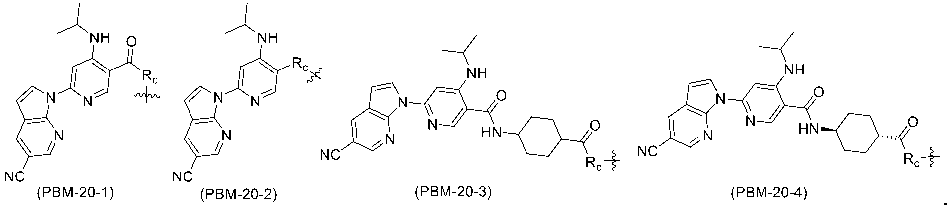

In some embodiments, PBM represents the structure of Formula (PBM-23): 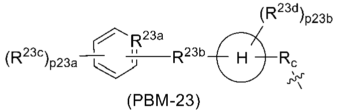
wherein R^{23a} in Formula (PBM-23) represents N or CH;
R^{23b} represents NHC(O), C(O)NH, NHS(O)₂ or S(O)₂NH;
(R^{23c})ₚ₂₃ₐ indicates that the 6-membered ring containing R^{23a} is substituted with p23a R^{23c} , wherein each R^{23c} is identical or different and each independently represents halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CHO-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O-or CH₂ClCH₂-O-), and p23a represents an integer of 0, 1, 2, 3 or 4; and
ring H represents 5- to 15-membered heteroarylene, and (R^{23d})_{p23b} indicates that the ring H to which it is attached is optionally substituted with p23b R^{23d} , wherein each R^{23d} is identical or different and each independently represents halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), hydroxy-substituted C₁₋₆ alkyl, amino-substituted C₁₋₆ alkyl, C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or optionally substituted C₃₋₁₅cycloalkyl, and p23b represents an integer of 0, 1, 2 or 3.

In some embodiments, R^{23a} represents N. In some embodiments, R^{23a} represents CH.

In some embodiments, (R^{23c})ₚ₂₃ₐ indicates that the 6-membered ring containing R^{23a} is substituted with p23a R^{23c} , wherein each R^{23c} is identical or different and each independently represents halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., deuterated C₁₋₃ alkyl, such as CD₃), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and p23a represents an integer of 0, 1, 2, 3 or 4, and the aromatic ring containing R^{23a} is a pyridine ring or a benzene ring. In some embodiments, the aromatic ring containing R^{23a} is a pyridine ring, and R^{23c} represents F₃C-, and p23a represents an integer of 1.

In some embodiments, ring H represents 5- to 15-membered heteroarylene, e.g., 5- to 12-membered, or 5- to 10-membered heteroarylene. In some embodiments, examples of ring H include, but are not limited to, benzimidazolylene, benzothiazolylene, 2H-indazolylene, benzoxazolylene, isoindolylene, benzofuranylene, isobenzofuranylene, benzothienylene, benzisoxazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, imidazo[1,2-b]pyridazinylene, and 6,7-dihydro-5H-pyrrolo[1,2-c]imidazolylene. Ring H is optionally substituted with p23b R^{23d} , wherein each R^{23d} is identical or different and each independently represents halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., deuterated C₁₋₃ alkyl, such as CD₃), hydroxy-substituted C₁₋₆ alkyl (e.g., hydroxy-substituted C₁₋₄ alkyl, such as hydroxypropyl, and hydroxyisopropyl), amino-substituted C₁₋₆ alkyl (e.g., amino-substituted C₁₋₄ alkyl, such as aminopropyl, aminoisopropyl), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, F₂ClC-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or optionally substituted C₃₋₁₅cycloalkyl, and p23b represents an integer of 0, 1, 2 or 3. In some embodiments, R^{23d} represents hydroxypropyl or hydroxyisopropyl, and hydroxypropyl or hydroxyisopropyl, and p23b represents an integer of 1. In some embodiments, R^{23d} represents optionally substituted C₃₋₁₅ cycloalkyl. Examples of C₃₋₁₅ cycloalkyl include, but are not limited to, cyclopropyl, cyclobutyl, cyclopentyl,, cyclopentenyl, cyclohexyl, cyclohexenyl, cycloheptyl, cyclooctyl, decalinyl, octahydropentalenyl, octahydro-1H-indenyl, 2,3-dihydro-1H-indenyl, spiro-cycloalkyl (e.g., C₅₋₁₅ spiro-cycloalkyl), adamantanyl, noradamantanyl, and norbornyl. The optional substituents of C₃₋₁₅ cycloalkyl are optionally selected from the group consisting of halogen (e.g., fluorine, chlorine, bromine, or iodine), hydroxy, mercapto, cyano, amino, nitro, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-).

In some embodiments, PBM represents a structure of Formula (PBM-23-1), Formula (PBM-23-2), Formula (PBM-23-3), Formula (PBM-23-4), Formula (PBM-23-5), Formula (PBM-23-6), Formula (PBM-23-7), Formula (PBM-23-8), Formula (PBM-23-9), Formula (PBM-23-10), Formula (PBM-23-11), Formula (PBM-23-12), or Formula (PBM-23-13):

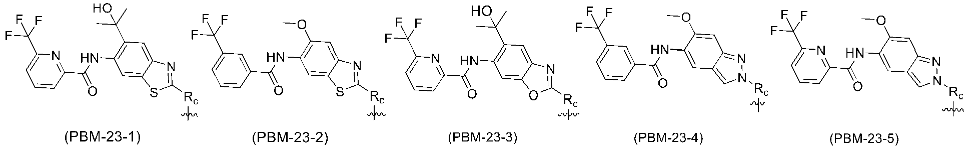

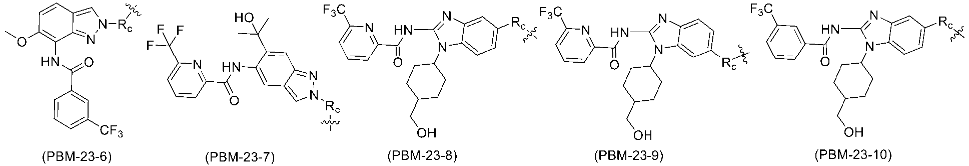

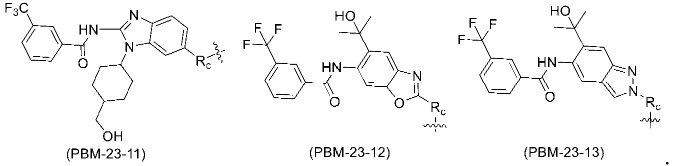

In some embodiments, PBM represents a small molecule ligand of CDK9.

In some embodiments, PBM represents a structure of Formula (PBM-13): 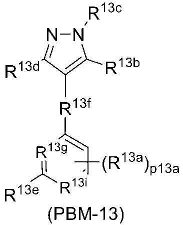
wherein R^{13g} in Formula (PBM-13) represents N or CR^{13h}, where R^{13h} represents hydrogen or halogen (e.g., fluorine, chlorine, bromine, or iodine), and R¹³ⁱ represents N or CR^{13j}, where R^{13j} represents hydrogen or halogen (e.g., fluorine, chlorine, bromine, or iodine);
R^{13f} represents a bond, -C(O)NH-, -NHC(O)- , -S(O)₂NH- or -NHS(O)₂-;
(R^{13a})ₚ₁₃ₐ indicates that the 6-membered ring containing R^{13g} and R¹³ⁱ in Formula (PBM-13) is optionally substituted with p13a R^{13a}, with each R^{13a} being identical or different and each independently representing deuterium, halogen (e.g., fluorine, chlorine, bromine, or iodine), hydroxy, cyano, amino, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-);
p13a represents an integer of 0, 1 or 2 ;
R^{13b} represents hydrogen, halogen (e.g., fluorine, chlorine, bromine, or iodine), C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl;
R^{13c} represents hydrogen, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R^{13b} and R^{13c} together with their respective attached nitrogen and carbon atoms form an optionally substituted 5- to 7-membered nitrogen-containing heterocycle;
R^{13d} represents hydrogen or R_{c}, and R^{13e} represents hydrogen or R_{c}, wherein R^{13d} and R^{13e} are not simultaneously hydrogen, and among R^{13d} and R^{13e}, there is one and only one that represents R_{c}, while the other represents hydrogen.

In some embodiments, R^{13f} in Formula (PBM-13) represents a bond.

In some embodiments, R^{13f} in Formula (PBM-13) represents -C(O)NH-, -NHC(O)- , -S(O)₂NH- or -NHS(O)₂-.

In some embodiments, R^{13b} in Formula (PBM-13) represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl (e.g., -methylene-cyclopropyl); and/or

R^{13c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{13b} and R^{13c} in Formula (PBM-13) together with their respective attached nitrogen and carbon atoms form an optionally substituted 5- to 7-membered nitrogen-containing heterocycle. In some embodiments, the 5- to 7-membered nitrogen-containing heterocycleincludes, but is not limited to, e.g., 5- to 7-membered heterocycle (e.g., 5- to 6-membered or 5-membered heterocycle) containing one nitrogen atom and optionally containing heteroatoms selected from nitrogen, oxygen, and sulfur. The 5- to 7-membered nitrogen-containing heterocycle is fused with the pyrazole ring in Formula (PBM-13) to form a fused ring system, including, but not limited to, a 5,6-dihydro-4H-pyrrolo[1,2-b]pyrazole ring. The 5- to 7-membered nitrogen-containing heterocycle is optionally substituted with one or more (e.g., 1 to 6, or 1 to 5, or 1 to 4, 1 to 3, or 2) substituents selected from the group consisting of: halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.).

In some embodiments, the 5- to 7-membered nitrogen-containing heterocycle, formed by R^{13b} and R^{13c} together with their respective attached nitrogen and carbon atoms, is fused with the adjacent pyrazole ring to form the following groups:

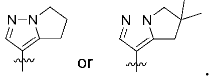

In some embodiments, PBM represents the structure of Formula (PBM-13-1A): 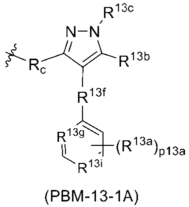
wherein R^{13g} represents N or CR^{13h}, where R^{13h} represents hydrogen or halogen, R¹³ⁱ represents N or CR^{13j}, where R^{13j} represents hydrogen or halogen;
R^{13f} represents a bond, -C(O)NH-, -NHC(O)- , -S(O)₂NH- or -NHS(O)₂-;
(R^{13a})ₚ₁₃ₐ indicates that the 6-membered ring containing R^{13g} and R¹³ⁱ to which it is attached is optionally substituted with p13a R^{13a} , wherein each R^{13a} represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p13a represents an integer of 0, 1 or 2 ;
R^{13b} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃ alkylene-C₃₋₆ cycloalkyl (e.g., -methylene-cyclopropyl);
R^{13c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R^{13b} and R^{13c} together with their respective attached nitrogen and carbon atoms form an optionally substituted 5- to 7-membered nitrogen-containing heterocycle.

In some embodiments, PBM represents the structure of Formula (PBM-13-1B): 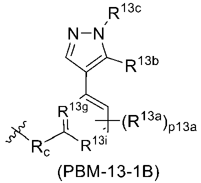
wherein R^{13g} represents N or CR^{13h}, where R^{13h} represents hydrogen or halogen, R¹³ⁱ represents N or CR^{13j}, where R^{13j} represents hydrogen or halogen;
(R^{13a})ₚ₁₃ₐ indicates that the 6-membered ring containing R^{13g} and R¹³ⁱ to which it is attached is optionally substituted with p13a R^{13a} , wherein each R^{13a} represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p13a represents an integer of 0, 1 or 2 ;
R^{13b} represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃alkylene-C₃₋₆cycloalkyl (e.g., -methylene-cyclopropyl);
R^{13c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R^{13b} and R^{13c} together with their respective attached nitrogen and carbon atoms form an optionally substituted 5- to 7-membered nitrogen-containing heterocycle.

In some embodiments, R^{13b} in each of Formula (PBM-13-1A) and Formula (PBM-13-1B) each independently represents hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or -C₁₋₃alkylene-C₃₋₆cycloalkyl (e.g., -methylene-cyclopropyl).

In some embodiments, R^{13c} in each of Formula (PBM-13-1A) and Formula (PBM-13-1B) each independently represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{13b} and R^{13c} in Formula (PBM-13-1A) each independently represents hydrogen.

In some embodiments, R^{13b} in Formula (PBM-13-1B) represents -methylene-cyclopropyl, and R^{13c} represents methyl.

In some embodiments of Formula (PBM-13-1A) and Formula (PBM-13-1B), the pyrazole ring is fused with a nitrogen-containing heterocycle, which is formed by R^{13b} and R^{13c} together with their respective attached nitrogen and carbon atoms, to form the following group:

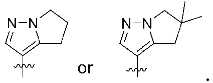

In some embodiments, PBM represents the structure of Formula (PBM-13-1) or Formula (PBM-13-2):

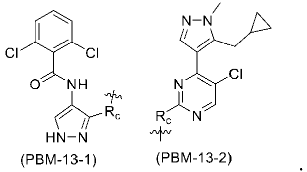

In some embodiments, PBM represents the structure of Formula (PBM-14): 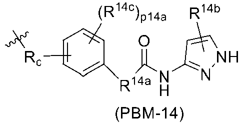
wherein R^{14a} represents a bond, C₁₋₃ alkylene (e.g., methylene, ethylene, or propylene) or halogenated C₁₋₃ alkylene (e.g., halogenated methylene, halogenated ethylene or halogenated propylene);
R^{14b} represents halogen (e.g., fluorine, chlorine, bromine, or iodine), C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), optionally substituted C₃₋₁₅cycloalkyl (e.g., optionally substituted cyclopropyl, optionally substituted cyclobutyl, optionally substituted cyclopentyl, or optionally substituted cyclohexyl; or e.g., halogenated C₃₋₆cycloalkyl, such as halogenated cyclopropyl, halogenated cyclobutyl, halogenated cyclopentyl, or halogenated cyclohexyl), or optionally substituted C₁₋₆ alkyl (e.g., optionally substituted C₁₋₅ alkyl, optionally substituted C₁₋₄ alkyl or optionally substituted C₁₋₃ alkyl);
(R^{14c})ₚ₁₄ₐ indicates that the benzene ring in Formula (PBM-14) is optionally substituted with p14a R^{14c}, with each R^{14c} being identical or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl or halogenated C₁₋₆ alkyl; and
p14a represents an integer of 0, 1, 2, 3 or 4.

In some embodiments, R^{14a} in Formula (PBM-14) represents a bond.

In some embodiments, R^{14a} in Formula (PBM-14) represents C₁₋₃ alkylene (e.g., methylene, ethylene, or propylene) or halogenated C₁₋₃ alkylene (e.g., halogenated methylene, halogenated ethylene or halogenated propylene).

In some embodiments, R^{14b} in Formula (PBM-14) represents optionally substituted C₃₋₁₅ cycloalkyl, e.g., optionally substituted C₃₋₈ cycloalkyl, such as optionally substituted cyclopropyl, optionally substituted cyclobutyl, optionally substituted cyclopentyl, or optionally substituted cyclohexyl. In some embodiments, C₃₋₁₅ cycloalkyl is optionally substituted with one or more (e.g., 1-3, 1, 2 or 3) substituents selected from the group consisting of: deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, C₁₋₆ alkoxy (e.g., C₁₋₄ alkoxy, such as methoxy, CD₃-O-, ethoxy, propoxy, isopropoxy, n-butoxy, sec-butoxy, or tert-butoxy), halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, R^{14b} in Formula (PBM-14) represents cyclobutyl.

In some embodiments, PBM represents the structure of Formula (PBM-14-1):

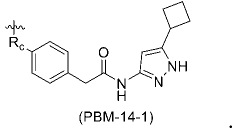

In some embodiments, PBM represents the structure of Formula (PBM-15): 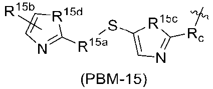
wherein R^{15a} in Formula (PBM-15) represents C₁₋₃ alkylene (e.g., methylene, ethylene, or propylene) or halogenated C₁₋₃ alkylene (e.g., halogenated methylene, halogenated ethylene or halogenated propylene); and
R^{15b} represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted C₃₋₆ cycloalkyl; and
R^{15c} and R^{15d} each independently represent NH, S or O.

In some embodiments, R^{15b} in Formula (PBM-15) represents optionally substituted C₃₋₆ cycloalkyl, e.g., optionally substituted cyclopropyl, optionally substituted cyclobutyl, optionally substituted cyclopentyl, or optionally substituted cyclohexyl. In some embodiments, C₃₋₆ cycloalkyl is optionally substituted with one or more (e.g., 1-3, 1, 2 or 3) substituents selected from the group consisting of: deuterium, hydroxy, amino, mercapto, nitro, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally deuterated C₃₋₆cycloalkyl (e.g., optionally perdeuterated cyclopropyl, optionally perdeuterated cyclobutyl, optionally perdeuterated cyclopentyl, or optionally perdeuterated cyclohexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or any combination thereof.

In some embodiments, PBM represents the structure of Formula (PBM-15-1):

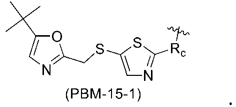

In some embodiments, PBM represents the structure of Formula (PBM-16): 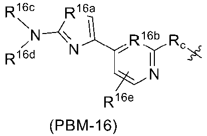
wherein R^{16a} in Formula (PBM-16) represents -S- or a fragment 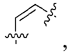 wherein when R^{16a} represents -S-, the heteroaryl containing R^{16a} and nitrogen atom in Formula (PBM-16) is thiazolyl, and when R^{16a} represents the fragment 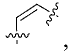 the heteroaryl containing R^{16a} and nitrogen atom in Formula (PBM-16) is pyridyl;
R^{16b} represents N or CH;
R^{16c} represents hydrogen or optionally substituted C₁₋₁₀ alkyl;
R^{16d} represents hydrogen, -C₁₋₃ alkylene-optionally substituted 4- to 10-membered heterocyclyl, or optionally substituted 4- to 10-membered heterocyclyl; and
R^{16c} represents hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{16a} in Formula (PBM-16) represents -S-, the heteroaryl containing R^{16a} and nitrogen atom in Formula (PBM-16) is thiazolyl.

In some embodiments, R^{16a} in Formula (PBM-16) represents the fragment 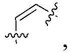 the heteroaryl containing R^{16a} and nitrogen atom in Formula (PBM-16) is pyridyl.

In some embodiments, R^{16c} in Formula (PBM-16) represents hydrogen or optionally substituted C₁₋₁₀ alkyl. In some embodiments, examples of the optionally substituted C₁₋₁₀ alkyl include, but are not limited to, optionally substituted C₁₋₅ alkyl, optionally substituted C₁₋₆ alkyl, or optionally substituted C₁₋₄ alkyl. In some embodiments, C₁₋₁₀ alkyl is optionally susbstituted with one or more (e.g., 1-3, 1, 2 or 3) substituents selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, and any combination thereof.

In some embodiments, R^{16d} in Formula (PBM-16) represents hydrogen, -C₁₋₃ alkylene-optionally substituted 4- to 10-membered heterocyclyl, or optionally substituted 4- to 10-membered heterocyclyl. In some embodiments, each optionally substituted 4- to 10-membered heterocyclyl is independently e.g., 4-to 10-membered (e.g., 4- to 9-membered, 4- to 8-membered, 5- to 7-membered, or 4- to 6-membered) monocyclic or bicyclic heterocyclyl group containing one or more (e.g., 1-4, or 1, 2, 3, or 4) heteroatoms selected from nitrogen, oxygen, and sulfur, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 10-membered heterocyclyl is optionally substituted with a substituent selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, and any combination thereof.

In some embodiments, PBM represents the structure of Formula (PBM-16-1A) or Formula (PBM-16-1B): 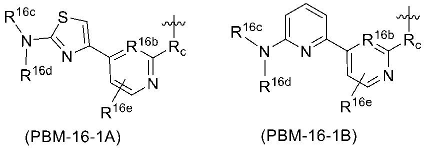
wherein R^{16b} represents N or CH;
R^{16c} represents hydrogen or optionally substituted C₁₋₁₀ alkyl;
R^{16d} represents hydrogen, -C₁₋₃ alkylene-optionally substituted 4- to 10-membered heterocyclyl, or optionally substituted 4- to 10-membered heterocyclyl; and
R^{16c} represents hydroxy, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{16c} in each of Formula (PBM-16-1A) and Formula (PBM-16-1B) each independently represents hydrogen or optionally substituted C₁₋₁₀ alkyl. In some embodiments, examples of the optionally substituted C₁₋₁₀ alkyl include, but are not limited to, optionally substituted C₁₋₄ alkyl, optionally substituted C₁₋₆ alkyl, or optionally substituted C₁₋₄ alkyl. In some embodiments, C₁₋₁₀ alkyl is optionally substituted with one or more (e.g., 1-3, 1, 2, or 3) substituents selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆cycloalkyl, and any combination thereof.

In some embodiments, R^{16d} in each of Formula (PBM-16-1A) and Formula (PBM-16-1B) each independently represents hydrogen, -C₁₋₃ alkylene-optionally substituted 4- to 10-membered heterocyclyl, or optionally substituted 4- to 10-membered heterocyclyl. In some embodiments, each optionally substituted 4- to 10-membered heterocyclyl is independently e.g., 4- to 10-membered (e.g., 4- to 9-membered, 4- to 8-membered, 5- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 10-membered heterocyclyl is optionally substituted with a substituent selected from the group consisting of deuterium, hydroxy, amino, mercapto, nitro, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₃₋₆ cycloalkyl, and any combination thereof.

In some embodiments, PBM represents a structure of Formula (PBM-16-1) or Formula (PBM-16-2):

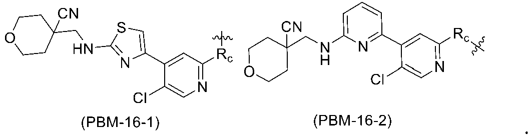

In some embodiments, PBM represents a small molecule ligand of EZH2.

In some embodiments, PBM represents the structure of Formula (PBM-17): 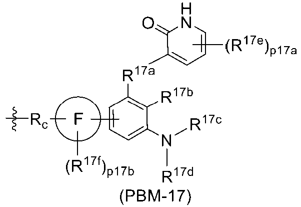
wherein R^{17a} in Formula (PBM-17) represents C(O)NH, NHC(O), (CH₂)₁₋₂C(O)NH, (CH₂)₁₋₂NHC(O), C(O)NH(CH₂)₁₋₂, NHC(O)(CH₂)₁₋₂, S(O)₂NH or NHS(O)₂;
R^{17b} represents hydrogen, hydroxy, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₅ alkoxy, C₁₋₄ alkoxy or C₁₋₃ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
R^{17c} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); or
R^{17b} and R^{17c}, together with their respective attached carbon and nitrogen atoms and the carbon atom on the benzene ring attached to the nitrogen atom, form an optionally substituted 4- to 6-membered heteroaromatic ring or an optionally substituted 4- to 6-membered heterocycle ring;
R^{17d} represents optionally substituted C₃₋₆cycloalkyl (e.g., optionally substituted cyclopropyl, optionally substituted cyclobutyl, optionally substituted cyclopentyl, or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 20-membered heterocyclyl;
(R^{17e})ₚ₁₇ₐ indicates that pyridin-2(1H)-one ring to which it is attached is substituted with p17a R^{17e}, with each R^{17e} being identical or different and each independently representing hydroxy, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p17a represents an integer of 0, 1, 2 or 3;
ring F in Formula (PBM-17) represents arylene or 5- to 20-membered monocyclic or bicyclic heteroarylene containing from 1 to 4 heteroatoms selected from the group consisting of oxygen, nitrogen, and sulfur; and (R^{17f})_{p17b} indicates that the ring F is optionally substituted with p17b R^{17f}, with each R^{17f} being identical or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, CICH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
p17b represents an integer of 0, 1, 2, 3 or 4.

In some embodiments, R^{17a} in Formula (PBM-17) represents C(O)NH, NHC(O), CH₂C(O)NH, CH₂CH₂C(O)NH, CH₂NHC(O), CH₂CH₂NHC(O), C(O)NHCH₂, C(O)NHCH₂CH₂, NHC(O)CH₂, NHC(O)CH₂CH₂, S(O)₂NH or NHS(O)₂.

In some embodiments, R^{17b} in Formula (PBM-17) represents hydrogen, hydroxy, halogen (e.g., fluorine, chlorine, bromine, or iodine), cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{17c} in Formula (PBM-17) represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-).

In some embodiments, R^{17b} and R^{17c} in Formula (PBM-17), together with their respective attached carbon and nitrogen atoms and the carbon atom on the benzene ring attached to the nitrogen atom, form an optionally substituted 4- to 6-membered heteroaromatic ring (e.g., optionally substituted 4-to 6-membered nitrogen-containing heteroaromatic ring) or optionally substituted 4- to 6-membered heterocycle ring (e.g., optionally substituted 4- to 6-membered nitrogen-containing heterocycle ring). In some embodiments, R^{17b} and R^{17c} in Formula (PBM-17) together represent the following fragment: -CH₂-, -CH=CH-, -CH=N-, -N=CH-, -N=N-, -CH₂-CH₂-, -NH-CH₂-, or -CH₂-CH₂-CH₂-. In some embodiments, the 4- to 6-membered nitrogen-containing heterocycle ring includes, but is not limited to, e.g., 4- to 6-membered (e.g., 4-membered, 5-membered or 6-membered) heterocycle containing one nitrogen atom and optionally containing heteroatoms selected from nitrogen, oxygen, and sulfur. The 4- to 6-membered nitrogen-containing heterocycle is fused with the benzene ring in Formula (PBM-17) to form a fused ring system. The 4- to 6-membered nitrogen-containing heterocycle is optionally substituted with one or more (e.g., 1-4, 1-3, or 2) substituents selected from the group consisting of: halogen (e.g., fluorine, chlorine, bromine, or iodine), hydroxy, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.). In some embodiments, R^{17d} in Formula (PBM-17) represents optionally substituted C₃₋₆ cycloalkyl (e.g., optionally substituted cyclopropyl, optionally substituted cyclobutyl, optionally substituted cyclopentyl, or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 20-membered heterocyclyl.

In some embodiments, R^{17d} in Formula (PBM-17) represents optionally substituted C₃₋₆ cycloalkyl, such as optionally substituted cyclopropyl, optionally substituted cyclobutyl, optionally substituted cyclopentyl, or optionally substituted cyclohexyl. In some embodiments, C₃₋₆ cycloalkyl is optionally substituted with one or more (e.g., 1-3, 1, 2 or 3) substituents selected from the group consisting of: deuterium, hydroxy, amino, mercapto, nitro, halogen, cyano, C₁₋₆ alkyl, deuterated C₁₋₆ alkyl, halogenated C₁₋₆ alkyl, C₃₋₆cycloalkyl, C₁₋₆ alkoxy, halogenated C₁₋₆ alkoxy or any combination thereof.

In some embodiments, R^{17d} in Formula (PBM-17) represents optionally substituted 4- to 20-membered heterocyclyl. The optionally substituted 4- to 20-membered heterocyclyl is e.g., optionally substituted 4- to 20-membered (e.g., 4- to 15-membered, 4- to 9-membered, 4- to 8-membered, 5- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 20-membered heterocyclyl is optionally substituted with a substituent selected from the group consisting of deuterium, hydroxy, amino, mercapto, nitro, halogen, cyano, C₁₋₆ alkyl, deuterated C₁₋₆ alkyl, halogenated C₁₋₆ alkyl, C₃₋₆ cycloalkyl, C₁₋₆ alkoxy, halogenated C₁₋₆ alkoxy, and any combination thereof.

In some embodiments, ring F in Formula (PBM-17) represents arylene (e.g., C₅₋₂₀ arylene, C₅₋₁₅ arylene, C₅₋₁₀ arylene, or C₅₋₆ arylene) or 5- to 20-membered (e.g., 5- to 15-membered, 5- to 10-membered, 5- to 9-membered, 5- to 8-membered, 5- to 7-membered, or 6-membered) monocyclic or bicyclic heteroarylene group containing from 1 to 4 (e.g., from 1 to 3, or from 1 to 2) heteroatoms selected from N, O, and S. Examples of the arylene include, but are not limited to, phenylene and naphthylene. Examples of the heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-furo[3,2-b]pyrrolylene, pyrrolo[2,1-b]thiazolylene, and imidazo[2,1-b]thiazolylene. In some embodiments, the ring F is optionally substituted with p17b R^{17f} , with each R^{17f} being identical or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, CICH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and p17b represents an integer of 0, 1, 2, 3 or 4.

In some embodiments, PBM represents the structure of Formula (PBM-17-1A), Formula (PBM-17-1B), Formula (PBM-17-1C), Formula (PBM-17-1D), Formula (PBM-17-1E), Formula (PBM-17-1F), Formula (PBM-17-1G), Formula (PBM-17-1H), Formula (PBM-17-1I), Formula (PBM-17-1J), or Formula (PBM-17-1K): 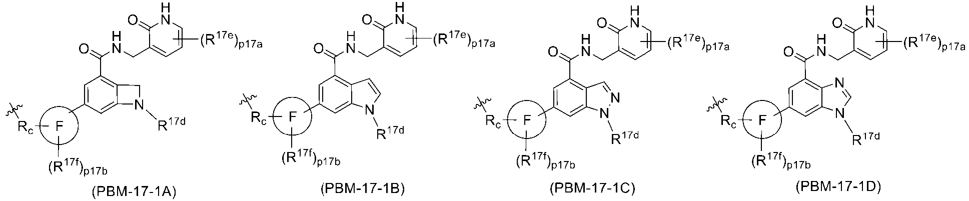 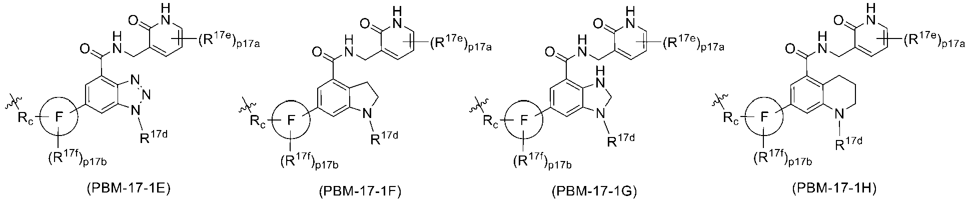 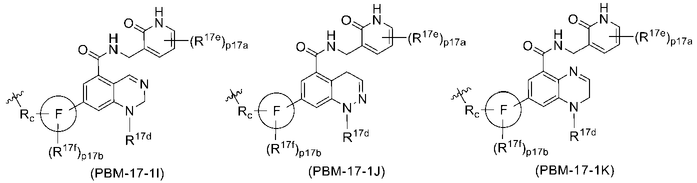
wherein R^{17d} in each of the above formulae independently represents optionally substituted C₃₋₆cycloalkyl (e.g., optionally substituted cyclopropyl, optionally substituted cyclobutyl, optionally substituted cyclopentyl, or optionally substituted cyclohexyl), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), or optionally substituted 4- to 10-membered heterocyclyl;
(R^{17e})ₚ₁₇ₐ in each of the above formulae independently represents indicates that pyridin-2(1H)-one ring to which it is attached is substituted with p17a R^{17e}, wherein each R^{17e} is identical or different and each independently represents hydroxy, halogen (e.g., fluorine, chlorine, bromine, or iodine), cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p17a in each of the above formulae independently represents an integer of 0, 1, 2 or 3;
ring F in each of the above formulae independently represents arylene or 5- to 20-membered monocyclic or bicyclic heteroarylene containing from 1 to 4 heteroatoms selected from the group consisting of oxygen, nitrogen, and sulfur; and (R^{17f})_{p17b} indicates that the ring F is optionally substituted with p17b R^{17f}, with each R^{17f} being identical or different and each independently representing deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), and halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and
p17b in each of the above formulae independently represents an integer of 0, 1, 2, 3 or 4.

In some embodiments, each R^{17d} in Formula (PBM-17-1A), Formula (PBM-17-1B), Formula (PBM-17-1C), Formula (PBM-17-1D), Formula (PBM-17-1E), Formula (PBM-17-1F), Formula (PBM-17-1G), Formula (PBM-17-1H), Formula (PBM-17-1I), Formula (PBM-17-1J), and Formula (PBM-17-1K) independently represents optionally substituted C₃₋₆ cycloalkyl, such as optionally substituted cyclopropyl, optionally substituted cyclobutyl, optionally substituted cyclopentyl, or optionally substituted cyclohexyl. In some embodiments, C₃₋₆ cycloalkyl is optionally substituted with one or more (e.g., 1-3, 1, 2 or 3) substituents selected from the group consisting of: deuterium, hydroxy, amino, mercapto, nitro, halogen, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, and any combination thereof.

In some embodiments, each R^{17d} in Formula (PBM-17-1A), Formula (PBM-17-1B), Formula (PBM-17-1C), Formula (PBM-17-1D), Formula (PBM-17-1E), Formula (PBM-17-1F), Formula (PBM-17-1G), Formula (PBM-17-1H), Formula (PBM-17-1I), Formula (PBM-17-1J), and Formula (PBM-17-1K) each independently represents optionally substituted 4- to 20-membered heterocyclyl. The optionally substituted 4- to 20-membered heterocyclyl is e.g., optionally substituted 4- to 20-membered (e.g., 4- to 15-membered, 4- to 12-membered, 4- to 9-membered, 4- to 8-membered, 5- to 7-membered, or 4- to 6-membered) monocyclic, bicyclic, or polycyclic heterocyclyl group containing one or more (e.g., 1-4, or 1, 2, 3, or 4) heteroatoms selected from nitrogen, oxygen, and sulfur, including but not limited to the following optionally substituted groups: azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). In some embodiments, the 4- to 20-membered heterocyclyl is optionally substituted with a substituent selected from the group consisting of deuterium, hydroxy, amino, mercapto, nitro, halogen, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, and any combination thereof.

In some embodiments, ring F in each of Formula (PBM-17-1A), Formula (PBM-17-1B), Formula (PBM-17-1C), Formula (PBM-17-1D), Formula (PBM-17-1E), Formula (PBM-17-1F), Formula (PBM-17-1G), Formula (PBM-17-1H), Formula (PBM-17-1I), Formula (PBM-17-1J), and Formula (PBM-17-1K) each independently represents arylene (e.g., C₅₋₂₀ arylene, C₅₋₁₅ arylene, C₅₋₁₀ arylene, or C₅₋₆arylene) or 5- to 20-membered (e.g., 5- to 15-membered, 5- to 10-membered, 5- to 9-membered, 5- to 8-membered, 5- to 7-membered, or 6-membered) monocyclic or bicyclic heteroarylene group containing from 1 to 4 (e.g., from 1 to 3, or from 1 to 2) heteroatoms selected from N, O, and S. Examples of the arylene include, but are not limited to, phenylene and naphthylene. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-furo[3,2-b]pyrrolylene, pyrrolo[2,1-b]thiazolylene, and imidazo[2,1-b]thiazolylene. In some embodiments, the ring F is optionally substituted with p17b R^{17f} , and each R^{17f} is identical or different and each independently represents deuterium, halogen (e.g., fluorine, chlorine, bromine, or iodine), hydroxy, cyano, amino, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, CICH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-); and p17b represents an integer of 0, 1, 2, 3 or 4.

In some embodiments, PBM represents a structure of Formula (PBM-17-1), Formula (PBM-17-2), or Formula (PBM-17-3):

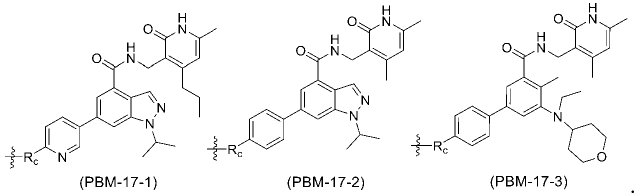

In some embodiments, PBM represents a small molecule ligand of BCL-2.

In some embodiments, PBM represents the structure of Formula (PBM-24): 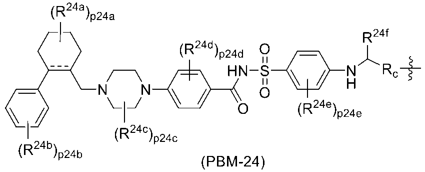
wherein the dashed line --- in Formula (PBM-24) indicates the optional presence of a double bond;
(R^{24a})ₚ₂₄ₐ indicates that the 6-membered cycloalkyl ring to which it is attached is optionally substituted with p24a R^{24a}, wherein each R^{24a} is identical or different and each independently represents halogen (e.g., fluorine, chlorine, bromine, or iodine), hydroxy, mercapto, cyano, amino, nitro, oxo, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p24a represents an integer of 0, 1, 2, 3, 4, 5, 6, 7 or 8;
(R^{24b})_{p24b} indicates that the benzene ring to which it is attached is optionally substituted with p24b R²⁴⁶, wherein each R²⁴⁶ is identical or different and each independently represents deuterium, halogen (e.g., fluorine, chlorine, bromine, or iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p24b represents an integer of 0, 1, 2, 3, 4 or 5;
(R^{24c})_{p24c} indicates that the 6-membered cycloalkyl ring to which it is attached is optionally substituted with p24c R^{24c} , wherein each R^{24c} is identical or different and each independently represents halogen (e.g., fluorine, chlorine, bromine, or iodine), cyano, oxo, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy or halogenated C₁₋₃ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p24c represents an integer of 0, 1, 2, 3, 4, 5, 6, 7 or 8;
(R^{24d})_{p24d} indicates that the benzene ring to which it is attached is optionally substituted with p24d R^{24d} , wherein each R^{24d} is identical or different and each independently represents halogen (e.g., fluorine, chlorine, bromine, or iodine), hydroxy, mercapto, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or 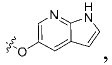 and p24d represents an integer of 0, 1 or 2;
(R^{24e})ₚ₂₄ₑ indicates that the benzene ring to which it is attached is optionally substituted with p24e R^{24e} , wherein each R^{24e} is identical or different and each independently represents deuterium, halogen (e.g., fluorine, chlorine, bromine, or iodine), hydroxy, mercapto, cyano, amino, nitro, -SO₂CF₃, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p24e represents an integer of 0, 1, 2, 3 or 4; and
R^{24f} represents hydrogen, deuterium, halogen (e.g., fluorine, chlorine, bromine, or iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy or halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or 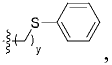 wherein y represents an integer of 1, 2 or 3.

In some sub-embodiments, the dashed line --- in Formula (PBM-24) indicates the presence of a double bond; that is, the six-membered ring group containing this dashed line represents

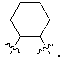

In some sub-embodiments, the dashed line --- in Formula (PBM-24) indicates the absence of a double bond; that is, the six-membered ring group containing this dashed line represents

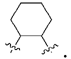

In some sub-embodiments, R^{24d} in Formula (PBM-24) represents 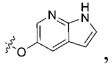 and p24d represents an integer of 1. In some sub-embodiments, p24d represents an integer of 0.

In some sub-embodiments, p24e in Formula (PBM-24) represents an integer of 1, and R^{24e} represents nitro (i.e., NO₂) or SO₂CF₃.

In some sub-embodiments, R^{24f} in Formula (PBM-24) represents hydrogen or 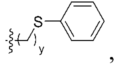 wherein y represents an integer of 1, 2 or 3.

In some sub-embodiments, PBM represents a structure of Formula (PBM-24-1), Formula (PBM-24-2), Formula (PBM-24-3), Formula (PBM-24-4), or Formula (PBM-24-5):

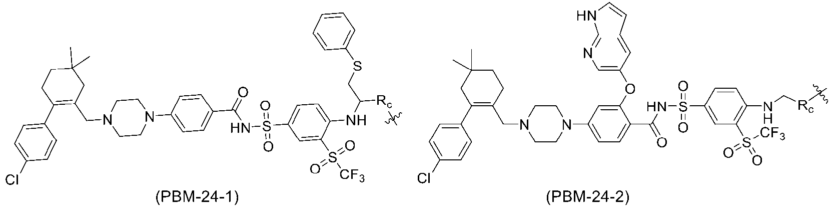

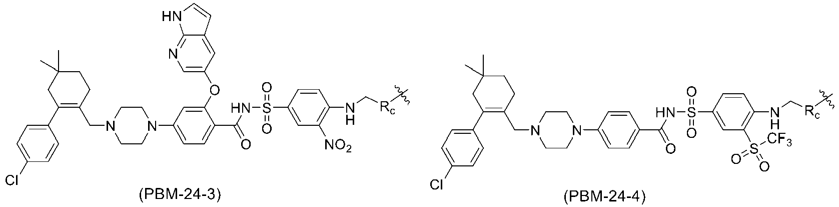

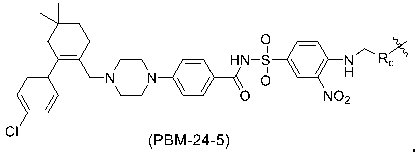

In some embodiments, PBM represents a small molecule ligand of RSK.

In some embodiments, PBM represents a structure of Formula (PBM-25): 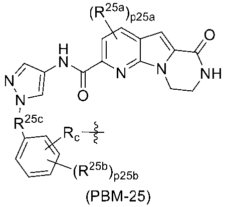
wherein (R^{25a})ₚ₂₅ₐ in Formula (PBM-25) represents p25a R^{25a} groups, wherein each R^{25a} is identical or different and each independently represents halogen (e.g., fluorine, chlorine, bromine, or iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p25a represents an integer of 0, 1, 2, 3, 4 or 5;
(R^{25b})_{p25b} indicates that the benzene ring to which it is attached is optionally substituted with p25b R^{25b}, wherein each R^{25b} is identical or different and each independently represents deuterium, halogen (e.g., fluorine, chlorine, bromine, or iodine), hydroxy, mercapto, cyano, amino, nitro, C₁₋₆ alkyl (e.g., C₁₋₄ alkyl, such as methyl, ethyl, propyl, isopropyl, butyl, sec-butyl, tert-butyl, pentyl, or hexyl), C₁₋₆ alkoxy (e.g., C₁₋₅ alkoxy or C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl or perdeuterated C₁₋₄ alkyl, such as CD₃, CD₃CD₂-, CD₃CD₂CD₂- etc.) or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), and p25b represents an integer of 0, 1, 2, 3 or 4; and
R^{25c} represents C₁₋₆ alkylene or halogenated C₁₋₆ alkylene.

In some embodiments, R^{25c} represents C₁₋₆ alkylene, such as methylene or ethylene.

In some embodiments, R^{25c} represents halogenated C₁₋₆ alkylene, e.g., difluoromethylene.

In some embodiments, PBM represents the structure of Formula (PBM-25-1):

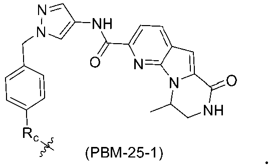

In some embodiments, PBM represents a small molecule ligand of SMARCA2/4.

In some embodiments, PBM represents the structure of Formula (PBM-18-1A) (which is an example of the structure of Formula (PBM-18)): 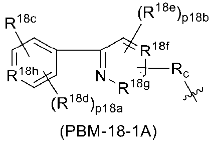
wherein R^{18f}, R^{18g}, and R^{18h} in Formula (PBM-18-1A) each independently represents CH or N;
R^{18c} represents halogen (e.g., fluorine, chlorine, bromine, or iodine), amino, hydroxy, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy or C₁₋₃ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), optionally substituted cycloalkyl (e.g., optionally substituted C₃₋₁₅cycloalkyl) or optionally substituted 4- to 15-membered nitrogen-containing heterocyclyl;
(R^{18d})ₚ₁₈ₐ indicates that the 6-membered ring to which it is attached is optionally substituted with p18a R^{18d} with each R^{18d} being identical or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p18a represents an integer of 0, 1, 2, 3 or 4;
(R^{13e})_{p18b} indicates that the 6-membered nitrogen-containing heteroaryl ring to which it is attached is optionally substituted with p18b R^{18e}, with each R^{18e} being identical or different and each independently representing hydroxy, amino, cyano, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p18b represents an integer of 0, 1 or 2.

In some embodiments, R^{18f} in Formula (PBM-18-1A) represents CH, and R^{18g} represents CH. In some embodiments, R^{18f} in Formula (PBM-18-1A) represents CH, and R^{18g} represents N. In some embodiments, R^{18f} in Formula (PBM-18-1A) represents N, and R^{18g} represents N. In some embodiments, R^{18f} in Formula (PBM-18-1A) represents N, and R^{18g} represents CH.

In some embodiments, R^{18f} in Formula (PBM-18-1A) represents CH, R^{18g} represents CH, and R^{18h} represents CH. In some embodiments, R^{18f} in Formula (PBM-18-1A) represents CH, R^{18g} represents N, and R^{18h} represents CH. In some embodiments, R^{18f} in Formula (PBM-18-1A) represents CH, R^{18g} represents N, and R^{18h} represents N. In some embodiments, R^{18f} in Formula (PBM-18-1A) represents CH, R^{18g} represents CH, and R^{18h} represents N. In some embodiments, R^{18f} in Formula (PBM-18-1A) represents N, R^{18g} represents N, and R^{18h} represents N. In some embodiments, R^{18f} in Formula (PBM-18-1A) represents N, R^{18g} represents CH, and R^{18h} represents N. In some embodiments, R^{18f} in Formula (PBM-18-1A) represents N, R^{18g} represents CH, and R^{18h} represents CH. In some embodiments, R^{18f} in Formula (PBM-18-1A) represents N, R^{18g} represents N, and R^{18h} represents CH.

In some embodiments, PBM represents a structure of Formula (PBM-18-1) or Formula (PBM-18-2):

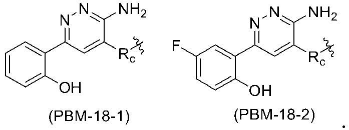

In some embodiments, PBM represents a small molecule ligand of BET.

In some embodiments, PBM represents the structure of Formula (PBM-26): 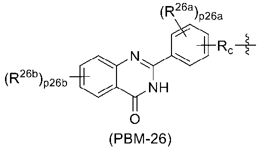
wherein (R^{26a})ₚ₂₆ₐ in Formula (PBM-26) indicates that the benzene ring to which it is attached is optionally substituted with p26a R^{26a} , wherein each R^{26a} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p26a represents an integer of 0, 1, 2, 3 or 4;
(R^{26b})_{p26b} indicates that the 4-oxo-3,4-dihydroquinazoline ring to which it is attached is substituted with p26b R^{26b} , wherein each R^{26b} is identical or different and each independently represents halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p26b represents an integer of 1, 2, 3 or 4.

In some embodiments, PBM represents the structure of Formula (PBM-26-1):

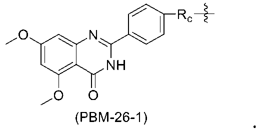

In some embodiments, PBM represents a small molecule ligand of CDK2.

In some embodiments, PBM represents the structure of Formula (PBM-27): 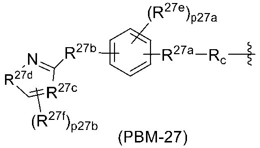
wherein R^{27a} represents S(O)₂ or a bond;
R^{27b} represents NH, NHC(O) or C(O)NH;
R^{27c} represents CH or N;
R^{27d} represents N(R^{27h}), wherein R^{27h} represents H, -C(O)-optionally substituted aryl or -C(O)-optionally substituted heteroaryl; or
R^{27d} represents a fragment 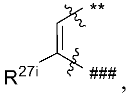 wherein symbol ** indicates the point of attachment to the adjacent nitrogen atom, symbol ### indicates the point of attachment to the adjacent carbon atom, and R²⁷ⁱ represents deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.);
(R^{27e})ₚ₂₇ₐ indicates that the benzene ring to which it is attached is optionally substituted with p27a R^{27e}, wherein each R^{27e} is identical or different and each independently represents deuterium, halogen (e.g., fluorine, chlorine, bromine, or iodine), hydroxy, amino, cyano, C₁₋₆ alkoxy, halogenated C₁₋₆ alkoxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p27a represents an integer of 0, 1, 2, 3 or 4;
(R^{27f})_{p27b} indicates that the nitrogen-containing ring to which it is attached is substituted by p27b R^{27f}, wherein each R^{27f} is identical or different and each independently represents deuterium, amino, halogen (e.g., fluorine, chlorine, bromine, or iodine), hydroxy, cyano, C₁₋₆ alkoxy, halogenated C₁₋₆ alkoxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), -NH-optionally substituted aryl, or -NH-optionally substituted heteroaryl; and
p27b represents an integer of 1 or 2.

In some embodiments, PBM represents the structure of Formula (PBM-27-A): 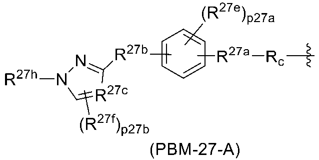
wherein R^{27a} represents S(O)₂ or a bond;
R^{27b} represents NH, NHC(O) or C(O)NH;
R^{27e} represents CH or N;
R^{27h} represents H, -C(O)-optionally substituted aryl or -C(O)-optionally substituted heteroaryl; or
(R^{27e})ₚ₂₇ₐ indicates that the benzene ring to which it is attached is optionally substituted with p27a R^{27e}, wherein each R^{27e} is identical or different and each independently represents deuterium, halogen (e.g., fluorine, chlorine, bromine, or iodine), hydroxy, amino, cyano, C₁₋₆ alkoxy, halogenated C₁₋₆ alkoxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p27a represents an integer of 0, 1, 2, 3 or 4;
(R^{27f})_{p27b} indicates that the nitrogen-containing ring to which it is attached is substituted by p27b R^{27f}, wherein each R^{27f} is identical or different and each independently represents deuterium, amino, halogen (e.g., fluorine, chlorine, bromine, or iodine), hydroxy, cyano, C₁₋₆ alkoxy, halogenated C₁₋₆ alkoxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), -NH-optionally substituted aryl, or -NH-optionally substituted heteroaryl; and
p27b represents an integer of 1 or 2.

In some embodiments, PBM represents the structure of Formula (PBM-27-B): 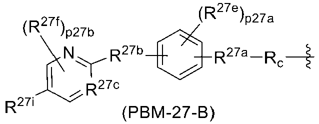
wherein R^{27a} represents S(O)₂ or a bond;
R^{27b} represents NH, NHC(O) or C(O)NH;
R^{27c} represents CH or N;
R²⁷ⁱ represents deuterium, hydrogen, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-) or deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.);
(R^{27e})ₚ₂₇ₐ indicates that the benzene ring to which it is attached is optionally substituted with p27a R^{27e}, wherein each R^{27e} is identical or different and each independently represents deuterium, halogen (e.g., fluorine, chlorine, bromine, or iodine), hydroxy, amino, cyano, C₁₋₆ alkoxy, halogenated C₁₋₆ alkoxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p27a represents an integer of 0, 1, 2, 3 or 4;
(R^{27f})_{p27b} indicates that the nitrogen-containing ring to which it is attached is substituted by p27b R^{27f}, wherein each R^{27f} is identical or different and each independently represents deuterium, amino, halogen (e.g., fluorine, chlorine, bromine, or iodine), hydroxy, cyano, C₁₋₆ alkoxy, halogenated C₁₋₆ alkoxy, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₅ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), -NH-optionally substituted aryl, or -NH-optionally substituted heteroaryl; and
p27b represents an integer of 1 or 2.

In some embodiments, R^{27h} in each of Formula (PBM-27) and Formula (PBM-27-A) independently represents H.

In some embodiments, R^{27h} in each of Formula (PBM-27) and Formula (PBM-27-A) independently represents -C(O)-optionally substituted aryl. In some sub-embodiments, examples of the aryl include, but are not limited to, C₆₋₂₀ aryl, C₆₋₁₅ aryl, and C₆₋₁₀ aryl, such as phenyl and naphthyl. The aryl is optionally substituted with one or more (e.g., 1-6, such as 1, 2, 3, 4, 5, or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-,Cl₂CH-O-, CF₃CF₂-O-, CF₃CHF-O-, CHF₂CF₂-O-, CHF₂CHF-O-, CF₃CH₂-O- or CH₂ClCH₂-O-), C(O)NH₂, and any combination thereof.

In some embodiments, R^{27h} in each of Formula (PBM-27) and Formula (PBM-27-A) independently represents -C(O)-optionally substituted heteroaryl. In some sub-embodiments, the heteroaryl comprises 5- to 20-membered heteroaryl (e.g., 5- to 15-membered heteroaryl), e.g., 5- to 20-membered heteroaryl containing one or two 5- to 7-membered rings and 1 to 4 heteroatoms selected from N, O, and S. Examples of the heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, imidazo[1,2-b]pyridazinyl, 6,7-dihydro-5H-pyrrolo[1,2-c]imidazolyl, and 7H-pyrrolo[2,3-d]pyrimidinyl. The heteroaryl group is optionally substituted with one or more (e.g., 1-6, such as 1, 2, 3, 4, 5, or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, (R^{27f})_{p27b} in each of Formula (PBM-27), Formula (PBM-27-A), and Formula (PBM-27-B) independently indicates that the nitrogen-containing ring to which it is attached is substituted by p27b R^{27f} , wherein each R^{27f} is identical or different and each independently represents halogen (e.g., fluorine, chlorine, bromine, or iodine), amino, -NH-optionally substituted aryl, or -NH-optionally substituted heteroaryl, and p27b represents an integer of 1 or 2. In some sub-embodiments, examples of the aryl include, but are not limited to, C₆₋₂₀ aryl, C₆₋₁₅ aryl, and C₆₋₁₀ aryl, such as phenyl and naphthyl. The aryl is optionally substituted with one or more (e.g., 1-6, such as 1, 2, 3, 4, 5, or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C(O)NH₂, and any combination thereof. In some sub-embodiments, the heteroaryl comprises 5- to 20-membered heteroaryl (e.g., 5- to 15-membered heteroaryl), e.g., 5- to 20-membered heteroaryl containing one or two 5- to 7-membered rings and 1 to 4 heteroatoms selected from N, O, and S. examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, imidazo[1,2-b]pyridazinyl, 6,7-dihydro-5H-pyrrolo[1,2-c]imidazolyl, and 7H-pyrrolo[2,3-d]pyrimidinyl. The heteroaryl group is optionally substituted with one or more (e.g., 1-6, such as 1, 2, 3, 4, 5, or 6) substituents independently selected from the group consisting of deuterium, halogen (e.g., fluorine, chlorine, bromine, or iodine), hydroxy, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃CO-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof.

In some embodiments, PBM represents the structure of Formula (PBM-27-1), Formula (PBM-27-2), Formula (PBM-27-3) or Formula (PBM-27-4):

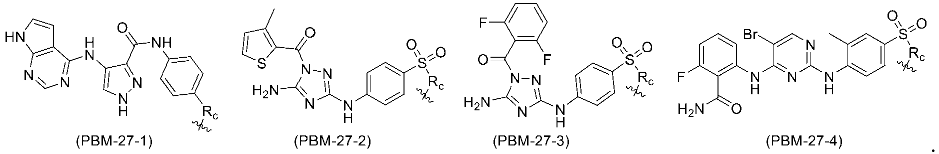

In some embodiments, PBM represents a small molecule ligand of Tau.

In some embodiments, PBM represents the structure of Formula (PBM-28): 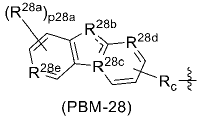
wherein (R^{28a})ₚ₂₈ₐ in Formula (PBM-28) indicates that the fused tricyclic ring to which it is attached is optionally substituted by p28a R^{28a} substituents, wherein each R^{28a} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p28a represents an integer of 0, 1, 2, 3, 4, 5 or 6;
-̅ -̅ -̅ in Formula (PBM-28) represents a single bond or a double bond;
the fragment 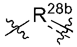 of the fused tricyclic ring in Formula (PBM-28) represents 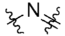 or 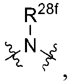 wherein R^{28f} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
wherein when the fragment 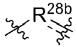 represents 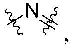 the fragment 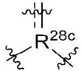 represents 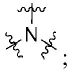
when the fragment 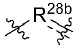 represents 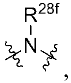 the fragment 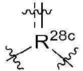 represents 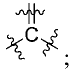 and
R^{28d} and R^{28e} are identical or different and each independently represent N or CH.

In some embodiments, PBM represents the structure of Formula (PBM-28-A): 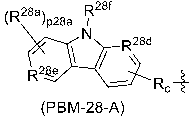
wherein (R^{28a})ₚ₂₈ₐ in Formula (PBM-28-A) indicates that the fused tricyclic ring to which it is attached is optionally substituted by p28a R^{28a} substituents, wherein each R^{28a} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p28a represents an integer of 0, 1, 2, 3, 4, 5 or 6;
R^{28f} represents hydrogen, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
R^{28d} and R^{28e} are identical or different and each independently represent N or CH.

In some embodiments, PBM represents the structure of Formula (PBM-28-B): 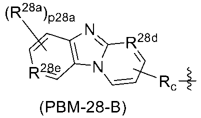
wherein (R^{28a})ₚ₂₈ₐ in Formula (PBM-28-B) indicates that the fused tricyclic ring to which it is attached is optionally substituted by p28a R^{28a} substituents, wherein each R^{28a} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p28a represents an integer of 0, 1, 2, 3, 4, 5 or 6; and
R^{28d} and R^{28e} are identical or different and each independently represent N or CH.

In some embodiments, PBM represents the structure of Formula (PBM-28-1), Formula (PBM-28-2), Formula (PBM-28-3), Formula (PBM-28-4) or Formula (PBM-28-5):

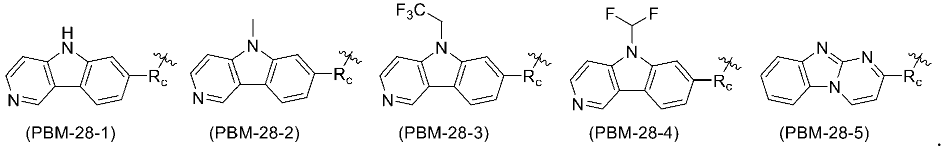

In some embodiments, PBM represents the structure of Formula (PBM-30): 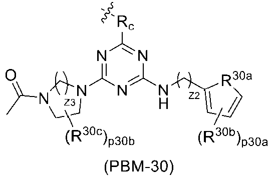
wherein R^{30a} in Formula (PBM-30) represents O, S or NH;
(R^{30b})ₚ₃₀ₐ indicates that the 5-membered ring containing R^{30a} to which it is attached is optionally substituted with p30a R^{30b} substituents, wherein each R^{30b} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p30a represents an integer of 0, 1, 2 or 3;
(R^{30c})_{p30b} indicates that the diazaheterocyclyl to which it is attached is optionally substituted by p30b R^{30c} substituents, wherein each R^{30c} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p30b represents an integer of 0, 1, 2, 3, 4, 5, 6, 7, 8, 9 or 10;
Z2 represents an integer of 1, 2 or 3; and
Z3 represents an integer of 1, 2, 3, 4, 5 or 6;

In some embodiments, PBM represents the structure of Formula (PBM-30-1):

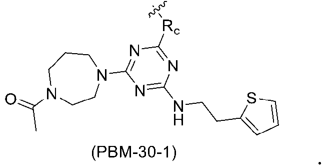

In some embodiments, PBM represents a small molecule ligand of AKT.

In some embodiments, PBM represents a structure of Formula (PBM-29): 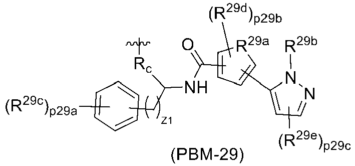
wherein R^{29a} in Formula (PBM-29) represents O, S or NH;
R^{29b} represents hydrogen, C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
Z1 represents an integer of 1, 2 or 3;
(R^{29c})ₚ₂₉ₐ indicates that the benzene ring to which it is attached is optionally substituted with p29a R^{29c} substituents, wherein each R^{29c} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p29a represents an integer of 0, 1, 2, 3, 4 or 5;
(R^{29d})_{p29b} indicates that the 5-membered ring containing R^{29a} to which it is attached is optionally substituted with p29b R^{29d} , wherein each R^{29d} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-);
p29b represents an integer of 0, 1 or 2;
(R^{29e})_{p29c} indicates that the pyrazole ring to which it is attached is optionally substituted with p29c R^{29e} , wherein each R^{29e} is identical or different and each independently represents deuterium, halogen (e.g., fluorine, chlorine, bromine, or iodine), hydroxy, amino, cyano, C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₁₋₆ alkyl (e.g., C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), or halogenated C₁₋₆ alkyl (e.g., halogenated C₁₋₄ alkyl or halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-); and
p29c represents an integer of 0, 1 or 2.

In some embodiments, PBM represents a structure of Formula (PBM-29-1) or Formula (PBM-29-2):

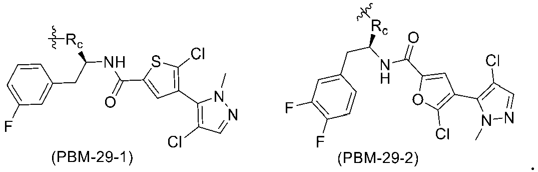

In the present disclosure, the PBM is connected to the LIN via R_{c} in its general formula.

In some embodiments, R_{c} in each of the general formulae of PBM represents a bond, -O-, - NHC(O)-*, -C(O)NH-*, -C(O)O-*, -OC(O)-*, or -NH-.

In some embodiments, R_{c} in each of the general formulae of PBM represents a structure of the following Formulae:
-N(R_{d})-, -N(R_{d})-R_{f}-N(Rₑ)-*, -R_{f}-C(O)NH-*, -R_{f}-NHC(O)-*, -R_{f}-C(O)O-*, -R_{f}-OC(O)-*, -R_{f}-, -R_{f}-N(R_{d})-*, -O-R_{f}-N(R_{d})-*, 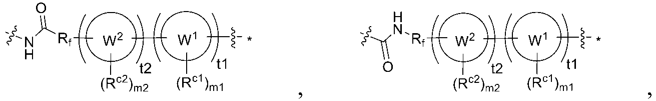 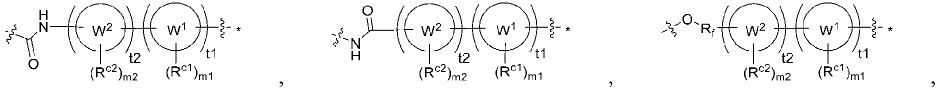 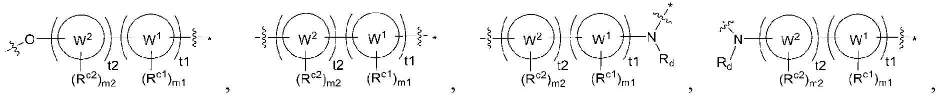 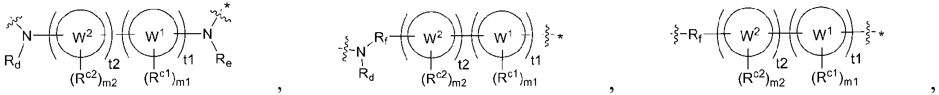 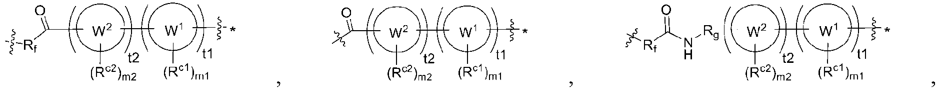 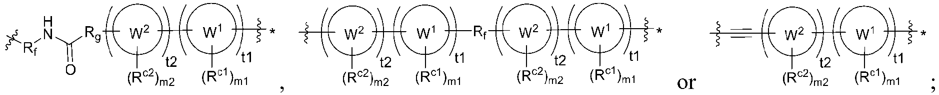
wherein each ring W¹ is identical or different and each independently represents 4- to 20-membered nitrogen-containing heterocyclylene, t1 represents an integer of 1 or 2, and (R^{c1})ₘₗ indicates that each ring W¹ is independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, or R^{c4}-R^{c3}-, wherein R^{c3} represents optionally substituted C₁₋₆alkylene or optionally substituted C₂₋₆alkenylene, and R^{c4} represents halogen, R^{c5}R^{c6}N-, hydroxy, carboxyl, ethynyl, or vinyl, wherein R^{c5} and R^{c6} are identical or different and each independently represent hydrogen or C₁₋₃ alkyl, and m1 represents an integer of 0-20;
each ring W² is identical or different and each independently represents 4- to 20-membered nitrogen-containing heterocyclylene, C₃₋₂₀cycloalkylene, C₅₋₂₀ arylene, or 5- to 20-membered heteroarylene, t2 represents an integer of 0 or 1, and (R^{c2})ₘ₂ indicates that each ring W² is independently optionally substituted with m2 R^{c2} groups, wherein each R^{c2} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, or R^{c8}-R^{c7}-, wherein R^{c7} represents optionally substituted C₁₋₆alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are identical or different and each independently represent hydrogen or C₁₋₃ alkyl, and m2 represents an integer of 0-20; and
R_{d} and Rₑ each independently represent hydrogen or C₁₋₆ alkyl; R_{f} and R_{g} each independently represent optionally substituted C₁₋₆ alkylene or optionally substituted C₂₋₆ alkenylene;
the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy; and
symbol * indicates the point of attachment to LIN.

In some embodiments, R_{d} and Rₑ each independently represent hydrogen or C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl).

In some embodiments, R_{f} and R_{g} each independently represent C₁₋₆ alkylene, and C₂₋₆ alkenylene. The C₁₋₆ alkylene and C₂₋₆ alkenylene are optionally substituted with one or more (e.g., 1-5, such as 1, 2, 3, 4, or 5) substituents selected from the group consisting of deuterium, halogen, hydroxy, mercapto, cyano, amino, nitro, C₁₋₆ alkyl, halogenated C₁₋₃ alkyl, deuterated C₁₋₆ alkyl, C₁₋₆ alkoxy or halogenated C₁₋₆ alkoxy, and any combination thereof.

In some embodiments, t1 represents an integer of 1.

In some embodiments, t1 represents an integer of 2.

In some embodiments, each ring W¹ is the same or different and each independently represents 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) nitrogen-containing heterocyclylene, e.g., 4- to 20-membered (e.g., 4- to 15-membered, 4- to 12-membered, 4-to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the nitrogen-containing heterocyclylene include, but are not limited to, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, or 3-methyl-2-oxa-8-azaspiro[4.5]decanylene. Each ring W¹ is independently optionally substituted with m1 R^{c1} groups, wherein each R^{c1} is independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{c4}-R^{c3}-, wherein R^{c3} represents optionally substituted C₁₋₆ alkylene (e.g., optionally substituted C₁₋₄alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, butenylene), and R^{c4} represents halogen (e.g., fluorine, chlorine, bromine, or iodine), R^{c5}R^{c6}N-, hydroxy, carboxyl, ethynyl, or vinyl, wherein R^{c5} and R^{c6} are identical or different and each independently represent hydrogen or C₁₋₃ alkyl (e.g., methyl, ethyl, or propyl); and the C₁₋₆ alkylene and C₂₋₆ alkenylene are optionally substituted with one or more (e.g., 1-5, such as 1, 2, 3, 4 or 5)substituents selected from the group consisting of deuterium, halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₃ alkoxy (e.g., methoxy, ethoxy, propoxy, or isopropoxy), halogenated C₁₋₃ alkoxy (e.g., halogenated C₁₋₂ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof. m1 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring W¹ is identical or different and each independently represents the following optionally substituted groups: 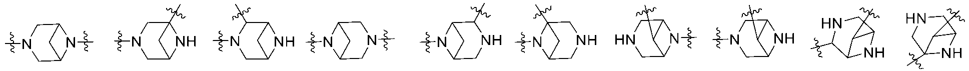 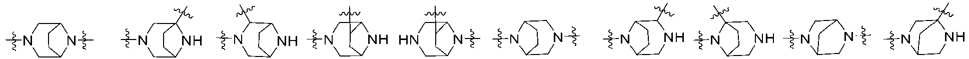 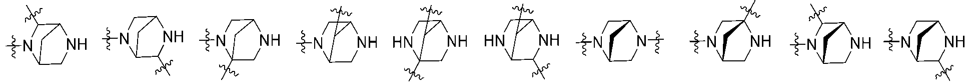 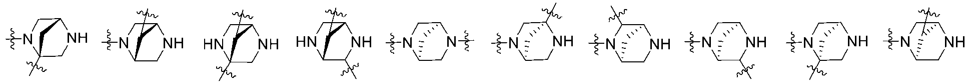 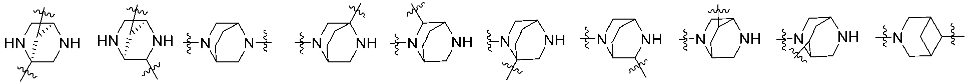 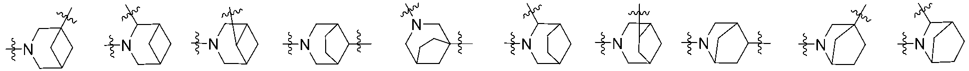 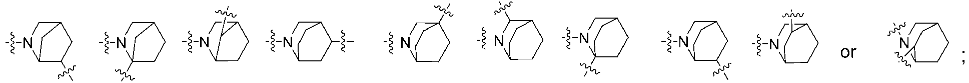 each ring W¹ is independently optionally substituted with m1 R^{c1} groups, with each R^{c1} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆cycloalkyl (e.g., optionally perdeuterated cyclopropyl, optionally perdeuterated cyclobutyl, optionally perdeuterated cyclopentyl, or optionally perdeuterated cyclohexyl), or R^{c4}-R^{c3}-, wherein R^{c3} represents optionally substituted C₁₋₆alkylene (e.g., optionally substituted C₁₋₄alkylene, such as optionally substituted methylene, ethylene, or propylene) or optionally substituted C₂₋₆alkenylene (e.g., optionally substituted C₂₋₄alkenylene, such as optionally substituted vinylene, propenylene, butenylene), and R^{c4} represents halogen (e.g., fluorine, chlorine, bromine, or iodine), R^{c5}R^{c6}N-, hydroxy, carboxyl, ethynyl, or vinyl, wherein R^{c5} and R^{c6} are identical or different and each independently represent hydrogen or C₁₋₃ alkyl (e.g., methyl, ethyl, or propyl); and the C₁₋₆ alkylene and C₂₋₆ alkenylene are optionally substituted with one or more substituents selected from the group consisting of deuterium, halogen (e.g., fluorine, chlorine, bromine, or iodine), hydroxy, mercapto, cyano, amino, nitro, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₃ alkyl (e.g., halogenated C₁₋₂ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₃ alkoxy (e.g., methoxy, ethoxy, propoxy, or isopropoxy), halogenated C₁₋₃ alkoxy (e.g., halogenated C₁₋₂ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), and any combination thereof. m1 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring W² is identical or different and each independently represents 4-to 20-membered (e.g., 4- to 15-membered , 4- to 12-membered, 4- to 11-membered, 4- to 10-membered, 4-to 9-membered, 4- to 8-membered, 4- to 7-membered, 4- to 6-membered, 5- to 15-membered, or 5- to 9-membered) nitrogen-containing heterocyclylene, C₃₋₂₀ cycloalkylene (e.g., C₃₋₁₅ cycloalkylene or C₃₋₁₁ cycloalkylene), C₅₋₂₀ arylene (e.g., C₆₋₂₀ arylene, C₅₋₁₅ arylene or C₆₋₁₅ arylene), or 5- to 20-membered (e.g., 5- to 15-membered , 5- to 10-membered, 5- to 9-membered, 5- to 8-membered, 5- to 7-membered, or 5-to 6-membered) heteroarylene. In some embodiments, the 4- to 20-membered nitrogen-containing heterocyclylene is a 4- to 20-membered (e.g., 4- to 15-membered, 4- to 12-membered, 4- to 11-membered, 4-to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the 4- to 20-membered nitrogen-containing heterocyclylene include, but are not limited to, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, or 3-methyl-2-oxa-8-azaspiro[4.5]decanylene. In some embodiments, examples of the C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene or naphthylene. In some embodiments, the 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring W² is independently optionally substituted with m2 R^{c2} groups, with each R^{c2} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆cycloalkyl (e.g., optionally perdeuterated cyclopropyl, optionally perdeuterated cyclobutyl, optionally perdeuterated cyclopentyl, or optionally perdeuterated cyclohexyl), or R^{c8}-R^{c7}-, wherein R^{c7} represents optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, butenylene), and R^{c8} represents halogen (e.g., fluorine, chlorine, bromine, or iodine), R^{c9}R^{c10}N-, hydroxy, carboxyl, ethynyl, or vinyl, wherein R^{c9} and R^{c10} are identical or different and each independently represent hydrogen or C₁₋₃ alkyl (e.g., methyl, ethyl, or propyl); and the C₁₋₆ alkylene and C₂₋₆ alkenylene are each independently optionally substituted with one or more (e.g., 1-5, such as 1, 2, 3, 4, or 5) substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy. m2 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring W² is identical or different and each independently represents : 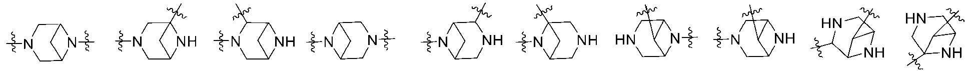 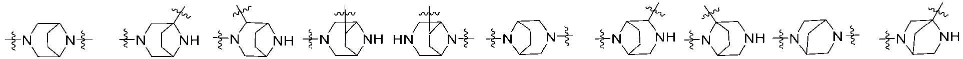 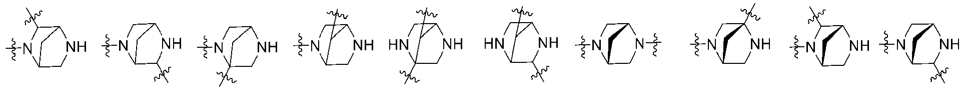 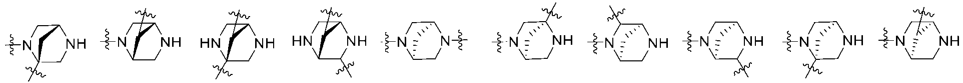 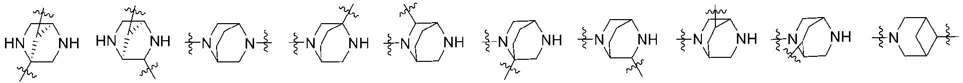 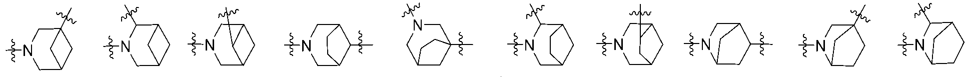 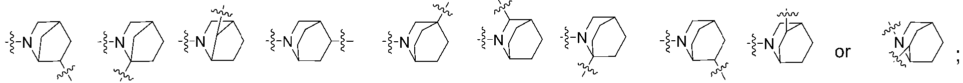 each ring W² is independently optionally substituted with m2 R^{c2} groups, with each R^{c2} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), or R^{c8}-R^{c7}-, wherein R^{c7} represents optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, butenylene), and R^{c8} represents halogen (e.g., fluorine, chlorine, bromine, or iodine), R^{c9}R^{c10}N-, hydroxy, carboxyl, ethynyl, or vinyl, wherein R^{c9} and R^{c10} are identical or different and each independently represent hydrogen or C₁₋₃ alkyl (e.g., methyl, ethyl, or propyl); and the C₁₋₆ alkylene and C₂₋₆ alkenylene are each independently optionally substituted with one or more (e.g., 1-5, such as 1, 2, 3, 4, or 5) substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy. m2 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, t2 represents an integer of 0.

In some embodiments, t2 represents an integer of 1.

In some embodiments, the moiety 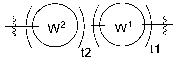 in each of the general formulae of R_{c} represents the following groups: 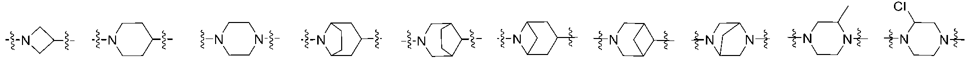 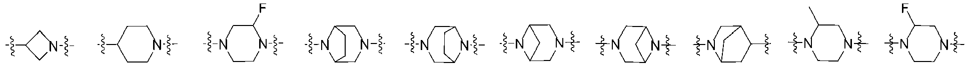 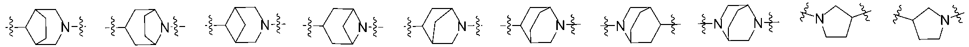 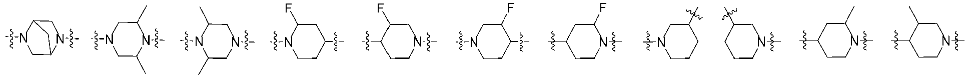 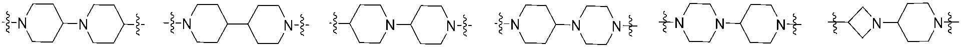 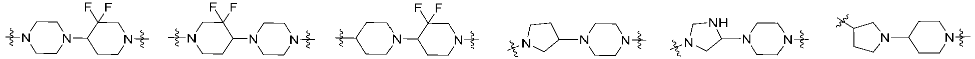 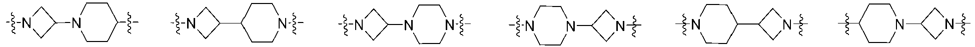 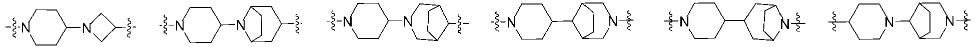 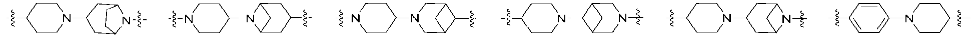 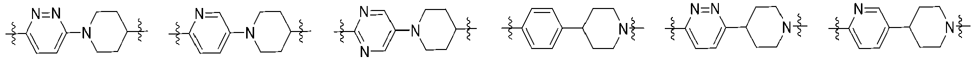 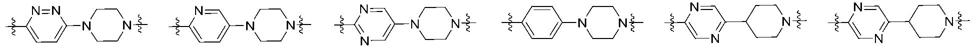 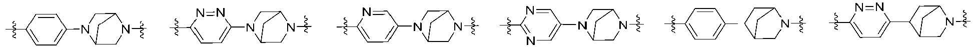 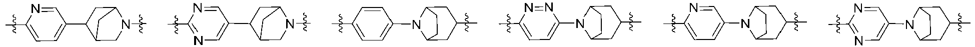 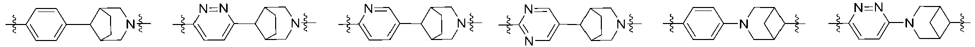 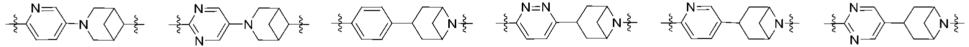 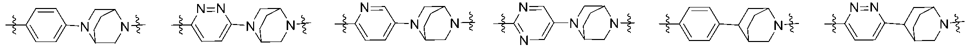 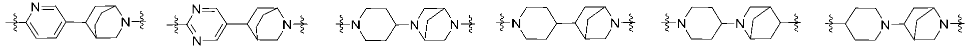 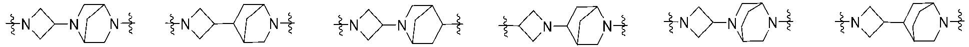 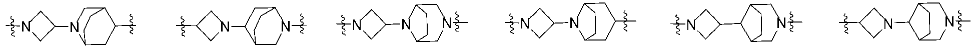 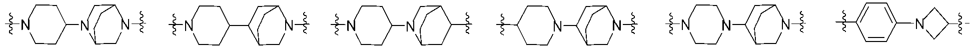 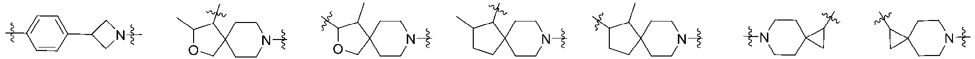 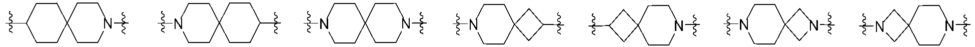 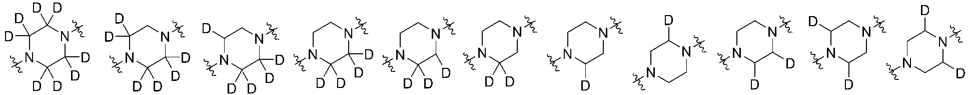 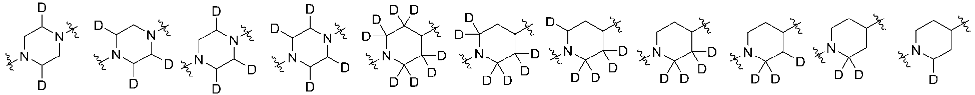 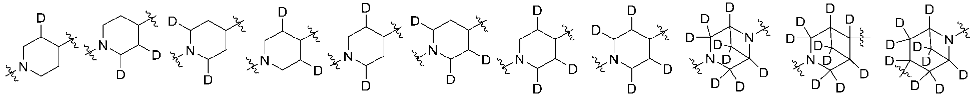 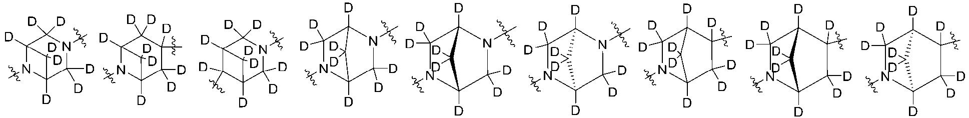 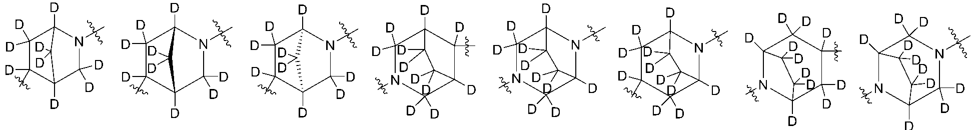 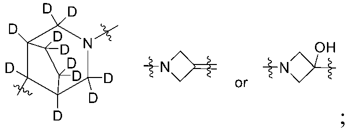 wherein the groups are each independently optionally substituted with one or more (e.g., 1-5, such as 1, 2, 3, 4, or 5) substituents selected from the group consisting of deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy (e.g., perdeuterated C₁₋₄ alkoxy, such as CD₃-O-, CD₃CD₂-O-, or CD₃CD₂CD₂-O-), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆ cycloalkyl (e.g., optionally perdeuterated cyclopropyl, optionally perdeuterated cyclobutyl, optionally perdeuterated cyclopentyl, or optionally perdeuterated cyclohexyl), R^{c12}-R^{c11}, and any combination thereof, wherein R^{c11} represents optionally substituted C₁₋₆ alkylene (e.g., optionally substituted C₁₋₄ alkylene, such as optionally substituted methylene, ethylene, or propylene) or optionally substituted C₂₋₆ alkenylene (e.g., optionally substituted C₂₋₄ alkenylene, such as optionally substituted vinylene, propenylene, butenylene), and R^{c12} represents halogen (e.g., fluorine, chlorine, bromine, or iodine), R^{c13}R^{c14}N-, hydroxy, carboxyl, ethynyl, or vinyl, wherein R^{c13} and R^{c14} are identical or different and each independently represent hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy.

In some embodiments, the R_{c} of the compound of Formula (I) represents the following groups:
a bond, -O-, -NHC(O)-*, -C(O)NH-*, -C(O)O-*, -OC(O)-*, -CH₂C(O)O-*, -CH₂OC(O)-*, -NH-, - N(CH₃)-, -N(CH₃)-(CH₂)₂-N(CH₃)-*, -N(CH₃)-(CH₂)₃-N(CH₃)-*, -NH-(CH₂)₂-N(CH₃)-*, -N(CH₃)-(CH₂)₃-NH-*, -CH₂-NHC(O)-*, -CH₂-C(O)NH-*, -CH₂-NH-*, -O-(CH₂)₂-N(CH₃)-*, -O-(CH₂)₂-NH-*, or -CH₂-N(CH₃)-*, or 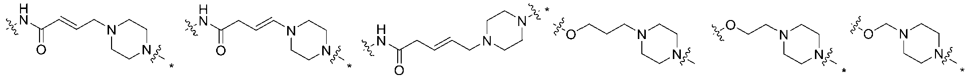 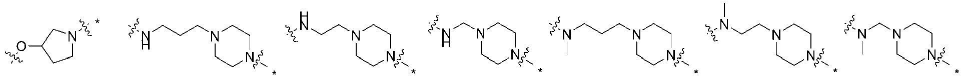 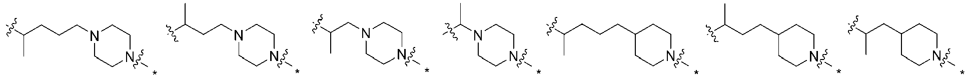 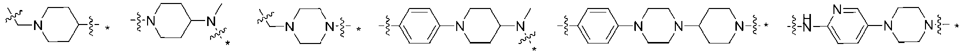 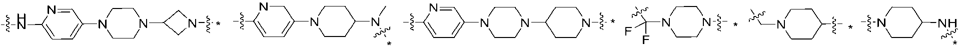 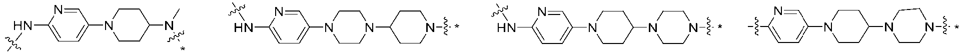 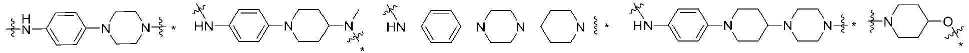 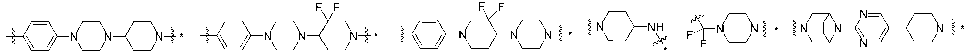 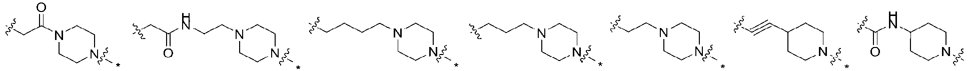 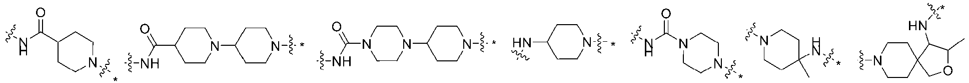 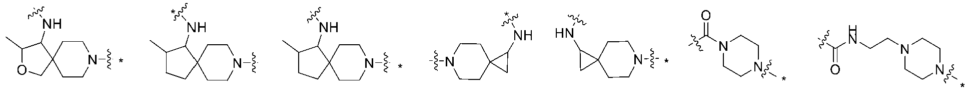 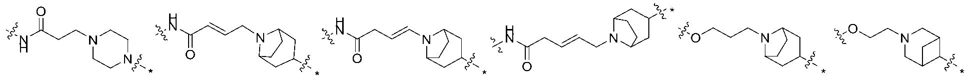 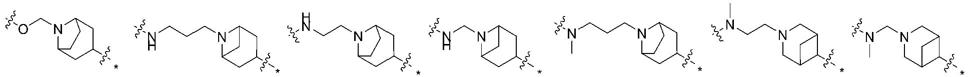 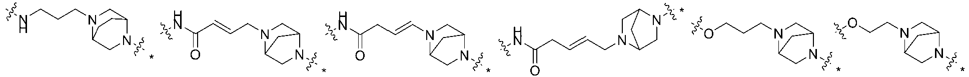 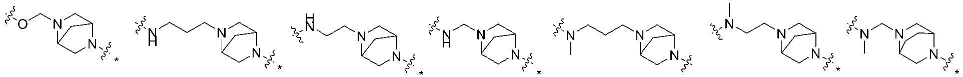 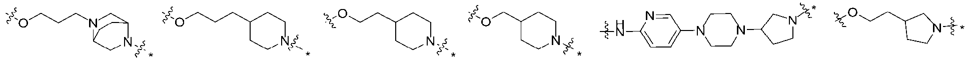 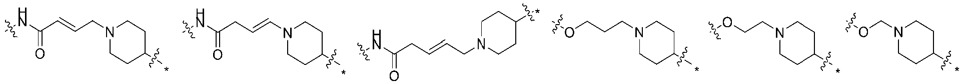 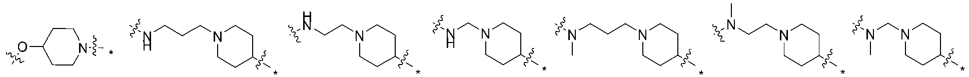 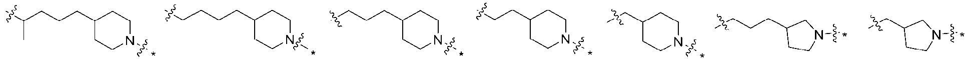 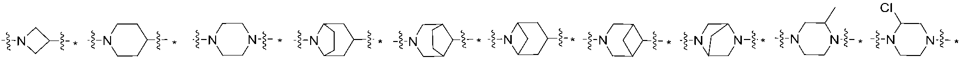 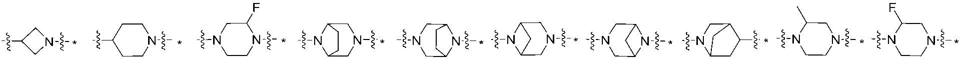 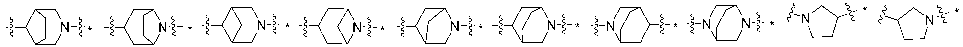 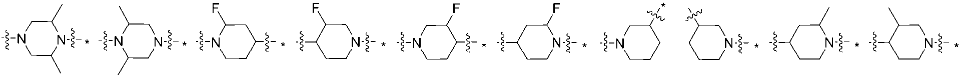 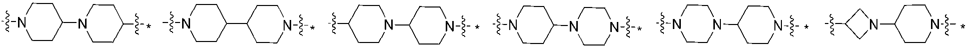 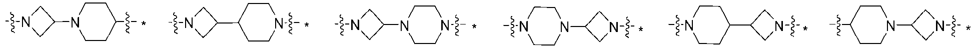 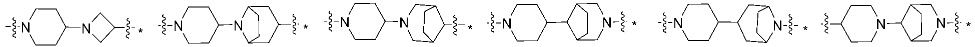 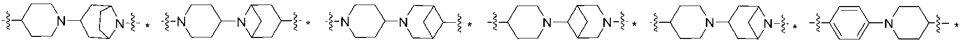 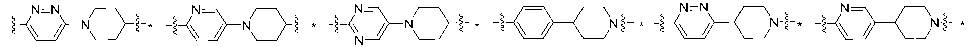 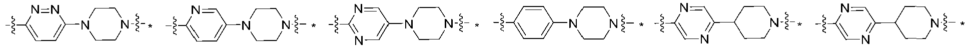 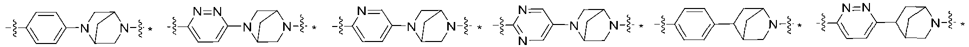 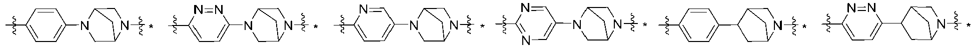 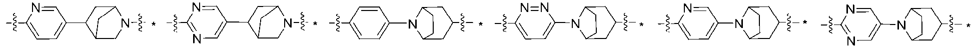 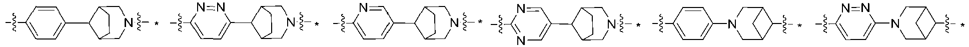 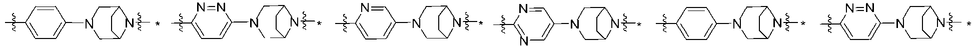 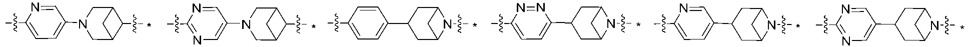 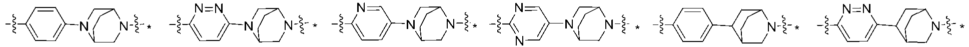 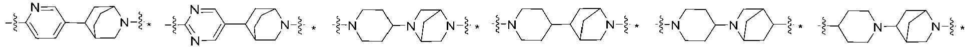 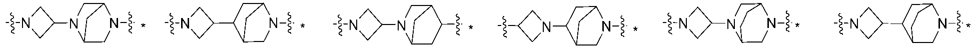 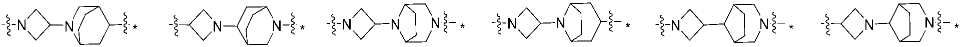 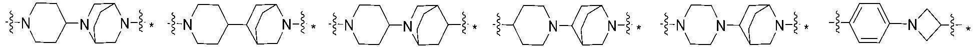 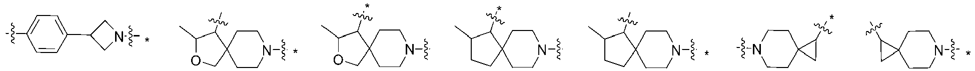 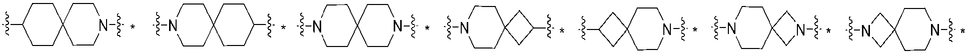 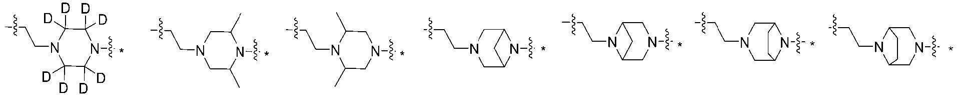 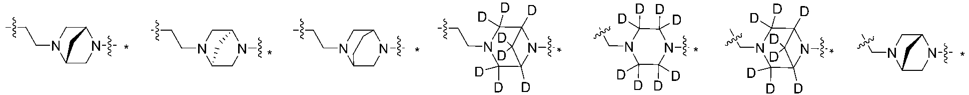 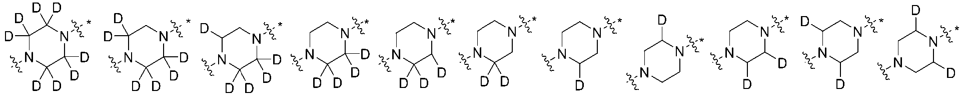 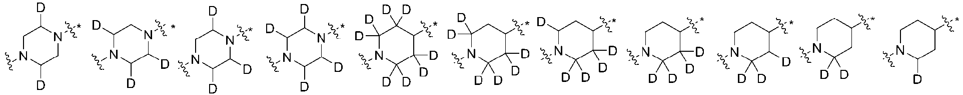 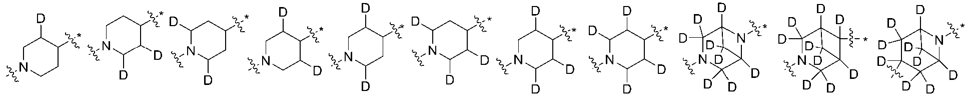 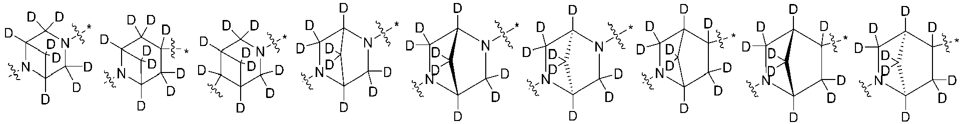 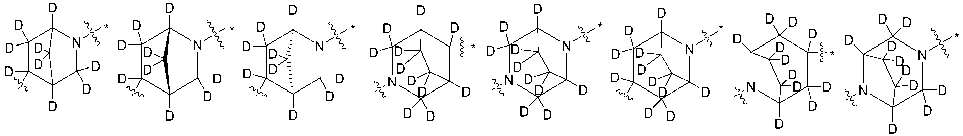 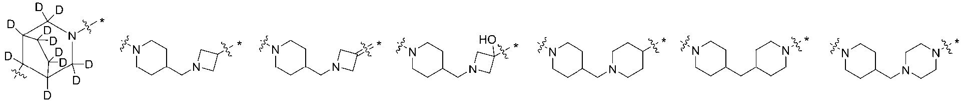 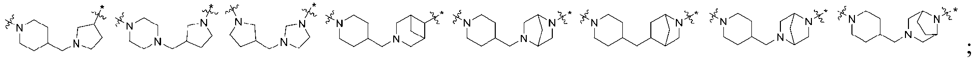 wherein the groups are each independently optionally substituted with one or more (e.g., 1-5, such as 1, 2, 3, 4, or 5) substituents selected from the group consisting of deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), halogen (e.g., fluorine, chlorine, bromine, or iodine), amino, hydroxy, mercapto, cyano, C₂₋₆ alkynyl (e.g., C₂₋₄ alkynyl, such as ethynyl, propynyl or butynyl), C₂₋₆ alkenyl (e.g., C₂₋₄ alkenyl, such as vinyl, propenyl, or butenyl), optionally deuterated C₃₋₆cycloalkyl (e.g., optionally perdeuterated cyclopropyl, optionally perdeuterated cyclobutyl, optionally perdeuterated cyclopentyl, or optionally perdeuterated cyclohexyl), R^{c12}-R^{c11}-, and any combination thereof, wherein R^{c11} represents optionally substituted C₁₋₆alkylene (e.g., optionally substituted C₁₋₄alkylene, such as optionally substituted methylene, ethylene, or propylene) or optionally substituted C₂₋₆alkenylene (e.g., optionally substituted C₂₋₄alkenylene, such as optionally substituted vinylene, propenylene, butenylene), and R^{c12} represents halogen (e.g., fluorine, chlorine, bromine, or iodine), R^{c13}R^{c14}N-, hydroxy, carboxyl, ethynyl, or vinyl, wherein R^{c13} and R^{c14} are identical or different and each independently represent hydrogen or C₁₋₃ alkyl (e.g., methyl, ethyl, or propyl); and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy; and
symbol * indicates the point of attachment to LIN.

In some embodiments, the ULM represents a ligand moiety capable of binding to an E3 ubiquitin ligase of the compound of Formula (I), and represents the structure of the following Formula (ULM): 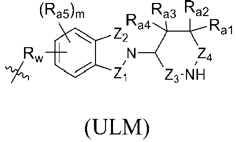
wherein Z₁ represents C(O), C(S), CH₂ or CD₂; Z₂, Z₃, and Z₄ each independently represent C(O) or C(S), wherein at least one of Z₁, Z₂, Z₃, and Z₄ represents C(S);
Rₐ₁, Rₐ₂, Rₐ₃, and Rₐ₄ are identical or different and each independently represent hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or deuterated C₁₋₆ alkoxy;
(Rₐ₅)ₘ indicates that the isoindoline ring to which it is attached is optionally substituted with m Rₐ₅, with each Rₐ₅ being identical or different and each independently representing halogen (e.g., fluorine, chlorine, bromine, or iodine), hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), deuterated C₁₋₆ alkoxy, halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₂₋₆ alkenyl, or C₂₋₆ alkynyl;
m represents an integer of 0, 1, 2 or 3;
R_{w} represents O, S, S(O), S(O)₂, alkenylene (e.g., C₂₋₆ alkenylene), alkynylene (e.g., C₂₋₆ alkynylene) or N(Rₐ₆), wherein Rₐ₆ represents H or C₁₋₃ alkyl; or R_{w} represents a bond; or
R_{w} represents: 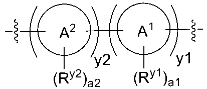
   wherein each ring A¹ identical or different and each independently represents nitrogen-containing heterocyclylene (e.g., 4- to 20-membered nitrogen-containing heterocyclylene), arylene (e.g., C₅₋₂₀arylene) or heteroarylene (e.g., 5- to 20-membered heteroarylene), y1 represents an integer of 1 or 2, and (R^{y1})ₐ₁ indicates that each ring A¹ is independently optionally substituted with a1 R^{y1} groups, with each R^{y1} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl, and a1 represents an integer of 0-20; and
   each ring A² is identical or different and each independently represents heterocyclylene (e.g., 4-to 20-membered heterocyclylene), cycloalkylene (e.g., C₃₋₂₀ cycloalkylene), arylene (e.g., C₅₋₂₀ arylene) or heteroarylene (e.g., 5- to 20-membered heteroarylene), y2 represents an integer of 0 or 1, and (R^{y2})ₐ₂ indicates that each ring A² is independently optionally substituted with a2 R^{y2} groups, with each R^{y2} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl, and a2 represents an integer of 0-20.

In some embodiments, at least one of Z₁, Z₂, Z₃, and Z₄ of the compound of Formula (I) represents C(S).

In some embodiments, at least two of Z₁, Z₂, Z₃, and Z₄ of the compound of Formula (I) represent C(S).

In some embodiments, at least three of Z₁, Z₂, Z₃, and Z₄ of the compound of Formula (I) represent C(S).

In some embodiments, each of Z₁, Z₂, Z₃, and Z₄ of the compound of Formula (I) represents C(S).

In some embodiments, Z₁ of the compound of Formula (I) represents C(S).

In some embodiments, Z₂ of the compound of Formula (I) represents C(S).

In some embodiments, Z₃ of the compound of Formula (I) represents C(S).

In some embodiments, Z₄ of the compound of Formula (I) represents C(S).

In some embodiments, Z₁ and Z₄ of the compound of Formula (I) represent C(S).

In some embodiments, Z₂ and Z₄ of the compound of Formula (I) represent C(S).

In some embodiments, Z₃ and Z₄ of the compound of Formula (I) represent C(S).

In some embodiments, Z₁ and Z₂ of the compound of Formula (I) represent C(S).

In some embodiments, Z₁, Z₃, and Z₄ of the compound of Formula (I) represent C(S).

In some embodiments, Z₂, Z₃, and Z₄ of the compound of Formula (I) represent C(S).

In some embodiments, Z₁, Z₂, and Z₃ of the compound of Formula (I) represent C(S).

In some embodiments, Z₁, Z₂, and Z₄ of the compound of Formula (I) represent C(S).

In some embodiments, for the compound of Formula (I), Z₁ represents CH₂, Z₂ represents C(S), and Z₃ and Z₄ represent C(O).

In some embodiments, for the compound of Formula (I), Z₁ represents CH₂, Z₂ represents C(S), Z₃ represents C(O), and Z₄ represents C(S).

In some embodiments, for the compound of Formula (I), Z₁ represents CH₂, Z₂ represents C(S), and Z₃ and Z₄ represent C(S).

In some embodiments, for the compound of Formula (I), Z₁ and Z₂ represent C(S), Z₃ represents C(O), and Z₄ represents C(S).

In some embodiments, Z₁, Z₂, Z₃, and Z₄ of the compound of Formula (I) represent C(S).

In some embodiments, Rₐ₁, Rₐ₂, Rₐ₃, and Rₐ₄ of the compound of Formula (I) represent hydrogen.

In some embodiments, R_{w} of the compound of Formula (I) represents a bond.

In some embodiments, R_{w} of the compound of Formula (I) represents O, S, S(O), S(O)₂, alkenylene (e.g., C₂₋₆ alkenylene), alkynylene (e.g., C₂₋₆ alkynylene) or N(Rₐ₆), wherein Rₐ₆ represents H or C₁₋₃ alkyl.

In some embodiments, R_{w} of the compound of Formula (I) represents: 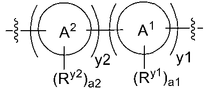 wherein each ring A¹ is identical or different and each independently represents 4- to 20-membered (e.g., 4- to 18-membered, 4- to 15-membered, 4- to 12-membered, 4- to 11-membered, 4- to 10-membered, 4-to 9-membered, 4- to 8-membered, 4- to 7-membered, 4- to 6-membered, 5- to 15-membered, or 5- to 9-membered) nitrogen-containing heterocyclylene, C₅₋₂₀ arylene (e.g., C₆₋₂₀ arylene, C₅₋₁₅ arylene or C₆₋₁₅ arylene) or 5- to 20-membered heteroarylene (e.g., 5- to 15-membered, 5- to 10-membered, 5- to 9-membered, 5- to 8-membered, 5- to 7-membered, or 5- to 6-membered heteroarylene). In some embodiments, the 4- to 20-membered nitrogen-containing heterocyclylene is a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the 4- to 20-membered nitrogen-containing heterocyclylene include, but are not limited to, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, or 3-methyl-2-oxa-8-azaspiro[4.5]decanylene. In some embodiments, examples of the 5- to 20-membered arylene include, but are not limited to, e.g., phenylene or naphthylene. In some embodiments, the 5- to 20-membered heteroarylene is a 5- to 20-membered (e.g., 5- to 15-membered, 5-to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A¹ is independently optionally substituted with a1 R^{y1} substituents, and each R^{y1} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl. a1 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, y1 of the compound of Formula (I) represents an integer of 1.

In some embodiments, y1 of the compound of Formula (I) represents an integer of 2.

In some embodiments, each ring A¹ of the compound of Formula (I) is identical or different and each independently represents the following optionally substituted groups: 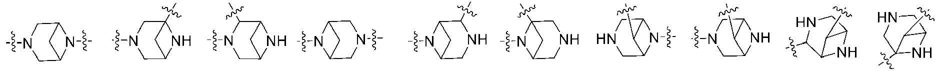 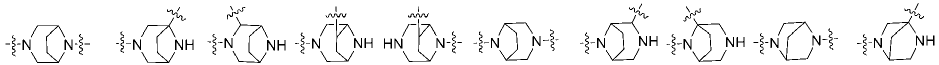 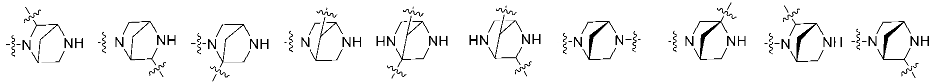 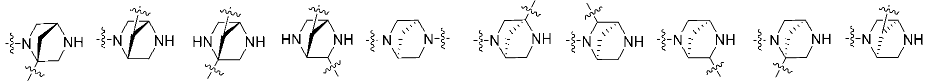 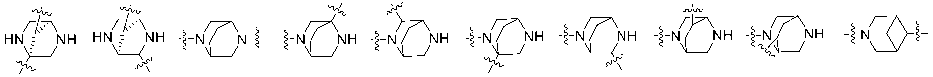 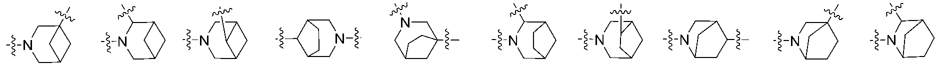 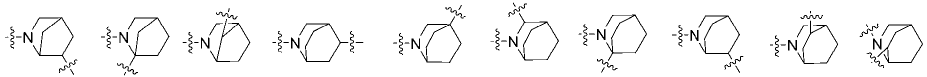 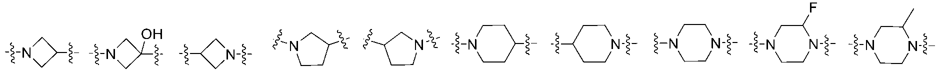 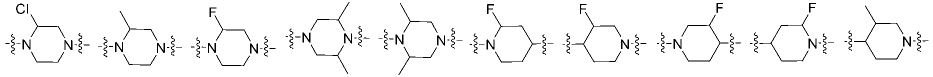 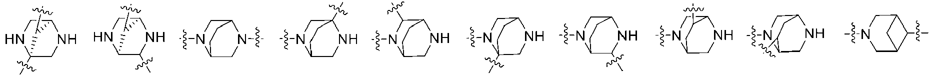 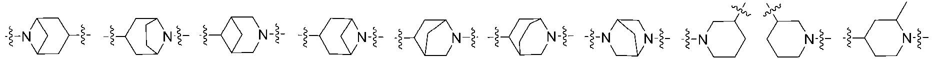 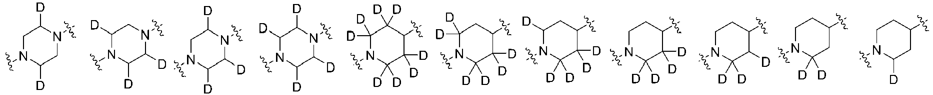 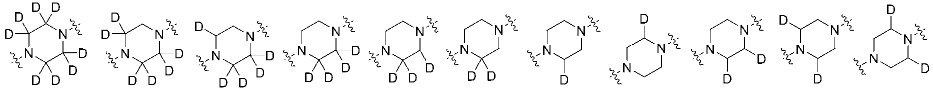 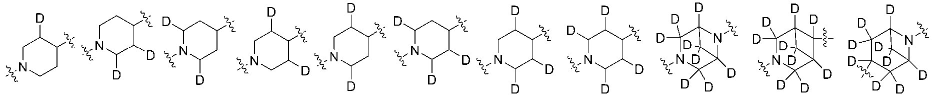 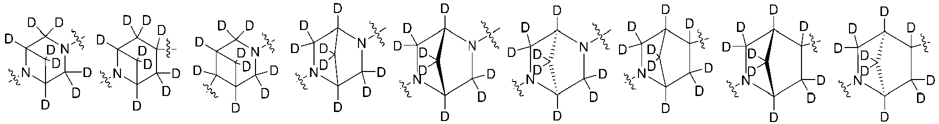 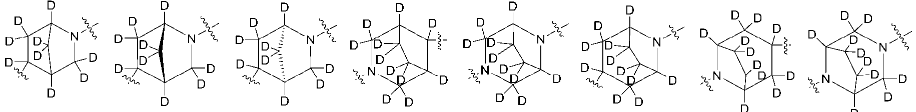 or 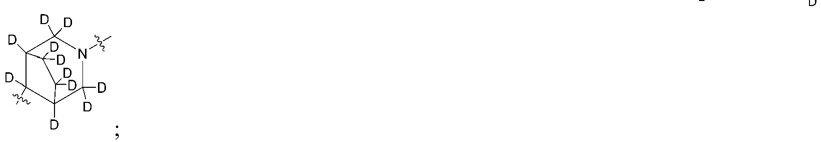 each ring A¹ is independently optionally substituted with a1 R^{y1} substituents, and each R^{y1} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl) or C₂₋₆ alkenyl (e.g., C₂₋₄ alkenyl, such as vinyl, propenyl, or butenyl). a1 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring A² of the compound of Formula (I) is identical or different and each independently represents 4- to 20-membered (e.g., 4- to 18-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene, C₃₋₂₀cycloalkylene (e.g., C₃₋₁₅cycloalkylene or C₃₋₁₁cycloalkylene), C₅₋₂₀ arylene (e.g., C₆₋₂₀arylene, C₅₋₁₅arylene or C₆₋₁₅arylene) or 5- to 20-membered heteroarylene (e.g., 5- to 15-membered, 5- to 10-membered, 5- to 9-membered, 5- to 8-membered, 5- to 7-membered, or 5- to 6-membered heteroarylene). In some embodiments, the 4- to 20-membered heterocyclylene is a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) saturated or partially unsaturated (i.e., containing one or more double bonds, but not having a fully conjugated π-electron system) divalent monocyclic, bicyclic, tricyclic or polycyclic cyclic hydrocarbon group containing one or more (e.g., from 1 to 5, from 1 to 4, or from 1 to 3, or from 1 to 2, or 1) heteroatoms selected from the group consisting of oxygen, nitrogen, and sulfur. Examples of the 4- to 20-membered heterocyclylene include, but are not limited to, monocyclic heterocyclylene (e.g., 4- to 20-membered monocyclic heterocyclylene), bridged heterocyclylene (e.g., 5- to 20-membered bridged heterocyclylene, or 7- to 15-membered bridged heterocyclylene), fused heterocyclylene, and spiro-heterocyclylene (e.g., 5-to 20-membered spiro-heterocyclylene), e.g,, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). In some embodiments, examples of the C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene or naphthylene. In some embodiments, the 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A² is independently optionally substituted with a2 R^{y2} substituents, and each R^{y2} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl. a2 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, y2 of the compound of Formula (I) represents an integer of 0.

In some embodiments, y2 of the compound of Formula (I) represents an integer of 1.

In some embodiments, each ring A² of the compound of Formula (I) is identical or different and each independently represents the following optionally substituted groups: 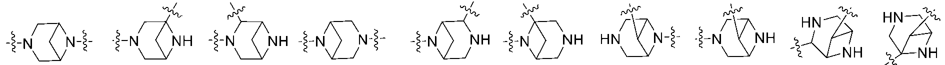 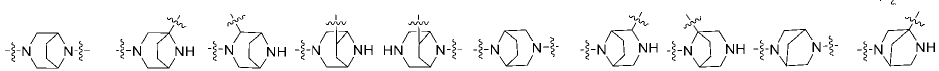 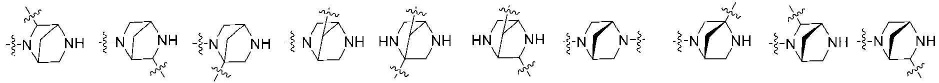 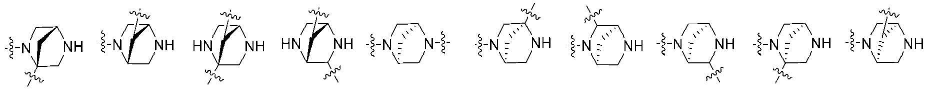 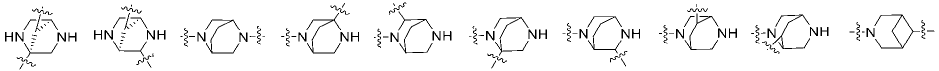 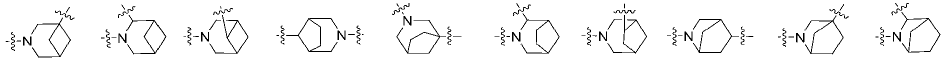 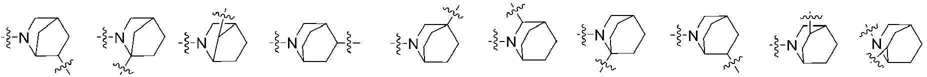 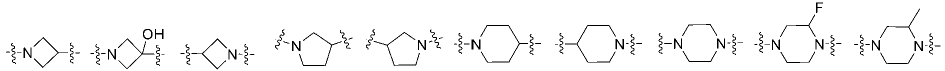 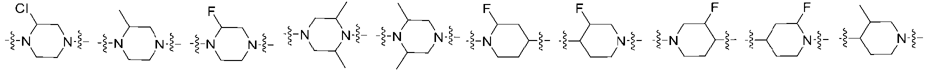 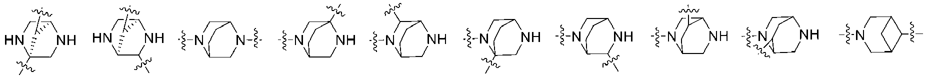 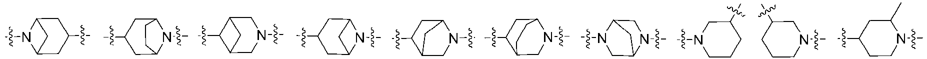 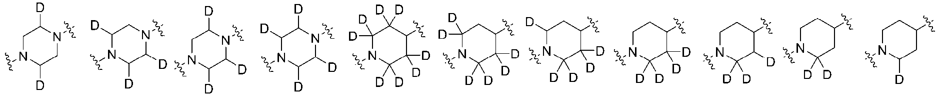 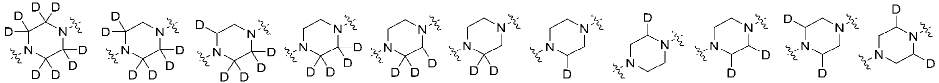 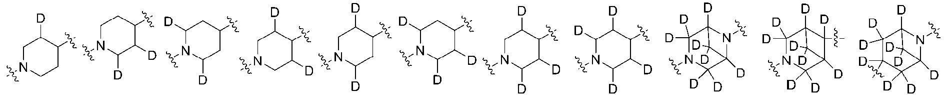 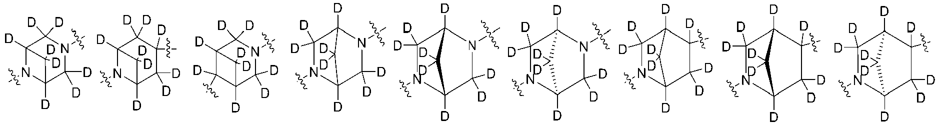 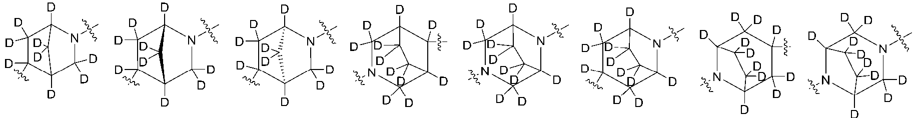 or 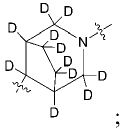 each ring A² is independently optionally substituted with a2 R^{y2} groups, wherein each R^{y2} represents independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl) or C₂₋₆ alkenyl (e.g., C₂₋₄ alkenyl, such as vinyl, propenyl, or butenyl). a2 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, R_{w} of the compound of Formula (I) represents: 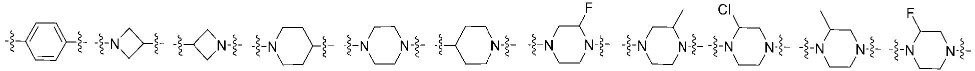 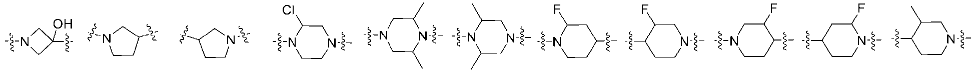 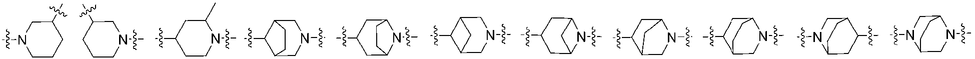 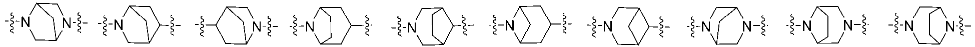 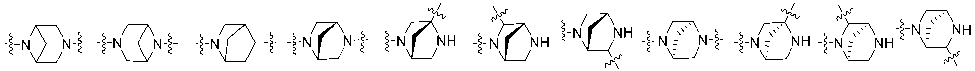 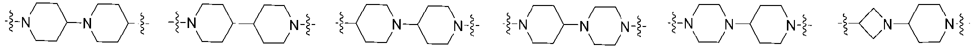 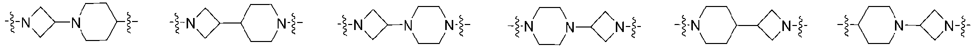 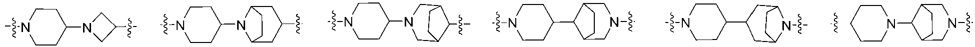 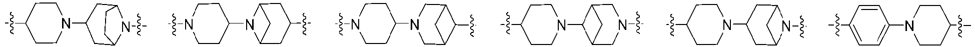 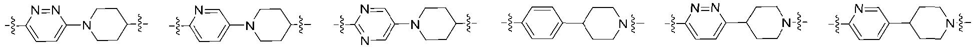 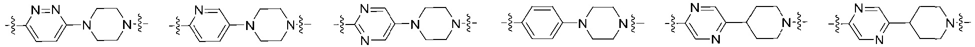 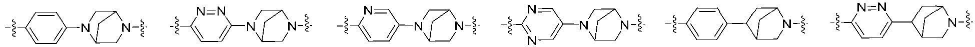 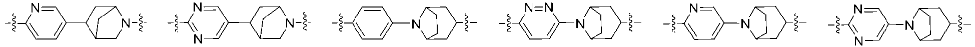 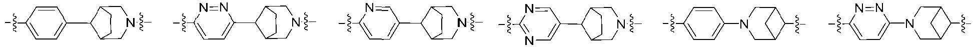 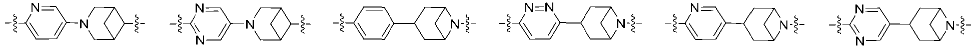 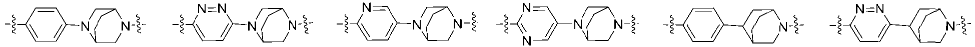 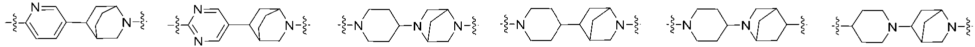 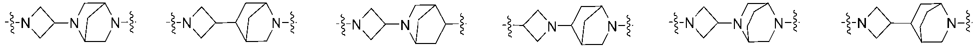 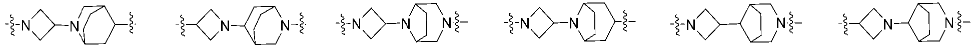 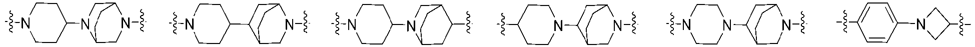 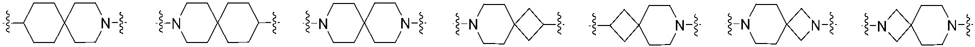 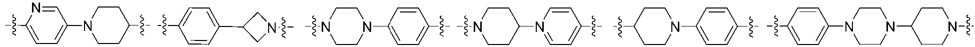 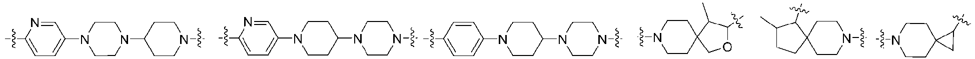 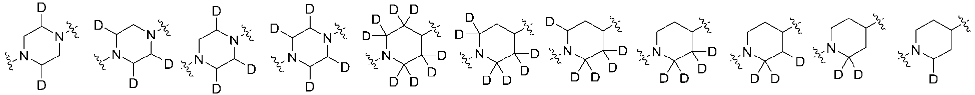 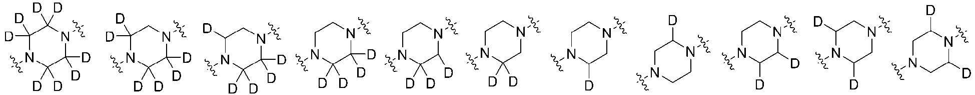 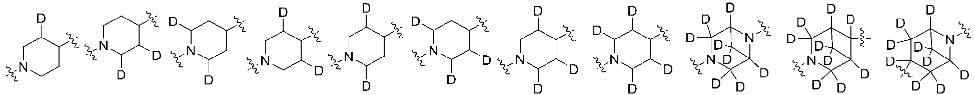 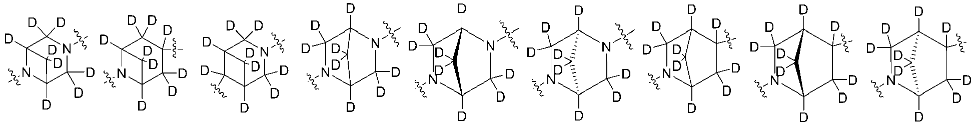 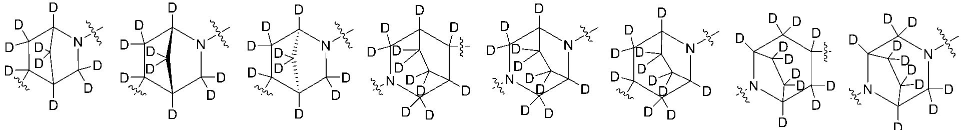 or 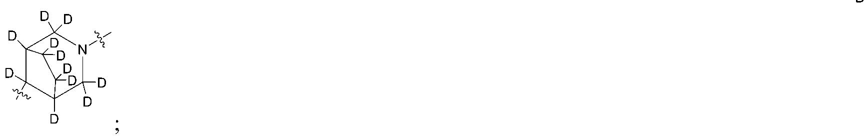 wherein the groups are optionally substituted with a substituent selected from the group consisting of deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), and C₂₋₆ alkenyl (e.g., C₂₋₄ alkenyl, such as vinyl, propenyl, or butenyl).

In some embodiments, ULM of the compound of Formula (I) represents a structure of Formula (ULM-1-1), Formula (ULM-1-2), Formula (ULM-1-3) , or Formula (ULM-1-4): 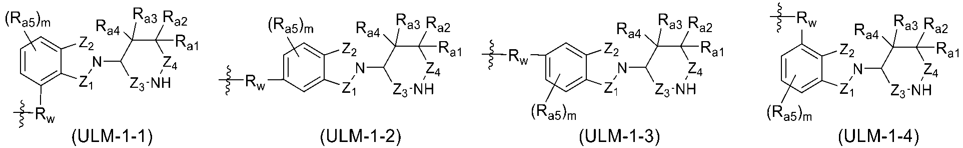
wherein Z₁ represents C(O), C(S), CH₂ or CD₂; Z₂, Z₃, and Z₄ each independently represent C(O) or C(S); wherein at least one of Z₁, Z₂, Z₃, and Z₄ represents C(S);
Rₐ₁, Rₐ₂, Rₐ₃, and Rₐ₄ are identical or different and each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy or halogenated C₁₋₆ alkoxy;
(Rₐ₅)ₘ indicates that the isoindoline ring to which it is attached is optionally substituted with m Rₐ₅, with each Rₐ₅ being identical or different and each independently representing halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2 or 3;
R_{w} represents O, S, S(O), S(O)₂, alkenylene, alkynylene or N(Rₐ₆), wherein Rₐ₆ represents H or C₁₋₃ alkyl; or
R_{w} represents a bond; or
R_{w} represents: 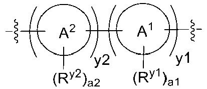
   wherein each ring A¹ is identical or different and each independently represents nitrogen-containing heterocyclylene (e.g., 4- to 20-membered nitrogen-containing heterocyclylene), arylene (e.g., C₅₋₂₀ arylene, C₆₋₁₅ arylene) or heteroarylene (e.g., 5- to 20-membered heteroarylene), y1 represents an integer of 1 or 2, and (R^{y1})ₐ₁ indicates that each ring A¹ is independently optionally substituted with a1 R^{y1} groups, with each R^{y1} being deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a1 represents an integer of 0-20 (e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units);
   each ring A² is identical or different and each independently represents heterocyclylene (e.g., 4-to 20-membered heterocyclylene), cycloalkylene (e.g., C₃₋₂₀cycloalkylene), arylene (e.g., C₅₋₂₀arylene or C₆₋₁₅arylene) or heteroarylene (e.g., 5- to 20-membered heteroarylene), y2 represents an integer of 0 or 1, and (R^{y2})ₐ₂ indicates that each ring A² is independently optionally substituted with a2 R^{y2} groups, with each R^{y2} being deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a2 represents an integer of 0-20 (e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units).

In some embodiments, ULM of the compound of Formula (I) represents a structure of the following Formulae: 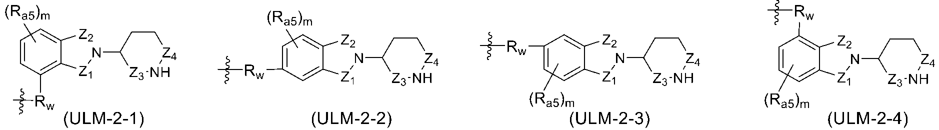 wherein Z₁, Z₂, Z₃, Z₄, (Rₐ₅)ₘ, and R_{w} are as defined herein.

In some embodiments, LIN of the compound of Formula (I) represents: 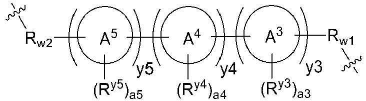
wherein R_{w2} is linked to PBM, and R_{w1} is linked to R_{w};
R_{w2} represents a bond, C(O), C(O)NH or NHC(O);
each ring A³ is identical or different and each independently represents heterocyclylene (e.g., 4- to 20-membered heterocyclylene), cycloalkylene (e.g., C₃₋₂₀ cycloalkylene), arylene (e.g., C₅₋₂₀ arylene), or heteroarylene (e.g., 5- to 20-membered heteroarylene), y3 represents an integer of 0, 1, 2 or 3, and (R^{y3})ₐ₃ indicates that each ring A³ is independently optionally substituted with a3 R^{y3} substituents, wherein each R^{y3} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a3 represents an integer of 0-10;
each ring A⁴ is identical or different and each independently represents heterocyclylene (e.g., 4- to 20-membered heterocyclylene), cycloalkylene (e.g., C₃₋₂₀ cycloalkylene), arylene (e.g., C₅₋₂₀ arylene), or heteroarylene (e.g., 5- to 20-membered heteroarylene), y4 represents an integer of 0, 1, 2 or 3, and (R^{y4})ₐ₄ indicates that each ring A⁴ is independently optionally substituted with a4 R^{y4} substituents, wherein each R^{y4} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a4 represents an integer of 0-10;
each ring A⁵ is identical or different and each independently represents heterocyclylene (e.g., 4- to 20-membered heterocyclylene), cycloalkylene (e.g., C₃₋₂₀cycloalkylene), arylene (e.g., C₅₋₂₀ arylene), or heteroarylene (e.g., 5- to 20-membered heteroarylene), y5 represents an integer of 0, 1, 2 or 3, and (R^{y5})ₐ₅ indicates that each ring A⁵ is independently optionally substituted with a5 R^{y5} substituents, wherein each R^{y5} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a5 represents an integer of 0-10;
R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof.

In some embodiments, R_{w2} of the compound of Formula (I) represents a bond.

In some embodiments, R_{w2} of the compound of Formula (I) represents C(O).

In some embodiments, R_{w2} of the compound of Formula (I) represents C(O)NH or NHC(O).

In some embodiments, each ring A³ of the compound of Formula (I) is identical or different and each independently represents heterocyclylene (e.g., 4- to 20-membered heterocyclylene, such as 4- to 18-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-memberedheterocyclylene), cycloalkylene (e.g., C₃₋₂₀cycloalkylene), arylene (e.g., C₅₋₂₀arylene, such as C₆₋₂₀arylene, C₅₋₁₅arylene or C₆₋₁₅arylene), or heteroarylene (e.g., 5- to 20-membered heteroarylene, such as 5- to 15-membered , 5- to 10-membered, 5- to 9-membered, 5- to 8-membered, 5- to 7-membered, or 5- to 6-membered heteroarylene). In some embodiments, 4- to 20-membered heterocyclylene is a 4- to 20-membered (e.g., 4- to 15-membered, 4- to 12-membered, 4- to 11-membered, 4- to 10-membered, 4-to 9-membered, 4- to 8-membered, 4- to 7-membered, 4- to 6-membered, 5- to 15-membered, or 5- to 9-membered) saturated or partially unsaturated (i.e., containing one or more double bonds, but not having a fully conjugated π-electron system) divalent monocyclic, bicyclic, tricyclic or polycyclic cyclic hydrocarbon group containing one or more (e.g., from 1 to 5, from 1 to 4, or from 1 to 3, or from 1 to 2, or 1) heteroatoms selected from the group consisting of oxygen, nitrogen, and sulfur. Examples of the 4-to 20-membered heterocyclylene include, but are not limited to, monocyclic heterocyclylene (e.g., 4- to 20-membered monocyclic heterocyclylene), bridged heterocyclylene (e.g., 5- to 20-membered bridged heterocyclylene, or 7- to 15-membered bridged heterocyclylene), fused heterocyclylene, and spiro-heterocyclylene (e.g., 5- to 20-membered spiro-heterocyclylene), e.g., azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). In some embodiments, examples of the C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene or naphthylene. In some embodiments, 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A³ is independently optionally substituted with a3 R^{y3} substituents, and each R^{y3} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl. a3 represents an integer of 0-10, e.g., an integer of 0, 1, 2, 3, 4, 5, 6, 7, 8, 9 or 10. The number of substituents is not theoretically limited in any way, or is automatically limited by the size of the building units.

In some embodiments, y3 of the compound of Formula (I) represents an integer of 0, 1, 2 or 3.

In some embodiments, each ring A³ of the compound of Formula (I) is identical or different and each independently represents the following optionally substituted groups: 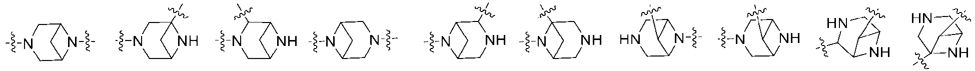 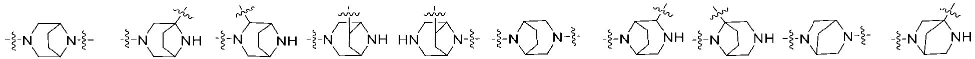 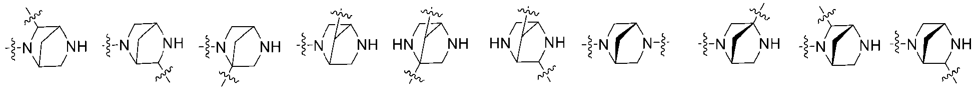 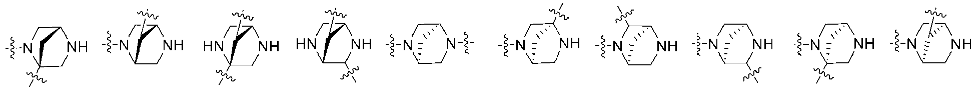 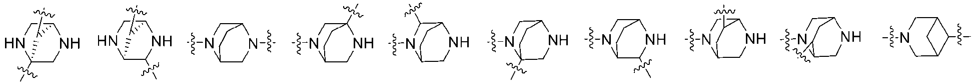 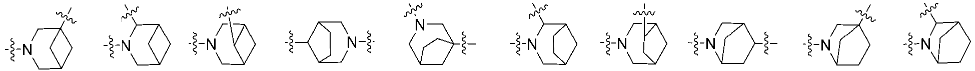 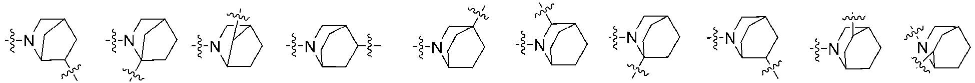 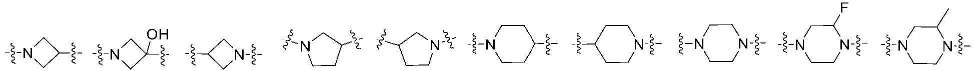 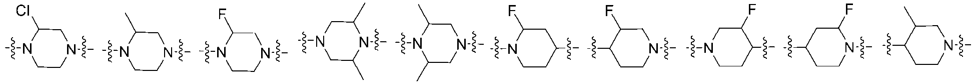 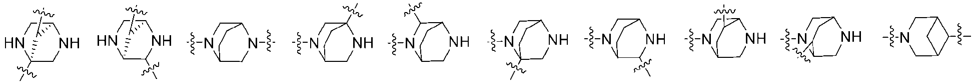 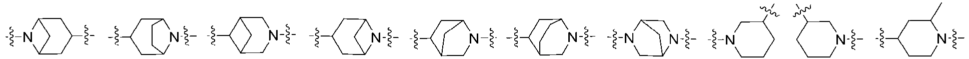 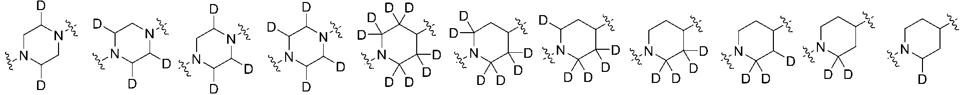 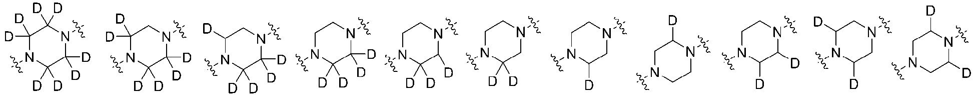 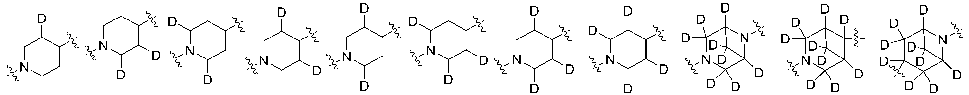 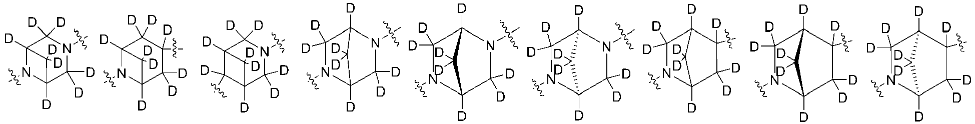 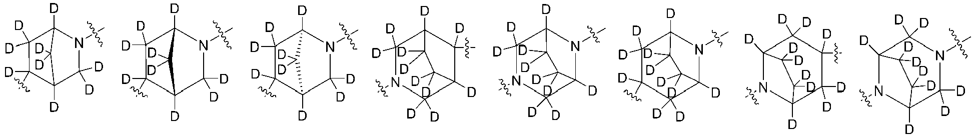 or 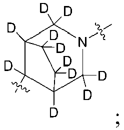 each ring A³ is independently optionally substituted with a3 R^{y3} substituents, and each R^{y3} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl) or C₂₋₆ alkenyl (e.g., C₂₋₄ alkenyl, such as vinyl, propenyl, or butenyl). a3 represents an integer of 0-10, e.g., an integer of 0, 1, 2, 3, 4, 5, 6, 7, 8, 9 or 10. The number of substituents is not theoretically limited in any way, or is automatically limited by the size of the building units.

In some embodiments, each ring A⁴ of the compound of Formula (I) is identical or different and each independently represents heterocyclylene (e.g., 4- to 20-membered heterocyclylene, such as 4- to 18-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-memberedheterocyclylene), cycloalkylene (e.g., C₃₋₂₀cycloalkylene), arylene (e.g., C₅₋₂₀arylene, such as C₆₋₂₀arylene, C₅₋₁₅arylene or C₆₋₁₅arylene), or heteroarylene (e.g., 5- to 20-membered heteroarylene, such as 5- to 15-membered , 5- to 10-membered, 5- to 9-membered, 5- to 8-membered, 5- to 7-membered, or 5- to 6-membered heteroarylene). In some embodiments, the 4- to 20-membered heterocyclylene is a 4- to 20-membered (e.g., 4- to 15-membered, 4- to 12-membered, 4- to 11-membered, 4- to 10-membered, 4-to 9-membered, 4- to 8-membered, 4- to 7-membered, 4- to 6-membered, 5- to 15-membered, or 5- to 9-membered) saturated or partially unsaturated (i.e., containing one or more double bonds, but not having a fully conjugated π-electron system) divalent monocyclic, bicyclic, tricyclic or polycyclic cyclic hydrocarbon group containing one or more (e.g., from 1 to 5, from 1 to 4, or from 1 to 3, or from 1 to 2, or 1) heteroatoms selected from the group consisting of oxygen, nitrogen, and sulfur. Examples of the 4-to 20-membered heterocyclylene include, but are not limited to, monocyclic heterocyclylene (e.g., 4- to 20-membered monocyclic heterocyclylene), bridged heterocyclylene (e.g., 5- to 20-membered bridged heterocyclylene, or 7- to 15-membered bridged heterocyclylene), fused heterocyclylene, and spiro-heterocyclylene (e.g., 5- to 20-membered spiro-heterocyclylene), e.g., azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). In some embodiments, examples of the C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene or naphthylene. In some embodiments, the 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A⁴ is independently optionally substituted with a4 R^{y4} substituents, and each R^{y4} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl. a4 represents an integer of 0-10, e.g., an integer of 0, 1, 2, 3, 4, 5, 6, 7, 8, 9 or 10. The number of substituents is not theoretically limited in any way, or is automatically limited by the size of the building units.

In some embodiments, y4 of the compound of Formula (I) represents an integer of 0, 1, 2 or 3.

In some embodiments, ach ring A⁴ of the compound of Formula (I) is identical or different and each independently represents the following optionally substituted groups: 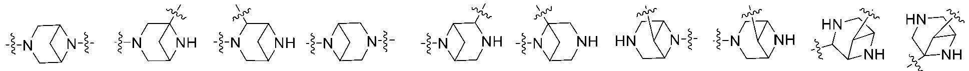 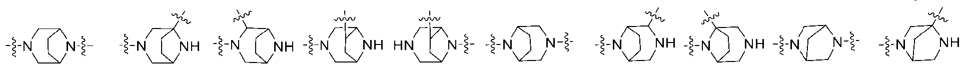 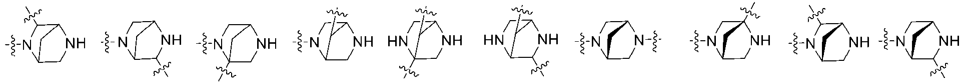 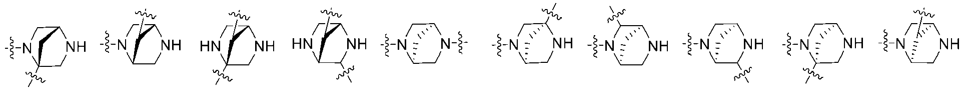 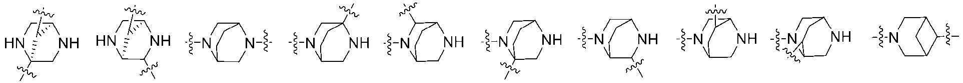 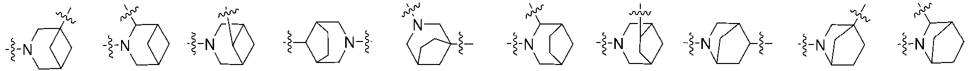 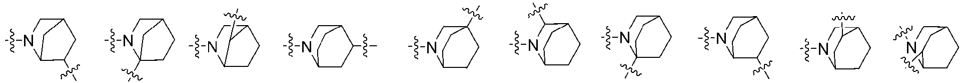 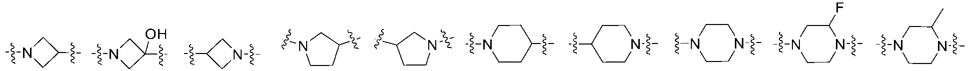 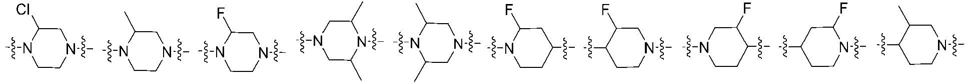 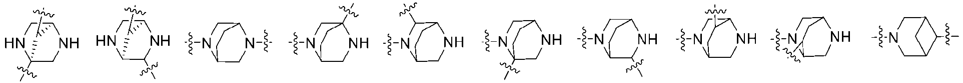 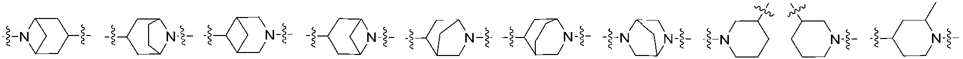 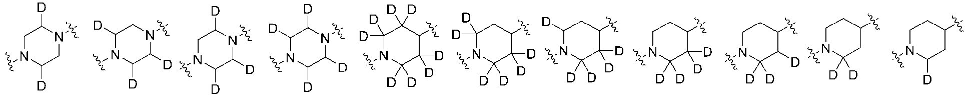 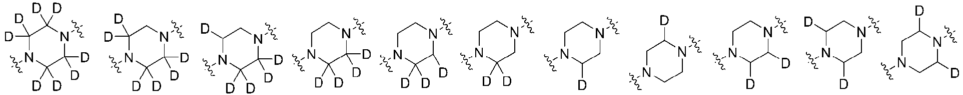 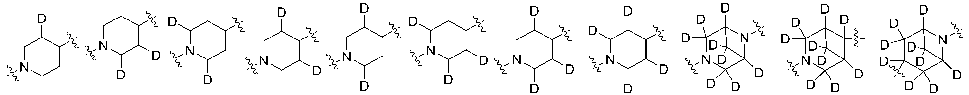 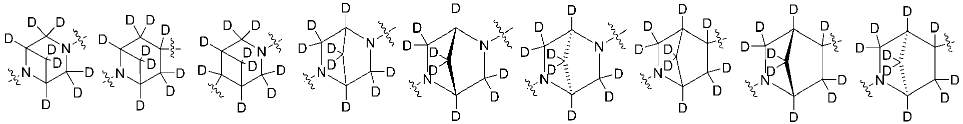 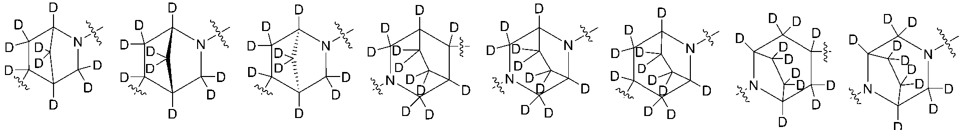 or 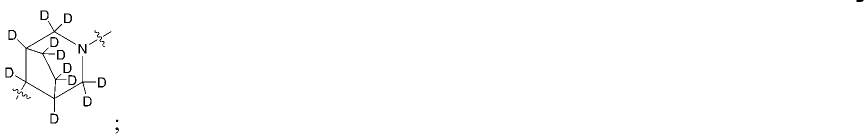 each ring A⁴ is independently optionally substituted with a4 R^{y4} groups, wherein each R^{y4} represents independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl) or C₂₋₆ alkenyl (e.g., C₂₋₄ alkenyl, such as vinyl, propenyl, or butenyl). a4 represents an integer of 0-10, e.g., an integer of 0, 1, 2, 3, 4, 5, 6, 7, 8, 9 or 10. The number of substituents is not theoretically limited in any way, or is automatically limited by the size of the building units.

In some embodiments, each ring A⁵ of the compound of Formula (I) is identical or different and each independently represents heterocyclylene (e.g., 4- to 20-membered heterocyclylene, such as 4- to 18-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-memberedheterocyclylene), cycloalkylene (e.g., C₃₋₂₀cycloalkylene), arylene (e.g., C₅₋₂₀arylene, such as C₆₋₂₀arylene, C₅₋₁₅arylene or C₆₋₁₅arylene), or heteroarylene (e.g., 5- to 20-membered heteroarylene, such as 5- to 15-membered , 5- to 10-membered, 5- to 9-membered, 5- to 8-membered, 5- to 7-membered, or 5- to 6-membered heteroarylene). In some embodiments, the 4- to 20-membered heterocyclylene is a 4- to 20-membered (e.g., 4- to 15-membered, 4- to 12-membered, 4- to 11-membered, 4- to 10-membered, 4-to 9-membered, 4- to 8-membered, 4- to 7-membered, 4- to 6-membered, 5- to 15-membered, or 5- to 9-membered) saturated or partially unsaturated (i.e., containing one or more double bonds, but not having a fully conjugated π-electron system) divalent monocyclic, bicyclic, tricyclic or polycyclic cyclic hydrocarbon group containing one or more (e.g., from 1 to 5, from 1 to 4, or from 1 to 3, or from 1 to 2, or 1) heteroatoms selected from the group consisting of oxygen, nitrogen, and sulfur. Examples of the 4-to 20-membered heterocyclylene include, but are not limited to, monocyclic heterocyclylene (e.g., 4- to 20-membered monocyclic heterocyclylene), bridged heterocyclylene (e.g., 5- to 20-membered bridged heterocyclylene, or 7- to 15-membered bridged heterocyclylene), fused heterocyclylene, and spiro-heterocyclylene (e.g., 5- to 20-membered spiro-heterocyclylene), e.g., azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). In some embodiments, examples of the C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene or naphthylene. In some embodiments, the 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A⁵ is independently optionally substituted with a5 R^{y5} substituents, and each R^{y5} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl. a5 represents an integer of 0-10, e.g., an integer of 0, 1, 2, 3, 4, 5, 6, 7, 8, 9 or 10. The number of substituents is not theoretically limited in any way, or is automatically limited by the size of the building units.

In some embodiments, y5 of the compound of Formula (I) represents an integer of 0, 1, 2 or 3.

In some embodiments, each ring A⁵ of the compound of Formula (I) is identical or different and each independently represents the following optionally substituted groups: 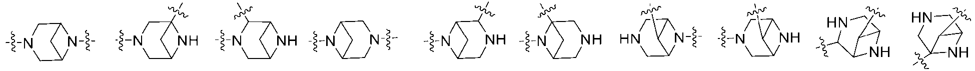 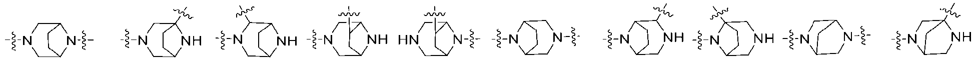 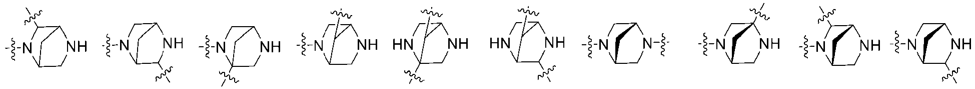 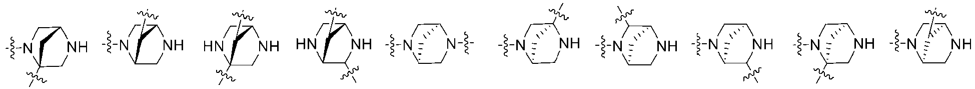 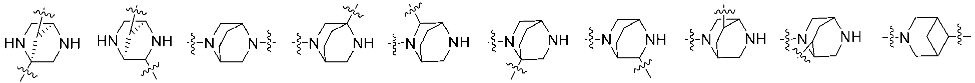 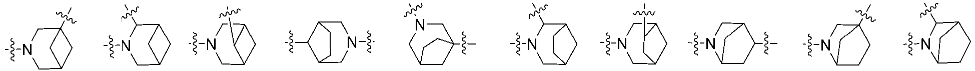 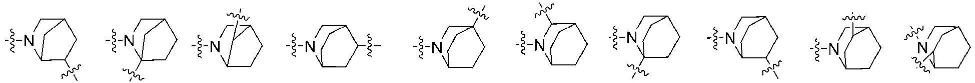 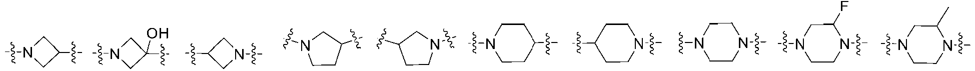 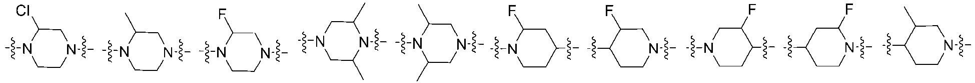 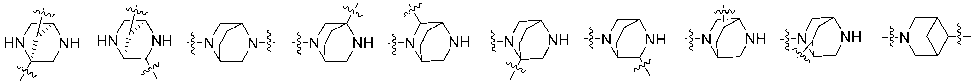 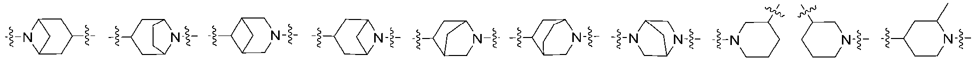 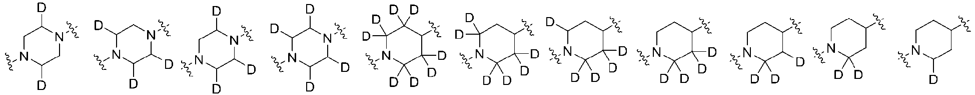 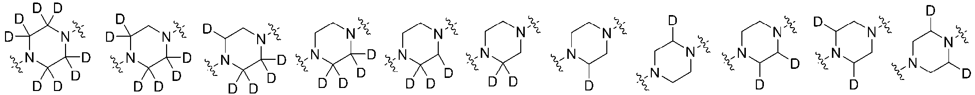 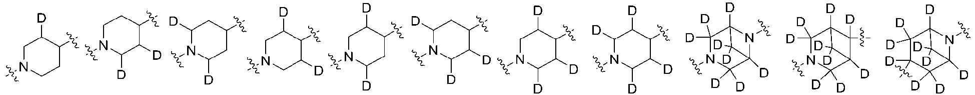 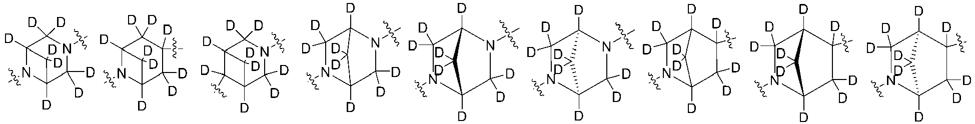 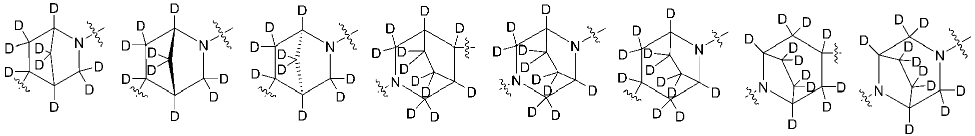 or 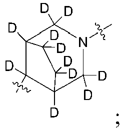 each ring A⁵ is independently optionally substituted with a5 R^{y5} groups, wherein each R^{y5} represents independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl) or C₂₋₆ alkenyl (e.g., C₂₋₄ alkenyl, such as vinyl, propenyl, or butenyl). a5 represents an integer of 0-10, e.g., an integer of 0, 1, 2, 3, 4, 5, 6, 7, 8, 9 or 10. The number of substituents is not theoretically limited in any way, or is automatically limited by the size of the building units.

In some embodiments, examples of the moiety 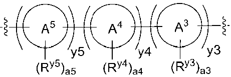 in each of the general formulae of the LIN of the compound of Formula (I) include, but are not limited to, the following groups: 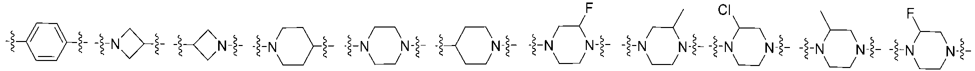 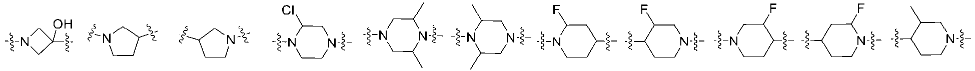 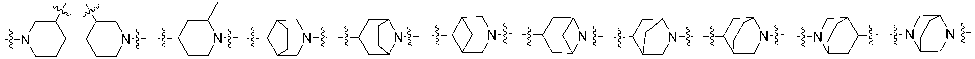 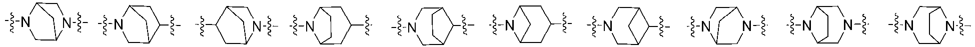 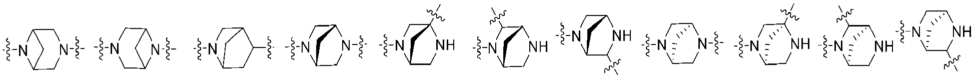 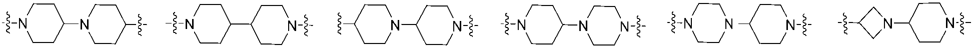 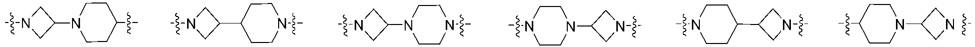 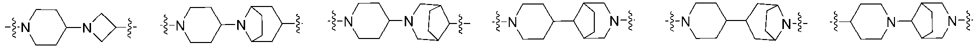 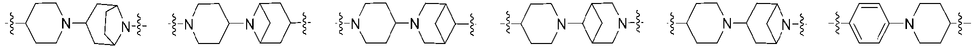 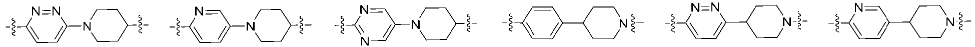 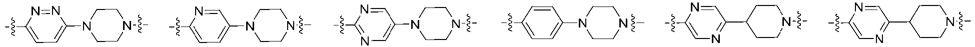 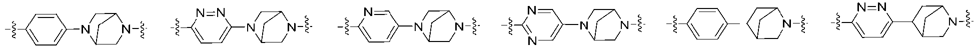 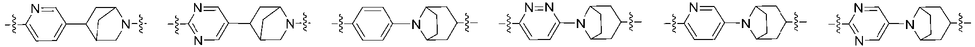 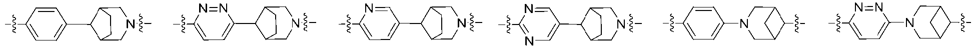 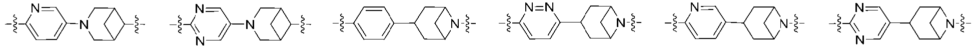 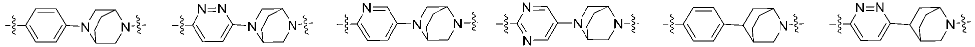 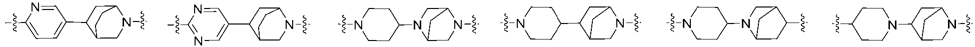 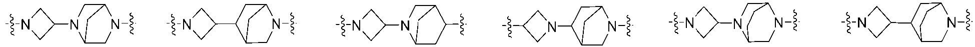 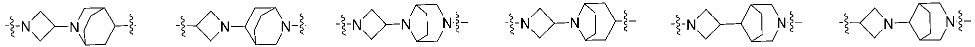 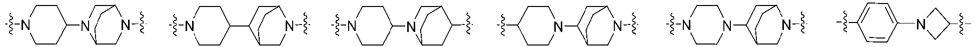 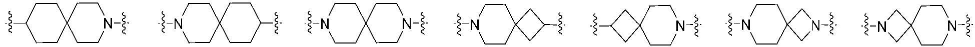 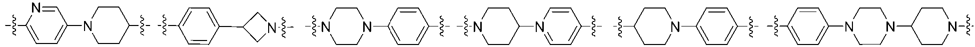 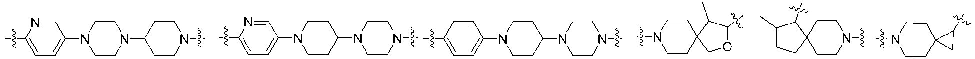 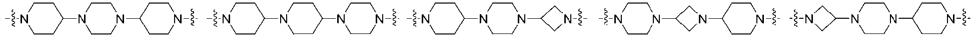 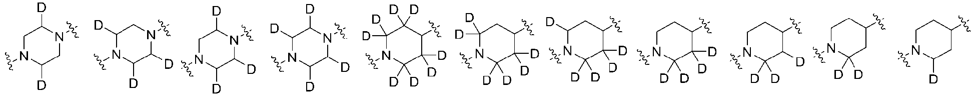 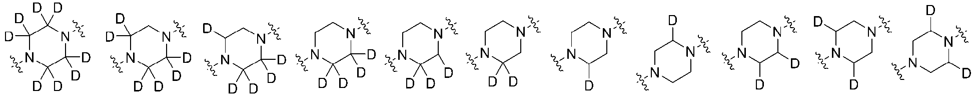 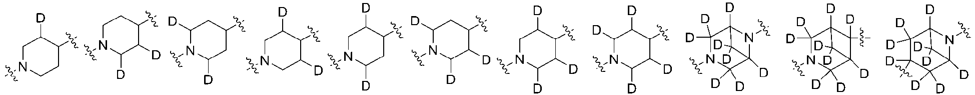 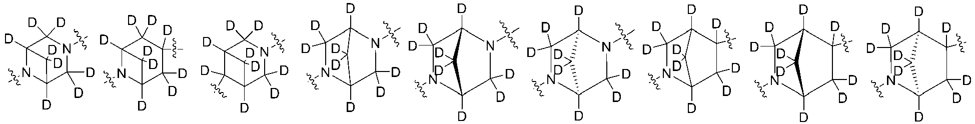 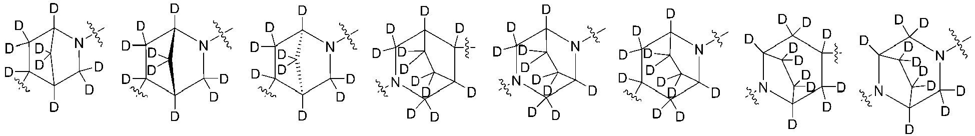 or 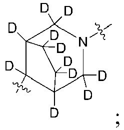 wherein the groups are optionally substituted with a substituent selected from the group consisting of deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen, amino, hydroxy, mercapto, cyano, carboxyl, C₂₋₆ alkynyl, and C₂₋₆ alkenyl. In the present disclosure, unless otherwise specified, any one end of the general formulas provided above may be connected to R_{w1} of the compound of Formula (I), while the other end is connected to R_{w2}.

In some embodiments, R_{w1} of the compound of Formula (I) represents a bond.

In some embodiments, R_{w1} of the compound of Formula (I) represents optionally substituted linear or branched C₁₋₂₀ alkylene, wherein any one or more (e.g., 1-15, 1-10, 1-9, 1-8, 1-7, 1-6, 1-5, 1-4, 1-3, or 1-2, or 1) methylene groups in the main carbon chain skeleton of the linear or branched C₁₋₂₀ alkylene are optionally substituted with one or more (e.g., 1-15, 1-10, 1-9, 1-8, 1-7, 1-6, 1-5, 1-4, 1-3, or 1-2, or 1) groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of optionally substituted cycloalkylene (e.g., optionally substituted C₃₋₂₀cycloalkylene), optionally substituted arylene (e.g., optionally substituted C₅₋₂₀arylene), optionally substituted heterocyclylene (e.g., optionally substituted 4-to 20-membered heterocyclylene), optionally substituted heteroarylene (e.g., optionally substituted 5- to 20-membered heteroarylene), alkynylene (e.g., C₂₋₆alkynylene), alkenylene (e.g., C₂₋₆alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆cycloalkyl, halogenated C₃₋₆cycloalkyl, deuterated C₃₋₆cycloalkyl, 4- to 10-membered heterocyclyl, and any combination thereof, and wherein one or more hydrogens of said linear or branched C₁₋₂₀ alkylene are optionally replaced by a substituent selected from: deuterium, hydroxy, amino, mercapto, nitro, halogen, cyano, deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆cycloalkyl, halogenated C₃₋₆cycloalkyl, deuterated C₃₋₆cycloalkyl, 4- to 10-membered heterocyclyl, and any combination thereof.

In some embodiments, R_{w1} of the compound of Formula (I) represents a structure of the following Formulae:
-C₁₋₁₅alkylene-;
-(C(Rₐ₈)(Rₐ₉))ₙ₁-(Rₐ(C(Ra₁₀)(Rₐ₁₁)ₙ₂)ₘ₃-;
-(C(Rₐ₈)(Rₐ₉))ₙ₁-(Rₐ(C(Rₐ₁₀)(Rₐ₁₁))ₙ₂)ₘ₃-(Rₐ(C(Rₐ₁₂)(Rₐ₁₃))ₙ₃)ₘ₄₋;
-(C(Rₐ₈)(Rₐ₉))ₙ₁-(Rₐ(C(Rₐ₁₀)(Rₐ₁₁))ₙ₂)ₘ₃-(Rₐ(C(Rₐ₁₂)(Rₐ₁₃))ₙ₃)ₘ₄-(Rₐ(C(Rₐ₁₄)(Rₐ₁₅))ₙ₄)ₘ₅-;
-(C(Rₐ₈)(Rₐ₉))ₙ₁-((C(Rₐ₁₀)(Rₐ₁₁))ₙ₂Rₐ)ₘ₃-;
-(C(Rₐ₈)(Rₐ₉))ₙ₁-((C(Rₐ₁₀)(Rₐ₁₁))ₙ₂Rₐ)ₘ₃-((C(Rₐ₁₂)(Rₐ₁₃))ₙ₅Rₐ)ₘ₄-;
-(C(Rₐ₈)(Rₐ₉))ₙ₁₋((C(Rₐ₁₀)(Rₐ₁₁))ₙ₂Rₐ)ₘ₃-((C(Rₐ₁₂)(Rₐ₁₃))ₙ₃Rₐ)ₘ₄-((C(Rₐ₁₄)(Rₐ₁₅))ₙ₄Rₐ)ₘ₅-;
-(C(Rₐ₈)(Rₐ₉))ₙ₁-(R_{b}(C(Rₐ₁₀)(Rₐ₁₁))ₙ₂)ₘ₃-;
-(C(Rₐ₈)(Rₐ₉))ₙ₁-(R_{b}(C(Rₐ₁₀)(Rₐ₁₁))ₙ₂)ₘ₃-(R_{b}(C(Rₐ₁₂)(Rₐ₁₃))ₙ₃)ₘ₄-;
-(C(Rₐ₈)(Rₐ₉))ₙ₁-(R_{b}(C(Rₐ₁₀)(Rₐ₁₁))ₙ₂)ₘ₃-(R_{b}(C(Rₐ₁₂)(Rₐ₁₃))ₙ₃)ₘ₄-(R_{b}(C(Rₐ₁₄)(Rₐ₁₅))ₙ₄)ₘ₅-;
-(C(Rₐ₈)(Rₐ₉))ₙ₁-((C(Rₐ₁₀)(Rₐ₁₁))ₙ₂R_{b})ₘ₃-;
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃R_{b})ₘ₄-;
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃R_{b})ₘ₄-((C(Rₐ₁₄)(Rₐ₁₅))ₙ₄R_{b})ₘ₅-;
-(C(Rₐ₈)(Rₐ₉))ₙ₁-(Rₐ-R_{b}-(C(Rₐ₁₀)(Rₐ₁₁))ₙ₂)ₘ₃-;
-(C(Rₐ₈)(Rₐ₉))ₙ₁-(Rₐ(C(Rₐ₁₀)(Rₐ₁₁))ₙ₂)ₘ₃-(R_{b}(C(Rₐ₁₂)(Rₐ₁₃))ₐ₃)ₘ₄-;
-(C(Rₐ₈)(Rₐ₉))ₙ₁-(R_{b}-Rₐ-(C(Rₐ₁₀)(Rₐ₁₁))ₙ₂)ₘ₃-;
-(C(Rₐ₈)(Rₐ₉))ₙ₁-(R_{b}(C(Rₐ₁₀)(Rₐ₁₁))ₙ₂)ₘ₃-(Rₐ(C(Rₐ₁₂)(Rₐ₁₃))ₙ₃)ₘ₄-;
-(C(Rₐ₈)(Rₐ₉))ₙ₁-((C(Rₐ₁₀)(Rₐ₁₁))ₙ₂-Rₐ-R_{b})ₘ₃-;
-(C(Rₐ₈)(Rₐ₉))ₙ₁₋((C(Rₐ₁₀)(Rₐ₁₁))ₙ₂Rₐ)ₘ₃-((C(Rₐ₁₂)(Rₐ₁₃))ₙ₃R_{b})ₘ₄-;
-(C(Rₐ₈)(Rₐ₉))ₙ₁-((C(Rₐ₁₀)(Rₐ₁₁))ₙ₂-R_{b}-Rₐ)ₘ₃-; or
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃Rₐ)ₘ₄-;
wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl; and each R_{b} is independently selected from the group consisting of optionally substituted cycloalkylene (e.g., optionally substituted C₃₋₂₀cycloalkylene), optionally substituted arylene (e.g., optionally substituted C₅₋₂₀arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆alkynylene), alkenylene (e.g., C₂₋₆alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof; and
n1, n2, n3, n4, m3, m4, m5 each independently represent an integer of 1, 2, 3, 4, 5, 6, 7, 8, 9 or 10; and
any one or more hydrogens of said C₁₋₁₅ alkylene are optionally replaced by a substituent selected from: deuterium, hydroxy, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof. In the present disclosure, unless otherwise specified, any one end of the general formulas provided above may be connected to R_{w} of the compound of Formula (I), while the other end is connected to ring A³. The number of carbon atoms of each of the above general formulas, namely the sum of n1+ n2*m3, the sum of n1+ n2*m3+ n3*m4, the sum of n1+ n2*m3+ n3*m4+ n4*m5, each independently is an integer less than or equal to 20.

In some embodiments, R_{w1} of the compound of Formula (I) represents -C₁₋₁₅alkylene-, wherein any one or more hydrogens of said C₁₋₁₅ alkylene are optionally replaced by a substituent selected from: deuterium, hydroxy, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

In some embodiments, R_{w1} of the compound of Formula (I) represents the following groups:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, - (CH₂)₁₁-, -(CH₂)₁₂-, -(CH₂)₁₃-, -(CH₂)₁₄- or -(CH₂)₁₅-;
wherein any one or more hydrogens of said groups are optionally replaced by a substituent selected from: deuterium, hydroxy, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

In some embodiments, R_{w1} of the compound of Formula (I) represents a structure of the following Formulae:

-(C(Rₐ₈)(Rₐ₉))ₙ₁-(O(C(Rₐ₁₀)(Rₐ₁₁))ₙ₂)ₘ₃-;

-(C(Rₐ₈)(Rₐ₉))ₙ₁₋(O(C(Rₐ₁₀)(Rₐ₁₁))ₙ₂)ₘ₃₋(O(C(Rₐ₁₂)(Rₐ₁₃))ₙ₃ )ₘ₄-;

-(C(Rₐ₈)(Rₐ₉))ₙ₁-(O(C(Rₐ₁₀)(Rₐ₁₁))ₙ₂)ₘ₃-(O(C(Rₐ₁₂)(Ra13))ₙ₃)ₘ₄-(O(C(Rₐ₁₄)(Rₐ₁₅))ₙ₄)ₘ₅-;

-(C(Rₐ₈)(Rₐ₉))ₙ₁-((C(Rₐ₁₀)(Rₐ₁₁))ₙ₂O)ₘ₃-;

-(C(Rₐ₈)(Rₐ₉))ₙ₁₋((C(Rₐ₁₀)(Rₐ₁₁))ₙ₂O)ₘ₃-((C(Rₐ₁₂)(Rₐ₁₃))ₙ₃O)ₘ₄-;

-(C(Rₐ₈)(Rₐ₉))ₙ₁-((C(Rₐ₁₀)(Rₐ₁₁))ₙ₂O)ₘ₃-((C(Rₐ₁₂)(Rₐ₁₃))ₙ₃O)ₘ₄-((C(Rₐ₁₄)(Rₐ₁₅))ₙ₄O)ₘ₅-;

-(C(Rₐ₈)(Rₐ₉))ₙ₁-(N(Rₐ₇)(C(Rₐ₁₀)(Rₐ₁₁))ₙ₂)ₘ₃-;

-(C(Rₐ₈)(Rₐ₉))ₙ₁-(N(Rₐ₇)(C(Rₐ₁₀)(Rₐ₁₁))ₙ₂)ₘ₃-(N(Rₐ₇)(C(Rₐ₁₂)(Rₐ₁₃))ₙ₃)ₘ₄-;

-(C(Rₐ₈)(Rₐ₉))ₙ₁-(N(Rₐ₇)(C(Rₐ₁₀)(Rₐ₁₁))ₙ₂)ₘ₃-(N(Rₐ₇)(C(Rₐ₁₂)(Rₐ₁₃))ₙ₃)ₘ₄-(N(Rₐ₇)(C(Rₐ₁₄)(Rₐ₁₅))ₙ₄)ₘ₅-;

-(C(Rₐ₈)(Rₐ₉))ₙ₁-((C(Rₐ₁₀)(Rₐ₁₁))ₙ₂N(Rₐ₇))ₘ₃-;

-(C(Rₐ₈)(Rₐ₉))ₙ₁-((C(Rₐ₁₀)(Rₐ₁₁))ₙ₂N(Rₐ₇))ₘ₃-((C(Rₐ₁₂)(Rₐ₁₃))ₙ₃N(Rₐ₇))ₘ₄-;

-(C(Rₐ₈)(Rₐ₉))ₙ₁₋((C(Rₐ₁₀₎(Rₐ₁₁))ₙ₂N(Rₐ₇)ₘ₃-((C(Rₐ₁₂)(Rₐ₁₃))ₙ₃N(Rₐ₇)ₘ₄-((C(Rₐ₁₄)(Rₐ₁₅))ₙ₄N(Rₐ₇))ₘ₅-;

-(C(Rₐ₈)(Rₐ₉))ₙ₁-(C(O)N(Rₐ₇)-(C(Rₐ₁₀)(Rₐ₁₁))ₙ₂)ₘ₃-;

-(C(Rₐ₈)(Rₐ₉))ₙ₁-(C(O)N(Rₐ₇)-(C(Rₐ₁₀)(Rₐ₁₁))ₙ₂)ₘ₃-(C(O)N(Rₐ₇)-(C(Rₐ₁₂)(Rₐ₁₃))ₙ₃)ₘ₄-;

-(C(Rₐ₈)(Rₐ₉))ₙ₁-(C(O)N(Rₐ₇)-(C(Rₐ₁₀)(Rₐ₁₁))ₙ₂)ₘ₃-(C(O)N(Rₐ₇)-(C(Rₐ₁₂)(Rₐ₁₃))ₙ₃)ₘ₄-(C(O)N(Rₐ₇)-(C(Rₐ₁₄)(Rₐ₁₅))ₙ₄)ₘ₅;

-(C(Rₐ₈)(Rₐ₉))ₙ₁-((C(Rₐ₁₀)(Rₐ₁₁))ₙ₂-C(O)N(Rₐ₇))ₘ₃-;

-(C(Rₐ₈)(Rₐ₉))ₙ₁-((C(Rₐ₁₀)(Rₐ₁₁))ₙ₂-C(O)N(Rₐ₇))ₘ₃-((C(Rₐ₁₂)(Rₐ₁₃))ₙ₃-C(O)N(Rₐ₇))ₘ₄-;

-(C(Rₐ₈)(Rₐ₉))ₙ₁₋((C(Rₐ₁₀₎(Rₐ₁₁))ₙ₂₋C(O)N(Rₐ₇))ₘ₃-((C(Rₐ₁₂)(Rₐ₁₃))ₙ₃-C(O)N(Rₐ₇))ₘ₄-((C(Rₐ₁₄)(Rₐ₁₅))ₙ₄-C(O)N(Rₐ₇))ₘ₅-;

-(C(Rₐ₈)(Rₐ₉))ₙ₁-(C(O)N(Rₐ₇)-(C(Rₐ₁₀)(Rₐ₁₁))ₙ₂)ₘ₃-(O-(C(Rₐ₁₂)(Rₐ₁₃))ₙ₃)ₘ₄-;

-(C(Rₐ₈)(Rₐ₉))ₙ₁-C(O)N(Rₐ₇)-(C(Rₐ₁₀)(Rₐ₁₁))ₙ₂-(O(C(Rₐ₁₂)(Rₐ₁₃))ₙ₃)ₘ₃-;

-(C(Rₐ₈)(Rₐ₉))ₙ₁-((C(Rₐ₁₀)(Rₐ₁₁))ₙ₂-C(O)N(Rₐ₇))ₘ₃-((C(Rₐ₁₂)(Rₐ₁₃))ₙ₃O)ₘ₄;

-(C(Rₐ₈)(Rₐ₉))ₙ₁-(C(Rₐ₁₀)(Rₐ₁₁))ₙ₂-C(O)N(Rₐ₇)-((C(Rₐ₁₂)(Rₐ₁₃))ₙ₃O)ₘ₃-;

-(C(Rₐ₈)(Rₐ₉))ₙ₁-(N(Rₐ₇)C(O)-(C(Rₐ₁₀)(Ra₁₁))ₙ₂)ₘ₃-;

-(C(Rₐ₈)(Rₐ₉))ₙ₁-(N(Rₐ₇)C(O)-(C(Rₐ₁₀)(Rₐ₁₁))ₙ₂)ₘ₃-(O-(C(Rₐ₁₂)(Rₐ₁₃)))ₙ₃)ₘ₄-;

-(C(Rₐ₈)(Rₐ₉))ₙ₁-(N(Rₐ₇)C(O)-(C(Rₐ₁₀)(Rₐ₁₁))ₙ₂)ₘ₃-(O-(C(Rₐ₁₂)(Rₐ₁₃))ₙ₃)ₘ₄-(O-(C(Rₐ₁₄)(Rₐ₁₅))ₙ₄)ₘ₅-;

-(C(Rₐ₈)(Rₐ₉))ₙ₁-N(Rₐ₇)C(O)-(C(Rₐ₁₀)(Rₐ₁₁))ₙ₂-(O(C(Rₐ₁₂)(Rₐ₁₃))ₙ₃)ₘ₃-;

-(C(Rₐ₈)(Rₐ₉))ₙ₁-N(Rₐ₇)C(O)N(Rₐ₇)-(C(Rₐ₁₀)(Rₐ₁₁))ₙ₂-;

-(C(Rₐ₈)(Rₐ₉))ₙ₁-((C(Rₐ₁₀)(Rₐ₁₁))ₙ₂-N(Rₐ₇)C(O))ₘ₃-;

-(C(Rₐ₈)(Rₐ₉))ₙ₁-((C(Rₐ₁₀)(Rₐ₁₁))ₙ₂-N(Rₐ₇)C(O))ₘ₃-((C(Rₐ₁₂)(Rₐ₁₃)))ₙ₃-O)ₘ₄-;

-(C(Rₐ₈)(Rₐ₉))ₙ₁₋((C(Rₐ₁₀)(Rₐ₁₁))ₙ₂-N(Rₐ₇)C(O))ₘ₃-((C(Rₐ₁₂)(Rₐ₁₃))ₙ₃O)ₘ₄-((C(Rₐ₁₄)(Rₐ₁₅))ₙ₄O)ₘ₅-;

-(C(Rₐ₈)(Rₐ₉))ₙ₁-(C(Rₐ₁₀)(Rₐ₁₁))ₙ₂-N(Rₐ₇)C(O)-((C(Rₐ₁₂)(Ra₁₃))ₙ₃O)ₘ₃-;

-(C(Rₐ₈)(Rₐ₉))ₙ₁-(arylene-(C(Rₐ₁₀)(Rₐ₁₁))ₙ₂)ₘ₃-;

-(C(Rₐ₈)(Rₐ₉))ₙ₁-(arylene-(C(Rₐ₁₀)(Rₐ₁₁))ₙ₂)ₘ₃-arylene-(C(Rₐ₁₂)(Rₐ₁₃))ₙ₃-;

-(C(Rₐ₈)(Rₐ₉))ₙ₁-((C(Rₐ₁₀)(Rₐ₁₁))ₙ₂-arylene)ₘ₃-;

-(C(Rₐ₈)(Rₐ₉))ₙ₁-((C(Rₐ₁₀)(Rₐ₁₁))ₙ₂-arylene)ₘ₃-(C(Rₐ₁₂)(Rₐ₁₃))ₙ₃-arylene-;

-(C(Rₐ₈)(Rₐ₉))ₙ₁-(heterocyclylene-(C(Rₐ₁₀)(Rₐ₁₁))ₙ₂)ₘ₃-;

-(C(Rₐ₈)(Rₐ₉))ₙ₁-(heterocyclylene-(C(Rₐ₁₀)(Rₐ₁₁))ₙ₂)ₘ₃-(heterocyclylene-(C(Rₐ₁₂)(Rₐ₁₃))ₙ₃)ₘ₄-;

-(C(Rₐ₈)(Rₐ₉))ₙ₁-((C(Rₐ₁₀)(Rₐ₁₁))ₙ₂-heterocyclylene)ₘ₃-;

-(C(Rₐ₈)(Rₐ₉))ₙ₁-((C(Rₐ₁₀)(Rₐ₁₁))ₙ₂-heterocyclylene)ₘ₃-((C(Rₐ₁₂)(Rₐ₁₃))ₙ₃-heterocyclylene)ₘ₄-;

-(C(Rₐ₈)(Rₐ₉))ₙ₁-(heteroarylene-(C(Rₐ₁₀)(Rₐ₁₁))ₙ₂)ₘ₃-;

-(C(Rₐ₈)(Rₐ₉))ₙ₁-(heteroarylene-(C(Rₐ₁₀)(Rₐ₁₁))ₙ₂)ₘ₃-(heteroarylene-(C(Rₐ₁₂)(Rₐ₁₃))ₙ₃)ₘ₄-;

-(C(Rₐ₈)(Rₐ₉))ₙ₁-((C(Rₐ₁₀)(Ra₁₁))ₙ₂-heteroarylene)ₘ₃-;

-(C(Rₐ₈)(Rₐ₉))ₙ₁-((C(Rₐ₁₀)(Rₐ₁₁))ₙ₂-heteroarylene )ₘ₃-((C(Rₐ₁₂)(Rₐ₁₃))ₙ₃-heteroarylene)ₘ₄-;

-(C(Rₐ₈)(Rₐ₉))ₙ₁-(cycloalkylene-(C(Rₐ₁₀)(Rₐ₁₁))ₙ₂)ₘ₃-;

-(C(Rₐ₈)(Rₐ₉))ₙ₁-(cycloalkylene-(C(Rₐ₁₀)(Rₐ₁₁))ₙ₂)ₘ₃-(cycloalkylene-(C(Rₐ₁₂)(Rₐ₁₃))ₙ₃)ₘ₄-;

-(C(Rₐ₈)(Rₐ₉))ₙ₁-((C(Rₐ₁₀)(Rₐ₁₁))ₙ₂-cycloalkylene)ₘ₃-; or

-(C(Rₐ₈)(Rₐ₉))ₙ₁-((C(Rₐ₁₀)(Rₐ₁₁))ₙ₂-cycloalkylene)ₘ₃-((C(Rₐ₁₂)(Rₐ₁₃))ₙ₃-cycloalkylene)ₘ₄-;

wherein each Rₐ₇ independently represents H or C₁₋₃ alkyl;
the cycloalkylene (e.g., C₃₋₂₀cycloalkylene), the arylene (e.g., C₅₋₂₀arylene), the heterocyclylene (e.g., 4- to 20-membered heterocyclylene), and the heteroarylene (e.g., 5- to 20-membered heteroarylene) are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof; and
n1, n2, n3, n4, m3, m4, and m5 each independently represent an integer of 1, 2, 3, 4, 5, 6, 7, 8, 9 or 10. The total number of carbon atoms of alkylene in each of the above general formulas, namely the sum of n1+ n2*m3, the sum of n1+ n2*m3+ n3*m4, the sum of n1+ n2*m3+ n3*m4+ n4*m5, each independently is an integer less than or equal to 20.

In some embodiments, examples of the cycloalkylene in the general formula structure represented by R_{w1} of the compound of Formula (I) include, but are not limited to, C₃₋₂₀ cycloalkylene, C₃₋₁₅ cycloalkylene, and C₃₋₁₁ cycloalkylene, such as cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. The cycloalkylene is optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, examples of the arylene in the general formula structure represented by R_{w1} of the compound of Formula (I) include, but are not limited to, C₅₋₂₀ arylene, C₆₋₂₀ arylene, C₅₋₁₅ arylene, and C₆₋₁₅ arylene, e.g., phenylene or naphthylene. The arylene is optionally substituted with a substituent selected from the group consisting of deuterium, hydroxy, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, examples of the heterocyclylene in the general formula structure represented by R_{w1} of the compound of Formula (I) include, but are not limited to, 4- to 20-membered , 4-to 15-membered , 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered heterocyclylene, e.g., azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and 5- to 20-membered azaspirocycloalkylene. The heterocyclylene is optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, examples of the heteroarylene in the general formula structure represented by R_{w1} of the compound of Formula (I) include, but are not limited to, 5- to 20-membered heteroarylene, 5- to 15-membered, 5- to 10-membered, 5- to 9-membered, 5- to 8-membered, 5- to 7-membered, and 5-to 6-membered heteroarylene, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. The heteroarylene is optionally substituted with a substituent selected from the group consisting of deuterium, hydroxy, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

In some embodiments, examples of R_{w1} of the compound of Formula (I) include, but are not limited to, the following groups:
-O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, -(CH₂)₁-O-, -(CH₂)₂-O-, -(CH₂)₃-O-, -(CH₂)₄-O-, -(CH₂)₅-O-, -(CH₂)₆-O-, -(CH₂)₇-O-, -(CH₂)₈-O-, -(CH₂)₉-O-, -(CH₂)₁₀-O-, -CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, -(CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, - CH(CH₃)-O-(CH₂)₇-, -CH(CH₃)-O-(CH₂)₈-, -CH(CH₃)-O-(CH₂)₉-, -CH(CH₃)-O-(CH₂)₁₀-, -(O(CH₂)₂)₂-, - (O(CH₂)₂)₃-, -(O(CH₂)₂)₄-, -(O(CH₂)₂)₅-, -(O(CH₂)₂)₆-, -(O(CH₂)₂)₇-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, - CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, - (CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -CH₂-(O(CH₂)₃)₂-, -CH₂-(O(CH₂)₃)₃-, - CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, - (CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, - CH₂-O-(CH₂)₂-O-CH₂-, -(CH₂)₂-O-(CH₂)₂-O-CH₂-, -(CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, - (CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, -N(Rₐ₇)-(CH₂)₆-, -N(Rₐ₇)-(CH₂)₇-, -N(Rₐ₇)-(CH₂)₈-, -N(Rₐ₇)-(CH₂)₉-, -N(Rₐ₇)-(CH₂)₁₀-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH2)4-, -(CH₂)₂-N(Rₐ₇)-(CH₂)₅-, -(CH₂)₂-N(Rₐ₇)-(CH₂)₆-, -(CH₂)₂-N(Rₐ₇)-(CH₂)₇-, -(CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, - (CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, - (CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, - CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, -CH(CH₃)-N(Rₐ₇)-(CH₂)₈-, -CH(CH₃)-N(Rₐ₇)-(CH₂)₉-, -CH(CH₃)-N(Rₐ₇)-(CH₂)₁₀-, -C(O)NHCH₂-, -C(O)NH(CH₂)₂-, -C(O)NH(CH₂)₃-, -C(O)NH(CH₂)₄-, -C(O)NH(CH₂)₅-, -CH₂C(O)NHCH₂-, -(CH₂)₂C(O)NH(CH₂)₂-, -(CH₂)₂C(O)NH(CH₂)₃-, -(CH₂)₂C(O)NH(CH₂)₄-, - (CH₂)₂C(O)NH(CH₂)₅-, -(CH₂)₃C(O)NH(CH₂)₃-, -(CH₂)₃C(O)NH(CH₂)₄-, -(CH₂)₄C(O)NH(CH₂)₄-, - (CH₂)₅C(O)NH(CH₂)₅-, -(CH₂)₆C(O)NH(CH₂)₇-, -(CH₂)₆C(O)NH(CH₂)₆-, -(CH₂)₇C(O)NH(CH₂)₇-, - (CH₂)₂C(O)NH(CH₂)₂-O-(CH₂)₂-, -NHC(O)CH₂-, -NHC(O)(CH₂)₂-, -NHC(O)(CH₂)₃-, -NHC(O)(CH₂)₄-, -NHC(O)(CH₂)₅-, -CH₂NHC(O)CH₂-, -(CH₂)₂NHC(O)(CH₂)₂-, -(CH₂)₂NHC(O)(CH₂)₃-, - (CH₂)₂NHC(O)(CH₂)₄-, -(CH₂)₂NHC(O)(CH₂)₅-, -(CH₂)₃NHC(O)(CH₂)₃-, -(CH₂)₃NHC(O)(CH₂)₄-, - (CH₂)₄NHC(O)(CH₂)₄-, -(CH₂)₅NHC(O)(CH₂)₅-, -(CH₂)₆NHC(O)(CH₂)₇-, -(CH₂)₆NHC(O)(CH₂)₆-, - (CH₂)₇NHC(O)(CH₂)₇-, -(CH₂)₄NHC(O)(CH₂)₈-, -(CH₂)₂NHC(O)(CH₂)₂-O-(CH₂)₂-, -(CH₂)₄NHC(O)CH₂-, -phenylene-CH₂-, -phenylene-(CH₂)₂-, -phenylene-(CH₂)₃-, -phenylene-(CH₂)₄-, -phenylene-(CH₂)₅-, - phenylene-(CH₂)₆-, -phenylene-(CH₂)₇-, -phenylene-(CH₂)₈-, -CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₈-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, -(CH₂)₂-phenylene-(CH₂)₅-, - (CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, -(CH₂)₃-phenylene-(CH₂)₆-, -(CH₂)₃-phenylene-(CH₂)₇-, -(CH₂)₃-phenylene-(CH₂)₈-, - (CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, -(CH₂)₅-phenylene-(CH₂)₁-, -(CH₂)₅-phenylene-(CH₂)₂-, -(CH₂)₅-phenylene-(CH₂)₃-, - (CH₂)₅-phenylene-(CH₂)₄-, -(CH₂)₅-phenylene-(CH₂)₅-, -(CH₂)₅-phenylene-(CH₂)₆-, -(CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, -(CH₂)₆-phenylene-(CH₂)₄-, -(CH₂)₆-phenylene-(CH₂)₅-, -(CH₂)₆-phenylene-(CH₂)₆-, - (CH₂)₆-phenylene-(CH₂)₇-, -(CH₂)₆-phenylene-(CH₂)₈-, -(CH₂)₇-phenylene-(CH₂)₁-, -(CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₈-phenylene-CH₂-, -(CH₂)₈-phenylene-(CH₂)₂-, -(CH₂)₈-phenylene-(CH₂)₃-, -(CH₂)₈-phenylene-(CH₂)₄-, - (CH₂)₈-phenylene-(CH₂)₅-, -(CH₂)₈-phenylene-(CH₂)₆-, -(CH₂)₈-phenylene-(CH₂)₇-, -N(Rₐ₇)-CH₂-phenylene-CH₂-, - N(Rₐ₇)-CH₂-phenylene-(CH₂)₂-, -N(Rₐ₇)-CH₂-phenylene-(CH₂)₃-, -N(Rₐ₇)-CH₂-phenylene-(CH₂)₄-, -N(Rₐ₇)-CH₂-phenylene-(CH₂)₅-, -N(Rₐ₇)-CH₂-phenylene-(CH₂)₆-, -N(Rₐ₇)-CH₂-phenylene-(CH₂)₇-, - N(Rₐ₇)-CH₂-phenylene-(CH₂)₈-, -CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, - CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, -CH₂-N(Rₐ₇)-(CH₂)₂-phenylene-(CH₂)₈-, -(CH₂)₂-N(Rₐ₇)-CH₂-phenylene-CH₂-, -(CH₂)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, -(CH₂)₂-N(Rₐ₇)-CH₂-phenylene-(CH₂)₅-, -(CH₂)₂-N(Rₐ₇)-CH₂-phenylene-(CH₂)₆-, - (CH₂)₂-N(Rₐ₇)-CH₂-phenylene-(CH₂)₇-, -(CH₂)₂-N(Rₐ₇)-CH₂-phenylene-(CH₂)₈-, -(CH₂)₃-N(Rₐ₇)-CH₂-phenylene-CH₂-, -(CH₂)₃-N(Rₐ₇)-CH₂-phenylene-(CH₂)₂-, -(CH₂)₃-N(Rₐ₇)-CH₂-phenylene-(CH₂)₃-, - (CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, -(CH₂)₄-N(Rₐ₇)-CH₂-phenylene-(CH₂)₃-, -(CH₂)₄-N(Rₐ₇)-CH₂-phenylene-(CH₂)₈-, - (CH₂)₅-N(Rₐ₇)-CH₂-phenylene-(CH₂)₃-, -(CH₂)₅-N(Rₐ₇)-CH₂-phenylene-(CH₂)₈-, -(CH₂)₆-N(Rₐ₇)-CH₂-phenylene-(CH₂)₃-, -(CH₂)₆-N(Rₐ₇)-CH₂-phenylene-(CH₂)₈-, -(CH₂)₇-N(Rₐ₇)-CH₂-phenylene-(CH₂)₃-, - (CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, -(CH₂)₈-N(Rₐ₇)-CH₂-phenylene-(CH₂)₄-, -(CH₂)₈-N(Rₐ₇)-CH₂-phenylene-(CH₂)₅-, - (CH₂)₈-N(Rₐ₇)-CH₂-phenylene-(CH₂)₆-, -piperidinylene-CH₂-, -piperidinylene-(CH₂)₂-, -piperidinylene-(CH₂)₃-, -piperidinylene-(CH₂)₄-, -piperidinylene-(CH₂)₅-, -piperidinylene-(CH₂)₆-, -piperidinylene-(CH₂)₇-, -piperidinylene-(CH₂)₈-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, - (CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₈-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperldinylene-(CH₂)₆-, -(CH₂)₈-piperidinylene-(CH₂)₇-, -N(Rₐ₇)-CH₂-piperidinylene-CH₂-, -N(Rₐ₇)-CH₂-piperidinylene-(CH₂)₂-, -N(Rₐ₇)-CH₂-piperidinylene-(CH₂)₃-, -N(Rₐ₇)-CH₂-piperidinylene-(CH₂)₄-, -N(Rₐ₇)-CH₂-piperidinylene-(CH₂)₅-, -N(Rₐ₇)-CH₂-piperidinylene-(CH₂)₆-, - N(Rₐ₇)-CH₂-piperidinylene-(CH₂)₇-, -N(Rₐ₇)-CH₂-piperidinylene-(CH₂)₈-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, -CH₂-N(Rₐ₇)-CH₂-piperidinylene-(CH₂)₄-, -CH₂-N(Rₐ₇)-CH₂-piperidinylene-(CH₂)₅-, -CH₂-N(Ra₇)-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇)-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇)-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-, - (CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, - (CH₂)₅-N(Rₐ₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, - (CH₂)₈-N(Rₐ₇)-CH₂-piperidinylene-(CH₂)₆-, -piperazinylene-CH₂-, -piperazinylene-(CH₂)₂-, - piperazinylene-(CH₂)₃-, -piperazinylene-(CH₂)₄-, -piperazinylene-(CH₂)₅-, -piperazinylene-(CH₂)₆-, - piperazinylene-(CH₂)₇-, -piperazinylene-(CH₂)₈-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₈-, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene and piperidinylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆ cycloalkyl, deuterated C₃₋₆ cycloalkyl, 4- to 10-membered heterocyclyl, and any combination thereof. In the present disclosure, unless otherwise specified, any one end of the general formulas provided above may be connected to R_{w} of the compound of Formula (I), while the other end is connected to ring A³. For example, in the group -O-CH₂-, the O can be connected to R_{w} of the compound of Formula (I), and the CH₂ can be connected to A³ of the compound of Formula (I), and vice versa.

In some embodiments, when R_{w2} and R_{w1} of the compound of Formula (I) both represent a bond, and y3, y4, and y5 all represent an integer of 0, then LIN represents a bond (i.e., LIN is absent), and R_{w} of the compound of Formula (I) is directly connected to the R_{c} group of the PBM.

In some embodiments, examples of LIN of the compound of Formula (I) include, but are not limited to, the following groups:
C(O), C(O)NH, NHC(O), -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄-, -(CH₂)₁₅-; -O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, -(CH₂)₁-O-, - (CH₂)₂-O-, -(CH₂)₃-O-, -(CH₂)₄-O-, (CH₂)₅-O-, -(CH₂)₆-O-, -(CH₂)₇-O-, -(CH₂)₈-O-, -(CH₂)₉-O-, - (CH₂)₁₀-O-, -CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, - (CH₂)₁-O-(CH₂)₆-, -(CH₂)₁-O-(CH₂)₇-, -(CH₂)₁-O-(CH₂)₈-, -(CH₂)₂-O-(CH₂)₁-, -(CH₂)₂-O-(CH₂)₂-, - (CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, - (CH₂)₂-O-(CH₂)₈-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, - (CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, - (CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, - (CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, - (CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, - (CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, - CH(CH₃)-O-(CH₂)₃-, -CH(CH₃)-O-(CH₂)₄-, -CH(CH₃)-O-(CH₂)₅-, -CH(CH₃)-O-(CH₂)₆-, -CH(CH₃)-O-(CH₂)₇-, -CH(CH₃)-O-(CH₂)₈-, -(O(CH₂)₂)₂-, -(O(CH₂)₂)₃-, -(O(CH₂)₂)₄-, -(O(CH₂)₂)₅-, -(O(CH₂)₂)₆-, - CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -CH₂-(O(CH₂)₂)₆-OCH₂-, -(CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, -(CH₂)₃-(O(CH₂)₂)₃-, - (CH₂)₃-(O(CH₂)₂)₄-, -(CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, - (CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -CH₂-O-(CH₂)₂-O-(CH₂)₃-, -CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, - (CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, (CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, - (CH₂)₁-N(Rₐ₇)-(CH₂)₄-, -(CH₂)₁-N(R.₇)-(CH₂)₅-, -(CH₂)₁-N(Rₐ₇)-(CH₂)₆-, -N(Ra₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, -N(Rₐ₇)-(CH₂)₆-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, -(CH₂)₂-N(Rₐ₇)-(CH₂)₆-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, - (CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH2)2-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -C(O)NHCH₂-, -C(O)NH(CH₂)₂-, - C(O)NH(CH₂)₃-, -C(O)NH(CH₂)₄-, -C(O)NH(CH₂)₅-, -CH₂C(O)NHCH₂-, -(CH₂)₂C(O)NH(CH₂)₂-, - (CH₂)₂C(O)NH(CH₂)₃-, -(CH₂)₂C(O)NH(CH₂)₄-, -(CH₂)₂C(O)NH(CH₂)₅-, -(CH₂)₃C(O)NH(CH₂)₃-, - (CH₂)₃C(O)NH(CH₂)₄-, -(CH₂)₄C(O)NH(CH₂)₄-, -(CH₂)₅C(O)NH(CH₂)₅-, -(CH₂)₂C(O)NH(CH₂)₂-O-(CH₂)₂-, -NHC(O)CH₂-, -NHC(O)(CH₂)₂-, -NHC(O)(CH₂)₃-, -NHC(O)(CH₂)₄-, -NHC(O)(CH₂)₅-, - CH₂NHC(O)CH₂-, -(CH₂)₂NHC(O)(CH₂)₂-, -(CH₂)₂NHC(O)(CH₂)₃-, -(CH₂)₂NHC(O)(CH₂)₄-, - (CH₂)₂NHC(O)(CH₂)₅-, -(CH₂)₃NHC(O)(CH₂)₃-, -(CH₂)₃NHC(O)(CH₂)₄-, -(CH₂)₄NHC(O)(CH₂)₄-, - (CH₂)₅NHC(O)(CH₂)₅-, -(CH₂)₂NHC(O)(CH₂)₂-O-(CH₂)₂-, -(CH₂)₄NHC(O)CH₂-, -CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, - CH₂-phenylene-(CH₂)₆-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, -(CH₂)₂-phenylene-(CH₂)₅-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, - (CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -(CH₂)₂-piperazinylene-(CH₂)₁-, - (CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, or -(CH₂)₈-piperazinylene-(CH₂)₅-;
wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl. In the present disclosure, unless otherwise specified, any one end of the above-mentioned groups may be connected to R_{w} of the compound of Formula (I), while the other end is connected to R_{c} of the PBM.

In some embodiments, examples of LIN of the compound of Formula (I) include, but are not limited to, the following groups: 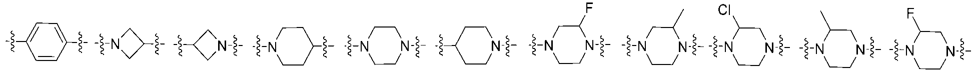 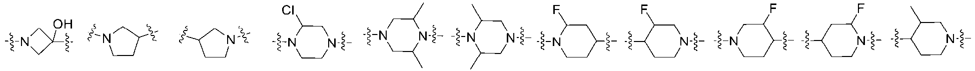 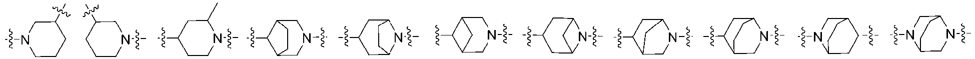 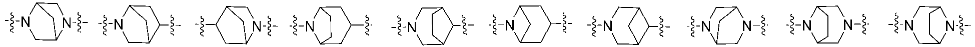 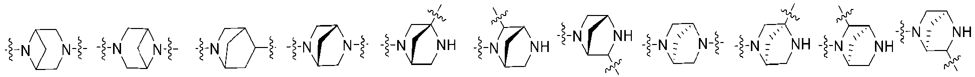 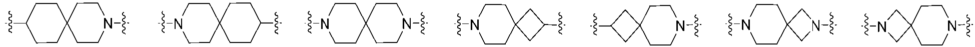 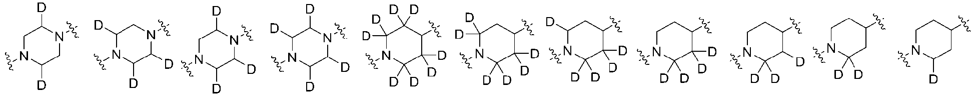 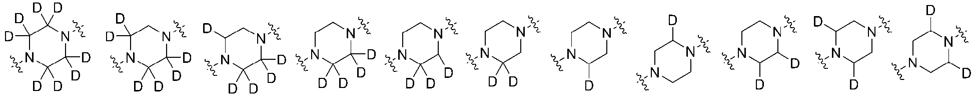 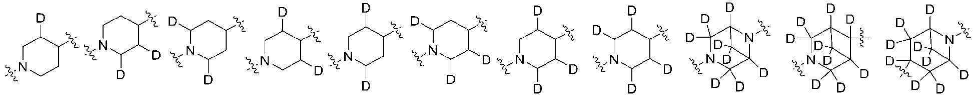 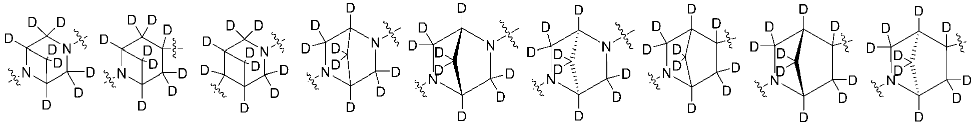 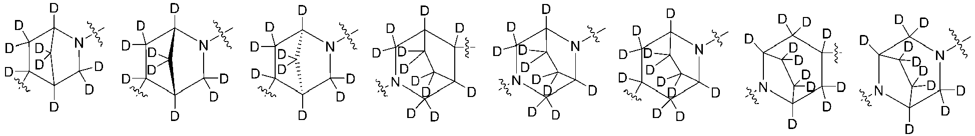 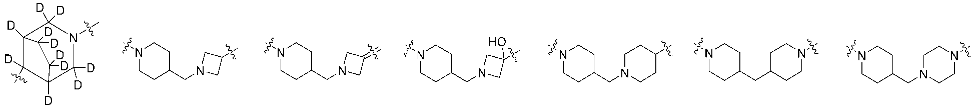 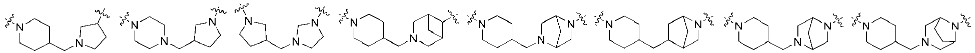 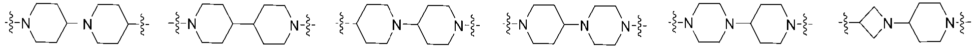 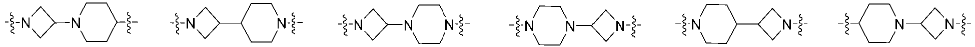 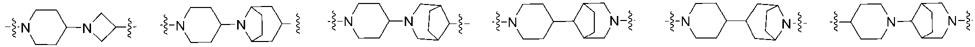 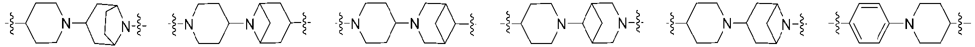 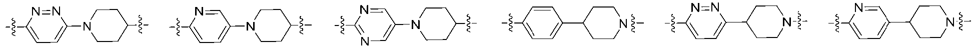 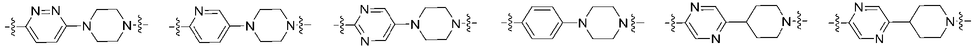 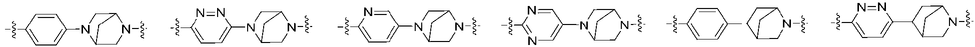 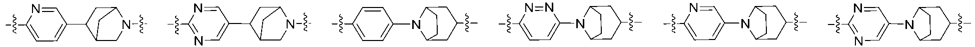 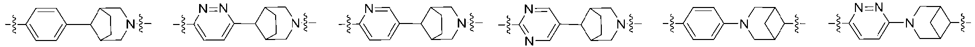 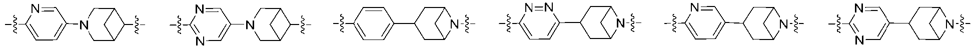 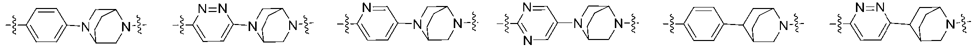 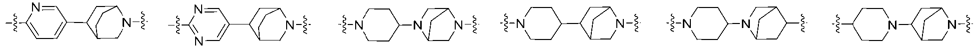 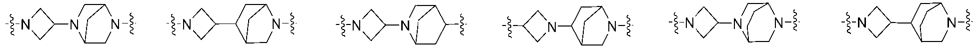 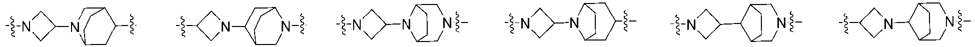 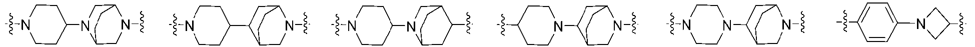 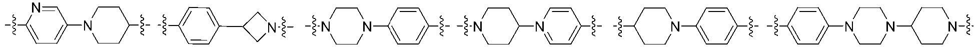 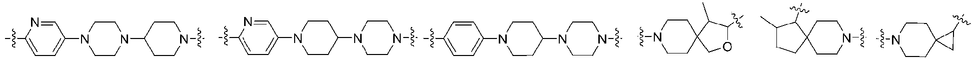 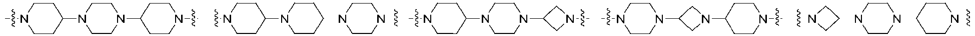 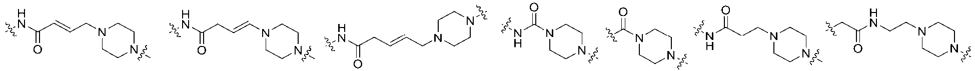 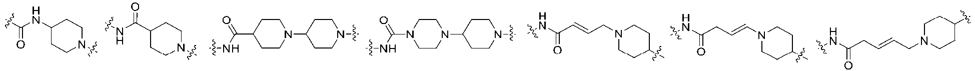 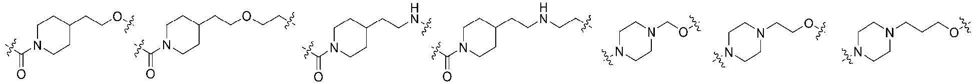 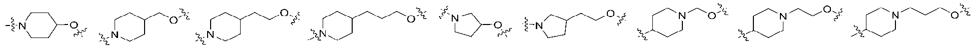 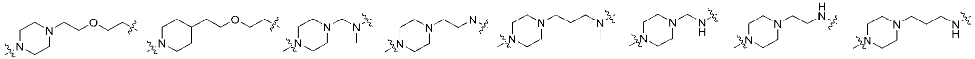 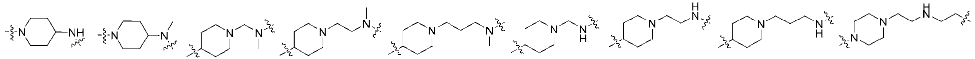 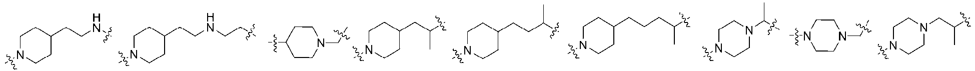 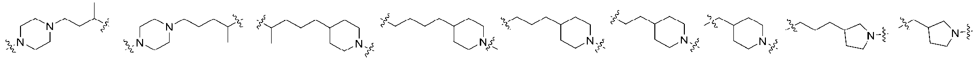 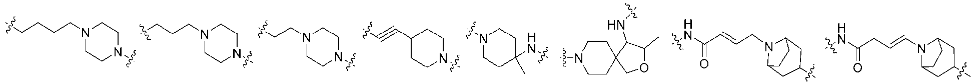 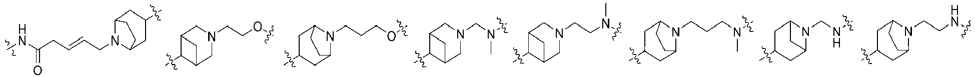 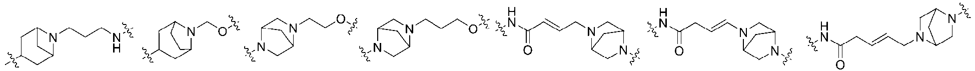 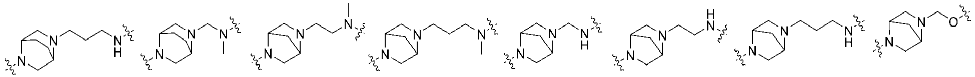 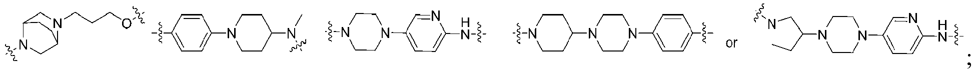 wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl. In the present disclosure, unless otherwise specified, any one end of the above-mentioned groups may be connected to R_{w} of the compound of Formula (I), while the other end is connected to R_{c} of the PBM.

In some embodiments, the compound of Formula (I) is also represented by Formula (I-1), Formula (I-2), Formula (I-3), Formula (I-4), Formula (I-5-1), Formula (I-5-2), Formula (I-5-3), Formula (I-5-4), Formula (I-6), Formula (I-7), Formula (I-8), Formula (I-9), Formula (1-10-1), Formula (1-10-2), Formula (I-11), Formula (I-12), Formula (I-13-1), Formula (I-13-2), Formula (I-14), Formula (I-15), Formula (I-16), Formula (I-17), Formula (1-18-1), Formula (I-18-2), Formula (I-19), Formula (I-20), Formula (I-21), Formula (I-22), Formula (I-23), Formula (I-24), Formula (I-25), Formula (I-26), Formula (I-27), Formula (I-28), Formula (I-29) or Formula (I-30): 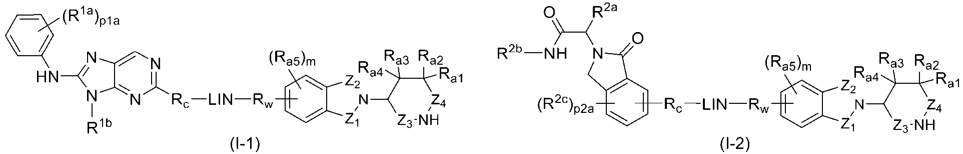 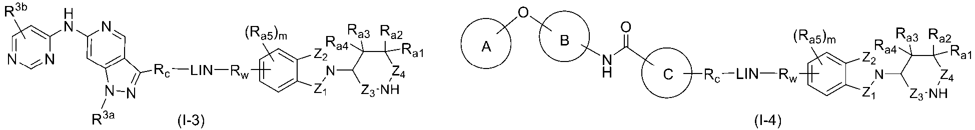 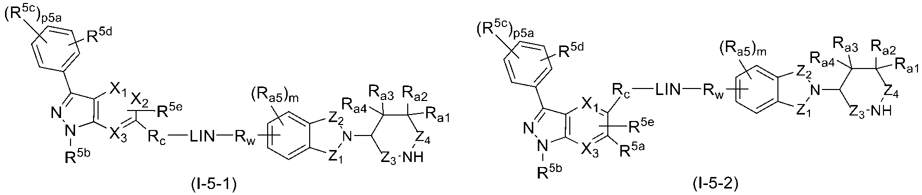 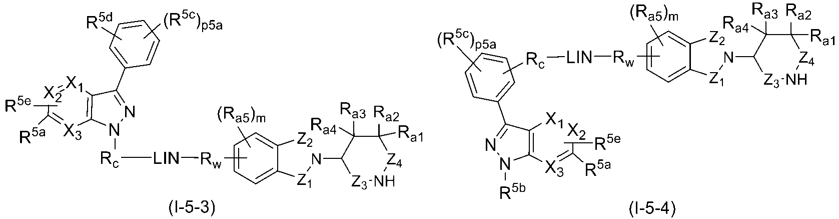 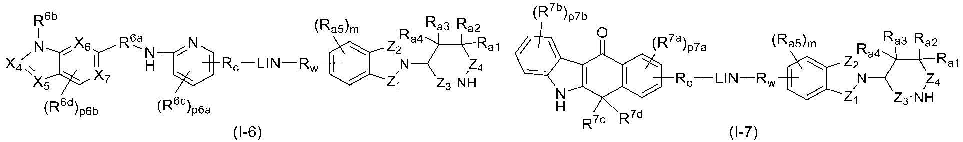 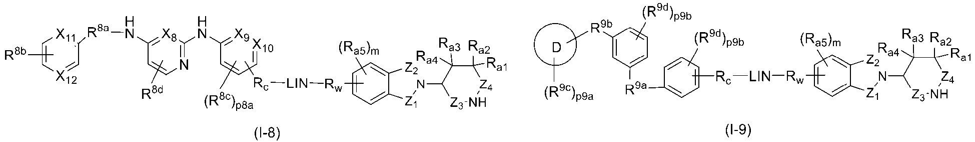 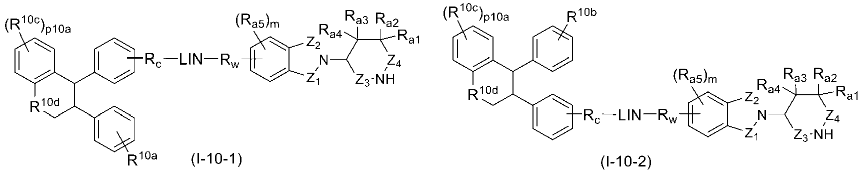 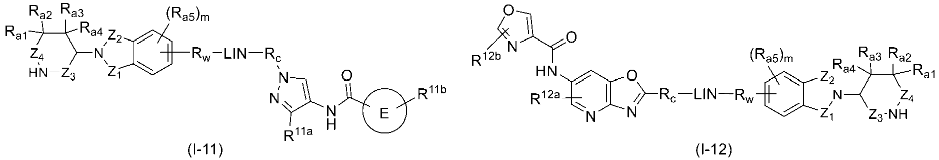 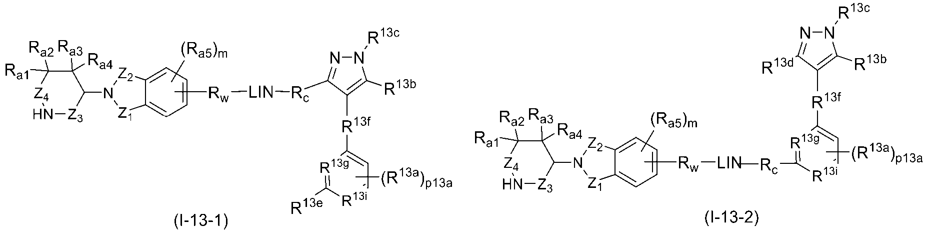 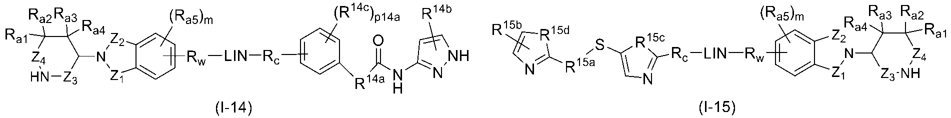 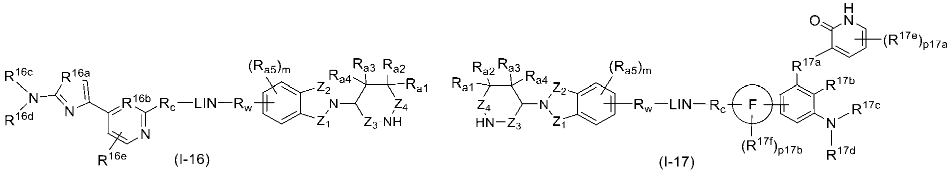 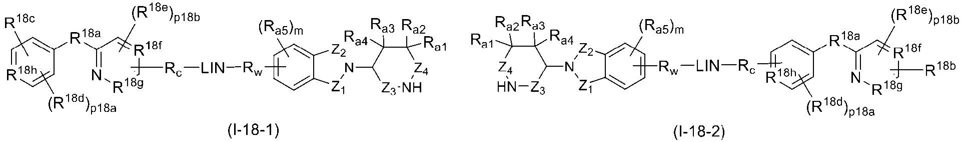 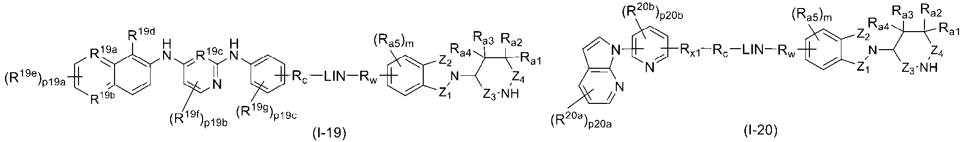 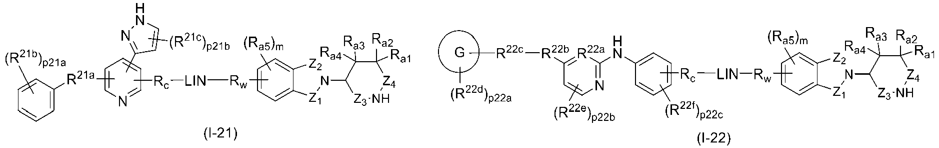 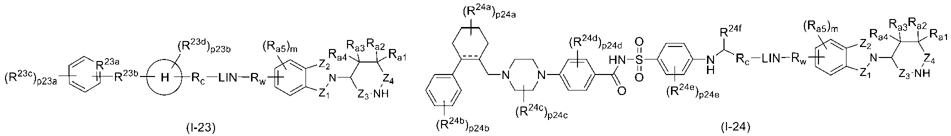 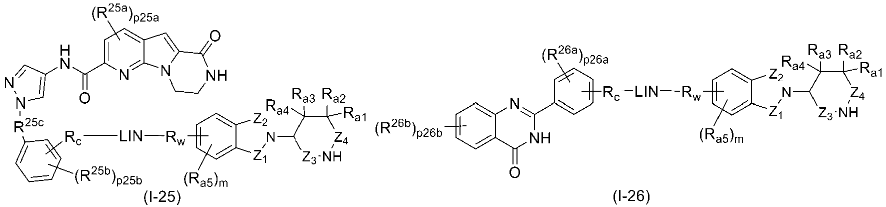 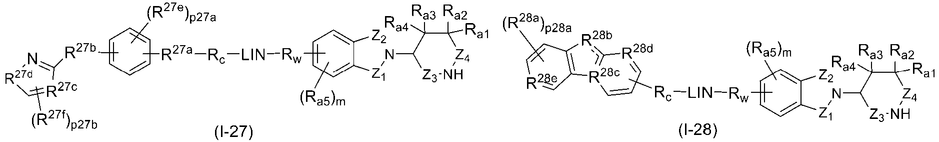 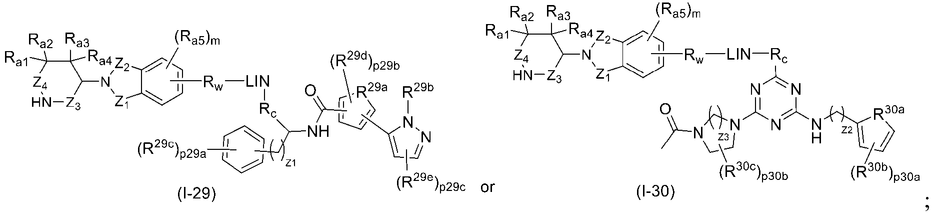 wherein Z₁, Z₂, Z₃, Z₄, Rₐ₁, Rₐ₂, Rₐ₃, Rₐ₄, (Rₐ₅)ₘ, R_{w}, LIN, R_{c}, (R^{1a})ₚ₁ₐ, R^{1b}, R^{2a}, R^{2b}, (R^{2c})ₚ₂ₐ, R^{3a}, R^{3b}, ring A, ring B, ring C, R^{5a}, R^{5b}, (R^{5c})ₚ₅ₐ, R^{5d}, R^{5e}, X₁, X₂, X₃, R^{6a}, R^{6b}, (R^{6c})ₚ₆ₐ, (R^{6d})_{p6b}, X₄, X₅, X₆, X₇, (R^{7a})ₚ₇ₐ, (R^{7b})_{p7b}, R^{7c}, R^{7d}, R^{8a}, R^{8b}, (R^{8c})ₚ₈ₐ, R^{8d}, X₈, X₉, X₁₀, X₁₁, X₁₂, R^{9a}, R^{9b}, (R^{9c})ₚ₉ₐ, (R^{9d})_{p9b}, R^{10a}, R^{10b}, (R^{10c})ₚ₁₀ₐ, R^{10d}, R^{11a}, R^{11b}, ring E, R^{12a}, R^{12b}, (R^{13a})ₚ₁₃ₐ, R^{13b}, R^{13c}, R^{13d}, R^{13e}, R^{13f}, R^{13g}, R¹³ⁱ, R^{14a}, R^{14b}, (R^{14c})ₚ₁₄ₐ, R^{15a}, R^{15b}, R^{15c}, R^{15d}, R^{16a}, R^{16b}, R^{16c}, R^{16d}, R^{16e}, R^{17a}, R^{17b}, R^{17c}, R^{17d}, (R^{17e})ₚ₁₇ₐ, (R^{17f})_{p17b}, ring F, R^{18a}, R^{18c}, (R^{18d})ₚ₁₈ₐ, (R^{18e})_{p18b}, R^{18f}, R^{18g}, R^{18h} , R^{19a}, R^{19b}, R^{19c}, R^{19d}, (R^{19e})ₚ₁₉ₐ, (R^{19f})_{p19b}, (R^{19g})_{p19c}, (R^{20a})ₚ₂₀ₐ, (R^{20b})_{p20b}, Rₓ₁, (R^{21b})ₚ₂₁ₐ, (R^{21c})_{p21b}, R^{22a}, R^{22b}, R^{22c}, (R^{22d})ₚ₂₂ₐ, (R^{22e})_{p22b}, (R^{22f})_{p22c}, ring G, R^{23a}, R^{23b}, (R^{23c})ₚ₂₃ₐ, (R^{23d})_{p23b}, ring H, (R^{24a})ₚ₂₄ₐ, (R^{24b})_{p24b}, (R^{24c})_{p24c}, (R^{24d})_{p24d}, (R^{24e})ₚ₂₄ₑ, R^{24f}, (R^{25a})ₚ₂₅ₐ, (R^{25b})_{p25b}, R^{25c}, (R^{26a})ₚ₂₆ₐ, (R^{26b})_{p26b}, R^{27a}, R^{27b}, R^{27c}, R^{27d}, (R^{27e})ₚ₂₇ₐ, (R^{27f})_{p27b}, (R^{28a})ₚ₂₈ₐ, R^{28b}, R^{28c}, R^{28d}, R^{28e}, R^{29a}, R^{29b}, Z1, (R^{29c})ₚ₂₉ₐ, (R^{29d})_{p29b}, (R^{29e})_{p29c}, R^{30a}, (R^{30b})ₚ₃₀ₐ, (R^{30c})_{p30b}, Z2, and Z3 are as defined in the compound of Formula (I) and each sub-embodiments thereof.

In some embodiments, the compounds of Formula (I) of the present invention and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 1 below are provided.

**Table 1. The compounds of the present disclosure**

| Compound No. | Structure of compound | Compound's name | Chinese translation of Compound's name |
|---|---|---|---|
| GT-05074 | 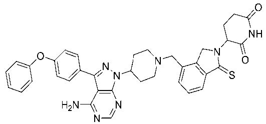 | 3-(4-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione | 3-(4-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione |
| GT-09436 | 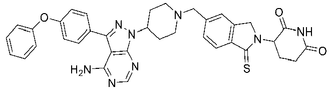 | 3-(5-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione | 3-(5-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione |
| GT-09441 | 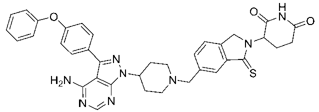 | 3-(6-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione | 3-(6-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione |
| | 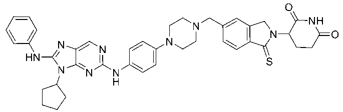 | 3-(5-((4-(4-((9-cyclopentyl-8-(phenylamino)-9H-purin-2-yl)amino)phenyl)piperazi n-1-yl)methyl)-1-thioxoisoindolin-2-yl)piperidine-2,6-dione | 3-(5-((4-(4-((9-cyclopentyl-8-(phenylamino)-9H-purin-2-yl)amino)phenyl)piperazi n-1-yl)methyl)-1-thioxoisoindolin-2-yl)piperidine-2,6-dione |
| | 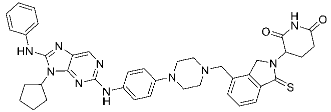 | 3-(4-((4-(4-((9-cyclopentyl-8-(phenylamino)-9H-purin-2-yl)amino)phenyl)piperazi n-1-yl)methyl)-1-thioxoisoindolin-2-yl)piperidine-2,6-dione | 3-(4-((4-(4-((9-cyclopentyl-8-(phenylamino)-9H-purin-2-yl)amino)phenyl)piperazi n-1-yl)methyl)-1-thioxoisoindolin-2-yl)piperidine-2,6-dione |
| | 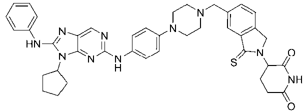 | 3-(6-((4-(4-((9-cyclopentyl-8-(phenylamino)-9H-purin-2-yl)amino)phenyl)piperazi n-1-yl)methyl)-1-thioxoisoindolin-2-yl)piperidine-2,6-dione | 3-(6-((4-(4-((9-cyclopentyl-8-(phenylamino)-9H-purin-2-yl)amino)phenyl)piperazi n-1-yl)methyl)-1-thioxoisoindolin-2-yl)piperidine-2,6-dione |
| | 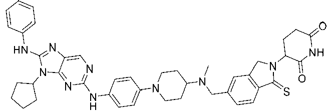 | 3-(5-(((1-(4-((9-cyclopentyl-8-(phenylamino)-9H-purin-2-yl)amino)phenyl)piperidi n-4-yl)(methyl)amino)methyl )-1-thioxoisoindolin-2-yl)piperidine-2,6-dione | 3-(5-(((1-(4-((9-cyclopentyl-8-(phenylamino)-9H-purin-2-yl)amino)phenyl)piperidi n-4-yl)(methyl)amino)methyl )-1-thioxoisoindolin-2-yl)piperidine-2,6-dione |
| | 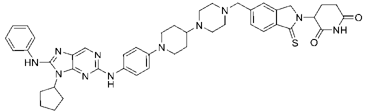 | 3-(5-((4-(1-(4-((9-cyclopentyl-8-(phenylamino)-9H-purin-2-yl)amino)phenyl)piperidi n-4-yl)piperazin-1-yl)methyl)-1-thioxoisoindolin-2-yl)piperidine-2,6-dione | 3-(5-((4-(1-(4-((9-cyclopentyl-8-(phenylamino)-9H-purin-2-yl)amino)phenyl)piperidi n-4-yl)piperazin-1-yl)methyl)-1-thioxoisoindolin-2-yl)piperidine-2,6-dione |
| | 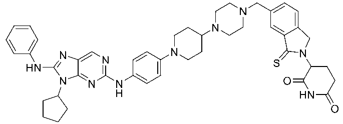 | 3-(6-((4-(1-(4-((9-cyclopentyl-8-(phenylamino)-9H-purin-2-yl)amino)phenyl)piperidi n-4-yl)piperazin-1-yl)methyl)-1-thioxoisoindolin-2-yl)piperidine-2,6-dione | 3-(6-((4-(1-(4-((9-cyclopentyl-8-(phenylamino)-9H-purin-2-yl)amino)phenyl)piperidi n-4-yl)piperazin-1-yl)methyl)-1-thioxoisoindolin-2-yl)piperidine-2,6-dione |
| | 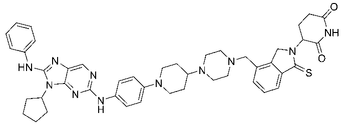 | 3-(4-((4-(1-(4-((9-cyclopentyl-8-(phenylamino)-9H-purin-2-yl)amino)phenyl)piperidi n-4-yl)piperazin-1-yl)methyl)-1-thioxoisoindolin-2-yl)piperidine-2,6-dione | 3-(4-((4-(1-(4-((9-cyclopentyl-8-(phenylamino)-9H-purin-2-yl)amino)phenyl)piperidi n-4-yl)piperazin-1-yl)methyl)-1-thioxoisoindolin-2-yl)piperidine-2,6-dione |
| | 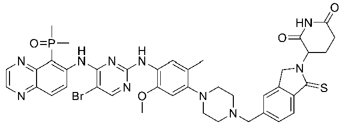 | 3-(5-((4-(4-((5-bromo-4-((5-(dimethylphosphoryl)qui noxalin-6-yl)amino)pyrimidin-2-yl)amino)-5-methoxy-2-methylphenyl)piperazin-1-yl)methyl)-1-thioxoisoindolin-2-yl)piperidine-2,6-dione | 3-(5-((4-(4-((5-bromo-4-((5-(dimethylphosphoryl)qui noxalin-6-yl)amino)pyrimidin-2-yl)amino)-5-methoxy-2-methylphenyl)piperazin-1-yl)methyl)-1-thioxoisoindolin-2-yl)piperidine-2,6-dione |
| | 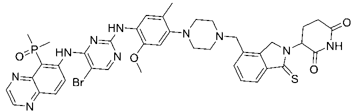 | 3-(4-((4-(4-((5-bromo-4-((5-(dimethylphosphoryl)qui noxalin-6-yl)amino)pyrimidin-2-yl)amino)-5-methoxy-2-methylphenyl)piperazin-1-yl)methyl)-1-thioxoisoindolin-2-yl)piperidine-2,6-dione | 3-(4-((4-(4-((5-bromo-4-((5-(dimethylphosphoryl)qui noxalin-6-yl)amino)pyrimidin-2-yl)amino)-5-methoxy-2-methylphenyl)piperazin-1-yl)methyl)-1-thioxoisoindolin-2-yl)piperidine-2,6-dione |
| | 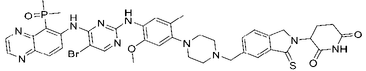 | 3-(6-((4-(4-((5-bromo-4-((5-(dimethylphosphoryl)qui noxalin-6-yl)amino)pyrimidin-2-yl)amino)-5-methoxy-2-methylphenyl)piperazin-1-yl)methyl)-1-thioxoisoindolin-2-yl)piperidine-2,6-dione | 3-(6-((4-(4-((5-bromo-4-((5-(dimethylphosphoryl)qui noxalin-6-yl)amino)pyrimidin-2-yl)amino)-5-methoxy-2-methylphenyl)piperazin-1-yl)methyl)-1-thioxoisoindolin-2-yl)piperidine-2,6-dione |
| GT-08754 | 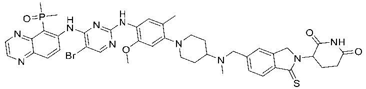 | 3-(5-(((1-(4-((5-bromo-4-((5-(dimethylphosphoryl)qui noxalin-6-yl)amino)pyrimidin-2-yl)amino)-5-methoxy-2-methylphenyl)piperidin-4-yl)(methyl)amino)methyl )-1-thioxoisoindolin-2-yl)piperidine-2,6-dione | 3-(5-(((1-(4-((5-bromo-4-((5-(dimethylphosphoryl)qui noxalin-6-yl)amino)pyrimidin-2-yl)amino)-5-methoxy-2-methylphenyl)piperidin-4-yl)(methyl)amino)methyl )-1-thioxoisoindolin-2-yl)piperidine-2,6-dione |
| GT-08755 | 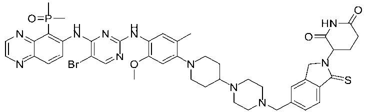 | 3-(5-((4-(1-(4-((5-bromo-4-((5-(dimethylphosphoryl)qui noxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione | 3-(5-((4-(1-(4-((5-bromo-4-((5-(dimethylphosphoryl)qui noxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione |
| GT-05058 | 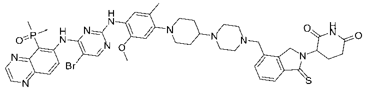 | 3-(4-((4-(1-(4-((5-bromo-4-((5-(dimethylphosphoryl)qui noxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione | 3-(4-((4-(1-(4-((5-bromo-4-((5-(dimethylphosphoryl)qui noxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione |
| | 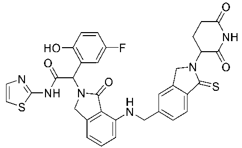 | 2-(7-(((2-(2,6-dioxopiperidin-3-yl)-1-thioxoisoindolin-5-yl)methyl)amino)-1-oxoisoindolin-2-yl)-2-(5-fluoro-2-hydroxyphenyl)-N-(thiazol-2-yl)acetamide | 2-(7-(((2-(2,6-dioxopiperidin-3-yl)-1-thioxoisoindolin-5-yl)methyl)amino)-1-oxoisoindolin-2-yl)-2-(5-fluoro-2-hydroxyphenyl)-N-(thiazol-2-yl)acetamide |
| | 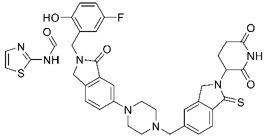 | 2-(6-(4-((2-(2,6-dioxopiperidin-3-yl)-1-thioxoisoindolin-5-yl)methyl)piperazin-1-yl)-1-oxoisoindolin-2-yl)-2-(5-fluoro-2-hydroxyphenyl)-N-(thiazol-2-yl)acetamide | 2-(6-(4-((2-(2,6-dioxopiperidin-3-yl)-1-thioxoisoindolin-5-yl)methyl)piperazin-1-yl)-1-oxoisoindolin-2-yl)-2-(5-fluoro-2-hydroxyphenyl)-N-(thiazol-2-vl)acetamide |
| | 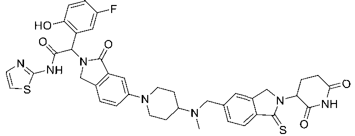 | 2-(6-(4-(((2-(2,6-dioxopiperidin-3-yl)-1-thioxoisoindolin-5-yl)methyl)(methyl)amino )piperidin-1-yl)-1-oxoisoindolin-2-yl)-2-(5-fluoro-2-hydroxyphenyl)-N-(thiazol-2-yl)acetamide | 2-(6-(4-(((2-(2,6-dioxopiperidin-3-yl)-1-thioxoisoindolin-5-yl)methyl)(methyl)amino )piperidin-1-yl)-1-oxoisoindolin-2-yl)-2-(5-fluoro-2-hydroxyphenyl)-N-(thiazol-2-yl)acetamide |
| | 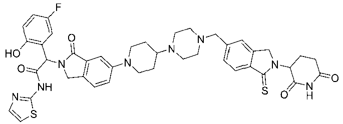 | 2-(6-(4-(4-((2-(2,6-dioxopiperidin-3-yl)-1-thioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide | 2-(6-(4-(4-((2-(2,6-dioxopiperidin-3-yl)-1-thioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide |
| | 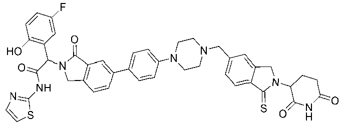 | 2-(6-(4-(4-((2-(2,6-dioxopiperidin-3-yl)-1-thioxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide | 2-(6-(4-(4-((2-(2,6-dioxopiperidin-3-yl)-1-thioxoisoindolin-5-yl)methyl)piperazin-1-yl)phenyl)-1-oxoisoindolin-2-yl)-2-(5-fluoro-2-hydroxyphenyl)-N-(thiazol-2-vl)acetamide |
| | 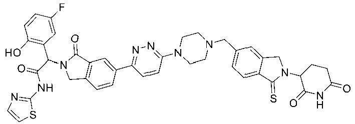 | 2-(6-(6-(4-((2-(2,6-dioxopiperidin-3-yl)-1-thioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((2-(2,6-dioxopiperidin-3-yl)-1-thioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide |
| GT-09423 | 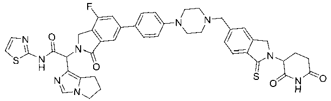 | 2-(6,7-dihydro-5H-pyrrolo[1,2-c]imidazol-1-yl)-2-(6-(4-(4-((2-(2,6-dioxopiperidin-3-yl)-1-thioxoisoindolin-5-yl)methyl)piperazin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-((2-(2,6-dioxopiperidin-3-yl)-1-thioxoisoindolin-5-yl)methyl)piperazin-1-yl)phenyl)-4-fluoro-1-oxoisoindolin-2-yl)-N-(thiazol-2-yl)acetamide |
| GT-09429 | 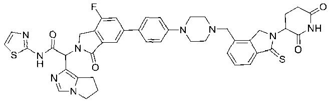 | 2-(6,7-dihydro-5H-pyrrolo[1,2-c]imidazol-1-yl)-2-(6-(4-(4-((2-(2,6-dioxopiperidin-3-yl)-1-thioxoisoindolin-4-yl)methyl)piperazin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-((2-(2,6-dioxopiperidin-3-yl)-1-thioxoisoindolin-4-yl)methyl)piperazin-1-yl)phenyl)-4-fluoro-1-oxoisoindolin-2-yl)-N-(thiazol-2-yl)acetamide |
| GT-09426 | 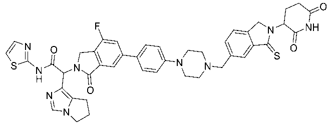 | 2-(6,7-dihydro-5H-pyrrolo[1,2-c]imidazol-1-yl)-2-(6-(4-(4-((2-(2,6-dioxopiperidin-3-yl)-3-thioxoisoindolin-5-yl)methyl)piperazin-1-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-(4-((2-(2,6-dioxopiperidin-3-yl)-3-thioxoisoindolin-5-yl)methyl)piperazin-1-yl)phenyl)-4-fluoro-1-oxoisoindolin-2-yl)-N-(thiazol-2-vl)acetamide |
| | 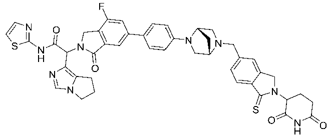 | 2-(6,7-dihydro-5H-pyrrolo[1,2-c]imidazol-1-yl)-2-(6-(4-((1S,4S)-5-((2-(2,6-dioxopiperidin-3-yl)-1-thioxoisoindolin-5-yl)methyl)-2,5-diazabicyclo[2.2.1]hepta n-2-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-((1S,4S)-5-((2-(2,6-dioxopiperidin-3-yl)-1-thioxoisoindolin-5-yl)methyl)-2,5-diazabicyclo[2.2.1]hepta n-2-yl)phenyl)-4-fluoro-1-oxoisoindolin-2-yl)-N-(thiazol-2-vl)acetamide |
| GT-08757 | 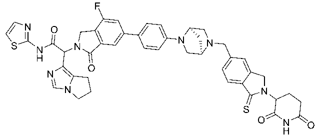 | 2-(6,7-dihydro-5H-pyrrolo[1,2-c]imidazol-1-yl)-2-(6-(4-((1R,4R)-5-((2-(2,6-dioxopiperidin-3-yl)-1-thioxoisoindolin-5-yl)methyl)-2,5-diazabicyclo[2.2.1]hepta n-2-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-((1R,4R)-5-((2-(2,6-dioxopiperidin-3-yl)-1-thioxoisoindolin-5-yl)methyl)-2,5-diazabicyclo[2.2.1]hepta n-2-yl)phenyl)-4-fluoro-1-oxoisoindolin-2-yl)-N-(thiazol-2-vl)acetamide |
| | 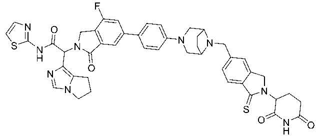 | 2-(6,7-dihydro-5H-pyrrolo[1,2-c]imidazol-1-yl)-2-(6-(4-(8-((2-(2,6-dioxopiperidin-3-yl)-1-thioxoisoindolin-5-yl)methyl)-3,8-diazabicyclo[3.2.1]octan-3-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-(8-((2-(2,6-dioxopiperidin-3-yl)-1-thioxoisoindolin-5-yl)methyl)-3,8-diazabicyclo[3.2.1]octan-3-yl)phenyl)-4-fluoro-1-oxoisoindolin-2-yl)-N-(thiazol-2-vl)acetamide |
| | 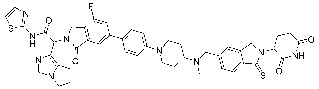 | 2-(6,7-dihydro-5H-pyrrolo[1,2-c]imidazol-1-yl)-2-(6-(4-(4-(((2-(2,6-dioxopiperidin-3-yl)-1-thioxoisoindolin-5-yl)methyl)(methyl)amino )piperidin-1-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-(4-(((2-(2,6-dioxopiperidin-3-yl)-1-thioxoisoindolin-5-yl)methyl)(methyl)amino )piperidin-1-yl)phenyl)-4-fluoro-1-oxoisoindolin-2-yl)-N-(thiazol-2-vl)acetamide |
| | 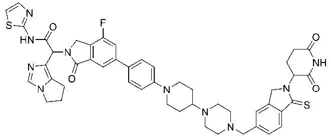 | 2-(6,7-dihydro-5H-pyrrolo[1,2-c]imidazol-1-yl)-2-(6-(4-(4-(4-((2-(2,6-dioxopiperidin-3-yl)-1-thioxoisoindolin-5-yl)methyl)piperazin-1-yl)piperidin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-(4-((2-(2,6-dioxopiperidin-3-yl)-1-thioxoisoindolin-5-yl)methyl)piperazin-1-yl)piperidin-1-yl)phenyl)-4-fluoro-1-oxoisoindolin-2-yl)-N-(thiazol-2-yl)acetamide |
| | 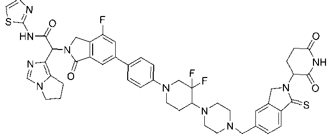 | 2-(6,7-dihydro-5H-pyrrolo[1,2-c]imidazol-1-yl)-2-(6-(4-(4-(4-((2-(2,6-dioxopiperidin-3-yl)-1-thioxoisoindolin-5-yl)methyl)piperazin-1-yl)-3,3-difluoropiperidin-1-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-(4-(4-((2-(2,6-dioxopiperidin-3-yl)-1-thioxoisoindolin-5-yl)methyl)piperazin-1-yl)-3,3-difluoropiperidin-1-yl)phenyl)-4-fluoro-1-oxoisoindolin-2-yl)-N-(thiazol-2-yl)acetamide |
| | 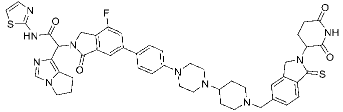 | 2-(6,7-dihydro-5H-pyrrolo[1,2-c]imidazol-1-yl)-2-(6-(4-(4-(1-((2-(2,6-dioxopiperidin-3-yl)-1-thioxoisoindolin-5-yl)methyl)piperidin-4-yl)piperazin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-(1-((2-(2,6-dioxopiperidin-3-yl)-1-thioxoisoindolin-5-yl)methyl)piperidin-4-yl)piperazin-1-yl)phenyl)-4-fluoro-1-oxoisoindolin-2-yl)-N-(thiazol-2-yl)acetamide |
| | 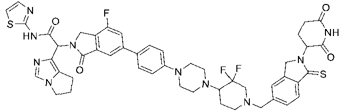 | 2-(6,7-dihydro-5H-pyrrolo[1,2-c]imidazol-1-yl)-2-(6-(4-(4-(1-((2-(2,6-dioxopiperidin-3-yl)-1-thioxoisoindolin-5-yl)methyl)-3,3-difluoropiperidin-4-yl)piperazin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-(1-((2-(2,6-dioxopiperidin-3-yl)-1-thioxoisoindolin-5-yl)methyl)-3,3-difluoropiperidin-4-yl)piperazin-1-yl)phenyl)-4-fluoro-1-oxoisoindolin-2-yl)-N-(thiazol-2-yl)acetamide |
| | 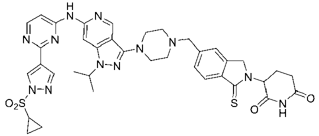 | 3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-1-thioxoisoindolin-2-yl)piperidine-2,6-dione | 3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-1-thioxoisoindolin-2-yl)piperidine-2,6-dione |
| | 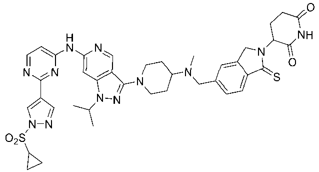 | 3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl )-1-thioxoisoindolin-2-yl)piperidine-2,6-dione | 3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl )-1-thioxoisoindolin-2-yl)piperidine-2,6-dione |
| | 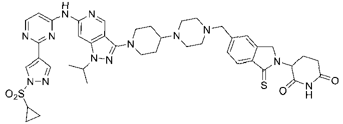 | 3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-1-thioxoisoindolin-2-yl)piperidine-2,6-dione | 3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-1-thioxoisoindolin-2-yl)piperidine-2,6-dione |
| GT-0875 6 | 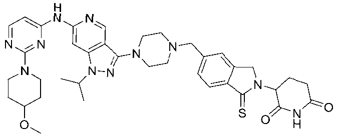 | 3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione | 3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione |
| | 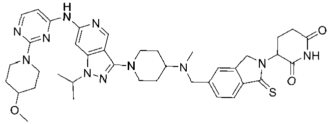 | 3-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl )-1-thioxoisoindolin-2-yl)piperidine-2,6-dione | 3-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl )-1-thioxoisoindolin-2-yl)piperidine-2,6-dione |
| | 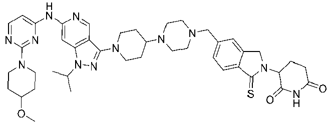 | 3- 5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-1-thioxoisoindolin-2-yl)piperidine-2,6-dione | 3-(5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-1-thioxoisoindolin-2-yl)piperidine-2,6-dione |
| GT-09435 | 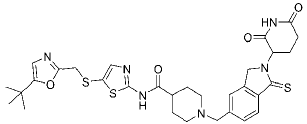 | N-(5-(((5-(tert-butyl)oxazol-2-yl)methyl)thio)thiazol-2-yl)-1-((2-(2,6-dioxopiperidin-3-yl)-1-thioxoisoindolin-5-yl)methyl)piperidine-4-carboxamide | N-(5-(((5-(tert-butyl)oxazol-2-yl)methyl)thio)thiazol-2-yl)-1-((2-(2,6-dioxopiperidin-3-yl)-1-thioxoisoindolin-5-yl)methyl)piperidine-4-carboxamide |
| | 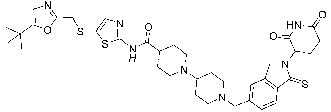 | N-(5-(((5-(tert-butyl)oxazol-2-yl)methyl)thio)thiazol-2-yl)-1'-((2-(2,6-dioxopiperidin-3-yl)-1-thioxoisoindolin-5-yl)methyl)-[1,4'-bipiperidine]-4-carboxamide | N-(5-(((5-(tert-butyl)oxazol-2-yl)methyl)thio)thiazol-2-yl)-1'-((2-(2,6-dioxopiperidin-3-yl)-1-thioxoisoindolin-5-yl)methyl)-[1,4'-bipiperidine]-4-carboxamide |
| GT-09434 | 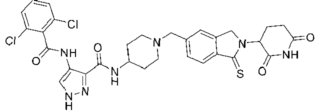 | 4-(2,6-dichlorobenzamido)-N-(1-((2-(2,6-dioxopiperidin-3-yl)-1-thioxoisoindolin-5-yl)methyl)piperidin-4-yl)-1H-pyrazole-3-carboxamide | 4-(2,6-dichlorobenzamido)-N-(1-((2-(2,6-dioxopiperidin-3-yl)-1-thioxoisoindolin-5-yl)methyl)piperidin-4-yl)-1H-pyrazole-3-carboxamide |
| | 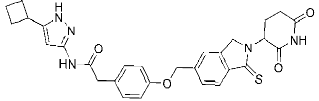 | N-(5-cyclobutyl-1H-pyrazol-3-yl)-2-(4-((2-(2,6-dioxopiperidin-3-yl)-1-thioxoisoindolin-5-yl)methoxy)phenyl)aceta mide | N-(5-cyclobutyl-1H-pyrazol-3-yl)-2-(4-((2-(2,6-dioxopiperidin-3-yl)-1-thioxoisoindolin-5-yl)methoxy)phenyl)aceta mide |
| GT-08682 | 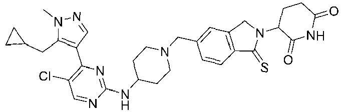 | 3-(5-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione | 3-(5-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione |
| GT-08681 | 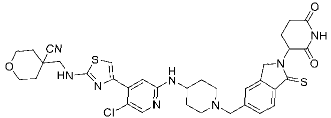 | 4-(((4-(5-chloro-2-((1-((2-(2,6-dioxopiperidin-3-yl)-1-thioxoisoindolin-5-yl)methyl)piperidin-4-yl)amino)pyridin-4-yl)thiazol-2-yl)amino)methyl)tetrahy dro-2H-pyran-4-carbonitrile | 4-(((4-(5-chloro-2-((1-((2-(2,6-dioxopiperidin-3-yl)-1-thioxoisoindolin-5-yl)methyl)piperidin-4-yl)amino)pyridin-4-yl)thiazol-2-yl)amino)methyl)tetrahy dro-2H-pyran-4-carbonitrile |
| GT-0868 3 | 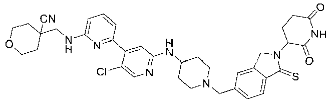 | 4-(((5'-chloro-2'-((1-((2-(2,6-dioxopiperidin-3-yl)-1-thioxoisoindolin-5-yl)methyl)piperidin-4-yl)amino)-[2,4'-bipyridin]-6-yl)amino)methyl)tetrahy dro-2H-pyran-4-carbonitrile | 4-(((5'-chloro-2'-((1-((2-(2,6-dioxopiperidin-3-yl)-1-thioxoisoindolin-5-yl)methyl)piperidin-4-yl)amino)-[2,4'-bipyridin]-6-yl)amino)methyl)tetrahy dro-2H-pyran-4-carbonitrile |
| GT-0868 7 | 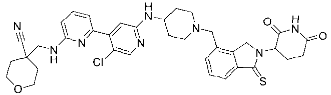 | 4-(((5'-chloro-2'-((1-((2-(2,6-dioxopiperidin-3-yl)-1-thioxoisoindolin-4-yl)methyl)piperidin-4-yl)amino)-[2,4'-bipyridin]-6-yl)amino)methyl)tetrahy dro-2H-pyran-4-carbonitrile | 4-(((S'-chloro-2'-((1-((2-(2,6-dioxopiperidin-3-yl)-1-thioxoisoindolin-4-yl)methyl)piperidin-4-yl)amino)-[2,4'-bipyridin]-6-yl)amino)methyl)tetrahy dro-2H-pyran-4-carbonitrile |
| GT-0876 9 | 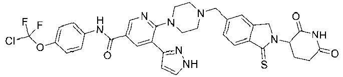 | N-(4-(chlorodifluoromethoxy) phenyl)-6-(4-((2-(2,6-dioxopiperidin-3-yl)-1-thioxoisoindolin-5-yl)methyl)piperazin-1-yl)-5-(1H-pyrazol-3-yl)nicotinamide | N-(4-(chlorodifluoromethoxy) phenyl)-6-(4-((2-(2,6-dioxopiperidin-3-yl)-1-thioxoisoindolin-5-yl)methyl)piperazin-1-yl)-5-(1H-pyrazol-3-yl)nicotinamide |
| | 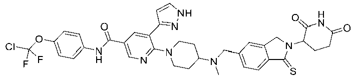 | N-(4-(chlorodifluoromethoxy) phenyl)-6-(4-(((2-(2,6-dioxopiperidin-3-yl)-1-thioxoisoindolin-5-yl)methyl)(methyl)amino )piperidin-1-yl)-5-(1H-pyrazol-3-yl)nicotinamide | N-(4-(chlorodifluoromethoxy) phenyl)-6-(4-(((2-(2,6-dioxopiperidin-3-yl)-1-thioxoisoindolin-5-yl)methyl)(methyl)amino )piperidin-1-yl)-5-(1H-pyrazol-3-yl)nicotinamide |
| | 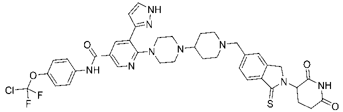 | N-(4-(chlorodifluoromethoxy) phenyl)-6-(4-(1-((2-(2,6-dioxopiperidin-3-yl)-1-thioxoisoindolin-5-yl)methyl)piperidin-4-yl)piperazin-1-yl)-5-(1H-pyrazol-3-yl)nicotinamide | N-(4-(chlorodifluoromethoxy) phenyl)-6-(4-(1-((2-(2,6-dioxopiperidin-3-yl)-1-thioxoisoindolin-5-yl)methyl)piperidin-4-yl)piperazin-1-yl)-5-(1H-pyrazol-3-yl)nicotinamide |
| GT-08770 | 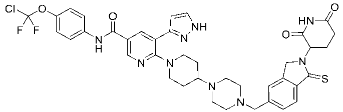 | N-(4-(chlorodifluoromethoxy) phenyl)-6-(4-(4-((2-(2,6-dioxopiperidin-3-yl)-1-thioxoisoindolin-5-yl)methyl)piperazin-1-yl)piperidin-1-yl)-5-(1H-pyrazol-3-yl)nicotinamide | N-(4-(chlorodifluoromethoxy) phenyl)-6-(4-(4-((2-(2,6-dioxopiperidin-3-yl)-1-thioxoisoindolin-5-yl)methyl)piperazin-1-yl)piperidin-1-yl)-5-(1H-pyrazol-3-yl)nicotinamide |
| GT-08767 | 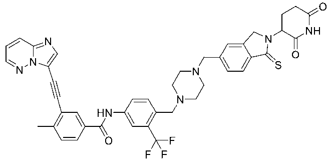 | N-(4-((4-((2-(2,6-dioxopiperidin-3-yl)-1-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide | N-(4-((4-((2-(2,6-dioxopiperidin-3-yl)-1-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide |
| GT-08773 | 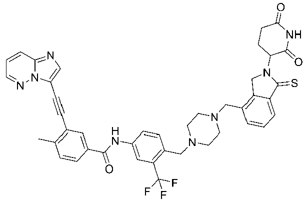 | N-(4-((4-((2-(2,6-dioxopiperidin-3-yl)-1-thioxoisoindolin-4-yl)methyl)piperazin-1-yl)methyl)-3-(trifluoromethyl)phenyl)-3-(imidazo[1,2-b]pyridazin-3-ylethynyl)-4-methylbenzamide | N-(4-((4-((2-(2,6-dioxopiperidin-3-yl)-1-thioxoisoindolin-4-yl)methyl)piperazin-1-yl)methyl)-3-(trifluoromethyl)phenyl)-3-(imidazo[1,2-b]pyridazin-3-ylethynyl)-4-methylbenzamide |
| GT-09439 | 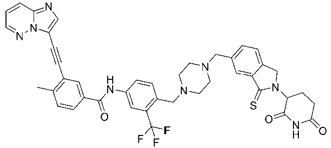 | N-(4-((4-((2-(2,6-dioxopiperidin-3-yl)-3-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide | N-(4-((4-((2-(2,6-dioxopiperidin-3-yl)-3-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide |
| GT-08768 | 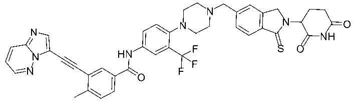 | N-(4-(4-((2-(2,6-dioxopiperidin-3-yl)-1-thioxoisoindolin-5-yl)methyl)piperazin-1-yl)-3-(trifluoromethyl)phenyl)-3-(imidazo[1,2-b]pyridazin-3-ylethynyl)-4-methylbenzamide | N-(4-(4-((2-(2,6-dioxopiperidin-3-yl)-1-thioxoisoindolin-5-yl)methyl)piperazin-1-yl)-3-(trifluoromethyl)phenyl)-3-(imidazo[1,2-b]pyridazin-3-ylethynyl)-4-methylbenzamide |
| GT-08774 | 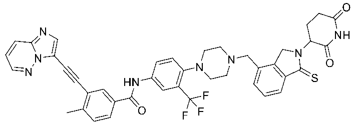 | N-(4-(4-((2-(2,6-dioxopiperidin-3-yl)-1-thioxoisoindolin-4-yl)methyl)piperazin-1-yl)-3-(trifluoromethyl)phenyl)-3-(imidazo[1,2-b]pyridazin-3-ylethynyl)-4-methylbenzamide | N-(4-(4-((2-(2,6-dioxopiperidin-3-yl)-1-thioxoisoindolin-4-yl)methyl)piperazin-1-yl)-3-(trifluoromethyl)phenyl)-3-(imidazo[1,2-b]pyridazin-3-ylethynyl)-4-methylbenzamide |
| GT-09440 | 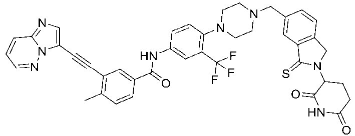 | N-(4-(4-((2-(2,6-dioxopiperidin-3-yl)-3-thioxoisoindolin-5-yl)methyl)piperazin-1-yl)-3-(trifluoromethyl)phenyl)-3-(imidazo[1,2-b]pyridazin-3-ylethynyl)-4-methylbenzamide | N-(4-(4-((2-(2,6-dioxopiperidin-3-yl)-3-thioxoisoindolin-5-yl)methyl)piperazin-1-yl)-3-(trifluoromethyl)phenyl)-3-(imidazo[1,2-b]pyridazin-3-ylethynyl)-4-methylbenzamide |
| | 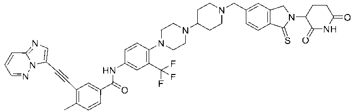 | N-(4-(4-(1-((2-(2,6-dioxopiperidin-3-yl)-1-thioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide | N-(4-(4-(1-((2-(2,6-dioxopiperidin-3-yl)-1-thioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide |
| | 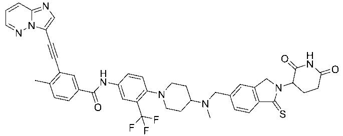 | N-(4-(4-(((2-(2,6-dioxopiperidin-3-yl)-1-thioxoisoindolin-5-yl)methyl)(methyl)amino )piperidin-1-yl)-3-(trifluoromethyl)phenyl)-3-(imidazo[1,2-b]pyridazin-3-ylethynyl)-4-methylbenzamide | N-(4-(4-(((2-(2,6-dioxopiperidin-3-yl)-1-thioxoisoindolin-5-yl)methyl)(methyl)amino )piperidin-1-yl)-3-(trifluoromethyl)phenyl)-3-(imidazo[1,2-b]pyridazin-3-ylethynyl)-4-methylbenzamide |
| GT-08765 | 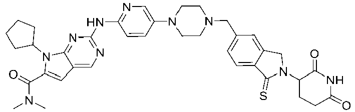 | 7-cyclopentyl-2-((5-(4-((2-(2,6-dioxopiperidin-3-yl)-1-thioxoisoindolin-5-yl)methyl)piperazin-1-yl)pyridin-2-yl)amino)-N,N-dimethyl-7H-pyrrolo[2,3-d]pyrimidine-6-carboxamide | 7-cyclopentyl-2-((5-(4-((2-(2,6-dioxopiperidin-3-yl)-1-thioxoisoindolin-5-yl)methyl)piperazin-1-yl)pyridin-2-yl)amino)-N,N-dimethyl-7H-pyrrolo[2,3-d]pyrimidine-6-carboxamide |
| GT-08771 | 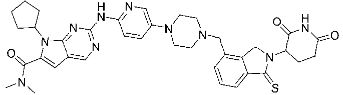 | 7-cyclopentyl-2-((5-(4-((2-(2,6-dioxopiperidin-3-yl)-1-thioxoisoindolin-4-yl)methyl)piperazin-1-yl)pyridin-2-yl)amino)-N,N-dimethyl-7H-pyrrolo[2,3-d]pyrimidine-6-carboxamide | 7-cyclopentyl-2-((5-(4-((2-(2,6-dioxopiperidin-3-yl)-1-thioxoisoindolin-4-yl)methyl)piperazin-1-yl)pyridin-2-yl)amino)-N,N-dimethyl-7H-pyrrolo[2,3-d]pyrimidine-6-carboxamide |
| GT-09427 | 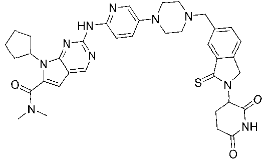 | 7-cyclopentyl-2-((5-(4-((2-(2,6-dioxopiperidin-3-yl)-3-thioxoisoindolin-5-yl)methyl)piperazin-1-yl)pyridin-2-yl)amino)-N,N-dimethyl-7H-pyrrolo[2,3-d]pyrimidine-6-carboxamide | 7-cyclopentyl-2-((5-(4-((2-(2,6-dioxopiperidin-3-yl)-3-thioxoisoindolin-5-yl)methyl)piperazin-1-yl)pyridin-2-yl)amino)-N,N-dimethyl-7H-pyrrolo[2,3-d]pyrimidine-6-carboxamide |
| | 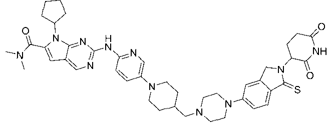 | 7-cyclopentyl-2-((5-(4-((4-(2-(2,6-dioxopiperidin-3-yl)-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide |
| | 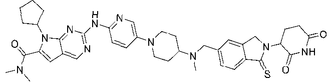 | 7-cyclopentyl-2-((5-(4-(((2-(2,6-dioxopiperidin-3-yl)-1-thioxoisoindolin-5-yl)methyl)(methyl)amino )piperidin-1-yl)pyridin-2-yl)amino)-N,N-dimethyl-7H-pyrrolo[2,3-d]pyrimidine-6-carboxamide | 7-cyclopentyl-2-((5-(4-(((2-(2,6-dioxopiperidin-3-yl)-1-thioxoisoindolin-5-yl)methyl)(methyl)amino )piperidin-1-yl)pyridin-2-yl)amino)-N,N-dimethyl-7H-pyrrolo[2,3-d]pyrimidine-6-carboxamide |
| | 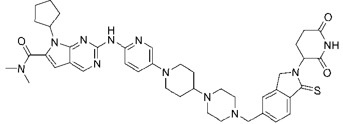 | 7-cyclopentyl-2-((5-(4-(4-((2-(2,6-dioxopiperidin-3-yl)-1-thioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide | 7-cyclopentyl-2-((5-(4-(4-((2-(2,6-dioxopiperidin-3-yl)-1-thioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide |
| | 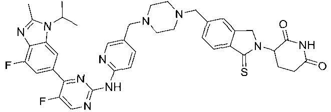 | 3-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione | 3-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione |
| | 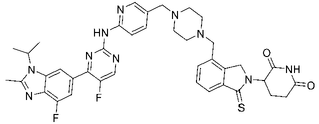 | 3-(4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione | 3-(4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione |
| | 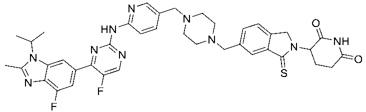 | 3-(6-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione | 3-(6-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione |
| | 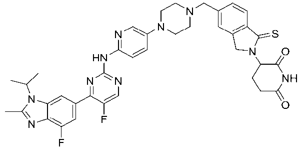 | 3-(5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-thioxoisoindolin-2-yl)piperidine-2,6-dione | 3-(5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-thioxoisoindolin-2-yl)piperidine-2,6-dione |
| | 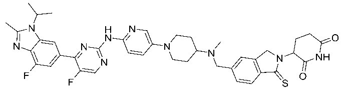 | 3-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl )-1-thioxoisoindolin-2-yl)piperidine-2,6-dione | 3-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl )-1-thioxoisoindolin-2-yl)piperidine-2,6-dione |
| | 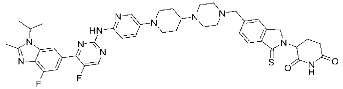 | 3-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-thioxoisoindolin-2-yl)piperidine-2,6-dione | 3-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-thioxoisoindolin-2-yl)piperidine-2,6-dione |
| GT-08766 | 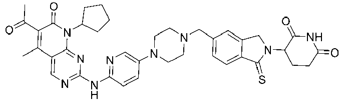 | 3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione | 3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione |
| GT-08772 | 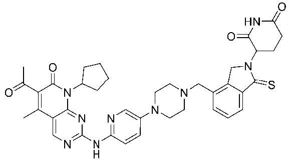 | 3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione | 3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione |
| GT-09428 | 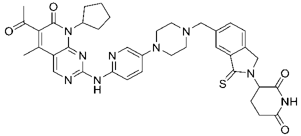 | 3-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione | 3-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione |
| | 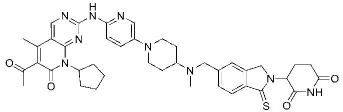 | 3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl )-1-thioxoisoindolin-2-yl)piperidine-2,6-dione | 3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl )-1-thioxoisoindolin-2-yl)piperidine-2,6-dione |
| | 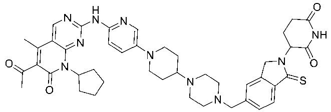 | 3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-1-thioxoisoindolin-2-yl)piperidine-2,6-dione | 3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-1-thioxoisoindolin-2-yl)piperidine-2,6-dione |
| | 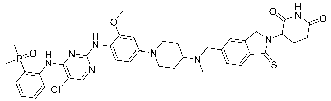 | 3-(5-(((1-(4-((5-chloro-4-((2-(dimethylphosphoryl)phe nyl)amino)pyrimidin-2-yl)amino)-3-methoxyphenyl)piperidin-4-yl)(methyl)amino)methyl )-1-thioxoisoindolin-2-yl)piperidine-2,6-dione | 3-(5-(((1-(4-((5-chloro-4-((2-(dimethylphosphoryl)phe nyl)amino)pyrimidin-2-yl)amino)-3-methoxyphenyl)piperidin-4-yl)(methyl)amino)methyl )-1-thioxoisoindolin-2-yl)piperidine-2,6-dione |
| | 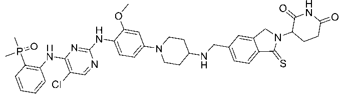 | 3-(5-(((1-(4-((5-chloro-4-((2-(dimethylphosphoryl)phe nyl)amino)pyrimidin-2-yl)amino)-3-methoxyphenyl)piperidin -4-yl)amino)methyl)-1-thioxoisoindolin-2-yl)piperidine-2,6-dione | 3-(5-(((1-(4-((5-chloro-4-((2-(dimethylphosphoryl)phe nyl)amino)pyrimidin-2-yl)amino)-3-methoxyphenyl)piperidin -4-yl)amino)methyl)-1-thioxoisoindolin-2-yl)piperidine-2,6-dione |
| GT-0867 5 | 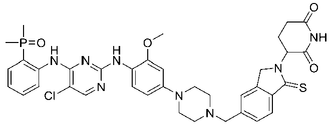 | 3-(5-((4-(4-((5-chloro-4-((2-(dimethylphosphoryl)phe nyl)amino)pyrimidin-2-yl)amino)-3-methoxyphenyl)piperazi n-1-yl)methyl)-1-thioxoisoindolin-2-yl)piperidine-2,6-dione | 3-(5-((4-(4-((5-chloro-4-((2-(dimethylphosphoryl)phe nyl)amino)pyrimidin-2-yl)amino)-3-methoxyphenyl)piperazi n-1-yl)methyl)-1-thioxoisoindolin-2-yl)piperidine-2,6-dione |
| GT-0942 5 | 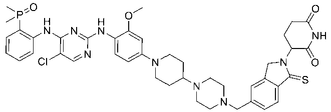 | 3-(5-((4-(1-(4-((5-chloro-4-((2-(dimethylphosphoryl)phe nyl)amino)pyrimidin-2-yl)amino)-3-methoxyphenyl)piperidin -4-yl)piperazin-1-yl)methyl)-1-thioxoisoindolin-2-yl)piperidine-2,6-dione | 3-(5-((4-(1-(4-((5-chloro-4-((2-(dimethylphosphoryl)phe nyl)amino)pyrimidin-2-yl)amino)-3-methoxyphenyl)piperidin -4-yl)piperazin-1-yl)methyl)-1-thioxoisoindolin-2-yl)piperidine-2,6-dione |
| GT-0509 7 | 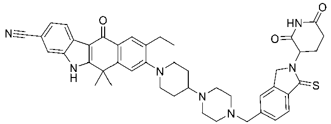 | 8-(4-(4-((2-(2,6-dioxopiperidin-3-yl)-1-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile | 8-(4-(4-((2-(2,6-dioxopiperidin-3-yl)-1-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile |
| GT-0503 0 | 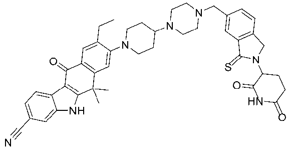 | 8-(4-(4-((2-(2,6-dioxopiperidin-3-yl)-3-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile | 8-(4-(4-((2-(2,6-dioxopiperidin-3-yl)-3-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile |
| GT-0942 4 | 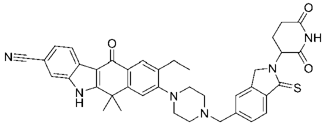 | 8-(4-((2-(2,6-dioxopiperidin-3-yl)-1-thioxoisoindolin-5-yl)methyl)piperazin-1-yl)-9-ethyl-6,6-dimethyl-11-oxo-6,11-dihydro-5H-benzo[b]carbazole-3-carbonitrile | 8-(4-((2-(2,6-dioxopiperidin-3-yl)-1-thioxoisoindolin-5-yl)methyl)piperazin-1-yl)-9-ethyl-6,6-dimethyl-11-oxo-6,11-dihydro-5H-benzo[b]carbazole-3-carbonitrile |
| | 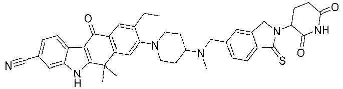 | 8-(4-(((2-(2,6-dioxopiperidin-3-yl)-1-thioxoisoindolin-5-yl)methyl)(methyl)amino )piperidin-1-yl)-9-ethyl-6,6-dimethyl-11-oxo-6,11-dihydro-5H-benzo[b]carbazole-3-carbonitrile | 8-(4-(((2-(2,6-dioxopiperidin-3-yl)-1-thioxoisoindolin-5-yl)methyl)(methyl)amino )piperidin-1-yl)-9-ethyl-6,6-dimethyl-11-oxo-6,11-dihydro-5H-benzo[b]carbazole-3-carbonitrile |
| | 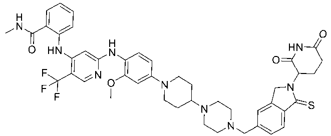 | 2-((2-((4-(4-(4-((2-(2,6-dioxopiperidin-3-yl)-1-thioxoisoindolin-5-yl)methyl)piperazin-1-yl)piperidin-1-yl)-2-methoxyphenyl)amino)-5-(trifluoromethyl)pyridin-4-yl)amino)-N-methylbenzamide | 2-((2-((4-(4-(4-((2-(2,6-dioxopiperidin-3-yl)-1-thioxoisoindolin-5-yl)methyl)piperazin-1-yl)piperidin-1-yl)-2-methoxyphenyl)amino)-5-(trifluoromethyl)pyridin-4-yl)amino)-N-methylbenzamide |
| | 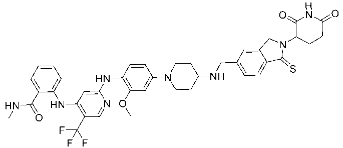 | 2-((2-((4-(4-(((2-(2,6-dioxopiperidin-3-yl)-1-thioxoisoindolin-5-yl)methyl)amino)piperidi n-1-yl)-2-methoxyphenyl)amino)-5-(trifluoromethyl)pyridin-4-yl)amino)-N-methylbenzamide | 2-((2-((4-(4-(((2-(2,6-dioxopiperidin-3-yl)-1-thioxoisoindolin-5-yl)methyl)amino)piperidi n-1-yl)-2-methoxyphenyl)amino)-5-(trifluoromethyl)pyridin-4-yl)amino)-N-methylbenzamide |
| | 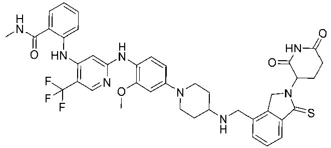 | 2-((2-((4-(4-(((2-(2,6-dioxopiperidin-3-yl)-1-thioxoisoindolin-4-yl)methyl)amino)piperidi n-1-yl)-2-methoxyphenyl)amino)-5-(trifluoromethyl)pyridin-4-yl)amino)-N-methylbenzamide | 2-((2-((4-(4-(((2-(2,6-dioxopiperidin-3-yl)-1-thioxoisoindolin-4-yl)methyl)amino)piperidi n-1-yl)-2-methoxyphenyl)amino)-5-(trifluoromethyl)pyridin-4-yl)amino)-N-methylbenzamide |
| | 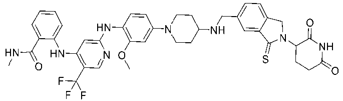 | 2-((2-((4-(4-(((2-(2,6-dioxopiperidin-3-yl)-3-thioxoisoindolin-5-yl)methyl)amino)piperidi n-1-yl)-2-methoxyphenyl)amino)-5-(trifluoromethyl)pyridin-4-yl)amino)-N-methylbenzamide | 2-((2-((4-(4-(((2-(2,6-dioxopiperidin-3-yl)-3-thioxoisoindolin-5-yl)methyl)amino)piperidi n-1-yl)-2-methoxyphenyl)amino)-5-(trifluoromethyl)pyridin-4-yl)amino)-N-methylbenzamide |
| | 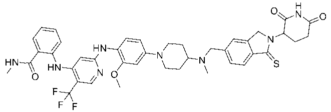 | 2-((2-((4-(4-(((2-(2,6-dioxopiperidin-3-yl)-1-thioxoisoindolin-5-yl)methyl)(methyl)amino )piperidin-1-yl)-2-methoxyphenyl)amino)-5-(trifluoromethyl)pyridin-4-yl)amino)-N-methylbenzamide | 2-((2-((4-(4-(((2-(2,6-dioxopiperidin-3-yl)-1-thioxoisoindolin-5-yl)methyl)(methyl)amino )piperidin-1-yl)-2-methoxyphenyl)amino)-5-(trifluoromethyl)pyridin-4-yl)amino)-N-methylbenzamide |
| | 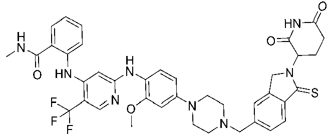 | 2-((2-((4-(4-((2-(2,6-dioxopiperidin-3-yl)-1-thioxoisoindolin-5-yl)methyl)piperazin-1-yl)-2-methoxyphenyl)amino)-5-(trifluoromethyl)pyridin-4-yl)amino)-N-methylbenzamide | 2-((2-((4-(4-((2-(2,6-dioxopiperidin-3-yl)-1-thioxoisoindolin-5-yl)methyl)piperazin-1-yl)-2-methoxyphenyl)amino)-5-(trifluoromethyl)pyridin-4-yl)amino)-N-methylbenzamide |
| | 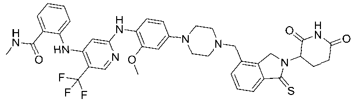 | 2-((2-((4-(4-((2-(2,6-dioxopiperidin-3-yl)-1-thioxoisoindolin-4-yl)methyl)piperazin-1-yl)-2-methoxyphenyl)amino)-5-(trifluoromethyl)pyridin-4-yl)amino)-N-methylbenzamide | 2-((2-((4-(4-((2-(2,6-dioxopiperidin-3-yl)-1-thioxoisoindolin-4-yl)methyl)piperazin-1-yl)-2-methoxyphenyl)amino)-5-(trifluoromethyl)pyridin-4-yl)amino)-N-methylbenzamide |
| | 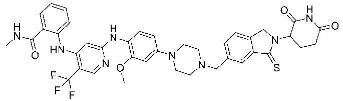 | 2-((2-((4-(4-((2-(2,6-dioxopiperidin-3-yl)-3-thioxoisoindolin-5-yl)methyl)piperazin-1-yl)-2-methoxyphenyl)amino)-5-(trifluoromethyl)pyridin-4-yl)amino)-N-methylbenzamide | 2-((2-((4-(4-((2-(2,6-dioxopiperidin-3-yl)-3-thioxoisoindolin-5-yl)methyl)piperazin-1-yl)-2-methoxyphenyl)amino)-5-(trifluoromethyl)pyridin-4-yl)amino)-N-methylbenzamide |
| GT-0878 2 | 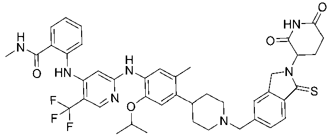 | 2-((2-((4-(1-((2-(2,6-dioxopiperidin-3-yl)-1-thioxoisoindolin-5-yl)methyl)piperidin-4-yl)-2-isopropoxy-5-methylphenyl)amino)-5-(trifluoromethyl)pyridin-4-yl)amino)-N-methylbenzamide | 2-((2-((4-(1-((2-(2,6-dioxopiperidin-3-yl)-1-thioxoisoindolin-5-yl)methyl)piperidin-4-yl)-2-isopropoxy-5-methylphenyl)amino)-5-(trifluoromethyl)pyridin-4-yl)amino)-N-methylbenzamide |
| GT-0888 4 | 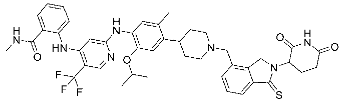 | 2-((2-((4-(1-((2-(2,6-dioxopiperidin-3-yl)-1-thioxoisoindolin-4-yl)methyl)piperidin-4-yl)-2-isopropoxy-5-methylphenyl)amino)-5-(trifluoromethyl)pyridin-4-yl)amino)-N-methylbenzamide | 2-((2-((4-(1-((2-(2,6-dioxopiperidin-3-yl)-1-thioxoisoindolin-4-yl)methyl)piperidin-4-yl)-2-isopropoxy-5-methylphenyl)amino)-5-(trifluoromethyl)pyridin-4-yl)amino)-N-methylbenzamide |
| | 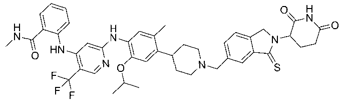 | 2-((2-((4-(1-((2-(2,6-dioxopiperidin-3-yl)-3-thioxoisoindolin-5-yl)methyl)piperidin-4-yl)-2-isopropoxy-5-methylphenyl)amino)-5-(trifluoromethyl)pyridin-4-yl)amino)-N-methylbenzamide | 2-((2-((4-(1-((2-(2,6-dioxopiperidin-3-yl)-3-thioxoisoindolin-5-yl)methyl)piperidin-4-yl)-2-isopropoxy-5-methylphenyl)amino)-5-(trifluoromethyl)pyridin-4-yl)amino)-N-methylbenzamide |
| | 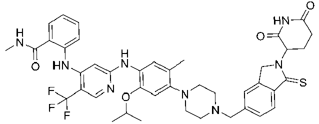 | 2-((2-((4-(4-((2-(2,6-dioxopiperidin-3-yl)-1-thioxoisoindolin-5-yl)methyl)piperazin-1-yl)-2-isopropoxy-5-methylphenyl)amino)-5-(trifluoromethyl)pyridin-4-yl)amino)-N-methylbenzamide | 2-((2-((4-(4-((2-(2,6-dioxopiperidin-3-yl)-1-thioxoisoindolin-5-yl)methyl)piperazin-1-yl)-2-isopropoxy-5-methylphenyl)amino)-5-(trifluoromethyl)pyridin-4-yl)amino)-N-methylbenzamide |
| | 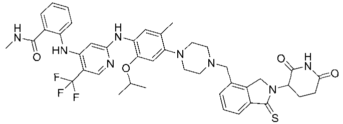 | 2-((2-((4-(4-((2-(2,6-dioxopiperidin-3-yl)-1-thioxoisoindolin-4-yl)methyl)piperazin-1-yl)-2-isopropoxy-5-methylphenyl)amino)-5-(trifluoromethyl)pyridin-4-yl)amino)-N-methylbenzamide | 2-((2-((4-(4-((2-(2,6-dioxopiperidin-3-yl)-1-thioxoisoindolin-4-yl)methyl)piperazin-1-yl)-2-isopropoxy-5-methylphenyl)amino)-5-(trifluoromethyl)pyridin-4-yl)amino)-N-methylbenzamide |
| | 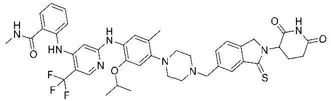 | 2-((2-((4-(4-((2-(2,6-dioxopiperidin-3-yl)-3-thioxoisoindolin-5-yl)methyl)piperazin-1-yl)-2-isopropoxy-5-methylphenyl)amino)-5-(trifluoromethyl)pyridin-4-yl)amino)-N-methylbenzamide | 2-((2-((4-(4-((2-(2,6-dioxopiperidin-3-yl)-3-thioxoisoindolin-5-yl)methyl)piperazin-1-yl)-2-isopropoxy-5-methylphenyl)amino)-5-(trifluoromethyl)pyridin-4-yl)amino)-N-methylbenzamide |
| | 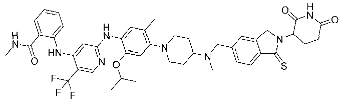 | 2-((2-((4-(4-(((2-(2,6-dioxopiperidin-3-yl)-1-thioxoisoindolin-5-yl)methyl)(methyl)amino )piperidin-1-yl)-2-isopropoxy-5-methylphenyl)amino)-5-(trifluoromethyl)pyridin-4-yl)amino)-N-methylbenzamide | 2-((2-((4-(4-(((2-(2,6-dioxopiperidin-3-yl)-1-thioxoisoindolin-5-yl)methyl)(methyl)amino )piperidin-1-yl)-2-isopropoxy-5-methylphenyl)amino)-5-(trifluoromethyl)pyridin-4-yl)amino)-N-methylbenzamide |
| | 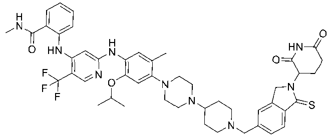 | 2-((2-((4-(4-(1-((2-(2,6-dioxopiperidin-3-yl)-1-thioxoisoindolin-5-yl)methyl)piperidin-4-yl)piperazin-1-yl)-2-isopropoxy-5-methylphenyl)amino)-5-(trifluoromethyl)pyridin-4-yl)amino)-N-methylbenzamide | 2-((2-((4-(4-(1-((2-(2,6-dioxopiperidin-3-yl)-1-thioxoisoindolin-5-yl)methyl)piperidin-4-yl)piperazin-1-yl)-2-isopropoxy-5-methylphenyl)amino)-5-(trifluoromethyl)pyridin-4-yl)amino)-N-methylbenzamide |
| | 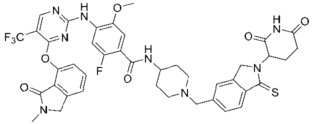 | N-(1-((2-(2,6-dioxopiperidin-3-yl)-1-thioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidi n-2-yl)amino)benzamide | N-(1-((2-(2,6-dioxopiperidin-3-yl)-1-thioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidi n-2-yl)amino)benzamide |
| | 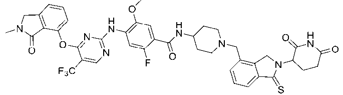 | N-(1-((2-(2,6-dioxopiperidin-3-yl)-1-thioxoisoindolin-4-yl)methyl)piperidin-4-yl)-2-fluoro-5-methoxy-4-((4-((2-methyl-3-oxoisoindolin-4-yl)oxy)-5-(trifluoromethyl)pyrimidi n-2-yl)amino)benzamide | N-(1-((2-(2,6-dioxopiperidin-3-yl)-1-thioxoisoindolin-4-yl)methyl)piperidin-4-yl)-2-fluoro-5-methoxy-4-((4-((2-methyl-3-oxoisoindolin-4-yl)oxy)-*5-*(trifluoromethyl)pyrimidi n-2-yl)amino)benzamide |
| | 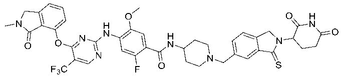 | N-(1-((2-(2,6-dioxopiperidin-3-yl)-3-thioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidi n-2-yl)amino)benzamide | N-(1-((2-(2,6-dioxopiperidin-3-yl)-3-thioxoisoindolin-5-yl)methyl)piperidin-4-yl)-2-fluoro-5-methoxy-4-((4-((2-methyl-3-oxoisoindolin-4-yl)oxy)-*5-*(trifluoromethyl)pyrimidi n-2-yl)amino)benzamide |
| GT-0877 9 | 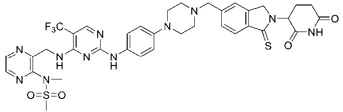 | N-(3-(((2-((4-(4-((2-(2,6-dioxopiperidin-3-yl)-1-thioxoisoindolin-5-yl)methyl)piperazin-1-yl)phenyl)amino)-5-(trifluoromethyl)pyrimidi n-4-yl)amino)methyl)pyrazin -2-yl)-N-methylmethanesulfonamide | N-(3-(((2-((4-(4-((2-(2,6-dioxopiperidin-3-yl)-1-thioxoisoindolin-5-yl)methyl)piperazin-1-yl)phenyl)amino)-5-(trifluoromethyl)pyrimidi n-4-yl)amino)methyl)pyrazin -2-yl)-N-methylmethanesulfonamide |
| GT-0878 3 | 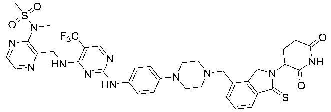 | N-(3-(((2-((4-(4-((2-(2,6-dioxopiperidin-3-yl)-1-thioxoisoindolin-4-yl)methyl)piperazin-1-yl)phenyl)amino)-5-(trifluoromethyl)pyrimidi n-4-yl)amino)methyl)pyrazin -2-yl)-N-methylmethanesulfonamide | N-(3-(((2-((4-(4-((2-(2,6-dioxopiperidin-3-yl)-1-thioxoisoindolin-4-yl)methyl)piperazin-1-yl)phenyl)amino)-5-(trifluoromethyl)pyrimidi n-4-yl)amino)methyl)pyrazin -2-yl)-N-methylmethanesulfonamide |
| GT-0943 7 | 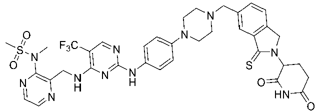 | N-(3-(((2-((4-(4-((2-(2,6-dioxopiperidin-3-yl)-3-thioxoisoindolin-5-yl)methyl)piperazin-1-yl)phenyl)amino)-5-(trifluoromethyl)pyrimidi n-4-yl)amino)methyl)pyrazin -2-yl)-N-methylmethanesulfonamide | N-(3-(((2-((4-(4-((2-(2,6-dioxopiperidin-3-yl)-3-thioxoisoindolin-5-yl)methyl)piperazin-1-yl)phenyl)amino)-5-(trifluoromethyl)pyrimidi n-4-yl)amino)methyl)pyrazin -2-yl)-N-methylmethanesulfonamide |
| | 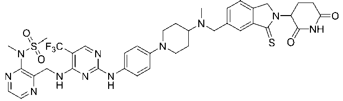 | N-(3-(((2-((4-(4-(((2-(2,6-dioxopiperidin-3-yl)-3-thioxoisoindolin-5-yl)methyl)(methyl)amino )piperidin-1-yl)phenyl)amino)-5-(trifluoromethyl)pyrimidi n-4-yl)amino)methyl)pyrazin -2-yl)-N-methylmethanesulfonamide | N-(3-(((2-((4-(4-(((2-(2,6-dioxopiperidin-3-yl)-3-thioxoisoindolin-5-yl)methyl)(methyl)amino )piperidin-1-yl)phenyl)amino)-5-(trifluoromethyl)pyrimidi n-4-yl)amino)methyl)pyrazin -2-yl)-N-methylmethanesulfonamide |
| | 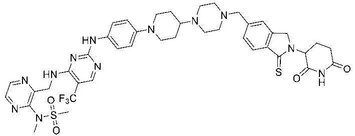 | N-(3-(((2-((4-(4-(4-((2-(2,6-dioxopiperidin-3-yl)-1-thioxoisoindolin-5-yl)methyl)piperazin-1-yl)piperidin-1-yl)phenyl)amino)-5-(trifluoromethyl)pyrimidi n-4-yl)amino)methyl)pyrazin -2-yl)-N-methylmethanesulfonami de | N-(3-(((2-((4-(4-(4-((2-(2,6-dioxopiperidin-3-yl)-1-thioxoisoindolin-5-yl)methyl)piperazin-1-yl)piperidin-1-yl)phenyl)amino)-5-(trifluoromethyl)pyrimidi n-4-yl)amino)methyl)pyrazin -2-yl)-N-methylmethanesulfonami de |
| GT-0878 1 | 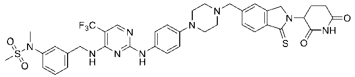 | N-(3-(((2-((4-(4-((2-(2,6-dioxopiperidin-3-yl)-1-thioxoisoindolin-5-yl)methyl)piperazin-1-yl)phenyl)amino)-5-(trifluoromethyl)pyrimidi n-4-yl)amino)methyl)phenyl) -N-methylmethanesulfonamide | N-(3-(((2-((4-(4-((2-(2,6-dioxopiperidin-3-yl)-1-thioxoisoindolin-5-yl)methyl)piperazin-1-yl)phenyl)amino)-5-(trifluoromethyl)pyrimidi n-4-yl)amino)methyl)phenyl) -N-methylmethanesulfonamide |
| GT-0888 3 | 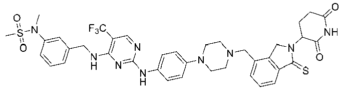 | N-(3-(((2-((4-(4-((2-(2,6-dioxopiperidin-3-yl)-1-thioxoisoindolin-4-yl)methyl)piperazin-1-yl)phenyl)amino)-5-(trifluoromethyl)pyrimidi n-4-yl)amino)methyl)phenyl) -N-methylmethanesulfonamide | N-(3-(((2-((4-(4-((2-(2,6-dioxopiperidin-3-yl)-1-thioxoisoindolin-4-yl)methyl)piperazin-1-yl)phenyl)amino)-5-(trifluoromethyl)pyrimidi n-4-yl)amino)methyl)phenyl) -N-methylmethanesulfonamide |
| GT-0943 8 | 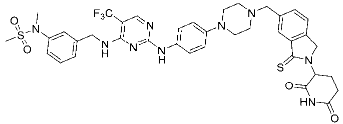 | N-(3-(((2-((4-(4-((2-(2,6-dioxopiperidin-3-yl)-3-thioxoisoindolin-5-yl)methyl)piperazin-1-yl)phenyl)amino)-5-(trifluoromethyl)pyrimidi n-4-yl)amino)methyl)phenyl) -N-methylmethanesulfonamide | N-(3-(((2-((4-(4-((2-(2,6-dioxopiperidin-3-yl)-3-thioxoisoindolin-5-yl)methyl)piperazin-1-yl)phenyl)amino)-5-(trifluoromethyl)pyrimidi n-4-yl)amino)methyl)phenyl) -N-methylmethanesulfonamide |
| | 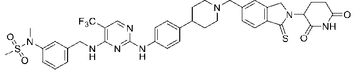 | N-(3-(((2-((4-(1-((2-(2,6-dioxopiperidin-3-yl)-1-thioxoisoindolin-5-yl)methyl)piperidin-4-yl)phenyl)amino)-5-(trifluoromethyl)pyrimidi n-4-yl)amino)methyl)phenyl) -N-methylmethanesulfonamide | N-(3-(((2-((4-(1-((2-(2,6-dioxopiperidin-3-yl)-1-thioxoisoindolin-5-yl)methyl)piperidin-4-yl)phenyl)amino)-5-(trifluoromethyl)pyrimidi n-4-yl)amino)methyl)phenyl) -N-methylmethanesulfonamide |
| | 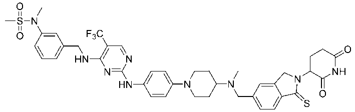 | N-(3-(((2-((4-(4-(((2-(2,6-dioxopiperidin-3-yl)-1-thioxoisoindolin-5-yl)methyl)(methyl)amino )piperidin-1-yl)phenyl)amino)-5-(trifluoromethyl)pyrimidi n-4-yl)amino)methyl)phenyl) -N-methylmethanesulfonamide | N-(3-(((2-((4-(4-(((2-(2,6-dioxopiperidin-3-yl)-1-thioxoisoindolin-5-yl)methyl)(methyl)amino )piperidin-1-yl)phenyl)amino)-5-(trifluoromethyl)pyrimidi n-4-yl)amino)methyl)phenyl) -N-methylmethanesulfonamide |
| | 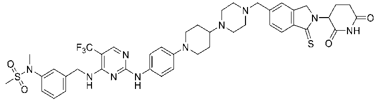 | N-(3-(((2-((4-(4-(4-((2-(2,6-dioxopiperidin-3-yl)-1-thioxoisoindolin-5-yl)methyl)piperazin-1-yl)piperidin-1-yl)phenyl)amino)-5-(trifluoromethyl)pyrimidi n-4-yl)amino)methyl)phenyl) -N-methylmethanesulfonamide | N-(3-(((2-((4-(4-(4-((2-(2,6-dioxopiperidin-3-yl)-1-thioxoisoindolin-5-yl)methyl)piperazin-1-yl)piperidin-1-yl)phenyl)amino)-5-(trifluoromethyl)pyrimidi n-4-yl)amino)methyl)phenyl) -N-methylmethanesulfonamide |
| GT-08780 | 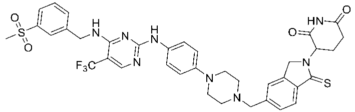 | 3-(5-((4-(4-((4-((3-(methylsulfonyl)benzyl)a mino)-5-(trifluoromethyl)pyrimidi n-2-yl)amino)phenyl)piperazi n-1-yl)methyl)-1-thioxoisoindolin-2-yl)piperidine-2,6-dione | 3-(5-((4-(4-((4-((3-(methylsulfonyl)benzyl)a mino)-5-(trifluoromethyl)pyrimidi n-2-yl)amino)phenyl)piperazi n-1-yl)methyl)-1-thioxoisoindolin-2-yl)piperidine-2,6-dione |
| GT-05286 | 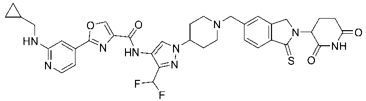 | 2-(2-((cyclopropylmethyl)ami no)pyridin-4-yl)-N-(3-(difluoromethyl)-1-(1-((2-(2,6-dioxopiperidin-3-yl)-1-thioxoisoindolin-5-yl)methyl)piperidin-4-yl)-1H-pyrazol-4-yl)oxazole-4-carboxamide | 2-(2-((cyclopropylmethyl)ami no)pyridin-4-yl)-N-(3-(difluoromethyl)-1-(1-((2-(2,6-dioxopiperidin-3-yl)-1-thioxoisoindolin-5-yl)methyl)piperidin-4-yl)-1H-pyrazol-4-yl)oxazole-4-carboxamide |
| | 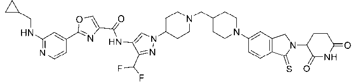 | 2-(2-((cyclopropylmethyl)ami no)pyridin-4-yl)-N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)oxazole-4-carboxamide | 2-(2-((cyclopropylmethyl)ami no)pyridin-4-yl)-N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)oxazole-4-carboxamide |
| | 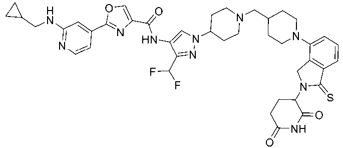 | 2-(2-((cyclopropylmethyl)ami no)pyridin-4-yl)-N-(3-(difluoromethyl)-1-(1-((1-(2-(2,6-dioxopiperidin-3-yl)-1-thioxoisoindolin-4-yl)piperidin-4-yl)methyl)piperidin-4-yl)-1H-pyrazol-4-yl)oxazole-4-carboxamide | 2-(2-((cyclopropylmethyl)ami no)pyridin-4-yl)-N-(3-(difluoromethyl)-1-(1-((1-(2-(2,6-dioxopiperidin-3-yl)-1-thioxoisoindolin-4-yl)piperidin-4-yl)methyl)piperidin-4-yl)-1H-pyrazol-4-yl)oxazole-4-carboxamide |
| | 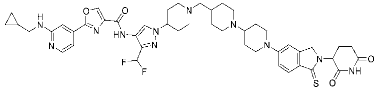 | 2-(2-((cyclopropylmethyl)ami no)pyridin-4-yl)-N-(3-(difluoromethyl)-1-(1-((1'-(2-(2,6-dioxopiperidin-3-yl)-1-thioxoisoindolin-5-yl)-[1,4'-bipiperidin]-4-yl)methyl)piperidin-4-yl)-1H-pyrazol-4-yl)oxazole-4-carboxamide | 2-(2-((cyclopropylmethyl)ami no)pyridin-4-yl)-N-(3-(difluoromethyl)-1-(1-((1'-(2-(2,6-dioxopiperidin-3-yl)-1-thioxoisoindolin-5-yl)-[1,4'-bipiperidin]-4-yl)methyl)piperidin-4-yl)-1H-pyrazol-4-yl)oxazole-4-carboxamide |
| | 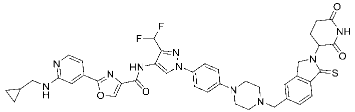 | 2-(2-((cyclopropylmethyl)ami no)pyridin-4-yl)-N-(3-(difluoromethyl)-1-(4-(4-((2-(2,6-dioxopiperidin-3-yl)-1-thioxoisoindolin-5-yl)methyl)piperazin-1-yl)phenyl)-1H-pyrazol-4-yl)oxazole-4-carboxamide | 2-(2-((cyclopropylmethyl)ami no)pyridin-4-yl)-N-(3-(difluoromethyl)-1-(4-(4-((2-(2,6-dioxopiperidin-3-yl)-1-thioxoisoindolin-5-yl)methyl)piperazin-1-yl)phenyl)-1H-pyrazol-4-yl)oxazole-4-carboxamide |
| GT-05295 | 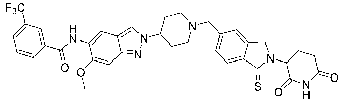 | N-(2-(1-((2-(2,6-dioxopiperidin-3-yl)-1-thioxoisoindolin-5-yl)methyl)piperidin-4-yl)-6-methoxy-2H-indazol-5-yl)-3-(trifluoromethyl)benzamide | N-(2-(1-((2-(2,6-dioxopiperidin-3-yl)-1-thioxoisoindolin-5-yl)methyl)piperidin-4-yl)-6-methoxy-2H-indazol-5-yl)-3-(trifluoromethyl)benzamide |
| GT-09414 | 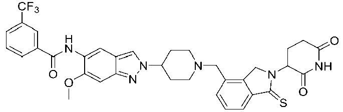 | N-(2-(1-((2-(2,6-dioxopiperidin-3-yl)-1-thioxoisoindolin-4-yl)methyl)piperidin-4-yl)-6-methoxy-2H-indazol-5-yl)-3-(trifluoromethyl)benzamide | N-(2-(1-((2-(2,6-dioxopiperidin-3-yl)-1-thioxoisoindolin-4-yl)methyl)piperidin-4-yl)-6-methoxy-2H-indazol-5-yl)-3-(trifluoromethyl)benzamide |
| | 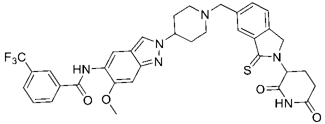 | N-(2-(1-((2-(2,6-dioxopiperidin-3-yl)-3-thioxoisoindolin-5-yl)methyl)piperidin-4-yl)-6-methoxy-2H-indazol-5-yl)-3-(trifluoromethyl)benzamide | N-(2-(1-((2-(2,6-dioxopiperidin-3-yl)-3-thioxoisoindolin-5-yl)methyl)piperidin-4-yl)-6-methoxy-2H-indazol-5-yl)-3-(trifluoromethyl)benzamide |
| | 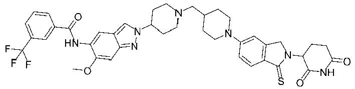 | N-(2-(1-((1-(2-(2,6-dioxopiperidin-3-yl)-1-thioxoisoindolin-5-yl)piperidin-4-yl)methyl)piperidin-4-yl)-6-methoxy-2H-indazol-5-yl)-3-(trifluoromethyl)benzamide | N-(2-(1-((1-(2-(2,6-dioxopiperidin-3-yl)-1-thioxoisoindolin-5-yl)piperidin-4-yl)methyl)piperidin-4-yl)-6-methoxy-2H-indazol-5-yl)-3-(trifluoromethyl)benzamide |
| GT-05296 | 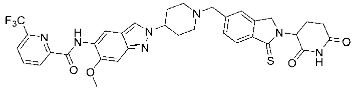 | N-(2-(1-((2-(2,6-dioxopiperidin-3-yl)-1-thioxoisoindolin-5-yl)methyl)piperidin-4-yl)-6-methoxy-2H-indazol-5-yl)-6-(trifluoromethyl)picolina mide | N-(2-(1-((2-(2,6-dioxopiperidin-3-yl)-1-thioxoisoindolin-5-yl)methyl)piperidin-4-yl)-6-methoxy-2H-indazol-5-yl)-6-(trifluoromethyl)picolina mide |
| GT-04950 | 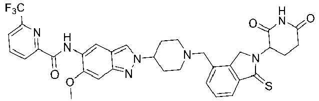 | N-(2-(1-((2-(2,6-dioxopiperidin-3-yl)-1-thioxoisoindolin-4-yl)methyl)piperidin-4-yl)-6-methoxy-2H-indazol-5-yl)-6-(trifluoromethyl)picolina mide | N-(2-(1-((2-(2,6-dioxopiperidin-3-yl)-1-thioxoisoindolin-4-yl)methyl)piperidin-4-yl)-6-methoxy-2H-indazol-5-yl)-6-(trifluoromethyl)picolina mide |
| | 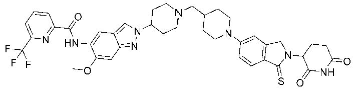 | N-(2-(1-((1-(2-(2,6-dioxopiperidin-3-yl)-1-thioxoisoindolin-5-yl)piperidin-4-yl)methyl)piperidin-4-yl)-6-methoxy-2H-indazol-5-yl)-6-(trifluoromethyl)picolinamide | N-(2-(1-((1-(2-(2,6-dioxopiperidin-3-yl)-1-thioxoisoindolin-5-yl)piperidin-4-yl)methyl)piperidin-4-yl)-6-methoxy-2H-indazol-5-yl)-6-(trifluoromethyl)picolinamide |
| GT-09413 | 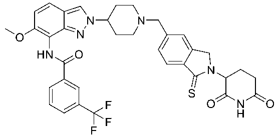 | N-(2-(1-((2-(2,6-dioxopiperidin-3-yl)-1-thioxoisoindolin-5-yl)methyl)piperidin-4-yl)-6-methoxy-2H-indazol-7-yl)-3-(trifluoromethyl)benzamide | N-(2-(1-((2-(2,6-dioxopiperidin-3-yl)-1-thioxoisoindolin-5-yl)methyl)piperidin-4-yl)-6-methoxy-2H-indazol-7-yl)-3-(trifluoromethyl)benzamide |
| GT-05098 | 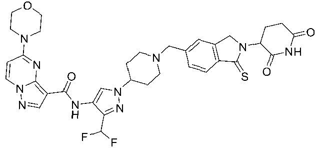 | N-(3-(difluoromethyl)-1-(1-((2-(2,6-dioxopiperidin-3-yl)-1-thioxoisoindolin-5-yl)methyl)piperidin-4-yl)-1H-pyrazol-4-yl)-5-morpholinopyrazolo[1,5-a]pyrimidine-3-carboxamide | N-(3-(difluoromethyl)-1-(1-((2-(2,6-dioxopiperidin-3-yl)-1-thioxoisoindolin-5-yl)methyl)piperidin-4-yl)-1H-pyrazol-4-yl)-5-morpholinopyrazolo[1,5-a]pyrimidine-3-carboxamide |
| GT-05287 | 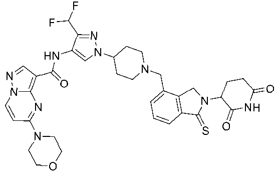 | N-(3-(difluoromethyl)-1-(1-((2-(2,6-dioxopiperidin-3-yl)-1-thioxoisoindolin-4-yl)methyl)piperidin-4-yl)-1H-pyrazol-4-yl)-5-morpholinopyrazolo[1,5-a]pyrimidine-3-carboxamide | N-(3-(difluoromethyl)-1-(1-((2-(2,6-dioxopiperidin-3-yl)-1-thioxoisoindolin-4-yl)methyl)piperidin-4-yl)-1H-pyrazol-4-yl)-5-morpholinopyrazolo[1,5-a]pyrimidine-3-carboxamide |
| GT-05321 | 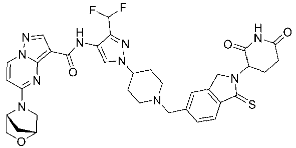 | 5-((1R,4R)-2-oxa-5-azabicyclo[2.2.1]heptan-5-yl)-N-(3-(difluoromethyl)-1-(1-((2-(2,6-dioxopiperidin-3-yl)-1-thioxoisoindolin-5-yl)methyl)piperidin-4-yl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-(1-((2-(2,6-dioxopiperidin-3-yl)-1-thioxoisoindolin-5-yl)methyl)piperidin-4-yl)-1H-pyrazol-4-yl)pyrazolo[1,5-a]pyrimidine-3-carboxamide |
| GT-0528 8 | 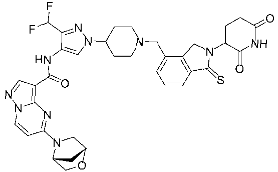 | 5-((1R,4R)-2-oxa-5-azabicyclo[2.2.1]heptan-5-yl)-N-(3-(difluoromethyl)-1-(1-((2-(2,6-dioxopiperidin-3-yl)-1-thioxoisoindolin-4-yl)methyl)piperidin-4-yl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-(1-((2-(2,6-dioxopiperidin-3-yl)-1-thioxoisoindolin-4-yl)methyl)piperidin-4-yl)-1H-pyrazol-4-yl)pyrazolo[1,5-a]pyrimidine-3-carboxamide |
| | 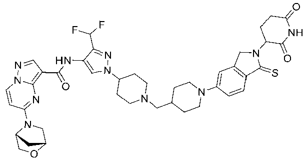 | 5-((1R,4R)-2-oxa-5-azabicyclo[2.2.1]heptan-5-yl)-N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)pyrazolo[1,5-a]pyrimidine-3-carboxamide |
| | 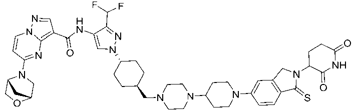 | 5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-1-thioxoisoindolin-5-yl)piperidin-4-yl)piperazin-1-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-1-thioxoisoindolin-5-yl)piperidin-4-yl)piperazin-1-yl)methyl)cyclohexyl)-1H-pyrazol-4-yl)pyrazolo[1,5-a]pyrimidine-3-carboxamide |
| | 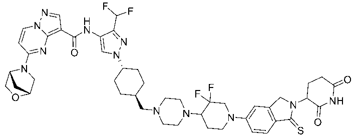 | 5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-1-thioxoisoindolin-5-yl)-3,3-difluoropiperidin-4-yl)piperazin-1-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-1-thioxoisoindolin-5-yl)-3,3-difluoropiperidin-4-yl)piperazin-1-yl)methyl)cyclohexyl)-1H-pyrazol-4-yl)pyrazolo[1,5-a]pyrimidine-3-carboxamide |
| | 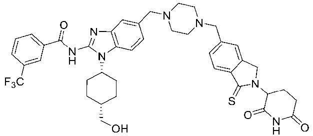 | N-(5-((4-((2-(2,6-dioxopiperidin-3-yl)-1-thioxoisoindolin-5-yl)methyl)piperazin-1-yl)methyl)-1-((1s,4s)-4-(hydroxymethyl)cyclohe xyl)-1H-benzo[d]imidazol-2-yl)-3-(trifluoromethyl)benzamide | N-(5-((4-((2-(2,6-dioxopiperidin-3-yl)-1-thioxoisoindolin-5-yl)methyl)piperazin-1-yl)methyl)-1-((1s,4s)-4-(hydroxymethyl)cyclohe xyl)-1H-benzo[d]imidazol-2-yl)-3-(trifluoromethyl)benzamide |
| GT-05293 | 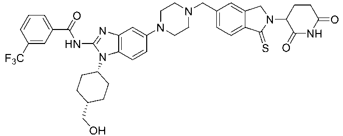 | N-(5-(4-((2-(2,6-dioxopiperidin-3-yl)-1-thioxoisoindolin-5-yl)methyl)piperazin-1-yl)-1-((1s,4s)-4-(hydroxymethyl)cyclohe xyl)-1H-benzo[d]imidazol-2-yl)-3-(trifluoromethyl)benzamide | N-(5-(4-((2-(2,6-dioxopiperidin-3-yl)-1-thioxoisoindolin-5-yl)methyl)piperazin-1-yl)-1-((1s,4s)-4-(hydroxymethyl)cyclohe xyl)-1H-benzo[d]imidazol-2-yl)-3-(trifluoromethyl)benzamide |
| | 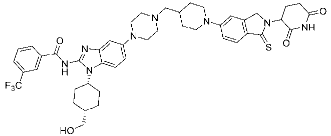 | N-(5-(4-((1-(2-(2,6-dioxopiperidin-3-yl)-1-thioxoisoindolin-5-yl)piperidin-4-yl)methyl)piperazin-1-yl)-1-((1s,4s)-4-(hydroxymethyl)cyclohe xyl)-1H-benzo[d]imidazol-2-yl)-3-(trifluoromethyl)benzamide | N-(5-(4-((1-(2-(2,6-dioxopiperidin-3-yl)-1-thioxoisoindolin-5-yl)piperidin-4-yl)methyl)piperazin-1-yl)-1-((1s,4s)-4-(hydroxymethyl)cyclohe xyl)-1H-benzo[d]imidazol-2-yl)-3-(trifluoromethyl)benzamide |
| | 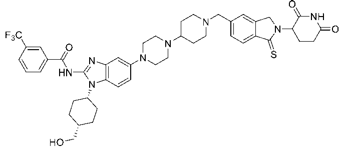 | N-(5-(4-(1-((2-(2,6-dioxopiperidin-3-yl)-1-thioxoisoindolin-5-yl)methyl)piperidin-4-yl)piperazin-1-yl)-1-((1s,4s)-4-(hydroxymethyl)cyclohe xyl)-1H-benzo[d]imidazol-2-yl)-3-(trifluoromethyl)benzamide | N-(5-(4-(1-((2-(2,6-dioxopiperidin-3-yl)-1-thioxoisoindolin-5-yl)methyl)piperidin-4-yl)piperazin-1-yl)-1-((1s,4s)-4-(hydroxymethyl)cyclohe xyl)-1H-benzo[d]imidazol-2-yl)-3-(trifluoromethyl)benzamide |
| GT-05294 | 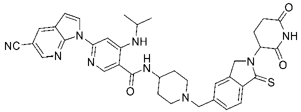 | 6-(5-cyano-1H-pyrrolo[2,3-b]pyridin-1-yl)-N-(1-((2-(2,6-dioxopiperidin-3-yl)-1-thioxoisoindolin-5-yl)methyl)piperidin-4-yl)-4-(isopropylamino)nicotinamide | 6-(5-cyano-1H-pyrrolo[2,3-b]pyridin-1-yl)-N-(1-((2-(2,6-dioxopiperidin-3-yl)-1-thioxoisoindolin-5-yl)methyl)piperidin-4-yl)-4-(isopropylamino)nicotinamide |
| GT-05292 | 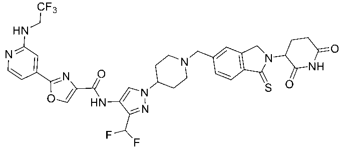 | N-(3-(difluoromethyl)-1-(1-((2-(2,6-dioxopiperidin-3-yl)-1-thioxoisoindolin-5-yl)methyl)piperidin-4-yl)-1H-pyrazol-4-yl)-2-(2-((2,2,2-trifluoroethyl)amino)pyri din-4-yl)oxazole-4-carboxamide | N-(3-(difluoromethyl)-1-(1-((2-(2,6-dioxopiperidin-3-yl)-1-thioxoisoindolin-5-yl)methyl)piperidin-4-yl)-1H-pyrazol-4-yl)-2-(2-((2,2,2-trifluoroethyl)amino)pyri din-4-yl)oxazole-4-carboxamide |
| | 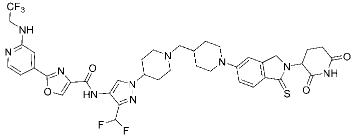 | N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)-2-(2-((2,2,2-trifluoroethyl)amino)pyri din-4-yl)oxazole-4-carboxamide | N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)-2-(2-((2,2,2-trifluoroethyl)amino)pyri din-4-yl)oxazole-4-carboxamide |
| | 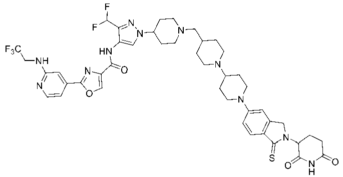 | N-(3-(difluoromethyl)-1-(1-((1'-(2-(2,6-dioxopiperidin-3-yl)-1-thioxoisoindolin-5-yl)-[1,4'-bipiperidin]-4-yl)methyl)piperidin-4-yl)-1H-pyrazol-4-yl)-2-(2-((2,2,2-trifluoroethyl)amino)pyri din-4-yl)oxazole-4-carboxamide | N-(3-(difluoromethyl)-1-(1-((1'-(2-(2,6-dioxopiperidin-3-yl)-1-thioxoisoindolin-5-yl)-[1,4'-bipiperidin]-4-yl)methyl)piperidin-4-yl)-1H-pyrazol-4-yl)-2-(2-((2,2,2-trifluoroethyl)amino)pyri din-4-yl)oxazole-4-carboxamide |
| | 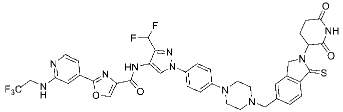 | N-(3-(difluoromethyl)-1-(4-(4-((2-(2,6-dioxopiperidin-3-yl)-1-thioxoisoindolin-5-yl)methyl)piperazin-1-yl)phenyl)-1H-pyrazol-4-yl)-2-(2-((2,2,2-trifluoroethyl)amino)pyri din-4-yl)oxazole-4-carboxamide | N-(3-(difluoromethyl)-1-(4-(4-((2-(2,6-dioxopiperidin-3-yl)-1-thioxoisoindolin-5-yl)methyl)piperazin-1-yl)phenyl)-1H-pyrazol-4-yl)-2-(2-((2,2,2-trifluoroethyl)amino)pyri din-4-yl)oxazole-4-carboxamide |
| GT-05322 | 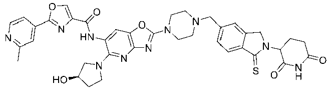 | N-(2-(4-((2-(2,6-dioxopiperidin-3-yl)-1-thioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide | N-(2-(4-((2-(2,6-dioxopiperidin-3-yl)-1-thioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide |
| GT-05289 | 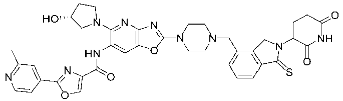 | N-(2-(4-((2-(2,6-dioxopiperidin-3-yl)-1-thioxoisoindolin-4-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide | N-(2-(4-((2-(2,6-dioxopiperidin-3-yl)-1-thioxoisoindolin-4-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide |
| GT-05298 | 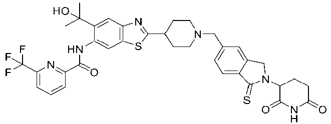 | N-(2-(1-((2-(2,6-dioxopiperidin-3-yl)-1-thioxoisoindolin-5-yl)methyl)piperidin-4-yl)-5-(2-hydroxypropan-2-yl)benzo[d]thiazol-6-yl)-6-(trifluoromethyl)picolina mide | N-(2-(1-((2-(2,6-dioxopiperidin-3-yl)-1-thioxoisoindolin-5-yl)methyl)piperidin-4-yl)-5-(2-hydroxypropan-2-yl)benzo[d]thiazol-6-yl)-6-(trifluoromethyl)picolina mide |
| GT-05320 | 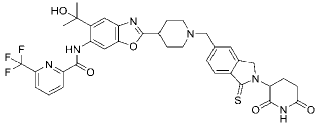 | N-(2-(1-((2-(2,6-dioxopiperidin-3-yl)-1-thioxoisoindolin-5-yl)methyl)piperidin-4-yl)-5-(2-hydroxypropan-2-yl)benzo[d]oxazol-6-yl)-6-(trifluoromethyl)picolina mide | N-(2-(1-((2-(2,6-dioxopiperidin-3-yl)-1-thioxoisoindolin-5-yl)methyl)piperidin-4-yl)-5-(2-hydroxypropan-2-yl)benzo[d]oxazol-6-yl)-6-(trifluoromethyl)picolina mide |
| GT-05297 | 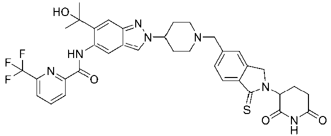 | N-(2-(1-((2-(2,6-dioxopiperidin-3-yl)-1-thioxoisoindolin-5-yl)methyl)piperidin-4-yl)-6-(2-hydroxypropan-2-yl)-2H-indazol-5-yl)-6-(trifluoromethyl)picolina mide | N-(2-(1-((2-(2,6-dioxopiperidin-3-yl)-1-thioxoisoindolin-5-yl)methyl)piperidin-4-yl)-6-(2-hydroxypropan-2-yl)-2H-indazol-5-yl)-6-(trifluoromethyl)picolina mide |
| GT-0888 5 | 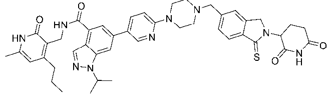 | 6-(6-(4-((2-(2,6-dioxopiperidin-3-yl)-1-thioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide | 6-(6-(4-((2-(2,6-dioxopiperidin-3-yl)-1-thioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide |
| | 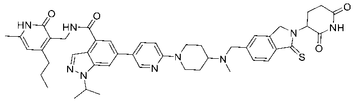 | 6-(6-(4-(((2-(2,6-dioxopiperidin-3-yl)-1-thioxoisoindolin-5-yl)methyl)(methyl)amino )piperidin-1-yl)pyridin-3-yl)-1-isopropyl-N-((6-methyl-2-oxo-4-propyl-1,2-dihydropyridin-3-yl)methyl)-1H-indazole-4-carboxamide | 6-(6-(4-(((2-(2,6-dioxopiperidin-3-yl)-1-thioxoisoindolin-5-yl)methyl)(methyl)amino )piperidin-1-yl)pyridin-3-yl)-1-isopropyl-N-((6-methyl-2-oxo-4-propyl-1,2-dihydropyridin-3-yl)methyl)-1H-indazole-4-carboxamide |
| | 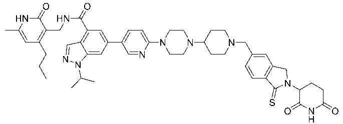 | 6-(6-(4-(1-((2-(2,6-dioxopiperidin-3-yl)-1-thioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide | 6-(6-(4-(1-((2-(2,6-dioxopiperidin-3-yl)-1-thioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide |
| GT-0888 6 | 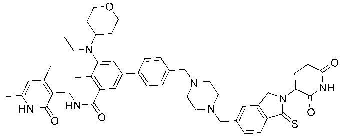 | N-((4,6-dimethyl-2-oxo-1,2-dihydropyridin-3-yl)methyl)-4'-((4-((2-(2,6-dioxopiperidin-3-yl)-1-thioxoisoindolin-5-yl)methyl)piperazin-1-yl)methyl)-5-(ethyl(tetrahydro-2H-pyran-4-yl)amino)-4-methyl-[1,1-biphenyl]-3-carboxamide | N-((4,6-dimethyl-2-oxo-1,2-dihydropyridin-3-yl)methyl)-4'-((4-((2-(2,6-dioxopiperidin-3-yl)-1-thioxoisoindolin-5-yl)methyl)piperazin-1-yl)methyl)-5-(ethyl(tetrahydro-2H-pyran-4-yl)amino)-4-methyl-[1,1-biphenyl]-3-carboxamide |
| GT-0888 7 | 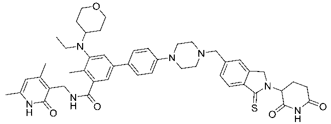 | N-((4,6-dimethyl-2-oxo-1,2-dihydropyridin-3-yl)methyl)-4'-(4-((2-(2,6-dioxopiperidin-3-yl)-1-thioxoisoindolin-5-yl)methyl)piperazin-1-yl)-5-(ethyl(tetrahydro-2H-pyran-4-yl)amino)-4-methyl-[1,1-biphenyl]-3-carboxamide | N-((4,6-dimethyl-2-oxo-1,2-dihydropyridin-3-yl)methyl)-4'-(4-((2-(2,6-dioxopiperidin-3-yl)-1-thioxoisoindolin-5-yl)methyl)piperazin-1-yl)-5-(ethyl(tetrahydro-2H-pyran-4-yl)amino)-4-methyl-[1,1-biphenyl]-3-carboxamide |
| | 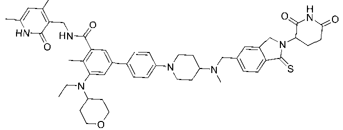 | N-((4,6-dimethyl-2-oxo-1,2-dihydropyridin-3-yl)methyl)-4'-(4-(((2-(2,6-dioxopiperidin-3-yl)-1-thioxoisoindolin-5-yl)methyl)(methyl)amino )piperidin-1-yl)-5-(ethyl(tetrahydro-2H-pyran-4-yl)amino)-4-methyl-[1,1'-biphenyl]-3-carboxamide | N-((4,6-dimethyl-2-oxo-1,2-dihydropyridin-3-yl)methyl)-4'-(4-(((2-(2,6-dioxopiperidin-3-yl)-1-thioxoisoindolin-5-yl)methyl)(methyl)amino )piperidin-1-yl)-5-(ethyl(tetrahydro-2H-pyran-4-yl)amino)-4-methyl-[1,1'-biphenyl]-3-carboxamide |
| | 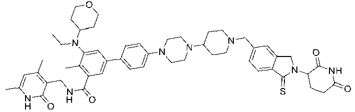 | N-((4,6-dimethyl-2-oxo-1,2-dihydropyridin-3-yl)methyl)-4'-(4-(1-((2-(2,6-dioxopiperidin-3-yl)-1-thioxoisoindolin-5-yl)methyl)piperidin-4-yl)piperazin-1-yl)-5-(ethyl(tetrahydro-2H-pyran-4-yl)amino)-4-methyl-[1,1'-biphenyl]-3-carboxamide | N-((4,6-dimethyl-2-oxo-1,2-dihydropyridin-3-yl)methyl)-4'-(4-(1-((2-(2,6-dioxopiperidin-3-yl)-1-thioxoisoindolin-5-yl)methyl)piperidin-4-yl)piperazin-1-yl)-5-(ethyl(tetrahydro-2H-pyran-4-yl)amino)-4-methyl-[1,1'-biphenyl]-3-carboxamide |
| | 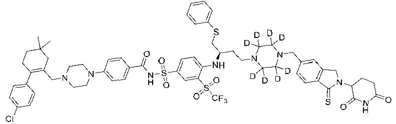 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)piperazin-1-yl-2,2,3,3,5,5,6,6-d8)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)piperazin-1-yl-2,2,3,3,5,5,6,6-d8)-1-(phenylthio)butan-2-yl)amino)-3-((trifluoromethyl)sulfony l)phenyl)sulfonyl)benza mide |
| | 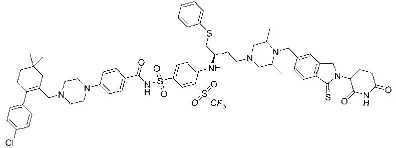 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)-3,5-dimethylpiperazin-1-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)-3,5-dimethylpiperazin-1-yl)-1-(phenylthio)butan-2-yl)amino)-3-((trifluoromethyl)sulfony l)phenyl)sulfonyl)benza mide |
| | 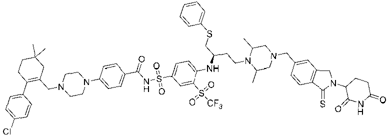 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)-2,6-dimethylpiperazin-1-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)-2,6-dimethylpiperazin-1-yl)-1-(phenylthio)butan-2-yl)amino)-3-((trifluoromethyl)sulfony l)phenyl)sulfonyl)benza mide |
| | 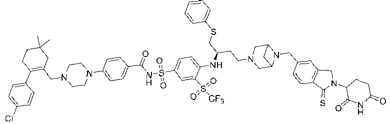 | 4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-1-thioxoisoindolin-5-yl)methyl)-3,6-diazabicyclo[3.1.1]hepta n-3-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-1-thioxoisoindolin-5-yl)methyl)-3,6-diazabicyclo[3.1.1]hepta n-3-yl)-1-(phenylthio)butan-2-yl)amino)-3-((trifluoromethyl)sulfony l)phenyl)sulfonyl)benza mide |
| | 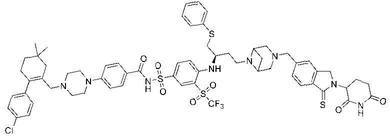 | 4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-thioxoisoindolin-5-yl)methyl)-3,6-diazabicyclo[3.1.1]hepta n-6-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-thioxoisoindolin-5-yl)methyl)-3,6-diazabicyclo[3.1.1]hepta n-6-yl)-1-(phenylthio)butan-2-yl)amino)-3-((trifluoromethyl)sulfony l)phenyl)sulfonyl)benza mide |
| GT-0941 6 | 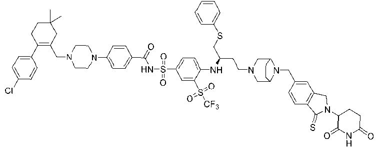 | 4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1-thioxoisoindolin-5-yl)methyl)-3,8-diazabicyclo[3.2.1]octan-3-yl)-1-(phenylthio)butan-2-yl)amino)-3-((trifluoromethyl)sulfony l)phenyl)sulfonyl)benzamide | 4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1-thioxoisoindolin-5-yl)methyl)-3,8-diazabicyclo[3.2.1]octan-3-yl)-1-(phenylthio)butan-2-yl)amino)-3-((trifluoromethyl)sulfony l)phenyl)sulfonyl)benzamide |
| | 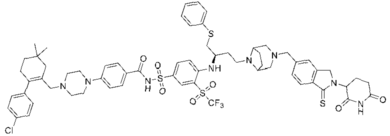 | 4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-thioxoisoindolin-5-yl)methyl)-3,8-diazabicyclo[3.2.1]octan-8-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-thioxoisoindolin-5-yl)methyl)-3,8-diazabicyclo[3.2.1]octan-8-yl)-1-(phenylthio)butan-2-yl)amino)-3-((trifluoromethyl)sulfony l)phenyl)sulfonyl)benza mide |
| | 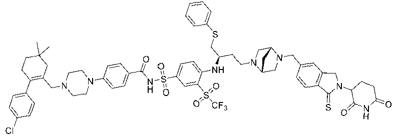 | 4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-1-thioxoisoindolin-5-yl)methyl)-2,5-diazabicyclo[2.2.1]hepta n-2-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-1-thioxoisoindolin-5-yl)methyl)-2,5-diazabicyclo[2.2.1]hepta n-2-yl)-1-(phenylthio)butan-2-yl)amino)-3-((trifluoromethyl)sulfony l)phenyl)sulfonyl)benza mide |
| | 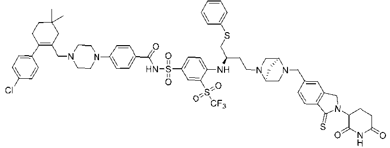 | 4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-1-thioxoisoindolin-5-yl)methyl)-2,5-diazabicyclo[2.2.1]hepta n-2-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-1-thioxoisoindolin-5-yl)methyl)-2,5-diazabicyclo[2.2.1]hepta n-2-yl)-1-(phenylthio)butan-2-yl)amino)-3-((trifluoromethyl)sulfony l)phenyl)sulfonyl)benza mide |
| | 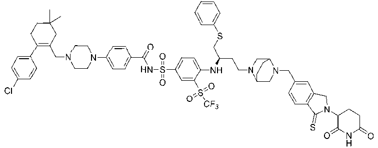 | 4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-1-thioxoisoindolin-5-yl)methyl)-2,5-diazabicyclo[2.2.2]octan-2-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-1-thioxoisoindolin-5-yl)methyl)-2,5-diazabicyclo[2.2.2]octan-2-yl)-1-(phenylthio)butan-2-yl)amino)-3-((trifluoromethyl)sulfony l)phenyl)sulfonyl)benza mide |
| GT-0941 5 | 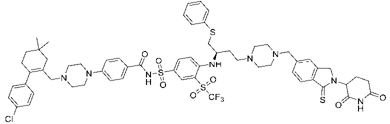 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)piperazin-1-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)piperazin-1-yl)-1-(phenylthio)butan-2-yl)amino)-3-((trifluoromethyl)sulfony l)phenyl)sulfonyl)benza mide |
| | 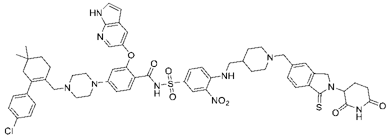 | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-(((1-((2-(2,6-dioxopiperidin-3-yl)-1-thioxoisoindolin-5-yl)methyl)piperidin-4-yl)methyl)amino)-3-nitrophenyl)sulfonyl)ben zamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-(((1-((2-(2,6-dioxopiperidin-3-yl)-1-thioxoisoindolin-5-yl)methyl)piperidin-4-yl)methyl)amino)-3-nitrophenyl)sulfonyl)ben zamide |
| | 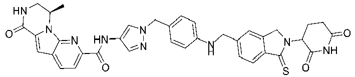 | (9R)-N-(1-(4-(((2-(2,6-dioxopiperidin-3-yl)-1-thioxoisoindolin-5-yl)methyl)amino)benzyl) -1H-pyrazol-4-yl)-9-methyl-6-oxo-6,7,8,9-tetrahydropyrido[3',2':4,5 ]pyrrolo[1,2-a]pyrazine-2-carboxamide | (9R)-N-(1-(4-(((2-(2,6-dioxopiperidin-3-yl)-1-thioxoisoindolin-5-yl)methyl)amino)benzyl) -1H-pyrazol-4-yl)-9-methyl-6-oxo-6,7,8,9-tetrahydropyrido[3',2':4,5 ]pyrrolo[1,2-a]pyrazine-2-carboxamide |
| GT-0877 7 | 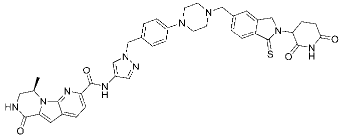 | (9R)-N-(1-(4-(4-((2-(2,6-dioxopiperidin-3-yl)-1-thioxoisoindolin-5-yl)methyl)piperazin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide | (9R)-N-(1-(4-(4-((2-(2,6-dioxopiperidin-3-yl)-1-thioxoisoindolin-5-yl)methyl)piperazin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide |
| | 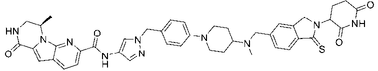 | (9R)-N-(1-(4-(4-(((2-(2,6-dioxopiperidin-3-yl)-1-thioxoisoindolin-5-yl)methyl)(methyl)amino )piperidin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide | (9R)-N-(1-(4-(4-(((2-(2,6-dioxopiperidin-3-yl)-1-thioxoisoindolin-5-yl)methyl)(methyl)amino )piperidin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide |
| | 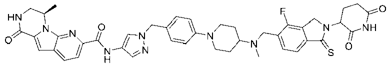 | (9R)-N-(1-(4-(4-(((2-(2,6-dioxopiperidin-3-yl)-4-fluoro-1-thioxoisoindolin-5-yl)methyl)(methyl)amino )piperidin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide | (9R)-N-(1-(4-(4-(((2-(2,6-dioxopiperidin-3-yl)-4-fluoro-1-thioxoisoindolin-5-yl)methyl)(methyl)amino )piperidin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide |
| GT-0877 8 | 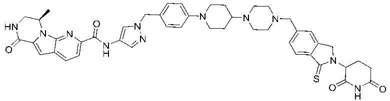 | (9R)-N-(1-(4-(4-(4-((2-(2,6-dioxopiperidin-3-yl)-1-thioxoisoindolin-5-yl)methyl)piperazin-1-yl)piperidin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide | (9R)-N-(1-(4-(4-(4-((2-(2,6-dioxopiperidin-3-yl)-1-thioxoisoindolin-5-yl)methyl)piperazin-1-yl)piperidin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide |
| GT-0878 5 | 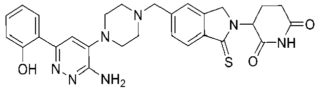 | 3-(5-((4-(3-amino-6-(2-hydroxyphenyl)pyridazin -4-yl)piperazin-1-yl)methyl)-1-thioxoisoindolin-2-yl)piperidine-2,6-dione | 3-(5-((4-(3-amino-6-(2-hydroxyphenyl)pyridazin -4-yl)piperazin-1-yl)methyl)-1-thioxoisoindolin-2-yl)piperidine-2,6-dione |
| GT-0871 2 | 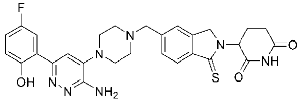 | 3-(5-((4-(3-amino-6-(5-fluoro-2-hydroxyphenyl)pyridazin -4-yl)piperazin-1-yl)methyl)-1-thioxoisoindolin-2-yl)piperidine-2,6-dione | 3-(5-((4-(3-amino-6-(5-fluoro-2-hydroxyphenyl)pyridazin -4-yl)piperazin-1-yl)methyl)-1-thioxoisoindolin-2-yl)piperidine-2,6-dione |
| | 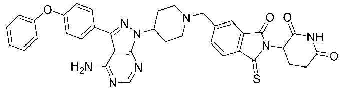 | 3-(5-((4-(4-amino-3-(4-phenoxyphenyl)-1H-pyrazolo[3,4-d]pyrimidin-1-yl)piperidin-1-yl)methyl)-3-oxo-1-thioxoisoindolin-2-yl)piperidine-2,6-dione | 3-(5-((4-(4-amino-3-(4-phenoxyphenyl)-1H-pyrazolo[3,4-d]pyrimidin-1-yl)piperidin-1-yl)methyl)-3-oxo-1-thioxoisoindolin-2-yl)piperidine-2,6-dione |
| | 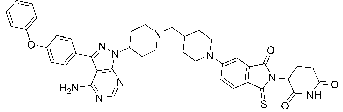 | 3-(5-(4-((4-(4-amino-3-(4-phenoxyphenyl)-1H-pyrazolo[3,4-d]pyrimidin-1-yl)piperidin-1-yl)methyl)piperidin-1-yl)-3-oxo-1-thioxoisoindolin-2-yl)piperidine-2,6-dione | 3-(5-(4-((4-(4-amino-3-(4-phenoxyphenyl)-1H-pyrazolo[3,4-d]pyrimidin-1-yl)piperidin-1-yl)methyl)piperidin-1-yl)-3-oxo-1-thioxoisoindolin-2-yl)piperidine-2,6-dione |
| | 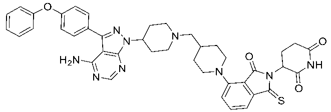 | 3-(4-(4-((4-(4-amino-3-(4-phenoxyphenyl)-1H-pyrazolo[3,4-d]pyrimidin-1-yl)piperidin-1-yl)methyl)piperidin-1-yl)-3-oxo-1-thioxoisoindolin-2-yl)piperidine-2,6-dione | 3-(4-(4-((4-(4-amino-3-(4-phenoxyphenyl)-1H-pyrazolo[3,4-d]pyrimidin-1-yl)piperidin-1-yl)methyl)piperidin-1-yl)-3-oxo-1-thioxoisoindolin-2-yl)piperidine-2,6-dione |
| | 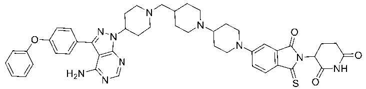 | 3-(5-(4-((4-(4-amino-3-(4-phenoxyphenyl)-1H-pyrazolo[3,4-d]pyrimidin-1-yl)piperidin-1-yl)methyl)-[1,4'-bipiperidin]-1'-yl)-3-oxo-1-thioxoisoindolin-2-yl)piperidine-2,6-dione | 3-(5-(4-((4-(4-amino-3-(4-phenoxyphenyl)-1H-pyrazolo[3,4-d]pyrimidin-1-yl)piperidin-1-yl)methyl)-[1,4'-bipiperidin]-1'-yl)-3-oxo-1-thioxoisoindolin-2-yl)piperidine-2,6-dione |
| | 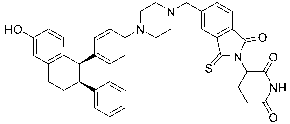 | 3-(5-((4-(4-((1R,2S)-6-hydroxy-2-phenyl-1,2,3,4-tetrahydronaphthalen-1-yl)phenyl)piperazin-1-yl)methyl)-1-oxo-3-thioxoisoindolin-2-yl)piperidine-2,6-dione | 3-(5-((4-(4-((1R,2S)-6-hydroxy-2-phenyl-1,2,3,4-tetrahydronaphthalen-1-yl)phenyl)piperazin-1-yl)methyl)-1-oxo-3-thioxoisoindolin-2-yl)piperidine-2,6-dione |
| | 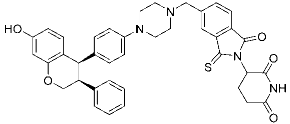 | 3-(5-((4-(4-((3S,4R)-7-hydroxy-3-phenylchroman-4-yl)phenyl)piperazin-1-yl)methyl)-1-oxo-3-thioxoisoindolin-2-yl)piperidine-2,6-dione | 3-(5-((4-(4-((3S,4R)-7-hydroxy-3-phenylchroman-4-yl)phenyl)piperazin-1-yl)methyl)-1-oxo-3-thioxoisoindolin-2-yl)piperidine-2,6-dione |
| | 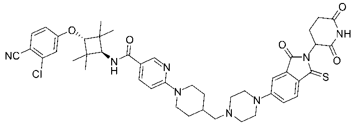 | N-((1r,3r)-3-(3-chloro-4-cyanophenoxy)-2,2,4,4-tetramethylcyclobutyl)-6-(4-((4-(2-(2,6-dioxopiperidin-3-yl)-3-oxo-1-thioxoisoindolin-5-yl)piperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-3-oxo-1-thioxoisoindolin-5-yl)piperazin-1-yl)methyl)piperidin-1-yl)nicotinamide |
| | 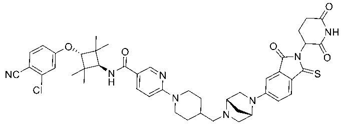 | N-((1r,3 S)-3-(3-chloro-4-cyanophenoxy)-2,2,4,4-tetramethylcyclobutyl)-6-(4-(((1 S,4S)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)nicotinamide | N-((1r,3S)-3-(3-chloro-4-cyanophenoxy)-2,2,4,4-tetramethylcyclobutyl)-6-(4-(((1S,4S)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)nicotinamide |
| | 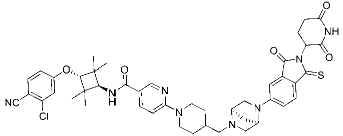 | N-((1r,3R)-3-(3-chloro-4-cyanophenoxy)-2,2,4,4-tetramethylcyclobutyl)-6-(4-(((1R,4R)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)nicotinamide | N-((1r,3R)-3-(3-chloro-4-cyanophenoxy)-2,2,4,4-tetramethylcyclobutyl)-6-(4-(((1R,4R)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)nicotinamide |
| | 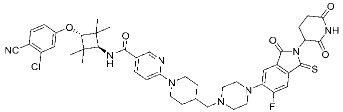 | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-3-oxo-1-thioxoisoindolin-5-yl)piperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-3-oxo-1-thioxoisoindolin-5-yl)piperazin-1-yl)methyl)piperidin-1-yl)nicotinamide |
| | 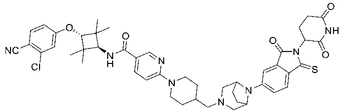 | N-((1r,3r)-3-(3-chloro-4-cyanophenoxy)-2,2,4,4-tetramethylcyclobutyl)-6-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)nicotinamide | N-((1r,3r)-3-(3-chloro-4-cyanophenoxy)-2,2,4,4-tetramethylcyclobutyl)-6-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)nicotinamide |
| | 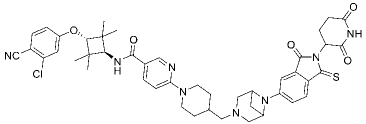 | N-((1r,3r)-3-(3-chloro-4-cyanophenoxy)-2,2,4,4-tetramethylcyclobutyl)-6-(4-((6-(2-(2,6-dioxopiperidin-3-yl)-3-oxo-1-thioxoisoindolin-5-yl)-3,6-diazabicyclo[3.1.1]hepta n-3-yl)methyl)piperidin-1-yl)nicotinamide | N-((1r,3r)-3-(3-chloro-4-cyanophenoxy)-2,2,4,4-tetramethylcyclobutyl)-6-(4-((6-(2-(2,6-dioxopiperidin-3-yl)-3-oxo-1-thioxoisoindolin-5-yl)-3,6-diazabicyclo[3.1.1]hepta n-3-yl)methyl)piperidin-1-yl)nicotinamide |
| | 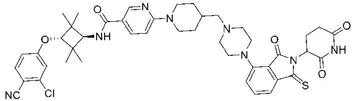 | N-((1r,3r)-3-(3-chloro-4-cyanophenoxy)-2,2,4,4-tetramethylcyclobutyl)-6-(4-((4-(2-(2,6-dioxopiperidin-3-yl)-3-oxo-1-thioxoisoindolin-4-yl)piperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-3-oxo-1-thioxoisoindolin-4-yl)piperazin-1-yl)methyl)piperidin-1-yl)nicotinamide |
| | 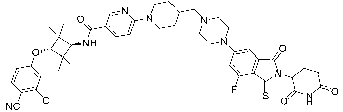 | N-((1r,3r)-3-(3-chloro-4-cyanophenoxy)-2,2,4,4-tetramethylcyclobutyl)-6-(4-((4-(2-(2,6-dioxopiperidin-3-yl)-7-fluoro-3-oxo-1-thioxoisoindolin-5-yl)piperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-7-fluoro-3-oxo-1-thioxoisoindolin-5-yl)piperazin-1-yl)methyl)piperidin-1-yl)nicotinamide |
| | 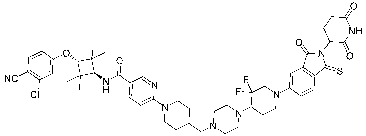 | N-((1r,3r)-3-(3-chloro-4-cyanophenoxy)-2,2,4,4-tetramethylcyclobutyl)-6-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)nicotinamide |
| | 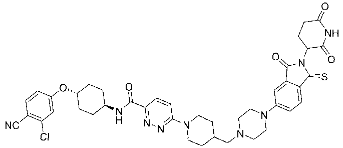 | N-((1r,4r)-4-(3-chloro-4-cyanophenoxy)cyclohex yl)-6-(4-((4-(2-(2,6-dioxopiperidin-3-yl)-3-oxo-1-thioxoisoindolin-5-yl)piperazin-1-yl)methyl)piperidin-1-yl)pyridazine-3-carboxamide | N-((1r,4r)-4-(3-chloro-4-cyanophenoxy)cyclohex yl)-6-(4-((4-(2-(2,6-dioxopiperidin-3-yl)-3-oxo-1-thioxoisoindolin-5-yl)piperazin-1-yl)methyl)piperidin-1-yl)pyridazine-3-carboxamide |
| | 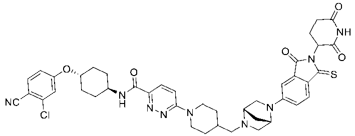 | N-((1r,4r)-4-(3-chloro-4-cyanophenoxy)cyclohex yl)-6-(4-(((1S,4S)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)pyridazine-3-carboxamide | N-((1r,4r)-4-(3-chloro-4-cyanophenoxy)cyclohex yl)-6-(4-(((1S,4S)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)pyridazine-3-carboxamide |
| | 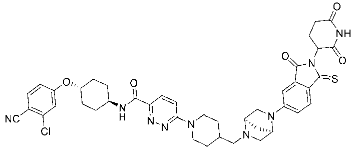 | N-((1r,4r)-4-(3-chloro-4-cyanophenoxy)cyclohex yl)-6-(4-(((1R,4R)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)pyridazine-3-carboxamide | N-((1r,4r)-4-(3-chloro-4-cyanophenoxy)cyclohex yl)-6-(4-(((1R,4R)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)pyridazine-3-carboxamide |
| | 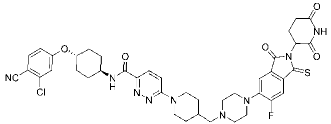 | N-((1r,4r)-4-(3-chloro-4-cyanophenoxy)cyclohex yl)-6-(4-((4-(2-(2,6-dioxopiperidin-3-yl)-6-fluoro-3-oxo-1-thioxoisoindolin-5-yl)piperazin-1-yl)methyl)piperidin-1-yl)pyridazine-3-carboxamide | N-((1r,4r)-4-(3-chloro-4-cyanophenoxy)cyclohex yl)-6-(4-((4-(2-(2,6-dioxopiperidin-3-yl)-6-fluoro-3-oxo-1-thioxoisoindolin-5-yl)piperazin-1-yl)methyl)piperidin-1-yl)pyridazine-3-carboxamide |
| | 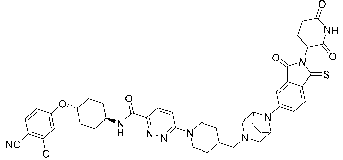 | N-((1r,4r)-4-(3-chloro-4-cyanophenoxy)cyclohex yl)-6-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)pyridazine-3-carboxamide | N-((1r,4r)-4-(3-chloro-4-cyanophenoxy)cyclohex yl)-6-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)pyridazine-3-carboxamide |
| | 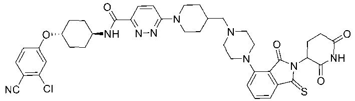 | N-((1r,4r)-4-(3-chloro-4-cyanophenoxy)cyclohex yl)-6-(4-((4-(2-(2,6-dioxopiperidin-3-yl)-3-oxo-1-thioxoisoindolin-4-yl)piperazin-1-yl)methyl)piperidin-1-yl)pyridazine-3-carboxamide | N-((1r,4r)-4-(3-chloro-4-cyanophenoxy)cyclohex yl)-6-(4-((4-(2-(2,6-dioxopiperidin-3-yl)-3-oxo-1-thioxoisoindolin-4-yl)piperazin-1-yl)methyl)piperidin-1-yl)pyridazine-3-carboxamide |
| | 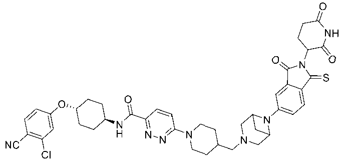 | N-((1r,4r)-4-(3-chloro-4-cyanophenoxy)cyclohex yl)-6-(4-((6-(2-(2,6-dioxopiperidin-3-yl)-3-oxo-1-thioxoisoindolin-5-yl)-3,6-diazabicyclo[3.1.1]hepta n-3-yl)methyl)piperidin-1-yl)pyridazine-3-carboxamide | N-((1r,4r)-4-(3-chloro-4-cyanophenoxy)cyclohex yl)-6-(4-((6-(2-(2,6-dioxopiperidin-3-yl)-3-oxo-1-thioxoisoindolin-5-yl)-3,6-diazabicyclo[3.1.1]hepta n-3-yl)methyl)piperidin-1-yl)pyridazine-3-carboxamide |
| | 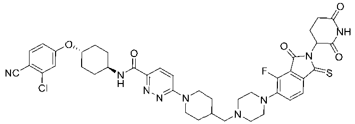 | N-((1r,4r)-4-(3-chloro-4-cyanophenoxy)cyclohex yl)-6-(4-((4-(2-(2,6-dioxopiperidin-3-yl)-4-fluoro-3-oxo-1-thioxoisoindolin-5-yl)piperazin-1-yl)methyl)piperidin-1-yl)pyridazine-3-carboxamide | N-((1r,4r)-4-(3-chloro-4-cyanophenoxy)cyclohex yl)-6-(4-((4-(2-(2,6-dioxopiperidin-3-yl)-4-fluoro-3-oxo-1-thioxoisoindolin-5-yl)piperazin-1-yl)methyl)piperidin-1-yl)pyridazine-3-carboxamide |
| | 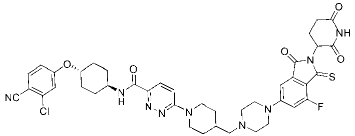 | N-((1r,4r)-4-(3-chloro-4-cyanophenoxy)cyclohex yl)-6-(4-((4-(2-(2,6-dioxopiperidin-3-yl)-7-fluoro-3-oxo-1-thioxoisoindolin-5-yl)piperazin-1-yl)methyl)piperidin-1-yl)pyridazine-3-carboxamide | N-((1r,4r)-4-(3-chloro-4-cyanophenoxy)cyclohex yl)-6-(4-((4-(2-(2,6-dioxopiperidin-3-yl)-7-fluoro-3-oxo-1-thioxoisoindolin-5-yl)piperazin-1-yl)methyl)piperidin-1-yl)pyridazine-3-carboxamide |
| | 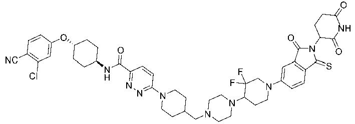 | N-((1r,4r)-4-(3-chloro-4-cyanophenoxy)cyclohex yl)-6-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)pyridazine-3-carboxamide | N-((1r,4r)-4-(3-chloro-4-cyanophenoxy)cyclohex yl)-6-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)pyridazine-3-carboxamide |
| | 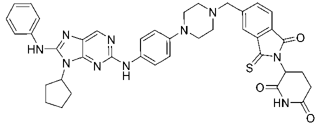 | 3-(5-((4-(4-((9-cyclopentyl-8-(phenylamino)-9H-purin-2-yl)amino)phenyl)piperazi n-1-yl)methyl)-1-oxo-3-thioxoisoindolin-2-yl)piperidine-2,6-dione | 3-(5-((4-(4-((9-cyclopentyl-8-(phenylamino)-9H-purin-2-yl)amino)phenyl)piperazi n-1-yl)methyl)-1-oxo-3-thioxoisoindolin-2-yl)piperidine-2,6-dione |
| | 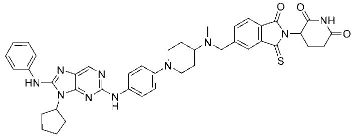 | 3-(5-(((1-(4-((9-cyclopentyl-8-(phenylamino)-9H-purin-2-yl)amino)phenyl)piperidi n-4-yl)(methyl)amino)methyl )-1-oxo-3-thioxoisoindolin-2-yl)piperidine-2,6-dione | 3-(5-(((1-(4-((9-cyclopentyl-8-(phenylamino)-9H-purin-2-yl)amino)phenyl)piperidi n-4-yl)(methyl)amino)methyl )-1-oxo-3-thioxoisoindolin-2-yl)piperidine-2,6-dione |
| | 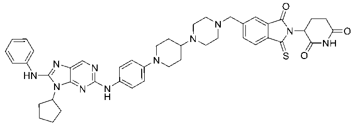 | 3-(5-((4-(1-(4-((9-cyclopentyl-8-(phenylamino)-9H-purin-2-yl)amino)phenyl)piperidi n-4-yl)piperazin-1-yl)methyl)-3-oxo-1-thioxoisoindolin-2-yl)piperidine-2,6-dione | 3-(5-((4-(1-(4-((9-cyclopentyl-8-(phenylamino)-9H-purin-2-yl)amino)phenyl)piperidi n-4-yl)piperazin-1-yl)methyl)-3-oxo-1-thioxoisoindolin-2-yl)piperidine-2,6-dione |
| | 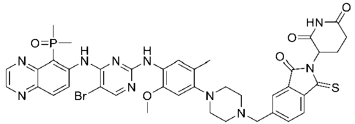 | 3-(5-((4-(4-((5-bromo-4-((5-(dimethylphosphoryl)qui noxalin-6-yl)amino)pyrimidin-2-yl)amino)-5-methoxy-2-methylphenyl)piperazin-1-yl)methyl)-3-oxo-1-thioxoisoindolin-2-yl)piperidine-2,6-dione | 3-(5-((4-(4-((5-bromo-4-*((5-*(dimethylphosphoryl)qui noxalin-6-yl)amino)pyrimidin-2-yl)amino)-5-methoxy-2-methylphenyl)piperazin-1-yl)methyl)-3-oxo-1-thioxoisoindolin-2-yl)piperidine-2,6-dione |
| | 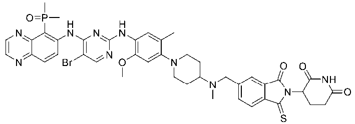 | 3-(5-(((1-(4-((5-bromo-4-((5-(dimethylphosphoryl)qui noxalin-6-yl)amino)pyrimidin-2-yl)amino)-5-methoxy-2-methylphenyl)piperidin-4-yl)(methyl)amino)methyl)-3-oxo-1-thioxoisoindolin-2-yl)piperidine-2,6-dione | 3-(5-(((1-(4-((5-bromo-4-((5-(dimethylphosphoryl)qui noxalin-6-yl)amino)pyrimidin-2-yl)amino)-5-methoxy-2-methylphenyl)piperidin-4-yl)(methyl)amino)methyl)-3-oxo-1-thioxoisoindolin-2-yl)piperidine-2,6-dione |
| | 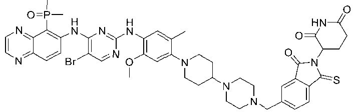 | 3-(5-((4-(1-(4-((5-bromo-4-((5-(dimethylphosphoryl)qui noxalin-6-yl)amino)pyrimidin-2-yl)amino)-5-methoxy-2-methylphenyl)piperidin-4-yl)piperazin-1-yl)methyl)-3-oxo-1-thioxoisoindolin-2-yl)piperidine-2,6-dione | 3-(5-((4-(1-(4-((5-bromo-4-((5-(dimethylphosphoryl)qui noxalin-6-yl)amino)pyrimidin-2-yl)amino)-5-methoxy-2-methylphenyl)piperidin-4-yl)piperazin-1-yl)methyl)-3-oxo-1-thioxoisoindolin-2-yl)piperidine-2,6-dione |
| | 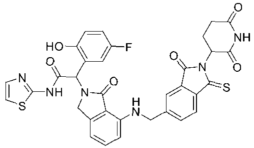 | 2-(7-(((2-(2,6-dioxopiperidin-3-yl)-3-oxo-1-thioxoisoindolin-5-yl)methyl)amino)-1-oxoisoindolin-2-yl)-2-(5-fluoro-2-hydroxyphenyl)-N-(thiazol-2-yl)acetamide | 2-(7-(((2-(2,6-dioxopiperidin-3-yl)-3-oxo-1-thioxoisoindolin-5-yl)methyl)amino)-1-oxoisoindolin-2-yl)-2-(5-fluoro-2-hydroxyphenyl)-N-(thiazol-2-yl)acetamide |
| | 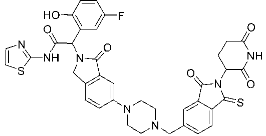 | 2-(6-(4-((2-(2,6-dioxopiperidin-3-yl)-3-oxo-1-thioxoisoindolin-5-yl)methyl)piperazin-1-yl)-1-oxoisoindolin-2-yl)-2-(5-fluoro-2-hydroxyphenyl)-N-(thiazol-2-yl)acetamide | 2-(6-(4-((2-(2,6-dioxopiperidin-3-yl)-3-oxo-1-thioxoisoindolin-5-yl)methyl)piperazin-1-yl)-1-oxoisoindolin-2-yl)-2-(5-fluoro-2-hydroxyphenyl)-N-(thiazol-2-yl)acetamide |
| | 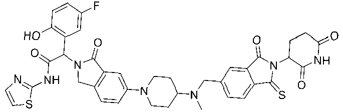 | 2-(6-(4-(((2-(2,6-dioxopiperidin-3-yl)-3-oxo-1-thioxoisoindolin-5-yl)methyl)(methyl)amino )piperidin-1-yl)-1-oxoisoindolin-2-yl)-2-(5-fluoro-2-hydroxyphenyl)-N-(thiazol-2-yl)acetamide | 2-(6-(4-(((2-(2,6-dioxopiperidin-3-yl)-3-oxo-1-thioxoisoindolin-5-yl)methyl)(methyl)amino )piperidin-1-yl)-1-oxoisoindolin-2-yl)-2-(5-fluoro-2-hydroxyphenyl)-N-(thiazol-2-yl)acetamide |
| | 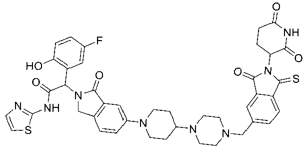 | 2-(6-(4-(4-((2-(2,6-dioxopiperidin-3-yl)-3-oxo-1-thioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide | 2-(6-(4-(4-((2-(2,6-dioxopiperidin-3-yl)-3-oxo-1-thioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide |
| | 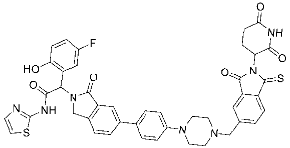 | 2-(6-(4-(4-((2-(2,6-dioxopiperidin-3-yl)-3-oxo-1-thioxoisoindolin-5-yl)methyl)piperazin-1-yl)phenyl)-1-oxoisoindolin-2-yl)-2-(5-fluoro-2-hydroxyphenyl)-N-(thiazol-2-vl)acetamide | 2-(6-(4-(4-((2-(2,6-dioxopiperidin-3-yl)-3-oxo-1-thioxoisoindolin-5-yl)methyl)piperazin-1-yl)phenyl)-1-oxoisoindolin-2-yl)-2-(5-fluoro-2-hydroxyphenyl)-N-(thiazol-2-vl)acetamide |
| | 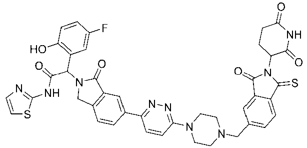 | 2-(6-(6-(4-((2-(2,6-dioxopiperidin-3-yl)-3-oxo-1-thioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-vl)acetamide | 2-(6-(6-(4-((2-(2,6-dioxopiperidin-3-yl)-3-oxo-1-thioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-vl)acetamide |
| | 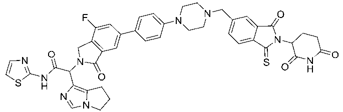 | 2-(6,7-dihydro-5H-pyrrolo[1,2-c]imidazol-1-yl)-2-(6-(4-(4-((2-(2,6-dioxopiperidin-3-yl)-3-oxo-1-thioxoisoindolin-5-yl)methyl)piperazin-1-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-(4-((2-(2,6-dioxopiperidin-3-yl)-3-oxo-1-thioxoisoindolin-5-yl)methyl)piperazin-1-yl)phenyl)-4-fluoro-1-oxoisoindolin-2-yl)-N-(thiazol-2-vl)acetamide |
| | 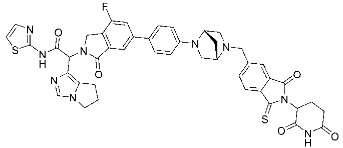 | 2-(6,7-dihydro-5H-pyrrolo[1,2-c]imidazol-1-yl)-2-(6-(4-((1S,4S)-5-((2-(2,6-dioxopiperidin-3-yl)-3-oxo-1-thioxoisoindolin-5-yl)methyl)-2,5-diazabicyclo[2.2.1]hepta n-2-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-((1S,4S)-5-((2-(2,6-dioxopiperidin-3-yl)-3-oxo-1-thioxoisoindolin-5-yl)methyl)-2,5-diazabicyclo[2.2.1]hepta n-2-yl)phenyl)-4-fluoro-1-oxoisoindolin-2-yl)-N-(thiazol-2-vl)acetamide |
| | 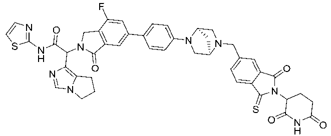 | 2-(6,7-dihydro-5H-pyrrolo[1,2-c]imidazol-1-yl)-2-(6-(4-((1R,4R)-5-((2-(2,6-dioxopiperidin-3-yl)-3-oxo-1-thioxoisoindolin-5-yl)methyl)-2,5-diazabicyclo[2.2.1]hepta n-2-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-((1R,4R)-5-((2-(2,6-dioxopiperidin-3-yl)-3-oxo-1-thioxoisoindolin-5-yl)methyl)-2,5-diazabicyclo[2.2.1]hepta n-2-yl)phenyl)-4-fluoro-1-oxoisoindolin-2-yl)-N-(thiazol-2-vl)acetamide |
| | 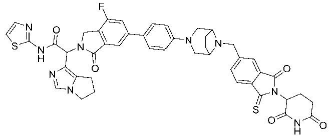 | 2-(6,7-dihydro-5H-pyrrolo[1,2-c]imidazol-1-yl)-2-(6-(4-(8-((2-(2,6-dioxopiperidin-3-yl)-3-oxo-1-thioxoisoindolin-5-yl)methyl)-3,8-diazabicyclo[3.2.1]octan-3-yl)phenyl)-4-fluoro-1-oxoisoindolin-2-yl)-N-(thiazol-2-vl)acetamide | 2-(6,7-dihydro-5H-pyrrolo[1,2-c]imidazol-1-yl)-2-(6-(4-(8-((2-(2,6-dioxopiperidin-3-yl)-3-oxo-1-thioxoisoindolin-5-yl)methyl)-3,8-diazabicyclo[3.2.1]octan-3-yl)phenyl)-4-fluoro-1-oxoisoindolin-2-yl)-N-(thiazol-2-vl)acetamide |
| | 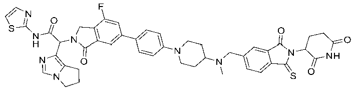 | 2-(6,7-dihydro-5H-pyrrolo[1,2-c]imidazol-1-yl)-2-(6-(4-(4-(((2-(2,6-dioxopiperidin-3-yl)-3-oxo-1-thioxoisoindolin-5-yl)methyl)(methyl)amino )piperidin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-(((2-(2,6-dioxopiperidin-3-yl)-3-oxo-1-thioxoisoindolin-5-yl)methyl)(methyl)amino )piperidin-1-yl)phenyl)-4-fluoro-1-oxoisoindolin-2-yl)-N-(thiazol-2-yl)acetamide |
| | 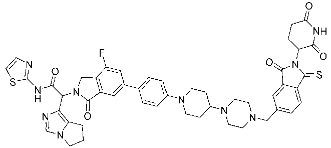 | 2-(6,7-dihydro-5H-pyrrolo[1,2-c]imidazol-1-yl)-2-(6-(4-(4-(4-((2-(2,6-dioxopiperidin-3-yl)-3-oxo-1-thioxoisoindolin-5-yl)methyl)piperazin-1-yl)piperidin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-(4-((2-(2,6-dioxopiperidin-3-yl)-3-oxo-1-thioxoisoindolin-5-yl)methyl)piperazin-1-yl)piperidin-1-yl)phenyl)-4-fluoro-1-oxoisoindolin-2-yl)-N-(thiazol-2-yl)acetamide |
| | 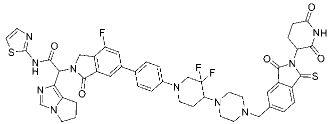 | 2-(6,7-dihydro-5H-pyrrolo[1,2-c]imidazol-1-yl)-2-(6-(4-(4-(4-((2-(2,6-dioxopiperidin-3-yl)-3-oxo-1-thioxoisoindolin-5-yl)methyl)piperazin-1-yl)-3,3-difluoropiperidin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-(4-((2-(2,6-dioxopiperidin-3-yl)-3-oxo-1-thioxoisoindolin-5-yl)methyl)piperazin-1-yl)-3,3-difluoropiperidin-1-yl)phenyl)-4-fluoro-1-oxoisoindolin-2-yl)-N-(thiazol-2-vl)acetamide |
| | 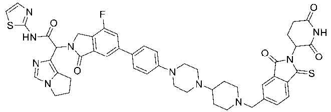 | 2-(6,7-dihydro-5H-pyrrolo[1,2-c]imidazol-1-yl)-2-(6-(4-(4-(1-((2-(2,6-dioxopiperidin-3-yl)-3-oxo-1-thioxoisoindolin-5-yl)methyl)piperidin-4-yl)piperazin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-(1-((2-(2,6-dioxopiperidin-3-yl)-3-oxo-1-thioxoisoindolin-5-yl)methyl)piperidin-4-yl)piperazin-1-yl)phenyl)-4-fluoro-1-oxoisoindolin-2-yl)-N-(thiazol-2-yl)acetamide |
| | 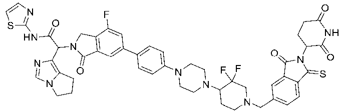 | 2-(6,7-dihydro-5H-pyrrolo[1,2-c]imidazol-1-yl)-2-(6-(4-(4-(1-((2-(2,6-dioxopiperidin-3-yl)-3-oxo-1-thioxoisoindolin-5-yl)methyl)-3,3-difluoropiperidin-4-yl)piperazin-1-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-(4-(1-((2-(2,6-dioxopiperidin-3-yl)-3-oxo-1-thioxoisoindolin-5-yl)methyl)-3,3-difluoropiperidin-4-yl)piperazin-1-yl)phenyl)-4-fluoro-1-oxoisoindolin-2-yl)-N-(thiazol-2-vl)acetamide |
| | 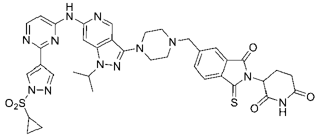 | 3-(5-((4-(6-((2-(1-(cyclopropylsulfonyl)-1H-pyrazol-4-yl)pyrimidin-4-yl)amino)- 1-isopropyl-1H-pyrazolo[4,3-c]pyridin-3-yl)piperazin-1-yl)methyl)-3-oxo-1-thioxoisoindolin-2-yl)piperidine-2,6-dione | 3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-3-oxo-1-thioxoisoindolin-2-yl)piperidine-2,6-dione |
| | 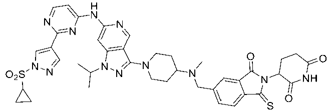 | 3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl )-3-oxo-1-thioxoisoindolin-2-yl)piperidine-2,6-dione | 3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl )-3-oxo-1-thioxoisoindolin-2-yl)piperidine-2,6-dione |
| | 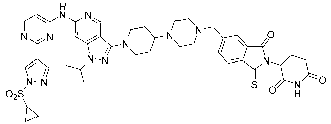 | 3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-3-oxo-1-thioxoisoindolin-2-yl)piperidine-2,6-dione | 3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-3-oxo-1-thioxoisoindolin-2-yl)piperidine-2,6-dione |
| | 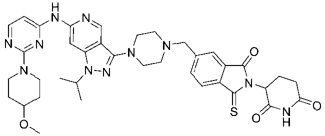 | 3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-3-oxo-1-thioxoisoindolin-2-yl)piperidine-2,6-dione | 3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-3-oxo-1-thioxoisoindolin-2-yl)piperidine-2,6-dione |
| | 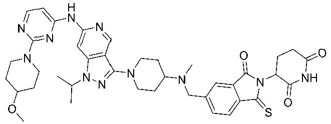 | 3-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl )-3-oxo-1-thioxoisoindolin-2-yl)piperidine-2,6-dione | 3-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl )-3-oxo-1-thioxoisoindolin-2-yl)piperidine-2,6-dione |
| | 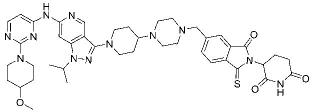 | 3-(5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-3-oxo-1-thioxoisoindolin-2-yl)piperidine-2,6-dione | 3-(5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-3-oxo-1-thioxoisoindolin-2-yl)piperidine-2,6-dione |
| | 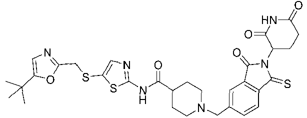 | N-(5-(((5-(tert-butyl)oxazol-2-yl)methyl)thio)thiazol-2-yl)-1-((2-(2,6-dioxopiperidin-3-yl)-3-oxo-1-thioxoisoindolin-5-yl)methyl)piperidine-4-carboxamide | N-(5-(((5-(tert-butyl)oxazol-2-yl)methyl)thio)thiazol-2-yl)-1-((2-(2,6-dioxopiperidin-3-yl)-3-oxo-1-thioxoisoindolin-5-yl)methyl)piperidine-4-carboxamide |
| | 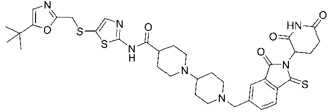 | N-(5-(((5-(tert-butyl)oxazol-2-yl)methyl)thio)thiazol-2-yl)-1'-((2-(2,6-dioxopiperidin-3-yl)-3-oxo-1-thioxoisoindolin-5-yl)methyl)-[1,4'-bipiperidine]-4-carboxamide | N-(5-(((5-(tert-butyl)oxazol-2-yl)methyl)thio)thiazol-2-yl)-1'-((2-(2,6-dioxopiperidin-3-yl)-3-oxo-1-thioxoisoindolin-5-yl)methyl)-[1,4'-bipiperidine]-4-carboxamide |
| | 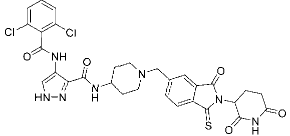 | 4-(2,6-dichlorobenzamido)-N-(1-((2-(2,6-dioxopiperidin-3-yl)-3-oxo-1-thioxoisoindolin-5-yl)methyl)piperidin-4-yl)-1H-pyrazole-3-carboxamide | 4-(2,6-dichlorobenzamido)-N-(1-((2-(2,6-dioxopiperidin-3-yl)-3-oxo-1-thioxoisoindolin-5-yl)methyl)piperidin-4-yl)-1H-pyrazole-3-carboxamide |
| | 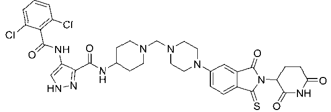 | 4-(2,6-dichlorobenzamido)-N-(1-((4-(2-(2,6-dioxopiperidin-3-yl)-3-oxo-1-thioxoisoindolin-5-yl)piperazin-1-yl)methyl)piperidin-4-yl)-1H-pyrazole-3-carboxamide | 4-(2,6-dichlorobenzamido)-N-(1-((4-(2-(2,6-dioxopiperidin-3-yl)-3-oxo-1-thioxoisoindolin-5-yl)piperazin-1-yl)methyl)piperidin-4-yl)-1H-pyrazole-3-carboxamide |
| | 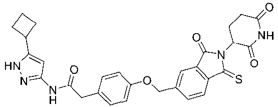 | N-(5-cyclobutyl-1H-pyrazol-3-yl)-2-(4-((2-(2,6-dioxopiperidin-3-yl)-3-oxo-1-thioxoisoindolin-5-yl)methoxy)phenyl)aceta mide | N-(5-cyclobutyl-1H-pyrazol-3-yl)-2-(4-((2-(2,6-dioxopiperidin-3-yl)-3-oxo-1-thioxoisoindolin-5-yl)methoxy)phenyl)aceta mide |
| | 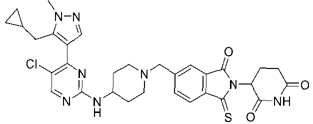 | 3-(5-((4-((5-chloro-4-(5-(cyclopropylmethyl)-1-methyl-1H-pyrazol-4-yl)pyrimidin-2-yl)amino)piperidin-1-yl)methyl)-3-oxo-1-thioxoisoindolin-2-yl)piperidine-2,6-dione | 3-(5-((4-((5-chloro-4-(5-(cyclopropylmethyl)-1-methyl-1H-pyrazol-4-yl)pyrimidin-2-yl)amino)piperidin-1-yl)methyl)-3-oxo-1-thioxoisoindolin-2-yl)piperidine-2,6-dione |
| | 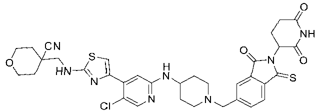 | 4-(((4-(5-chloro-2-((1-((2-(2,6-dioxopiperidin-3-yl)-3-oxo-1-thioxoisoindolin-5-yl)methyl)piperidin-4-yl)amino)pyridin-4-yl)thiazol-2-yl)amino)methyl)tetrahydro-2H-pyran-4-carbonitrile | 4-(((4-(5-chloro-2-((1-((2-(2,6-dioxopiperidin-3-yl)-3-oxo-1-thioxoisoindolin-5-yl)methyl)piperidin-4-yl)amino)pyridin-4-yl)thiazol-2-yl)amino)methyl)tetrahydro-2H-pyran-4-carbonitrile |
| | 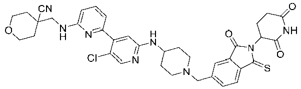 | 4-(((5'-chloro-2'-((1-((2-(2,6-dioxopiperidin-3-yl)-3-oxo-1-thioxoisoindolin-5-yl)methyl)piperidin-4-yl)amino)-[2,4'-bipyridin]-6-yl)amino)methyl)tetrahy dro-2H-pyran-4-carbonitrile | 4-(((5'-chloro-2'-((1-((2-(2,6-dioxopiperidin-3-yl)-3-oxo-1-thioxoisoindolin-5-yl)methyl)piperidin-4-yl)amino)-[2,4'-bipyridin]-6-yl)amino)methyl)tetrahy dro-2H-pyran-4-carbonitrile |
| | 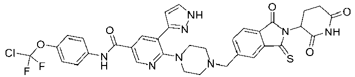 | N-(4-(chlorodifluoromethoxy) phenyl)-6-(4-((2-(2,6-dioxopiperidin-3-yl)-1-oxo-3-thioxoisoindolin-5-yl)methyl)piperazin-1-yl)-5-(1H-pyrazol-3-yl)nicotinamide | N-(4-(chlorodifluoromethoxy) phenyl)-6-(4-((2-(2,6-dioxopiperidin-3-yl)-1-oxo-3-thioxoisoindolin-5-yl)methyl)piperazin-1-yl)-5-(1H-pyrazol-3-yl)nicotinamide |
| | 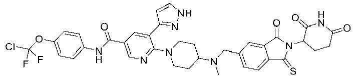 | N-(4-(chlorodifluoromethoxy) phenyl)-6-(4-(((2-(2,6-dioxopiperidin-3-yl)-3-oxo-1-thioxoisoindolin-5-yl)methyl)(methyl)amino )piperidin-1-yl)-5-(1H-pyrazol-3-yl)nicotinamide | N-(4-(chlorodifluoromethoxy) phenyl)-6-(4-(((2-(2,6-dioxopiperidin-3-yl)-3-oxo-1-thioxoisoindolin-5-yl)methyl)(methyl)amino )piperidin-1-yl)-5-(1H-pyrazol-3-yl)nicotinamide |
| | 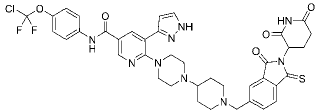 | N-(4-(chlorodifluoromethoxy) phenyl)-6-(4-(1-((2-(2,6-dioxopiperidin-3-yl)-3-oxo-1-thioxoisoindolin-5-yl)methyl)piperidin-4-yl)piperazin-1-yl)-5-(1H-pyrazol-3-yl)nicotinamide | N-(4-(chlorodifluoromethoxy) phenyl)-6-(4-(1-((2-(2,6-dioxopiperidin-3-yl)-3-oxo-1-thioxoisoindolin-5-yl)methyl)piperidin-4-yl)piperazin-1-yl)-5-(1H-pyrazol-3-yl)nicotinamide |
| | 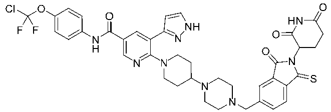 | N-(4-(chlorodifluoromethoxy) phenyl)-6-(4-(4-((2-(2,6-dioxopiperidin-3-yl)-3-oxo-1-thioxoisoindolin-5-yl)methyl)piperazin-1-yl)piperidin-1-yl)-5-(1H-pyrazol-3-yl)nicotinamide | N-(4-(chlorodifluoromethoxy) phenyl)-6-(4-(4-((2-(2,6-dioxopiperidin-3-yl)-3-oxo-1-thioxoisoindolin-5-yl)methyl)piperazin-1-yl)piperidin-1-yl)-5-(1H-pyrazol-3-yl)nicotinamide |
| | 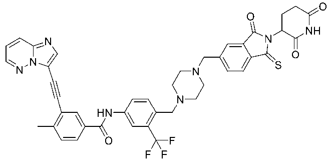 | N-(4-((4-((2-(2,6-dioxopiperidin-3-yl)-3-oxo-1-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide | N-(4-((4-((2-(2,6-dioxopiperidin-3-yl)-3-oxo-1-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide |
| | 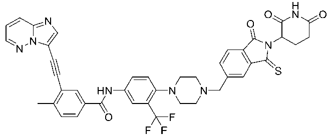 | N-(4-(4-((2-(2,6-dioxopiperidin-3-yl)-1-oxo-3-thioxoisoindolin-5-yl)methyl)piperazin-1-yl)-3-(trifluoromethyl)phenyl)-3-(imidazo[1,2-b]pyridazin-3-ylethynyl)-4-methylbenzamide | N-(4-(4-((2-(2,6-dioxopiperidin-3-yl)-1-oxo-3-thioxoisoindolin-5-yl)methyl)piperazin-1-yl)-3-(trifluoromethyl)phenyl)-3-(imidazo[1,2-b]pyridazin-3-ylethynyl)-4-methylbenzamide |
| | 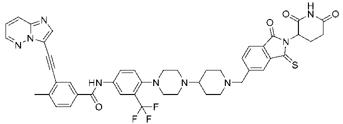 | N-(4-(4-(1-((2-(2,6-dioxopiperidin-3-yl)-1-oxo-3-thioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide | N-(4-(4-(1-((2-(2,6-dioxopiperidin-3-yl)-1-oxo-3-thioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide |
| | 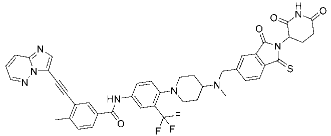 | N-(4-(4-(((2-(2,6-dioxopiperidin-3-yl)-3-oxo-1-thioxoisoindolin-5-yl)methyl)(methyl)amino )piperidin-1-yl)-3-(trifluoromethyl)phenyl)-3-(imidazo[1,2-b]pyridazin-3-ylethynyl)-4-methylbenzamide | N-(4-(4-(((2-(2,6-dioxopiperidin-3-yl)-3-oxo-1-thioxoisoindolin-5-yl)methyl)(methyl)amino )piperidin-1-yl)-3-(trifluoromethyl)phenyl)-3-(imidazo[1,2-b]pyridazin-3-ylethynyl)-4-methylbenzamide |
| | 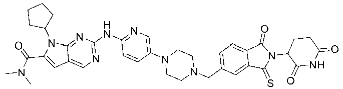 | 7-cyclopentyl-2-((5-(4-((2-(2,6-dioxopiperidin-3-yl)-1-oxo-3-thioxoisoindolin-5-yl)methyl)piperazin-1-yl)pyridin-2-yl)amino)-N,N-dimethyl-7H-pyrrolo[2,3-d]pyrimidine-6-carboxamide | 7-cyclopentyl-2-((5-(4-((2-(2,6-dioxopiperidin-3-yl)-1-oxo-3-thioxoisoindolin-5-yl)methyl)piperazin-1-yl)pyridin-2-yl)amino)-N,N-dimethyl-7H-pyrrolo[2,3-d]pyrimidine-6-carboxamide |
| | 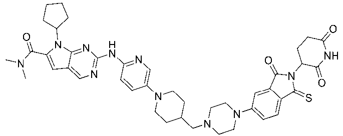 | 7-cyclopentyl-2-((5-(4-((4-(2-(2,6-dioxopiperidin-3-yl)-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide |
| | 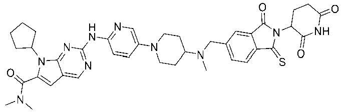 | 7-cyclopentyl-2-((5-(4-(((2-(2,6-dioxopiperidin-3-yl)-3-oxo-1-thioxoisoindolin-5-yl)methyl)(methyl)amino )piperidin-1-yl)pyridin-2-yl)amino)-N,N-dimethyl-7H-pyrrolo[2,3-d]pyrimidine-6-carboxamide | 7-cyclopentyl-2-((5-(4-(( (2-(2,6-dioxopiperidin-3-yl)-3-oxo-1-thioxoisoindolin-5-yl)methyl)(methyl)amino )piperidin-1-yl)pyridin-2-yl)amino)-N,N-dimethyl-7H-pyrrolo[2,3-d]pyrimidine-6-carboxamide |
| | 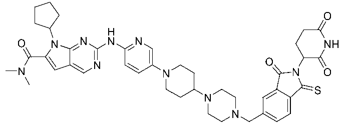 | 7-cyclopentyl-2-((5-(4-(4-((2-(2,6-dioxopiperidin-3-yl)-3-oxo-1-thioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide | 7-cyclopentyl-2-((5-(4-(4-((2-(2,6-dioxopiperidin-3-yl)-3-oxo-1-thioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide |
| | 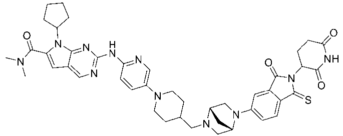 | 7-cyclopentyl-2-((5-(4-(((1S,4S)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-(((1S,4S)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)pyridin-2-yl)amino)-N,N-dimethyl-7H-pyrrolo[2,3-d]pyrimidine-6-carboxamide |
| | 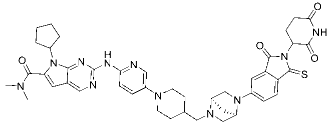 | 7-cyclopentyl-2-((5-(4-(((1R,4R)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-(((1R,4R)-5-(2-(2,6-dioxopiperidin-3-yl)-3-oxo-1-thioxoisoindolin-5-yl)-2,5-diazabicyclo[2.2.1]hepta n-2-yl)methyl)piperidin-1-yl)pyridin-2-yl)amino)-N,N-dimethyl-7H-pyrrolo[2,3-d]pyrimidine-6-carboxamide |
| | 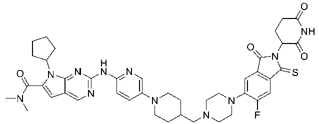 | 7-cyclopentyl-2-((5-(4-((4-(2-(2,6-dioxopiperidin-3-yl)-6-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-6-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide |
| | 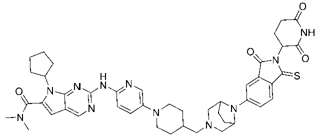 | 7-cyclopentyl-2-((5-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3-d]pyrimidine-6-carboxamide |
| | 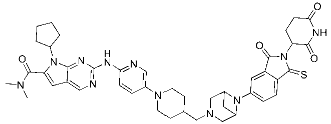 | 7-cyclopentyl-2-((5-(4-((6-(2-(2,6-dioxopiperidin-3-yl)-3-oxo-1-thioxoisoindolin-5-yl)-3,6-diazabicyclo[3.1.1]hepta n-3-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((6-(2-(2,6-dioxopiperidin-3-yl)-3-oxo-1-thioxoisoindolin-5-yl)-3,6-diazabicyclo[3.1.1]hepta n-3-yl)methyl)piperidin-1-yl)pyridin-2-yl)amino)-N,N-dimethyl-7H-pyrrolo[2,3-d]pyrimidine-6-carboxamide |
| | 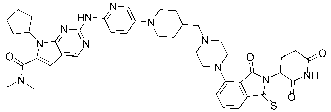 | 7-cyclopentyl-2-((5-(4-((4-(2-(2,6-dioxopiperidin-3-yl)-3-oxo-1-thioxoisoindolin-4-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-3-oxo-1-thioxoisoindolin-4-yl)piperazin-1-yl)methyl)piperidin-1-yl)pyridin-2-yl)amino)-N,N-dimethyl-7H-pyrrolo[2,3-d]pyrimidine-6-carboxamide |
| | 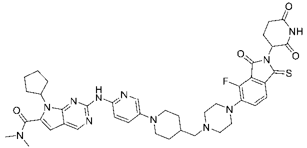 | 7-cyclopentyl-2-((5-(4-((4-(2-(2,6-dioxopiperidin-3-yl)-4-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-4-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide |
| | 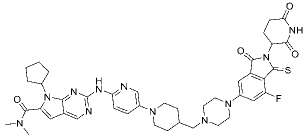 | 7-cyclopentyl-2-((5-(4-((4-(2-(2,6-dioxopiperidin-3-yl)-7-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-7-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide |
| | 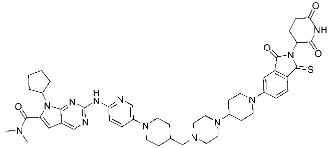 | 7-cyclopentyl-2-((5-(4-((4-(1-(2-(2,6-dioxopiperidin-3-yl)-3-oxo-1-thioxoisoindolin-5-yl)piperidin-4-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(1-(2-(2,6-dioxopiperidin-3-yl)-3-oxo-1-thioxoisoindolin-5-yl)piperidin-4-yl)piperazin-1-yl)methyl)piperidin-1-yl)pyridin-2-yl)amino)-N,N-dimethyl-7H-pyrrolo[2,3-d]pyrimidine-6-carboxamide |
| | 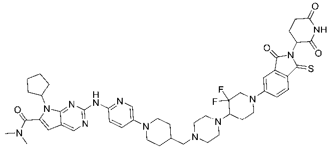 | 7-cyclopentyl-2-((5-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)pyridin-2-yl)amino)-N,N-dimethyl-7H-pyrrolo[2,3-d]pyrimidine-6-carboxamide |
| | 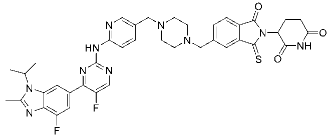 | 3-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxo-3-thioxoisoindolin-2-yl)piperidine-2,6-dione | 3-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxo-3-thioxoisoindolin-2-yl)piperidine-2,6-dione |
| | 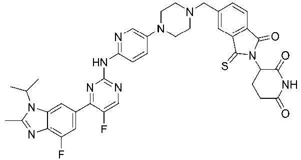 | 3-(5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-oxo-3-thioxoisoindolin-2-yl)piperidine-2,6-dione | 3-(5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-oxo-3-thioxoisoindolin-2-yl)piperidine-2,6-dione |
| | 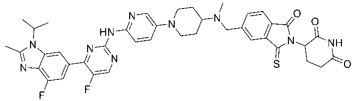 | 3-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl )-1-oxo-3-thioxoisoindolin-2-yl)piperidine-2,6-dione | 3-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl )-1-oxo-3-thioxoisoindolin-2-yl)piperidine-2,6-dione |
| | 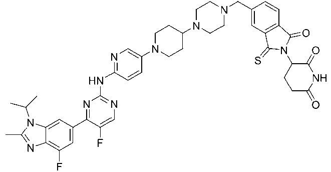 | 3-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxo-3-thioxoisoindolin-2-yl)piperidine-2,6-dione | 3-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxo-3-thioxoisoindolin-2-yl)piperidine-2,6-dione |
| | 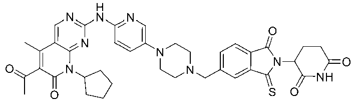 | 3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-oxo-3-thioxoisoindolin-2-yl)piperidine-2,6-dione | 3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-oxo-3-thioxoisoindolin-2-yl)piperidine-2,6-dione |
| | 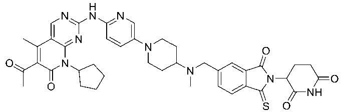 | 3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl )-3-oxo-1-thioxoisoindolin-2-yl)piperidine-2,6-dione | 3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl )-3-oxo-1-thioxoisoindolin-2-yl)piperidine-2,6-dione |
| | 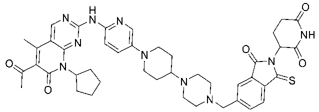 | 3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-3-oxo-1-thioxoisoindolin-2-yl)piperidine-2,6-dione | 3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-3-oxo-1-thioxoisoindolin-2-yl)piperidine-2,6-dione |
| | 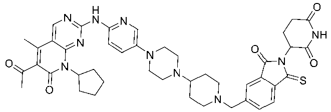 | 3-(5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-3-oxo-1-thioxoisoindolin-2-yl)piperidine-2,6-dione | 3-(5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-3-oxo-1-thioxoisoindolin-2-yl)piperidine-2,6-dione |
| | 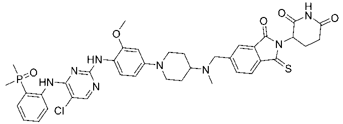 | 3-(5-(((1-(4-((5-chloro-4-((2-(dimethylphosphoryl)phe nyl)amino)pyrimidin-2-yl)amino)-3-methoxyphenyl)piperidin -4-yl)(methyl)amino)methyl )-3-oxo-1-thioxoisoindolin-2-yl)piperidine-2,6-dione | 3-(5-(((1-(4-((5-chloro-4-((2-(dimethylphosphoryl)phe nyl)amino)pyrimidin-2-yl)amino)-3-methoxyphenyl)piperidin -4-yl)(methyl)amino)methyl )-3-oxo-1-thioxoisoindolin-2-yl)piperidine-2,6-dione |
| | 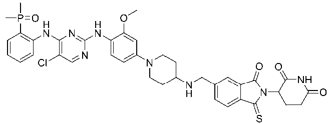 | 3-(5-(((1-(4-((5-chloro-4-((2-(dimethylphosphoryl)phe nyl)amino)pyrimidin-2-yl)amino)-3-methoxyphenyl)piperidin -4-yl)amino)methyl)-3-oxo-1-thioxoisoindolin-2-yl)piperidine-2,6-dione | 3-(5-(((1-(4-((5-chloro-4-((2-(dimethylphosphoryl)phe nyl)amino)pyrimidin-2-yl)amino)-3-methoxyphenyl)piperidin -4-yl)amino)methyl)-3-oxo-1-thioxoisoindolin-2-yl)piperidine-2,6-dione |
| | 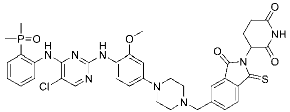 | 3-(5-((4-(4-((5-chloro-4-((2-(dimethylphosphoryl)phe nyl)amino)pyrimidin-2-yl)amino)-3-methoxyphenyl)piperazi n-1-yl)methyl)-3-oxo-1-thioxoisoindolin-2-yl)piperidine-2,6-dione | 3-(5-((4-(4-((5-chloro-4-((2-(dimethylphosphoryl)phe nyl)amino)pyrimidin-2-yl)amino)-3-methoxyphenyl)piperazi n-1-yl)methyl)-3-oxo-1-thioxoisoindolin-2-yl)piperidine-2,6-dione |
| | 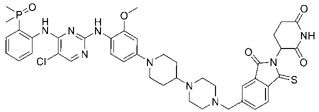 | 3-(5-((4-(1-(4-((5-chloro-4-((2-(dimethylphosphoryl)phe nyl)amino)pyrimidin-2-yl)amino)-3-methoxyphenyl)piperidin -4-yl)piperazin-1-yl)methyl)-3-oxo-1-thioxoisoindolin-2-yl)piperidine-2,6-dione | 3-(5-((4-(1-(4-((5-chloro-4-((2-(dimethylphosphoryl)phe nyl)amino)pyrimidin-2-yl)amino)-3-methoxyphenyl)piperidin -4-yl)piperazin-1-yl)methyl)-3-oxo-1-thioxoisoindolin-2-yl)piperidine-2,6-dione |
| | 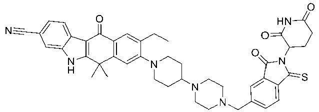 | 8-(4-(4-((2-(2,6-dioxopiperidin-3-yl)-3-oxo-1-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile | 8-(4-(4-((2-(2,6-dioxopiperidin-3-yl)-3-oxo-1-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile |
| | 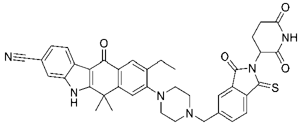 | 8-(4-((2-(2,6-dioxopiperidin-3-yl)-3-oxo-1-thioxoisoindolin-5-yl)methyl)piperazin-1-yl)-9-ethyl-6,6-dimethyl-11-oxo-6,11-dihydro-5H-benzo[b]carbazole-3-carbonitrile | 8-(4-((2-(2,6-dioxopiperidin-3-yl)-3-oxo-1-thioxoisoindolin-5-yl)methyl)piperazin-1-yl)-9-ethyl-6,6-dimethyl-11-oxo-6,11-dihydro-5H-benzo[b]carbazole-3-carbonitrile |
| | 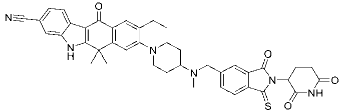 | 8-(4-(((2-(2,6-dioxopiperidin-3-yl)-3-oxo-1-thioxoisoindolin-5-yl)methyl)(methyl)amino )piperidin-1-yl)-9-ethyl-6,6-dimethyl-11-oxo-6,11-dihydro-5H-benzo[b]carbazole-3-carbonitrile | 8-(4-(((2-(2,6-dioxopiperidin-3-yl)-3-oxo-1-thioxoisoindolin-5-yl)methyl)(methyl)amino )piperidin-1-yl)-9-ethyl-6,6-dimethyl-11-oxo-6,11-dihydro-5H-benzo[b]carbazole-3-carbonitrile |
| | 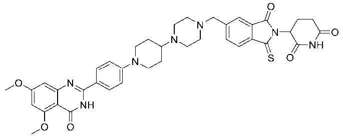 | 3-(5-((4-(1-(4-(5,7-dimethoxy-4-oxo-3,4-dihydroquinazolin-2-yl)phenyl)piperidin-4-yl)piperazin-1-yl)methyl)-3-oxo-1-thioxoisoindolin-2-yl)piperidine-2,6-dione | 3-(5-((4-(1-(4-(5,7-dimethoxy-4-oxo-3,4-dihydroquinazolin-2-yl)phenyl)piperidin-4-yl)piperazin-1-yl)methyl)-3-oxo-1-thioxoisoindolin-2-yl)piperidine-2,6-dione |
| | 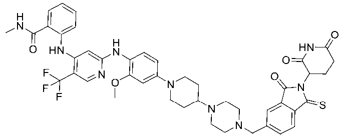 | 2-((2-((4-(4-(4-((2-(2,6-dioxopiperidin-3-yl)-3-oxo-1-thioxoisoindolin-5-yl)methyl)piperazin-1-yl)piperidin-1-yl)-2-methoxyphenyl)amino)-5-(trifluoromethyl)pyridin-4-yl)amino)-N-methylbenzamide | 2-((2-((4-(4-(4-((2-(2,6-dioxopiperidin-3-yl)-3-oxo-1-thioxoisoindolin-5-yl)methyl)piperazin-1-yl)piperidin-1-yl)-2-methoxyphenyl)amino)-5-(trifluoromethyl)pyridin-4-yl)amino)-N-methylbenzamide |
| | 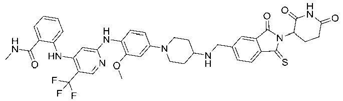 | 2-((2-((4-(4-(((2-(2,6-dioxopiperidin-3-yl)-3-oxo-1-thioxoisoindolin-5-yl)methyl)amino)piperidi n-1-yl)-2-methoxyphenyl)amino)-5-(trifluoromethyl)pyridin-4-yl)amino)-N-methylbenzamide | 2-((2-((4-(4-(((2-(2,6-dioxopiperidin-3-yl)-3-oxo-1-thioxoisoindolin-5-yl)methyl)amino)piperidi n-1-yl)-2-methoxyphenyl)amino)-5-(trifluoromethyl)pyridin-4-yl)amino)-N-methylbenzamide |
| | 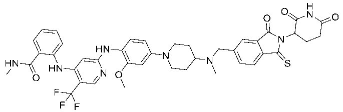 | 2-((2-((4-(4-(((2-(2,6-dioxopiperidin-3-yl)-3-oxo-1-thioxoisoindolin-5-yl)methyl)(methyl)amino )piperidin-1-yl)-2-methoxyphenyl)amino)-5-(trifluoromethyl)pyridin-4-yl)amino)-N-methylbenzamide | 2-((2-((4-(4-(((2-(2,6-dioxopiperidin-3-yl)-3-oxo-1-thioxoisoindolin-5-yl)methyl)(methyl)amino )piperidin-1-yl)-2-methoxyphenyl)amino)-5-(trifluoromethyl)pyridin-4-yl)amino)-N-methylbenzamide |
| | 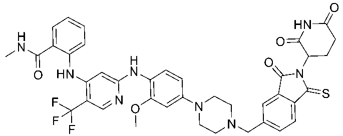 | 2-((2-((4-(4-((2-(2,6-dioxopiperidin-3-yl)-3-oxo-1-thioxoisoindolin-5-yl)methyl)piperazin-1-yl)-2-methoxyphenyl)amino)-5-(trifluoromethyl)pyridin-4-yl)amino)-N-methylbenzamide | 2-((2-((4-(4-((2-(2,6-dioxopiperidin-3-yl)-3-oxo-1-thioxoisoindolin-5-yl)methyl)piperazin-1-yl)-2-methoxyphenyl)amino)-5-(trifluoromethyl)pyridin-4-yl)amino)-N-methylbenzamide |
| | 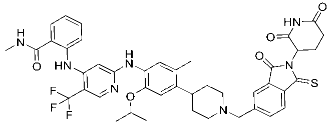 | 2-((2-((4-(1-((2-(2,6-dioxopiperidin-3-yl)-3-oxo-1-thioxoisoindolin-5-yl)methyl)piperidin-4-yl)-2-isopropoxy-5-methylphenyl)amino)-5-(trifluoromethyl)pyridin-4-yl)amino)-N-methylbenzamide | 2-((2-((4-(1-((2-(2,6-dioxopiperidin-3-yl)-3-oxo-1-thioxoisoindolin-5-yl)methyl)piperidin-4-yl)-2-isopropoxy-5-methylphenyl)amino)-5-(trifluoromethyl)pyridin-4-yl)amino)-N-methylbenzamide |
| | 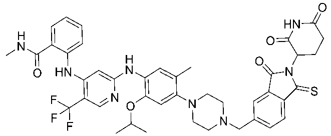 | 2-((2-((4-(4-((2-(2,6-dioxopiperidin-3-yl)-3-oxo-1-thioxoisoindolin-5-yl)methyl)piperazin-1-yl)-2-isopropoxy-5-methylphenyl)amino)-5-(trifluoromethyl)pyridin-4-yl)amino)-N-methylbenzamide | 2-((2-((4-(4-((2-(2,6-dioxopiperidin-3-yl)-3-oxo-1-thioxoisoindolin-5-yl)methyl)piperazin-1-yl)-2-isopropoxy-5-methylphenyl)amino)-5-(trifluoromethyl)pyridin-4-yl)amino)-N-methylbenzamide |
| | 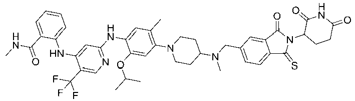 | 2-((2-((4-(4-(((2-(2,6-dioxopiperidin-3-yl)-3-oxo-1-thioxoisoindolin-5-yl)methyl)(methyl)amino )piperidin-1-yl)-2-isopropoxy-5-methylphenyl)amino)-5-(trifluoromethyl)pyridin-4-yl)amino)-N-methylbenzamide | 2-((2-((4-(4-(((2-(2,6-dioxopiperidin-3-yl)-3-oxo-1-thioxoisoindolin-5-yl)methyl)(methyl)amino )piperidin-1-yl)-2-isopropoxy-5-methylphenyl)amino)-5-(trifluoromethyl)pyridin-4-yl)amino)-N-methylbenzamide |
| | 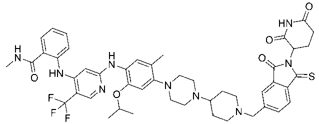 | 2-((2-((4-(4-(1-((2-(2,6-dioxopiperidin-3-yl)-3-oxo-1-thioxoisoindolin-5-yl)methyl)piperidin-4-yl)piperazin-1-yl)-2-isopropoxy-5-methylphenyl)amino)-5-(trifluoromethyl)pyridin-4-yl)amino)-N-methylbenzamide | 2-((2-((4-(4-(1-((2-(2,6-dioxopiperidin-3-yl)-3-oxo-1-thioxoisoindolin-5-yl)methyl)piperidin-4-yl)piperazin-1-yl)-2-isopropoxy-5-methylphenyl)amino)-5-(trifluoromethyl)pyridin-4-yl)amino)-N-methylbenzamide |
| | 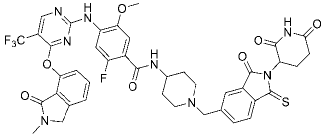 | N-(1-((2-(2,6-dioxopiperidin-3-yl)-3-oxo-1-thioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidi n-2-yl)amino)benzamide | N-(1-((2-(2,6-dioxopiperidin-3-yl)-3-oxo-1-thioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidi n-2-yl)amino)benzamide |
| | 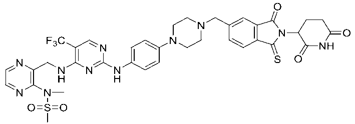 | N-(3-(((2-((4-(4-((2-(2,6-dioxopiperidin-3-yl)-3-oxo-1-thioxoisoindolin-5-yl)methyl)piperazin-1-yl)phenyl)amino)-5-(trifluoromethyl)pyrimidi n-4-yl)amino)methyl)pyrazin -2-yl)-N-methylmethanesulfonamide | N-(3-(((2-((4-(4-((2-(2,6-dioxopiperidin-3-yl)-3-oxo-1-thioxoisoindolin-5-yl)methyl)piperazin-1-yl)phenyl)amino)-5-(trifluoromethyl)pyrimidi n-4-yl)amino)methyl)pyrazin -2-yl)-N-methylmethanesulfonamide |
| | 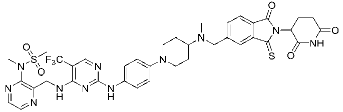 | N-(3-(((2-((4-(4-(((2-(2,6-dioxopiperidin-3-yl)-1-oxo-3-thioxoisoindolin-5-yl)methyl)(methyl)amino )piperidin-1-yl)phenyl)amino)-5-(trifluoromethyl)pyrimidi n-4-yl)amino)methyl)pyrazin -2-yl)-N-methylmethanesulfonamide | N-(3-(((2-((4-(4-(((2-(2,6-dioxopiperidin-3-yl)-1-oxo-3-thioxoisoindolin-5-yl)methyl)(methyl)amino )piperidin-1-yl)phenyl)amino)-5-(trifluoromethyl)pyrimidi n-4-yl)amino)methyl)pyrazin -2-yl)-N-methylmethanesulfonamide |
| | 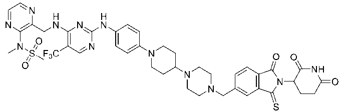 | N-(3-(((2-((4-(4-(4-((2-(2,6-dioxopiperidin-3-yl)-1-oxo-3-thioxoisoindolin-5-yl)methyl)piperazin-1-yl)piperidin-1-yl)phenyl)amino)-5-(trifluoromethyl)pyrimidi n-4-yl)amino)methyl)pyrazin -2-yl)-N-methylmethanesulfonamide | N-(3-(((2-((4-(4-(4-((2-(2,6-dioxopiperidin-3-yl)-1-oxo-3-thioxoisoindolin-5-yl)methyl)piperazin-1-yl)piperidin-1-yl)phenyl)amino)-5-(trifluoromethyl)pyrimidi n-4-yl)amino)methyl)pyrazin -2-yl)-N-methylmethanesulfonamide |
| | 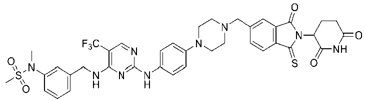 | N-(3-(((2-((4-(4-((2-(2,6-dioxopiperidin-3-yl)-3-oxo-1-thioxoisoindolin-5-yl)methyl)piperazin-1-yl)phenyl)amino)-5-(trifluoromethyl)pyrimidi n-4-yl)amino)methyl)phenyl) -N-methylmethanesulfonamide | N-(3-(((2-((4-(4-((2-(2,6-dioxopiperidin-3-yl)-3-oxo-1-thioxoisoindolin-5-yl)methyl)piperazin-1-yl)phenyl)amino)-5-(trifluoromethyl)pyrimidi n-4-yl)amino)methyl)phenyl) -N-methylmethanesulfonamide |
| | 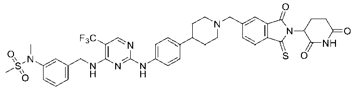 | N-(3-(((2-((4-(1-((2-(2,6-dioxopiperidin-3-yl)-3-oxo-1-thioxoisoindolin-5-yl)methyl)piperidin-4-yl)phenyl)amino)-5-(trifluoromethyl)pyrimidi n-4-yl)amino)methyl)phenyl) -N-methylmethanesulfonamide | N-(3-(((2-((4-(1-((2-(2,6-dioxopiperidin-3-yl)-3-oxo-1-thioxoisoindolin-5-yl)methyl)piperidin-4-yl)phenyl)amino)-5-(trifluoromethyl)pyrimidi n-4-yl)amino)methyl)phenyl) -N-methylmethanesulfonamide |
| | 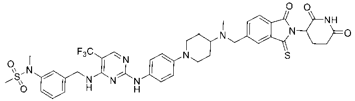 | N-(3-(((2-((4-(4-(((2-(2,6-dioxopiperidin-3-yl)-1-oxo-3-thioxoisoindolin-5-yl)methyl)(methyl)amino )piperidin-1-yl)phenyl)amino)-5-(trifluoromethyl)pyrimidi n-4-yl)amino)methyl)phenyl) -N-methylmethanesulfonami de | N-(3-(((2-((4-(4-(((2-(2,6-dioxopiperidin-3-yl)-1-oxo-3-thioxoisoindolin-5-yl)methyl)(methyl)amino )piperidin-1-yl)phenyl)amino)-5-(trifluoromethyl)pyrimidi n-4-yl)amino)methyl)phenyl) -N-methylmethanesulfonami de |
| | 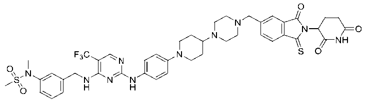 | N-(3-(((2-((4-(4-(4-((2-(2,6-dioxopiperidin-3-yl)-3-oxo-1-thioxoisoindolin-5-yl)methyl)piperazin-1-yl)piperidin-1-yl)phenyl)amino)-5-(trifluoromethyl)pyrimidi n-4-yl)amino)methyl)phenyl) -N-methylmethanesulfonami de | N-(3-(((2-((4-(4-(4-((2-(2,6-dioxopiperidin-3-yl)-3-oxo-1-thioxoisoindolin-5-yl)methyl)piperazin-1-yl)piperidin-1-yl)phenyl)amino)-5-(trifluoromethyl)pyrimidi n-4-yl)amino)methyl)phenyl) -N-methylmethanesulfonami de |
| | 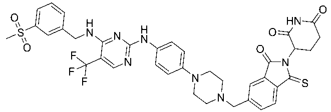 | 3-(5-((4-(4-((4-((3-(methylsulfonyl)benzyl)a mino)-5-(trifluoromethyl)pyrimidi n-2-yl)amino)phenyl)piperazi n-1-yl)methyl)-3-oxo-1-thioxoisoindolin-2-yl)piperidine-2,6-dione | 3-(5-((4-(4-((4-((3-(methylsulfonyl)benzyl)a mino)-5-(trifluoromethyl)pyrimidi n-2-yl)amino)phenyl)piperazi n-1-yl)methyl)-3-oxo-1-thioxoisoindolin-2-yl)piperidine-2,6-dione |
| | 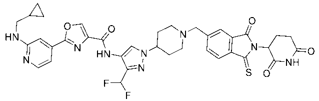 | 2-(2-((cyclopropylmethyl)ami no)pyridin-4-yl)-N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)oxazole-4-carboxamide | 2-(2-((cyclopropylmethyl)ami no)pyridin-4-yl)-N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)oxazole-4-carboxamide |
| | 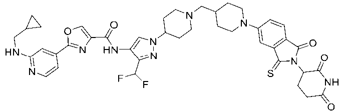 | 2-(2-((cyclopropylmethyl)ami no)pyridin-4-yl)-N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)oxazole-4-carboxamide | 2-(2-((cyclopropylmethyl)ami no)pyridin-4-yl)-N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)oxazole-4-carboxamide |
| | 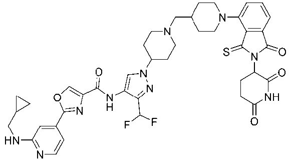 | 2-(2-((cyclopropylmethyl)ami no)pyridin-4-yl)-N-(3-(difluoromethyl)-1-(1-((1-(2-(2,6-dioxopiperidin-3-yl)-1-oxo-3-thioxoisoindolin-4-yl)piperidin-4-yl)methyl)piperidin-4-yl)-1H-pyrazol-4-yl)oxazole-4-carboxamide | 2-(2-((cyclopropylmethyl)ami no)pyridin-4-yl)-N-(3-(difluoromethyl)-1-(1-((1-(2-(2,6-dioxopiperidin-3-yl)-1-oxo-3-thioxoisoindolin-4-yl)piperidin-4-yl)methyl)piperidin-4-yl)-1H-pyrazol-4-yl)oxazole-4-carboxamide |
| | 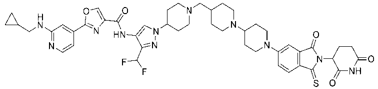 | 2-(2-((cyclopropylmethyl)ami no)pyridin-4-yl)-N-(3-(difluoromethyl)-1-(1-((1'-(2-(2,6-dioxopiperidin-3-yl)-3-oxo-1-thioxoisoindolin-5-yl)-[1,4'-bipiperidin]-4-yl)methyl)piperidin-4-yl)-1H-pyrazol-4-yl)oxazole-4-carboxamide | 2-(2-((cyclopropylmethyl)ami no)pyridin-4-yl)-N-(3-(difluoromethyl)-1-(1-((1'-(2-(2,6-dioxopiperidin-3-yl)-3-oxo-1-thioxoisoindolin-5-yl)-[1,4'-bipiperidin]-4-yl)methyl)piperidin-4-yl)-1H-pyrazol-4-yl)oxazole-4-carboxamide |
| | 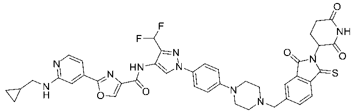 | 2-(2-((cyclopropylmethyl)ami no)pyridin-4-yl)-N-(3-(difluoromethyl)-1-(4-(4-((2-(2,6-dioxopiperidin-3-yl)-3-oxo-1-thioxoisoindolin-5-yl)methyl)piperazin-1-yl)phenyl)-1H-pyrazol-4-yl)oxazole-4-carboxamide | 2-(2-((cyclopropylmethyl)ami no)pyridin-4-yl)-N-(3-(difluoromethyl)-1-(4-(4-((2-(2,6-dioxopiperidin-3-yl)-3-oxo-1-thioxoisoindolin-5-yl)methyl)piperazin-1-yl)phenyl)-1H-pyrazol-4-yl)oxazole-4-carboxamide |
| | 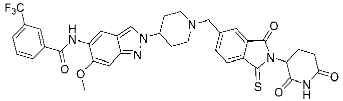 | N-(2-(1-((2-(2,6-dioxopiperidin-3-yl)-3-oxo-1-thioxoisoindolin-5-yl)methyl)piperidin-4-yl)-6-methoxy-2H-indazol-5-yl)-3-(trifluoromethyl)benzami de | N-(2-(1-((2-(2,6-dioxopiperidin-3-yl)-3-oxo-1-thioxoisoindolin-5-yl)methyl)piperidin-4-yl)-6-methoxy-2H-indazol-5-yl)-3-(trifluoromethyl)benzami de |
| | 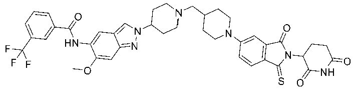 | N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-5-yl)-3-(trifluoromethyl)benzami de | N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-5-yl)-3-(trifluoromethyl)benzami de |
| | 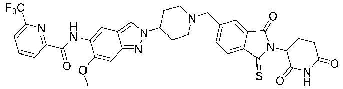 | N-(2-(1-((2-(2,6-dioxopiperidin-3-yl)-3-oxo-1-thioxoisoindolin-5-yl)methyl)piperidin-4-yl)-6-methoxy-2H-indazol-5-yl)-6- (trifluoromethyl)picolina mide | N-(2-(1-((2-(2,6-dioxopiperidin-3-yl)-3-oxo-1-thioxoisoindolin-5-yl)methyl)piperidin-4-yl)-6-methoxy-2H-indazol-5-yl)-6-(trifluoromethyl)picolina mide |
| | 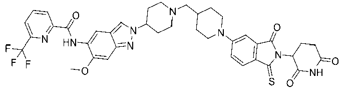 | N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-5-yl)-6-(trifluoromethyl)picolina mide | N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-5-yl)-6-(trifluoromethyl)picolina mide |
| | 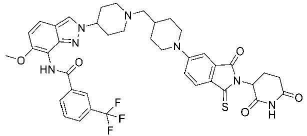 | N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-7-yl)-3-(trifluoromethyl)benzamide | N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-7-yl)-3-(trifluoromethyl)benzamide |
| | 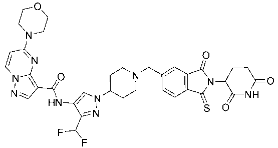 | N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)-5-morpholinopyrazolo[1,5-a]pyrimidine-3-carboxamide | N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)-5-morpholinopyrazolo[1,5-a]pyrimidine-3-carboxamide |
| | 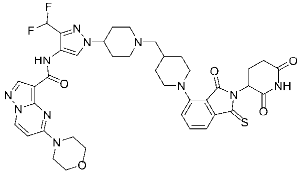 | N-(3-(difluoromethyl)-1-(1-((1-(2-(2,6-dioxopiperidin-3-yl)-3-oxo-1-thioxoisoindolin-4-yl)piperidin-4-yl)methyl)piperidin-4-yl)-1H-pyrazol-4-yl)-5-morpholinopyrazolo[1,5-a]pyrimidine-3-carboxamide | N-(3-(difluoromethyl)-1-(1-((1-(2-(2,6-dioxopiperidin-3-yl)-3-oxo-1-thioxoisoindolin-4-yl)piperidin-4-yl)methyl)piperidin-4-yl)-1H-pyrazol-4-yl)-5-morpholinopyrazolo[1,5-a]pyrimidine-3-carboxamide |
| | 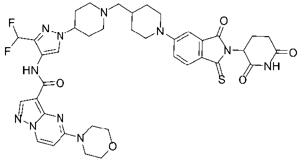 | N-(3-(difluoromethyl)-1-(1-((1-(2-(2,6-dioxopiperidin-3-yl)-3-oxo-1-thioxoisoindolin-5-yl)piperidin-4-yl)methyl)piperidin-4-yl)-1H-pyrazol-4-yl)-5-morpholinopyrazolo[1,5-a]pyrimidine-3-carboxamide | N-(3-(difluoromethyl)-1-(1-((1-(2-(2,6-dioxopiperidin-3-yl)-3-oxo-1-thioxoisoindolin-5-yl)piperidin-4-yl)methyl)piperidin-4-yl)-1H-pyrazol-4-yl)-5-morpholinopyrazolo[1,5-a]pyrimidine-3-carboxamide |
| | 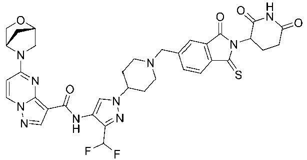 | 5-((1S,4S)-2-oxa-5-azabicyclo[2.2.1]heptan-5-yl)-N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)pyrazolo[1,5-a]pyrimidine-3-carboxamide | 5-((1S,4S)-2-oxa-5-azabicyclo[2.2.1]heptan-5-yl)-N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)pyrazolo[1,5-a]pyrimidine-3-carboxamide |
| | 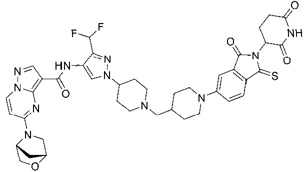 | 5-((1R,4R)-2-oxa-5-azabicyclo[2.2.1]heptan-5-yl)-N-(3-(difluoromethyl)-1-(1-((1-(2-(2,6-dioxopiperidin-3-yl)-3-oxo-1-thioxoisoindolin-5-yl)piperidin-4-yl)methyl)piperidin-4-yl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-(1-((1-(2-(2,6-dioxopiperidin-3-yl)-3-oxo-1-thioxoisoindolin-5-yl)piperidin-4-yl)methyl)piperidin-4-yl)-1H-pyrazol-4-yl)pyrazolo[1,5-a]pyrimidine-3-carboxamide |
| | 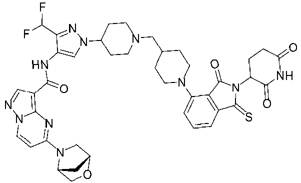 | 5-((1R,4R)-2-oxa-5-azabicyclo[2.2.1]heptan-5-yl)-N-(3-(difluoromethyl)-1-(1-((1-(2-(2,6-dioxopiperidin-3-yl)-3-oxo-1-thioxoisoindolin-4-yl)piperidin-4-yl)methyl)piperidin-4-yl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-(1-((1-(2-(2,6-dioxopiperidin-3-yl)-3-oxo-1-thioxoisoindolin-4-yl)piperidin-4-yl)methyl)piperidin-4-yl)-1H-pyrazol-4-yl)pyrazolo[1,5-a]pyrimidine-3-carboxamide |
| | 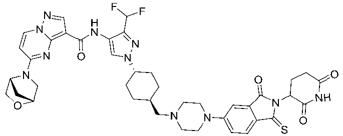 | 5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-3-oxo-1-thioxoisoindolin-5-yl)piperazin-1-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-3-oxo-1-thioxoisoindolin-5-yl)piperazin-1-yl)methyl)cyclohexyl)-1H-pyrazol-4-yl)pyrazolo[1,5-a]pyrimidine-3-carboxamide |
| | 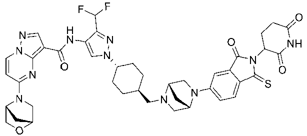 | 5-((1R,4R)-2-oxa-5-azabicyclo[2.2.1]heptan-5-yl)-N-(3-(difluoromethyl)-1-((1S,4R)-4-(((1S,4S)-5-(2-(2,6-dioxopiperidin-3-yl)-3-oxo-1-thioxoisoindolin-5-yl)-2,5-diazabicyclo[2.2.1]hepta n-2-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1S,4R)-4-(((1S,4S)-5-(2-(2,6-dioxopiperidin-3-yl)-3-oxo-1-thioxoisoindolin-5-yl)-2,5-diazabicyclo[2.2.1]hepta n-2-yl)methyl)cyclohexyl)-1H-pyrazol-4-yl)pyrazolo[1,5-a]pyrimidine-3-carboxamide |
| | 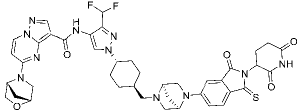 | 5-((1R,4R)-2-oxa-5-azabicyclo[2.2.1]heptan-5-yl)-N-(3-(difluoromethyl)-1-((1R,4R)-4-(((1R,4R)-5-(2-(2,6-dioxopiperidin-3-yl)-3-oxo-1-thioxoisoindolin-5-yl)-2,5-diazabicyclo[2.2.1]hepta n-2-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1R,4R)-4-(((1R,4R)-5-(2-(2,6-dioxopiperidin-3-yl)-3-oxo-1-thioxoisoindolin-5-yl)-2,5-diazabicyclo[2.2.1]hepta n-2-yl)methyl)cyclohexyl)-1H-pyrazol-4-yl)pyrazolo[1,5-a]pyrimidine-3-carboxamide |
| | 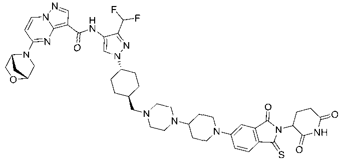 | 5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-3-oxo-1-thioxoisoindolin-5-yl)piperidin-4-yl)piperazin-1-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-3-oxo-1-thioxoisoindolin-5-yl)piperidin-4-yl)piperazin-1-yl)methyl)cyclohexyl)-1H-pyrazol-4-yl)pyrazolo[1,5-a]pyrimidine-3-carboxamide |
| | 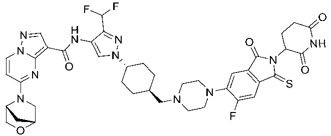 | 5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-6-fluoro-3-oxo-1-thioxoisoindolin-5-yl)piperazin-1-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-6-fluoro-3-oxo-1-thioxoisoindolin-5-yl)piperazin-1-yl)methyl)cyclohexyl)-1H-pyrazol-4-yl)pyrazolo[1,5-a]pyrimidine-3-carboxamide |
| | 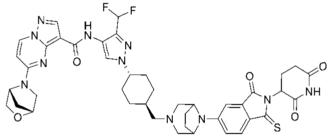 | 5-((1R,4R)-2-oxa-5-azabicyclo[2.2.1]heptan-5-yl)-N-(3-(difluoromethyl)-1-((1r,4R)-4-((8-(2-(2,6-dioxopiperidin-3-yl)-3-oxo-1-thioxoisoindolin-5-yl)-3,8-diazabicyclo[3.2.1]octan-3-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((8-(2-(2,6-dioxopiperidin-3-yl)-3-oxo-1-thioxoisoindolin-5-yl)-3,8-diazabicyclo[3.2.1]octan-3-yl)methyl)cyclohexyl)-1H-pyrazol-4-yl)pyrazolo[1,5-a]pyrimidine-3-carboxamide |
| | 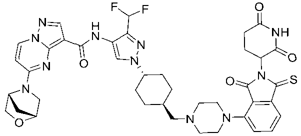 | 5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-3-oxo-1-thioxoisoindolin-4-yl)piperazin-1-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-3-oxo-1-thioxoisoindolin-4-yl)piperazin-1-yl)methyl)cyclohexyl)-1H-pyrazol-4-yl)pyrazolo[1,5-a]pyrimidine-3-carboxamide |
| | 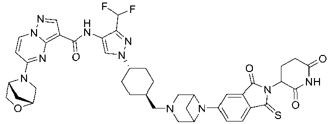 | 5-((1R,4R)-2-oxa-5-azabicyclo[2.2.1]heptan-5-yl)-N-(3-(difluoromethyl)-1-((1r,4R)-4-((6-(2-(2,6-dioxopiperidin-3-yl)-3-oxo-1-thioxoisoindolin-5-yl)-3,6-diazabicyclo[3.1.1]hepta n-3-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((6-(2-(2,6-dioxopiperidin-3-yl)-3-oxo-1-thioxoisoindolin-5-yl)-3,6-diazabicyclo[3.1.1]hepta n-3-yl)methyl)cyclohexyl)-1H-pyrazol-4-yl)pyrazolo[1,5-a]pyrimidine-3-carboxamide |
| | 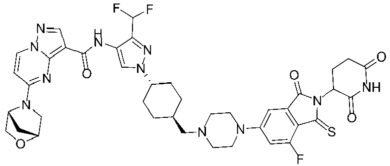 | 5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-7-fluoro-3-oxo-1-thioxoisoindolin-5-yl)piperazin-1-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-7-fluoro-3-oxo-1-thioxoisoindolin-5-yl)piperazin-1-yl)methyl)cyclohexyl)-1H-pyrazol-4-yl)pyrazolo[1,5-a]pyrimidine-3-carboxamide |
| | 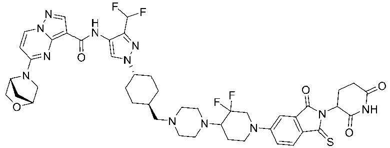 | 5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-3-oxo-1-thioxoisoindolin-5-yl)-3,3-difluoropiperidin-4-yl)piperazin-1-yl)methyl)cyclohexyl)-1H-pyrazol-4-yl)pyrazolo[1,5-a]pyrimidine-3-carboxamide | 5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-3-oxo-1-thioxoisoindolin-5-yl)-3,3-difluoropiperidin-4-yl)piperazin-1-yl)methyl)cyclohexyl)-1H-pyrazol-4-yl)pyrazolo[1,5-a]pyrimidine-3-carboxamide |
| | 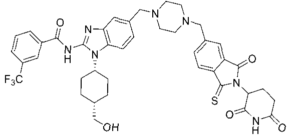 | N-(5-((4-((2-(2,6-dioxopiperidin-3-yl)-3-oxo-1-thioxoisoindolin-5-yl)methyl)piperazin-1-yl)methyl)-1-((1s,4s)-4-(hydroxymethyl)cyclohe xyl)-1H-benzo[d] 222 midazole-2-yl)-3-(trifluoromethyl)benzami de | N-(5-((4-((2-(2,6-dioxopiperidin-3-yl)-3-oxo-1-thioxoisoindolin-5-yl)methyl)piperazin-1-yl)methyl)-1-((1s,4s)-4-(hydroxymethyl)cyclohe xyl)-1H-benzo[d] 222 midazole-2-yl)-3-(trifluoromethyl)benzami de |
| | 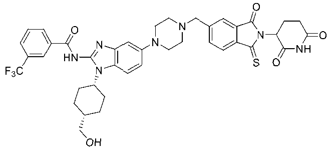 | N-(5-(4-((2-(2,6-dioxopiperidin-3-yl)-3-oxo-1-thioxoisoindolin-5-yl)methyl)piperazin-1-yl)-1-((1s,4s)-4-(hydroxymethyl)cyclohe xyl)-1H-benzo[d] 222 midazole-2-yl)-3-(trifluoromethyl)benzami de | N-(5-(4-((2-(2,6-dioxopiperidin-3-yl)-3-oxo-1-thioxoisoindolin-5-yl)methyl)piperazin-1-yl)-1-((1s,4s)-4-(hydroxymethyl)cyclohe xyl)-1H-benzo[d] 222 midazole-2-yl)-3-(trifluoromethyl)benzami de |
| | 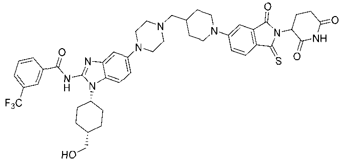 | N-(5-(4-((1-(2-(2,6-dioxopiperidin-3-yl)-3-oxo-1-thioxoisoindolin-5-yl)piperidin-4-yl)methyl)piperazin-1-yl)-1-((1s,4s)-4-(hydroxymethyl)cyclohe xyl)-1H-benzo[d] 222 midazole-2-yl)-3-(trifluoromethyl)benzamide | N-(5-(4-((1-(2-(2,6-dioxopiperidin-3-yl)-3-oxo-1-thioxoisoindolin-5-yl)piperidin-4-yl)methyl)piperazin-1-yl)-1-((1s,4s)-4-(hydroxymethyl)cyclohe xyl)-1H-benzo[d] 222 midazole-2-yl)-3-(trifluoromethyl)benzamide |
| | 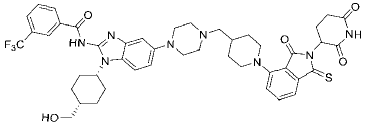 | N-(5-(4-((1-(2-(2,6-dioxopiperidin-3-yl)-3-oxo-1-thioxoisoindolin-4-yl)piperidin-4-yl)methyl)piperazin-1-yl)-1-((1s,4s)-4-(hydroxymethyl)cyclohe xyl)-1H-benzo[d]imidazol-2-yl)-3-(trifluoromethyl)benzamide | N-(5-(4-((1-(2-(2,6-dioxopiperidin-3-yl)-3-oxo-1-thioxoisoindolin-4-yl)piperidin-4-yl)methyl)piperazin-1-yl)-1-((1s,4s)-4-(hydroxymethyl)cyclohe xyl)-1H-benzo[d]imidazol-2-yl)-3-(trifluoromethyl)benzamide |
| | 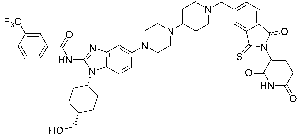 | N-(5-(4-(1-((2-(2,6-dioxopiperidin-3-yl)-1-oxo-3-thioxoisoindolin-5-yl)methyl)piperidin-4-yl)piperazin-1-yl)-1-((1s,4s)-4-(hydroxymethyl)cyclohe xyl)-1H-benzo[d]imidazol-2-yl)-3-(trifluoromethyl)benzamide | N-(5-(4-(1-((2-(2,6-dioxopiperidin-3-yl)-1-oxo-3-thioxoisoindolin-5-yl)methyl)piperidin-4-yl)piperazin-1-yl)-1-((1s,4s)-4-(hydroxymethyl)cyclohe xyl)-1H-benzo[d]imidazol-2-yl)-3-(trifluoromethyl)benzamide |
| | 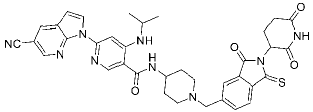 | 6-(5-cyano-1H-pyrrolo[2,3-b]pyridin-1-yl)-N-(1-((2-(2,6-dioxopiperidin-3-yl)-3-oxo-1-thioxoisoindolin-5-yl)methyl)piperidin-4-yl)-4-(isopropylamino)nicotinamide | 6-(5-cyano-1H-pyrrolo[2,3-b]pyridin-1-yl)-N-(1-((2-(2,6-dioxopiperidin-3-yl)-3-oxo-1-thioxoisoindolin-5-yl)methyl)piperidin-4-yl)-4-(isopropylamino)nicotinamide |
| | 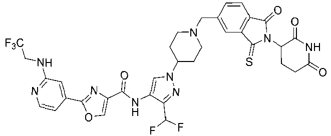 | N-(3-(difluoromethyl)-1-(1-((2-(2,6-dioxopiperidin-3-yl)-1-oxo-3-thioxoisoindolin-5-yl)methyl)piperidin-4-yl)-1H-pyrazol-4-yl)-2-(2-((2,2,2-trifluoroethyl)amino)pyri din-4-yl)oxazole-4-carboxamide | N-(3-(difluoromethyl)-1-(1-((2-(2,6-dioxopiperidin-3-yl)-1-oxo-3-thioxoisoindolin-5-yl)methyl)piperidin-4-yl)-1H-pyrazol-4-yl)-2-(2-((2,2,2-trifluoroethyl)amino)pyri din-4-yl)oxazole-4-carboxamide |
| | 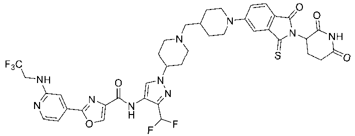 | N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)-2-(2-((2,2,2-trifluoroethyl)amino)pyri din-4-yl)oxazole-4-carboxamide | N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)-2-(2-((2,2,2-trifluoroethyl)amino)pyri din-4-yl)oxazole-4-carboxamide |
| | 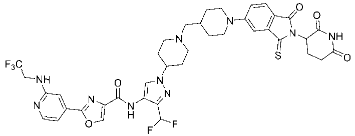 | N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)-2-(2-((2,2,2-trifluoroethyl)amino)pyri din-4-yl)oxazole-4-carboxamide | N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)-2-(2-((2,2,2-trifluoroethyl)amino)pyri din-4-yl)oxazole-4-carboxamide |
| | 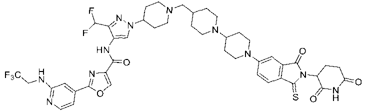 | N-(3-(difluoromethyl)-1-(1-((1'-(2-(2,6-dioxopiperidin-3-yl)-3-oxo-1-thioxoisoindolin-5-yl)-[1,4'-bipiperidin]-4-yl)methyl)piperidin-4-yl)-1H-pyrazol-4-yl)-2-(2-((2,2,2-trifluoroethyl)amino)pyri din-4-yl)oxazole-4-carboxamide | N-(3-(difluoromethyl)-1-(1-((1'-(2-(2,6-dioxopiperidin-3-yl)-3-oxo-1-thioxoisoindolin-5-yl)-[1,4'-bipiperidin]-4-yl)methyl)piperidin-4-yl)-1H-pyrazol-4-yl)-2-(2-((2,2,2-trifluoroethyl)amino)pyri din-4-yl)oxazole-4-carboxamide |
| | 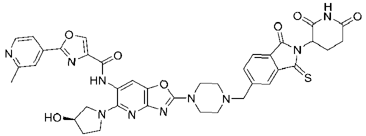 | N-(2-(4-((2-(2,6-dioxopiperidin-3-yl)-1-oxo-3-thioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide | N-(2-(4-((2-(2,6-dioxopiperidin-3-yl)-1-oxo-3-thioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide |
| | 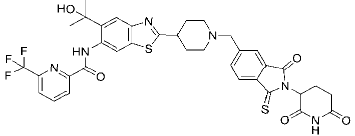 | N-(2-(1-((2-(2,6-dioxopiperidin-3-yl)-3-oxo-1-thioxoisoindolin-5-yl)methyl)piperidin-4-yl)-5-(2-hydroxypropan-2-yl)benzo[d]thiazol-6-yl)-6-(trifluoromethyl)picolina mide | N-(2-(1-((2-(2,6-dioxopiperidin-3-yl)-3-oxo-1-thioxoisoindolin-5-yl)methyl)piperidin-4-yl)-5-(2-hydroxypropan-2-yl)benzo[d]thiazol-6-yl)-6-(trifluoromethyl)picolina mide |
| | 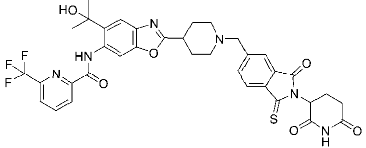 | N-(2-(1-((2-(2,6-dioxopiperidin-3-yl)-3-oxo-1-thioxoisoindolin-5-yl)methyl)piperidin-4-yl)-5-(2-hydroxypropan-2-yl)benzo[d]oxazol-6-yl)-6-(trifluoromethyl)picolina mide | N-(2-(1-((2-(2,6-dioxopiperidin-3-yl)-3-oxo-1-thioxoisoindolin-5-yl)methyl)piperidin-4-yl)-5-(2-hydroxypropan-2-yl)benzo[d]oxazol-6-yl)-6-(trifluoromethyl)picolina mide |
| | 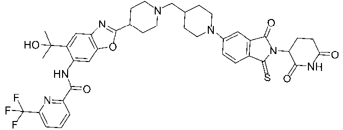 | N-(2-(1-((1-(2-(2,6-dioxopiperidin-3-yl)-3-oxo-1-thioxoisoindolin-5-yl)piperidin-4-yl)methyl)piperidin-4-yl)-5-(2-hydroxypropan-2-yl)benzo[d]oxazol-6-yl)-6-(trifluoromethyl)picolina mide | N-(2-(1-((1-(2-(2,6-dioxopiperidin-3-yl)-3-oxo-1-thioxoisoindolin-5-yl)piperidin-4-yl)methyl)piperidin-4-yl)-5-(2-hydroxypropan-2-yl)benzo[d]oxazol-6-yl)-6-(trifluoromethyl)picolina mide |
| | 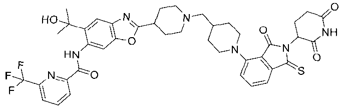 | N-(2-(1-((1-(2-(2,6-dioxopiperidin-3-yl)-3-oxo-1-thioxoisoindolin-4-yl)piperidin-4-yl)methyl)piperidin-4-yl)-5-(2-hydroxypropan-2-yl)benzo[d]oxazol-6-yl)-6-(trifluoromethyl)picolina mide | N-(2-(1-((1-(2-(2,6-dioxopiperidin-3-yl)-3-oxo-1-thioxoisoindolin-4-yl)piperidin-4-yl)methyl)piperidin-4-yl)-5-(2-hydroxypropan-2-yl)benzo[d]oxazol-6-yl)-6-(trifluoromethyl)picolina mide |
| | 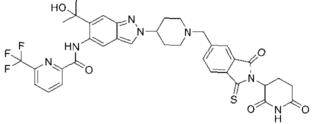 | N-(2-(1-((2-(2,6-dioxopiperidin-3-yl)-3-oxo-1-thioxoisoindolin-5-yl)methyl)piperidin-4-yl)-6-(2-hydroxypropan-2-yl)-2H-indazol-5-yl)-6-(trifluoromethyl)picolinamide | N-(2-(1-((2-(2,6-dioxopiperidin-3-yl)-3-oxo-1-thioxoisoindolin-5-yl)methyl)piperidin-4-yl)-6-(2-hydroxypropan-2-yl)-2H-indazol-5-yl)-6-(trifluoromethyl)picolinamide |
| | 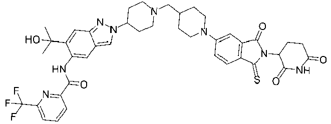 | N-(2-(1-((1-(2-(2,6-dioxopiperidin-3-yl)-3-oxo-1-thioxoisoindolin-5-yl)piperidin-4-yl)methyl)piperidin-4-yl)-6-(2-hydroxypropan-2-yl)-2H-indazol-5-yl)-6-(trifluoromethyl)picolina mide | N-(2-(1-((1-(2-(2,6-dioxopiperidin-3-yl)-3-oxo-1-thioxoisoindolin-5-yl)piperidin-4-yl)methyl)piperidin-4-yl)-6-(2-hydroxypropan-2-yl)-2H-indazol-5-yl)-6-(trifluoromethyl)picolina mide |
| | 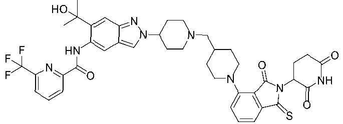 | N-(2-(1-((1-(2-(2,6-dioxopiperidin-3-yl)-3-oxo-1-thioxoisoindolin-4-yl)piperidin-4-yl)methyl)piperidin-4-yl)-6-(2-hydroxypropan-2-yl)-2H-indazol-5-yl)-6-(trifluoromethyl)picolina mide | N-(2-(1-((1-(2-(2,6-dioxopiperidin-3-yl)-3-oxo-1-thioxoisoindolin-4-yl)piperidin-4-yl)methyl)piperidin-4-yl)-6-(2-hydroxypropan-2-yl)-2H-indazol-5-yl)-6-(trifluoromethyl)picolina mide |
| | 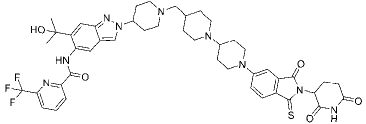 | N-(2-(1-((1'-(2-(2,6-dioxopiperidin-3-yl)-3-oxo-1-thioxoisoindolin-5-yl)-[1,4'-bipiperidin]-4-yl)methyl)piperidin-4-yl)-6-(2-hydroxypropan-2-yl)-2H-indazol-5-yl)-6-(trifluoromethyl)picolina mide | N-(2-(1-((1'-(2-(2,6-dioxopiperidin-3-yl)-3-oxo-1-thioxoisoindolin-5-yl)-[1,4'-bipiperidin]-4-yl)methyl)piperidin-4-yl)-6-(2-hydroxypropan-2-yl)-2H-indazol-5-yl)-6-(trifluoromethyl)picolina mide |
| | 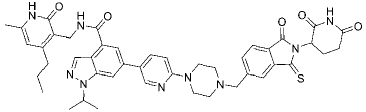 | 6-(6-(4-((2-(2,6-dioxopiperidin-3-yl)-1-oxo-3-thioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide | 6-(6-(4-((2-(2,6-dioxopiperidin-3-yl)-1-oxo-3-thioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide |
| | 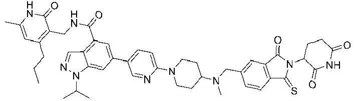 | 6-(6-(4-(((2-(2,6-dioxopiperidin-3-yl)-3-oxo-1-thioxoisoindolin-5-yl)methyl)(methyl)amino )piperidin-1-yl)pyridin-3-yl)-1-isopropyl-N-((6-methyl-2-oxo-4-propyl-1,2-dihydropyridin-3-yl)methyl)-1H-indazole-4-carboxamide | 6-(6-(4-(((2-(2,6-dioxopiperidin-3-yl)-3-oxo-1-thioxoisoindolin-5-yl)methyl)(methyl)amino )piperidin-1-yl)pyridin-3-yl)-1-isopropyl-N-((6-methyl-2-oxo-4-propyl-1,2-dihydropyridin-3-yl)methyl)-1H-indazole-4-carboxamide |
| | 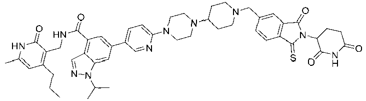 | 6-(6-(4-(1-((2-(2,6-dioxopiperidin-3-yl)-3-oxo-1-thioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide | 6-(6-(4-(1-((2-(2,6-dioxopiperidin-3-yl)-3-oxo-1-thioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide |
| | 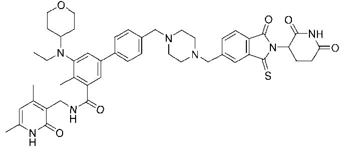 | N-((4,6-dimethyl-2-oxo-1,2-dihydropyridin-3-yl)methyl)-4'-((4-((2-(2,6-dioxopiperidin-3-yl)-1-oxo-3-thioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide | N-((4,6-dimethyl-2-oxo-1,2-dihydropyridin-3-yl)methyl)-4'-((4-((2-(2,6-dioxopiperidin-3-yl)-1-oxo-3-thioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide |
| | 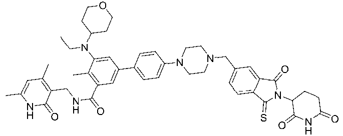 | N-((4,6-dimethyl-2-oxo-1,2-dihydropyridin-3-yl)methyl)-4'-(4-((2-(2,6-dioxopiperidin-3-yl)-3-oxo-1-thioxoisoindolin-5-yl)methyl)piperazin-1-yl)-5-(ethyl(tetrahydro-2H-pyran-4-yl)amino)-4-methyl-[1,1'-biphenyl]-3-carboxamide | N-((4,6-dimethyl-2-oxo-1,2-dihydropyridin-3-yl)methyl)-4'-(4-((2-(2,6-dioxopiperidin-3-yl)-3-oxo-1-thioxoisoindolin-5-yl)methyl)piperazin-1-yl)-5-(ethyl(tetrahydro-2H-pyran-4-yl)amino)-4-methyl-[1,1'-biphenyl]-3-carboxamide |
| | 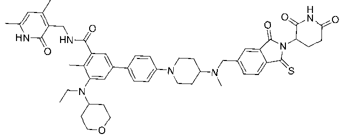 | N-((4,6-dimethyl-2-oxo-1,2-dihydropyridin-3-yl)methyl)-4'-(4-(((2-(2,6-dioxopiperidin-3-yl)-3-oxo-1-thioxoisoindolin-5-yl)methyl)(methyl)amino )piperidin-1-yl)-5-(ethyl(tetrahydro-2H-pyran-4-yl)amino)-4-methyl-[1,1'-biphenyl]-3-carboxamide | N-((4,6-dimethyl-2-oxo-1,2-dihydropyridin-3-yl)methyl)-4'-(4-(((2-(2,6-dioxopiperidin-3-yl)-3-oxo-1-thioxoisoindolin-5-yl)methyl)(methyl)amino )piperidin-1-yl)-5-(ethyl(tetrahydro-2H-pyran-4-yl)amino)-4-methyl-[1, 1'-biphenyl]-3-carboxamide |
| | 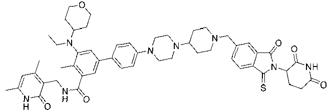 | N-((4,6-dimethyl-2-oxo-1,2-dihydropyridin-3-yl)methyl)-4'-(4-(1-((2-(2,6-dioxopiperidin-3-yl)-3-oxo-1-thioxoisoindolin-5-yl)methyl)piperidin-4-yl)piperazin-1-yl)-5-(ethyl(tetrahydro-2H-pyran-4-yl)amino)-4-methyl-[1,1'-biphenyl]-3-carboxamide | N-((4,6-dimethyl-2-oxo-1,2-dihydropyridin-3-yl)methyl)-4'-(4-(1-((2-(2,6-dioxopiperidin-3-yl)-3-oxo-1-thioxoisoindolin-5-yl)methyl)piperidin-4-yl)piperazin-1-yl)-5-(ethyl(tetrahydro-2H-pyran-4-yl)amino)-4-methyl-[1, 1'-biphenyl]-3-carboxamide |
| | 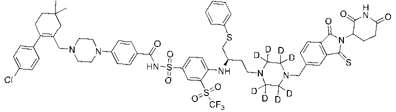 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)piperazin-1-yl-2,2,3,3,5,5,6,6-d8)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)piperazin-1-yl-2,2,3,3,5,5,6,6-d8)-1-(phenylthio)butan-2-yl)amino)-3-((trifluoromethyl)sulfony l)phenyl)sulfonyl)benza mide |
| | 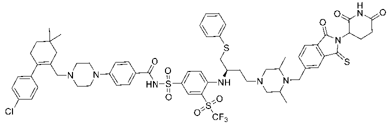 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)-3,5-dimethylpiperazin-1-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)-3,5-dimethylpiperazin-1-yl)-1-(phenylthio)butan-2-yl)amino)-3-((trifluoromethyl)sulfony l)phenyl)sulfonyl)benza mide |
| | 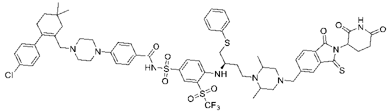 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)-2,6-dimethylpiperazin-1-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)-2,6-dimethylpiperazin-1-yl)-1-(phenylthio)butan-2-yl)amino)-3-((trifluoromethyl)sulfony l)phenyl)sulfonyl)benza mide |
| | 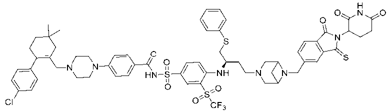 | 4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-1-oxo-3-thioxoisoindolin-5-yl)methyl)-3,6-diazabicyclo[3.1.1]hepta n-3-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-1-oxo-3-thioxoisoindolin-5-yl)methyl)-3,6-diazabicyclo[3.1.1]hepta n-3-yl)-1-(phenylthio)butan-2-yl)amino)-3-((trifluoromethyl)sulfony l)phenyl)sulfonyl)benza mide |
| | 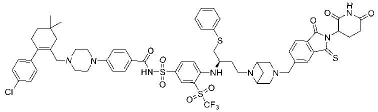 | 4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-oxo-3-thioxoisoindolin-5-yl)methyl)-3,6-diazabicyclo[3.1.1]hepta n-6-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-oxo-3-thioxoisoindolin-5-yl)methyl)-3,6-diazabicyclo[3.1.1]hepta n-6-yl)-1-(phenylthio)butan-2-yl)amino)-3-((trifluoromethyl)sulfony l)phenyl)sulfonyl)benza mide |
| | 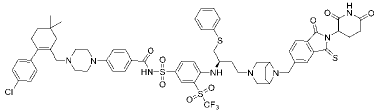 | 4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1-oxo-3-thioxoisoindolin-5-yl)methyl)-3,8-diazabicyclo[3.2.1]octan-3-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1-oxo-3-thioxoisoindolin-5-yl)methyl)-3,8-diazabicyclo[3.2.1]octan-3-yl)-1-(phenylthio)butan-2-yl)amino)-3-((trifluoromethyl)sulfony l)phenyl)sulfonyl)benza mide |
| | 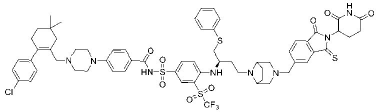 | 4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-oxo-3-thioxoisoindolin-5-yl)methyl)-3,8-diazabicyclo[3.2.1]octan-8-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-oxo-3-thioxoisoindolin-5-yl)methyl)-3,8-diazabicyclo[3.2.1]octan-8-yl)-1-(phenylthio)butan-2-yl)amino)-3-((trifluoromethyl)sulfony l)phenyl)sulfonyl)benza mide |
| | 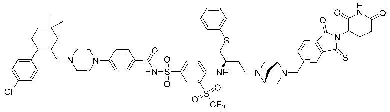 | 4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-1-oxo-3-thioxoisoindolin-5-yl)methyl)-2,5-diazabicyclo[2.2.1]hepta n-2-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-1-oxo-3-thioxoisoindolin-5-yl)methyl)-2,5-diazabicyclo[2.2.1]hepta n-2-yl)-1-(phenylthio)butan-2-yl)amino)-3-((trifluoromethyl)sulfony l)phenyl)sulfonyl)benza mide |
| | 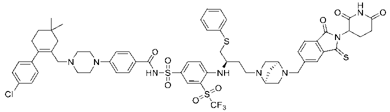 | 4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-1-oxo-3-thioxoisoindolin-5-yl)methyl)-2,5-diazabicyclo[2.2.1]hepta n-2-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-1-oxo-3-thioxoisoindolin-5-yl)methyl)-2,5-diazabicyclo[2.2.1]hepta n-2-yl)-1-(phenylthio)butan-2-yl)amino)-3-((trifluoromethyl)sulfony l)phenyl)sulfonyl)benza mide |
| | 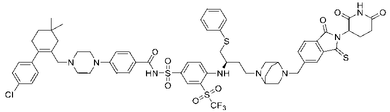 | 4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-1-oxo-3-thioxoisoindolin-5-yl)methyl)-2,5-diazabicyclo [2.2.2] octan-2-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-1-oxo-3-thioxoisoindolin-5-yl)methyl)-2,5-diazabicyclo[2.2.2]octan-2-yl)-1-(phenylthio)butan-2-yl)amino)-3-((trifluoromethyl)sulfony l)phenyl)sulfonyl)benza mide |
| | 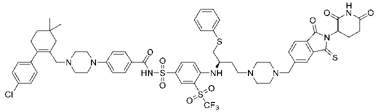 | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)piperazin-1-yl)-1-(phenylthio)butan-2-yl)amino)-3-((trifluoromethyl)sulfony l)phenyl)sulfonyl)benza mide | 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)piperazin-1-yl)-1-(phenylthio)butan-2-yl)amino)-3-((trifluoromethyl)sulfony l)phenyl)sulfonyl)benza mide |
| | 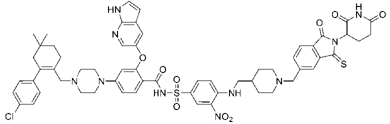 | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-(((1-((2-(2,6-dioxopiperidin-3-yl)-1-oxo-3-thioxoisoindolin-5-yl)methyl)piperidin-4-yl)methyl)amino)-3-nitrophenyl)sulfonyl)ben zamide | 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-(((1-((2-(2,6-dioxopiperidin-3-yl)-1-oxo-3-thioxoisoindolin-5-yl)methyl)piperidin-4-yl)methyl)amino)-3-nitrophenyl)sulfonyl)ben zamide |
| | 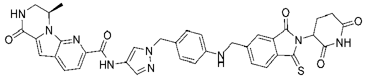 | (9R)-N-(1-(4-(((2-(2,6-dioxopiperidin-3-yl)-3-oxo-1-thioxoisoindolin-5-yl)methyl)amino)benzyl) -1H-pyrazol-4-yl)-9-methyl-6-oxo-6,7,8,9-tetrahydropyrido[3',2':4,5 ]pyrrolo[1,2-a]pyrazine-2-carboxamide | (9R)-N-(1-(4-(((2-(2,6-dioxopiperidin-3-yl)-3-oxo-1-thioxoisoindolin-5-yl)methyl)amino)benzyl) -1H-pyrazol-4-yl)-9-methyl-6-oxo-6,7,8,9-tetrahydropyrido[3',2':4,5 ]pyrrolo[1,2-a]pyrazine-2-carboxamide |
| | 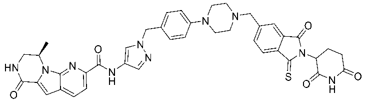 | (9R)-N-(1-(4-(4-((2-(2,6-dioxopiperidin-3-yl)-3-oxo-1-thioxoisoindolin-5-yl)methyl)piperazin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide | (9R)-N-(1-(4-(4-((2-(2,6-dioxopiperidin-3-yl)-3-oxo-1-thioxoisoindolin-5-yl)methyl)piperazin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide |
| | 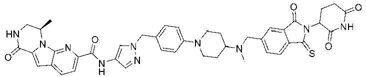 | (9R)-N-(1-(4-(4-(((2-(2,6-dioxopiperidin-3-yl)-3-oxo-1-thioxoisoindolin-5-yl)methyl)(methyl)amino )piperidin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide | (9R)-N-(1-(4-(4-(((2-(2,6-dioxopiperidin-3-yl)-3-oxo-1-thioxoisoindolin-5-yl)methyl)(methyl)amino )piperidin-1-yl)benzyl)-1H-pyrazol-4-yl)-9-methyl-6-oxo-6,7,8,9-tetrahydropyrido[3',2':4,5 ]pyrrolo[1,2-a]pyrazine-2-carboxamide |
| | 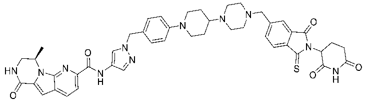 | (9R)-N-(1-(4-(4-(4-((2-(2,6-dioxopiperidin-3-yl)-3-oxo-1-thioxoisoindolin-5-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide | (9R)-N-(1-(4-(4-(4-((2-(2,6-dioxopiperidin-3-yl)-3-oxo-1-thioxoisoindolin-5-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide |
| | 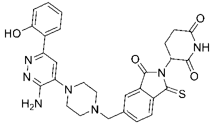 | 3-(5-((4-(3-amino-6-(2-hydroxyphenyl)pyridazin -4-yl)piperazin-1-yl)methyl)-3-oxo-1-thioxoisoindolin-2-yl)piperidine-2,6-dione | 3-(5-((4-(3-amino-6-(2-hydroxyphenyl)pyridazin -4-yl)piperazin-1-yl)methyl)-3-oxo-1-thioxoisoindolin-2-yl)piperidine-2,6-dione |
| | 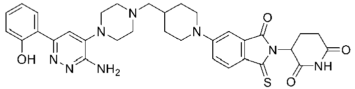 | 3-(5-(4-((4-(3-amino-6-(2-hydroxyphenyl)pyridazin -4-yl)piperazin-1-yl)methyl)piperidin-1-yl)-3-oxo-1-thioxoisoindolin-2-yl)piperidine-2,6-dione | 3-(5-(4-((4-(3-amino-6-(2-hydroxyphenyl)pyridazin -4-yl)piperazin-1-yl)methyl)piperidin-1-yl)-3-oxo-1-thioxoisoindolin-2-yl)piperidine-2,6-dione |
| | 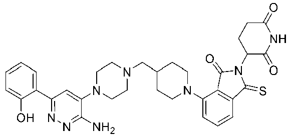 | 3-(4-(4-((4-(3-amino-6-(2-hydroxyphenyl)pyridazin -4-yl)piperazin-1-yl)methyl)piperidin-1-yl)-3-oxo-1-thioxoisoindolin-2-yl)piperidine-2,6-dione | 3-(4-(4-((4-(3-amino-6-(2-hydroxyphenyl)pyridazin -4-yl)piperazin-1-yl)methyl)piperidin-1-yl)-3-oxo-1-thioxoisoindolin-2-yl)piperidine-2,6-dione |
| | 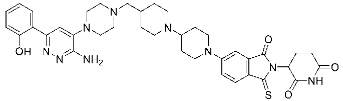 | 3-(5-(4-((4-(3-amino-6-(2-hydroxyphenyl)pyridazin -4-yl)piperazin-1-yl)methyl)-[1,4'-bipiperidin]-1'-yl)-3-oxo-1-thioxoisoindolin-2-yl)piperidine-2,6-dione | 3-(5-(4-((4-(3-amino-6-(2-hydroxyphenyl)pyridazin -4-yl)piperazin-1-yl)methyl)-[1,4'-bipiperidin]-1'-yl)-3-oxo-1-thioxoisoindolin-2-yl)piperidine-2,6-dione |
| | 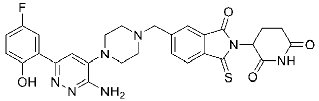 | 3-(5-((4-(3-amino-6-(5-fluoro-2-hydroxyphenyl)pyridazin -4-yl)piperazin-1-yl)methyl)-3-oxo-1-thioxoisoindolin-2-yl)piperidine-2,6-dione | 3-(5-((4-(3-amino-6-(5-fluoro-2-hydroxyphenyl)pyridazin -4-yl)piperazin-1-yl)methyl)-3-oxo-1-thioxoisoindolin-2-yl)piperidine-2,6-dione |
| | 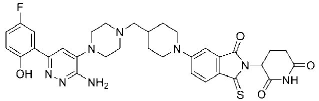 | 3-(5-(4-((4-(3-amino-6-(5-fluoro-2-hydroxyphenyl)pyridazin -4-yl)piperazin-1-yl)methyl)piperidin-1-yl)-3-oxo-1-thioxoisoindolin-2-yl)piperidine-2,6-dione | 3-(5-(4-((4-(3-amino-6-(5-fluoro-2-hydroxyphenyl)pyridazin -4-yl)piperazin-1-yl)methyl)piperidin-1-yl)-3-oxo-1-thioxoisoindolin-2-yl)piperidine-2,6-dione |
| | 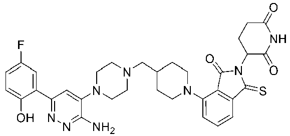 | 3-(4-(4-((4-(3-amino-6-(5-fluoro-2-hydroxyphenyl)pyridazin -4-yl)piperazin-1-yl)methyl)piperidin-1-yl)-3-oxo-1-thioxoisoindolin-2-yl)piperidine-2,6-dione | 3-(4-(4-((4-(3-amino-6-(5-fluoro-2-hydroxyphenyl)pyridazin -4-yl)piperazin-1-yl)methyl)piperidin-1-yl)-3-oxo-1-thioxoisoindolin-2-yl)piperidine-2,6-dione |
| | 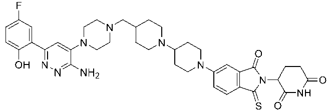 | 3-(5-(4-((4-(3-amino-6-(5-fluoro-2-hydroxyphenyl)pyridazin -4-yl)piperazin-1-yl)methyl)-[1,4'-bipiperidin]-1'-yl)-3-oxo-1-thioxoisoindolin-2-yl)piperidine-2,6-dione | 3-(5-(4-((4-(3-amino-6-(5-fluoro-2-hydroxyphenyl)pyridazin -4-yl)piperazin-1-yl)methyl)-[1,4'-bipiperidin]-1'-yl)-3-oxo-1-thioxoisoindolin-2-yl)piperidine-2,6-dione |
| GT-08758 | 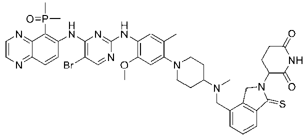 | 3-(4-(((1-(4-((5-bromo-4-((5-(dimethylphosphoryl)qui noxalin-6-yl)amino)pyrimidin-2-yl)amino)-5-methoxy-2-methylphenyl)piperidin-4-yl)(methyl)amino)methyl )-1-thioxoisoindolin-2-yl)piperidine-2,6-dione | 3-(4-(((1-(4-((5-bromo-4-((5-(dimethylphosphoryl)qui noxalin-6-yl)amino)pyrimidin-2-yl)amino)-5-methoxy-2-methylphenyl)piperidin-4-yl)(methyl)amino)methyl )-1-thioxoisoindolin-2-yl)piperidine-2,6-dione |
| GT-08761 | 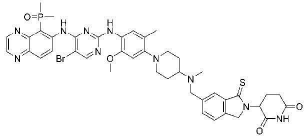 | 3-(6-(((1-(4-((5-bromo-4-((5-(dimethylphosphoryl)qui noxalin-6-yl)amino)pyrimidin-2-yl)amino)-5-methoxy-2-methylphenyl)piperidin-4-yl)(methyl)amino)methyl )-1-thioxoisoindolin-2-yl)piperidine-2,6-dione | 3-(6-(((1-(4-((5-bromo-4-((5-(dimethylphosphoryl)qui noxalin-6-yl)amino)pyrimidin-2-yl)amino)-5-methoxy-2-methylphenyl)piperidin-4-yl)(methyl)amino)methyl )-1-thioxoisoindolin-2-yl)piperidine-2,6-dione |
| GT-08762 | 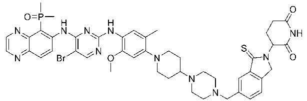 | 3-(6-((4-(1-(4-((5-bromo-4-((5-(dimethylphosphoryl)qui noxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione | 3-(6-((4-(1-(4-((5-bromo-4-((5-(dimethylphosphoryl)qui noxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione |
| GT-08760 | 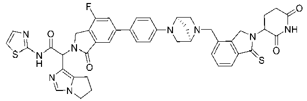 | 2-(6,7-dihydro-5H-pyrrolo[1,2-c]imidazol-1-yl)-2-(6-(4-((1R,4R)-5-((2-(2,6-dioxopiperidin-3-yl)-1-thioxoisoindolin-4-yl)methyl)-2,5-diazabicyclo[2.2.1]hepta n-2-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-((1R,4R)-5-((2-(2,6-dioxopiperidin-3-yl)-1-thioxoisoindolin-4-yl)methyl)-2,5-diazabicyclo[2.2.1]hepta n-2-yl)phenyl)-4-fluoro-1-oxoisoindolin-2-yl)-N-(thiazol-2-yl)acetamide |
| GT-08764 | 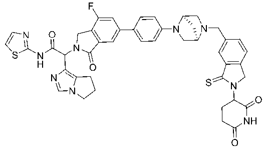 | 2-(6,7-dihydro-5H-pyrrolo[1,2-c]imidazol-1-yl)-2-(6-(4-((1R,4R)-5-((2-(2,6-dioxopiperidin-3-yl)-3-thioxoisoindolin-5-yl)methyl)-2,5-diazabicyclo[2.2.1]hepta n-2-yl)phenyl)-4-fluoro-1-oxoisoindolin-2-yl)-N-(thiazol-2-yl)acetamide | 2-(6,7-dihydro-5H-pyrrolo[1,2-c]imidazol-1-yl)-2-(6-(4-((1R,4R)-5-((2-(2,6-dioxopiperidin-3-yl)-3-thioxoisoindolin-5-yl)methyl)-2,5-diazabicyclo[2.2.1]hepta n-2-yl)phenyl)-4-fluoro-1-oxoisoindolin-2-yl)-N-(thiazol-2-yl)acetamide |
| GT-08759 | 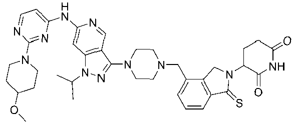 | 3-(4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione | 3-(4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione |
| GT-0876 3 | 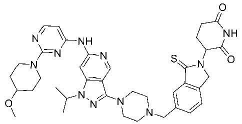 | 3-(6-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione | 3-(6-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione |
| GT-0871 4 | 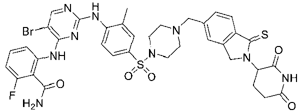 | 2-((5-bromo-2-((4-((4-((2-(2,6-dioxopiperidin-3-yl)-1-thioxoisoindolin-5-yl)methyl)piperazin-1-yl)sulfonyl)-2-methylphenyl)amino)pyr imidin-4-yl)amino)-6-fluorobenzamide | 2-((5-bromo-2-((4-((4-((2-(2,6-dioxopiperidin-3-yl)-1-thioxoisoindolin-5-yl)methyl)piperazin-1-yl)sulfonyl)-2-methylphenyl)amino)pyr imidin-4-yl)amino)-6-fluorobenzamide |
| GT-0879 3 | 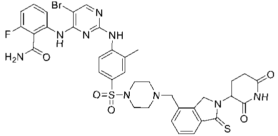 | 2-((5-bromo-2-((4-((4-((2-(2,6-dioxopiperidin-3-yl)-1-thioxoisoindolin-4-yl)methyl)piperazin-1-yl)sulfonyl)-2-methylphenyl)amino)pyr imidin-4-yl)amino)-6-fluorobenzamide | 2-((5-bromo-2-((4-((4-((2-(2,6-dioxopiperidin-3-yl)-1-thioxoisoindolin-4-yl)methyl)piperazin-1-yl)sulfonyl)-2-methylphenyl)amino)pyr imidin-4-yl)amino)-6-fluorobenzamide |
| GT-0879 4 | 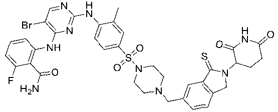 | 2-((5-bromo-2-((4-((4-((2-(2,6-dioxopiperidin-3-yl)-3-thioxoisoindolin-5-yl)methyl)piperazin-1-yl)sulfonyl)-2-methylphenyl)amino)pyr imidin-4-yl)amino)-6-fluorobenzamide | 2-((5-bromo-2-((4-((4-((2-(2,6-dioxopiperidin-3-yl)-3-thioxoisoindolin-5-yl)methyl)piperazin-1-yl)sulfonyl)-2-methylphenyl)amino)pyr imidin-4-yl)amino)-6-fluorobenzamide |
| GT-0879 5 | 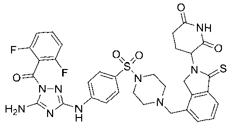 | 3-(4-((4-((4-((5-amino-1-(2,6-difluorobenzoyl)-1H-1,2,4-triazol-3-yl)amino)phenyl)sulfony 1)piperazin-1-yl)methyl)-1-thioxoisoindolin-2-yl)piperidine-2,6-dione | 3-(4-((4-((4-((5-amino-1-(2,6-difluorobenzoyl)-1H-1,2,4-triazol-3-yl)amino)phenyl)sulfony 1)piperazin-1-yl)methyl)-1-thioxoisoindolin-2-yl)piperidine-2,6-dione |
| GT-0879 6 | 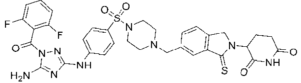 | 3-(6-((4-((4-((5-amino-1-(2,6-difluorobenzoyl)-1H-1,2,4-triazol-3-yl)amino)phenyl)sulfonyl)piperazin-1-yl)methyl)-1-thioxoisoindolin-2-yl)piperidine-2,6-dione | 3-(6-((4-((4-((5-amino-1-(2,6-difluorobenzoyl)-1H-1,2,4-triazol-3-yl)amino)phenyl)sulfonyl)piperazin-1-yl)methyl)-1-thioxoisoindolin-2-yl)piperidine-2,6-dione |
| GT-0879 2 | 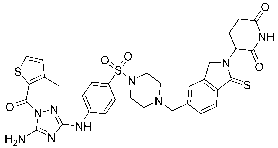 | 3-(5-((4-((4-((5-amino-1-(3-methylthiophene-2-carbonyl)-1H-1,2,4-triazol-3-yl)amino)phenyl)sulfony 1)piperazin-1-yl)methyl)-1-thioxoisoindolin-2-yl)piperidine-2,6-dione | 3-(5-((4-((4-((5-amino-1-(3-methylthiophene-2-carbonyl)-1H-1,2,4-triazol-3-yl)amino)phenyl)sulfony 1)piperazin-1-yl)methyl)-1-thioxoisoindolin-2-yl)piperidine-2,6-dione |
| GT-0879 7 | 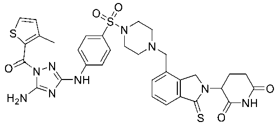 | 3-(4-((4-((4-((5-amino-1-(3-methylthiophene-2-carbonyl)-1H-1,2,4-triazol-3-yl)amino)phenyl)sulfony 1)piperazin-1-yl)methyl)-1-thioxoisoindolin-2-yl)piperidine-2,6-dione | 3-(4-((4-((4-((5-amino-1-(3-methylthiophene-2-carbonyl)-1H-1,2,4-triazol-3-yl)amino)phenyl)sulfony 1)piperazin-1-yl)methyl)-1-thioxoisoindolin-2-yl)piperidine-2,6-dione |
| GT-0879 8 | 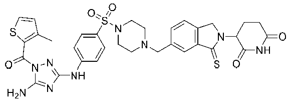 | 3-(6-((4-((4-((5-amino-1-(3-methylthiophene-2-carbonyl)-1H-1,2,4-triazol-3-yl)amino)phenyl)sulfony 1)piperazin-1-yl)methyl)-1-thioxoisoindolin-2-yl)piperidine-2,6-dione | 3-(6-((4-((4-((5-amino-1-(3-methylthiophene-2-carbonyl)-1H-1,2,4-triazol-3-yl)amino)phenyl)sulfony 1)piperazin-1-yl)methyl)-1-thioxoisoindolin-2-yl)piperidine-2,6-dione |
| GT-0877 5 | 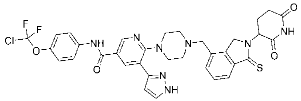 | N-(4-(chlorodifluoromethoxy) phenyl)-6-(4-((2-(2,6-dioxopiperidin-3-yl)-1-thioxoisoindolin-4-yl)methyl)piperazin-1-yl)-5-(1H-pyrazol-3-yl)nicotinamide | N-(4-(chlorodifluoromethoxy) phenyl)-6-(4-((2-(2,6-dioxopiperidin-3-yl)-1-thioxoisoindolin-4-yl)methyl)piperazin-1-yl)-5-(1H-pyrazol-3-yl)nicotinamide |
| GT-0877 6 | 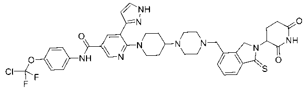 | N-(4-(chlorodifluoromethoxy) phenyl)-6-(4-(4-((2-(2,6-dioxopiperidin-3-yl)-1-thioxoisoindolin-4-yl)methyl)piperazin-1-yl)piperidin-1-yl)-5-(1H-pyrazol-3-yl)nicotinamide | N-(4-(chlorodifluoromethoxy) phenyl)-6-(4-(4-((2-(2,6-dioxopiperidin-3-yl)-1-thioxoisoindolin-4-yl)methyl)piperazin-1-yl)piperidin-1-yl)-5-(1H-pyrazol-3-yl)nicotinamide |
| GT-0878 4 | 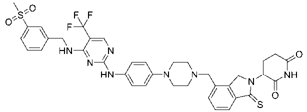 | 3-(4-((4-(4-((4-((3-(methylsulfonyl)benzyl)a mino)-5-(trifluoromethyl)pyrimidi n-2-yl)amino)phenyl)piperazi n-1-yl)methyl)-1-thioxoisoindolin-2-yl)piperidine-2,6-dione | 3-(4-((4-(4-((4-((3-(methylsulfonyl)benzyl)a mino)-5-(trifluoromethyl)pyrimidi n-2-yl)amino)phenyl)piperazi n-1-yl)methyl)-1-thioxoisoindolin-2-yl)piperidine-2,6-dione |
| GT-0878 6 | 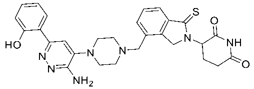 | 3-(4-((4-(3-amino-6-(2-hydroxyphenyl)pyridazin -4-yl)piperazin-1-yl)methyl)-1-thioxoisoindolin-2-yl)piperidine-2,6-dione | 3-(4-((4-(3-amino-6-(2-hydroxyphenyl)pyridazin -4-yl)piperazin-1-yl)methyl)-1-thioxoisoindolin-2-yl)piperidine-2,6-dione |
| GT-0878 7 | 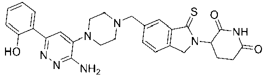 | 3-(6-((4-(3-amino-6-(2-hydroxyphenyl)pyridazin -4-yl)piperazin-1-yl)methyl)-1-thioxoisoindolin-2-yl)piperidine-2,6-dione | 3-(6-((4-(3-amino-6-(2-hydroxyphenyl)pyridazin -4-yl)piperazin-1-yl)methyl)-1-thioxoisoindolin-2-yl)piperidine-2,6-dione |
| GT-0878 8 | 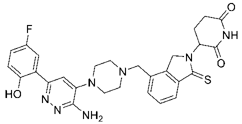 | 3-(4-((4-(3-amino-6-(5-fluoro-2-hydroxyphenyl)pyridazin -4-yl)piperazin-1-yl)methyl)-1-thioxoisoindolin-2-yl)piperidine-2,6-dione | 3-(4-((4-(3-amino-6-(5-fluoro-2-hydroxyphenyl)pyridazin -4-yl)piperazin-1-yl)methyl)-1-thioxoisoindolin-2-yl)piperidine-2,6-dione |
| GT-0878 9 | 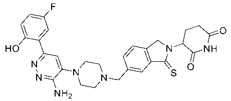 | 3-(6-((4-(3-amino-6-(5-fluoro-2-hydroxyphenyl)pyridazin -4-yl)piperazin-1-yl)methyl)-1-thioxoisoindolin-2-yl)piperidine-2,6-dione | 3-(6-((4-(3-amino-6-(5-fluoro-2-hydroxyphenyl)pyridazin -4-yl)piperazin-1-yl)methyl)-1-thioxoisoindolin-2-yl)piperidine-2,6-dione |
| GT-0871 3 | 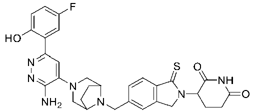 | 3-(5-((3-(3-amino-6-(5-fluoro-2-hydroxyphenyl)pyridazin -4-yl)-3,8-diazabicyclo[3.2.1]octan-8-yl)methyl)-1-thioxoisoindolin-2-yl)piperidine-2,6-dione | 3-(5-((3-(3-amino-6-(5-fluoro-2-hydroxyphenyl)pyridazin -4-yl)-3,8-diazabicyclo[3.2.1]octan-8-yl)methyl)-1-thioxoisoindolin-2-yl)piperidine-2,6-dione |
| GT-0879 0 | 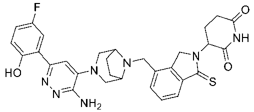 | 3-(4-((3-(3-amino-6-(5-fluoro-2-hydroxyphenyl)pyridazin -4-yl)-3,8-diazabicyclo[3.2.1]octan-8-yl)methyl)-1-thioxoisoindolin-2-yl)piperidine-2,6-dione | 3-(4-((3-(3-amino-6-(5-fluoro-2-hydroxyphenyl)pyridazin -4-yl)-3,8-diazabicyclo[3.2.1]octan-8-yl)methyl)-1-thioxoisoindolin-2-yl)piperidine-2,6-dione |
| GT-0879 1 | 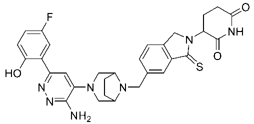 | 3-(6-((3-(3-amino-6-(5-fluoro-2-hydroxyphenyl)pyridazin -4-yl)-3,8-diazabicyclo[3.2.1]octan-8-yl)methyl)-1-thioxoisoindolin-2-yl)piperidine-2,6-dione | 3-(6-((3-(3-amino-6-(5-fluoro-2-hydroxyphenyl)pyridazin -4-yl)-3,8-diazabicyclo[3.2.1]octan-8-yl)methyl)-1-thioxoisoindolin-2-yl)piperidine-2,6-dione |
| GT-0867 4 | 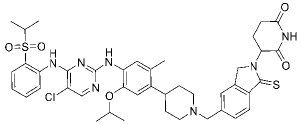 | 3-(5-((4-(4-((5-chloro-4-((2-(isopropylsulfonyl)pheny 1)amino)pyrimidin-2-yl)amino)-5-isopropoxy-2-methylphenyl)piperidin-1-yl)methyl)-1-thioxoisoindolin-2-yl)piperidine-2,6-dione | 3-(5-((4-(4-((5-chloro-4-((2-(isopropylsulfonyl)pheny 1)amino)pyrimidin-2-yl)amino)-5-isopropoxy-2-methylphenyl)piperidin-1-yl)methyl)-1-thioxoisoindolin-2-yl)piperidine-2,6-dione |
| GT-0867 6 | 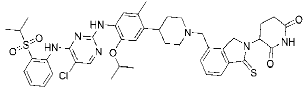 | 3-(4-((4-(4-((5-chloro-4-((2-(isopropylsulfonyl)pheny 1)amino)pyrimidin-2-yl)amino)-5-isopropoxy-2-methylphenyl)piperidin-1-yl)methyl)-1-thioxoisoindolin-2-yl)piperidine-2,6-dione | 3-(4-((4-(4-((5-chloro-4-((2-(isopropylsulfonyl)pheny 1)amino)pyrimidin-2-yl)amino)-5-isopropoxy-2-methylphenyl)piperidin-1-yl)methyl)-1-thioxoisoindolin-2-yl)piperidine-2,6-dione |
| GT-0867 8 | 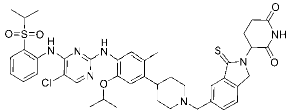 | 3-(6-((4-(4-((5-chloro-4-((2-(isopropylsulfonyl)pheny 1)amino)pyrimidin-2-yl)amino)-5-isopropoxy-2-methylphenyl)piperidin-1-yl)methyl)-1-thioxoisoindolin-2-yl)piperidine-2,6-dione | 3-(6-((4-(4-((5-chloro-4-((2-(isopropylsulfonyl)pheny 1)amino)pyrimidin-2-yl)amino)-5-isopropoxy-2-methylphenyl)piperidin-1-yl)methyl)-1-thioxoisoindolin-2-yl)piperidine-2,6-dione |
| GT-0867 7 | 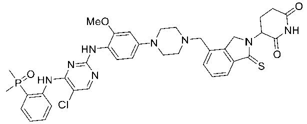 | 3-(4-((4-(4-((5-chloro-4-((2-(dimethylphosphoryl)phe nyl)amino)pyrimidin-2-yl)amino)-3-methoxyphenyl)piperazi n-1-yl)methyl)-1-thioxoisoindolin-2-yl)piperidine-2,6-dione | 3-(4-((4-(4-((5-chloro-4-((2-(dimethylphosphoryl)phe nyl)amino)pyrimidin-2-yl)amino)-3-methoxyphenyl)piperazi n-1-yl)methyl)-1-thioxoisoindolin-2-yl)piperidine-2,6-dione |
| GT-0867 9 | 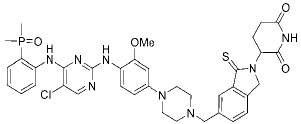 | 3-(6-((4-(4-((5-chloro-4-((2-(dimethylphosphoryl)phe nyl)amino)pyrimidin-2-yl)amino)-3-methoxyphenyl)piperazi n-1-yl)methyl)-1-thioxoisoindolin-2-yl)piperidine-2,6-dione | 3-(6-((4-(4-((5-chloro-4-((2-(dimethylphosphoryl)phe nyl)amino)pyrimidin-2-yl)amino)-3-methoxyphenyl)piperazi n-1-yl)methyl)-1-thioxoisoindolin-2-yl)piperidine-2,6-dione |
| GT-0868 0 | 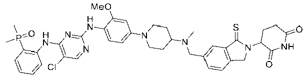 | 3-(6-(((1-(4-((5-chloro-4-((2-(dimethylphosphoryl)phe nyl)amino)pyrimidin-2-yl)amino)-3-methoxyphenyl)piperidin -4-yl)(methyl)amino)methyl )-1-thioxoisoindolin-2-yl)piperidine-2,6-dione | 3-(6-(((1-(4-((5-chloro-4-((2-(dimethylphosphoryl)phe nyl)amino)pyrimidin-2-yl)amino)-3-methoxyphenyl)piperidin -4-yl)(methyl)amino)methyl )-1-thioxoisoindolin-2-yl)piperidine-2,6-dione |
| GT-0898 2 | 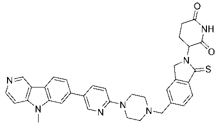 | 3-(5-((4-(5-(5-methyl-5H-pyrido[4,3-b]indol-7-yl)pyridin-2-yl)piperazin-1-yl)methyl)-1-thioxoisoindolin-2-yl)piperidine-2,6-dione | 3-(5-((4-(5-(5-methyl-5H-pyrido[4,3-b]indol-7-yl)pyridin-2-yl)piperazin-1-yl)methyl)-1-thioxoisoindolin-2-yl)piperidine-2,6-dione |
| GT-0897 9 | 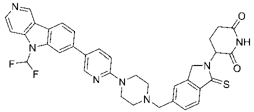 | 3-(5-((4-(5-(5-(difluoromethyl)-5H-pyrido[4,3-b]indol-7-yl)pyridin-2-yl)piperazin-1-yl)methyl)-1-thioxoisoindolin-2-yl)piperidine-2,6-dione | 3-(5-((4-(5-(5-(difluoromethyl)-5H-pyrido[4,3-b]indol-7-yl)pyridin-2-yl)piperazin-1-yl)methyl)-1-thioxoisoindolin-2-yl)piperidine-2,6-dione |
| GT-0898 0 | 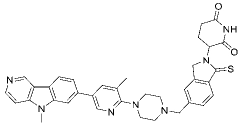 | 3-(5-((4-(3-methyl-5-(5-methyl-5H-pyrido[4,3-b]indol-7-yl)pyridin-2-yl)piperazin-1-yl)methyl)-1-thioxoisoindolin-2-yl)piperidine-2,6-dione | 3-(5-((4-(3-methyl-5-(5-methyl-5H-pyrido[4,3-b]indol-7-yl)pyridin-2-yl)piperazin-1-yl)methyl)-1-thioxoisoindolin-2-yl)piperidine-2,6-dione |
| GT-0897 8 | 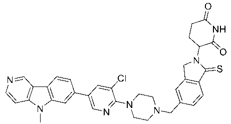 | 3-(5-((4-(3-chloro-5-(5-methyl-5H-pyrido[4,3-b]indol-7-yl)pyridin-2-yl)piperazin-1-yl)methyl)-1-thioxoisoindolin-2-yl)piperidine-2,6-dione | 3-(5-((4-(3-chloro-5-(5-methyl-5H-pyrido[4,3-b]indol-7-yl)pyridin-2-yl)piperazin-1-yl)methyl)-1-thioxoisoindolin-2-yl)piperidine-2,6-dione |
| GT-0898 1 | 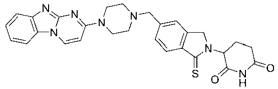 | 3-(5-((4-(benzo[4,5]imidazo[1,2-a]pyrimidin-2-yl)piperazin-1-yl)methyl)-1-thioxoisoindolin-2-yl)piperidine-2,6-dione | 3-(5-((4-(benzo[4,5]imidazo[1,2-a]pyrimidin-2-yl)piperazin-1-yl)methyl)-1-thioxoisoindolin-2-yl)piperidine-2,6-dione |
| GT-0889 5 | 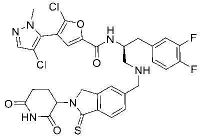 | 5-chloro-4-(4-chloro-1-methyl-1H-pyrazol-5-yl)-N-((2S)-1-(3,4-difluorophenyl)-3-(((2-(2,6-dioxopiperidin-3-yl)-1-thioxoisoindolin-5-yl)methyl)amino)propan-2-yl)furan-2-carboxamide | 5-chloro-4-(4-chloro-1-methyl-1H-pyrazol-5-yl)-N-((2S)-1-(3,4-difluorophenyl)-3-(((2-(2,6-dioxopiperidin-3-yl)-1-thioxoisoindolin-5-yl)methyl)amino)propan-2-yl)furan-2-carboxamide |
| GT-0889 6 | 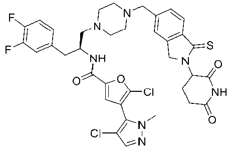 | 5-chloro-4-(4-chloro-1-methyl-1H-pyrazol-5-yl)-N-((2S)-1-(3,4-difluorophenyl)-3-(4-((2-(2,6-dioxopiperidin-3-yl)-1-thioxoisoindolin-5-yl)methyl)piperazin-1-yl)propan-2-yl)furan-2-carboxamide | 5-chloro-4-(4-chloro-1-methyl-1H-pyrazol-5-yl)-N-((2S)-1-(3,4-difluorophenyl)-3-(4-((2-(2,6-dioxopiperidin-3-yl)-1-thioxoisoindolin-5-yl)methyl)piperazin-1-yl)propan-2-yl)furan-2-carboxamide |
| GT-0889 7 | 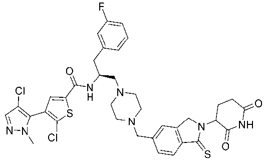 | 5-chloro-4-(4-chloro-1-methyl-1H-pyrazol-5-yl)-N-((2S)-1-(4-((2-(2,6-dioxopiperidin-3-yl)-1-thioxoisoindolin-5-yl)methyl)piperazin-1-yl)-3-(3-fluorophenyl)propan-2-yl)thiophene-2-carboxamide | 5-chloro-4-(4-chloro-1-methyl-1H-pyrazol-5-yl)-N-((2S)-1-(4-((2-(2,6-dioxopiperidin-3-yl)-1-thioxoisoindolin-5-yl)methyl)piperazin-1-yl)-3-(3-fluorophenyl)propan-2-yl)thiophene-2-carboxamide |
| GT-0868 5 | 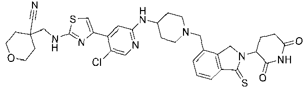 | 4-(((4-(5-chloro-2-((1-((2-(2,6-dioxopiperidin-3-yl)-1-thioxoisoindolin-4-yl)methyl)piperidin-4-yl)amino)pyridin-4-yl)thiazol-2-yl)amino)methyl)tetrahy dro-2H-pyran-4-carbonitrile | 4-(((4-(5-chloro-2-((1-((2-(2,6-dioxopiperidin-3-yl)-1-thioxoisoindolin-4-yl)methyl)piperidin-4-yl)amino)pyridin-4-yl)thiazol-2-yl)amino)methyl)tetrahy dro-2H-pyran-4-carbonitrile |
| GT-0875 0 | 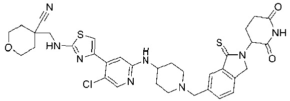 | 4-(((4-(5-chloro-2-((1-((2-(2,6-dioxopiperidin-3-yl)-3-thioxoisoindolin-5-yl)methyl)piperidin-4-yl)amino)pyridin-4-yl)thiazol-2-yl)amino)methyl)tetrahy dro-2H-pyran-4-carbonitrile | 4-(((4-(5-chloro-2-((1-((2-(2,6-dioxopiperidin-3-yl)-3-thioxoisoindolin-5-yl)methyl)piperidin-4-yl)amino)pyridin-4-yl)thiazol-2-yl)amino)methyl)tetrahy dro-2H-pyran-4-carbonitrile |
| GT-0868 6 | 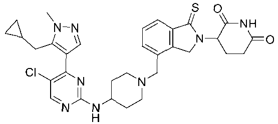 | 3-(4-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione | 3-(4-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione |
| GT-0875 1 | 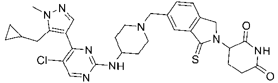 | 3-(6-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione | 3-(6-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione |
| GT-0875 2 | 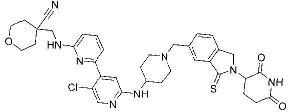 | 4-(((5'-chloro-2'-((1-((2-(2,6-dioxopiperidin-3-yl)-3-thioxoisoindolin-5-yl)methyl)piperidin-4-yl)amino)-[2,4'-bipyridin]-6-yl)amino)methyl)tetrahy dro-2H-pyran-4-carbonitrile | 4-(((5'-chloro-2'-((1-((2-(2,6-dioxopiperidin-3-yl)-3-thioxoisoindolin-5-yl)methyl)piperidin-4-yl)amino)-[2,4'-bipyridin]-6-yl)amino)methyl)tetrahy dro-2H-pyran-4-carbonitrile |
| GT-0868 4 | 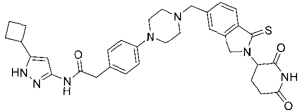 | N-(5-cyclobutyl-1H-pyrazol-3-yl)-2-(4-(4-((2-(2,6-dioxopiperidin-3-yl)-1-thioxoisoindolin-5-yl)methyl)piperazin-1-yl)phenyl)acetamide | N-(5-cyclobutyl-1H-pyrazol-3-yl)-2-(4-(4-((2-(2,6-dioxopiperidin-3-yl)-1-thioxoisoindolin-5-yl)methyl)piperazin-1-yl)phenyl)acetamide |
| GT-0868 8 | 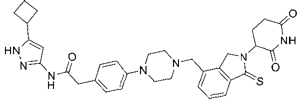 | N-(5-cyclobutyl-1H-pyrazol-3-yl)-2-(4-(4-((2-(2,6-dioxopiperidin-3-yl)-1-thioxoisoindolin-4-yl)methyl)piperazin-1-yl)phenyl)acetamide | N-(5-cyclobutyl-1H-pyrazol-3-yl)-2-(4-(4-((2-(2,6-dioxopiperidin-3-yl)-1-thioxoisoindolin-4-yl)methyl)piperazin-1-yl)phenyl)acetamide |
| GT-0875 3 | 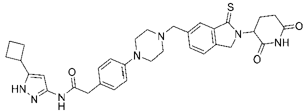 | N-(5-cyclobutyl-1H-pyrazol-3-yl)-2-(4-(4-((2-(2,6-dioxopiperidin-3-yl)-3-thioxoisoindolin-5-yl)methyl)piperazin-1-yl)phenyl)acetamide | N-(5-cyclobutyl-1H-pyrazol-3-yl)-2-(4-(4-((2-(2,6-dioxopiperidin-3-yl)-3-thioxoisoindolin-5-yl)methyl)piperazin-1-yl)phenyl)acetamide |
| GT-0886 7 | 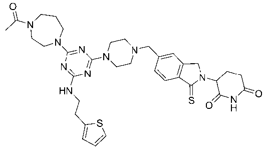 | 3-(5-((4-(4-(4-acetyl-1,4-diazepan-1-yl)-6-((2-(thiophen-2-yl)ethyl)amino)-1,3,5-triazin-2-yl)piperazin-1-yl)methyl)-1-thioxoisoindolin-2-yl)piperidine-2,6-dione | 3-(5-((4-(4-(4-acetyl-1,4-diazepan-1-yl)-6-((2-(thiophen-2-yl)ethyl)amino)-1,3,5-triazin-2-yl)piperazin-1-yl)methyl)-1-thioxoisoindolin-2-yl)piperidine-2,6-dione |

### Compound of Formula (II)

The present disclosure provides a compound of Formula (II) or salts (including pharmaceutically acceptable salts), stereoisomers (including enantiomers), solvates, isotopically enriched analogs, prodrugs, or polymorphs thereof: 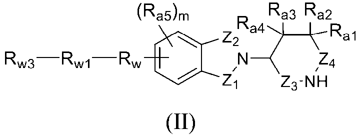
wherein Z₁ represents C(O), C(S), CH₂ or CD₂; Z₂, Z₃, and Z₄ each independently represent C(O) or C(S), wherein at least one of Z₁, Z₂, Z₃, and Z₄ represents C(S);
Rₐ₁, Rₐ₂, Rₐ₃, and Rₐ₄ are identical or different and each independently represent hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or deuterated C₁₋₆ alkoxy;
(Rₐ₅)ₘ indicates that the isoindoline ring to which it is attached is optionally substituted with m Rₐ₅, with each Rₐ₅ being identical or different and each independently representing halogen (e.g., fluorine, chlorine, bromine, or iodine), hydroxy, mercapto, nitro, amino, cyano, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), deuterated C₁₋₆ alkoxy, C₂₋₆ alkenyl, or C₂₋₆ alkynyl;
m represents an integer of 0, 1, 2 or 3;
R_{w} represents O, S, S(O), S(O)₂, alkenylene (e.g., C₂₋₆ alkenylene), alkynylene (e.g., C₂₋₆ alkynylene) or N(Rₐ₆), wherein Rₐ₆ represents H or C₁₋₃ alkyl; or
R_{w} represents a bond; or
R_{w} represents: 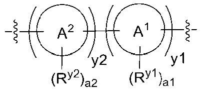
   wherein each ring A¹ is identical or different and each independently represents nitrogen-containing heterocyclylene (e.g., 4- to 20-membered nitrogen-containing heterocyclylene), arylene (e.g., C₅₋₂₀arylene) or heteroarylene (e.g., 5- to 20-membered heteroarylene), y1 represents an integer of 1 or 2, and (R^{y1})ₐ₁ indicates that each ring A¹ is independently optionally substituted with a1 R^{y1} groups, with each R^{y1} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl, and a1 represents an integer of 0-20;
   each ring A² is identical or different and each independently represents heterocyclylene (e.g., 4-to 20-membered heterocyclylene), cycloalkylene (e.g., C₃₋₂₀cycloalkylene), arylene (e.g., C₅₋₂₀arylene) or heteroarylene (e.g., 5- to 20-membered heteroarylene), y2 represents an integer of 0 or 1, and (R^{y2})ₐ₂ indicates that each ring A² is independently optionally substituted with a2 R^{y2} groups, with each R^{y2} being independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl, and a2 represents an integer of 0-20; and
R_{w1} represents a bond or an optionally substituted linear or branched C₁₋₂₀ alkylene, wherein any one or more (e.g., 1-20, 1-16, 1-14, 1-12, 1-8, 1-6, 1-4 or 2) methylene groups in the main carbon chain skeleton of the linear or branched C₁₋₂₀ alkylene are optionally replaced by one or more (e.g., 1-20, 1-16, 1-14, 1-12, 1-8, 1-6, 1-4 or 2) groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof; and
R_{w3} represents hydrogen, deuterium, leaving group, hydroxy, halogen, COOH, NH₂, N₃, CHO, methanesulfonyloxy, trifluoromethanesulfonyloxy or p-toluenesulfonyloxy.

The compounds of Formula (II) of the present disclosure, or their salts (including pharmaceutically acceptable salts), stereoisomers (including enantiomers), solvates, isotope-enriched analogs, prodrugs, or polymorphs, possess CRBN-binding activity and contribute to the recruitment of substrate proteins. They can act as molecular glues binding to the CRBN E3 ubiquitin ligase and can also be applied to bifunctional proteolysis-targeting chimera small-molecule drugs. The compounds of Formula (II) of the present disclosure exhibit antitumor activity and/or favorable pharmacokinetic properties.

In some embodiments, at least one of Z₁, Z₂, Z₃, and Z₄ of the compound of Formula (II) represents C(S).

In some embodiments, at least two of Z₁, Z₂, Z₃, and Z₄ of the compound of Formula (II) represent C(S).

In some embodiments, at least three of Z₁, Z₂, Z₃, and Z₄ of the compound of Formula (II) represent C(S).

In some embodiments, each of Z₁, Z₂, Z₃, and Z₄ of the compound of Formula (II) represents C(S).

In some embodiments, Z₁ of the compound of Formula (II) represents C(S).

In some embodiments, Z₂ of the compound of Formula (II) represents C(S).

In some embodiments, Z₃ of the compound of Formula (II) represents C(S).

In some embodiments, Z₄ of the compound of Formula (II) represents C(S).

In some embodiments, Z₁ and Z₄ of the compound of Formula (II) represent C(S).

In some embodiments, Z₂ and Z₄ of the compound of Formula (II) represent C(S).

In some embodiments, Z₃ and Z₄ of the compound of Formula (II) represent C(S).

In some embodiments, Z₁ and Z₂ of the compound of Formula (II) represent C(S).

In some embodiments, Z₁, Z₃, and Z₄ of the compound of Formula (II) represent C(S).

In some embodiments, Z₂, Z₃, and Z₄ of the compound of Formula (II) represent C(S).

In some embodiments, Z₁, Z₂, and Z₃ of the compound of Formula (II) represent C(S).

In some embodiments, Z₁, Z₂, and Z₄ of the compound of Formula (II) represent C(S).

In some embodiments, for the compound of Formula (II), Z₁ represents CH₂, Z₂ represents C(S), and Z₃ and Z₄ represents C(O).

In some embodiments, for the compound of Formula (II), Z₁ represents CH₂, Z₂ represents C(S), Z₃ represents C(O), and Z₄ represents C(S).

In some embodiments, for the compound of Formula (II), Z₁ represents CH₂, Z₂ represents C(S), and Z₃ and Z₄ represents C(S).

In some embodiments, for the compound of Formula (II), Z₁ and Z₂ represents C(S), Z₃ represents C(O), and Z₄ represents C(S).

In some embodiments, Z₁, Z₂, Z₃, and Z₄ of the compound of Formula (II) represent C(S).

In some embodiments, Rₐ₁, Rₐ₂, Rₐ₃, and Rₐ₄ of the compound of Formula (II) are identical or different and each independently represent hydrogen, deuterium, halogen (e.g., fluorine, chlorine, bromine, or iodine), C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-) or deuterated C₁₋₆ alkoxy.

In some embodiments, Rₐ₁, Rₐ₂, Rₐ₃, and Rₐ₄ of the compound of Formula (II) represent hydrogen.

In some embodiments, m of the compound of Formula (II) represents an integer of 0, 1, 2 or 3.

In some embodiments, R_{w3} of the compound of Formula (II) represents hydrogen, deuterium, COOH, NH₂, N₃, CHO, halogen, hydroxy or a leaving group (e.g., -N₃, chlorine, bromine, iodine sulfonate, such as methanesulfonyloxy (MsO-), trifluoromethanesulfonyloxy (TfO-), or p-toluenesulfonyloxy (TsO-)).

In some embodiments, R_{w} of the compound of Formula (II) represents a bond.

In some embodiments, R_{w} of the compound of Formula (II) represents O, S, S(O), S(O)₂, alkenylene (e.g., C₂₋₆alkenylene), alkynylene (e.g., C₂₋₆alkynylene) or N(Rₐ₆), wherein Rₐ₆ represents H or C₁₋₃ alkyl.

In some embodiments, R_{w} of the compound of Formula (II) represents: 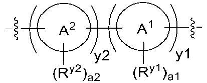 wherein each ring A¹ is identical or different and each independently represents 4- to 20-membered (e.g., 4- to 18-membered, 4- to 15-membered, 4- to 12-membered, 4- to 11-membered, 4- to 10-membered, 4-to 9-membered, 4- to 8-membered, 4- to 7-membered, 4- to 6-membered, 5- to 15-membered, or 5- to 9-membered) nitrogen-containing heterocyclylene, C₅₋₂₀arylene (e.g., C₆₋₂₀arylene, C₅₋₁₅arylene or C₆₋₁₅arylene) or 5- to 20-membered heteroarylene (e.g., 5- to 15-membered , 5- to 10-membered, 5- to 9-membered, 5- to 8-membered, 5- to 7-membered, or 5- to 6-membered heteroarylene). In some embodiments, 4- to 20-membered nitrogen-containing heterocyclylene is a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) heterocyclylene containing one nitrogen atom and optionally containing a heteroatom(s) selected from nitrogen, oxygen, and sulfur. Examples of the 4- to 20-membered nitrogen-containing heterocyclylene include, but are not limited to, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, or 3-methyl-2-oxa-8-azaspiro[4.5]decanylene. In some embodiments, examples of the 5- to 20-membered arylene include, but are not limited to, e.g., phenylene or naphthylene. In some embodiments, the 5- to 20-membered heteroarylene is a 5- to 20-membered (e.g., 5- to 15-membered, 5-to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A¹ is independently optionally substituted with a1 R^{y1} substituents, and each R^{y1} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl. a1 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, y1 of the compound of Formula (II) represents an integer of 1.

In some embodiments, y1 of the compound of Formula (II) represents an integer of 2.

In some embodiments, each ring A¹ of the compound of Formula (II) is identical or different and each independently represents the following optionally substituted groups: 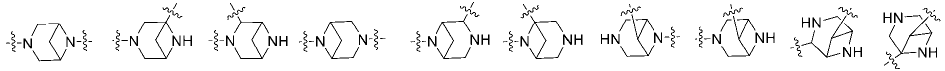 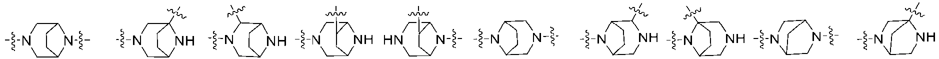 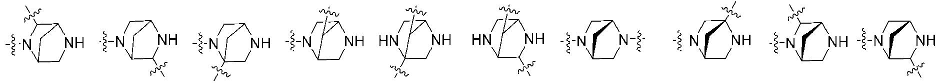 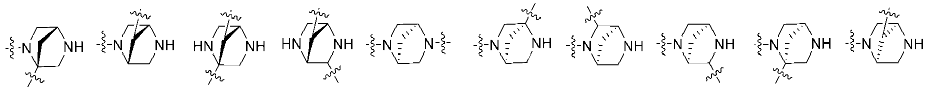 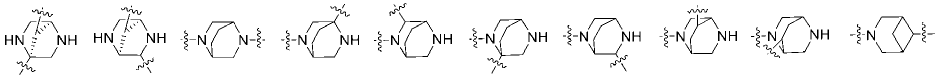 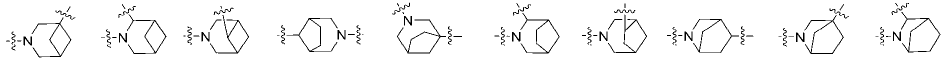 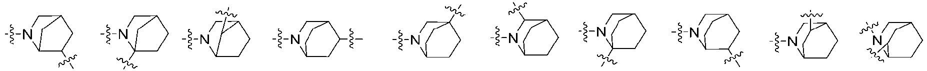 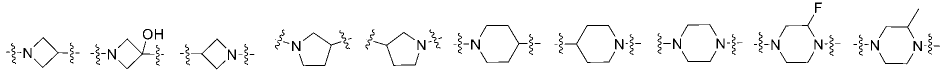 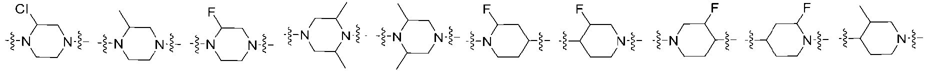 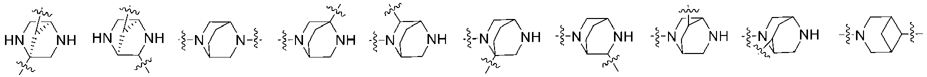 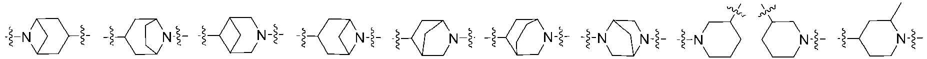 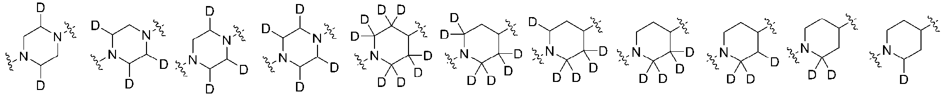 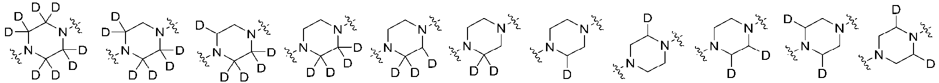 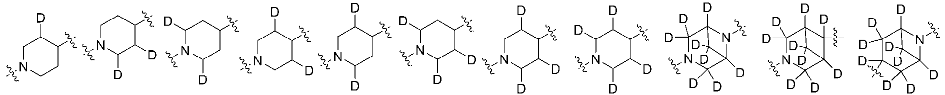 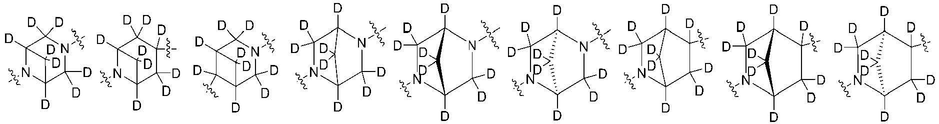 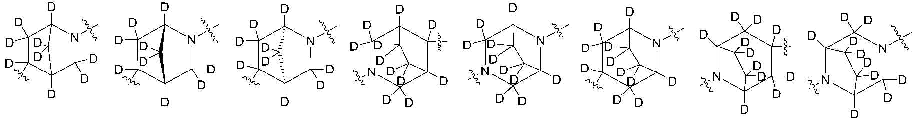 or 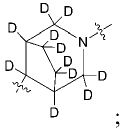 each ring A¹ is independently optionally substituted with a1 R^{y1} substituents, and each R^{y1} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl) or C₂₋₆ alkenyl (e.g., C₂₋₄ alkenyl, such as vinyl, propenyl, or butenyl). a1 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring A² in the compound of Formula (II) is identical or different and each independently represents heterocyclylene (e.g., 4- to 20-membered heterocyclylene, such as 4- to 18-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-memberedheterocyclylene), cycloalkylene (e.g., C₃₋₂₀cycloalkylene, C₃₋₁₅cycloalkylene or C₃₋₁₁cycloalkylene), arylene (e.g., C₅₋₂₀arylene, such as C₆₋₂₀arylene, C₅₋₁₅arylene or C₆₋₁₅arylene), or heteroarylene (e.g., 5- to 20-membered heteroarylene, such as 5- to 15-membered , 5- to 10-membered, 5-to 9-membered, 5- to 8-membered, 5- to 7-membered, or 5- to 6-membered heteroarylene). In some embodiments, the 4- to 20-membered heterocyclylene is a 4- to 20-membered (e.g., 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, or 5- to 9-membered) saturated or partially unsaturated (i.e., containing one or more double bonds, but not having a fully conjugated π-electron system) divalent monocyclic, bicyclic, tricyclic or polycyclic cyclic hydrocarbon group containing one or more (e.g., from 1 to 5, from 1 to 4, or from 1 to 3, or from 1 to 2, or 1) heteroatoms selected from the group consisting of oxygen, nitrogen, and sulfur. Examples of the 4- to 20-membered heterocyclylene include, but are not limited to, monocyclic heterocyclylene (e.g., 4- to 20-membered monocyclic heterocyclylene), bridged heterocyclylene (e.g., 5- to 20-membered bridged heterocyclylene, or 7- to 15-membered bridged heterocyclylene), fused heterocyclylene, and spiro-heterocyclylene (e.g., 5- to 20-membered spiro-heterocyclylene), e.g., azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 5- to 20-membered azaspirocycloalkylene, such as 3-azaspiro[5.5]undecanylene). In some embodiments, examples of the C₃₋₂₀ cycloalkylene include, but are not limited to, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. In some embodiments, examples of the 5- to 20-membered arylene include, but are not limited to, e.g., phenylene or naphthylene. In some embodiments, 5- to 20-membered heteroarylene is a 5- to 20-membered (e.g., 5- to 15-membered, 5- to 12-membered, 5- to 9-membered, 5- to 8-membered, 5- to 7-membered, or 5- to 6-membered) heteroarylene containing one nitrogen atom and optionally containing a heteroatom(s) selected from nitrogen, oxygen, and sulfur. In some embodiments, examples of the 5- to 20-membered heteroarylene include, but are not limited to, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. Each ring A² is independently optionally substituted with a2 R^{y2} substituents, and each R^{y2} independently represents deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl or C₂₋₆ alkenyl. a2 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, each ring A² of the compound of Formula (II) is identical or different and each independently represents the following optionally substituted groups: 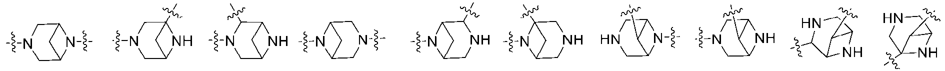 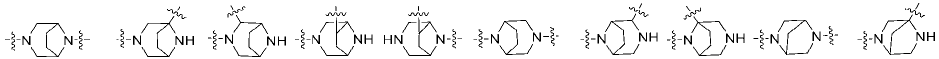 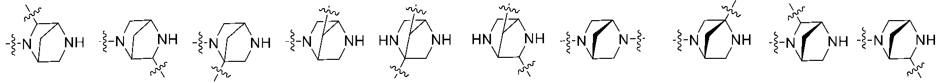 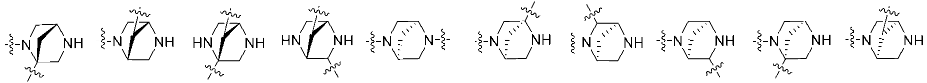 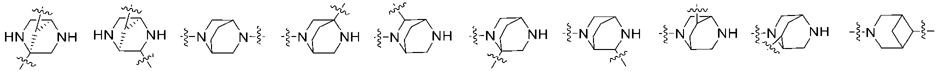 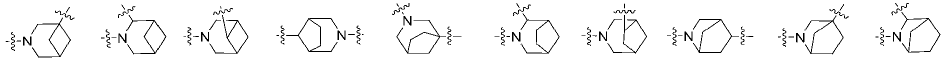 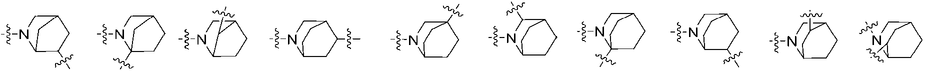 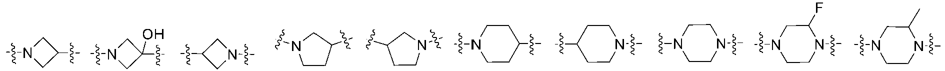 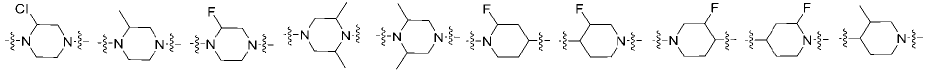 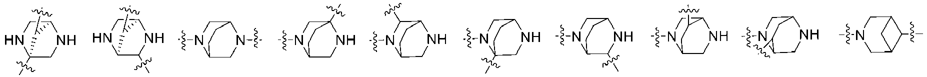 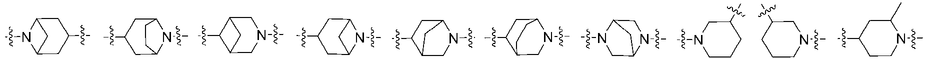 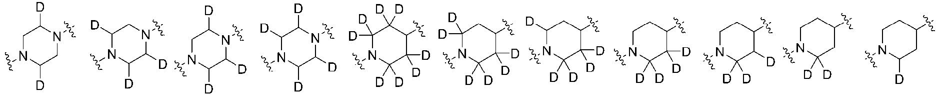 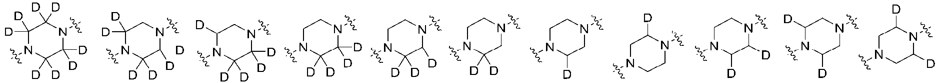 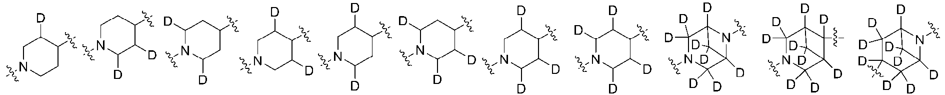 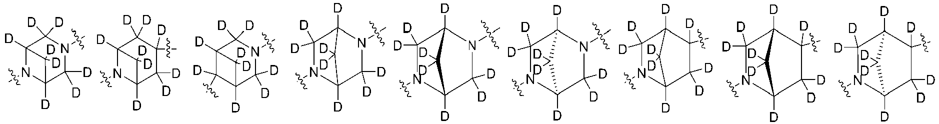 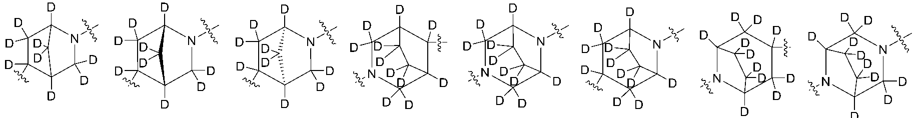 or 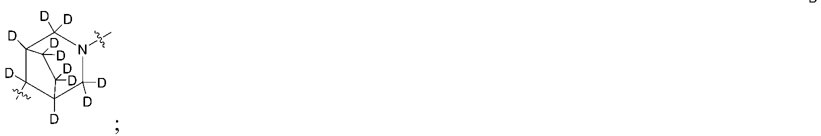 each ring A² is independently optionally substituted with a2 R^{y2} groups, wherein each R^{y2} is independently deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl) or C₂₋₆ alkenyl (e.g., C₂₋₄ alkenyl, such as vinyl, propenyl, or butenyl). a2 represents an integer of 0-20, e.g., an integer of 0, 1, 2, 3, 4, 5, 6, 7, 8, 9, 10, 11, 12, 13, 14, 15 or 20. The number of substituents is not theoretically limited in any way, or is automatically limited by the size of the building units.

In some embodiments, y2 of the compound of Formula (II) represents an integer of 0.

In some embodiments, y2 of the compound of Formula (II) represents an integer of 1.

In some embodiments, R_{w} of the compound of Formula (II) represents: 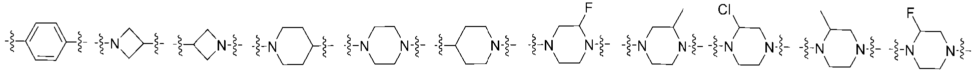 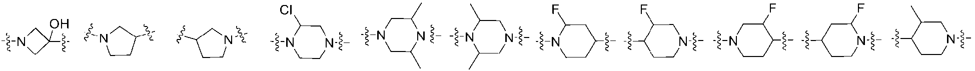 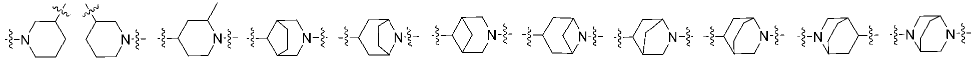 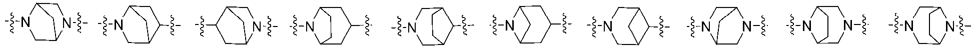 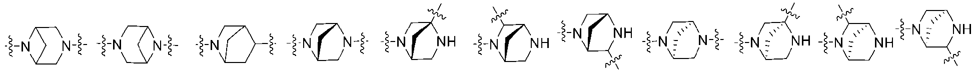 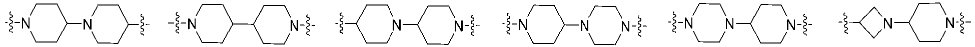 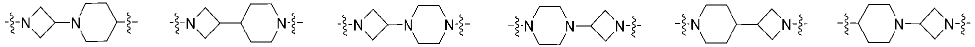 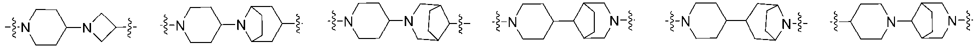 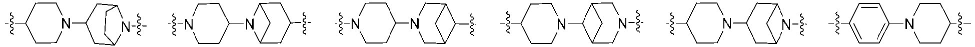 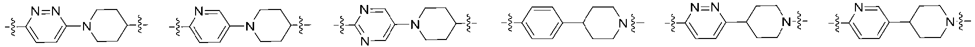 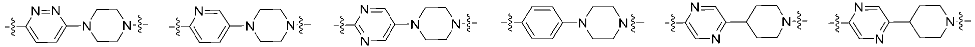 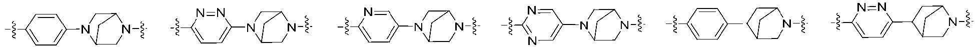 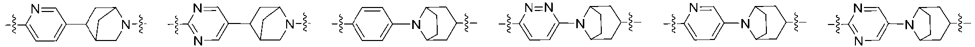 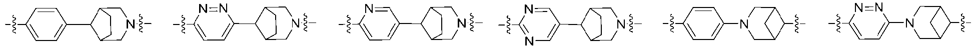 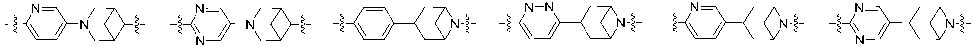 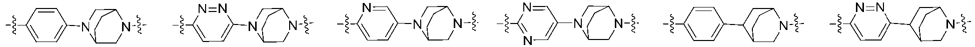 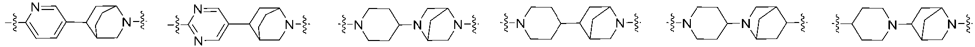 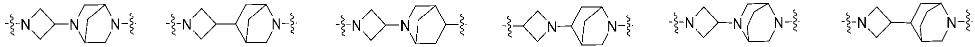 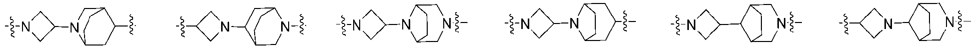 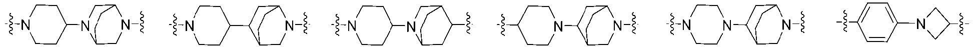 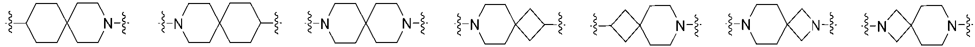 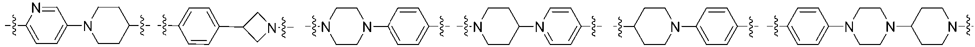 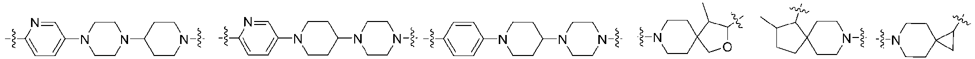 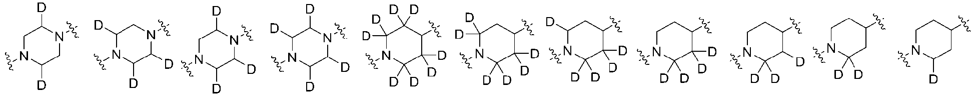 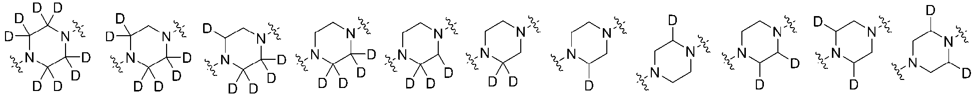 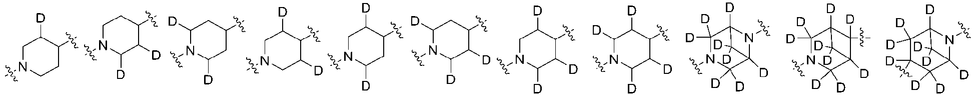 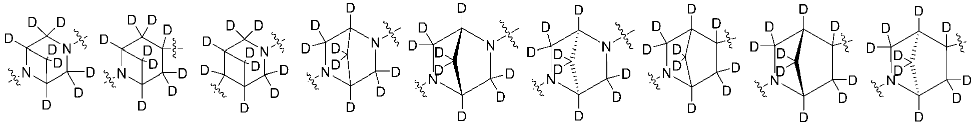 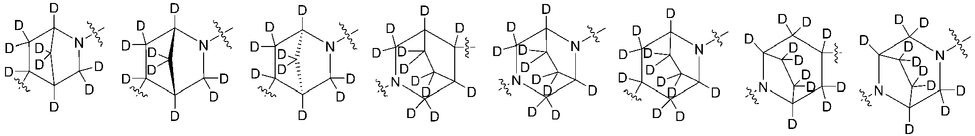 or 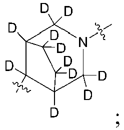 wherein the groups are optionally substituted with a substituent selected from the group consisting of deuterium, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogen (e.g., fluorine, chlorine, bromine, or iodine), amino, hydroxy, mercapto, cyano, carboxyl, C₂₋₆ alkynyl (e.g., C₂₋₄ alkynyl, such as ethynyl, propynyl or butynyl), and C₂₋₆ alkenyl (e.g., C₂₋₄ alkenyl, such as vinyl, propenyl, or butenyl).

In some embodiments, R_{w1} of the compound of Formula (II) represents a bond.

In some embodiments, R_{w1} of the compound of Formula (II) represents an optionally substituted linear or branched C₁₋₂₀ alkylene, wherein any one or more (e.g., 1-15, 1-10, 1-9, 1-8, 1-7, 1-6, 1-5, 1-4, 1-3 or 1-2, or 1) methylene groups in the main carbon chain skeleton of the linear or branched C₁₋₂₀ alkylene are optionally replaced by one or more (e.g., 1-20, or 1-15, 1-10, 1-9, 1-8, 1-7, 1-6, 1-5, 1-4, 1-3 or 1-2, or 1) groups selected from: Rₐ, R_{b}, and any combination thereof, wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene (e.g., optionally substituted C₃₋₂₀ cycloalkylene), optionally substituted arylene (e.g., optionally substituted C₅₋₂₀arylene), optionally substituted heterocyclylene (e.g., optionally substituted 4-to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl (e.g., C₁₋₅ alkyl, C₁₋₄ alkyl or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆cycloalkyl, halogenated C₃₋₆cycloalkyl, deuterated C₃₋₆cycloalkyl, 4- to 10-membered heterocyclyl, and any combination thereof; and wherein one or more hydrogens of said linear or branched C₁₋₂₀ alkylene are optionally replaced by a substituent selected from: deuterium, hydroxy, amino, mercapto, nitro, halogen, cyano, deuterated C₁₋₆ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆cycloalkyl, halogenated C₃₋₆cycloalkyl, deuterated C₃₋₆cycloalkyl, 4- to 10-membered heterocyclyl, and any combination thereof.

In some embodiments, R_{w1} of the compound of Formula (II) represents a structure of the following Formulae:

-C₁₋₁₅alkylene-;

-(C(Rₐ₈)(Rₐ₉))ₙ₁-(Rₐ(C(Rₐ₁₀)(Rₐ₁₁))ₙ₂)ₘ₃-;

-(C(Rₐ₈)(Rₐ₉))ₙ₁-(Rₐ(C(Ra₁₀)(Ra₁₁))ₙ₂)ₘ₃-(Rₐ(C(Rₐ₁₂)(Rₐ₁₃))ₙ₃)ₘ₄₋;

-(C(Rₐ₈(Rₐ₉))ₙ₁-(Rₐ(C(Rₐ₁₀)(Rₐ₁₁))ₙ₂)ₘ₃-(Rₐ(C(Rₐ₁₂)(Rₐ₁₃))ₙ₃)ₘ₄-(Rₐ(C(Rₐ₁₄)(Rₐ₁₄))ₙ₄)ₘ₅-;

-(C(Rₐ₈)(Rₐ₉))ₙ₁-((C(Rₐ₁₀)(Rₐ₁₁))ₙ₂Rₐ)ₘ₃-;

-(C(Rₐ₈)(Rₐ₉))ₙ₁-((C(Rₐ₁₀)(Rₐ₁₁))ₙ₂Rₐ)ₘ₃-((C(Rₐ₁₂)(Rₐ₁₃))ₙ₃Rₐ)ₘ₄-;

-(C(Rₐ₈)(Rₐ₉))ₙ₁₋((C(Rₐ₁₀)(Rₐ₁₁))ₙ₂Rₐ)ₘ₃-((C(Rₐ₁₂)(Rₐ₁₃))ₙ₃Rₐ)ₘ₄-((C(Rₐ₁₄)(Rₐ₁₅))ₙ₄Rₐ)ₘ₅-;

-(C(Rₐ₈)(Rₐ₉))ₙ₁-(R_{b}(C(Rₐ₁₀)(Rₐ₁₁))ₙ₂)ₘ₃-;

-(C(Rₐ₈)(Rₐ₉))ₙ₁-(R_{b}(C(Rₐ₁₀)(Rₐ₁₁))ₙ₂)ₘ₃-(R_{b}(C(Rₐ₁₂)(Rₐ₁₃))ₙ₃)ₘ₄-;

-(C(Rₐ₈)(Rₐ₉))ₙ₁-(R_{b}(C(Rₐ₁₀)(Rₐ₁₁))ₙ₂)ₘ₃-(R_{b}(C(Rₐ₁₂)(Rₐ₁₃))ₙ₃)ₘ₄-(R_{b}(C(Rₐ₁₄)(Rₐ₁₅))ₙ₄)ₘ₅-;

-(C(Rₐ₈)(Rₐ₉))ₙ₁-((C(Rₐ₁₀)(Rₐ₁₁))ₙ₂R_{b})ₘ₃-;

-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃R_{b})ₘ₄-;

-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃R_{b})ₘ₄-((C(Rₐ₁₄)(Rₐ₁₅))ₙ₄R_{b})ₘ₅-;

-(C(Rₐ₈)(Rₐ₉))ₙ₁-(Rₐ-R_{b}-(C(Rₐ₁₀)(Rₐ₁₁))ₙ₂)ₘ₃-;

-(C(Rₐ₈)(Rₐ₉))ₙ₁-(Rₐ(C(Rₐ₁₀)(Rₐ₁₁))ₙ₂)ₘ₃-(R_{b}(C(Rₐ₁₂)(Rₐ₁₃))ₙ₃)ₘ₄-;

-(C(Rₐ₈)(Rₐ₉))ₙ₁-(R_{b}-Rₐ-(C(Rₐ₁₀)(Rₐ₁₁))ₙ₂)ₘ₃-;

-(C(Rₐ₈)(Rₐ₉))ₙ₁-(Rb(C(Rₐ₁₀)(Rₐ₁₁))ₙ₂)ₘ₃-(Rₐ(C(Rₐ₁₂)(Rₐ₁₃))ₙ₃)ₘ₄-;

-(C(Rₐ₈)(Rₐ₉))ₙ₁-((C(Rₐ₁₀)(Rₐ₁₁))ₙ₂-Rₐ-R_{b})ₘ₃-;

-(C(Rₐ₈)(Rₐ₉))ₙ₁-((C(Rₐ₁₀)(Rₐ₁₁))ₙ₂Rₐ)ₘ₃-((C(Rₐ₁₂)(Rₐ₁₃))ₙ₃R_{b})ₘ₄-;

-(C(Rₐ₈)(Rₐ₉))ₙ₁-((C(Rₐ₁₀)(Rₐ₁₁))ₙ₂-R_{b}-Rₐ)ₘ₃-; or

-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃Rₐ)ₘ₄-;

wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl; and each R_{b} is independently selected from the group consisting of optionally substituted cycloalkylene (e.g., optionally substituted C₃₋₂₀cycloalkylene), optionally substituted arylene (e.g., optionally substituted C₅₋₂₀arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆alkynylene), alkenylene (e.g., C₂₋₆alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₅ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₅ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof; and
n1, n2, n3, n4, m3, m4, and m5 each independently represent an integer of 1, 2, 3, 4, 5, 6, 7, 8, 9 or 10; and
any one or more hydrogens of said C₁₋₁₅ alkylene are optionally replaced by a substituent selected from: deuterium, hydroxy, amino, mercapto, nitro, halogen, cyano, halogenated C₁₋₆ alkyl, deuterated C₁₋₆ alkyl, C₁₋₆ alkoxy, halogenated C₁₋₅ alkoxy, C₃₋₆cycloalkyl, halogenated C₃₋₆cycloalkyl, deuterated C₃₋₆cycloalkyl, 4- to 10-membered heterocyclyl, and any combination thereof. In the present disclosure, unless otherwise specified, any one end of the general formulas provided above may be connected to R_{w} of the compound of Formula (II), while the other end is connected to R_{w3}. The number of carbon atoms of each of the above general formulas (excluding the number of carbon atoms in the Rₐ and R_{b} groups), namely the sum of n1+ n2*m3, the sum of n1+ n2*m3+ n3*m4, the sum of n1+ n2*m3+ n3*m4+ n4*m5, each independently is an integer less than or equal to 20.

In some embodiments, R_{w1} of the compound of Formula (II) represents -C₁₋₁₅alkylene-, wherein any one or more hydrogens of said C₁₋₁₅ alkylene are optionally replaced by a substituent selected from: deuterium, hydroxy, amino, mercapto, nitro, halogen, cyano, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₅ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆ cycloalkyl, halogenated C₃₋₆ cycloalkyl, deuterated C₃₋₆ cycloalkyl, 4- to 10-membered heterocyclyl, and any combination thereof.

In some embodiments, R_{w1} of the compound of Formula (II) represents the following groups:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, - (CH₂)₁₁-, -(CH₂)₁₂-, -(CH₂)₁₃-, -(CH₂)₁₄-, or -(CH₂)₁₅-;
wherein any one or more hydrogens of said groups are optionally replaced by a substituent selected from: deuterium, hydroxy, amino, mercapto, nitro, halogen, cyano, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂- or CH₂ClCH₂-), deuterated C₁₋₅ alkyl (e.g., perdeuterated C₁₋₆ alkyl, perdeuterated C₁₋₅ alkyl or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-), C₃₋₆cycloalkyl, halogenated C₃₋₆cycloalkyl, deuterated C₃₋₆cycloalkyl, 4- to 10-membered heterocyclyl, and any combination thereof.

In some embodiments, R_{w1} of the compound of Formula (II) represents a structure of the following Formulae:

-(C(Rₐ₈)(Rₐ₉))ₙ₁-(O(C(Rₐ₁₀)(Rₐ₁₁))ₙ₂)ₘ₃-;

-(C(Rₐ₈)(Rₐ₉))ₙ₁₋(O(C(Rₐ₁₀)(Rₐ₁₁))ₙ₂)ₘ₃₋(O(C(Rₐ₁₂)(Rₐ₁₃))ₙ₃)ₘ₄-;

-(C(Rₐ₈)(Rₐ₉))ₙ₁-(O(C(Rₐ₁₀)(Rₐ₁₁))ₙ₂)ₘ₃-(O(C(Rₐ₁₂)(Rₐ₁₃))ₙ₃)ₘ₄- (O(C(Rₐ₁₄)(Rₐ₁₅))ₙ₄)ₘ₅-;

-(C(Rₐ₈)(Rₐ₉))ₙ₁-((C(Rₐ₁₀)(Rₐ₁₁))ₙ₂O)ₘ₃-;

-(C(Rₐ₈)(Rₐ₉))ₙ₁₋((C(Rₐ₁₀₎(Rₐ₁₁))ₙ₂O)ₘ₃-((C(Rₐ₁₂)(Rₐ₁₃))ₙ₃O)ₘ₄-;

-(C(Rₐ₈)(Rₐ₉))ₙ₁-((C(Rₐ₁₀)(Rₐ₁₁))ₙ₂O)ₘ₃-((C(Rₐ₁₂)(Rₐ₁₃))ₙ₃O)ₘ₄-((C(Rₐ₁₄)(Rₐ₁₅))ₙ₄O)ₘ₅-;

-(C(Rₐ₈)(Rₐ₉))ₙ₁-(N(Rₐ₇)(C(Rₐ₁₀)(Rₐ₁₁))ₙ₂)ₘ₃-;

-(C(Rₐ₈)(Rₐ₉))ₙ₁-(N(Rₐ₇)(C(Rₐ₁₀)(Rₐ₁₁))ₙ₂)ₘ₃-(N(Rₐ₇)(C(Rₐ₁₂)(Rₐ₁₃))ₙ₃)ₘ₄-;

-(C(Rₐ₈)(Rₐ₉))ₙ₁-(N(Rₐ₇)(C(Rₐ₁₀)(Rₐ₁₁))ₙ₂)ₘ₃-(N(Rₐ₇)(C(Rₐ₁₂)(Rₐ₁₃))ₙ₃)ₘ₄-(N(Rₐ₇)(C(Rₐ₁₄)(Rₐ₁₅))ₙ₄)ₘ₅-;

-(C(Rₐ₈)(Rₐ₉))ₙ₁-((C(Rₐ₁₀)(Rₐ₁₁))ₙ₂N(Rₐ₇))ₘ₃-;

-(C(Rₐ₈)(Rₐ₉))ₙ₁-((C(Rₐ₁₀)(Rₐ₁₁))ₙ₂N(Rₐ₇))ₘ₃-((C(Rₐ₁₂)(Rₐ₁₃))ₙ₃N(Rₐ₇))ₘ₄-;

-(C(Rₐ₈)(Rₐ₉))ₙ₁-((C(Rₐ₁₀)(Rₐ₁₁))ₙ₂N(Rₐ₇))ₘ₃-((C(Rₐ₁₂)(Rₐ₁₃))ₙ₃N(Rₐ₇))ₘ₄-((C(Rₐ₁₄)(Rₐ₁₅))ₙ₄N(Rₐ₇))ₘ₅-;

-(C(Rₐ₈)(Rₐ₉))ₙ₁-(C(O)N(Rₐ₇)-(C(Rₐ₁₀)(Rₐ₁₁))ₙ₂)ₘ₃-;

-(C(Rₐ₈)(Rₐ₉))ₙ₁-(C(O)N(Rₐ₇)-(C(Rₐ₁₀)(Rₐ₁₁))ₙ₂)ₘ₃-(C(O)N(Rₐ₇)-(C(Rₐ₁₂)(Rₐ₁₃))ₙ₃)ₘ₄-;

-(C(Rₐ₈)(Rₐ₉))ₙ₁-(C(O)N(Rₐ₇)-(C(Rₐ₁₀)(Rₐ₁₁))ₙ₂)ₘ₃-(C(O)N(Rₐ₇)-(C(Rₐ₁₂)(Rₐ₁₃))ₙ₃)ₘ₄-(C(O)N(Rₐ₇)-(C(Rₐ₁₄)(Rₐ₁₅))ₙ₄)ₘ₅-;

-(C(Rₐ₈)(Rₐ₉))ₙ₁-((C(Rₐ₁₀)(Rₐ₁₁))ₙ₂-C(O)N(Rₐ₇))ₘ₃-;

-(C(Rₐ₈)(Rₐ₉))ₙ₁-((C(Rₐ₁₀)(Rₐ₁₁))ₙ₂-C(O)N(Rₐ₇))ₘ₃-((C(Rₐ₁₂)(Rₐ₁₃))ₙ₃-C(O)N(Rₐ₇))ₘ₄-;

-(C(Rₐ₈)(Rₐ₉))ₙ₁₋((C(Rₐ₁₀)(Rₐ₁₁))ₙ₂₋C(O)N(Rₐ₇))ₘ₃-((C(Rₐ₁₂)(Rₐ₁₃))ₙ₃-C(O)N(Rₐ₇))ₘ₄-((C(Rₐ₁₄)(Rₐ₁₅₎)ₙ₄-C(O)N(Rₐ₇))ₘ₅-;

-(C(Rₐ₇)(Rₐ₉))ₙ₁-(C(O)N(Rₐ₇)-(C(Rₐ₁₀)(Rₐ₁₁))ₙ₂)ₘ₃-(O-(C(Rₐ₁₂)(Rₐ₁₃))ₙ₃)ₘ₄-;

-(C(Rₐ₈)(Rₐ₉))ₙ₁-C(O)N(Rₐ₇)-(C(Rₐ₁₀)(Rₐ₁₁))ₙ₂-(O(C(Rₐ₁₂)(Rₐ₁₃))ₙ₃)ₘ₃-;

-(C(Rₐ₈)(Rₐ₉))ₙ₁-((C(Rₐ₁₀)(Rₐ₁₁))ₙ₂-C(O)N(Rₐ₇))ₘ₃-((C(Rₐ₁₂)(Rₐ₁₃))ₙ₃O)ₘ₄-;

-(C(Rₐ₈)(Rₐ₉))ₙ₁-(C(Rₐ₁₀)(Rₐ₁₁))ₙ₂-C(O)N(Rₐ₇)-((C(Rₐ₁₂)(Rₐ₁₃))ₙ₃O)ₘ₃-;

-(C(Rₐ₈)(Rₐ₉))ₙ₁-(N(Rₐ₇)C(O)-(C(Rₐ₁₀)(Ra₁₁))ₙ₂)ₘ₃-;

-(C(Rₐ₈)(Rₐ₉))ₙ₁-(N(Rₐ₇)C(O)-(C(Rₐ₁₀)(Rₐ₁₁))ₙ₂)ₘ₃-(O-(C(Rₐ₁₂)(Rₐ₁₃)))ₙ₃)ₘ₄-;

-(C(Rₐ₈)(Rₐ₉))ₙ₁-(N(Rₐ₇)C(O)-(C(Rₐ₁₀)(Rₐ₁₁))ₙ₂)ₘ₃-(O-(C(Rₐ₁₂)(Rₐ₁₃))ₙ₃)ₘ₄-(O-(C(Rₐ₁₄)(Rₐ₁₅))ₙ₄)ₘ₅-;

-(C(Rₐ₈)(Rₐ₉))ₙ₁-N(Rₐ₇)C(O)-(C(Rₐ₁₀)(Rₐ₁₁))ₙ₂-(O(C(Rₐ₁₂)(Rₐ₁₃))ₙ₃)ₘ₃-;

-(C(R₈)(Ra₉))ₐ₁-N(Rₐ₇)C(O)N(Rₐ₇)-(C(Rₐ₁₀)(Rₐ₁₁))ₙ₂-;

-(C(Rₐ₈)(Rₐ₉))ₙ₁-((C(Rₐ₁₀)(Rₐ₁₁))ₙ₂-N(Rₐ₇)C(O))ₘ₃-;

-(C(Rₐ₈)(Rₐ₉))ₙ₁₋((C(Rₐ₁₀₎(Rₐ₁₁))ₙ₂-N(Rₐ₇)C(O))ₘ₃-((C(Rₐ₁₂)(Rₐ₁₃)))ₙ₃₋O)ₘ₄-;

-(C(Rₐ₈)(Rₐ₉))ₙ₁-((C(Rₐ₁₀₎(Rₐ₁₁))ₙ₂-N(Rₐ₇)C(O))ₘ₃-((C(Rₐ₁₂)(Ra₁₃))₁₃O)ₘ₄-((C(Rₐ₁₄)(Rₐ₁₅))ₙ₄O)ₘ₅-;

-(C(Rₐ₈)(Rₐ₉))ₙ₁- (C(Rₐ₁₀)(Ra₁₁))ₙ₂-N(Rₐ₇)C(O)-((C(Rₐ₁₂)(Ra₁₃))ₙ₃O)ₘ₃-;

-(C(Rₐ₈)(Rₐ₉))ₙ₁-(arylene-(C(Rₐ₁₀)(Rₐ₁₁))ₙ₂)ₘ₃-;

-(C(Rₐ₈)(Rₐ₉))ₙ₁-(arylene-(C(Rₐ₁₀)(Rₐ₁₁))ₙ₂)ₘ₃-arylene-(C(Rₐ₁₂)(Rₐ₁₃))ₙ₃-;

-(C(Rₐ₈)(Rₐ₉))ₙ₁-((C(Rₐ₁₀)(Rₐ₁₁))ₙ₂-arylene)ₘ₃-;

-(C(Rₐ₈)(Rₐ₉))ₙ₁-((C(Rₐ₁₀)(Rₐ₁₁))ₙ₂-arylene)ₘ₃-(C(Rₐ₁₂)(Rₐ₁₃))ₙ₃-arylene-;

-(C(Rₐ₈)(Rₐ₉))ₙ₁-(heterocyclylene-(C(Rₐ₁₀)(Rₐ₁₁))ₙ₂)ₘ₃-;

-(C(Rₐ₈)(Rₐ₉))ₙ₁-(heterocyclylene-(C(Rₐ₁₀)(Rₐ₁₁))ₙ₂)ₘ₃-(heterocyclylene-(C(Rₐ₁₂)(Rₐ₁₃))ₙ₃)ₘ₄-;

-(C(Rₐ₈)(Rₐ₉))ₙ₁-((C(Rₐ₁₀)(Rₐ₁₁))ₙ₂-heterocyclylene)ₘ₃-;

-(C(Rₐ₈)(Rₐ₉))ₙ₁-((C(Rₐ₁₀)(Ra₁₁))ₙ₂-heterocyclylene)ₘ₃-((C(Rₐ₁₂)(Rₐ₁₃))ₙ₃-heterocyclylene)ₘ₄-;

-(C(Rₐ₈)(Rₐ₉))ₙ₁-(heteroarylene-(C(Rₐ₁₀)(Rₐ₁₁))ₙ₂)ₘ₃-;

-(C(Rₐ₈)(Rₐ₉))ₙ₁-(heteroarylene-(C(Rₐ₁₀)(Rₐ₁₁))ₙ₂)ₘ₃-(heteroarylene-(C(Rₐ₁₂)(Rₐ₁₃))ₙ₃)ₘ₄-;

-(C(Rₐ₈)(Ra₉))ₙ₁-((C(Rₐ₁₀)(Ra₁₁))ₙ₂eteroarylene)ₘ₃-;

-(C(Rₐ₈)(Rₐ₉))ₙ₁-((C(Rₐ₁₀)(Ra₁₁))ₙ₂-heteroarylene)ₘ₃-((C(Rₐ₁₂)(Rₐ₁₃))ₙ₃-heteroarylene)ₘ₄-;

-(C(Rₐ₈)(Rₐ₉))ₙ₁-(cycloalkylene-(C(Rₐ₁₀)(Rₐ₁₁))ₙ₂)ₘ₃-;

-(C(Rₐ₈)(Rₐ₉))ₙ₁-(cycloalkylene-(C(Rₐ₁₀)(Rₐ₁₁))ₙ₂)ₘ₃-(cycloalkylene-(C(Rₐ₁₂)(Rₐ₁₃))ₙ₃)ₘ₄-;

-(C(Rₐ₈)(Rₐ₉))ₙ₁-((C(Rₐ₁₀)(Rₐ₁₁))ₙ₂-cycloalkylene)ₘ₃-; or

-(C(Rₐ₈)(Rₐ₉))ₙ₁-((C(Rₐ₁₀)(Rₐ₁₁))ₙ₂-cycloalkylene)ₘ₃-((C(Rₐ₁₂)(Rₐ₁₃))ₙ₃-cycloalkylene)ₘ₄-;

wherein each Rₐ₇ independently represents H or C₁₋₃ alkyl;
the cycloalkylene (e.g., C₃₋₂₀cycloalkylene), the arylene (e.g., C₅₋₂₀arylene), the heterocyclylene (e.g., 4- to 20-membered heterocyclylene), and the heteroarylene (e.g., 5- to 20-membered heteroarylene) are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof; and
n1, n2, n3, n4, m3, m4, and m5 each independently represent an integer of 1, 2, 3, 4, 5, 6, 7, 8, 9 or 10. The total number of carbon atoms of alkylene in each of the above general formulas, namely the sum of n1+ n2*m3, the sum of n1+ n2*m3+ n3*m4, the sum of n1+ n2*m3+ n3*m4+ n4*m5, each independently is an integer less than or equal to 20.

In some embodiments, examples of the cycloalkylene in the general formula structure represented by R_{w1} of the compound of Formula (II) include, but are not limited to, C₃₋₂₀cycloalkylene, C₃₋₁₅cycloalkylene, and C₃₋₁₁cycloalkylene, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅₋₁₅ spiro-cycloalkylene), adamantanylene, noradamantanylene, and norbomylene. The cycloalkylene is optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

In some embodiments, examples of the arylene in the general formula structure represented by R_{w1} of the compound of Formula (II) include, but are not limited to, C₅₋₂₀arylene, C₆₋₂₀arylene, C₅₋₁₅arylene, and C₆₋₁₅arylene, such as phenylene or naphthylene. The arylene is optionally substituted with a substituent selected from the group consisting of deuterium, hydroxy, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

In some embodiments, examples of the heterocyclylene in the general formula structure represented by R_{w1} of the compound of Formula (II) include, but are not limited to, 4- to 20-membered, 4-to 15-membered, 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered heterocyclylene, e.g., azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and 5- to 20-membered azaspirocycloalkylene. The heterocyclylene is optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

In some embodiments, examples of the heteroarylene in the general formula structure represented by R_{w1} of the compound of Formula (II) include, but are not limited to, 5- to 20-membered heteroarylene, 5- to 15-membered, 5- to 10-membered, 5- to 9-membered, 5- to 8-membered, 5- to 7-membered, and 5-to 6-membered heteroarylene, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene. The heteroarylene is optionally substituted with a substituent selected from the group consisting of deuterium, hydroxy, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

In some embodiments, examples of R_{w1} of the compound of Formula (II) include, but are not limited to, the following groups:
-O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, -(CH₂)₁-O-, -(CH₂)₂-O-, -(CH₂)₃-O-, -(CH₂)₄-O-, -(CH₂)₅-O-, -(CH₂)₆-O-, -(CH₂)₇-O-, -(CH₂)₈-O-, -(CH₂)₉-O-, -(CH₂)₁₀-O-, -CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)_{S}-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, -(CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, - CH(CH₃)-O-(CH₂)₇-, -CH(CH₃)-O-(CH₂)₉-, -CH(CH₃)-O-(CH₂)₉-, -CH(CH₃)-O-(CH₂)₁₀-, -(O(CH₂)₂)₂-, - (O(CH₂)₂)₃-, -(O(CH₂)₂)₄-, -(O(CH₂)₂)₅-, -(O(CH₂)₂)₆-, -(O(CH₂)₂)₇-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, - CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, - (CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -CH₂-(O(CH₂)₃)₂-, -CH₂-(O(CH₂)₃)₃-, - CH₂-(O(CH₂)₃)₄-, -(CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, - (CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, - CH₂-O-(CH₂)₂-O-CH₂-, -(CH₂)₂-O-(CH₂)₂-O-CH₂-, -(CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, - (CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, -N(Rₐ₇)-(CH₂)₆-, -N(Rₐ₇)-(CH₂)₇-, -N(Rₐ₇)-(CH₂)₈-, -N(Rₐ₇)-(CH₂)₉-, -N(Rₐ₇)-(CH₂)₁₀-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH2)4-, -(CH₂)₂-N(Rₐ₇)-(CH₂)₅-, -(CH₂)₂-N(Rₐ₇)-(CH₂)₆-, -(CH₂)₂-N(Rₐ₇)-(CH₂)₇-, -(CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, - (CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄₋N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, -(CH₂)₅-N(Rₐ₇)-(CH2)4-, -(CH₂)⁵-N(Rₐ₇)-(CH²)⁵-, -(CH₂)₆-N(Rₐ₇)-(CH₂)₁-, -(CH₂)₆₋N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, - (CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, - CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, -CH(CH₃)-N(Rₐ₇)-(CH₂)₈-, -CH(CH₃)-N(Rₐ₇)-(CH₂)₉-, -CH(CH₃)-N(Rₐ₇)-(CH₂)₁₀-, -C(O)NHCH₂-, -C(O)NH(CH₂)₂-, -C(O)NH(CH₂)₃-, -C(O)NH(CH₂)₄-, -C(O)NH(CH₂)₅-, -CH₂C(O)NHCH₂-, -(CH₂)₂C(O)NH(CH₂)₂-, -(CH₂)₂C(O)NH(CH₂)₃-, -(CH₂)₂C(O)NH(CH₂)₄-, - (CH₂)₂C(O)NH(CH₂)₅-, -(CH₂)₃C(O)NH(CH₂)₃-, -(CH₂)₃C(O)NH(CH₂)₄-, -(CH₂)₄C(O)NH(CH₂)₄-, - (CH₂)₅C(O)NH(CH₂)₅-, -(CH₂)₆C(O)NH(CH₂)₇-, -(CH₂)₆C(O)NH(CH₂)₆-, -(CH₂)₇C(O)NH(CH₂)₇-, - (CH₂)₂C(O)NH(CH₂)₂-O-(CH₂)₂-, -NHC(O)CH₂-, -NHC(O)(CH₂)₂-, -NHC(O)(CH₂)₃-, -NHC(O)(CH₂)₄-, -NHC(O)(CH₂)₅-, -CH₂NHC(O)CH₂-, -(CH₂)₂NHC(O)(CH₂)₂-, -(CH₂)₂NHC(O)(CH₂)₃-, - (CH₂)₂NHC(O)(CH₂)₄-, -(CH₂)₂NHC(O)(CH₂)₅-, -(CH₂)₃NHC(O)(CH₂)₃-, -(CH₂)₃NHC(O)(CH₂)₄-, - (CH₂)₄NHC(O)(CH₂)₄-, -(CH₂)₅NHC(O)(CH₂)₅-, -(CH₂)₆NHC(O)(CH₂)₇-, -(CH₂)₆NHC(O)(CH₂)₆-, - (CH₂)₇NHC(O)(CH₂)₇-, -(CH₂)₄NHC(O)(CH₂)₈-, -(CH₂)₂NHC(O)(CH₂)₂-O-(CH₂)₂-, -(CH₂)₄NHC(O)CH₂-, -phenylene-CH₂-, -phenylene-(CH₂)₂-, -phenylene-(CH₂)₃-, -phenylene-(CH₂)₄-, -phenylene-(CH₂)₅-, - phenylene-(CH₂)₆-, -phenylene-(CH₂)₇-, -phenylene-(CH₂)₈-, -CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₈-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, -(CH₂)₂-phenylene-(CH₂)₅-, - (CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, -(CH₂)₃-phenylene-(CH₂)₆-, -(CH₂)₃-phenylene-(CH₂)₇-, -(CH₂)₃-phenylene-(CH₂)₈-, - (CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, -(CH₂)₅-phenylene-(CH₂)₁-, -(CH₂)₅-phenylene-(CH₂)₂-, -(CH₂)₅-phenylene-(CH₂)₃-, - (CH₂)₅-phenylene-(CH₂)₄-, -(CH₂)₅-phenylene-(CH₂)₅-, -(CH₂)₅-phenylene-(CH₂)₆-, -(CH₂)₅-phenylene-(CH₂)₇-, -(CH₂)₅-phenylene-(CH₂)₉-, -(CH₂)₆-phenylene-(CH₂)₁-, -(CH₂)₆-phenylene-(CH₂)₂-, -(CH₂)₆-phenylene-(CH₂)₃-, -(CH₂)₆-phenylene-(CH₂)₄-, -(CH₂)₆-phenylene-(CH₂)₅-, -(CH₂)₆-phenylene-(CH₂)₆-, - (CH₂)₆-phenylene-(CH₂)₇-, -(CH₂)₆-phenylene-(CH₂)₈-, -(CH₂)₇-phenylene-(CH₂)₁-, -(CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₉-phenylene-CH₂-, -(CH₂)₈-phenylene-(CH₂)₂-, -(CH₂)₈-phenylene-(CH₂)₃-, -(CH₂)₈-phenylene-(CH₂)₄-, - (CH₂)₈-phenylene-(CH₂)₅-, -(CH₂)₈-phenylene-(CH₂)₆-, -(CH₂)₈-phenylene-(CH₂)₇-, -N(Rₐ₇)-CH₂-phenylene-CH₂-, -N(Rₐ₇)-CH₂-phenylene-(CH₂)₂-, -N(Rₐ₇)-CH₂-phenylene-(CH₂)₃-, -N(Rₐ₇)-CH₂-phenylene-(CH₂)₄-, -N(Rₐ₇)-CH₂-phenylene-(CH₂)₅-, -N(Rₐ₇)-CH₂-phenylene-(CH₂)₆-, -N(Rₐ₇)-CH₂-phenylene-(CH₂)₇-, -N(Rₐ₇)-CH₂-phenylene-(CH₂)₈-, -CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, - CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, -CH₂-N(Rₐ₇)-(CH₂)₂-phenylene-(CH₂)_{S}-, -(CH₂)₂-N(Ra₇)-CH₂-phenylene-CH₂-, -(CH₂)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, -(CH₂)₂-N(Rₐ₇)-CH₂-phenylene-(CH₂)₅-, -(CH₂)₂-N(Rₐ₇)-CH₂-phenylene-(CH₂)₆-,-(CH₂)₂-N(Rₐ₇)-CH₂-phenylene-(CH₂)₇-, -(CH₂)₂-N(Rₐ₇)-CH₂-phenylene-(CH₂)₈-, -(CH₂)₃-N(Rₐ₇)-CH₂-phenylene-CH₂-, -(CH₂)₃-N(Rₐ₇)-CH₂-phenylene-(CH₂)₂-, -(CH₂)₃-N(Rₐ₇)-CH₂-phenylene-(CH₂)₃-, - (CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, -(CH₂)₄-N(Rₐ₇)-CH₂-phenylene-(CH₂)₃-, -(CH₂)₄-N(Rₐ₇)-CH₂-phenylene-(CH₂)₈-, - (CH₂)₅-N(Rₐ₇)-CH₂-phenylene-(CH₂)₃-, -(CH₂)₅-N(Rₐ₇)-CH₂-phenylene-(CH₂)₈-, -(CH₂)₆-N(Rₐ₇)-CH₂-phenylene-(CH₂)₃-, -(CH₂)₆-N(Rₐ₇)-CH₂-phenylene-(CH₂)₈-, -(CH₂)₇-N(Rₐ₇)-CH₂-phenylene-(CH₂)₃-, - (CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, -(CH₂)₈-N(Rₐ₇)-CH₂-phenylene-(CH₂)₄-, -(CH₂)₈-N(Rₐ₇)-CH₂-phenylene-(CH₂)₅-, - (CH₂)₈-N(Rₐ₇)-CH₂-phenylene-(CH₂)₆-, -piperidinylene-CH₂-, -piperidinylene-(CH₂)₂-, -piperidinylene-(CH₂)₃-, -piperidinylene-(CH₂)₄-, -piperidinylene-(CH₂)₅-, -piperidinylene-(CH₂)₆-, -piperidinylene-(CH₂)₇-, -piperidinylene-(CH₂)₈-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, - (CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperldinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₉-piperidinylene-(CH₂)₂-, -(CH₂)₈-piperidinylene-(CH₂)₃-, -(CH₂)₈-piperidinylene-(CH₂)₄-, -(CH₂)₉-piperidinylene-(CH₂)₅-, -(CH₂)₈-piperidinylene-(CH₂)₆-, -(CH₂)₈-piperidinylene-(CH₂)₇-, -N(Rₐ₇)-CH₂-piperidinylene-CH₂-, -N(Rₐ₇)-CH₂-piperidinylene-(CH₂)₂-, -N(Rₐ₇)-CH₂-piperidinylene-(CH₂)₃-, -N(Rₐ₇)-CH₂-piperidinylene-(CH₂)₄-, -N(Rₐ₇)-CH₂-piperidinylene-(CH₂)₅-, -N(Rₐ₇)-CH₂-piperidinylene-(CH₂)₆-, - N(Rₐ₇)-CH₂-piperidinylene-(CH₂)₇-, -N(Rₐ₇)-CH₂-piperidinylene-(CH₂)₈-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, -CH₂-N(Rₐ₇)-CH₂-piperidinylene-(CH₂)₄-, -CH₂-N(Rₐ₇)-CH₂-piperidinylene-(CH₂)₅-, -CH₂-N(Ra₇)-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇)-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇)-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-, - (CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, - (CH₂)₅-N(Rₐ₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, - (CH₂)₈-N(Rₐ₇)-CH₂-piperidinylene-(CH₂)₆-, -piperazinylene-CH₂-, -piperazinylene-(CH₂)₂-, - piperazinylene-(CH₂)₃-, -piperazinylene-(CH₂)₄-, -piperazinylene-(CH₂)₅-, -piperazinylene-(CH₂)₆-,-piperazinylene-(CH₂)₇-, -piperazinylene-(CH₂)₈-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₉-, -(CH₂)₅-piperazinylene-( CH₂) ₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -( CH₂)₈-piperazinylene-( CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
each phenylene is independently optionally substituted with a substituent selected from the group consisting of deuterium, hydroxy, amino, mercapto, nitro, halogen, cyano, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof; and
the piperazinylene and piperidinylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof. In the present disclosure, unless otherwise specified, any one end of the above-mentioned groups may be connected to R_{w} of the compound of Formula (II), while the other end is connected to R_{w3}. For example, in the group -O-CH₂-, the O can be connected to R_{w} of the compound of Formula (II), and the CH₂ can be connected to R_{w3} of the compound of Formula (II), and vice versa.

In some embodiments, the compound of Formula (II) is also represented by Formula (II-A), Formula (II-B), Formula (II-C), or Formula (II-D): 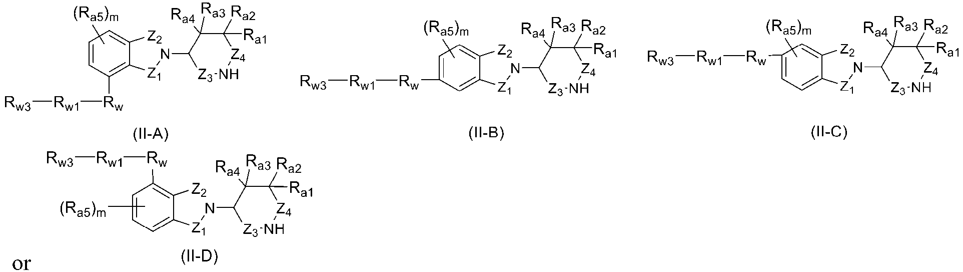 wherein Z₁, Z₂, Z₃, Z₄, (Rₐ₅)ₘ, Rₐ₁, Rₐ₂, Rₐ₃, Rₐ₄, R_{w}, R_{w1}, and R_{w3} are as defined in the compound of Formula (II) above and embodiments thereof.

In some embodiments, the compound of Formula (II) is also represented by Formula (II-1): 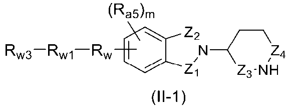 wherein Z₁, Z₂, Z₃, Z₄, (Rₐ₅)ₘ, R_{w}, R_{w1}, and R_{w3} are as defined in the compound of Formula (II) above and embodiments thereof.

In some embodiments, R_{w} of the compound of Formula (II-1) represents O, S, S(O), S(O)₂, alkenylene, alkynylene or N(Rₐ₆), wherein Rₐ₆ represents H or C₁₋₃ alkyl. In some embodiments, R_{w} of the compound of Formula (II-1) represents a bond.

In some embodiments, the compound of Formula (II) is also represented by Formula (II-2): 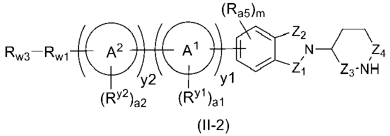 wherein Z₁, Z₂, Z₃, Z₄, (Rₐ₅)ₘ, ring A¹, ring A², y1, (R^{y1})ₐ₁, y2, (R^{y2})ₐ₂, R_{w1}, and R_{w3} are as defined in the compound of Formula (II) above and embodiments thereof.

In some embodiments, the compound of Formula (II) is also represented by Formula (II-3): 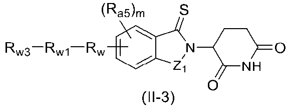 wherein Z₁, (Rₐ₅)ₘ, R_{w}, R_{w1}, and R_{w3} are as defined in the compound of Formula (II) above and embodiments thereof.

In some embodiments, the compound of Formula (II) is also represented by Formula (II-4): 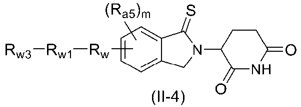 wherein (Rₐ₅)ₘ, R_{w}, R_{w1}, and R_{w3} are as defined in the compound of Formula (II) above and embodiments thereof.

In some embodiments, the compound of Formula (II) is also represented by Formula (II-5): 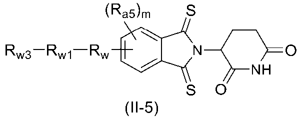 wherein (Rₐ₅)ₘ, R_{w}, R_{w1}, and R_{w3} are as defined in the compound of Formula (II) above and embodiments thereof.

In some embodiments, the compound of Formula (II) is also represented by Formula (II-6): 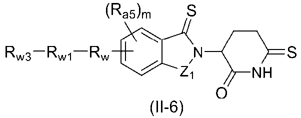 wherein Z₁, (Rₐ₅)ₘ, R_{w}, R_{w1}, and R_{w3} are as defined in the compound of Formula (II) above and embodiments thereof.

In some embodiments, the compound of Formula (II) is also represented by Formula (II-7): 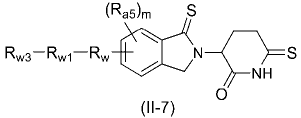 wherein (Rₐ₅)ₘ, R_{w}, R_{w1}, and R_{w3} are as defined in the compound of Formula (II) above and embodiments thereof.

In some embodiments, the compound of Formula (II) is also represented by Formula (II-8): 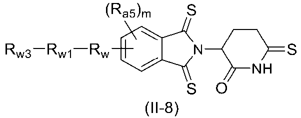 wherein (Rₐ₅)ₘ, R_{w}, R_{w1}, and R_{w3} are as defined in the compound of Formula (II) above and embodiments thereof.

In some embodiments, the compounds of Formula (II) of the present invention and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 2 below are provided.

**Table 2. The compounds of the present disclosure**

| Compound No. | Structure of compound | Compound's name | Chinese translation of Compound's name |
|---|---|---|---|
| | 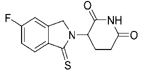 | 3-(5-fluoro-1-thioxoisoindolin-2-yl)piperidine-2,6-dione | 3-(5-fluoro-1-thioxoisoindolin-2-yl)piperidine-2,6-dione |
| | 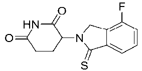 | 3-(4-fluoro- 1-thioxoisoindolin-2-yl)piperidine-2,6-dione | 3-(4-fluoro- 1-thioxoisoindolin-2-yl)piperidine-2,6-dione |
| | 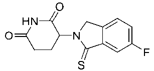 | 3-(6-fluoro-1-thioxoisoindolin-2-yl)piperidine-2,6-dione | 3-(6-fluoro- 1-thioxoisoindolin-2-yl)piperidine-2,6-dione |
| | 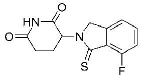 | 3-(7-fluoro-1-thioxoisoindolin-2-yl)piperidine-2,6-dione | 3-(7-fluoro-1-thioxoisoindolin-2-yl)piperidine-2,6-dione |
| | 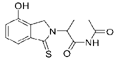 | 3-(4-hydroxy-1-thioxoisoindolin-2-yl)piperidine-2,6-dione | 3-(4-hydroxy-1-thioxoisoindolin-2-yl)piperidine-2,6-dione |
| | 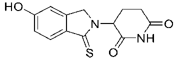 | 3-(5-hydroxy-1-thioxoisoindolin-2-yl)piperidine-2,6-dione | 3-(5-hydroxy-1-thioxoisoindolin-2-yl)piperidine-2,6-dione |
| | 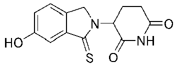 | 3-(6-hydroxy-1-thioxoisoindolin-2-yl)piperidine-2,6-dione | 3-(6-hydroxy-1-thioxoisoindolin-2-yl)piperidine-2,6-dione |
| | 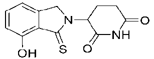 | 3-(7-hydroxy-1-thioxoisoindolin-2-yl)piperidine-2,6-dione | 3-(7-hydroxy-1-thioxoisoindolin-2-yl)piperidine-2,6-dione |
| | 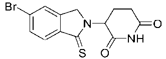 | 3-(5-bromo-1-thioxoisoindolin-2-yl)piperidine-2,6-dione | 3-(5-bromo-1-thioxoisoindolin-2-yl)piperidine-2,6-dione |
| | 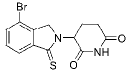 | 3-(4-bromo-1-thioxoisoindolin-2-yl)piperidine-2,6-dione | 3-(4-bromo-1-thioxoisoindolin-2-yl)piperidine-2,6-dione |
| GT-09531 | 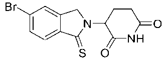 | 3-(5-bromo-1-thioxoisoindolin-2-yl)piperidine-2,6-dione | 3-(5-bromo-1-thioxoisoindolin-2-yl)piperidine-2,6-dione |
| | 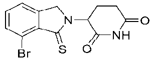 | 3-(7-bromo-1-thioxoisoindolin-2-yl)piperidine-2,6-dione | 3-(7-bromo-1-thioxoisoindolin-2-yl)piperidine-2,6-dione |
| GT-06977 | 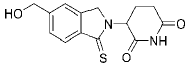 | 3-(5-(hydroxymethyl)-1-thioxoisoindolin-2-yl)piperidine-2,6-dione | 3-(5-(hydroxymethyl)-1-thioxoisoindolin-2-yl)piperidine-2,6-dione |
| GT-06981 | 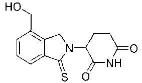 | 3-(4-(hydroxymethyl)-1-thioxoisoindolin-2-yl)piperidine-2,6-dione | 3-(4-(hydroxymethyl)-1-thioxoisoindolin-2-yl)piperidine-2,6-dione |
| GT-05566 | 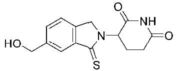 | 3-(6-(hydroxymethyl)-1-thioxoisoindolin-2-yl)piperidine-2,6-dione | 3-(6-(hydroxymethyl)-1-thioxoisoindolin-2-yl)piperidine-2,6-dione |
| | 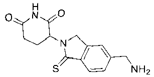 | 3-(5-(aminomethyl)-1-thioxoisoindolin-2-yl)piperidine-2,6-dione | 3-(5-(aminomethyl)-1-thioxoisoindolin-2-yl)piperidine-2,6-dione |
| | 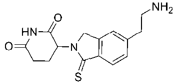 | 3-(5-(2-aminoethyl)-1-thioxoisoindolin-2-yl)piperidine-2,6-dione | 3-(5-(2-aminoethyl)-1-thioxoisoindolin-2-yl)piperidine-2,6-dione |
| | 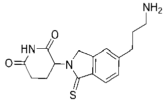 | 3-(5-(3-aminopropyl)-1-thioxoisoindolin-2-yl)piperidine-2,6-dione | 3-(5-(3-aminopropyl)-1-thioxoisoindolin-2-yl)piperidine-2,6-dione |
| | 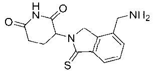 | 3-(4-(aminomethyl)-1-thioxoisoindolin-2-yl)piperidine-2,6-dione | 3-(4-(aminomethyl)-1-thioxoisoindolin-2-yl)piperidine-2,6-dione |
| | 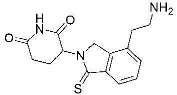 | 3-(4-(2-aminoethyl)-1-thioxoisoindolin-2-yl)piperidine-2,6-dione | 3-(4-(2-aminoethyl)-1-thioxoisoindolin-2-yl)piperidine-2,6-dione |
| | 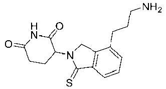 | 3-(4-(3-aminopropyl)-1-thioxoisoindolin-2-yl)piperidine-2,6-dione | 3-(4-(3-aminopropyl)-1-thioxoisoindolin-2-yl)piperidine-2,6-dione |
| | 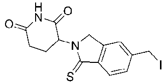 | 3-(5-(iodomethyl)-1-thioxoisoindolin-2-yl)piperidine-2,6-dione | 3-(5-(iodomethyl)-1-thioxoisoindolin-2-yl)piperidine-2,6-dione |
| | 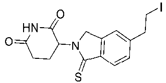 | 3-(5-(2-iodoethyl)-1-thioxoisoindolin-2-yl)piperidine-2,6-dione | 3-(5-(2-iodoethyl)-1-thioxoisoindolin-2-yl)piperidine-2,6-dione |
| | 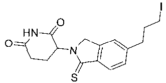 | 3-(5-(3-iodopropyl)-1-thioxoisoindolin-2-yl)piperidine-2,6-dione | 3-(5-(3-iodopropyl)-1-thioxoisoindolin-2-yl)piperidine-2,6-dione |
| | 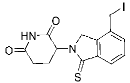 | 3-(4-(iodomethyl)-1-thioxoisoindolin-2-yl)piperidine-2,6-dione | 3-(4-(iodomethyl)-1-thioxoisoindolin-2-yl)piperidine-2,6-dione |
| | 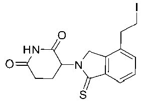 | 3-(4-(2-iodoethyl)-1-thioxoisoindolin-2-yl)piperidine-2,6-dione | 3-(4-(2-iodoethyl)-1-thioxoisoindolin-2-yl)piperidine-2,6-dione |
| | 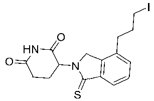 | 3-(4-(3-iodopropyl)-1-thioxoisoindolin-2-yl)piperidine-2,6-dione | 3-(4-(3-iodopropyl)-1-thioxoisoindolin-2-yl)piperidine-2,6-dione |
| | 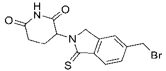 | 3-(5-(bromomethyl)-1-thioxoisoindolin-2-yl)piperidine-2,6-dione | 3-(5-(bromomethyl)-1-thioxoisoindolin-2-yl)piperidine-2,6-dione |
| | 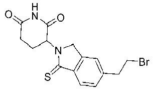 | 3-(5-(2-bromoethyl)-1-thioxoisoindolin-2-yl)piperidine-2,6-dione | 3-(5-(2-bromoethyl)-1-thioxoisoindolin-2-yl)piperidine-2,6-dione |
| | 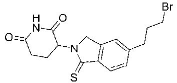 | 3-(5-(3-bromopropyl)-1-thioxoisoindolin-2-yl)piperidine-2,6-dione | 3-(5-(3-bromopropyl)-1-thioxoisoindolin-2-yl)piperidine-2,6-dione |
| | 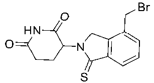 | 3-(4-(bromomethyl)-1-thioxoisoindolin-2-yl)piperidine-2,6-dione | 3-(4-(bromomethyl)-1-thioxoisoindolin-2-yl)piperidine-2,6-dione |
| | 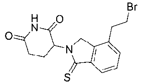 | 3-(4-(2-bromoethyl)-1-thioxoisoindolin-2-yl)piperidine-2,6-dione | 3-(4-(2-bromoethyl)-1-thioxoisoindolin-2-yl)piperidine-2,6-dione |
| | 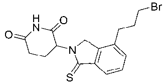 | 3-(4-(3-bromopropyl)-1-thioxoisoindolin-2-yl)piperidine-2,6-dione | 3-(4-(3-bromopropyl)-1-thioxoisoindolin-2-yl)piperidine-2,6-dione |
| | 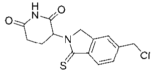 | 3-(5-(chloromethyl)-1-thioxoisoindolin-2-yl)piperidine-2,6-dione | 3-(5-(chloromethyl)-1-thioxoisoindolin-2-yl)piperidine-2,6-dione |
| | 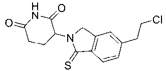 | 3-(5-(2-chloroethyl)-1-thioxoisoindolin-2-yl)piperidine-2,6-dione | 3-(5-(2-chloroethyl)-1-thioxoisoindolin-2-yl)piperidine-2,6-dione |
| | 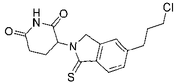 | 3-(5-(3-chloropropyl)-1-thioxoisoindolin-2-yl)piperidine-2,6-dione | 3-(5-(3-chloropropyl)-1-thioxoisoindolin-2-yl)piperidine-2,6-dione |
| | 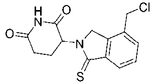 | 3-(4-(chloromethyl)-1-thioxoisoindolin-2-yl)piperidine-2,6-dione | 3-(4-(chloromethyl)-1-thioxoisoindolin-2-yl)piperidine-2,6-dione |
| | 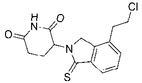 | 3-(4-(2-chloroethyl)-1-thioxoisoindolin-2-yl)piperidine-2,6-dione | 3-(4-(2-chloroethyl)-1-thioxoisoindolin-2-yl)piperidine-2,6-dione |
| | 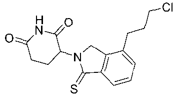 | 3-(4-(3-chloropropyl)-1-thioxoisoindolin-2-yl)piperidine-2,6-dione | 3-(4-(3-chloropropyl)-1-thioxoisoindolin-2-yl)piperidine-2,6-dione |
| GT-09596 | 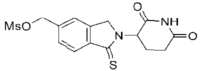 | (2-(2,6-dioxopiperidin-3-yl)-1-thioxoisoindolin-5-yl)methyl methanesulfonate | (2-(2,6-dioxopiperidin-3-yl)-1-thioxoisoindolin-5-yl)methyl methanesulfonate |
| | 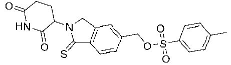 | (2-(2,6-dioxopiperidin-3-yl)-1-thioxoisoindolin-5-yl)methyl 4-methylbenzenesulfonate | (2-(2,6-dioxopiperidin-3-yl)-1-thioxoisoindolin-5-yl)methyl 4-methylbenzenesulfonate |
| | 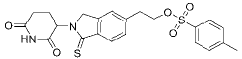 | 2-(2-(2,6-dioxopiperidin-3-yl)-1-thioxoisoindolin-5-yl)ethyl 4-methylbenzenesulfonate | 2-(2-(2,6-dioxopiperidin-3-yl)-1-thioxoisoindolin-5-yl)ethyl 4-methylbenzenesulfonate |
| | 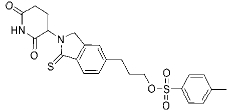 | 3-(2-(2,6-dioxopiperidin-3-yl)-1-thioxoisoindolin-5-yl)propyl 4-methylbenzenesulfonate | 3-(2-(2,6-dioxopiperidin-3-yl)-1-thioxoisoindolin-5-yl)propyl 4-methylbenzenesulfonate |
| | 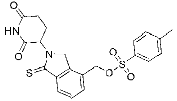 | (2-(2,6-dioxopiperidin-3-yl)-1-thioxoisoindolin-4-yl)methyl 4-methylbenzenesulfonate | (2-(2,6-dioxopiperidin-3-yl)-1-thioxoisoindolin-4-yl)methyl 4-methylbenzenesulfonate |
| | 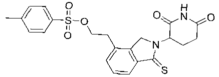 | 2-(2-(2,6-dioxopiperidin-3-yl)-1-thioxoisoindolin-4-yl)ethyl 4-methylbenzenesulfonate | 2-(2-(2,6-dioxopiperidin-3-yl)-1-thioxoisoindolin-4-yl)ethyl 4-methylbenzenesulfonate |
| | 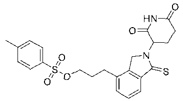 | 3-(2-(2,6-dioxopiperidin-3-yl)-1-thioxoisoindolin-4-yl)propyl 4-methylbenzenesulfonate | 3-(2-(2,6-dioxopiperidin-3-yl)-1-thioxoisoindolin-4-yl)propyl 4-methylbenzenesulfonate |
| | 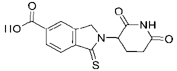 | 2-(2,6-dioxopiperidin-3-yl)-1-thioxoisoindoline-5-carboxylic acid | 2-(2,6-dioxopiperidin- 3-yl)-1-thioxoisoindoline-5-carboxylic acid |
| | 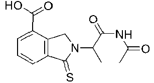 | 2-(2,6-dioxopiperidin-3-yl)-1-thioxoisoindoline-4-carboxylic acid | 2-(2,6-dioxopiperidin-3-yl)-1-thioxoisoindoline-4-carboxylic acid |
| | 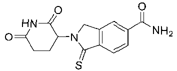 | 2-(2,6-dioxopiperidin-3-yl)-1-thioxoisoindoline-5-carboxamide | 2-(2,6-dioxopiperidin-3-yl)-1-thioxoisoindoline-5-carboxamide |
| | 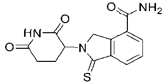 | 2-(2,6-dioxopiperidin-3-yl)-1-thioxoisoindoline-4-carboxamide | 2-(2,6-dioxopiperidin-3-yl)-1-thioxoisoindoline-4-carboxamide |
| | 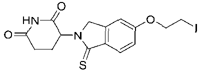 | 3-(5-(2-iodoethoxy)-1-thioxoisoindolin-2-yl)piperidine-2,6-dione | 3-(5-(2-iodoethoxy)-1-thioxoisoindolin-2-yl)piperidine-2,6-dione |
| | 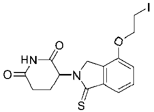 | 3-(4-(2-iodoethoxy)-1-thioxoisoindolin-2-yl)piperidine-2,6-dione | 3-(4-(2-iodoethoxy)-1-thioxoisoindolin-2-yl)piperidine-2,6-dione |
| | 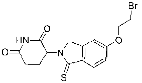 | 3-(5-(2-bromoethoxy)-1-thioxoisoindolin-2-yl)piperidine-2,6-dione | 3-(5-(2-bromoethoxy)-1-thioxoisoindolin-2-yl)piperidine-2,6-dione |
| | 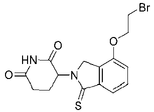 | 3-(4-(2-bromoethoxy)-1-thioxoisoindolin-2-yl)piperidine-2,6-dione | 3-(4-(2-bromoethoxy)-1-thioxoisoindolin-2-yl)piperidine-2,6-dione |
| | 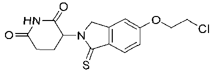 | 3-(5-(2-chloroethoxy)-1-thioxoisoindolin-2-yl)piperidine-2,6-dione | 3-(5-(2-chloroethoxy)-1-thioxoisoindolin-2-yl)piperidine-2,6-dione |
| | 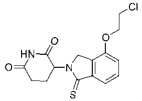 | 3-(4-(2-chloroethoxy)-1-thioxoisoindolin-2-yl)piperidine-2,6-dione | 3-(4-(2-chloroethoxy)-1-thioxoisoindolin-2-yl)piperidine-2,6-dione |
| | 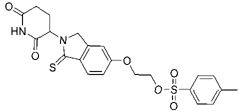 | 2-((2-(2,6-dioxopiperidin-3-yl)-1-thioxoisoindolin-5-yl)oxy)ethyl 4-methylbenzenesulfonate | 2-((2-(2,6-dioxopiperidin-3-yl)-1-thioxoisoindolin-5-yl)oxy)ethyl 4-methylbenzenesulfonate |
| | 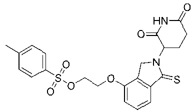 | 2-((2-(2,6-dioxopiperidin-3-yl)-1-thioxoisoindolin-4-yl)oxy)ethyl 4-methylbenzenesulfonate | 2-((2-(2,6-dioxopiperidin-3-yl)-1-thioxoisoindolin-4-yl)oxy)ethyl 4-methylbenzenesulfonate |
| | 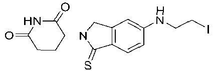 | 3-(5-((2-iodoethyl)amino)-1-thioxoisoindolin-2-yl)piperidine-2,6-dione | 3-(5-((2-iodoethyl)amino)-1-thioxoisoindolin-2-yl)piperidine-2,6-dione |
| | 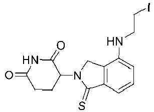 | 3-(4-((2-iodoethyl)amino)-1-thioxoisoindolin-2-yl)piperidine-2,6-dione | 3-(4-((2-iodoethyl)amino)-1-thioxoisoindolin-2-yl)piperidine-2,6-dione |
| | 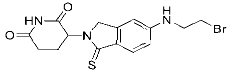 | 3-(5-((2-bromoethyl)amino)-1-thioxoisoindolin-2-yl)piperidine-2,6-dione | 3-(5-((2-bromoethyl)amino)-1-thioxoisoindolin-2-yl)piperidine-2,6-dione |
| | 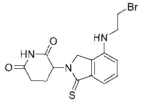 | 3-(4-((2-bromoethyl)amino)-1-thioxoisoindolin-2-yl)piperidine-2,6-dione | 3-(4-((2-bromoethyl)amino)-1-thioxoisoindolin-2-yl)piperidine-2,6-dione |
| | 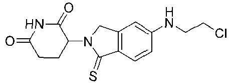 | 3-(5-((2-chloroethyl)amino)-1-thioxoisoindolin-2-yl)piperidine-2,6-dione | 3-(5-((2-chloroethyl)amino)-1-thioxoisoindolin-2-yl)piperidine-2,6-dione |
| | 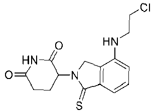 | 3-(4-((2-chloroethyl)amino)-1-thioxoisoindolin-2-yl)piperidine-2,6-dione | 3-(4-((2-chloroethyl)amino)-1-thioxoisoindolin-2-yl)piperidine-2,6-dione |
| | 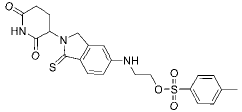 | 2-((2-(2,6-dioxopiperidin-3-yl)-1-thioxoisoindolin-5-yl)amino)ethyl 4-methylbenzenesulfonate | 2-((2-(2,6-dioxopiperidin-3-yl)-1-thioxoisoindolin-5-yl)amino)ethyl 4-methylbenzenesulfonate |
| | 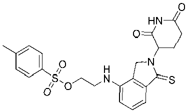 | 2-((2-(2,6-dioxopiperidin-3-yl)-1-thioxoisoindolin-4-yl)amino)ethyl 4-methylbenzenesulfonate | 2-((2-(2,6-dioxopiperidin-3-yl)-1-thioxoisoindolin-4-yl)amino)ethyl 4-methylbenzenesulfonate |
| | 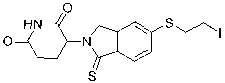 | 3-(5-((2-iodoethyl)thio)-1-thioxoisoindolin-2-yl)piperidine-2,6-dione | 3-(5-((2-iodoethyl)thio)-1-thioxoisoindolin-2-yl)piperidine-2,6-dione |
| | 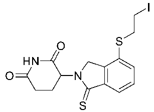 | 3-(4-((2-iodoethyl)thio)-1-thioxoisoindolin-2-yl)piperidine-2,6-dione | 3-(4-((2-iodoethyl)thio)-1-thioxoisoindolin-2-yl)piperidine-2,6-dione |
| | 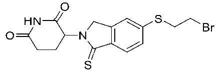 | 3-(5-((2-bromoethyl)thio)-1-thioxoisoindolin-2-yl)piperidine-2,6-dione | 3-(5-((2-bromoethyl)thio)-1-thioxoisoindolin-2-yl)piperidine-2,6-dione |
| | 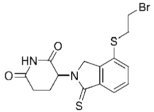 | 3-(4-((2-bromoethyl)thio)-1-thioxoisoindolin-2-yl)piperidine-2,6-dione | 3-(4-((2-bromoethyl)thio)-1-thioxoisoindolin-2-yl)piperidine-2,6-dione |
| | 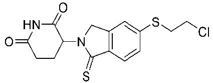 | 3-(5-((2-chloroethyl)thio)-1-thioxoisoindolin-2-yl)piperidine-2,6-dione | 3-(5-((2-chloroethyl)thio)-1-thioxoisoindolin-2-yl)piperidine-2,6-dione |
| | 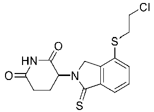 | 3-(4-((2-chloroethyl)thio)-1-thioxoisoindolin-2-yl)piperidine-2,6-dione | 3-(4-((2-chloroethyl)thio)-1-thioxoisoindolin-2-yl)piperidine-2,6-dione |
| | 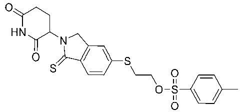 | 2-((2-(2,6-dioxopiperidin-3-yl)-1-thioxoisoindolin-5-yl)thio)ethyl 4-methylbenzenesulfonate | 2-((2-(2,6-dioxopiperidin-3-yl)-1-thioxoisoindolin-5-yl)thio)ethyl 4-methylbenzenesulfonate |
| | 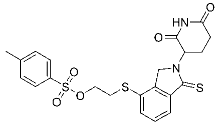 | 2-((2-(2,6-dioxopiperidin-3-yl)-1-thioxoisoindolin-4-yl)thio)ethyl 4-methylbenzenesulfonate | 2-((2-(2,6-dioxopiperidin-3-yl)-1-thioxoisoindolin-4-yl)thio)ethyl 4-methylbenzenesulfonate |
| | 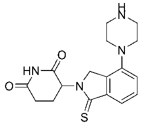 | 3-(4-(piperazin-1-yl)-1-thioxoisoindolin-2-yl)piperidine-2,6-dione | 3-(4-(piperazin-1-yl)-1-thioxoisoindolin-2-yl)piperidine-2,6-dione |
| | 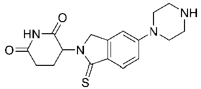 | 3-(5-(piperazin-1-yl)-1-thioxoisoindolin-2-yl)piperidine-2,6-dione | 3-(5-(piperazin-1-yl)-1-thioxoisoindolin-2-yl)piperidine-2,6-dione |
| | 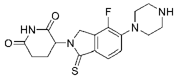 | 3-(4-fluoro-5-(piperazin-1-yl)-1-thioxoisoindolin-2-yl)piperidine-2,6-dione | 3-(4-fluoro-5-(piperazin-1-yl)-1-thioxoisoindolin-2-yl)piperidine-2,6-dione |
| | 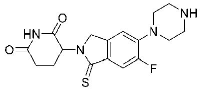 | 3-(6-fluoro-5-(piperazin-1-yl)-1-thioxoisoindolin-2-yl)piperidine-2,6-dione | 3-(6-fluoro-5-(piperazin-1-yl)-1-thioxoisoindolin-2-yl)piperidine-2,6-dione |
| | 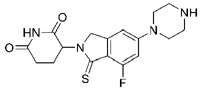 | 3-(7-fluoro-5-(piperazin-1-yl)-1-thioxoisoindolin-2-yl)piperidine-2,6-dione | 3-(7-fluoro-5-(piperazin-1-yl)-1-thioxoisoindolin-2-yl)piperidine-2,6-dione |
| | 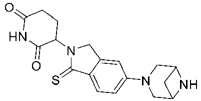 | 3-(5-(3,6-diazabicyclo[3.1.1]heptan -3-yl)-1-thioxoisoindolin-2-yl)piperidine-2,6-dione | 3-(5-(3,6-diazabicyclo[3.1.1]heptan -3-yl)-1-thioxoisoindolin-2-yl)piperidine-2,6-dione |
| | 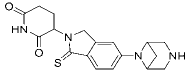 | 3-(5-(3,6-diazabicyclo[3.1.1]heptan -6-yl)-1-thioxoisoindolin-2-yl)piperidine-2,6-dione | 3-(5-(3,6-diazabicyclo[3.1.1]heptan -6-yl)-1-thioxoisoindolin-2-yl)piperidine-2,6-dione |
| | 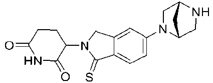 | 3-(5-((1R,4R)-2,5-diazabicyclo[2.2.1]heptan -2-yl)-1-thioxoisoindolin-2-yl)piperidine-2,6-dione | 3-(5-((1R,4R)-2,5-diazabicyclo[2.2.1]heptan -2-yl)-1-thioxoisoindolin-2-yl)piperidine-2,6-dione |
| | 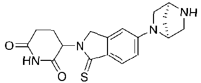 | 3-(5-((1S,4S)-2,5-diazabicyclo[2.2.1]heptan -2-yl)-1-thioxoisoindolin-2-yl)piperidine-2,6-dione | 3-(5-((1S,4S)-2,5-diazabicyclo[2.2.1]heptan -2-yl)-1-thioxoisoindolin-2-yl)piperidine-2,6-dione |
| | 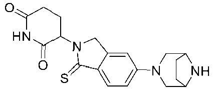 | 3-(5-(3,8-diazabicyclo[3.2.1]octan-3-yl)-1-thioxoisoindolin-2-vl)piperidine-2,6-dione | 3-(5-(3,8-diazabicyclo[3.2.1]octan-3-yl)-1-thioxoisoindolin-2-yl)piperidine-2,6-dione |
| | 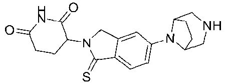 | 3-(5-(3,8-diazabicyclo[3.2.1]octan-8-yl)-1-thioxoisoindolin-2-yl)piperidine-2,6-dione | 3-(5-(3,8-diazabicyclo[3.2.1]octan-8-yl)-1-thioxoisoindolin-2-yl)piperidine-2,6-dione |
| | 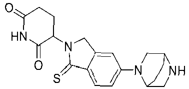 | 3-(5-(2,5-diazabicyclo[2.2.2]octan-2-yl)-1-thioxoisoindolin-2-yl)piperidine-2,6-dione | 3-(5-(2,5-diazabicyclo[2.2.2]octan-2-yl)-1-thioxoisoindolin-2-yl)piperidine-2,6-dione |
| | 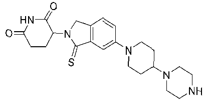 | 3-(6-(4-(piperazin-1-yl)piperidin-1-yl)-1-thioxoisoindolin-2-yl)piperidine-2,6-dione | 3-(6-(4-(piperazin-1-yl)piperidin-1-yl)-1-thioxoisoindolin-2-yl)piperidine-2,6-dione |
| | 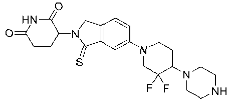 | 3-(6-(3,3-difluoro-4-(piperazin-1-yl)piperidin-1-yl)-1-thioxoisoindolin-2-yl)piperidine-2,6-dione | 3-(6-(3,3-difluoro-4-(piperazin-1-yl)piperidin-1-yl)-1-thioxoisoindolin-2-yl)piperidine-2,6-dione |
| | 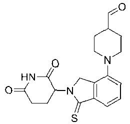 | 1-(2-(2,6-dioxopiperidin-3-yl)-1-thioxoisoindolin-4-yl)piperidine-4-carbaldehyde | 1-(2-(2,6-dioxopiperidin-3-yl)-1-thioxoisoindolin-4-yl)piperidine-4-carbaldehyde |
| | 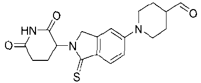 | 1-(2-(2,6-dioxopiperidin-3-yl)-1-thioxoisoindolin-5-yl)piperidine-4-carbaldehyde | 1-(2-(2,6-dioxopiperidin-3-yl)-1-thioxoisoindolin-5-yl)piperidine-4-carbaldehyde |
| | 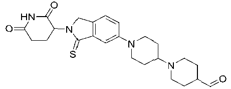 | 1'-(2-(2,6-dioxopiperidin-3-yl)-3-thioxoisoindolin-5-yl)-[1,4'-bipiperidine]-4-carbaldehyde | 1 '-(2-(2,6-dioxopiperidin-3-yl)-3-thioxoisoindolin-5-yl)-[1,4'-bipiperidine]-4-carbaldehyde |
| | 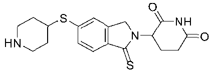 | 3-(5-(piperidin-4-ylthio)-1-thioxoisoindolin-2-yl)piperidine-2,6-dione | 3-(5-(piperidin-4-ylthio)-1-thioxoisoindolin-2-yl)piperidine-2,6-dione |
| | 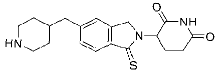 | 3-(5-(piperidin-4-ylmethyl)-1-thioxoisoindolin-2-yl)piperidine-2,6-dione | 3-(5-(piperidin-4-ylmethyl)-1-thioxoisoindolin-2-yl)piperidine-2,6-dione |
| | 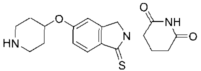 | 3-(5-(piperidin-4-yloxy)-1-thioxoisoindolin-2-yl)piperidine-2,6-dione | 3-(5-(piperidin-4-yloxy)-1-thioxoisoindolin-2-yl)piperidine-2,6-dione |
| | 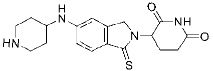 | 3-(5-(piperidin-4-ylamino)-1-thioxoisoindolin-2-yl)piperidine-2,6-dione | 3-(5-(piperidin-4-ylamino)-1-thioxoisoindolin-2-yl)piperidine-2,6-dione |
| | 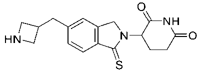 | 3-(5-(azetidin-3-ylmethyl)-1-thioxoisoindolin-2-yl)piperidine-2,6-dione | 3-(5-(azetidin-3-ylmethyl)-1-thioxoisoindolin-2-yl)piperidine-2,6-dione |
| | 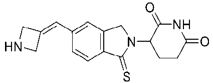 | 3-(5-(azetidin-3-ylidenemethyl)-1-thioxoisoindolin-2-yl)piperidine-2,6-dione | 3-(5-(azetidin-3-ylidenemethyl)-1-thioxoisoindolin-2-yl)piperidine-2,6-dione |
| | 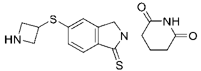 | 3-(5-(azetidin-3-ylthio)-1-thioxoisoindolin-2-yl)piperidine-2,6-dione | 3-(5-(azetidin-3-ylthio)-1-thioxoisoindolin-2-yl)piperidine-2,6-dione |
| | 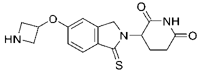 | 3-(5-(azetidin-3-yloxy)-1-thioxoisoindolin-2-yl)piperidine-2,6-dione | 3-(5-(azetidin-3-yloxy)-1-thioxoisoindolin-2-yl)piperidine-2,6-dione |
| | 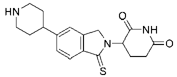 | 3-(5-(piperidin-4-yl)-1-thioxoisoindolin-2-yl)piperidine-2,6-dione | 3-(5-(piperidin-4-yl)-1-thioxoisoindolin-2-yl)piperidine-2,6-dione |
| | 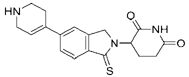 | 3-(5-(1,2,3,6-tetrahydropyridin-4-yl)-1-thioxoisoindolin-2-yl)piperidine-2,6-dione | 3-(5-(1,2,3,6-tetrahydropyridin-4-yl)-1-thioxoisoindolin-2-yl)piperidine-2,6-dione |
| | 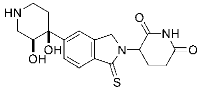 | rel-3-(5-((3S,4S)-3,4-dihydroxypiperidin-4-yl)-1-thioxoisoindolin-2-yl)piperidine-2,6-dione | rel-3-(5-((3S,4S)-3,4-dihydroxypiperidin-4-yl)-1-thioxoisoindolin-2-yl)piperidine-2,6-dione |
| | 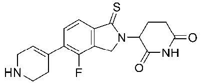 | 3-(4-fluoro-5-(1,2,3,6-tetrahydropyridin-4-yl)-1-thioxoisoindolin-2-yl)piperidine-2,6-dione | 3-(4-fluoro-5-(1,2,3,6-tetrahydropyridin-4-yl)-1-thioxoisoindolin-2-yl)piperidine-2,6-dione |
| | 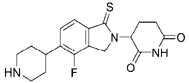 | 3-(4-fluoro-5-(piperidin-4-yl)-1-thioxoisoindolin-2-yl)piperidine-2,6-dione | 3-(4-fluoro-5-(piperidin-4-yl)-1-thioxoisoindolin-2-yl)piperidine-2,6-dione |
| | 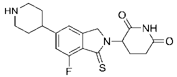 | 3-(7-fluoro-5-(piperidin-4-yl)-1-thioxoisoindolin-2-yl)piperidine-2,6-dione | 3-(7-fluoro-5-(piperidin-4-yl)-1-thioxoisoindolin-2-yl)piperidine-2,6-dione |
| | 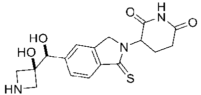 | 3-(5-((S)-hydroxy(3-hydroxyazetidin-3-yl)methyl)-1-thioxoisoindolin-2-yl)piperidine-2,6-dione | 3-(5-((S)-hydroxy(3-hydroxyazetidin-3-yl)methyl)-1-thioxoisoindolin-2-yl)piperidine-2,6-dione |
| | 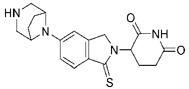 | 3-(5-(3,8-diazabicyclo[3.2.1]octan-8-yl)-1-thioxoisoindolin-2-yl)piperidine-2,6-dione | 3-(5-(3,8-diazabicyclo[3.2.1]octan-8-yl)-1-thioxoisoindolin-2-yl)piperidine-2,6-dione |
| | 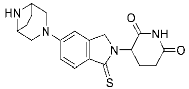 | 3-(5-(3,8-diazabicyclo[3.2.1]octan-3-yl)-1-thioxoisoindolin-2-yl)piperidine-2,6-dione | 3-(5-(3,8-diazabicyclo[3.2.1]octan-3-yl)-1-thioxoisoindolin-2-yl)piperidine-2,6-dione |
| | 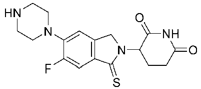 | 3-(6-fluoro-5-(piperazin-1-yl)-1-thioxoisoindolin-2-yl)piperidine-2,6-dione | 3-(6-fluoro-5-(piperazin-1-yl)-1-thioxoisoindolin-2-yl)piperidine-2,6-dione |
| | 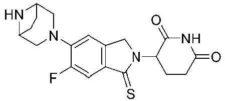 | 3-(5-(3,8-diazabicyclo[3.2.1]octan-3-yl)-6-fluoro-1-thioxoisoindolin-2-yl)piperidine-2,6-dione | 3-(5-(3,8-diazabicyclo[3.2.1]octan-3-yl)-6-fluoro-1-thioxoisoindolin-2-yl)piperidine-2,6-dione |
| | 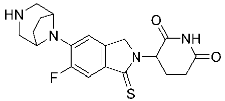 | 3-(5-(3,8-diazabicyclo[3.2.1]octan-8-yl)-6-fluoro-1-thioxoisoindolin-2-yl)piperidine-2,6-dione | 3-(5-(3,8-diazabicyclo[3.2.1]octan-8-yl)-6-fluoro-1-thioxoisoindolin-2-yl)piperidine-2,6-dione |
| | 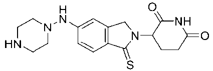 | 3-(5-(piperazin-1-ylamino)-1-thioxoisoindolin-2-yl)piperidine-2,6-dione | 3-(5-(piperazin-1-ylamino)-1-thioxoisoindolin-2-yl)piperidine-2,6-dione |
| | 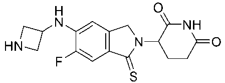 | 3-(5-(azetidin-3-ylamino)-6-fluoro-1-thioxoisoindolin-2-yl)piperidine-2,6-dione | 3-(5-(azetidin-3-ylamino)-6-fluoro-1-thioxoisoindolin-2-yl)piperidine-2,6-dione |
| | 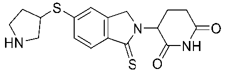 | 3-(5-(pyrrolidin-3-ylthio)-1-thioxoisoindolin-2-yl)piperidine-2,6-dione | 3-(5-(pyrrolidin-3-ylthio)-1-thioxoisoindolin-2-yl)piperidine-2,6-dione |
| | 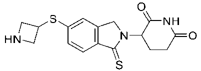 | 3-(5-(azetidin-3-ylthio)-1-thioxoisoindolin-2-yl)piperidine-2,6-dione | 3-(5-(azetidin-3-ylthio)-1-thioxoisoindolin-2-yl)piperidine-2,6-dione |
| | 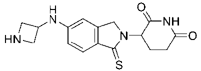 | 3-(5-(azetidin-3-ylamino)-1-thioxoisoindolin-2-yl)piperidine-2,6-dione | 3-(5-(azetidin-3-ylamino)-1-thioxoisoindolin-2-yl)piperidine-2,6-dione |
| | 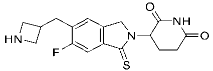 | 3-(5-(azetidin-3-ylmethyl)-6-fluoro-1-thioxoisoindolin-2-yl)piperidine-2,6-dione | 3-(5-(azetidin-3-ylmethyl)-6-fluoro-1-thioxoisoindolin-2-yl)piperidine-2,6-dione |
| GT-05563 | 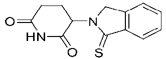 | 3-(1-thioxoisoindolin-2-yl)piperidine-2,6-dione | 3-(1-thioxoisoindolin-2-yl)piperidine-2,6-dione |
| GT-09597 | 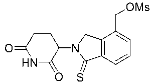 | (2-(2,6-dioxopiperidin-3-yl)-1-thioxoisoindolin-4-yl)methyl methanesulfonate | (2-(2,6-dioxopiperidin-3-yl)-1-thioxoisoindolin-4-yl)methyl methanesulfonate |
| GT-09598 | 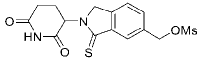 | (2-(2,6-dioxopiperidin-3-yl)-3-thioxoisoindolin-5-yl)methyl methanesulfonate | (2-(2,6-dioxopiperidin-3-yl)-3-thioxoisoindolin-5-yl)methyl methanesulfonate |

### II. Other Forms of Compounds (including salts, enantiomers, stereoisomers, solvates, isotopically enriched analogs, prodrugs, or polymorphs of compounds)

The compounds of the present disclosure have the structures of any one ofFormula (I), Formula (I-1), Formula (I-2), Formula (I-3), Formula (I-4), Formula (I-5-1), Formula (I-5-2), Formula (I-5-3), Formula (I-5-4), Formula (I-6), Formula (I-7), Formula (I-8), Formula (I-9), Formula (I-10-1), Formula (I-10-2), Formula (I-11), Formula (I-12), Formula (I-13-1), Formula (I-13-2), Formula (I-14), Formula (I-15), Formula (I-16), Formula (I-17), Formula (I-18-1), Formula (I-18-2), Formula (I-19), Formula (I-20), Formula (I-21), Formula (I-22), Formula (I-23), Formula (I-24), Formula (I-25), Formula (I-26), Formula (I-27), Formula (I-28), Formula (I-29), Formula (I-30), Formula (II), Formula (II-A), Formula (II-B), Formula (II-C), Formula (II-D), Formula (II-1), Formula (II-2), Formula (II-3), Formula (II-4), Formula (II-5), Formula (II-6), Formula (II-7) or Formula (II-8). Unless otherwise specified, all references to the compounds of the present disclosure also include compounds of any one of Formula (I), Formula (I-1), Formula (I-2), Formula (I-3), Formula (I-4), Formula (I-5-1), Formula (I-5-2), Formula (I-5-3), Formula (I-5-4), Formula (I-6), Formula (I-7), Formula (I-8), Formula (I-9), Formula (I-10-1), Formula (I-10-2), Formula (I-11), Formula (I-12), Formula (I-13-1), Formula (I-13-2), Formula (I-14), Formula (I-15), Formula (I-16), Formula (I-17), Formula (I-18-1), Formula (I-18-2), Formula (I-19), Formula (I-20), Formula (I-21), Formula (I-22), Formula (I-23), Formula (I-24), Formula (I-25), Formula (I-26), Formula (I-27), Formula (I-28), Formula (I-29), Formula (I-30), Formula (II), Formula (II-A), Formula (II-B), Formula (II-C), Formula (II-D), Formula (II-1), Formula (II-2), Formula (II-3), Formula (II-4), Formula (II-5), Formula (II-6), Formula (II-7) or Formula (II-8) and specific compounds within the scope of these general formulae.

It should be recognized that compounds of the present disclosure (including Formula (I), Formula (I-1), Formula (I-2), Formula (I-3), Formula (I-4), Formula (I-5-1), Formula (I-5-2), Formula (I-5-3), Formula (I-5-4), Formula (I-6), Formula (I-7), Formula (I-8), Formula (I-9), Formula (I-10-1), Formula (I-10-2), Formula (I-11), Formula (I-12), Formula (I-13-1), Formula (I-13-2), Formula (I-14), Formula (I-15), Formula (I-16), Formula (I-17), Formula (I-18-1), Formula (I-18-2), Formula (I-19), Formula (I-20), Formula (I-21), Formula (I-22), Formula (I-23), Formula (I-24), Formula (I-25), Formula (I-26), Formula (I-27), Formula (I-28), Formula (I-29), Formula (I-30), Formula (II), Formula (II-A), Formula (II-B), Formula (II-C), Formula (II-D), Formula (II-1), Formula (II-2), Formula (II-3), Formula (II-4), Formula (II-5), Formula (II-6), Formula (II-7) or Formula (II-8)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to, hydrohalides (including hydrochlorides and hydrobromates), sulfate, citrate, maleate, mesylate, citrate, lactate, L-tartrate, fumarate, L-malate, phosphate, dihydrogen phosphate, pyrophosphate, metaphosphate, oxalate, malonate, benzoate, mandelate, succinate, trifluoroacetate, glycolate, or 4-methylbenzenesulfonate, etc. The compounds of the present disclosure can exist as non-solvated or solvated forms in pharmaceutically acceptable solvents such as water, ethanol, and the like. In some embodiments, the compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or ²H).

### III. Compositions/Formulations

**In** some embodiments, the present disclosure provides a pharmaceutical composition comprising as active ingredient the compound of the present disclosure (the compound of Formula (I), or the compound of Formula (II)) or a pharmaceutically acceptable salt, solvate, isotopically enriched analog, polymorph, prodrug, stereoisomer (including enantiomer), or mixture of stereoisomers thereof, and at least one pharmaceutically acceptable carrier.

**In** some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) of the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of the present disclosure (the compounds of Formula (I) or Formula (II)) or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of the present disclosure (the compounds of Formula (I) or Formula (II)) into conventional, dispersible, chewable, orally disintegrating or rapidly dissolving formulations, or sustained-release capsules or controlled-release capsules as needed.

The compounds of the present disclosure (the compounds of Formula (I) or Formula (II)), as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of the present disclosure (the compounds of Formula (I) or Formula (II)) or pharmaceutically acceptable salts thereof may be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

### IV. Kits/Packaged Products

The compounds of the present disclosure (the compound of Formula (I) or Formula (II)) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of diseases or disorders selected from the group consisting of: tumors, cancers, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases. In some embodiments, the diseases or disorders include, but are not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), monocytic leukemia, myelomonocytic leukemia, acute T-lymphocytic leukemia, T-lymphocytic leukemia; lymphoma, including diffuse large B-cell lymphoma, non-Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; auto inflammatory diseases, including gout; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The present disclosure provides a method for treating and/or preventing a disease or disorder in a subject, comprising administering to the subject a therapeutically effective amount of the compound of Formula (I) or a pharmaceutically acceptable salt thereof, or the pharmaceutical composition of the present disclosure comprising as an active ingredient the compounds of Formula (I) of the present disclosure or a pharmaceutically acceptable salt thereof, wherein the diseases or disorders include, but are not limited to, tumors, cancers, auto inflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases. In some embodiments, the diseases or disorders include, but are not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), monocytic leukemia, myelomonocytic leukemia, acute T-lymphocytic leukemia, T-lymphocytic leukemia; lymphoma, including diffuse large B-cell lymphoma, non-Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

In the method for preventing and/or treating a disease or disorder (including a tumor or cancer) in a subject, the compound of Formula (I) of the present disclosure or the pharmaceutical composition comprising as an active ingredient the compound of Formula (I) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The compound of Formula (II) of the present disclosure, or a pharmaceutically acceptable salt, solvate, isotopically enriched analog, polymorph, prodrug, stereoisomer (including enantiomer), or mixture of stereoisomers thereof, is a novel CRBN E3 ubiquitin ligase ligand, which can act as a molecular glue binding to the CRBN E3 ligase.

The compound of Formula (II) of the present disclosure, or a pharmaceutically acceptable salt, solvate, isotopically enriched analog, polymorph, prodrug, stereoisomer (including enantiomer), or mixture of stereoisomers thereof, can be used for the preparation of the compound of Formula (I) of the present disclosure. The compound of Formula (I) of the present disclosure can be used for treating and/or preventing a disease or disorder selected from the group consisting of: tumors or cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

The compound of Formula (II) of the present disclosure or a pharmaceutically acceptable salt, solvate, isotopically enriched analog, polymorph, prodrug, stereoisomer (including enantiomer), or mixture of stereoisomers thereof can be used as a medicament. Especially the compound of Formula (II) of the present disclosure or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a disease or disorder. In some embodiments, the disease or disorder comprises a disease or disorder associated with a cereblon protein. In some embodiments, the disease or disorder includes, but is not limited to: tumors or cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes. In some embodiments, the disease or disorder includes, but is not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), monocytic leukemia, myelomonocytic leukemia, acute T-lymphocytic leukemia, T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; auto inflammatory diseases, including gout; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The present disclosure provides a method for preventing and/or treating a disease or disorder in a subject, comprising administering to the subject a therapeutically effective amount of the compounds of Formula (II) of the present disclosure or a pharmaceutically acceptable salt thereof, or the pharmaceutical composition of the present disclosure comprising as an active ingredient the compounds of Formula (II) of the present disclosure or a pharmaceutically acceptable salt thereof. In some embodiments, the disease or disorder comprises a disease or disorder associated with a cereblon protein. In some embodiments, the disease or disorder includes, but is not limited to: tumors or cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes. In some embodiments, the disease or disorder includes, but is not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), monocytic leukemia, myelomonocytic leukemia, acute T-lymphocytic leukemia, T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

In the method for preventing and/or treating diseases or disorders associated with cereblon protein, the compound of Formula (II) of the present disclosure or the pharmaceutical composition comprising as an active ingredient the compound of Formula (II) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The term "treatment" or "treating" refers to administering to a subject the compound of the present disclosure (the compounds of Formula (I) or Formula (II)), or a pharmaceutically acceptable salt thereof, or the pharmaceutical composition comprising, as an active ingredient, the compound of the present disclosure (the compounds of Formula (I) or Formula (II)) or a pharmaceutically acceptable salt thereof, to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

### VI. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "R_{w} represents a bond" means that R_{w} is a bond linker. In other words, when R_{w} represents a bond, LIN is directly bonded to the benzene ring of the benzannulated ring system in Formula (ULM). For example, the wording "R_{w2} represents a bond" means that R_{w2} is a bond linker. In other words, when R_{w2} represents a bond, the PBM in the compound of Formula (I) is directly linked to the ring A⁵.

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally represents the replacement of one or more hydrogen atoms in the referenced structure by identical or different specific substituents. The number of substituents is not theoretically limited in any way, or is automatically limited by the size of the building units (i.e., the total number of replaceable hydrogen atoms in the building units), or as clearly defined herein.

As used herein, the term "replaced" in the expression "any one or more methylene groups in the main carbon chain skeleton of a linear or branched C₁₋₂₀ alkylene are optionally be replaced by one or more groups selected from: Rₐ, R_{b}, and any combination thereof" used alone or in combination, has a definition known in the art, namely it may refer to one or more groups Rₐ, R_{b}, or any combination of groups Rₐ and R_{b} optionally replacing any one or more methylene groups in the main carbon chain skeleton of a C₁₋₂₀ alkylene. Herein, examples of the above-mentioned expression may include, but are not limited to, that any one or more (e.g., 1-20 or 1-15, 1-10, 1-9, 1-8, 1-7, 1-6, 1-5, 1-4, 1-3, or 1-2, or 1) methylene groups in the main carbon chain skeleton of a C₁₋₂₀ alkylene optionally are replaced by one or more (e.g., 1-20 or 1-15, 1-10, 1-9, 1-8, 1-7, 1-6, 1-5, 1-4, 1-3, or 1-2, or 1) groups Rₐ as defined herein, and/or one or more (e.g., 1-20 or 1-15, 1-10, 1-9, 1-8, 1-7, 1-6, 1-5, 1-4, 1-3, or 1-2, or 1) groups R_{b}, and/or one or more (e.g., 1-20 or 1-15, 1-10, 1-9, 1-8, 1-7, 1-6, 1-5, 1-4, 1-3, or 1-2, or 1) of any combination of groups Rₐ and R_{b}, and the resulting main chain skeleton group conforms to the covalent bond theory. When two or more methylene groups in the main carbon chain skeleton of the linear or branched C₁₋₂₀ alkylene are replaced by two or more groups Rₐ, the respective groups Rₐ are not directly connected to each other. When any one or more methylene groups in the main carbon chain skeleton of the linear or branched C₁₋₂₀ alkylene are replaced by one or more groups Rₐ, and/or one or more groups R_{b}, and/or one or more of any combination of groups Rₐ and R_{b}, the resulting main chain skeleton group may contain one or more (e.g., 1-20 or 1-15, 1-10, 1-9, 1-8, 1-7, 1-6, 1-5, 1-4, 1-3, or 1-2, or 1) of the following fragments, such as but not limited to: "-(CH₂)₀₋₅-Rₐ-(CH₂)₀₋₅-" fragment, "-Rₐ-(CH₂)₁₋₅-Rₐ-" fragment, "-(CH₂)₀₋₅-(R_{b})₀₋₅-(CH₂)₀₋₅-" fragment, "-(R_{b})₀₋₅-(CH₂)₀₋₅-(R_{b})₀₋₅-" fragment, "-(CH₂)₀₋₅-Rₐ-(R_{b})₀₋₅-(CH₂)₀₋₅-" fragment, "-Rₐ-(CH₂)₀₋₅-(R_{b})₀₋₅-(CH₂)₀₋₅-" fragment, "-(CH₂)₀₋₅-Rₐ-(CH₂)₀₋₅-(R_{b})₀₋₅-" fragment etc., wherein each Rₐ is the same or different, each R_{b} is the same or different, and as defined herein.

Herein, a bond interrupted by a wavy line shows the point of attachment of the depicted group to the rest of the molecule. For example, the group of Formula (ULM) depicted below 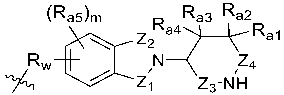 shows the point of attachment of R_{w} in said group to LIN of the compound of Formula (I). Herein, when a depicted group has two bonds interrupted by a wavy line, for example, when R_{w} represents 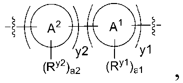 unless otherwise specified, it indicates that either end (e.g., ring A²) of said group may be connected to LIN, while the other end is connected to the isoindoline ring of Formula (ULM). Herein, when R_{c} represents the following group: 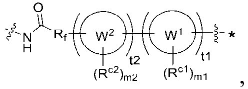 the symbol * indicates the point of attachment to LIN.

As used herein, the expression "one or more hydrogens of the C_{x-y} alkylene are replaced by...", used alone or in combination, means that any one or more hydrogens of the linear or branched C_{x-y} alkylene is replaced by a substituent(s) as defined herein. Herein, the term "one or more" may refer to part or all hydrogen atoms of each referenced alkylene group, including but not limited to 1-40 hydrogens, e.g., 1-30, such as 1-25, 1-20, 1-15, 1-10, 1-5, 1-4, 1-3, 1-2 or 1 hydrogen atoms. The number of hydrogens to be replaced is in principle not limited in any way, or is automatically limited by the size of the building unit.

As used herein, the term "deuterated" used alone or in combination, indicates that one or more hydrogen atoms in the referenced group are replaced by deuterium atoms.

As used herein, the term "oxo" or "oxo group" used alone or in combination, refers to =O.

As used herein, the term "C(O)" or "C(=O)" used alone or in combination, refers to a carbonyl group.

As used herein, the term "C(S)" or "C(=S)" used alone or in combination, refers to a thiocarbonyl group.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. Examples of the C₁₋₁₀ alkyl of the present disclosure may include a C₁₋₉ alkyl, C₁₋₈ alkyl, C₂₋₈ alkyl, C₁₋₇ alkyl, C₁₋₈ alkyl, C₁₋₅ alkyl, C₁₋₄ alkyl, and C₁₋₃ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "C₁₋₃ alkyl" or "C₁-C₃ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted with one or more substituents optionally selected from the group consisting of halogen, hydroxyl, cyano, C₁₋₃ alkyl, C₁₋₃ alkoxy, trifluoromethyl, 4- to 15-membered heterocyclyl, or a combination thereof.

As used herein, the term "halogenated alkyl", used alone or in combination, refers to a linear or branched alkyl group substituted with one or more halogens, wherein one or more hydrogen atom(s) of the alkyl group are replaced with one or more halogens. The term "halogenated Cₓ₋C_{y} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present disclosure refers to a linear or branched alkyl group containing from 1 to 10 carbon atoms substituted with one or more halogens. Examples of the halogenated C₁₋₁₀ alkyl group of the present disclosure include halogenated C₁₋₉ alkyl group, e.g., halogenated C₁₋₈ alkyl group, halogenated C₂₋₈ alkyl group, halogenated C₁₋₇ alkyl group, halogenated C₁₋₆ alkyl, halogenated C₁₋₅ alkyl, or halogenated C₁₋₄ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halogenated C₁₋₃ alkyl" or "halogenated C₁₋C₃ alkyl" of the present disclosure refers to an alkyl group containing from 1 to 3 carbon atoms substituted with one or more halogens, and its representative examples include halomethyl, haloethyl, halo-n-propyl and haloisopropyl.

As used herein, the term "deuterated alkyl", used alone or in combination, refers to a linear or branched alkyl group substituted with one or more deuterium atoms, wherein one or more hydrogen atoms of the alkyl group are replaced with one or more deuterium atoms. The term "deuterated Cₓ₋C_{y} alkyl" or "deuterated C_{x-y} alkyl" (x and y are each an integer) refers to a linear or branched alkyl containing from x to y carbon atoms substituted with one or more deuterium atoms. The term "deuterated C₁₋₁₀ alkyl" used alone or in combination in the present disclosure refers to a linear or branched alkyl group containing from 1 to 10 carbon atoms substituted with one or more deuterium atoms. Examples of the deuterated C₁₋₁₀ alkyl group of the present disclosure include deuterated C₁₋₉ alkyl group, e.g., deuterated C₁₋₈ alkyl group, deuterated C₂₋₈ alkyl group, deuterated C₁₋₇ alkyl group, deuterated C₁₋₆ alkyl, deuterated C₁₋₅ alkyl, or deuterated C₁₋₄ alkyl. Representative examples include perdeuterated methyl (CD₃), perdeuterated ethyl (CD₃CD₂), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, perdeuterated hexyl, perdeuterated heptyl, perdeuterated octyl, perdeuterated nonyl, and perdeuterated decyl. The term "deuterated C₁₋₃ alkyl" or "deuterated C₁₋C₃ alkyl" of the present disclosure refers to an alkyl group containing from 1 to 3 carbon atoms substituted with one or more deuterium atoms, and its representative examples include perdeuterated methyl (CD₃) and perdeuterated ethyl (CD₃CD₂).

As used herein, the term "alkylene" (which is used interchangeably with "alkylene chain"), used alone or in combination, refers to a linear or branched divalent saturated hydrocarbon group composed of carbon and hydrogen atoms. The term "Cₓ-C_{y} alkylene" or "C_{x-y} alkylene" (x and y each being an integer) refers to a linear or branched alkylene group containing from x to y carbon atoms. Examples of the C₁-C₂₀ alkylene in the present disclosure may include, but are not limited to C₁-C₂₀ alkylene, C₁-C₁₅ alkylene, C₁-C₁₄ alkylene, C₁-C₁₃ alkylene, C₁-C₁₂ alkylene, C₁-C₁₁ alkylene, C₁-C₁₀ alkylene, C₁-C₉ alkylene, C₁-C₈ alkylene, C₁-C₇ alkylene, C₁-C₆ alkylene, C₁-C₅ alkylene, C₁-C₄ alkylene, C₁-C₃ alkylene, or C₁-C₂ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, and pentadecylene. In the present disclosure, the "alkylene" is optionally substituted with one or more substituents optionally selected from C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "alkoxy", used alone or in combination, refers to a linear or branched alkoxy group having structural formula of alkyl-O-. Optionally, the alkyl portion of the alkoxy group may contain 1-10 (e.g., 1-6, 1-4, or 1-3) carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "C₁-C₃ alkoxy" or "C₁₋₃ alkoxy" refers to a linear or branched alkoxy group containing from 1 to 3 carbon atoms. Representative examples of C₁₋₃ alkoxy include, but are not limited to, methoxy, ethoxy, n-propoxy, and isopropoxy.

As used herein, the term "halogenated alkoxy", used alone or in combination, refers to an alkoxy group substituted with one or more halogen atoms. Optionally, the alkyl portion of the alkoxy group may contain 1-10 (e.g., 1-6, 1-4, or 1-3) carbon atoms. Examples of "halogenated alkoxy" include, but are not limited to, halogenated C₁₋₆ alkoxy or halogenated C₁₋₄ alkoxy. Representative examples include, but are not limited to, F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O- or CH₂ClCH₂-O-.

As used herein, the term "alkylamino", used alone or in combination, refers to a linear or branched alkylamino group having structural formula of alkyl-NH-. Optionally, the alkyl portion of the alkylamino group may contain 1-10 (e.g., 1-6, 1-4, or 1-3) carbon atoms. Representative examples of "alkylamino" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "C₁-C₃ alkyl-NH-" or "C₁₋₃ alkyl-NH-" refers to a linear or branched alkyl-NH- group containing from 1 to 3 carbon atoms. Representative examples of C₁₋₃ alkyl-NH- include, but are not limited to, methyl-NH-, ethyl-NH-, n-propyl-NH-, and isopropyl-NH-.

As used herein, the term "amino-substituted alkylene", used alone or in combination, refers to a linear or branched alkylene group substituted with amino having structural formula of NH₂-alkylene. Optionally, the alkylene portion of the amino-substituted alkylene group may contain 1-10 carbon atoms. The term "amino-substituted C₁₋₃ alkylene" or " NH₂-C₁₋₃ alkyl" refers to a linear or branched alkylene group containing from 1 to 3 carbon atoms which is substituted with amino. Representative examples of amino-substituted C₁₋₃ alkylene include, but are not limited to, NH₂-CH₂-, NH₂-CH₂CH₂-, and NH₂-CH₂CH₂CH₂-.

As used herein, the term "alkyl-NHC(O)-", used alone or in combination, refers to a linear or branched alkyl-NHC(O)- group having structural formula of alkyl-NHC(O)-. Optionally, the alkyl portion of the alkyl-NHC(O)- group may contain 1-10 carbon atoms. The term "C₁₋₃ alkyl-NHC(O)-" or "C₁-C₃ alkyl-NHC(O)-" refers to a linear or branched alkyl-NHC(O)- group containing from 1 to 3 carbon atoms. Representative examples of C₁₋₃ alkyl-NHC(O)- include, but are not limited to, CH₃-NHC(O)-, CH₃CH₂-NHC(O)-, and CH₃CH₂CH₂-NHC(O)-.

As used herein, the term "alkyl-C(O)NH-", used alone or in combination, refers to a linear or branched alkyl-C(O)NH- group having structural formula of alkyl-C(O)NH-. Optionally, the alkyl portion of the alkyl-C(O)NH- group may contain 1-10 carbon atoms. The term "C₁₋₃ alkyl-C(O)NH-" or "C₁-C₃ alkyl-C(O)NH-" refers to a linear or branched alkyl-C(O)NH- group containing from 1 to 3 carbon atoms. Representative examples of C₁₋₃ alkyl-C(O)NH- include, but are not limited to, CH₃-C(O)NH-, CH₃CH₂-C(O)NH-, and CH₃CH₂CH₂-C(O)NH-.

As used herein, the term "heteroaryl", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, 6- to 9-membered, 6- to 20-membered, or 6- to 15-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, chromanyl, and 6,7-dihydrothieno[3,2-d]pyrimidinyl. Examples of tricyclic or polycyclic heteroaryl groups include, but are not limited to, acridinyl, benzindolyl, carbazolyl, dibenzofuranyl, xanthyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of C₁-C₃ alkyl, C₃₋₆cycloalkyl, hydroxy, amino, mercapto, halogen, C₁-C₃ alkoxy, C₁₋₃ alkylamino, halogenated C₁-C₃ alkyl, amino-substituted C₁₋₃ alkylene, C₁₋₃ alkyl-NHC(O)-, C₁₋₃ alkyl-C(O)NH-, cyano, and any combination thereof.

As used herein, the term "heteroarylene", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, 6- to 9-membered, or 6- to 20-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, chromanylene, and 6,7-dihydrothieno[3,2-d]pyrimidinylene. Examples of tricyclic or polycyclic heteroarylene groups include, but are not limited to, acridinylene, benzindolylene, carbazolylene, dibenzofuranylene, xanthylene, 5,6,7,8-tetrahydrobenzo[4,5]thieno[2,3-d]pyrimidinylene, and 6,7-dihydro-5H-cyclopenta[4,5]thieno[2,3-d]pyrimidinylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of C₁-C₃ alkyl, C₃₋₆cycloalkyl, hydroxy, amino, mercapto, halogen, C₁-C₃ alkoxy, C₁-C₃ alkylamino, halogenated C₁-C₃ alkyl, amino-substituted C₁₋₃alkylene, C₁₋₃ alkyl-NHC(O)-, C₁₋₃ alkyl-C(O)NH-, cyano, and any combination thereof.

As used herein, the term "aryl", used alone or in combination, refers to a monovalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, or from 6 to 20) carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, tri-, or poly-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "arylene", used alone or in combination, refers to a divalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, or from 6 to 20) carbon atoms and optionally one or more fused rings, such as phenylene, naphthylene, or fluorenylene. As used herein, the "arylene" is optionally substituted. A substituted arylene refers to an arylene group substituted one or more times (e.g., 1-4, 1-3, or 1-2 times) with a substituent(s). For example, arylene is mono-, di-, tri-, or poly-substituted with a substituent(s) optionally selected from e.g., C₁₋₃ alkyl, C₃₋₆ cycloalkyl, hydroxyl, amino, mercapto, halogen, C₁₋₃ alkoxy, C₁₋₃ alkylamino, halogenated C₁₋₃ alkyl, amino-substituted C₁₋₃ alkylene, C₁₋₃ alkyl-NHC(O)-, C₁₋₃ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated π-electron system) monocyclic or bicyclic or tricyclic or polycyclic cyclic hydrocarbon radical, which in some embodiments contains from 3 to 30 carbon atoms (i.e., C₃₋₃₀ cycloalkyl), or from 3 to 25 carbon atoms (i.e., C₃₋₂₅ cycloalkyl), or from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms ( i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl), or from 4 to 15 carbon atoms ( i.e., C₄₋₁₅ cycloalkyl), or from 4 to 12 carbon atoms (i.e., C₄₋₁₂ cycloalkyl), or from 4 to 10 carbon atoms (i.e., C₄₋₁₀ cycloalkyl). The term "cycloalkyl" includes monocyclic, bicyclic, tricyclic, and polycyclic cyclic hydrocarbon radical having from 3 to 30 carbon atoms. Examples of the term "cycloalkyl" include, but are not limited to, monocyclic cycloalkyl, bridged cycloalkyl (e.g., C₅₋₃₀ bridged cycloalkyl or C₅₋₂₀ bridged cycloalkyl or C₅₋₁₅ bridged cycloalkyl or C₇₋₁₅ bridged cycloalkyl), fused cycloalkyl (e.g., C₅₋₃₀ fused cycloalkyl, C₅₋₂₀ fused cycloalkyl, C₅₋₁₅ fused cycloalkyl, C₆₋₃₀ fused cycloalkyl, C₆₋₂₀ fused cycloalkyl, C₆₋₁₅ fused cycloalkyl, C₇₋₃₀ fused cycloalkyl, C₇₋₂₀ fused cycloalkyl, C₇₋₁₅ fused cycloalkyl, and C₈₋₁₅ fused cycloalkyl), and spiro-cycloalkyl (e.g., C₅₋₃₀ spiro-cycloalkyl, C₅₋₂₀ spiro-cycloalkyl, C₅₋₁₅ spiro-cycloalkyl, C₆₋₃₀ spiro-cycloalkyl, C₆₋₂₀ spiro-cycloalkyl, C₆₋₁₅ spiro-cycloalkyl, C₇₋₃₀ spiro-cycloalkyl, C₇₋₂₀ spiro-cycloalkyl, C₇₋₁₅ spiro-cycloalkyl, and C₈₋₁₅ spiro-cycloalkyl). Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Examples of bridged cycloalkyl, fused cycloalkyl and spiro-cycloalkyl groups include, but are not limited to, decalinyl, octahydropentalenyl, octahydro-1H-indenyl, C₅₋₂₀ spiro-cycloalkyl, C₅₋₁₅ spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from C₁-C₃ alkyl, C₃₋₆cycloalkyl, hydroxy, amino, mercapto, halogen, C₁-C₃ alkoxy, C₁-C₃ alkylamino, halogenated C₁-C₃ alkyl, amino-substituted C₁₋₃alkylene, C₁₋₃ alkyl-NHC(O)-, C₁₋₃ alkyl-C(O)NH-, cyano, and any combination thereof. Examples of "C₃₋₆ cycloalkyl" include, but are not limited to, cyclopropyl, cyclobutyl, cyclopentyl,, cyclopentenyl, and cyclohexyl.

As used herein, the term "C_{x-y} spiro-cycloalkyl" (x and y each being an integer), used alone or in combination, refers to a spiro-cycloalkyl group containing from x to y carbon atoms. As used herein, the term "C₅₋₃₀ spiro-cycloalkyl", used alone or in combination, refers to a spiro-cycloalkyl group containing from 5 to 30 carbon atoms (e.g., but not limited to, 5-20, 5-15, 7-20, 7-15, 5-11, 5-10, and 7-9 carbon atoms). The term "C₅₋₃₀ spirocycloalkyl" includes "C₅₋₂₀ spirocycloalkyl", "C₅₋₁₅ spirocycloalkyl", and "C₇₋₁₅ spirocycloalkyl", and representative examples thereof include, but are not limited to, spiro[3.3]heptanyl, spiro[2.5]octanyl, spiro[3.5]nonanyl, spiro[4.4]nonanyl, spiro[4.5]decanyl, and spiro[5.5]undecanyl. The "C₅₋₃₀ spiro-cycloalkyl" is optionally further substituted with one or more substituents selected from the group consisting of C₁₋₃ alkyl, halogenated C₁₋₃ alkyl, C₁₋₃ alkoxy, deuterated C₁₋₃ alkoxy, C₁₋₃ alkylamino, amino, oxo, halogen, hydroxy, cyano, and any combination thereof.

As used herein, the term "C_{x-y} bridged cycloalkyl" (where x and y are integers) used alone or in combination, refers to a bridged cycloalkyl group containing x to y carbon atoms. In the present invention, the term "C₅₋₃₀ bridged cycloalkyl" used alone or in combination, refers to a bridged cycloalkyl group containing from 5 to 30 carbon atoms (e.g., but not limited to, 5-20, 6-20, 5-15, 7-20, 7-15, 5-11, 5-10, and 7-9 carbon atoms). The term "C₅₋₃₀ bridged cycloalkyl" includes "C₅₋₂₀ bridged cycloalkyl", "C₆₋₂₀ bridged cycloalkyl", "C₇₋₂₀ bridged cycloalkyl", "C₅₋₁₅ bridged cycloalkyl", and "C₇₋₁₅ bridged cycloalkyl", and representative examples thereof include, but are not limited to, adamantanyl, noradamantanyl, norbornanyl (systematically named bicyclo[2.2.1]heptanyl), 2-oxobicyclo[2.2.1]heptanyl, bicyclo[2.2.1]heptenyl, and cubanyl. Said " C₅₋₃₀ bridged cycloalkyl" may be optionally substituted with 1 to 10 substituents selected from: C₁₋₃ alkyl, deuterated C₁₋₃ alkyl, C₁₋₃ alkoxy, halogen, halogenated C₁₋₃ alkyl, C₁₋₃ alkylamino, amino, hydroxy, cyano, oxo, and any combination thereof.

As used herein, the term "cycloalkylene", used alone or in combination, refers to a saturated or partially unsaturated (i.e., containing one or more double bonds, but not having a fully conjugated π-electron system) monocyclic, bicyclic, tricyclic or polycyclic divalent cyclic hydrocarbon radical, which in some embodiments contains from 3 to 30 carbon atoms (i.e., C₃₋₃₀ cycloalkylene), or from 3 to 25 carbon atoms (i.e., C₃₋₂₅ cycloalkylene), from 3 to 20 carbon atoms (i.e., C₃₋₂₀ cycloalkylene), or from 3 to 15 carbon atoms (i.e., C₃₋₁₅ cycloalkylene), or from 3 to 12 carbon atoms (i.e., C₃₋₁₂ cycloalkylene), or from 3 to 11 carbon atoms (i.e., C₃₋₁₁ cycloalkylene), or from 3 to 10 carbon atoms (i.e., C₃₋₁₀ cycloalkylene), or from 3 to 8 carbon atoms ( i.e., C₃₋₈ cycloalkylene), or from 3 to 7 carbon atoms (i.e., C₃₋₇ cycloalkylene), or from 3 to 6 carbon atoms (i.e., C₃₋₆ cycloalkylene), or from 4 to 20 carbon atoms (i.e., C₄₋₂₀ cycloalkylene), or from 4 to 15 carbon atoms ( i.e., C₄₋₁₅ cycloalkylene), or from 4 to 12 carbon atoms (i.e., C₄₋₁₂ cycloalkylene), or from 4 to 10 carbon atoms (i.e., C₄₋₁₀ cycloalkylene). The term "cycloalkylene" includes monocyclic, bicyclic, tricyclic and polycyclic divalent cyclic hydrocarbon radical having from 3 to 30 carbon atoms. Examples of the term "cycloalkylene" include, but are not limited to, monocyclic cycloalkylene, bridged cycloalkylene (e.g., C₅₋₃₀ bridged cycloalkylene, C₅₋₂₀ bridged cycloalkylene, C₅₋₁₅ bridged cycloalkylene or C₇₋₁₅ bridged cycloalkylene), fused cycloalkylene (e.g., C₅₋₃₀ fused cycloalkylene, C₅₋₂₀ fused cycloalkylene, C₅₋₁₅ fused cycloalkylene, C₆₋₃₀ fused cycloalkylene, C₆₋₂₀ fused cycloalkylene, C₆₋₁₅ fused cycloalkylene, C₇₋₃₀ fused cycloalkylene, C₇₋₂₀ fused cycloalkylene, C₇₋₁₅ fused cycloalkylene, and C₈₋₁₅ fused cycloalkylene), and spiro-cycloalkylene (e.g., C₅₋₃₀ spiro-cycloalkylene, C₅₋₂₀ spiro-cycloalkylene, C₅₋₁₅ spiro-cycloalkylene, C₆₋₃₀ spiro-cycloalkylene, C₆₋₂₀ spiro-cycloalkylene, C₆₋₁₅ spiro-cycloalkylene, C₇₋₃₀ spiro-cycloalkylene, C₇₋₂₀ spiro-cycloalkylene, C₇₋₁₅ spiro-cycloalkylene, and C₈₋₁₅ spiro-cycloalkylene). Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Examples of bridged cycloalkylene, fused cycloalkylene and spiro-cycloalkylene groups include, but are not limited to, decalinylene, octahydropentalenylene, octahydro-1H-indenylene, 2,3-dihydro-1H-indenylene, C₅₋₂₀ spiro-cycloalkylene (e.g., C₅₋₁₅ spiro-cycloalkylene), adamantanylene, noradamantanylene, and norbornylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more (e.g., 1-5, 1-4, 1-3, 1-2, or 1) substituents selected from the group consisting of C₁-C₃ alkyl, C₃₋₆cycloalkyl, hydroxy, amino, mercapto, halogen, C₁-C₃ alkoxy, C₁-C₃ alkylamino, halogenated C₁-C₃ alkyl, amino-substituted C₁₋₃alkylene, C₁₋₃ alkyl-NHC(O)-, C₁₋₃ alkyl-C(O)NH-, cyano, and any combination thereof.

As used herein, the term "C_{x-y} spiro-cycloalkylene" (x and y each being an integer), used alone or in combination, refers to a spiro-cycloalkylene group containing from x to y carbon atoms. As used herein, the term "C₅₋₃₀ spiro-cycloalkylene", used alone or in combination, refers to a spiro-cycloalkylene group containing from 5 to 30 carbon atoms (e.g., 5-20, 5-15, 7-20, 7-15, 5-11, 5-10, or 7-9 carbon atoms). The term "C₅₋₃₀ spiro-cycloalkylene" includes "C₅₋₂₀ spiro-cycloalkylene", "C₅₋₁₅ spiro-cycloalkylene", "C₇₋₁₅ spiro-cycloalkylene", and "C₇₋₂₀ spiro-cycloalkylene", and representative examples thereof include, but are not limited to, spiro[3.3]heptylene, spiro[2.5]octylene, spiro[3.5]nonylene, spiro[4.4]nonylene, spiro[4.5]decylene, and spiro[5.5]undecylene. The "C₅₋₃₀ spiro-cycloalkylene" is optionally further substituted with one or more substituents selected from the group consisting of C₁-C₃ alkyl, C₃₋₆cycloalkyl, hydroxy, amino, mercapto, halogen, C₁-C₃ alkoxy, C₁-C₃ alkylamino, halogenated C₁-C₃ alkyl, amino-substituted C₁₋₃alkylene, C₁₋₃ alkyl-NHC(O)-, C₁₋₃ alkyl-C(O)NH-, cyano, and any combination thereof.

As used herein, the term "C_{x-y} bridged cycloalkylene" (where x and y are integers) used alone or in combination, refers to a bridged cycloalkylene group containing x to y carbon atoms. In the present invention, the term "C₅₋₃₀ bridged cycloalkylene" used alone or in combination, refers to a bridged cycloalkylene group containing 5 to 30 (e.g., 5-20, 6-20, 7-20, 5-15, 7-15, 5-11, 5-10, and 7-9) carbon atoms. The term "C₅₋₃₀ bridged cycloalkylene" includes "C₅₋₂₀ bridged cycloalkylene", "C₆₋₂₀ bridged cycloalkylene", "C₇₋₂₀ bridged cycloalkylene", "C₅₋₁₅ bridged cycloalkylene", and "C₇₋₁₅ bridged cycloalkylene", and representative examples thereof include, but are not limited to, adamantanylene, noradamantanylene, norbornanylene, bicyclo[2.2.1]heptanylene, 2-oxobicyclo[2.2.1]heptanylene, bicyclo[2.2.1]heptenylene, and cubanylene. Said "C₅₋₁₅ bridged cycloalkylene" may be optionally substituted with one or more substituents selected from: C₁-C₃ alkyl, C₃₋₆cycloalkyl, hydroxy, amino, mercapto, halogen, C₁-C₃ alkoxy, C₁-C₃ alkylamino, halogenated C₁-C₃ alkyl, amino-substituted C₁₋₃alkylene, C₁₋₃ alkyl-NHC(O)-, C₁₋₃ alkyl-C(O)NH-, cyano, and any combination thereof.

As used herein, the term "heterocyclyl" or "heterocyclic group", used alone or in combination, refers to a 4- to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated π-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl group include, but not limited to, monocyclic heterocyclyl (e.g., 4- to 30-membered monocyclic heterocyclyl, and 4- to 20-membered monocyclic heterocyclyl), bridged heterocyclyl (e.g., 5- to 30-membered bridged heterocyclyl, 5- to 20-membered bridged heterocyclyl, 7- to 20-membered bridged heterocyclyl, and 7- to 15-membered bridged heterocyclyl), fused heterocyclyl (e.g., 5- to 30-membered fused heterocyclyl, and 5- to 20-membered fused heterocyclyl), and spiro-heterocyclyl groups (e.g., 5- to 30-membered spiro-heterocyclyl, and 5- to 20-membered spiro-heterocyclyl). Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl) and diazacyclooctyl. Examples of bridged heterocyclyl, fused heterocyclyl and spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, octahydro-1H-indolyl, and azaspirocycloalkyl (e.g., 5- to 20-membered azaspirocycloalkyl, such as 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆cycloalkyl, optionally deuterated C₁₋₆ alkoxy, optionally deuterated C₁₋₆ alkyl-NH-, NH₂-C₁₋₆alkylene, optionally deuterated C₁₋₆ alkyl-NHC(O)-, optionally deuterated C₁₋₆ alkyl-C(O)NH-, and any combination thereof.

As used herein, the term "nitrogen-containing heterocyclyl", used alone or in combination, refers to 4- to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated π-electron system) monocyclic, bicyclic, tricyclic or polycyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclyl group include, but not limited to, nitrogen-containing monocyclic heterocyclyl (e.g., 4- to 30-membered nitrogen-containing monocyclic heterocyclyl, and 4- to 20-membered nitrogen-containing monocyclic heterocyclyl), nitrogen-containing bridged heterocyclyl (e.g., 5- to 30-membered nitrogen-containing bridged heterocyclyl, 5- to 20-membered nitrogen-containing bridged heterocyclyl, 5-to 15-membered nitrogen-containing bridged heterocyclyl, 7- to 20-membered nitrogen-containing bridged heterocyclyl, and 7- to 15-membered nitrogen-containing bridged heterocyclyl), nitrogen-containing fused heterocyclyl (e.g., 5- to 30-membered nitrogen-containing fused heterocyclyl, and 5- to 20-membered nitrogen-containing fused heterocyclyl), and nitrogen-containing spiro-heterocyclyl groups (e.g., 5- to 30-membered nitrogen-containing spiro-heterocyclyl, and 5- to 20-membered nitrogen-containing spiro-heterocyclyl). Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. Examples of nitrogen-containing bridged heterocyclyl, nitrogen-containing fused heterocyclyl and nitrogen-containing spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, octahydro-1H-indolyl, and azaspirocycloalkyl (e.g., 5- to 20-membered azaspirocycloalkyl, such as 3-azaspiro[5.5]undecan-3-yl). The nitrogen-containing heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆cycloalkyl, optionally deuterated C₁₋₆ alkoxy, optionally deuterated C₁₋₆ alkyl-NH-, NH₂-C₁₋₆alkylene, optionally deuterated C₁₋₆ alkyl-NHC(O)-, optionally deuterated C₁₋₆ alkyl-C(O)NH-, and any combination thereof.

As used herein, the term "nitrogen-containing bridged heterocyclyl", used alone or in combination, refers to 5- to 30-membered (e.g., 5- to 20-membered, 5- to 15-membered, 5- to 14-membered, 6- to 12-membered, 6- to 11-membered, 6- to 10-membered, 5- to 9-membered, 5- to 8-membered, 5- to 7-membered, 5- to 6-membered, or 6- to 9-membered) saturated or partially unsaturated (i.e., containing one or more double bonds, but not having a fully conjugated π-electron system) bicyclic, tricyclic or polycyclic monovalent bridged cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of nitrogen-containing bridged heterocyclyl include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, and 2,5-diazabicyclo[2.2.2]octan-2-yl. The nitrogen-containing bridged heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆cycloalkyl, optionally deuterated C₁₋₆ alkoxy, optionally deuterated C₁₋₆ alkyl-NH-, NH₂-C₁₋₆alkylene, optionally deuterated C₁₋₆ alkyl-NHC(O)-, optionally deuterated C₁₋₆ alkyl-C(O)NH-, and any combination thereof.

As used herein, the term "-O-optionally substituted heterocyclyl", used alone or in combination, refers to a group formed by optionally substituted heterocyclyl connected to oxygen. The definition of "heterocyclyl" may be as defined above. Examples of the term "heterocyclyl" include, but are not limited to, monocyclic heterocyclyl (e.g., 4- to 20-membered monocyclic heterocyclyl), bridged heterocyclyl (e.g., 5- to 20-membered bridged heterocyclyl or 7- to 15-membered bridged heterocyclyl), fused heterocyclyl, and spiro heterocyclyl (e.g., 5- to 20-membered spiro heterocyclyl). Optionally, the heterocyclyl in the term "-O-optionally substituted heterocyclyl" is e.g., a 4- to 20-membered mono-, bi-, tri-, or polycyclic heterocyclyl containing one or more (e.g., 1-4, or 1, 2, 3, or 4) heteroatoms selected from nitrogen, oxygen, and sulfur. Examples of the "heterocyclyl" include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl). As used herein, the heterocyclyl is optionally substituted with a substituent selected from deuterium, hydroxy, amino, mercapto, nitro, halogen, cyano, optionally deuterated C₁₋₆ alkyl, halogenated C₁₋₆ alkyl, optionally deuterated C₃₋₆cycloalkyl, optionally deuterated C₁₋₆ alkoxy, optionally deuterated C₁₋₆ alkyl-NH-, NH₂-C₁₋₆alkylene, optionally deuterated C₁₋₆ alkyl-NHC(O)-, optionally deuterated C₁₋₆ alkyl-C(O)NH-, and any combination thereof.

As used herein, the term "heterocyclylene", used alone or in combination, refers to a 4- to 30-membered saturated or partially unsaturated (i.e., containing one or more double bonds, but not having a fully conjugated π-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 4- to 30-membered (optionally 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated π-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but are not limited to, monocyclic heterocyclylene (e.g., 4- to 30-membered monocyclic heterocyclylene, and 4- to 20-membered monocyclic heterocyclylene), bridged heterocyclylene (e.g., 5- to 30-membered bridged heterocyclylene, 5- to 20-membered bridged heterocyclylene, 7- to 20-membered bridged heterocyclylene, and 7- to 15-membered bridged heterocyclylene), fused heterocyclylene (e.g., 5- to 30-membered fused heterocyclylene, and 5- to 20-membered fused heterocyclylene) and spiro-heterocyclylene groups (e.g., 5- to 30-membered spiro-heterocyclylene, and 5- to 20-membered spiro-heterocyclylene). Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene (e.g., 1,4-diazacycloheptanylene, 4,5-diazacycloheptanylene, 1,3-diazacycloheptanylene), and diazacyclooctyl. Examples of the bridged heterocyclylene, fused heterocyclylene and spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, octahydro-1H-indolylene, and azaspirocycloalkylene (e.g., 5- to 20-membered azaspirocycloalkylene, such as 3-azaspiro[5.5]undecanylene), 5,6,7,8-tetrahydrobenzo[4,5]thieno[2,3-d]pyrimidinylene, 6,7-dihydro-5H-cyclopenta[4,5]thieno[2,3-d]pyrimidinylene, chromanylene, and 6,7-dihydrothieno[3,2-d]pyrimidinylene. The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆cycloalkyl, optionally deuterated C₁₋₆ alkoxy, optionally deuterated C₁₋₆ alkyl-NH-, NHz-C₁₋₆alkylene, optionally deuterated C₁₋₆ alkyl-NHC(O)-, optionally deuterated C₁₋₆ alkyl-C(O)NH-, and any combination thereof.

As used herein, the term " nitrogen-containing heterocyclylene", used alone or in combination, refers to a 4- to 30-membered (e.g., 4- to 30-membered, 4- to 25-membered, 4- to 20-membered, 4- to 15-membered, 4- to 14-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated π-electron system) monocyclic, bicyclic, tricyclic or polycyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclylene group include, but not limited to, nitrogen-containing monocyclic heterocyclylene (e.g., 4- to 30-membered nitrogen-containing monocyclic heterocyclylene, and 4- to 20-membered nitrogen-containing monocyclic heterocyclylene), nitrogen-containing bridged heterocyclylene (e.g., 5- to 30-membered nitrogen-containing bridged heterocyclylene, 5- to 20-membered nitrogen-containing bridged heterocyclylene, 7- to 20-membered nitrogen-containing bridged heterocyclylene, and 7- to 15-membered nitrogen-containing bridged heterocyclylene), nitrogen-containing fused heterocyclylene (e.g., 5- to 30-membered nitrogen-containing fused heterocyclylene, and 5- to 20-membered nitrogen-containing fused heterocyclylene), and nitrogen-containing spiro-heterocyclylene (e.g., 5- to 30-membered nitrogen-containing spiro-heterocyclylene, and 5- to 20-membered nitrogen-containing spiro-heterocyclylene). Representative examples of the nitrogen-containing monocyclic heterocyclylene include, but are not limited to, azetidinylene, pyrrolidinylene, imidazolidinylene, pyrazolidylene, oxazolidinylene, thiazolidinylene, piperidinylene, piperazinylene, morpholinylene, thiomorpholinylene, azacycloheptylene, azacyclooctylene, diazacycloheptanylene (e.g., 1,4-diazacycloheptanylene, 4,5-diazacycloheptanylene, 1,3-diazacycloheptanylene), and diazacyclooctylene. Examples of nitrogen-containing bridged heterocyclylene, nitrogen-containing fused heterocyclylene, and nitrogen-containing spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, octahydro-1H-indolylene, and azaspirocycloalkylene (e.g., 5- to 20-membered azaspirocycloalkylene, such as 3-azaspiro[5.5]undecanylene). The nitrogen-containing heterocyclylenemay be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆cycloalkyl, optionally deuterated C₁₋₆ alkoxy, optionally deuterated C₁₋₆ alkyl-NH-, NH₂-C₁₋₆alkylene, optionally deuterated C₁₋₆ alkyl-NHC(O)-, optionally deuterated C₁₋₆ alkyl-C(O)NH-, and any combination thereof.

As used herein, the term "nitrogen-containing bridged heterocyclylene", used alone or in combination, refers to 5- to 30-membered (e.g., 5- to 20-membered, 5- to 15-membered, 5- to 14-membered, 6- to 12-membered, 6- to 11-membered, 6- to 10-membered, 5- to 9-membered, 5- to 8-membered, 5- to 7-membered, 5- to 6-membered, or 6- to 9-membered) saturated or partially unsaturated (i.e., containing one or more double bonds, but not having a fully conjugated π-electron system) bicyclic, tricyclic or polycyclic bivalent bridged cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing bridged heterocyclylene include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, and 2,5-diazabicyclo[2.2.2]octanylene. The nitrogen-containing bridged heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated C₁₋₆ alkyl, halogenated C₁₋₆ alkyl, optionally deuterated C₃₋₆cycloalkyl, optionally deuterated C₁₋₆ alkoxy, optionally deuterated C₁₋₆ alkyl-NH-, NH₂-C₁₋₆alkylene, optionally deuterated C₁₋₆ alkyl-NHC(O)-, optionally deuterated C₁₋₆ alkyl-C(O)NH-, and any combination thereof.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include C₂₋₈ alkynylene, C₂₋₆ alkynylene, or C₂₋₄ alkynylene, with representative but non-limiting examples including ethynylene, 1-propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynyl include C₂₋₉ alkynyl, C₂₋₆ alkynyl or C₂₋₄ alkynyl, with representative but non-limiting examples including ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include C₂₋₈ alkenylene, C₂₋₆ alkenylene or C₂₋₄ alkenylene, with representative but non-limiting examples including vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of C₂₋₆ alkenyl group include C₂₋₈ alkenyl, C₂₋₆ alkenyl or C₂₋₄ alkenyl, with representative but non-limiting examples including, vinyl (e.g., CH₂=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

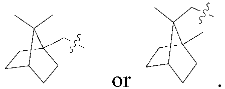

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.1^{3,7}]decane) has a definition known to those skilled in the art, and its structural formula is e.g., as follows: 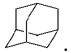 As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows: 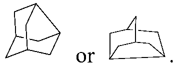 As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1^{3,7}]decan-1-amine; CAS No.: 768-94-5), with the following structural Formula:

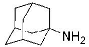

In the present disclosure, the term "leaving group" used alone or in combination is well known to those skilled in the art, which is a leaving molecular fragment (ion or neutral molecule) that carries a pair of electrons from a reactant in chemical reactions, as is a term used in nucleophilic substitution and elimination reactions. Common ionic leaving groups include Cl⁻, Br⁻, I⁻ and sulfonate (such as p-toluenesulfonate, TsO⁻), and neutral molecular leaving groups include water, ammonia and alcohol. In the present disclosure, those skilled in the art can select an appropriate leaving group as needed, such as but not limited to -N₃, halogen, methanesulfonyloxy (MsO-), trifluoromethanesulfonyloxy (TfO-) or p-toluenesulfonyloxy (TsO-), etc.

Salts or pharmaceutically acceptable salts, enantiomers, stereoisomers, solvates, polymorphs of the compounds of Formula (I) or Formula (II) of the present disclosure are also encompassed within the scope of the present invention.

In all embodiments of the present disclosure, the salts or pharmaceutically acceptable salts of the compounds of Formula (I) or Formula (II) refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, hydroxyacetates, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and the compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

### Description of the Drawings

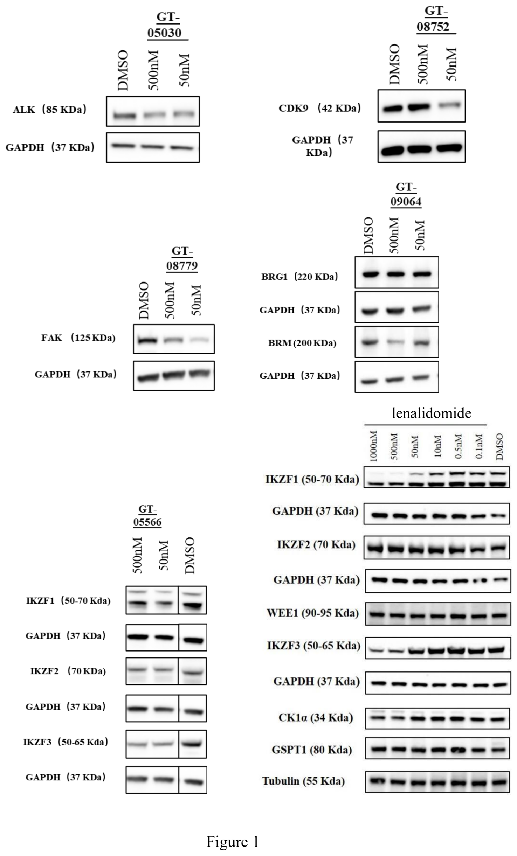

Fig. 1 shows the Western blot assay results of the degradation of target substrate proteins by the compounds of the present disclosure in cells.

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

| | | | |
|---|---|---|---|
| ACN | acetonitrile | MeCN | acetonitrile |
| AcOH | acetic acid | MeOH | Methanol |
| AcOK | potassium acetate | MS | mass spectrometry |
| AIBN | 2,2'-azobis(2-methylpropionitrile) | MsO- | Methanesulfonyloxy |
| Bn | Benzyl | MsCl | methanesulfonyl chloride |
| Boc | t-Butyloxy carbonyl | NaHMDS | Sodium bis(trimethylsilyl)amide |
| Brettphos Pd G3 | Methanesulfonato(2-dicyclohexylphosphino-3,6-dimethoxy-2',4',6'-tri-i-propyl-1,1'-biphenyl)(2'-amino-1,1'-biphenyl-2-yl)palladium(II) | NBS | N-Bromosuccinimide |
| Brettphos | Dicyclohexyl[3,6-dimethoxy-2',4',6'-tris(1-methylethyl) [1,1'-biphenyl]-2-yl]phosphine | NMI | N-Methylimidazole |
| BINAP | 2,2'-Bis(diphenylphosphino)-1,1'-binaphthalene | ¹H NMR | Proton nuclear magnetic resonance |
| BPO | Dibenzoyl peroxide | HFIP | Hexafluoroisopropanol |
| Cbz | Carbobenzoxy | MeO- | Methoxy |
| CNBr | cyanogen bromide | o/n | Overnight |
| Con. | Concentration | PCC | Pyridinium chlorochromate |
| DCE | 1,2-Dichloroethane | PE | petroleum ether |
| DCM | dichloromethane | Pin₂B₂ | pinacolborane |
| DEA | Diethylamine | PyBOP | Benzotriazol-1-yl-oxy-tris(pyrrolidino)phosphonium hexafluorophosphate |
| DIAD | Diisopropyl azodicarboxylate | Ruphos Pd G3 | Methanesulfonato(2- |
| | | | dicyclohexylphosphino-2',6'-diisopropoxy-1,1'-biphenyl)(2'-amino-1,1'-biphenyl-2-yl)palladium(II) (CAS No.: 1445085-77-7) |
| DIEA or DIPEA | N, N-diisopropylethylamine | rt | room temperature |
| DMA | N,N-Dimethylacetamide | tBu | tert-butyl |
| DMAP | 4-Dimethylaminopyridine | tBuONa | Sodium tert-butoxide |
| DME | Dimethylether | TBSCl | tert-Butyldimethylchlorosilane |
| DMEDA | N,N'-dimethylethylenediamine | TCFH | N,N,N',N'-Tetramethylchloroformamidinium hexafluorophosphate |
| DMF | N,N-dimethylformamide | TEA | triethylamine |
| DMSO | dimethyl sulfoxide | TFA | trifluoroacetic acid |
| EDCI | 1-(3-dimethylaminopropyl)-3-ethylcarbodiimide hydrochloride | T_{f}O- | Trifluoromethanesulfonate |
| ESI | electrospray ionization | Tf₂O | Triflic anhydride |
| equiv | equivalent | THF | Tetrahydrofuran |
| EtOH | ethanol | THP | tetrahydropyran |
| EtOAc or EA | Ethyl acetate | TLC | thin layer chromatography |
| HATU | 2-(7-Azabenzotriazol-1-yl)-N,N,N',N'-tetramethyluronium hexafluorophosphate | TMS | tetramethylsilane |
| HPLC | high performance liquid chromatography | TsCl | p-toluenesulfonyl chloride |
| HRMS | high resolution mass spectrometry | TsO- | Tosyloxy |
| IPA | Isopropyl alcohol | TsOH | p-Toluenesulfonic acid |
| LC-MS | liquid chromatography-mass spectrometry | ^{®}T₃P | propylphosphonic anhydride |
| LiHMDS | Lithium hexamethyldisilazide | TTIP | titanium(IV) isopropoxide |
| LRMS | low resolution mass spectrometry | Xantphos | 4,5-bis(diphenylphosphino)-9,9-dimethylxanthene |
| LC | liquid chromatography | Xphos | 2-dicyclohexylphosphino-2',4',6'-triisopropylbiphenyl |
| Me | methyl | | |

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). LC-MS spectra were recorded on a Sciex API 2000 mass spectrometer equipped with an Agilent 1100 binary pump, DAD and ELSD detectors, or on an Agilent 1260-6125B single quadrupole LC-MS system equipped with an Agilent 1260 quaternary pump, DAD and ELSD detectors. HPLC preparative purification was performed using a SHIMADZU LC-20AP system. HPLC purity was determined using either a SHIMADZU LC-30AP or Waters 1525 system. All reactions were carried out under ambient atmosphere unless otherwise specified. Reaction progress was monitored by TLC or LC-MS.

Solvents and reagents are processed as follows:
the solvents used in the reactions such as DCM, DMF, NMP, anhydrous EtOH, and anhydrous MeOH were commercially available;
preparative HPLC was performed using HPLC-grade CH₃CN and deionized water;
all other reactants, reagents and chemicals were commercially available, unless otherwise specified, or were synthesized using methods known in the art.
Unless otherwise specified, the materials and reagents used in the following examples are commercially available and used directly, or can be synthesized by using methods known in the art.

### General synthesis methods

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) and Formula (II) of the present disclosure according to routine techniques in the art.

### General synthesis method 1 for intermediate compounds:

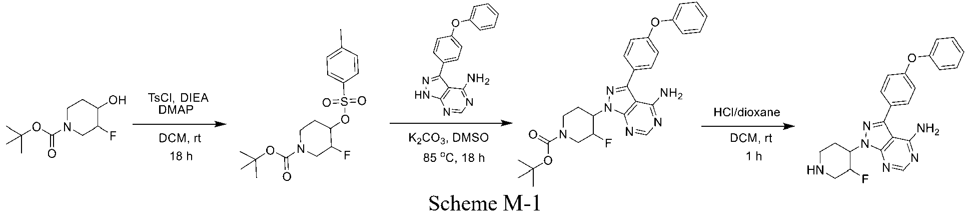

### General synthesis method 2 for intermediate compounds:

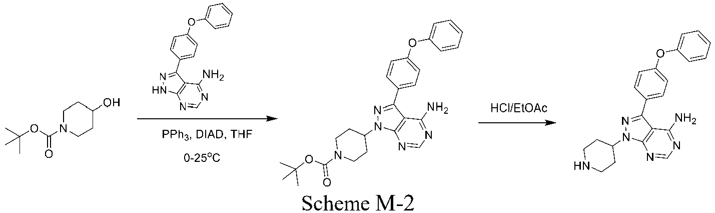

### General synthesis method 3 for intermediate compounds:

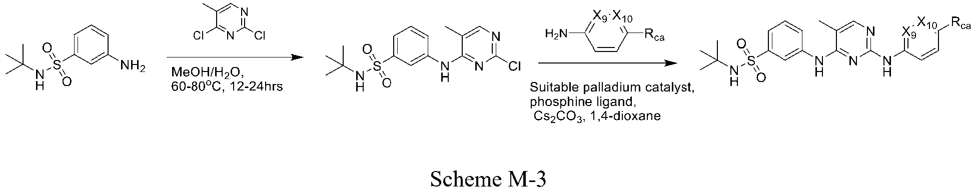

In Scheme M-3, X₉ and X₁₀ of the amine substrate used in Step 2 are the same or different and each independently represent N or CH. R_{ca} is a monovalent group corresponding to the divalent group R_{c} of the compound of Formula (I). For example, R_{ca} represents -OH, -NH(R_{d}), -C(O)NH₂, -N(R_{d})-Rr-NH(Rₑ), -R_{f}-C(O)NH₂, -R_{f}-C(O)OH, -C(O)OH, -R_{f}-H, -R_{f}-NH(R_{d}), -O-R_{f}-NH(R_{d}), 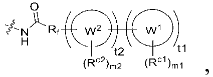 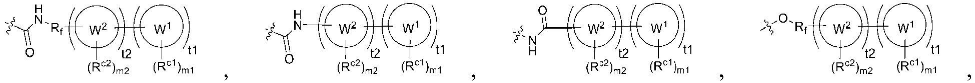 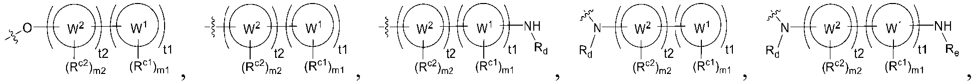 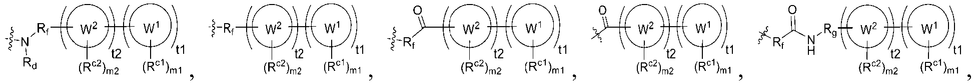 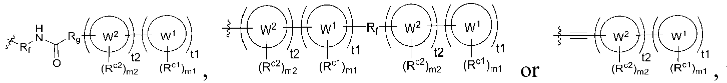 wherein R_{d}, Rₑ, R_{f}, R_{g}, R^{c1}, R^{c2}, ring W¹, ring W², t1, t2, m1, and m2 are as defined in the compound of Formula (I) and its various embodiments in the present disclosure. It should be understood that when the group R_{ca} contains reactive hydrogen that can participate in the reaction of Step 2, the reactive hydrogen can be protected by techniques and methods known to those skilled in the art, such as using protecting groups. For example, when R_{ca} represents a piperidinyl or piperazinyl group, conventional protecting groups such as Boc can be used to protect the hydrogen on the N. The removal of the protecting group can be achieved by techniques and methods known to those skilled in the art. For instance, the removal of the Boc protecting group can be accomplished under acidic conditions such as hydrochloric acid or trifluoroacetic acid.

The palladium catalyst and phosphine ligand used in Step 2 of Scheme M-3 can be conventional ones suitable for coupling reactions. There are many types of conventional palladium catalysts used for coupling reactions, such as palladium acetate (Pd(OAc)₂), tris(dibenzylideneacetone)dipalladium (Pd₂(dba)₃), diphenylphosphinoferrocene palladium dichloride, tetraphenylphosphine palladium, dichlorobis(triphenylphosphine) palladium, palladium on carbon, and so on. The phosphine ligand can be a conventional one like Xantphos or BINAP.

In Step 1 of Scheme M-3, to a solution of 3-amino-N-(tert-butyl)benzenesulfonamide (9.50 g, 41.608 mmol) in MeOH/H₂O (400 mL) was added 2,4-dichloro-5-methylpyrimidine (6.78 g, 41.608 mmol), and the reaction was stirred at 80°C for 18 hours. After monitoring the reaction via LCMS and confirming its completion, the reaction mixture was cooled to room temperature, resulting in the precipitation of a large amount of white solid. The reaction mixture was filtered, and the filter cake was washed with methanol/water (50 mL, 1:1), dried under high vacuum at 50°C, yielding a compound N-(tert-butyl)-3-((2-chloro-5-methylpyrimidin-4-yl)amino)benzenesulfonamide as white solid (8.00 g, 22.544 mmol, yield 54.18%). LCMS (ESI): calcd. for (M) 354.09, found, (M+H)⁺ 355.10.

In Step 2 of Scheme M-3, to a solution of N-(tert-butyl)-3-((2-chloro-5-methylpyrimidin-4-yl)amino)benzenesulfonamide (1 equiv) and the corresponding amine substrate (1.3 equiv) in dioxane (50 mL) were added Cs₂CO₃ and a suitable palladium catalyst/phosphine ligand. The reaction was stirred at 100°C for 4 hours. The reaction mixture was diluted with ethyl acetate and saturated NaCl solution, dried over Na₂SO₄, and concentrated under vacuum to obtain the crude product. The crude product was purified by silica gel flash column chromatography (eluent (v/v): EtOAc/PE = 0/1-1/4) to give the target compound.

### General synthesis method 4 for intermediate compounds:

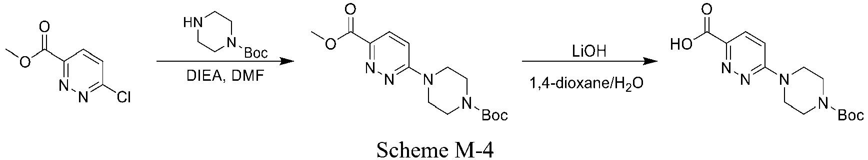

### General synthesis method 5 for intermediate compounds:

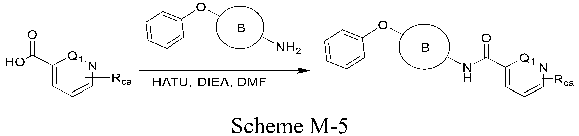

In Scheme M-5, ring B of the amine substrate and Q₁ of the acid substrate are as defined in Formula (PBM-4-1A) and its various embodiments in the present disclosure. The R_{ca} of the acid substrate used is a monovalent group corresponding to the divalent group R_{c} of the compound of Formula (I). For example, R_{ca} represents -OH, -NH(R_{d}), -C(O)NH₂, -N(R_{d})-R_{f}-NH(Rₑ), -R_{f}-C(O)NH₂, -R_{f}-C(O)OH, -C(O)OH, -R_{f}-H, - R_{f}-NH(R_{d}), -O-R_{f}-NH(R_{d}), 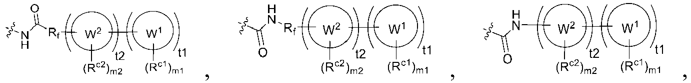 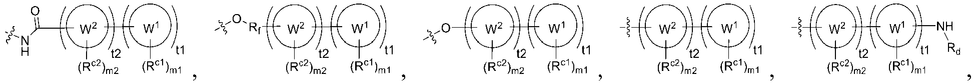 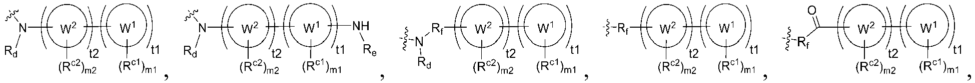 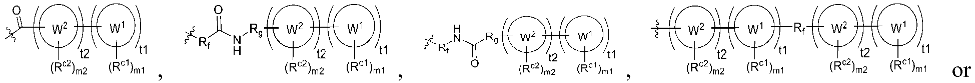 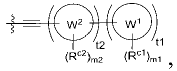 wherein R_{d}, Rₑ, R_{f}, R_{g}, R^{c1}, R^{c2}, ring W¹, ring W², t1, t2, m1, and m2 are as defined in the compound of Formula (I) and its various embodiments in the present disclosure. It should be understood that when the group R_{ca} contains an active hydrogen or reactive group that can participate in the reaction of Scheme M-5, the active hydrogen or reactive group can be protected by techniques and methods well known to those skilled in the art, such as protective groups. For example, when R_{ca} represents a piperidinyl or piperazinyl group, conventional protective groups such as Boc can be used to protect the hydrogen on N. The removal of the protective group can be achieved by techniques and methods well known to those skilled in the art, such as removing the protective group Boc under acidic conditions such as hydrochloric acid or trifluoroacetic acid.

In Scheme M-5, to a solution of the acid substrate (1 equiv) and amine substrate (1 equiv) in DMF were added HATU (1.5 equiv) and DIEA (5 equiv). The reaction mixture was then allowed to react at room temperature for 30 minutes. After monitoring the reaction via LCMS and confirming its completion, water was added to the reaction mixture, and the resulting mixture was extracted with ethyl acetate. The organic phase was washed with saturated saline and concentrated to obtain the crude product. The crude product was purified using a chromatography column (eluent (v/v): DCM/MeOH=100/1) to give the target compound.

### General synthesis method 6 for intermediate compounds:

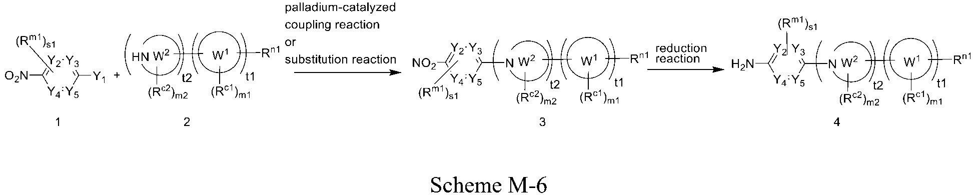

In Scheme M-6, (R^{m1})ₛ₁ of compound 1 indicates that the six-membered ring to which it is attached is optionally substituted with s1 R^{m1} groups, with each R^{m1} being identical or different and each independently representing halogen, hydroxy, C₁₋₆ alkyl (e.g., methyl or ethyl), halogenated C₁₋₆ alkyl, deuterated C₁₋₆ alkyl, C₁₋₆ alkoxy (e.g., methoxy or isopropoxy), halogenated C₁₋₆ alkoxy, deuterated C₁₋₆ alkoxy, NO₂, NH₂, or cyano, s1 represents an integer of 0, 1, 2, 3 or 4, Y₁ represents halogen (e.g., fluorine, chlorine, or bromine), and Y₂, Y₃, Y₄, and Y₅ each independently represent N or CH, with the provision that Y₂, Y₃, Y₄, and Y₅ are not all N simultaneously. Ring W¹, ring W², t1, t2, (R^{c1})ₘ₁, and (R^{c2})ₘ₂ are as defined in the compound of Formula (I) of the present disclosure. Rⁿ¹ represents Boc, H or -NHRₕ, wherein Rₕ represents hydrogen or C₁₋₆ alkyl (e.g., methyl or ethyl). It should be understood that when ring W¹-Rⁿ¹ contains additional reactive groups (e.g., -NH- or -NH₂) that can participate in the reaction of Scheme M-6, the reactive groups can be protected by techniques and methods known to those skilled in the art, such as using protecting groups.

Depending upon the target compound, the reaction substrate, reaction conditions (including reaction dosage, temperature, time, etc.), and work up, etc. in Scheme M-6 can be appropriately modified and adjusted using techniques and methods well known to those skilled in the art to obtain the desired target compound. In Scheme M-6, the substitution reaction or palladium-catalyzed coupling reaction of step 1 can be conventional techniques and methods known to those skilled in the art. When step 1 is a substitution reaction, Y₁ represents fluorine, and compounds 1 and 2 react under basic conditions (such as K₂CO₃). When step 1 is a palladium-catalyzed coupling reaction, Y₁ represents bromine, and the palladium catalyst and phosphine ligand used can be conventional ones suitable for coupling reactions. There are many types of conventional palladium catalysts for coupling reactions, such as palladium acetate (Pd(OAc)₂), tris(dibenzylideneacetone)dipalladium (Pd₂(dba)₃), diphenylphosphinoferrocene dichloropalladium, tetrakis(triphenylphosphine)palladium, dichlorobis(triphenylphosphine)palladium, palladium on carbon, etc. The phosphine ligand can be a conventional ligand such as Xantphos or BINAP. The reduction reaction of step 2 in Scheme M-6 can be conventional techniques and methods known to those skilled in the art, such as H₂/Pd/C or Fe/NH₄Cl/MeOH.

### General synthesis method 7 for intermediate compounds:

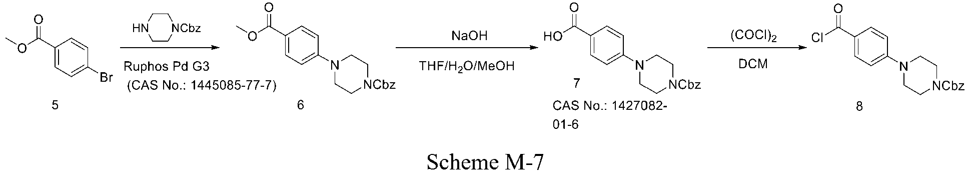

### General synthesis method 8 for intermediate compounds:

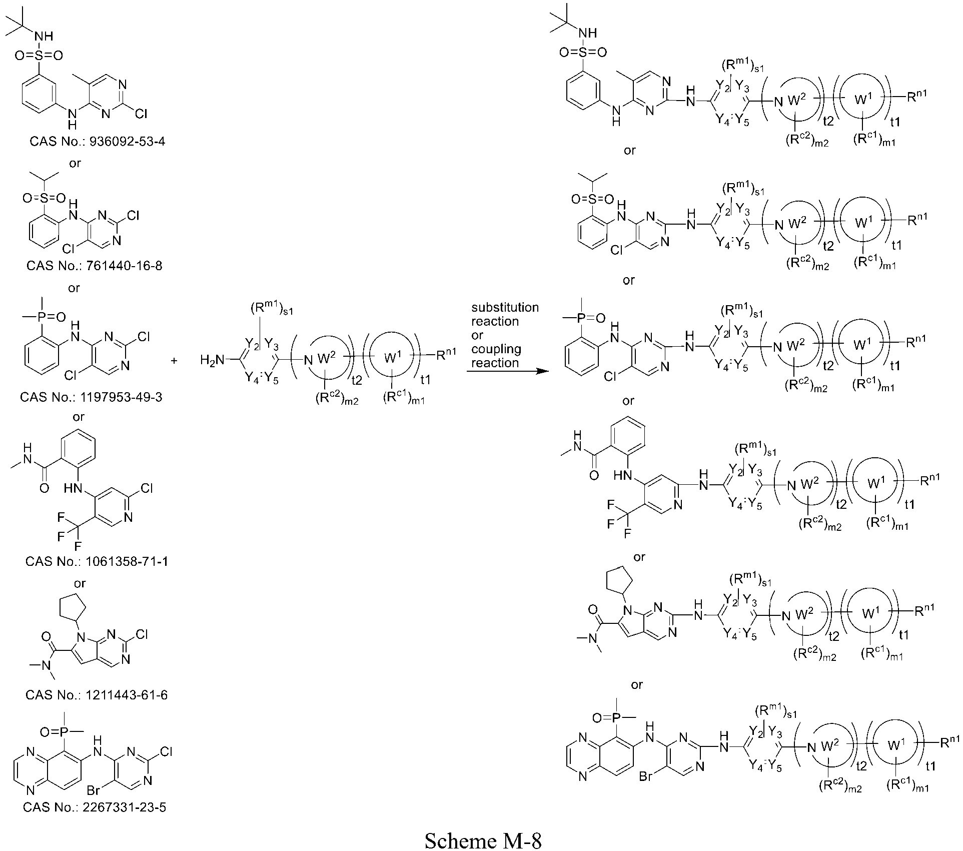

In Scheme M-8, (R^{m1})ₛ₁ of the compounds indicate that the six-membered ring to which it is attached is optionally substituted with s1 R^{m1} groups, with each R^{m1} being identical or different and each independently representing halogen, hydroxy, C₁₋₆ alkyl (e.g., methyl or ethyl), halogenated C₁₋₆ alkyl, deuterated C₁₋₆ alkyl, C₁₋₆ alkoxy (e.g., methoxy or isopropoxy), halogenated C₁₋₆ alkoxy, deuterated C₁₋₆ alkoxy, NO₂, NH₂, or cyano. Y₁ represents halogen (e.g., fluorine, chlorine, or bromine), and Y₂, Y₃, Y₄, and Y₅ each independently represent N or CH, with the provision that Y₂, Y₃, Y₄, and Y₅ are not all N simultaneously. Ring W¹, ring W², t1, t2, (R^{c1})ₘ₁, and (R^{c2})ₘ₂ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W¹-Rⁿ¹ contains additional reactive groups (such as - NH- or -NH₂) that can participate in the reaction of Scheme M-8, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme M-8 can be appropriately modified and adjusted using techniques and methods well known to those skilled in the art to obtain the desired target compound. In Scheme M-8, the substitution reaction or coupling reaction can be conventional techniques and methods known to those skilled in the art. For example, substitution reactions can be carried out under heating conditions in the presence of triethylamine/isopropanol, p-toluenesulfonic acid/n-butanol, DIEA/ethanol, NaHMDS, or NaI/DIEA/DMF; coupling reactions can be performed under heating conditions in the presence of Brettphos Pd G3/Brettphos ligand/Cs₂CO₃.

### General synthesis method 9 for intermediate compounds:

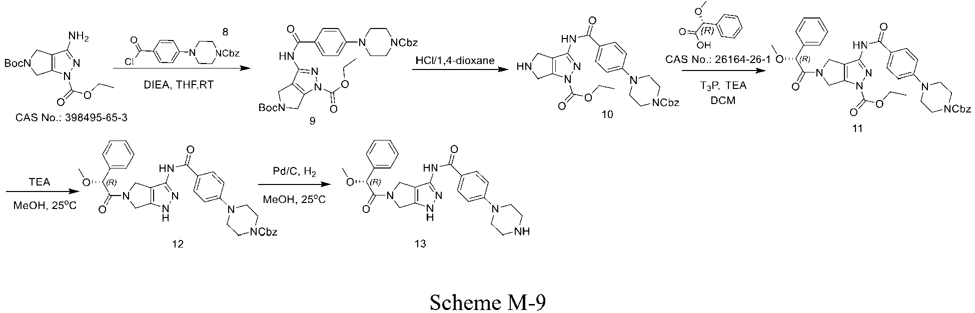

The compound 8 in Step 1 of Scheme M-9 can be prepared by referring to Scheme M-7.

### General synthesis method 10 for intermediate compounds:

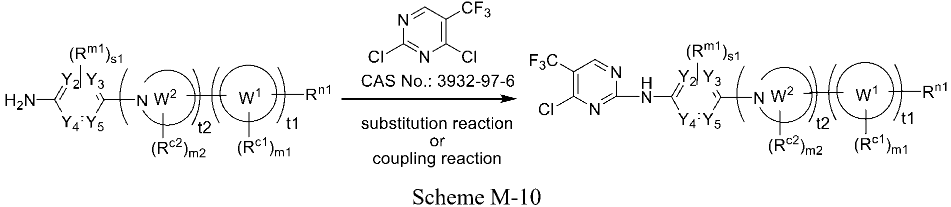

In Scheme M-10, (R^{m1})ₛ₁ of the compounds indicates that the benzene ring is optionally substituted with s1 R^{m1} groups, with each R^{m1} being identical or different and each independently representing halogen, hydroxy, C₁₋₆ alkyl (e.g., methyl or ethyl), halogenated C₁₋₆ alkyl, deuterated C₁₋₆ alkyl, C₁₋₆ alkoxy (e.g., methoxy or isopropoxy), halogenated C₁₋₆ alkoxy, deuterated C₁₋₆ alkoxy, NO₂, NH₂, or cyano, and s1 represents an integer of 0, 1, 2, 3 or 4. Y₁ represents halogen (e.g., fluorine, chlorine, or bromine), and Y₂, Y₃, Y₄, and Y₅ each independently represent N or CH, with the proviso that Y₂, Y₃, Y₄, and Y₅ are not all N at the same time. Rings W¹, W², t1, t2, (R^{c1})ₘ₁, (R^{c2})ₘ₂ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W¹-Rⁿ¹ contains additional reactive groups (such as - NH- or -NH₂) that can participate in the reaction of Scheme M-10, the reactive groups can be protected by techniques and methods known to those skilled in the art, such as using protecting groups.

Depending upon the target compound, the reaction substrate, reaction conditions (including reaction dosage, temperature, time, etc.), and work up, etc. in Scheme M-10 can be appropriately modified and adjusted using techniques and methods well known to those skilled in the art to obtain the desired target compound. In Scheme M-10, the substitution reaction or coupling reaction can be conventional techniques and methods known to those skilled in the art. For example, substitution reactions can be carried out under heating conditions in the presence of triethylamine/isopropanol, DIEA/ethanol, NaHMDS, or NaI/DIEA/DMF; coupling reactions can be performed under heating conditions in the presence of Brettphos Pd G3/Brettphos ligand/Cs₂CO₃.

### General synthesis method 11 for intermediate compounds:

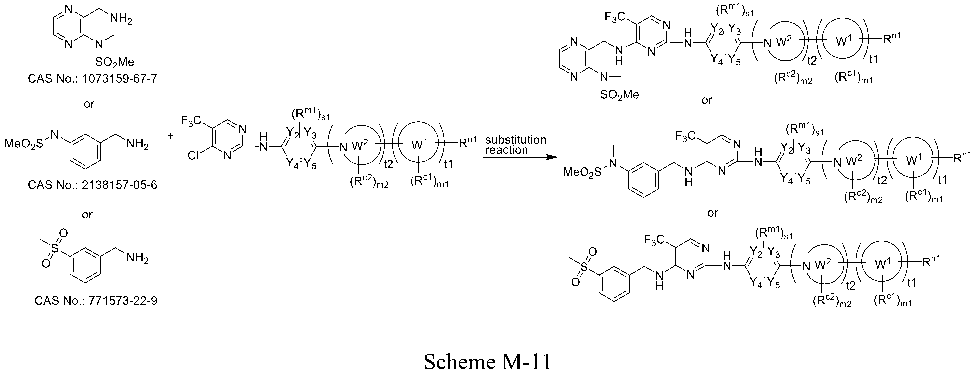

In Scheme M-11, (R^{m1})ₛ₁ of the compounds indicates that the benzene ring is optionally substituted with s1 R^{m1} groups, with each R^{m1} being identical or different and each independently representing halogen, hydroxy, C₁₋₆ alkyl (e.g., methyl or ethyl), halogenated C₁₋₆ alkyl, deuterated C₁₋₆ alkyl, C₁₋₆ alkoxy (e.g., methoxy or isopropoxy), halogenated C₁₋₆ alkoxy, deuterated C₁₋₆ alkoxy, NO₂, NH₂, or cyano. Y₁ represents halogen (e.g., fluorine, chlorine, or bromine), and Y₂, Y₃, Y₄, and Y₅ each independently represent N or CH, with the proviso that Y₂, Y₃, Y₄, and Y₅ are not all N at the same time. Rings W¹, W², t1, t2, (R^{c1})ₘ₁, (R^{c2})ₘ₂ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W¹-Rⁿ¹ contains additional reactive groups (such as -NH- or -NH₂) that can participate in the reaction of Scheme M-11, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme M-11 can be appropriately modified and adjusted using techniques and methods well known to those skilled in the art to obtain the desired target compound. In Scheme M-11, the substitution reaction can be conventional techniques and methods known to those skilled in the art. For example, substitution reactions can be carried out under heating conditions in the presence of triethylamine/isopropanol, DIEA/ethanol, or NaI/DIEA/DMF.

### General synthesis method 12 for intermediate compounds:

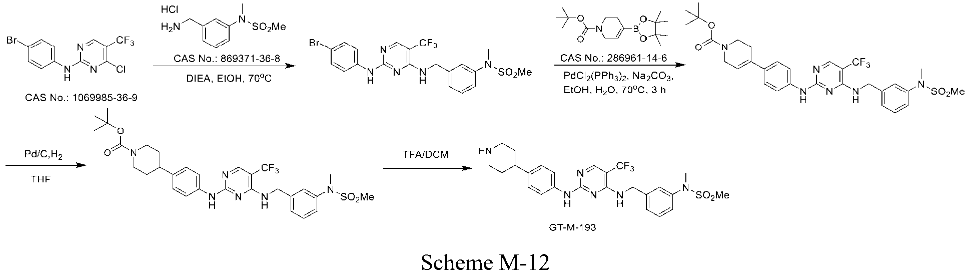

### General synthesis method 13 for intermediate compounds:

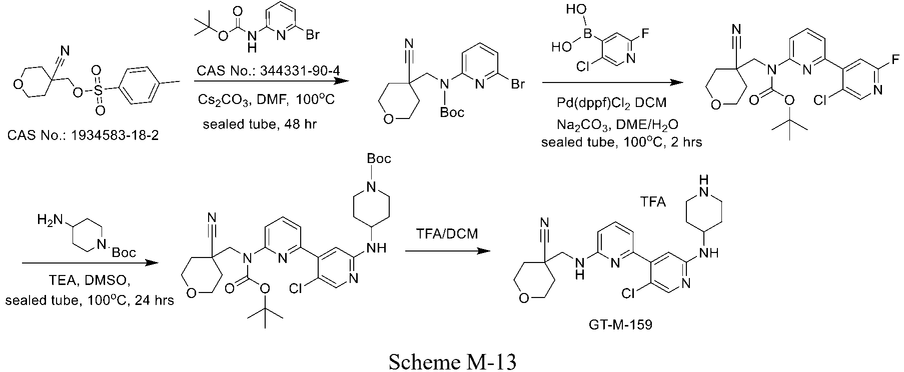

### General synthesis method 14 for intermediate compounds:

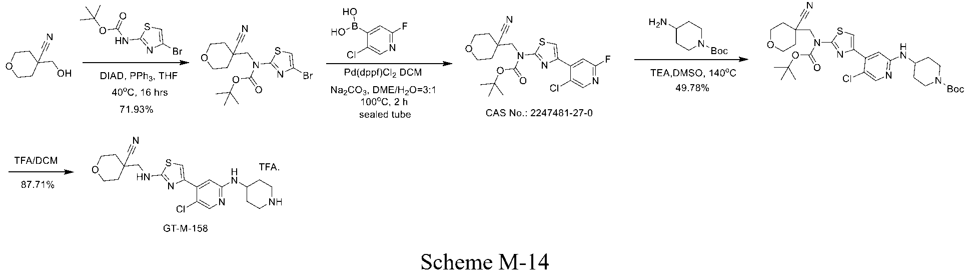

### General synthesis method 15 for intermediate compounds:

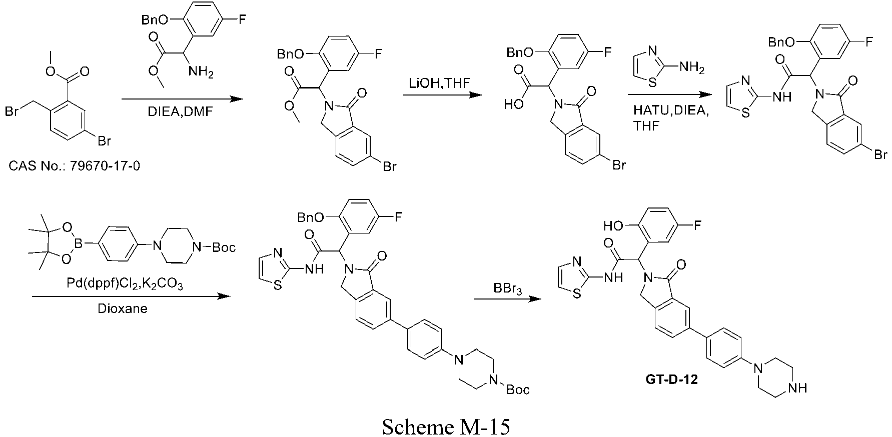

### General synthesis method 16 for intermediate compounds:

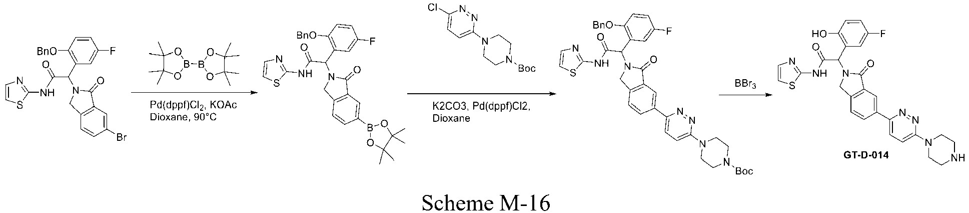

### General synthesis method 17 for intermediate compounds:

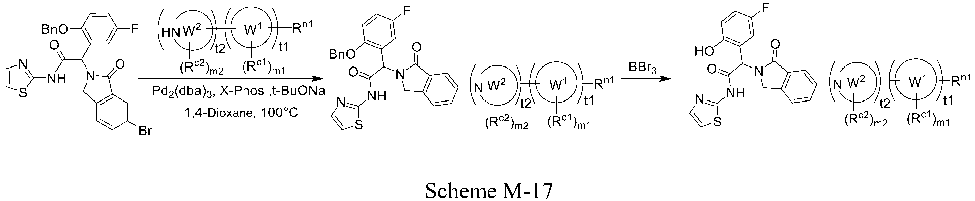

In Scheme M-17, the ring W¹, ring W², t1, t2, (R^{c1})ₘ₁, (R^{c2})ₘ₂ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W¹-Rⁿ¹ contains additional reactive groups (such as -NH- or -NH₂) that can participate in the reaction of Scheme M-17, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

Depending upon the target compound, the reaction substrate, reaction conditions (including reaction dosage, temperature, time, etc.), and work up, etc. in Scheme M-17 can be appropriately modified and adjusted using techniques and methods well known to those skilled in the art to obtain the desired target compound.

### General synthesis method 18 for intermediate compounds:

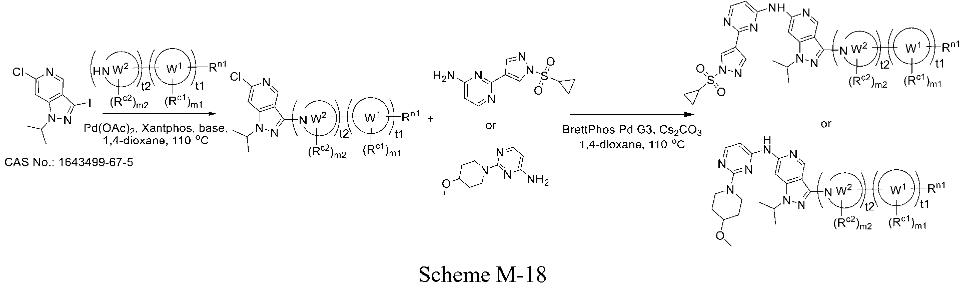

In Scheme M-18, the ring W¹, ring W², t1, t2, (R^{c1})ₘ₁, (R^{c2})ₘ₂ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W¹-Rⁿ¹ contains additional reactive groups (such as -NH- or -NH₂) that can participate in the reaction of Scheme M-18, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method 19 for intermediate compounds:

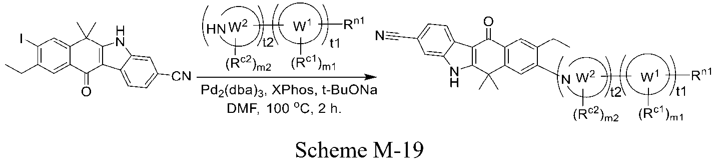

In Scheme M-19, the ring W¹, ring W², t1, t2, (R^{c1})ₘ₁, (R^{c2})ₘ₂ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W¹-Rⁿ¹ contains additional reactive groups (such as -NH- or -NH₂) that can participate in the reaction of Scheme M-19, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method 20 for intermediate compounds:

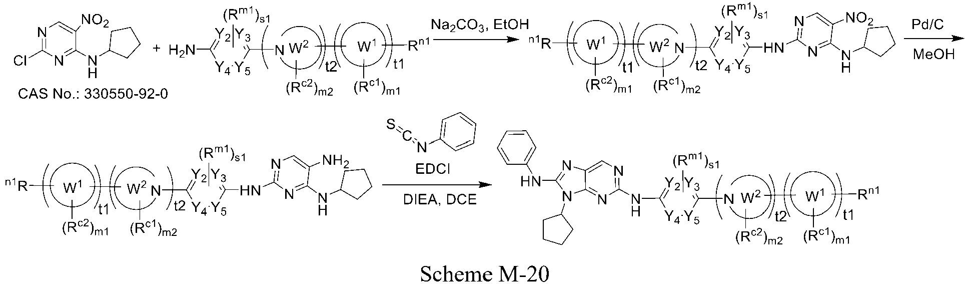

In Scheme M-20, (R^{m1})ₛ₁ of compounds indicates that the benzene ring is optionally substituted with s1 R^{m1} groups, with each R^{m1} being the same or different and independently representing halogen, hydroxy, C₁₋₆ alkyl (such as methyl or ethyl), halo C₁₋₆ alkyl, deuterated C₁₋₆ alkyl, C₁₋₆ alkoxy (such as methoxy or isopropoxy), halo C₁₋₆ alkoxy, deuterated C₁₋₆ alkoxy, NO₂, NH₂, or cyano. Y₁ represents halogen (such as fluorine, chlorine, or bromine), and Y₂, Y₃, Y₄, and Y₅ each independently represent N or CH, with the provision that Y₂, Y₃, Y₄, and Y₅ are not all N simultaneously. Ring W¹, ring W², t1, t2, (R^{c1})ₘ₁, (R^{c2})ₘ₂ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W¹-Rⁿ¹ contains additional reactive groups (such as -NH- or -NH₂) that can participate in the reaction of Scheme M-20, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method 21 for intermediate compounds:

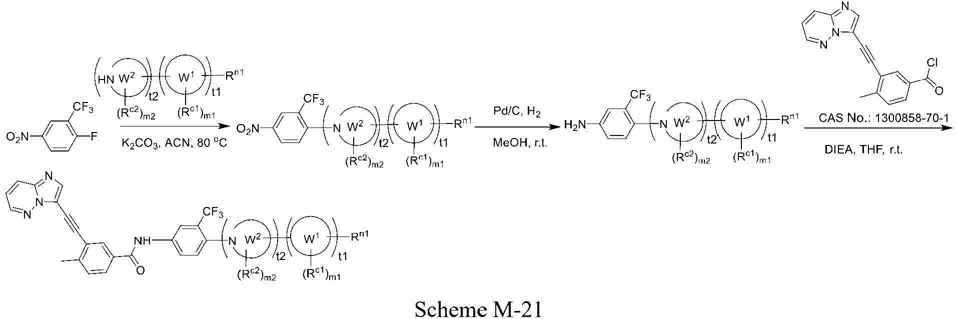

In Scheme 21, the rings W¹, W², t1, t2, (R^{c1})ₘ₁, (R^{c2})ₘ₂ are as defined in the compound of Formula (I) of the present disclosure. Rⁿ¹ represents Boc, H, or -NHRₕ, where Rₕ represents hydrogen or C₁₋₆ alkyl (such as methyl or ethyl). It should be understood that when ring W¹-Rⁿ¹ contains additional reactive groups (such as - NH- or -NH₂) that can participate in the reaction, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions (such as concentrated sulfuric acid, etc.).

### General synthesis method 22 for intermediate compounds:

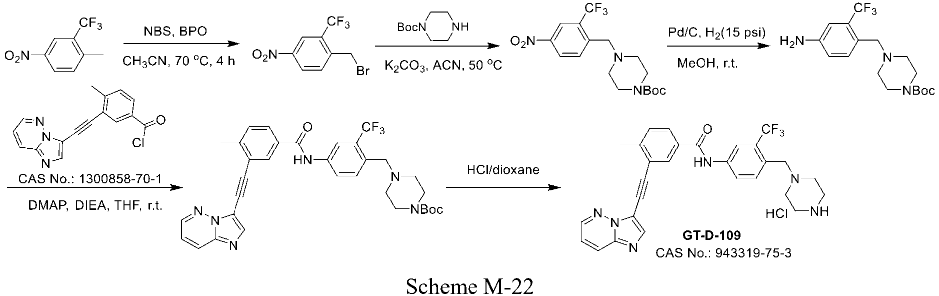

### General synthesis method 23 for intermediate compounds:

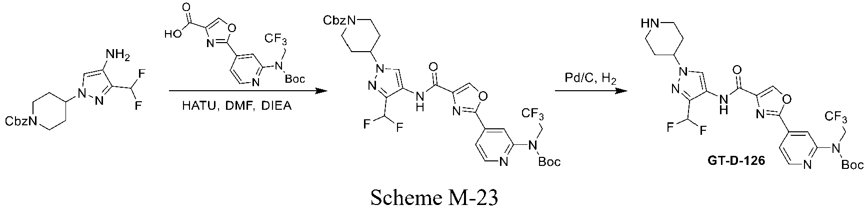

### General synthesis method 24 for intermediate compounds:

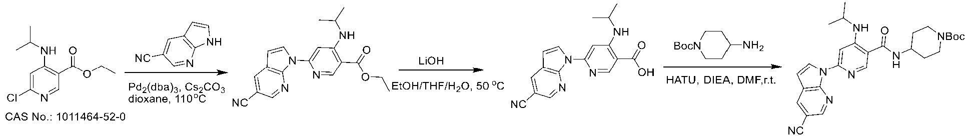

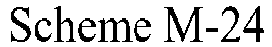

The removal of protecting groups can be achieved through techniques and methods known to those skilled in the art. For example, the removal of the Boc protecting group can be achieved under acidic conditions such as concentrated sulfuric acid.

### General synthesis method 25 for intermediate compounds:

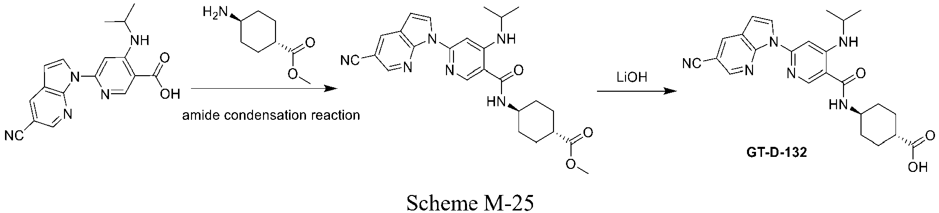

Depending upon the target compound, the reaction substrate, reaction conditions (including reaction dosage, temperature, time, etc.), and work up, etc. in Scheme M-25 can be appropriately modified and adjusted using techniques and methods well known to those skilled in the art to obtain the desired target compound. In Scheme M-25, the amide condensation reaction can be conventional techniques and methods known to those skilled in the art. For example, the amide condensation reaction can be carried out at room temperature in the presence of HATU/DIEA/DMF or HOAt/EDCI/TEA/DCM.

### General synthesis method 26 for intermediate compounds:

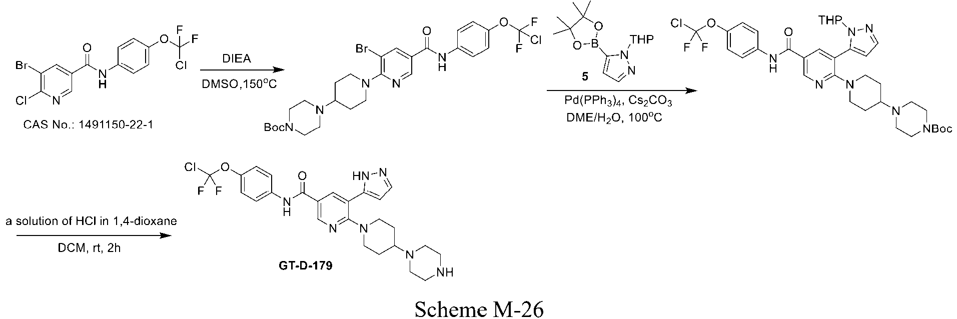

### General synthesis method 27 for intermediate compounds:

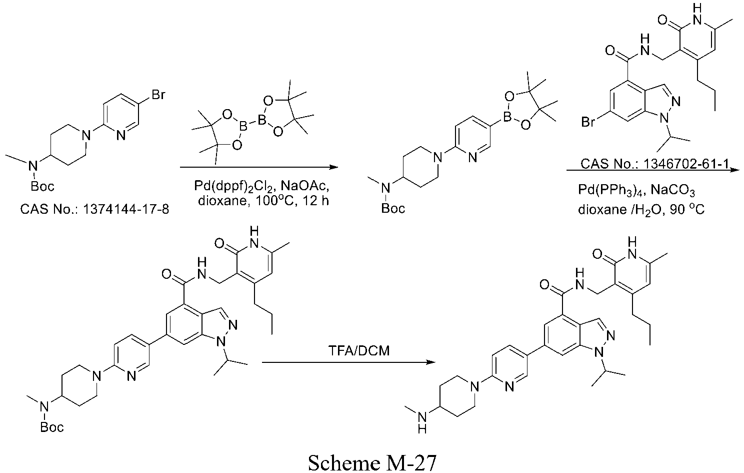

### General synthesis method 28 for intermediate compounds:

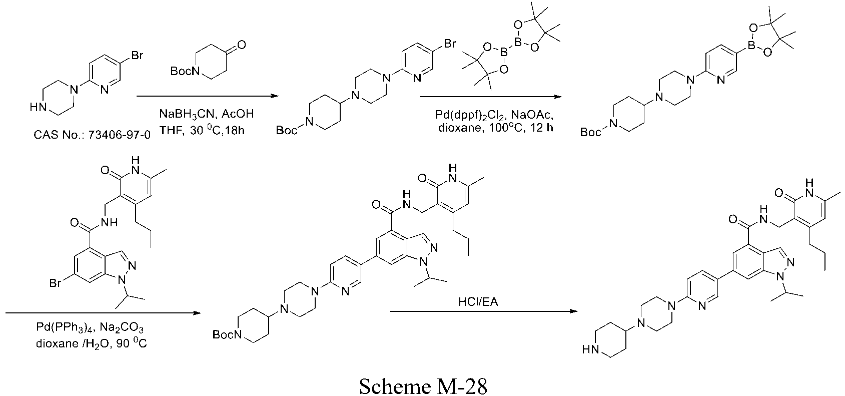

### General synthesis method 29 for intermediate compounds:

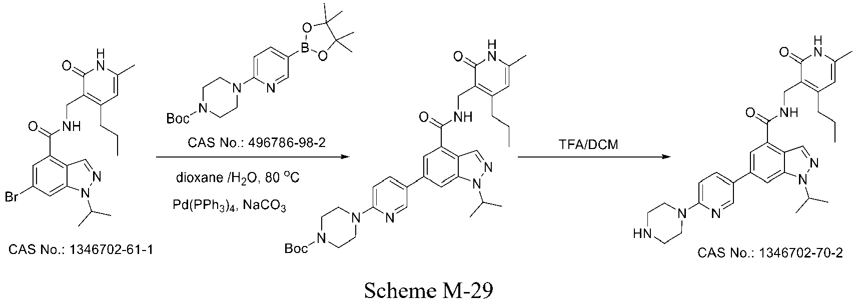

### General synthesis method 30 for intermediate compounds:

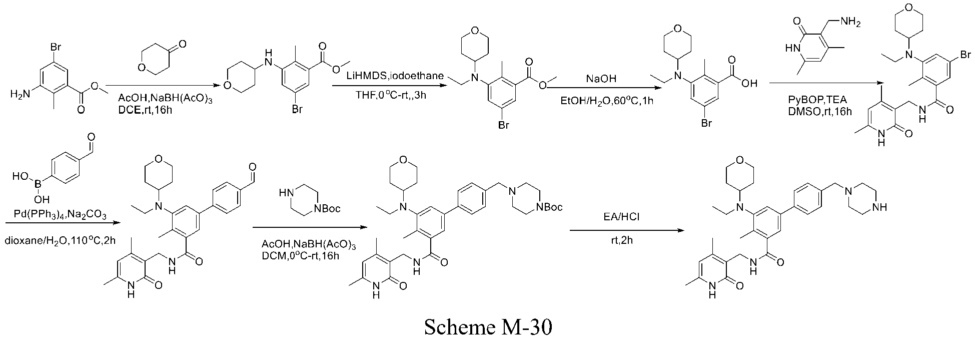

### General synthesis method 31 for intermediate compounds:

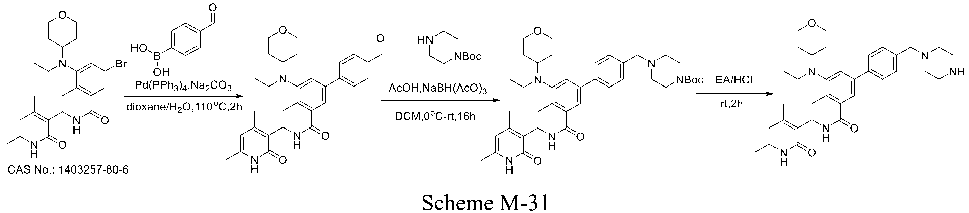

### General synthesis method 32 for intermediate compounds:

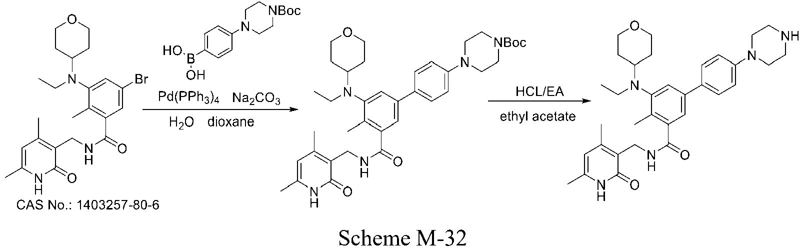

### General synthesis method 33 for intermediate compounds:

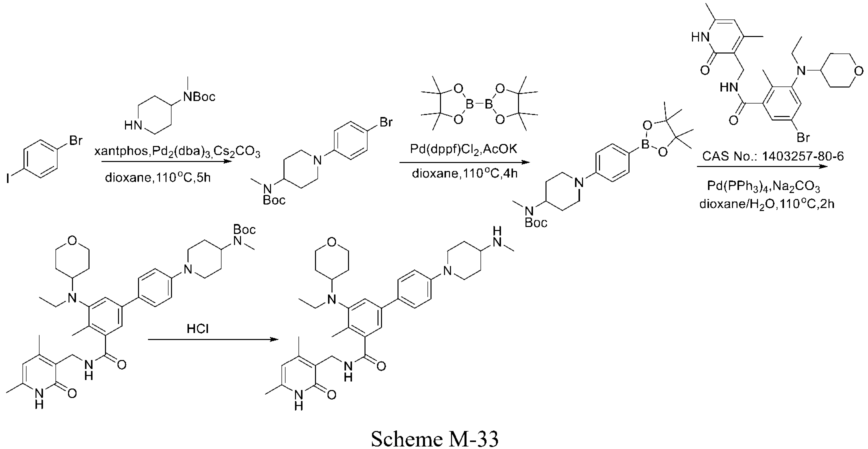

### General synthesis method 34 for intermediate compounds:

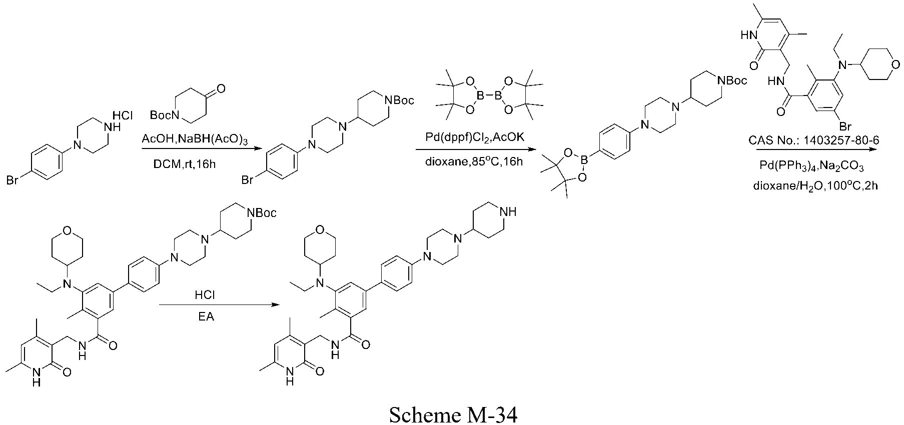

### General synthesis method 35 for intermediate compounds:

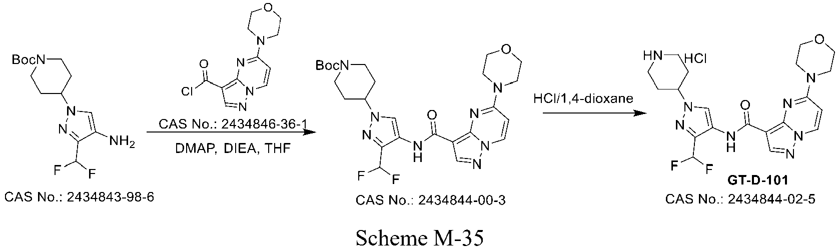

### General synthesis method 36 for intermediate compounds:

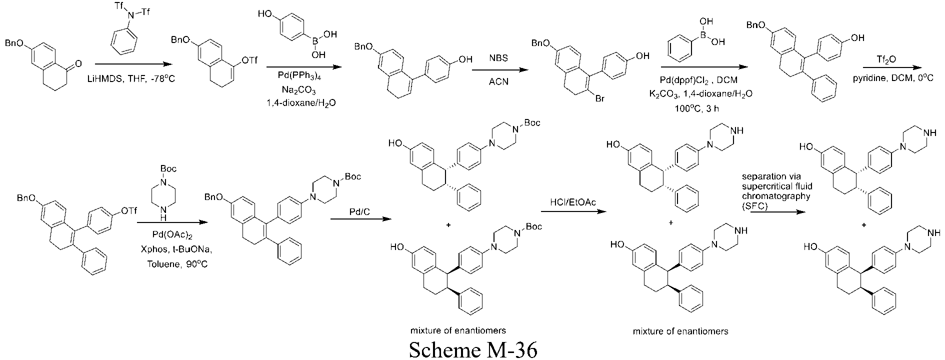

Pure enantiomeric products can be obtained through resolution/separation via supercritical fluid chromatography (SFC) and according to Scheme M-36. The conditions of supercritical fluid chromatography (SFC) can be appropriately modified and adjusted using techniques and methods well-known to those skilled in the art to obtain the desired target compound.

### General synthesis method 37 for intermediate compounds:

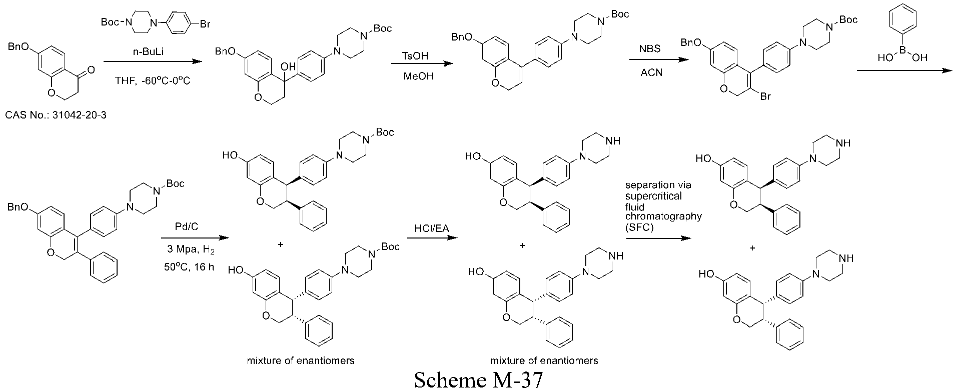

Pure enantiomeric products can be obtained through resolution/separation via supercritical fluid chromatography (SFC) and according to Scheme M-37. The conditions of supercritical fluid chromatography (SFC) can be appropriately modified and adjusted using techniques and methods well-known to those skilled in the art to obtain the desired target compound.

### General synthesis method 38 for intermediate compounds:

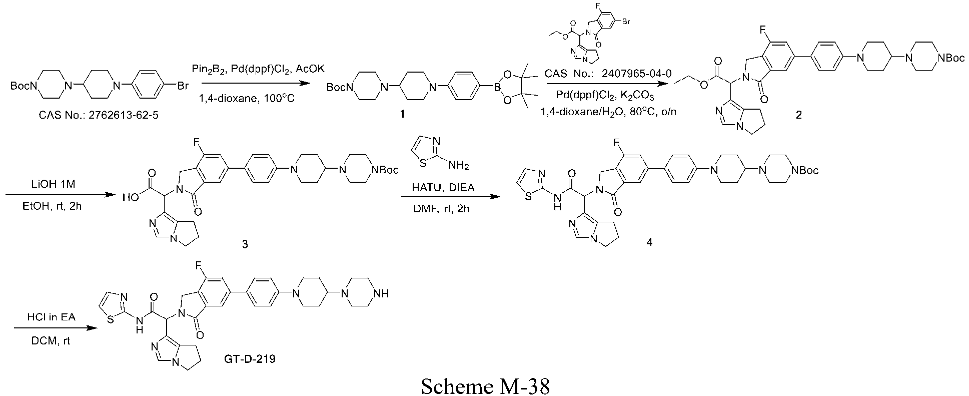

### General synthesis method 39 for intermediate compounds:

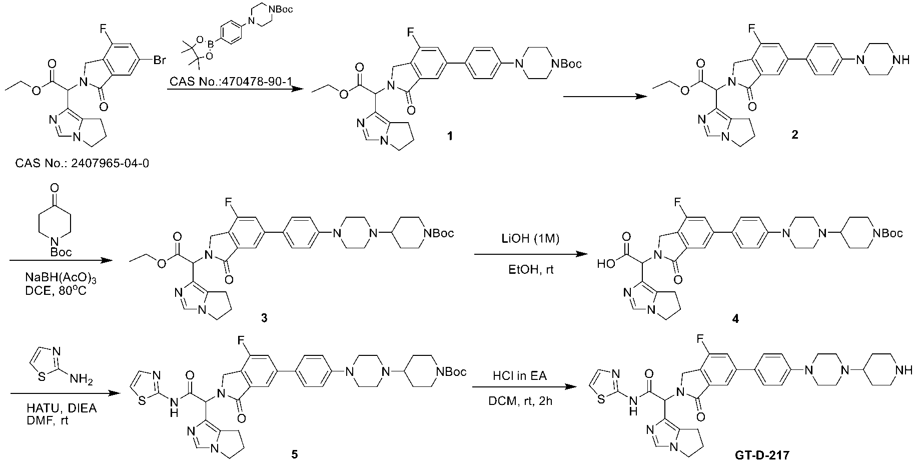

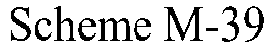

### General synthesis method 40 for intermediate compounds:

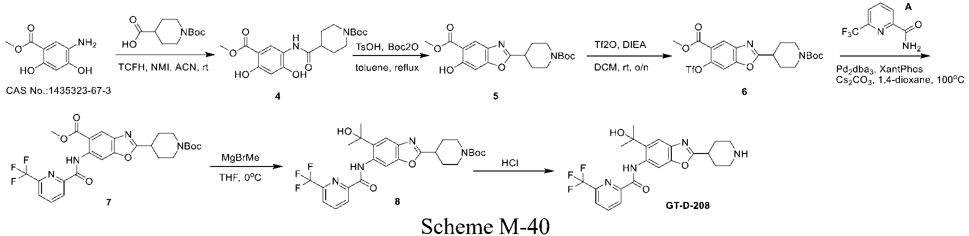

### General synthesis method 41 for intermediate compounds:

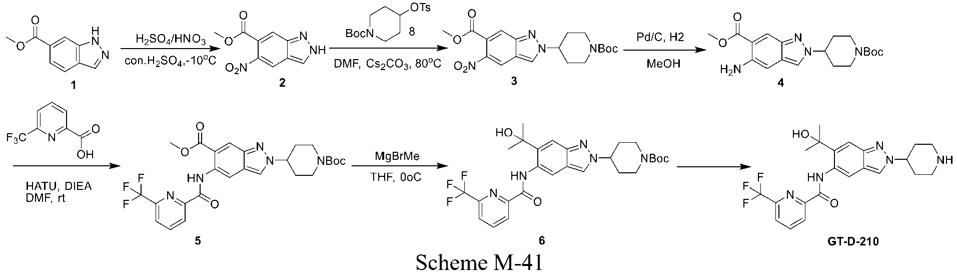

### General synthesis method 42 for intermediate compounds:

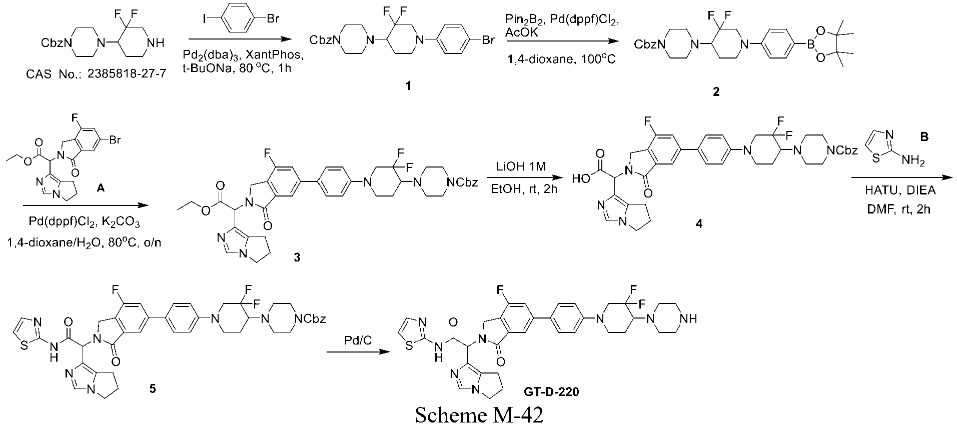

### General synthesis method 43 for intermediate compounds:

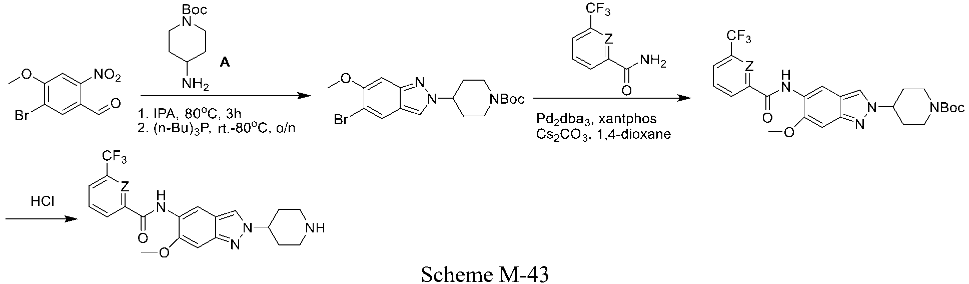

**In Scheme M-43, Z represents CH or N.**

### General synthesis method 44 for intermediate compounds:

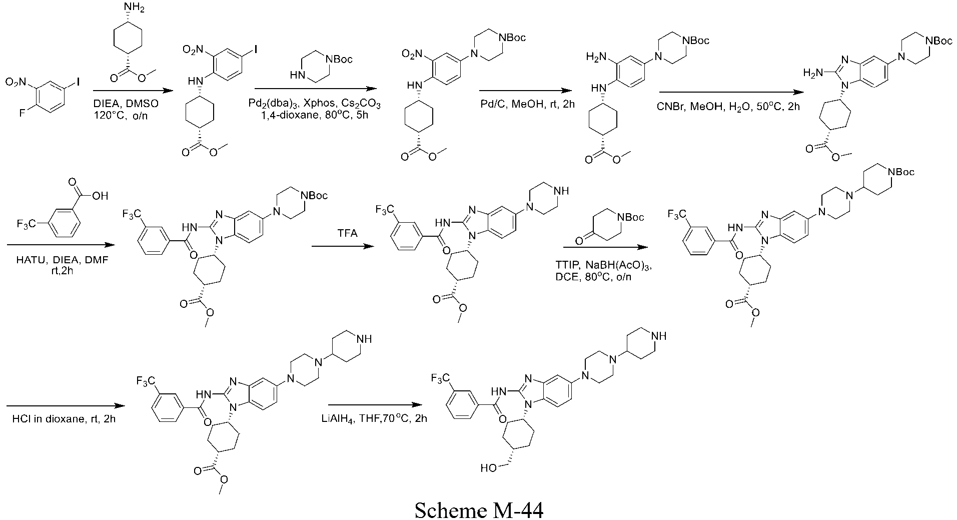

### General synthesis method 45 for intermediate compounds:

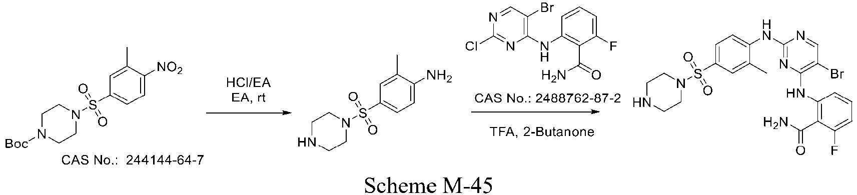

### General synthesis method 46 for intermediate compounds:

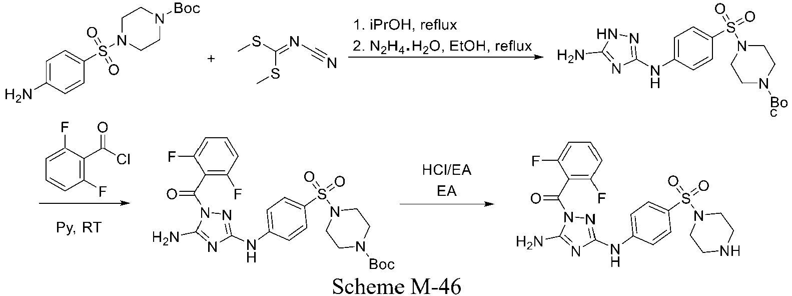

### General synthesis method 47 for intermediate compounds:

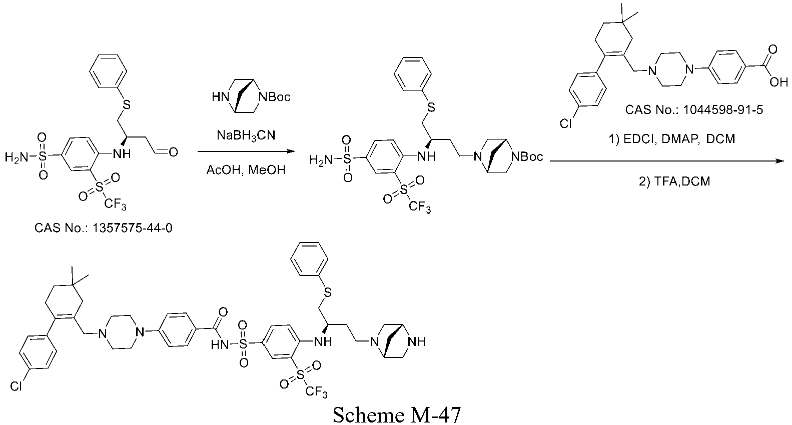

### General synthesis method 48 for intermediate compounds:

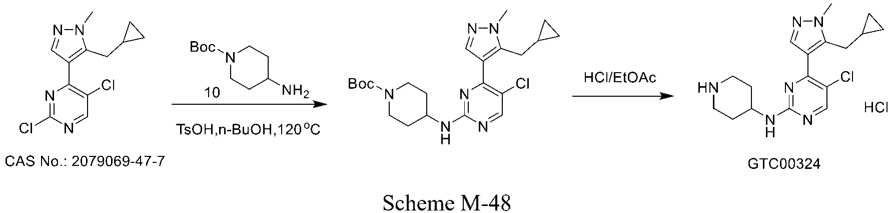

### General synthesis method 49 for intermediate compounds:

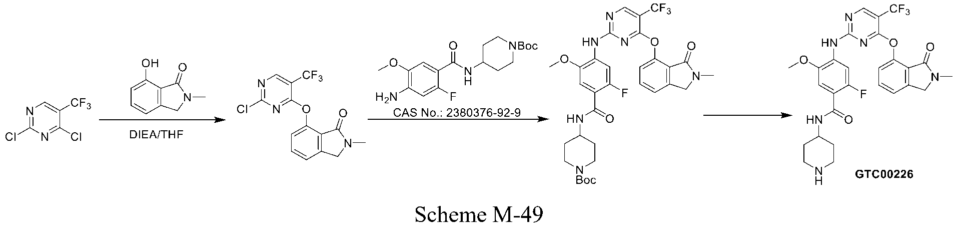

### General synthesis method 50 for intermediate compounds:

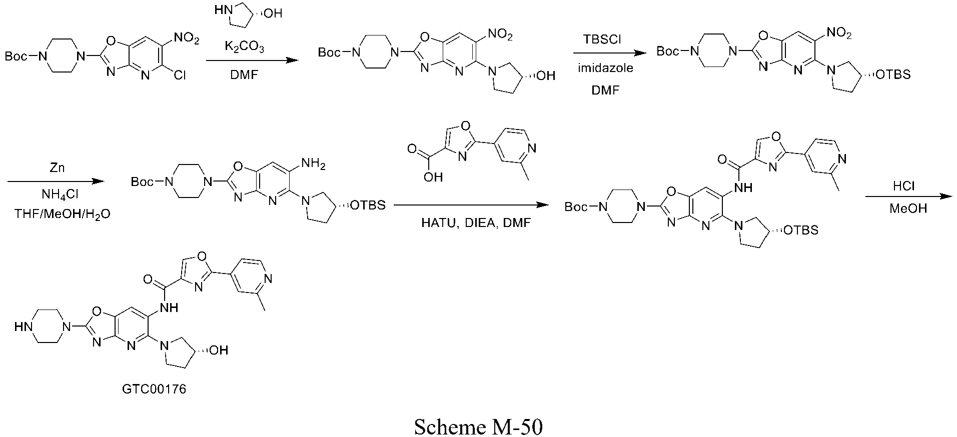

### General synthesis method 51 for intermediate compounds:

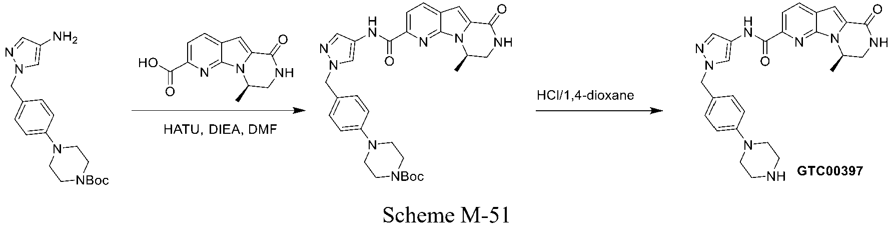

### General synthesis method 52 for intermediate compounds:

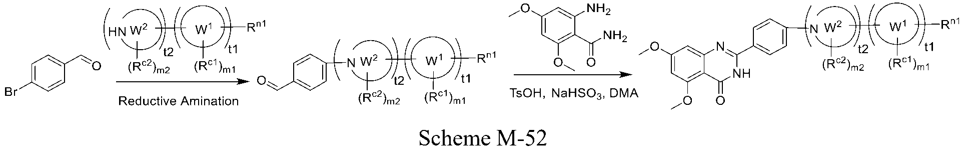

Ring W¹, ring W², t1, t2, (R^{c1})ₘ₁, and (R^{c2})ₘ₂ of the compounds in Scheme M-52 are as defined in the compound of Formula (I) of the present disclosure. Rⁿ¹ represents Boc, H, or -NHR_{g}, where R_{g} represents hydrogen or C₁₋₆ alkyl (e.g., methyl or ethyl). It should be understood that when ring W⁶-Rⁿ¹ contains additional reactive groups (such as -NH- or -NH₂) that can participate in the reaction of Scheme M-52, the reactive groups can be protected by techniques and methods known to those skilled in the art, such as using protecting groups. The removal of protecting groups can be achieved through techniques and methods known to those skilled in the art. For example, the removal of the Boc protecting group can be achieved under acidic conditions such as hydrochloric acid or trifluoroacetic acid.

### General synthesis method 53 for intermediate compounds:

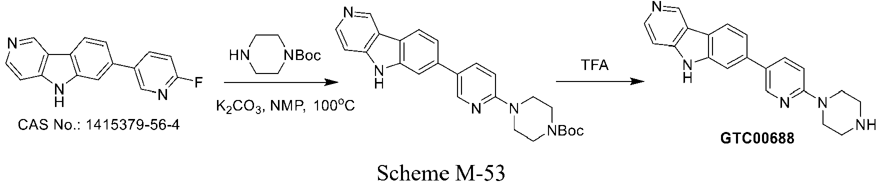

### General synthesis method 54 for intermediate compounds:

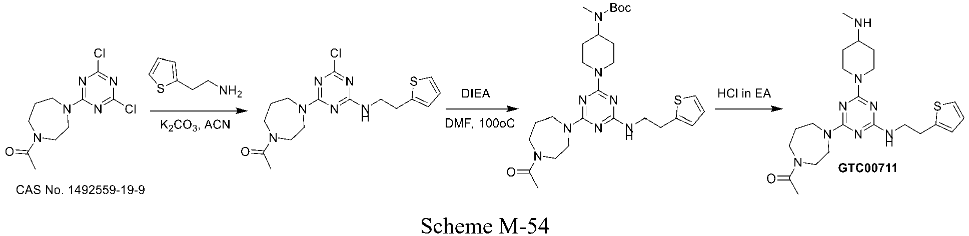

Depending upon the target compound, the reaction substrate, reaction conditions (including reaction dosage, temperature, time, etc.), and work up, etc. in Scheme M-54 can be appropriately modified and adjusted using techniques and methods well known to those skilled in the art to obtain the desired target compound. In Scheme M-54, the reductive amination can be conventional techniques and methods known to those skilled in the art. For example, the reductive amination can be carried out at room temperature in the presence of sodium triacetoxyborohydride/1,2-dichloroethane, or sodium cyanoborohydride/1,2-dichloroethane.

### General synthesis method 1 for compounds of Formula (I):

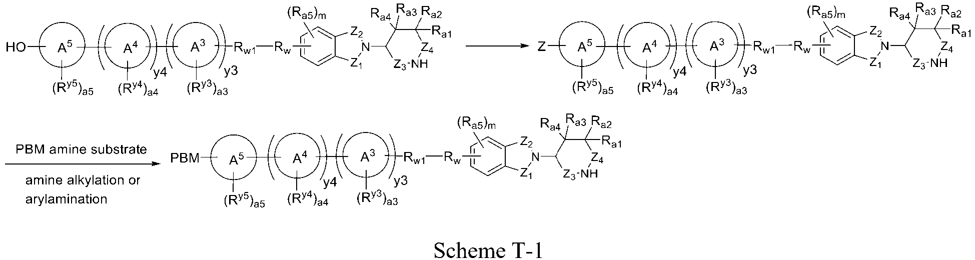

In Step 2 of Scheme T-1, the PBM amine substrate is a compound corresponding to the PBM moiety of the compound of Formula (I) of the present disclosure, containing aliphatic amine or heterocyclic amine (such as nitrogen-containing monocyclic heterocycle, nitrogen-containing bridged heterocycle, nitrogen-containing spiro heterocycle, or nitrogen-containing fused heterocycle) groups or amino compounds. The other substrate has Rₐ₁, Rₐ₂, Rₐ₃, Rₐ₄, Z₁, Z₂, Z₃, Z₄, (Rₐ₅)ₘ, R_{w}, R_{w1}, ring A³, ring A⁴, ring A⁵, y3, y4, (R^{y3})ₐ₃, (R^{y4})ₐ₄, and (R^{y5})ₐ₅ as defined in the Formula (I) compound herein. The group Z may be Br, Cl, I, OMs, OTs, OTf, or the like. (Rₐ₅)ₘ indicates that the benzene ring to which it is attached is substituted with m Rₐ₅ groups, with each Rₐ₅ being the same or different and independently representing halogen, hydroxy, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl or C₂₋₆ alkynyl; m represents an integer of 0, 1, 2, 3, or 4.

In some embodiments, when group Z in the substrate containing group Z in step 2 of Scheme T-1 is attached to an aryl group, step 2 is an arylamination reaction. This reaction may be conducted at 80-100°C in the presence of a palladium catalyst, a ligand, a base, and a solvent. Examples of the palladium catalyst include, but are not limited to, Pd₂(dba)₂. Examples of the ligand include, but are not limited to, XantPhos. Examples of the base include, but are not limited to, Cs₂CO₃. Examples of the solvent include, but are not limited to, 1,4-dioxane.

In some embodiments, when group Z in the substrate containing group Z in step 2 of Scheme T-1 is attached to an aliphatic cycloalkyl, heterocyclyl, or alkyl group, step 2 is an amine alkylation reaction. This reaction may be conducted at a temperature ranging from room temperature to 80°C (e.g., 40°C~60°C, 40°C~50°C, or 50°C~60°C) in the presence of, for example, an organic base and sodium iodide. Examples of the organic base include, but are not limited to, DIEA or triethylamine.

For example, the procedure for Scheme T-1 can be as follows:
Step 1: (1) When the group Z is OMs, OTs, or OTf, the alcohol substrate can undergo esterification with, for example, MsCl (methanesulfonyl chloride), TsCl (p-toluenesulfonyl chloride), or TfCl (trifluoromethanesulfonyl chloride) under basic conditions (such as TEA, DIEA) to obtain the corresponding target intermediate. Alternatively, (2) when the group Z is Br, Cl, or I, the alcohol substrate can undergo halogenation with, for example, CBr₄/PPh₃/DCM to obtain the corresponding target intermediate.
Step 2 (Amine Alkylation): to a solution of the PBM amine substrate (1 equiv) and target intermediate (1.1 equiv) from step 1 in anhydrous DMF were added sodium iodide (1.0 equiv) and DIEA (3.0 equiv). The reaction mixture was stirred at room temperature to 80°C for 0.5-24 hours. After monitoring the reaction via LCMS and confirming its completion, the reaction solution was filtered, and the filtrate was separated and purified by preparative HPLC to give the target compound.

### General synthesis method 2 for compounds of Formula (I):

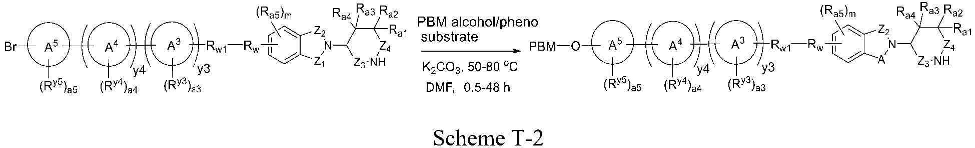

In Scheme T-2, the PBM alcohol/pheno substrate has the formula of PBM-OH. PBM, along with Rₐ₁, Rₐ₂, Rₐ₃, Rₐ₄, Z₁, Z₂, Z₃, Z₄, (Rₐ₅)ₘ, R_{w}, R_{w1}, ring A³, ring A⁴, ring A⁵, y3, y4, (R^{y3})ₐ₃, (R^{y4})ₐ₄, and (R^{y5})ₐ₅ of the other substrate, are as defined in the compound of Formula (I) of the present disclosure.

To a solution of the PBM alcohol/pheno substrate (1 equiv) and Bromo substrate (1.2 equiv) in DMF was added K₂CO₃ (5 equiv). The reaction mixture was stirred at 50-80°C for 0.5-48 hours. After monitoring the reaction via LCMS and confirming its completion, the reaction solution was filtered, and the filtrate was separated and purified by preparative HPLC to give the target compound.

### General synthesis method 3 for compounds of Formula (I):

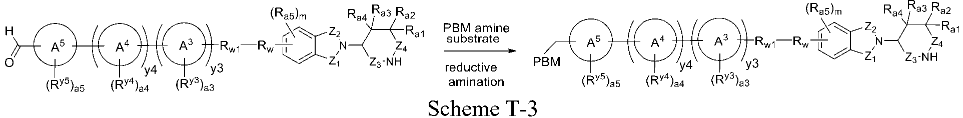

In Scheme T-3, the PBM amine substrate is a compound corresponding to the PBM moiety of the compound of Formula (I) of the present disclosure, containing aliphatic amine or heterocyclic amine (such as nitrogen-containing monocyclic heterocycle, nitrogen-containing bridged heterocycle, nitrogen-containing spiro heterocycle, or nitrogen-containing fused heterocycle) groups or amino compounds. PBM, along with Rₐ₁, Rₐ₂, Rₐ₃, Rₐ₄, Z₁, Z₂, Z₃, Z₄, (Rₐ₅)ₘ, R_{w}, R_{w1}, ring A³, ring A⁴, ring A³, y3, y4, (R^{y3})ₐ₃, (R^{y4})ₐ₄, and (R^{y5})ₐ₅ of the other aldehyde or ketone substrate, are as defined in the compound of Formula (I) of the present disclosure. It should be understood that when the PBM amine substrate and/or aldehyde or ketone substrate contains additional reactive groups (such as -NH or -NH₂) that can participate in the reaction of Scheme T-3, the reactive groups can be protected by techniques and methods known to those skilled in the art, such as using protecting groups.

In Scheme T-3, the reductive amination can be conventional techniques and methods known to those skilled in the art. For example, the reductive amination can be conducted at a temperature ranging from room temperature to 80 °C in the presence of sodium triacetoxyborohydride and 1,2-dichloroethane. Alternatively, the reaction may be carried out using sodium borohydride in 1,4-dioxane over the same temperature range.

For example, the procedure of Scheme T-3 can be performed as follows: to a solution of the corresponding substrate aldehyde or carbonyl compound (1.0 equiv) and the PBM substrate amine (1.0 equiv) in dichloromethane (DCM) was added an appropriate amount of acetic acid. The resulting reaction mixture was reacted at room temperature for 30 minutes, followed by addition of sodium cyanoborohydride (3.0 equiv), and the reaction was continued at room temperature. After monitoring the reaction via LCMS and confirming its completion, the reaction mixture was separated by using C₁₈ reverse phase column chromatography to give the target compound.

### General synthesis method 4 for compounds of Formula (I):

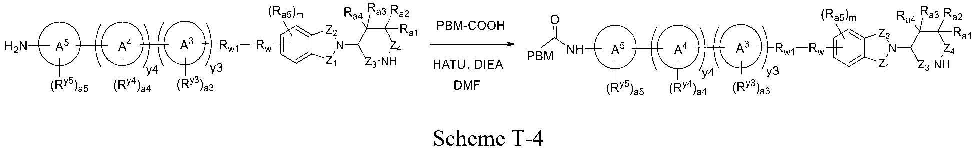

In Scheme T-4, the PBM, Rₐ₁, Rₐ₂, Rₐ₃, Rₐ₄, Z₁, Z₂, Z₃, Z₄, (Rₐ₅)ₘ, R_{w}, R_{w1}, ring A³, ring A⁴, ring A⁵, y3, y4, (R^{y3})ₐ₃, (R^{y4})ₐ₄, and (R^{y5})ₐ₅ are as defined herein for the Formula (I) compound. The condensation reaction in Scheme T-4 can follow conventional techniques and methods that are well-known to those skilled in the art. For example, it can be carried out using conventional condensing agents such as HOAt/EDCI, T₃P, etc., under alkaline conditions (e.g., in the presence of DMAP) at room temperature or under heating.

### General synthesis method 5 for compounds of Formula (I):

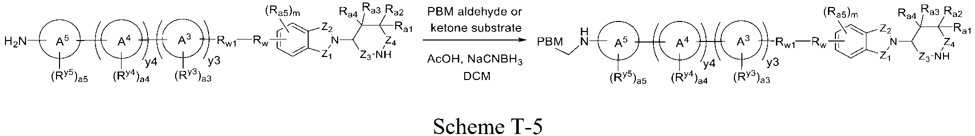

In Scheme T-5, the PBM, Rₐ₁, Rₐ₂, Rₐ₃, Rₐ₄, A, (Rₐ₅)ₘ, R_{w}, R_{w1}, ring A², ring A⁴, ring A⁵, y3, y4, (R^{y3})ₐ₃, (R^{y4})ₐ₄, and (R^{y5})ₐ₅ are as defined herein for the Formula (I) compound.

The reductive amination reaction in Scheme T-5 can follow conventional techniques and methods that are known to those skilled in the art. For example, the reductive amination reaction can be carried out at temperatures ranging from room temperature to 80°C in the presence of sodium borohydride acetate and 1,2-dichloroethane.

For example, to a solution of the corresponding PBM aldehyde substrate (1.0 equivalent) and amine substrate (1.0 equivalent) in dichloromethane was added an appropriate amount of acetic acid. The resulting reaction mixture was allowed to react at room temperature for 30 minutes, followed by the addition of sodium cyanoborohydride (3.0 equivalents). The reaction was then continued at room temperature until completion, as monitored by LCMS. After the reaction was complete, the product was separated via C₁₈ reverse-phase column chromatography. The collected fractions were rotary evaporated to remove acetonitrile, and the resulting residue was lyophilized to give the target compound.

### General synthesis method 1 for compounds of Formula (II):

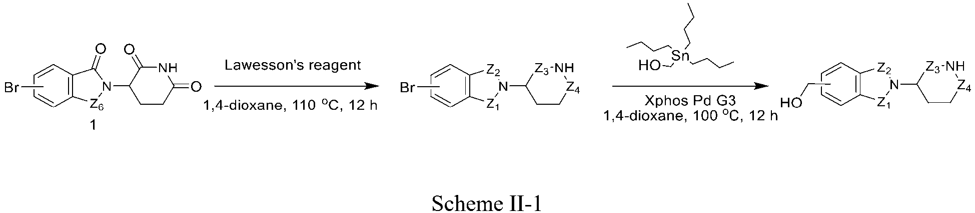

In Scheme II-1, Z₆ represents C(O), CH₂ or CD₂; Z₁ represents C(O), C(S), CH₂ or CD₂; Z₂, Z₃, and Z₄ each independently represent C(O) or C(S); wherein at least one of Z₁, Z₂, Z₃, and Z₄ represents C(S).

In some embodiments, at least two of Z₁, Z₂, Z₃, and Z₄ in the product of step 1 represent C(S). In some embodiments, at least three of Z₁, Z₂, Z₃, and Z₄ represent C(S). In some embodiments, all of Z₁, Z₂, Z₃, and Z₄ represent C(S).

### General synthesis method 2 for compounds of Formula (II):

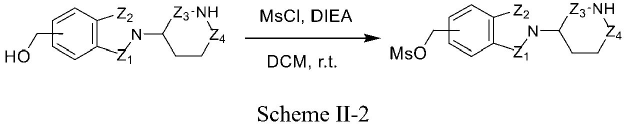

In Scheme II-2, Z₁ represents C(O), C(S), CH₂ or CD₂; Z₂, Z₃, and Z₄ each independently represent C(O) or C(S), wherein at least one of Z₁, Z₂, Z₃, and Z₄ represents C(S).

The sulfonic esterification in Scheme II-2 can be performed using conventional techniques and methods well-known to a person skilled in the art. For example, the sulfonic esterification may be carried out at a temperature ranging from room temperature to 80 °C in the presence of Ms₂O, TEA, DMF, and DCM.

For example, to a solution of the alcohol substrate (1.0 eq.) in DMF and DCM were added Ms₂O (1.2 eq.) and TEA (3.0 eq.). The resulting reaction mixture was allowed to react at room temperature for 30 minutes. After monitoring the reaction via TLC and confirming its completion, the reaction mixture was concentrated under reduced pressure to give the target compound.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

### Examples

### Intermediate Example 1: preparation of 1-((3S,4R)-3-fluoropiperidin-4-yl)-3-(4-phenoxyphenyl)-1H-pyrazolo[3,4-d]pyrimidin-4-amine

1-((3S,4R)-3-fluoropiperidin-4-yl)-3-(4-phenoxyphenyl)-1H-pyrazolo[3,4-d]pyrimidin-4-amine was prepared by referring to the method of Scheme M-1.

Step 1: to a solution of tert-butyl (3S,4S)- 3-fluoro-4-hydroxypiperidine-1-carboxylate (370 mg, 1.69 mmol) in anhydrous dichloromethane were added DIEA (0.5 ml, 3.38 mmol), p-toluenesulfonyl chloride (386 mg, 2.03 mmol), and DMAP (206.16 mg, 1.69 mmol). The reaction mixture was stirred at room temperature for 18 hours, and the reaction was completed as detected by TLC. The reaction mixture was washed with H₂O (50 mL) and extracted with DCM (50 mL x 3). The combined organic phases were dried over anhydrous sodium sulfate, filtered, and the filtrate was concentrated under reduced pressure. The residue was purified by column chromatography (eluent (v/v): PE/EtOAc = 1/1) to give the white solid compound tert-butyl (3S,4S)-3-fluoro-4-(tosyloxy)piperidine-1-carboxylate (350 mg, yield 55%).

Step 2: to a solution of the compound tert-butyl (3S,4S)-3-fluoro-4-(tosyloxy)piperidine-1-carboxylate (350 mg, 0.94 mmol) in anhydrous DMSO (20 mL) were added 3-(4-phenoxyphenyl)-1H-pyrazolo[3,4-d]pyrimidin-4-amine (284 mg, 0.94 mmol) and K₂CO₃ (388 mg, 2.81 mmol). The reaction mixture was heated to 85°C and stirred for 18 hours, and the reaction was completed as detected by TLC. The reaction mixture was cooled to room temperature, washed with H₂O (100 mL), and extracted with EtOAc (100 mL x 3). The combined organic phases were dried over anhydrous sodium sulfate, filtered, and the filtrate was concentrated under reduced pressure. The residue was purified by column chromatography with eluent (DCM/MeOH = 20/1) to give the white solid compound tert-butyl (3S,4R)-4-(4-amino-3-(4-phenoxyphenyl)-1H-pyrazolo[3,4-d]pyrimidin-1-yl)-3-fluoropiperidine-1-carboxylate (350 mg, yield 74%). LC/MS (ESI, *m*/*z*) 505.3 [M + H]⁺.

Step 3: to a solution of the compound tert-butyl (3S,4R)-4-(4-amino-3-(4-phenoxyphenyl)-1H-pyrazolo[3,4-d]pyrimidin-1-yl)-3-fluoropiperidine-1-carboxylate (350 mg, 0.69 mmol) in anhydrous DCM (15 mL) was added HCl/dioxane (1.7 mL, 4M). The reaction mixture was stirred at room temperature for 1 hour, and the reaction was completed as detected by TLC. The reaction mixture was concentrated under reduced pressure to give the white solid target compound 1-((3S,4R)-3-fluoropiperidin-4-yl)-3-(4-phenoxyphenyl)-1H-pyrazolo[3,4-d]pyrimidin-4-amine (205 mg, yield 67%). ¹H NMR (400 MHz, DMSO) δ 8.24 (d, J = 5.6 Hz, 1H), 7.69 (d, J = 8.6 Hz, 2H), 7.44 (dd, J = 8.4, 7.6 Hz, 2H), 7.28 - 7.04 (m, 5H), 5.15 - 4.76 (m, 2H), 3.34 (d, J = 7.3 Hz, 1H), 2.97 (d, J = 12.3 Hz, 1H), 2.63 (ddd, J = 15.0, 12.2, 3.5 Hz, 2H), 2.12 (dd, J = 12.3, 4.1 Hz, 1H), 1.94 (d, J = 12.7 Hz, 1H). ¹⁹F NMR (377 MHz, DMSO) δ -185.23 (s). LC/MS (ESI, m/z) calcd for C₂₂H₂₂FN₆O⁺ [M + H]⁺: 405.2; found, 405.1.

### Intermediate Example 2: preparation of 3-(4-phenoxyphenyl)-1-(piperidin-4-yl)-1H-pyrazolo[3,4-d]pyrimidin-4-amine

3-(4-phenoxyphenyl)-1-(piperidin-4-yl)-1H-pyrazolo[3,4-d]pyrimidin-4-amine hydrochloride was prepared by referring to the method of Scheme M-2.

**Step 1:** to a solution of triphenylphosphine (154.59 g, 574.1 mmol) in tetrahydrofuran (3 L) was added DIAD (117.54 g, 581.3 mmol) at 0°C. After addition, the mixture was stirred for 0.5 hours. Then, to the mixture was added 2-methylpropan-2-yl 4-hydroxypiperidine-1-carboxylate (118.6 g, 589.3 mmol) at 0°C, and the mixture was stirred for 0.5 hours. Subsequently, to the reaction solution was added 3-[4-(phenoxy)phenyl]-1H-pyrazolo[3,4-d]pyrimidin-4-amine (90 g, 296.7 mmol), and the temperature was slowly raised to 25°C and stirred for 16 hours. TLC analysis showed the completion of the reaction. The reaction mixture was concentrated under vacuum to obtain the crude product. The crude product was purified by silica gel flash column chromatography (eluent (v/v): PE/EtOAc = 1/0-1/1) to obtain the white solid compound tert-butyl 4-(4-amino-3-(4-phenoxyphenyl)-1H-pyrazolo[3,4-d]pyrimidin-1-yl)piperidine-1-carboxylate (92.0 g, 189.3 mmol, yield 63.8%). LCMS (ESI) calcd. for C₂₇H₃₁N₆O₃⁺[M+H]⁺: 487.24; found, 487.30.

**Step 2:** tert-butyl 4-(4-amino-3-(4-phenoxyphenyl)-1H-pyrazolo[3,4-d]pyrimidin-1-yl)piperidine-1-carboxylate (92.0 g, 189.3 mmol) was dissolved in HCl/EtOAc (340 mL, 3 M) at 10°C. The mixture was stirred at room temperature for 3 hours. TLC analysis showed the completion of the reaction. A large amount of white solid precipitated, which was filtered and ground with ethyl acetate/petroleum ether (1: 8, 300 ml) and filtered again. The solid was dried under high vacuum to obtain the white solid compound 4-(4-amino-3-(4-phenoxyphenyl)-1H-pyrazolo[3,4-d]pyrimidin-1-yl)piperidine hydrochloride (60 g, 154.957 mmol, yield 52%). ¹H NMR (400 MHz, DMSO) δ 8.25 (s, 1H), 7.69 - 7.61 (m, 2H), 7.47 - 7.39 (m, 2H), 7.23 - 7.09 (m, 6H), 4.98 - 4.89 (m, 1H), 3.35 - 3.26 (m, 3H), 2.99 (t, *J =* 12.1 Hz, 2H), 2.35 - 2.19 (m, 2H), 2.07 - 1.98 (m, 2H). LCMS (ESI) calcd. for C₂₂H₂₃N₆O⁺ [M+H]⁺: 387.19; found, 387.20.

### Intermediate Example 3: preparation of tert-butyl 4-(2-(4-aminophenoxy)ethyl)piperazine-1-carboxylate

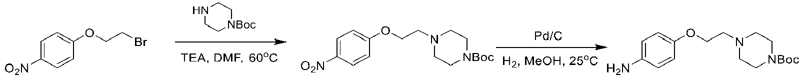

**Step 1:** To a solution of 1-(2-bromoethoxy)-4-nitrobenzene (4.50 g, 18.288 mmol) in N,N-dimethylformamide (100 mL) was added TEA (3.66 g, 36.576 mmol), and the reaction mixture was stirred at 60°C for 30 minutes. TLC analysis showed the completion of the reaction. The reaction mixture was diluted with EtOAc (100 mL) and ice water (100 mL). The organic layer was separated and further washed with saturated brine (100 mL), then concentrated under vacuum. The residue was purified by silica gel column chromatography (eluent (v/v): PE/EtOAc = 1/0-0/1) to obtain the colorless oily compound tert-butyl 4-(2-(4-nitrophenoxy)ethyl)piperazine-1-carboxylate (4 g, 11.383 mmol, yield 62.24%). ¹H NMR (300 MHz, CDCl₃) δ 8.19 (2H, m), 6.97 (2H, m), 4.19 (t, J = 5.6; LCMS (ESI) calcd. for C₁₇H₂₆N₃O₅⁺ [M+H]⁺: 352.19(M) 351.18, found, (M+H)⁺ 352.30.

**Step 2:** To a solution of tert-butyl 4-(2-(4-nitrophenoxy)ethyl)piperazine-1-carboxylate (4.00 g, 11.383 mmol) in MeOH (50 mL) was added Pd/C (0.12 g, 1.138 mmol, 10%), and the reaction was stirred under a hydrogen balloon at room temperature for 16 hours. LCMS analysis showed the completion of the reaction. The mixture was filtered through a celite bed and washed with methanol (20 mL). The filtrate was concentrated to obtain the off-white solid tert-butyl 4-(2-(4-aminophenoxy)ethyl)piperazine-1-carboxylate (3.1 g, yield 84.73%). LCMS (ESI) calcd. for C₁₇H₂₈N₃O₃⁺ [M+H]⁺: (M) 322.21, found, (M+H)⁺ 322.20.

### Intermediate Example 4: preparation of tert-butyl 4-(6-aminopyridazin-3-yl)piperazine-1-carboxylate

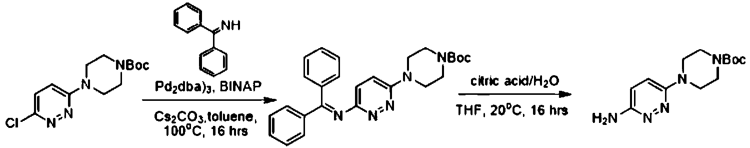

**Step 1:** Under argon protection, to a solution of tert-butyl 4-(6-chloropyridazin-3-yl)piperazine-1-carboxylate (3.00 g, 10.041 mmol) in toluene (60 mL) were added diphenylmethanimine (2.022 mL, 12.049 mmol), BINAP (0.06 g, 0.100 mmol), Cs₂CO₃ (6.54 g, 20.082 mmol) and Pd₂(dba)₃ (0.46 g, 0.502 mmol). The resulting suspension was stirred at 100°C for 16 hours. LCMS analysis showed the completion of the reaction. The reaction mixture was cooled to room temperature, then filtered through celite and washed with ethyl acetate (30 mL). The filtrate was washed with saturated brine (20 mL), dried over anhydrous magnesium sulfate, and filtered to separate the solid. The filtrate was concentrated under reduced pressure, and the resulting residue was purified by silica gel column chromatography to obtain the white solid compound tert-butyl 4-(6-((diphenylmethylene)amino)pyridazin-3-yl)piperazine-1-carboxylate (2.60 g, 5.862 mmol, yield 58.38%). LCMS (ESI) calcd. for C₂₆H₃₀N₅O₂⁺ [M+H]⁺: 443.20; found, [M+H]⁺=444.20.

**Step** 2: To a solution of tert-butyl 4-(6-((diphenylmethylene)amino)pyridazin-3-yl)piperazine-1-carboxylate (2.60 g, 5.862 mmol) in THF (50 mL) was added citric acid/H₂O (20 mL), and the mixture was stirred at 20°C for 16 hours. LCMS analysis showed the completion of the reaction. The reaction mixture was concentrated under vacuum to remove THF. The resulting aqueous phase was adjusted to pH = 8 with saturated NaHCO₃ and extracted with EtOAc (100 mL × 3). The combined organic phases were dried over Na₂SO₄ and concentrated under reduced pressure to obtain the white solid tert-butyl 4-(6-aminopyridazin-3-yl)piperazine-1-carboxylate (1.6 g, yield 97.71%). LCMS (ESI) calcd. for C₁₃H₂₂N₅O₂⁺ [M+H]⁺: M: 280.18279.20; found, [M+H]⁺ =280.20.

### Intermediate Example 5: preparation of 6-(4-(tert-butoxycarbonyl)piperazin-1-yl)pyridazine-3-carboxylic acid

6-(4-(tert-butoxycarbonyl)piperazin-1-yl)pyridazine-3-carboxylic acid was prepared by referring to the method of Scheme M-4.

**Step 1:** to a solution of methyl 6-Chloropyridazine-3-carboxylate (500.00 mg, 2.897 mmol) and tert-butyl piperazine-1-carboxylate (809.45 mg, 4.346 mmol) in DMF (10.00 mL) was added DIEA (1.437 mL, 8.692 mmol), and the reaction mixture was stirred at 60°C for 12 h. LC-MS analysis showed completion of the reaction. Water was added to the reaction mixture, which was then extracted with ethyl acetate. The organic phase was separated, washed with saturated brine, and concentrated to obtain a crude product. The crude product was purified by chromatography (eluent (v/v): PE/EA = 5/1) to yield methyl 6-(4-(tert-butoxycarbonyl)piperazin-1-yl)pyridazine-3-carboxylate (900 mg, yield 75.16%) as a white solid. LCMS (ESI) calcd. for C₁₅H₂₃N₄O₄⁺ [M+H]⁺: 323.16, found, 323.20.

**Step 2:** to a solution of methyl 6-(4-(tert-butoxycarbonyl)piperazin-1-yl)pyridazine-3-carboxylate (400 mg, 1.241 mmol) in a mixed solvent of 1,4-dioxane and water (1/1; 10 mL) was added Lithium hydroxide (260.32 mg, 6.204 mmol), and the reaction mixture was stirred at 60°C for 12 h. LC-MS analysis showed completion of the reaction. The reaction mixture was concentrated under reduced pressure to remove the organic phase. The remaining aqueous phase was adjusted to pH = 6 with concentrated hydrochloric acid and then extracted with ethyl acetate. The organic phase was separated, concentrated under reduced pressure to obtain 6-(4-(tert-butoxycarbonyl)piperazin-1-yl)pyridazine-3-carboxylic acid (300 mg, yield 76.06%) as a white solid, which can be used for the next step without further purification. LCMS (ESI) calcd. for C₁₄H₂₁N₄O₄⁺ [M+H]⁺: 309.15, found, 309.20.

### Intermediate Example 6: preparation of N-((1r,4r)-4-(3-chloro-4-cyanophenoxy)cyclohexyl)-6-(piperazin-1-yl)pyridazine-3-carboxamide

N-((1r,4r)-4-(3-chloro-4-cyanophenoxy)cyclohexyl)-6-(piperazin-1-yl)pyridazine-3-carboxamide was prepared by referring to the method of Scheme M-5.

To a solution of 6-(4-(tert-butoxycarbonyl)piperazin-1-yl)pyridazine-3-carboxylic acid (300 mg, 1.135 mmol) prepared from Intermediate Example 5 and 4-(((1r,4r)-4-aminocyclohexyl)oxy)-2-chlorobenzonitrile (326.61 mg, 1.135 mmol) in DMF (5.00 mL) were added HATU (647.40 mg, 1.703 mmol) and DIEA (0.938 mL, 5.676 mmol), and the resulting reaction mixture was stirred at room temperature for 30 minutes. LC-MS analysis showed completion of the reaction. Water was added to the reaction mixture, which was then extracted with ethyl acetate. The organic phase was separated, washed with saturated brine, and concentrated to obtain a crude product. The crude product was purified by chromatography (eluent (v/v): DCM/MeOH = 100/1) to yield the white solid compound tert-butyl 4-(6-(((1r,4r)-4-(3-chloro-4-cyanophenoxy)cyclohexyl)carbamoyl)pyridazin-3-yl)piperazine-1-carboxylate (250 mg, yield 39.49%). LCMS (ESI) calcd. for C₂₇H₃₄ClN₆O₄⁺ [M+H]⁺: 541.23, found, 541.30.

Tert-butyl 4-(6-(((1r,4r)-4-(3-chloro-4-cyanophenoxy)cyclohexyl)carbamoyl)pyridazin-3-yl)piperazine-1-carboxylate (250 mg, 0.462 mmol) was dissolved in a mixed solvent of DMF (5.00 mL) and TFA (1.00 mL). The reaction mixture was stirred at room temperature for 3 h. LC-MS analysis showed completion of the reaction. The reaction mixture was concentrated under reduced pressure to remove the organic phase. The remaining aqueous phase was adjusted to pH = 8 with saturated NaHCO₃ solution and then extracted with ethyl acetate. The organic phase was separated, concentrated under reduced pressure to obtain N-((1r,4r)-4-(3-chloro-4-cyanophenoxy)cyclohexyl)-6-(piperazin-1-yl)pyridazine-3-carboxamide (170 mg, yield 75.10%) as a white solid, which can be used for the next step without further purification. LCMS (ESI) calcd. for C₂₂H₂₆ClN₆O₂⁺ [M+H]⁺: 441.17, found, 441.20.

### Intermediate Example 7: preparation of 5-chloro-N²-(2-isopropoxy-5-methyl-4-(piperazin-1-yl)phenyl)-N⁴-(2-(isopropylsulfonyl)phenyl)pyrimidine-2,4-diamine (GT-D-86)

5-chloro-N²-(2-isopropoxy-5-methyl-4-(piperazin-1-yl)phenyl)-N⁴-(2-(isopropylsulfonyl)phenyl)pyrimidine-2,4-diamine (GT-D-86) was prepared by referring to Scheme M-8 (brown solid, 2.46 g). ¹H NMR (400 MHz, DMSO) δ 9.51 (s, 1H), 9.14 (s, 2H), 8.43 (d, *J =* 8.3 Hz, 1H), 8.26 (s, 1H), 8.20 (s, 1H), 7.85 (dd, *J =* 7.9, 1.2 Hz, 1H), 7.63 (t, *J =* 7.7 Hz, 1H), 7.51 (s, 1H), 7.37 (t, *J =* 7.5 Hz, 1H), 6.70 (s, 1H), 4.56 (dd, *J =* 12.0, 6.0 Hz, 1H), 3.23 (s, 4H), 3.04 (d, *J =* 4.3 Hz, 4H), 2.09 (s, 3H), 1.19 (dd, *J* = 24.2, 6.4 Hz, 12H). LCMS (ESI) calcd. for C₂₇H₃₆ClN₆O₃S⁺ [M+H]⁺: 559.2; found, 559.1.

### Intermediate Example 8: preparation of 5-chloro-N²-(2-isopropoxy-5-methyl-4-(4-(methylamino)piperidin-1-yl)phenyl)-N⁴-(2-(isopropylsulfonyl)phenyl)pyrimidine-2,4-diamine (GT-D-87)

5-chloro-N²-(2-isopropoxy-5-methyl-4-(4-(methylamino)piperidin-1-yl)phenyl)-N⁴-(2-(isopropylsulfonyl)phenyl)pyrimidine-2,4-diamine (GT-D-87) was prepared by referring to Scheme M-8 (white solid, 1.2 g). ¹H NMR (400 MHz, DMSO) δ 9.93 (s, 1H), 9.19 (s, 2H), 8.93 (s, 1H), 8.42 (s, 1H), 8.20 (s, 1H), 7.90 (s, 1H), 7.73 (t, *J =* 7.7 Hz, 1H), 7.51 (t, *J =* 7.7 Hz, 1H), 7.39 (s, 1H), 6.81 (s, 1H), 4.71 - 4.36 (m, 1H), 3.48 (dt, *J =* 13.4, 6.7 Hz, 1H), 2.57 (t, *J =* 5.3 Hz, 3H), 2.13 (s, 2H), 2.04 (s, 3H), 1.82 (s, 2H), 1.24 (d, *J* = 6.0 Hz, 6H), 1.14 (d, *J* = 6.8 Hz, 6H). LCMS (ESI) calcd. for C₂₉H₄₀ClN₆O₃S⁺ [M+H]⁺: 587.3; found, 587.3.

### Intermediate Example 9: preparation of 5-chloro-N²-(2-isopropoxy-5-methyl-4-(4-(piperazin-1-yl)piperidin-1-yl)phenyl)-N⁴-(2-(isopropylsulfonyl)phenyl)pyrimidine-2,4-diamine (GT-D-88)

5-chloro-N²-(2-isopropoxy-5-methyl-4-(4-(piperazin-1-yl)piperidin-1-yl)phenyl)-N⁴-(2-(isopropylsulfonyl)phenyl)pyrimidine-2,4-diamine (GT-D-88) was prepared by referring to Scheme M-8 (brown solid, 1.2 g). ¹H NMR (400 MHz, DMSO) δ 9.47 (s, 1H), 8.46 (d, *J =* 7.9 Hz, 1H), 8.24 (s, 1H), 8.09 (s, 1H), 7.83 (d, *J* = 7.9 Hz, 1H), 7.61 (t, *J =* 7.8 Hz, 1H), 7.49 (s, 1H), 7.35 (t, *J =* 7.7 Hz, 1H), 6.70 (s, 1H), 4.60 - 4.42 (m, 1H), 3.54 - 3.36 (m, 8H), 3.26 - 3.06 (m, 4H), 2.66 (t, *J =* 11.1 Hz, 2H), 2.15 (d, *J =* 11.0 Hz, 1H), 2.09 (s, 3H), 1.79 (s, 2H), 1.26 (dd, *J* = 13.1, 7.2 Hz, 3H), 1.22 - 1.09 (m, 9H). LCMS (ESI) calcd. for C₃₂H₄₅ClN₇O₃S⁺ [M+H]⁺: 642.3; found, 642.3.

### Intermediate Example 10: preparation of (2-((5-chloro-2-((2-methoxy-4-(piperazin-1-yl)phenyl)amino)pyrimidin-4-yl)amino)phenyl)dimethylphosphine oxide (GT-D-39)

(2-((5-chloro-2-((2-methoxy-4-(piperazin-1-yl)phenyl)amino)pyrimidin-4-yl)amino)phenyl)dimethylphosphine oxide (GT-D-39) was prepared by referring to Scheme M-8 (yellow solid, 7.9 g). ¹H NMR (400 MHz, DMSO) δ 11.57 (s, 1H), 9.55 (s, 1H), 9.17 (s, 2H), 8.76 (s, 1H), 8.44 (s, 1H), 8.17 (s, 1H), 7.59 (dd, *J=* 13.3, 7.7 Hz, 1H), 7.50 - 7.30 (m, 2H), 7.17 (t, *J=* 7.3 Hz, 1H), 6.73 (d, *J= 2.1* Hz, 1H), 6.56 (dd, *J =* 8.6, 2.2 Hz, 1H), 3.79 (s, 3H), 3.49 - 3.36 (m, 4H), 3.26 (m, 4H), 1.80 (s, 3H), 1.77 (s, 3H). LCMS (ESI) calcd. for C₂₃H₂₉ClN₆O₂P⁺ [M+H]⁺: 487.2; found, 487.2.

### Intermediate Example 11: preparation of 2-((2-((2-methoxy-4-(piperazin-1-yl)phenyl)amino)-5-(trifluoromethyl)pyridin-4-yl)amino)-N-methylbenzamide (GT-M-10)

2-((2-((2-methoxy-4-(piperazin-1-yl)phenyl)amino)-5-(trifluoromethyl)pyridin-4-yl)amino)-N-methylbenzamide (GT-M-10) was prepared by referring to Scheme M-8 (white solid, 1.87 g). ¹H NMR (400 MHz, MeOD) δ 8.01 (s, 1H), 7.73 (d, J = 7.9 Hz, 1H), 7.59 (d, J = 3.8 Hz, 2H), 7.36 (dt, J = 8.4, 4.1 Hz, 1H), 7.18 (d, J = 8.6 Hz, 1H), 6.79 (d, J = 2.1 Hz, 1H), 6.68 (dd, J = 8.7, 2.2 Hz, 1H), 6.48 (s, 1H), 3.87 (s, 3H), 3.66 (s, 4H), 3.56 - 3.48 (m, 4H), 3.43 - 3.37 (m, 4H), 2.91 (d, J = 13.7 Hz, 3H). LCMS (ESI) calcd. for C₂₅H₂₈F₃N₆O₂⁺ [M+H]⁺: 501.2; found, 501.2.

### Intermediate Example 12: preparation of 2-((2-((4-(4-aminopiperidin-1-yl)-2-methoxyphenyl)amino)-5-(trifluoromethyl)pyridin-4-yl)amino)-N-methylbenzamide (GT-M-11)

2-((2-((4-(4-aminopiperidin-1-yl)-2-methoxyphenyl)amino)-5-(trifluoromethyl)pyridin-4-yl)amino)-N-methylbenzamide (GT-M-11) was prepared by referring to Scheme M-8 (brown solid, 0.4 g). ¹H NMR (400 MHz, DMSO) δ 10.13 (s, 1H), 8.63 (d, J = 4.2 Hz, 1H), 8.27 (s, 1H), 8.14 (s, 1H), 7.74 - 7.60 (m, 1H), 7.44 (dd, J = 6.3, 1.3 Hz, 2H), 7.36 (d, J = 8.7 Hz, 1H), 7.08 - 7.00 (m, 1H), 6.57 (d, J = 2.5 Hz, 1H), 6.51 (s, 1H), 6.43 (dd, J = 8.8, 2.5 Hz, 1H), 3.77 (s, 3H), 3.57 (d, J = 12.6 Hz, 3H), 2.75 (d, J = 3.7 Hz, 4H). LCMS (ESI) calcd. for C₂₆H₃₀F₃N₆O₂⁺ [M+H]⁺: 515.2; found, 515.2.

### Intermediate Example 13: preparation of 2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12)

2-((2-((2-methoxy-4-(4-(piperazin-1-yl)piperidin-1-yl)phenyl)amino)-5-(trifluoromethyl)pyridin-4-yl)amino)-N-methylbenzamide (GT-M-12) was prepared by referring to Scheme M-8 (purple solid, 0.34 g). ¹H NMR (400 MHz, DMSO) δ 10.60 (s, 1H), 10.10 (s, 1H), 9.99 (s, 1H), 9.75 (s, 1H), 8.82 (d, J = 4.6 Hz, 1H), 8.14 (s, 1H), 7.77 (d, J *=* 7.7 Hz, 1H), 7.57 (d, J = 3.2 Hz, 2H), 7.33 - 7.26 (m, 1H), 7.19 (s, 1H), 6.81 (s, 1H), 6.68 (s, 1H), 6.54 (s, 1H), 3.78 (d, J = 26.5 Hz, 15H), 3.53 (s, 9H), 2.84 (s, 2H), 2.76 (d, J = 4.5 Hz, 3H), 2.22 (d, J = 10.6 Hz, 2H), 1.87 (d, J = 9.5 Hz, 2H), 1.23 (s, 2H). LCMS (ESI) calcd. for C₃₀H₃₇F₃N₇O₂⁺ [M+H]⁺: 584.3; found, 584.0.

### Intermediate Example 14: preparation of 2-((2-((2-methoxy-4-(piperazin-1-yl)phenyl)amino)-5-(trifluoromethyl)pyridin-4-yl)amino)-N-methylbenzamide (GT-M-171)

2-((2-((2-methoxy-4-(piperazin-1-yl)phenyl)amino)-5-(trifluoromethyl)pyridin-4-yl)amino)-N-methylbenzamide (GT-M-171) was prepared by referring to Scheme M-8 (yellow solid, 20.5 g). ¹H NMR (400 MHz, DMSO) δ 10.39 (s, 1H), 9.20 (s, 1H), 8.80 (s, 2H), 8.69 (d, *J =* 4.6 Hz, 1H), 8.13 (s, 1H), 7.73 (d, *J =* 7.7 Hz, 1H), 7.53 (s, 2H), 7.35 (d, *J =* 8.6 Hz, 1H), 7.20 (s, 1H), 6.70 (d, *J =* 2.2 Hz, 1H), 6.59 - 6.49 (m, 2H), 3.80 (s, 3H), 3.36 (d, *J =* 5.2 Hz, 4H), 3.25 (s, 4H), 2.76 (d, *J =* 4.5 Hz, 3H). LCMS (ESI) calcd. for C₂₅H₂₈F₃N₆O₂⁺ [M+H]⁺: 501.2; found, 501.3.

### Intermediate Example 15: preparation of 2-((2-((2-methoxy-4-(4-(methylamino)piperidin-1-yl)phenyl)amino)-5-(trifluoromethyl)pyridin-4-yl)amino)-N-methylbenzamide (GT-M-172)

2-((2-((2-methoxy-4-(4-(methylamino)piperidin-1-yl)phenyl)amino)-5-(trifluoromethyl)pyridin-4-yl)amino)-N-methylbenzamide (GT-M-172) was prepared by referring to Scheme M-8 (white solid, 1.8 g). ¹H NMR (400 MHz, DMSO) δ 10.60 (s, 1H), 9.98 (s, 1H), 9.38 (s, 2H), 8.83 (d, *J =* 4.5 Hz, 1H), 8.17 (s, 1H), 7.78 (d, *J =* 7.8 Hz, 1H), 7.57 (d, *J =* 3.9 Hz, 2H), 7.34 - 7.15 (m, 2H), 6.87 (d, *J =* 54.9 Hz, 2H), 6.59 (s, 1H), 3.82 (s, 5H), 3.21 (s, 1H), 2.98 (s, 2H), 2.69 (t, *J =* 41.5 Hz, 4H), 2.54 (t, *J =* 5.3 Hz, 3H), 2.17 (s, 2H), 1.84 (s, 2H). LCMS (ESI) calcd. for C₂₇H₃₂F₃N₆O₂⁺ [M+H]⁺: 529.3; found, 529.3.

### Intermediate Example 16: preparation of N-methyl-N-(3-(((2-((4-(piperazin-1-yl)phenyl)amino)-5-(trifluoromethyl)pyrimidin-4-yl)amino)methyl)pyrazin-2-yl)methanesulfonamide (GT-M-151)

N-methyl-N-(3-(((2-((4-(piperazin-1-yl)phenyl)amino)-5-(trifluoromethyl)pyrimidin-4-yl)amino)methyl)pyrazin-2-yl)methanesulfonamide (GT-M-151) was prepared by referring to Scheme M-11 (white solid, 1.7 g). ¹H NMR(400 MHz, MeOD) δ 8.60 (s, 1H), 8.56 (s, 1H), 8.30 (s, 1H), 7.41 (d, *J =* 8.3 Hz, 2H), 7.20 (d, *J =* 8.6 Hz, 2H), 5.13 (s, 2H), 3.59 (d, *J =* 4.7 Hz, 4H), 3.49 (s, 4H), 3.20 (d, *J =* 16.1 Hz, 3H), 3.13 (s, 3H). LCMS (ESI) calcd. for C₂₂H₂₇F₃N₉O₂S⁺ [M+H]⁺: 538.20; found, 538.3.

### Intermediate Example 17: preparation of N-methyl-N-(3-(((2-((4-(piperazin-1-yl)phenyl)amino)-5-(trifluoromethyl)pyrimidin-4-yl)amino)methyl)phenyl)methanesulfonamide (GT-M-152)

N-methyl-N-(3-(((2-((4-(piperazin-1-yl)phenyl)amino)-5-(trifluoromethyl)pyrimidin-4-yl)amino)methyl)phenyl)methanesulfonamide (GT-M-152) was prepared by referring to Scheme M-11 (brown solid, 1.2 g). ¹H NMR (400 MHz, DMSO) δ 10.92 (s, 1H), 9.52 (s, 2H), 9.06 (s, 1H), 8.48 (s, 1H), 7.43 - 7.25 (m, 6H), 7.18 (s, 1H), 6.97 (d, J = 8.3 Hz, 2H), 4.64 (d, J = 5.2 Hz, 2H), 3.37 (s, 4H), 3.20 (s, 4H), 3.15 (s, 3H), 2.87 (s, 3H). LCMS (ESI) calcd. for C₂₄H₂₉F₃N₇O₂S⁺ [M+H]⁺: 536.2; found, 536.7.

### Intermediate Example 18: preparation of N-methyl-N-(3-(((2-((4-(4-(methylamino)piperidin-1-yl)phenyl)amino)-5-(trifluoromethyl)pyrimidin-4-yl)amino)methyl)phenyl)methanesulfonamide (GT-M-153)

N-methyl-N-(3-(((2-((4-(4-(methylamino)piperidin-1-yl)phenyl)amino)-5-(trifluoromethyl)pyrimidin-4-yl)amino)methyl)phenyl)methanesulfonamide (GT-M-153) was prepared by referring to Scheme M-11 (white solid, 1.069 g). ¹H NMR (400 MHz, MeOD) δ 8.36 (d, *J =* 22.7 Hz, 1H), 7.77 (t, *J =* 8.4 Hz, 2H), 7.55 (d, *J* = 8.5 Hz, 2H), 7.47 (t, *J =* 7.9 Hz, 1H), 7.35 (d, *J =* 7.8 Hz, 1H), 7.26 (s, 2H), 7.14 (s, 1H), 4.75 (s, 2H), 3.82 (dd, *J* = 24.4, 12.5 Hz, 4H), 3.63 (d, *J* = 11.4 Hz, 1H), 3.27 (d, *J =* 8.0 Hz, 3H), 2.89 (d, *J* = 18.3 Hz, 3H), 2.81 (s, 3H), 2.50 (d, *J =* 13.0 Hz, 2H), 2.37 (d, *J =* 10.7 Hz, 2H). LCMS (ESI) calcd. for C₂₆H₃₃F₃N₇O₂S⁺ [M+H]⁺: 564.2; found, 564.1.

### Intermediate Example 19: preparation of N-methyl-N-(3-(((2-((4-(4-(methylamino)piperidin-1-yl)phenyl)amino)-5-(trifluoromethyl)pyrimidin-4-yl)amino)methyl)pyrazin-2-yl)methanesulfonamide (GT-M-174)

N-methyl-N-(3-(((2-((4-(4-(methylamino)piperidin-1-yl)phenyl)amino)-5-(trifluoromethyl)pyrimidin-4-yl)amino)methyl)pyrazin-2-yl)methanesulfonamide (GT-M-174) was prepared by referring to Scheme M-11 (white solid, 707 mg). ¹H NMR (400 MHz, MeOD) δ 8.58 (dd, *J =* 17.7, 2.3 Hz, 2H), 8.39 (s, 1H), 7.76 (dd, *J* = 44.2, 9.0 Hz, 4H), 5.16 (s, 2H), 3.83 (d, *J =* 13.7 Hz, 4H), 3.27 (s, 3H), 3.16 (s, 3H), 2.80 (s, 3H), 2.55 - 2.33 (m, 4H). LCMS (ESI) calcd. for C₂₄H₃₁F₃N₉O₂S⁺ [M+H]⁺: 566.2; found, 566.3.

### Intermediate Example 20: preparation of N-methyl-N-(3-(((2-((4-(4-(piperazin-1-yl)piperidin-1-yl)phenyl)amino)-5-(trifluoromethyl)pyrimidin-4-yl)amino)methyl)pyrazin-2-yl)methanesulfonamide (GT-M-175)

N-methyl-N-(3-(((2-((4-(4-(piperazin-1-yl)piperidin-1-yl)phenyl)amino)-5-(trifluoromethyl)pyrimidin-4-yl)amino)methyl)pyrazin-2-yl)methanesulfonamide (GT-M-175) was prepared by referring to Scheme M-11 (brown solid, 560 mg). ¹H NMR (400 MHz, MeOD) δ 8.58 (dd, *J =* 18.6, 2.4 Hz, 1H), 8.37 (s, 1H), 7.66 (s, 2H), 5.15 (s, 1H), 4.00 - 3.60 (m, 6H), 3.26 (s, 2H), 3.16 (s, 2H), 2.54 (dd, *J=* 43.4, 11.6 Hz, 2H). LCMS (ESI) calcd. for C₂₇H₃₆F₃N₁₀O₂S⁺ [M+H]⁺: 621.3; found, 621.4.

### Intermediate Example 21: preparation of N⁴-(3-(methylsulfonyl)benzyl)-N²-(4-(piperazin-1-yl)phenyl)-5-(trifluoromethyl)pyrimidine-2,4-diamine (GT-M-191)

N⁴-(3-(methylsulfonyl)benzyl)-N²-(4-(piperazin-1-yl)phenyl)-5-(trifluoromethyl)pyrimidine-2,4-diamine (GT-M-191) was prepared by referring to Scheme M-11 (purple solid, 500 mg). ¹H NMR (400 MHz, DMSO) δ 9.78 (s, 1H), 8.62 (s, 3H), 8.26 (s, 1H), 7.90 (s, 1H), 7.81 (d, *J =* 7.1 Hz, 1H), 7.61 (t, *J =* 7.6 Hz, 2H), 7.31 (d, *J* = 7.9 Hz, 2H), 6.91 (d, *J* = 8.7 Hz, 2H), 4.71 (d, *J =* 5.5 Hz, 2H), 3.35 - 3.20 (m, 8H), 3.16 (d, *J* = 11.3 Hz, 3H). LCMS (ESI) calcd. for C₂₃H₂₆F₃N₆O₂S⁺ [M+H]⁺: 507.2; found, 507.4.

### Intermediate Example 22: preparation of N-methyl-N-(3-(((2-((4-(piperidin-4-yl)phenyl)amino)-5-(trifluoromethyl)pyrimidin-4-yl)amino)methyl)phenyl)methanesulfonamide (GT-M-193)

N-methyl-N-(3-(((2-((4-(piperidin-4-yl)phenyl)amino)-5-(trifluoromethyl)pyrimidin-4-yl)amino)methyl)phenyl)methanesulfonamide (GT-M-193) was prepared by referring to Scheme M-12 (white solid, 0.87 g). ¹H NMR (400 MHz, DMSO) δ 9.75 (s, 1H), 8.63 (s, 1H), 8.38 (s, 1H), 8.25 (s, 1H), 7.98 (s, 1H), 7.51 (d, *J =* 8.0 Hz, 2H), 7.41 - 7.31 (m, 2H), 7.24 (dd, *J =* 16.3, 7.7 Hz, 2H), 7.09 (d, *J =* 8.4 Hz, 2H), 4.66 (d, *J =* 5.6 Hz, 2H), 3.37 (d, *J =* 12.2 Hz, 2H), 3.15 (s, 3H), 3.00 (q, *J =* 11.9 Hz, 2H), 2.86 (s, 3H), 2.77 (t, *J =* 12.0 Hz, 1H), 1.92 (d, *J =* 13.5 Hz, 2H), 1.73 (dd, *J =* 22.9, 12.5 Hz, 2H). LCMS (ESI) calcd. for C₂₅H₃₀F₃N₆O₂S⁺ [M+H]⁺: 535.2; found, 535.3.

### Intermediate Example 23: preparation of 4-(((4-(5-chloro-2-(piperidin-4-ylamino)pyridin-4-yl)thiazol-2-yl)amino)methyl)tetrahydro-2H-pyran-4-carbonitrile (GT-M-158)

4-(((4-(5-chloro-2-(piperidin-4-ylamino)pyridin-4-yl)thiazol-2-yl)amino)methyl)tetrahydro-2H-pyran-4-carbonitrile (GT-M-158) was prepared by referring to Scheme M-14 (yellow solid, 900 mg). ¹H NMR (400 MHz, DMSO) δ 8.52 (s, 1H), 8.34 (s, 1H), 8.12 (t, J = 6.2 Hz, 1H), 8.02 (s, 1H), 7.39 (s, 1H), 7.11 (s, 1H), 6.99 (s, 1H), 3.92 (s, 2H), 3.68 (d, J = 6.1 Hz, 2H), 3.49 (t, J = 11.3 Hz, 3H), 3.31 (d, *J =* 12.7 Hz, 2H), 3.03 (d, J = 10.3 Hz, 2H), 2.07 (d, J = 10.8 Hz, 2H), 1.88 (d, J = 13.4 Hz, 2H), 1.77 - 1.59 (m, 4H). LCMS (ESI) calcd. for C₂₀H₂₅ClN₆OS⁺ [M+H]⁺: 433.16; found, 433.1.

### Intermediate Example 24: preparation of 4-(((5'-chloro-2'-(piperidin-4-ylamino)-[2,4'-bipyridin]-6-yl)amino)methyl)tetrahydro-2H-pyran-4-carbonitrile (GT-M-159)

4-(((5'-chloro-2'-(piperidin-4-ylamino)-[2,4'-bipyridin]-6-yl)amino)methyl)tetrahydro-2H-pyran-4-carbonitrile (GT-M-159) was prepared by referring to Scheme M-13 (yellow solid, 1.5 g, yield 86.9%). ¹H NMR (400 MHz, DMSO) δ 8.55 (s, 1H), 8.39 (s, 1H), 8.04 (s, 1H), 7.57 - 7.43 (m, 1H), 7.10 (t, *J =* 6.4 Hz, 1H), 6.95 (d, *J* = 7.3 Hz, 1H), 6.76 (d, *J =* 7.2 Hz, 1H), 6.67 (d, *J =* 10.0 Hz, 2H), 4.08 - 3.80 (m, 3H), 3.66 (d, *J =* 6.4 Hz, 2H), 3.46 (dd, *J =* 11.9, 10.5 Hz, 2H), 3.29 (s, 2H), 3.04 (dd, *J =* 18.8, 7.9 Hz, 2H), 2.07 (d, *J =* 10.8 Hz, 2H), 1.84 (d, *J =* 13.2 Hz, 2H), 1.73 - 1.46 (m, 4H). LCMS (ESI) calcd. for C₂₂H₂₈ClN₆O⁺ [M+H]⁺: 427.2; found, 427.2.

### Intermediate Example 25: preparation of 2-(5-fluoro-2-hydroxyphenyl)-2-(1-oxo-6-(4-(piperazin-1-yl)phenyl)isoindolin-2-yl)-N-(thiazol-2-yl)acetamide (GT-D-012)

2-(5-fluoro-2-hydroxyphenyl)-2-(1-oxo-6-(4-(piperazin-1-yl)phenyl)isoindolin-2-yl)-N-(thiazol-2-yl)acetamide (GT-D-012) was prepared by referring to Scheme M-15 (yellow solid, 110 mg). ¹H NMR (400 MHz, DMSO) δ 10.20 - 8.61 (m, 1H), 7.87 (d, *J =* 8.5 Hz, 2H), 7.63 (dd, *J =* 11.3, 8.4 Hz, 4H), 7.49 (d, *J =* 3.5 Hz, 1H), 7.27 (d, *J* = 3.6 Hz, 1H), 7.12 (td, *J =* 8.6, 3.1 Hz, 1H), 7.07 (d, *J =* 8.8 Hz, 2H), 6.92 (dd, *J =* 8.9, 4.8 Hz, 1H), 6.87 (dd, *J =* 9.2, 3.0 Hz, 1H), 6.33 (s, 1H), 4.64 (d, *J =* 17.5 Hz, 1H), 4.02 (d, *J =* 17.5 Hz, 1H), 3.29 (d, *J =* 4.4 Hz, 5H), 3.12 - 3.00 (m, 6H), 1.24 (s, 1H). LCMS (ESI) calcd. for C₂₉H₂₇FN₅O₃S⁺ [M+H]⁺: 544.2; found, 544.2.

### Intermediate Example 26: preparation of 2-(5-fluoro-2-hydroxyphenyl)-2-(1-oxo-6-(6-(piperazin-1-yl)pyridazin-3-yl)isoindolin-2-yl)-N-(thiazol-2-yl)acetamide (GT-D-014)

2-(5-fluoro-2-hydroxyphenyl)-2-(1-oxo-6-(6-(piperazin-1-yl)pyridazin-3-yl)isoindolin-2-yl)-N-(thiazol-2-yl)acetamide (GT-D-014) was prepared by referring to Scheme M-16 (brown solid, 130 mg). ¹H NMR (400 MHz, DMSO) δ 8.31 (d, *J =* 8.3 Hz, 2H), 8.06 (d, *J =* 9.6 Hz, 1H), 7.68 (d, *J =* 7.9 Hz, 1H), 7.48 (d, *J* = 3.6 Hz, 1H), 7.33 (d, *J =* 9.7 Hz, 1H), 7.25 (d, *J =* 3.5 Hz, 1H), 7.11 (td, *J =* 8.6, 3.1 Hz, 1H), 6.94 - 6.84 (m, 2H), 6.33 (s, 1H), 4.69 (d, *J =* 17.8 Hz, 1H), 4.07 (d, *J =* 17.8 Hz, 1H), 3.66 - 3.54 (m, 4H), 3.17 (s, 1H), 2.91 - 2.78 (m, 4H), 1.23 (s, 1H). LCMS (ESI) calcd. for C₂₇H₂₅FN₇O₃S⁺ [M+H]⁺: 546.2; found, 546.2.

**Intermediate Example 27: preparation of 2-(5-fluoro-2-hydroxyphenyl)-2-(1-oxo-6-(piperazin-1-yl)isoindolin-2-yl)-N-(thiazol-2-yl)acetamide (GT-D-69)**

2-(5-fluoro-2-hydroxyphenyl)-2-(1-oxo-6-(piperazin-1-yl)isoindolin-2-yl)-N-(thiazol-2-yl)acetamide (GT-D-69) was prepared by referring to Scheme M-17 (yellow solid, 515 mg). ¹H NMR (400 MHz, DMSO) δ 7.48 (d, *J =* 3.5 Hz, 1H), 7.39 (d, *J =* 8.4 Hz, 1H), 7.24 (dd, *J =* 11.9, 2.8 Hz, 2H), 7.15 (d, *J =* 1.8 Hz, 1H), 7.13 - 7.05 (m, 1H), 6.90 (dd, *J =* 8.9, 4.8 Hz, 1H), 6.82 (dd, *J =* 9.2, 2.9 Hz, 1H), 6.29 (s, 1H), 4.50 (d, *J =* 17.0 Hz, 1H), 3.89 (d, *J =* 17.0 Hz, 2H), 3.13 (s, 5H), 2.91 (s, 5H), 1.24 (s, 1H). LCMS (ESI) calcd. for C₂₃H₂₃FN₅O₃S⁺ [M+H]⁺: 468.2; found, 468.2.

### Intermediate Example 28: preparation of 2-(5-fluoro-2-hydroxyphenyl)-2-(6-(4-(methylamino)piperidin-1-yl)-1-oxoisoindolin-2-yl)-N-(thiazol-2-yl)acetamide (GT-D-70)

2-(5-fluoro-2-hydroxyphenyl)-2-(6-(4-(methylamino)piperidin-1-yl)-1-oxoisoindolin-2-yl)-N-(thiazol-2-yl)acetamide (GT-D-70) was prepared by referring to Scheme M-17 (brown solid, 186 mg). ¹H NMR (400 MHz, DMSO) δ 7.40 (dd, *J =* 29.5, 5.8 Hz, 2H), 7.22 (d, *J =* 7.5 Hz, 1H), 7.16 (d, *J =* 19.7 Hz, 3H), 7.05 (d, *J* = 6.2 Hz, 1H), 6.91 - 6.76 (m, 2H), 6.24 (s, 1H), 4.54 (d, *J =* 17.0 Hz, 1H), 3.94 (d, *J =* 17.0 Hz, 1H), 3.67 (d, *J =* 12.1 Hz, 2H), 3.17 (s, 1H), 2.78 (t, *J =* 11.3 Hz, 2H), 2.31 (d, *J =* 5.8 Hz, 3H), 1.89 (d, *J =* 11.2 Hz, 2H), 1.35 (d, *J =* 11.1 Hz, 2H). LCMS (ESI) calcd. for C₂₅H₂₇FN₅O₃S⁺ [M+H]⁺: 496.2; found, 496.2.

### Intermediate Example 29: preparation of 2-(5-fluoro-2-hydroxyphenyl)-2-(1-oxo-6-(4-(piperazin-1-yl)piperidin-1-yl)isoindolin-2-yl)-N-(thiazol-2-yl)acetamide (GT-D-71)

2-(5-fluoro-2-hydroxyphenyl)-2-(1-oxo-6-(4-(piperazin-1-yl)piperidin-1-yl)isoindolin-2-yl)-N-(thiazol-2-yl)acetamide (GT-D-71) was prepared by referring to Scheme M-17 (brown solid, 110 mg). ¹H NMR (400 MHz, DMSO) δ 7.45 (s, 1H), 7.36 (d, *J =* 8.4 Hz, 1H), 7.22 (d, *J =* 9.5 Hz, 4H), 7.07 (s, 1H), 6.91 - 6.77 (m, 3H), 6.25 (s, 1H), 4.51 (d, *J =* 17.1 Hz, 1H), 3.91 (d, *J =* 16.9 Hz, 1H), 3.77 (d, *J =* 12.1 Hz, 3H), 3.17 (s, 2H), 2.72 (s, 5H), 2.44 (s, 5H), 1.82 (d, *J =* 10.7 Hz, 2H), 1.51 (d, *J =* 11.4 Hz, 3H), 1.25 (s, 1H). LCMS (ESI) calcd. for C₂₈H₃₂FN₆O₃S⁺ [M+H]⁺: 551.2; found, 551.2.

### Intermediate Example 30: preparation of 9-ethyl-6,6-dimethyl-11-oxo-8-(piperazin-1-yl)-6,11-dihydro-5H-benzo[b]carbazole-3-carbonitrile (GT-D-42)

9-ethyl-6,6-dimethyl-11-oxo-8-(piperazin-1-yl)-6,11-dihydro-5H-benzo[b]carbazole-3-carbonitrile (GT-D-42) was prepared by referring to Scheme M-19 (white solid, 240 mg). ¹H NMR (400 MHz, DMSO) δ 13.03 (s, 1H), 9.39 (s, 2H), 8.32 (d, *J =* 8.1 Hz, 1H), 8.05 (d, *J =* 29.2 Hz, 2H), 7.61 (d, *J =* 9.0 Hz, 1H), 7.38 (s, 1H), 3.24 (d, *J =* 14.9 Hz, 8H), 2.73 (d, *J =* 7.5 Hz, 2H), 1.79 (s, 6H), 1.29 (t, *J =* 7.5 Hz, 3H). LCMS (ESI) calcd. for C₂₅H₂₇N₄O⁺ [M+H]⁺: 399.2; found, 399.2.

### Intermediate Example 31: preparation of 9-ethyl-6,6-dimethyl-11-oxo-8-(4-(piperazin-1-yl)piperidin-1-yl)-6,11-dihydro-5H-benzo[b]carbazole-3-carbonitrile (GT-D-113)

9-ethyl-6,6-dimethyl-11-oxo-8-(4-(piperazin-1-yl)piperidin-1-yl)-6,11-dihydro-5H-benzo[b]carbazole-3-carbonitrile (GT-D-113) was prepared by referring to Scheme M-19 (off-white solid, 1.4 g). ¹H NMR (400 MHz, MeOD) δ 8.39 (d, *J =* 8.2 Hz, 1H), 8.23 (s, 1H), 7.86 (s, 1H), 7.62 - 7.48 (m, 2H), 3.75 (s, 9H), 3.53 (d, *J =* 12.0 Hz, 2H), 3.18 (t, *J =* 11.7 Hz, 2H), 2.86 (q, *J =* 7.4 Hz, 2H), 2.43 (d, *J =* 11.4 Hz, 2H), 2.22 (dd, *J* = 20.7, 11.9 Hz, 2H), 1.82 (s, 6H), 1.37 (t, *J* = 7.5 Hz, 3H). LCMS (ESI) calcd. for C₃₀H₃₆N₅O⁺ [M+H]⁺: 482.3; found, 482.2.

### Intermediate Example 32: preparation of 9-ethyl-6,6-dimethyl-8-(4-(methylamino)piperidin-1-yl)-11-oxo-6,11-dihydro-5H-benzo[b]carbazole-3-carbonitrile (GT-D-85)

9-ethyl-6,6-dimethyl-8-(4-(methylamino)piperidin-1-yl)-11-oxo-6,11-dihydro-5H-benzo[b]carbazole-3-carbonitrile (GT-D-85) was prepared by referring to Scheme M-19 (white solid, 130 mg). ¹H NMR (400 MHz, MeOD) δ 8.31 (d, *J =* 8.2 Hz, 1H), 8.11 (s, 1H), 7.77 (s, 1H), 7.45 (d, *J =* 8.2 Hz, 1H), 7.36 (s, 1H), 3.29 (d, *J* = 11.9 Hz, 2H), 2.91 (t, *J =* 11.7 Hz, 2H), 2.78 - 2.64 (m, 5H), 2.18 (d, *J =* 11.7 Hz, 2H), 1.86 - 1.75 (m, 2H), 1.71 (s, 6H), 1.25 (t, *J =* 7.5 Hz, 3H), 1.22 - 1.18 (m, 1H). LCMS (ESI) calcd. for C₂₇H₃₁N₄O⁺ [M+H]⁺: 427.3; found, 427.2 .

### Intermediate Example 33: preparation of 9-cyclopentyl-N⁸-phenyl-N²-(4-(piperazin-1-yl)phenyl)-9H-purine-2,8-diamine (GT-D-62)

9-cyclopentyl-N³-phenyl-N²-(4-(piperazin-1-yl)phenyl)-9H-purine-2,8-diamine (GT-D-62) was prepared by referring to Scheme M-20 (white solid, 1.18 g). ¹H NMR (400 MHz, MeOD) δ 8.15 (s, 1H), 7.67 - 7.52 (m, 4H), 7.46 (dd, *J =* 15.9, 8.0 Hz, 3H), 7.25 (d, *J =* 9.0 Hz, 2H), 3.66 (s, 1H), 3.61 - 3.53 (m, 4H), 3.47 (dd, *J =* 6.5, 3.6 Hz, 4H), 2.40 (dd, *J =* 12.8, 7.3 Hz, 2H), 2.20 (d, *J =* 7.9 Hz, 2H), 1.92 (s, 2H), 1.77 - 1.62 (m, 2H). LCMS (ESI) calcd. for C₂₆H₃₁N₈⁺ [M+H]⁺: 455.3; found, 455.0.

### Intermediate Example 34: preparation of 9-cyclopentyl-N²-(4-(4-(methylamino)piperidin-1-yl)phenyl)-N⁸-phenyl-9H-purine-2,8-diamine (GT-D-67)

9-cyclopentyl-N²-(4-(4-(methylamino)piperidin-1-yl)phenyl)-N⁸-phenyl-9H-purine-2,8-diamine (GT-D-67) was prepared by referring to Scheme M-20 (yellow solid, 4.6 g). ¹H NMR (400 MHz, MeOD) δ 8.34 (s, 1H), 7.94 - 7.82 (m, 4H), 7.57 (dd, *J =* 7.4, 6.3 Hz, 4H), 7.45 (dd, *J =* 7.7, 4.2 Hz, 1H), 3.95 - 3.81 (m, 4H), 3.71 - 3.60 (m, 2H), 2.89 (s, 1H), 2.81 (s, 3H), 2.51 (t, *J =* 15.4 Hz, 4H), 2.43 - 2.37 (m, 2H), 2.25 (d, *J =* 8.3 Hz, 2H), 2.09 - 2.02 (m, 2H), 1.83 - 1.71 (m, 2H). LCMS (ESI) calcd. for C₂₈H₃₅N₈⁺ [M+H]⁺: 483.3; found, 483.3.

### Intermediate Example 35: preparation of 9-cyclopentyl-N⁸-phenyl-N²-(4-(4-(piperazin-1-yl)piperidin-1-yl)phenyl)-9H-purine-2,8-diamine (GT-D-68)

9-cyclopentyl-N⁸-phenyl-N²-(4-(4-(piperazin-1-yl)piperidin-1-yl)phenyl)-9H-purine-2,8-diamine (GT-D-68) was prepared by referring to Scheme M-20 (yellow solid, 4.1 g). ¹H NMR (400 MHz, DMSO) δ 7.62 (d, *J* = 8.9 Hz, 2H), 7.56 (t, *J =* 3.7 Hz, 3H), 7.01 - 6.94 (m, 2H), 6.80 (d, *J* = 9.0 Hz, 2H), 6.42 (t, *J* = 7.1 Hz, 1H), 3.53 (d, *J =* 11.9 Hz, 2H), 2.67 (s, 4H), 2.54 (d, *J =* 10.1 Hz, 2H), 2.42 (s, 2H), 2.38 - 2.31 (m, 6H), 2.20 (t, *J =* 11.2 Hz, 1H), 1.99 (s, 2H), 1.92 - 1.87 (m, 1H), 1.81 (d, *J =* 10.0 Hz, 4H), 1.69 - 1.61 (m, 2H), 1.52 (dt, *J =* 11.7, 8.7 Hz, 2H). LCMS (ESI) calcd. for C₃₁H₄₀N₉⁺ [M+H]⁺: 538.3; found, 538.4.

### Intermediate Example 36: preparation of N-(2-(1-(cyclopropylsulfonyl)-1H-pyrazol-4-yl)pyrimidin-4-yl)-1-isopropyl-3-(piperazin-1-yl)-1H-pyrazolo[4,3-c]pyridin-6-amine (GT-D-18)

N-(2-(1-(cyclopropylsulfonyl)-1H-pyrazol-4-yl)pyrimidin-4-yl)-1-isopropyl-3-(piperazin-1-yl)-1H-pyrazolo[4,3-c]pyridin-6-amine (GT-D-18) was prepared by referring to Scheme M-18 (yellow solid, 0.75 g). ¹H NMR (400 MHz, CDCl₃) δ 8.73 (s, 1H), 8.69 (s, 1H), 8.47 (s, 1H), 8.39 (d, *J =* 5.9 Hz, 1H), 8.12 (s, 1H), 7.97 (s, 1H), 6.81 (d, *J =* 5.8 Hz, 1H), 4.65 (dt, *J =* 13.3, 6.6 Hz, 1H), 3.56 - 3.43 (m, 4H), 3.16 - 3.00 (m, 4H), 2.90 - 2.80 (m, 1H), 1.58 (d, *J =* 6.7 Hz, 6H), 1.55 - 1.49 (m, 2H), 1.26 - 1.19 (m, 2H). LCMS (ESI) calcd. for C₂₃H₂₉N₁₀O₂S⁺ [M+H]⁺: 509.2; found, 509.2.

### Intermediate Example 37: preparation of N-(2-(1-(cyclopropylsulfonyl)-1H-pyrazol-4-yl)pyrimidin-4-yl)-1-isopropyl-3-(4-(methylamino)piperidin-1-yl)-1H-pyrazolo[4,3-c]pyridin-6-amine (GT-D-72)

N-(2-(1-(cyclopropylsulfonyl)-1H-pyrazol-4-yl)pyrimidin-4-yl)-1-isopropyl-3-(4-(methylamino)piperidin-1-yl)-1H-pyrazolo[4,3-c]pyridin-6-amine (GT-D-72) was prepared by referring to Scheme M-18 (yellow solid, 0.4 g). ¹H NMR (400 MHz, CDCl₃) δ 8.73 (s, 1H), 8.69 (s, 1H), 8.47 (s, 1H), 8.40 (d, *J* = 5.9 Hz, 1H), 8.11 (s, 1H), 7.80 (s, 1H), 6.82 (d, *J* = 5.8 Hz, 1H), 4.65 (dt, *J =* 13.3, 6.6 Hz, 1H), 4.09 (t, *J =* 12.3 Hz, 2H), 3.05 (t, *J =* 11.3 Hz, 2H), 2.85 (ddd, *J =* 12.5, 7.8, 4.7 Hz, 2H), 2.61 (s, 3H), 2.16 (d, *J =* 10.7 Hz, 2H), 1.81 (dd, *J =* 20.3, 11.2 Hz, 2H), 1.58 (d, *J =* 6.7 Hz, 6H), 1.54 (dd, *J =* 5.8, 3.7 Hz, 2H), 1.23 (dt, *J =* 7.0, 3.6 Hz, 2H). LCMS (ESI) calcd. for C₂₅H₃₃N₁₀O₂S⁺ [M+H]⁺: 537.3; found, 537.4.

### Intermediate Example 38: preparation of N-(2-(1-(cyclopropylsulfonyl)-1H-pyrazol-4-yl)pyrimidin-4-yl)-1-isopropyl-3-(4-(piperazin-1-yl)piperidin-1-yl)-1H-pyrazolo[4,3-c]pyridin-6-amine (GT-D-73)

N-(2-(1-(cyclopropylsulfonyl)-1H-pyrazol-4-yl)pyrimidin-4-yl)-1-isopropyl-3-(4-(piperazin-1-yl)piperidin-1-yl)-1H-pyrazolo[4,3-c]pyridin-6-amine (GT-D-73) was prepared by referring to Scheme M-18 (yellow solid, 1.19 g). ¹H NMR (400 MHz, DMSO) δ 10.37 (s, 1H), 8.86 (s, 1H), 8.70 (s, 1H), 8.48 (s, 1H), 8.41 (d, *J =* 5.9 Hz, 1H), 8.32 (s, 1H), 7.13 (d, *J =* 5.5 Hz, 1H), 4.72 (s, 1H), 4.02 (d, *J =* 12.0 Hz, 2H), 3.53 (d, *J* = 57.7 Hz, 1H), 3.33 - 3.27 (m, 2H), 2.88 (t, *J =* 11.6 Hz, 2H), 2.76 (s, 4H), 2.35 (d, *J =* 12.0 Hz, 1H), 1.87 (d, *J* = 10.4 Hz, 2H), 1.60 (d, *J =* 10.0 Hz, 2H), 1.47 (d, *J =* 6.6 Hz, 6H), 1.36 (s, 2H), 1.28 (dd, *J =* 7.5, 5.1 Hz, 2H). LCMS (ESI) calcd. for C₂₈H₃₈N₁₁O₂S⁺ [M+H]⁺: 592.3; found, 592.4.

### Intermediate Example 39: preparation of 1-isopropyl-N-(2-(4-methoxypiperidin-1-yl)pyrimidin-4-yl)-3-(4-(methylamino)piperidin-1-yl)-1H-pyrazolo[4,3-c]pyridin-6-amine (GT-D-74)

1-isopropyl-N-(2-(4-methoxypiperidin-1-yl)pyrimidin-4-yl)-3-(4-(methylamino)piperidin-1-yl)-1H-pyrazolo[4,3-c]pyridin-6-amine (GT-D-74) was prepared by referring to Scheme M-18 (yellow solid, 0.97 g). ¹H NMR (400 MHz, MeOD) δ 9.38 (s, 1H), 8.03 (d, *J =* 7.1 Hz, 1H), 7.80 (s, 1H), 6.59 (d, *J =* 7.1 Hz, 1H), 4.89 (s, 1H), 4.30 (d, *J* = 13.0 Hz, 2H), 3.89 (d, *J =* 8.7 Hz, 2H), 3.67 (d, *J =* 15.3 Hz, 2H), 3.59 (s, 1H), 3.39 (s, 3H), 3.37 - 3.32 (m, 1H), 3.20 (s, 2H), 2.77 (s, 3H), 2.27 (d, *J =* 11.6 Hz, 2H), 2.07 - 1.94 (m, 2H), 1.83 (ddd, *J =* 33.0, 16.1, 8.8 Hz, 4H), 1.54 (d, *J =* 6.5 Hz, 6H). LCMS (ESI) calcd. for C₂₅H₃₈N₉O⁺ [M+H]⁺: 480.3; found, 480.4.

### Intermediate Example 40: preparation of 1-isopropyl-N-(2-(4-methoxypiperidin-1-yl)pyrimidin-4-yl)-3-(4-(piperazin-1-yl)piperidin-1-yl)-1H-pyrazolo[4,3-c]pyridin-6-amine (GT-D-75)

1-isopropyl-N-(2-(4-methoxypiperidin-1-yl)pyrimidin-4-yl)-3-(4-(piperazin-1-yl)piperidin-1-yl)-1H-pyrazolo[4,3-c]pyridin-6-amine (GT-D-75) was prepared by referring to Scheme M-18 (yellow solid, 1.1 g). ¹H NMR(400 MHz, MeOD) δ 9.40 (s, 1H), 8.05 (d, *J =* 7.0 Hz, 1H), 7.83 (s, 1H), 6.61 (d, *J =* 7.0 Hz, 1H), 4.39 (d, *J =* 12.7 Hz, 2H), 3.93 (s, 3H), 3.67 (dd, *J =* 33.6, 15.5 Hz, 12H), 3.41 (s, 3H), 3.23 (t, *J =* 12.4 Hz, 2H), 2.41 (d, *J =* 10.4 Hz, 2H), 2.07 (dd, *J =* 23.8, 8.9 Hz, 4H), 1.79 (s, 2H), 1.57 (d, *J =* 6.4 Hz, 6H). LCMS (ESI) calcd. for C₂₈H₄₃N₁₀O⁺ [M+H]⁺: 535.4; found, 535.5.

### Intermediate Example 41: preparation of 7-cyclopentyl-N,N-dimethyl-2-((5-(4-(methylamino)piperidin-1-yl)pyridin-2-yl)amino)-7H-pyrrolo[2,3-d]pyrimidine-6-carboxamide (GT-D-79)

7-cyclopentyl-N,N-dimethyl-2-((5-(4-(methylamino)piperidin-1-yl)pyridin-2-yl)amino)-7H-pyrrolo[2,3-d]pyrimidine-6-carboxamide (GT-D-79) was prepared by referring to Scheme M-8 (yellow solid, 800 mg). ¹H NMR (400 MHz, DMSO) δ 9.38 (s, 1H), 8.77 (s, 1H), 8.15 (d, *J =* 9.1 Hz, 1H), 8.03 (d, *J* = 2.9 Hz, 1H), 7.45 (dd, *J =* 9.1, 3.0 Hz, 1H), 6.60 (s, 1H), 4.74 (p, *J =* 8.8 Hz, 1H), 3.67 (d, *J =* 12.5 Hz, 2H), 3.51 (s, 1H), 3.06 (s, 6H), 2.90 - 2.80 (m, 1H), 2.72 (t, *J =* 11.1 Hz, 2H), 2.47 (s, 3H), 2.43 (d, *J =* 11.5 Hz, 2H), 2.05 - 1.94 (m, 6H), 1.67 - 1.51 (m, 4H). LCMS (ESI) calcd. for C₂₅H₃₅N₈O⁺ [M+H]⁺: 463.3; found, 463.2.

### Intermediate Example 42: preparation of 7-cyclopentyl-N,N-dimethyl-2-((5-(4-(piperazin-1-yl)piperidin-1-yl)pyridin-2-yl)amino)-7H-pyrrolo[2,3-d]pyrimidine-6-carboxamide (GT-D-80)

7-cyclopentyl-N,N-dimethyl-2-((5-(4-(piperazin-1-yl)piperidin-1-yl)pyridin-2-yl)amino)-7H-pyrrolo[2,3-d]pyrimidine-6-carboxamide (GT-D-80) was prepared by referring to Scheme M-8 (off-white solid, 305 mg). ¹H NMR (400 MHz, DMSO) δ 9.22 (s, 1H), 8.74 (s, 1H), 8.12 (d, *J =* 9.2 Hz, 1H), 7.98 (d, *J =* 2.8 Hz, 1H), 7.42 (dd, *J=* 9.1, 2.9 Hz, 1H), 6.59 (s, 1H), 4.80 - 4.66 (m, 1H), 3.05 (s, 6H), 2.70 - 2.66 (m, 4H), 2.63 (d, *J=* 11.8 Hz, 2H), 2.42 (s, 6H), 2.24 (d, *J =* 11.4 Hz, 1H), 1.97 (s, 6H), 1.84 (d, *J=* 11.2 Hz, 2H), 1.64 (s, 2H), 1.58 - 1.50 (m, 2H), 1.23 (s, 1H). LCMS (ESI) calcd. for C₂₈H₄₀N₉O⁺ [M+H]⁺: 518.3; found, 518.4.

### Intermediate Example 43: preparation of N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101)

N-(3-(difluoromethyl)-1-(piperidin-4-yl)-1H-pyrazol-4-yl)-5-morpholinopyrazolo[1,5-a]pyrimidine-3-carboxamide hydrochloride (GT-D-101) was prepared by referring to Scheme M-35 (yellow solid, 1.3 g). ¹H NMR (400 MHz, MeOD) δ 8.55 (d, *J =* 8.0 Hz, 1H), 8.42 (s, 1H), 8.32 (s, 1H), 7.03 - 6.75 (m, 2H), 4.60 (s, 1H), 3.86 - 3.80 (m, 8H), 3.58 (dd, *J =* 10.0, 6.5 Hz, 2H), 3.27 - 3.17 (m, 2H), 2.39 - 2.24 (m, 4H). LCMS (ESI) calcd. for C₂₀H₂₅F₂N₈O₂⁺ [M+H]⁺: 447.2 ; found, 447.2.

### Intermediate Example 44: preparation of 3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109)

3-(imidazo[1,2-b]pyridazin-3-ylethynyl)-4-methyl-N-(4-(piperazin-1-ylmethyl)-3-(trifluoromethyl)phenyl)benzamide hydrochloride (GT-D-109) was prepared by referring to Scheme M-22 (off-white solid, 670 mg). ¹H NMR (400 MHz, MeOD) δ 9.10 (dd, *J =* 4.5, 1.3 Hz, 1H), 8.62 (s, 1H), 8.52 (dd, *J=* 9.3, 1.3 Hz, 1H), 8.39 (d, *J =* 1.8 Hz, 1H), 8.24 (d, *J =* 1.8 Hz, 1H), 8.14 (d, *J =* 1.8 Hz, 1H), 8.09 (d, *J =* 8.6 Hz, 1H), 7.98 (ddd, *J =* 13.9, 8.7, 3.2 Hz, 2H), 7.55 (d, *J =* 8.1 Hz, 1H), 4.53 (s, 2H), 3.60 (dd, *J =* 26.0, 12.5 Hz, 8H), 2.69 (s, 3H). LCMS (ESI) calcd. for C₂₈H₂₆F₃N₆O⁺ [M+H]⁺: 519.2; found, 519.4.

### Intermediate Example 45: preparation of 3-(imidazo[1,2-b]pyridazin-3-ylethynyl)-4-methyl-N-(4-(piperazin-1-yl)-3-(trifluoromethyl)phenyl)benzamide (GT-D-110)

3-(imidazo[1,2-b]pyridazin-3-ylethynyl)-4-methyl-N-(4-(piperazin-1-yl)-3-(trifluoromethyl)phenyl)benzamide (GT-D-110) was prepared by referring to Scheme M-21 (off-white solid, 930 mg). ¹H NMR (400 MHz, MeOD) δ 9.10 - 9.00 (m, 1H), 8.55 (s, 1H), 8.47 (dd, J = 9.3, 1.3 Hz, 1H), 8.22 (d, J = 1.8 Hz, 1H), 8.15 (d, J = 2.4 Hz, 1H), 8.05 (dd, J = 8.7, 2.4 Hz, 1H), 7.97 (dd, J = 8.0, 1.9 Hz, 1H), 7.89 (dd, J = 9.3, 4.5 Hz, 1H), 7.65 (t, J = 6.5 Hz, 1H), 7.59 - 7.56 (m, 1H), 3.38 - 3.34 (m, 4H), 3.23 - 3.14 (m, 4H), 2.68 (s, 3H). LCMS (ESI) calcd. for C₂₇H₂₄F₃N₆O⁺ [M+H]⁺: 505.2; found, 505.3.

### Intermediate Example 46: preparation of 3-(imidazo[1,2-b]pyridazin-3-ylethynyl)-4-methyl-N-(4-(4-(methylamino)piperidin-1-yl)-3-(trifluoromethyl)phenyl)benzamide (GT-D-111)

3-(imidazo[1,2-b]pyridazin-3-ylethynyl)-4-methyl-N-(4-(4-(methylamino)piperidin-1-yl)-3-(trifluoromethyl)phenyl)benzamide (GT-D-111) was prepared by referring to Scheme M-21 (white solid, 1.8 g). ¹H NMR (400 MHz, DMSO) δ 10.63 (s, 1H), 9.21 (s, 2H), 8.86 (dd, *J* = 4.4*,* 1.1 Hz, 1H), 8.43 (s, 1H), 8.21 (dd, *J =* 14.6, 1.9 Hz, 2H), 8.08 (s, 1H), 8.00 (dd, *J =* 8.0, 1.6 Hz, 1H), 7.59 - 7.53 (m, 3H), 3.00 (d, *J* = 11.7 Hz, 3H), 2.78 (s, 2H), 2.61 (s, 3H), 2.54 (t, *J =* 5.4 Hz, 3H), 2.11 (d, *J =* 10.0 Hz, 2H), 1.70 (dt, *J =* 11.3, 8.1 Hz, 2H). LCMS (ESI) calcd. for C₂₉H₂₈F₃N₆O⁺ [M+H]⁺: 533.2; found, 533.2.

### Intermediate Example 47: preparation of 3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112)

3-(imidazo[1,2-b]pyridazin-3-ylethynyl)-4-methyl-N-(4-(4-(piperidin-4-yl)piperazin-1-yl)-3-(trifluoromethyl)phenyl)benzamide (GT-D-112) was prepared by referring to Scheme M-21 (brown solid, 2.1 g). ¹H NMR (400 MHz, DMSO) δ 10.60 (s, 1H), 8.74 (dd, *J =* 4.4, 1.5 Hz, 1H), 8.28 (dd, *J =* 9.2, 1.5 Hz, 1H), 8.25 (s, 1H), 8.22 (d, *J =* 2.1 Hz, 2H), 8.19 - 8.13 (m, 1H), 7.96 (dd, *J =* 8.0, 1.8 Hz, 1H), 7.58 (t, *J =* 9.0 Hz, 2H), 7.42 (dd, *J =* 9.2, 4.5 Hz, 1H), 3.49 (d, *J =* 13.0 Hz, 4H), 3.15 (s, 6H), 2.98 - 2.82 (m, 2H), 2.62 (s, 3H), 2.33 (d, *J =* 10.9 Hz, 2H), 1.80 (d, *J =* 9.7 Hz, 2H). LCMS (ESI) calcd. for C₃₂H₃₃F₃N₇O⁺ [M+H]⁺: 588.3; found, 588.2.

### Intermediate Example 48: preparation of tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126)

tert-butyl (4-(4-((3-(difluoromethyl)-1-(piperidin-4-yl)-1H-pyrazol-4-yl)carbamoyl)oxazol-2-yl)pyridin-2-yl)(2,2,2-trifluoroethyl)carbamate (GT-D-126) was prepared by referring to Scheme M-23 (white solid, 2.2 g). ¹H NMR (400 MHz, CDCl₃) δ 9.06 (s, 1H), 8.53 (d, *J =* 5.1 Hz, 1H), 8.39 (s, 1H), 8.34 (d, *J* = 5.3 Hz, 2H), 7.72 (dd, *J* = 5.1*,* 1.2 Hz, 1H), 6.84 (t, *J =* 54.7 Hz, 1H), 4.88 (q, *J =* 8.7 Hz, 2H), 4.21 (s, 1H), 3.26 (d, *J =* 12.6 Hz, 2H), 2.77 (td, *J =* 12.5, 2.1 Hz, 2H), 2.16 (d, *J =* 12.2 Hz, 2H), 1.92 (dd, *J =* 12.1, 3.8 Hz, 2H), 1.58 (s, 9H). LCMS (ESI) calcd. for C₂₅H₂₉F₅N₇O₄⁺ [M+H]⁺: 586.2; found, 586.3.

### Intermediate Example 49: preparation of (1r,4r)-4-(6-(5-cyano-1H-pyrrolo[2,3-b]pyridin-1-yl)-4-(isopropylamino)nicotinamido)cyclohexane-1-carboxylic acid (GT-D-132)

(1r,4r)-4-(6-(5-cyano-1H-pyrrolo[2,3-b]pyridin-1-yl)-4-(isopropylamino)nicotinamido)cyclohexane-1-carboxylic acid (GT-D-132) was prepared by referring to Scheme M-25 (white solid, 3.1 g). ¹H NMR (400 MHz, DMSO) δ 12.11 (s, 1H), 8.81 (d, *J* = 1.9 Hz, 1H), 8.67 (d, *J =* 2.0 Hz, 1H), 8.63 - 8.55 (m, 2H), 8.52 (d, *J* = 3.9 Hz, 1H), 8.37 (d, *J* = 7.6 Hz,1H), 8.07 (s, 1H), 6.89 (d, *J* = 3.9 Hz, 1H), 3.77 (dt, *J =* 19.1, 6.3 Hz, 2H), 2.17 (dd, *J =* 11.2, 7.9 Hz, 1H), 2.04 - 1.85 (m, 4H), 1.39 (dd, *J =* 16.9, 8.0 Hz, 4H), 1.30 (s, 3H), 1.29 (s, 3H). LCMS (ESI) calcd. for C₂₄H₂₇N₆O₃⁺ [M+H]⁺: 447.2; found, 447.2.

### Intermediate Example 50: preparation of 6-(5-cyano-1H-pyrrolo[2,3-b]pyridin-1-yl)-4-(isopropylamino)-N-(piperidin-4-yl)nicotinamide (GT-D-133)

6-(5-cyano-1H-pyrrolo[2,3-b]pyridin-1-yl)-4-(isopropylamino)-N-(piperidin-4-yl)nicotinamide (GT-D-133) was prepared by referring to Scheme M-24 (white solid, 2.8 g). ¹H NMR (400 MHz, MeOD) δ 8.80 (d, *J =* 1.3 Hz, 1H), 8.74 (s, 1H), 8.62 (d, *J =* 1.5 Hz, 1H), 8.39 (d, *J =* 4.0 Hz, 1H), 7.44 (s, 1H), 7.09 (d, *J =* 4.0 Hz, 1H), 4.34 - 4.04 (m, 2H), 3.52 (d, *J =* 13.1 Hz, 2H), 3.17 (dd, *J =* 12.4, 10.3 Hz, 2H), 2.23 (d, *J* = 11.3 Hz, 2H), 1.97 (td, *J =* 14.5, 4.2 Hz, 2H), 1.39 (d, *J =* 6.4 Hz, 6H). LCMS (ESI) calcd. for C₂₂H₂₆N₇O⁺ [M+H]⁺: 404.2; found, 404.3.

### Intermediate Example 51: preparation of N-(4-(chlorodifluoromethoxy)phenyl)-6-(4-(piperazin-1-yl)piperidin-1-yl)-5-(1H-pyrazol-5-yl)nicotinamide (GT-D-179)

N-(4-(chlorodifluoromethoxy)phenyl)-6-(4-(piperazin-1-yl)piperidin-1-yl)-5-(1H-pyrazol-5-yl)nicotinamide (GT-D-179) was prepared by referring to Scheme M-26 (brown solid, 1.03 g). ¹H NMR (400 MHz, MeOD) δ 8.79 (d, *J =* 2.3 Hz, 1H), 8.68 (d, *J =* 2.3 Hz, 1H), 8.00 (d, *J =* 2.3 Hz, 1H), 7.88 - 7.80 (m, 2H), 7.30 (d, *J* = 9.1 Hz, 2H), 6.88 (d, *J* = 2.2 Hz, 1H), 3.90 (dd, *J =* 11.4, 5.8 Hz, 3H), 3.72 (s, 8H), 3.24 (d, *J* = 12.2 Hz, 2H), 2.31 (d, *J* = 10.7 Hz, 2H), 2.09 (dd, *J* = 16.3, 7.7 Hz, 2H). LCMS (ESI) calcd. for C₂₅H₂₉ClF₂N₇O₂⁺ [M+H]⁺: 532.2; found, 532.2.

### Intermediate Example 52: preparation of (6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50)

(6-((5-bromo-2-((2-methoxy-5-methyl-4-(piperazin-1-yl)phenyl)amino)pyrimidin-4-yl)amino)quinoxalin-5-yl)dimethylphosphine oxide (GT-S-50) was prepared by referring to Scheme M-8 (yellow solid, 950 mg). ¹H NMR (400 MHz, DMSO-d6): δ ppm 12.76 (s, 1H), 9.23 (br, 2H), 8.91(d, 3H), 8.38 - 8.32 (m, 2H), 8.00 (d, 1H), 7.49(s, 1H), 6.77 (s, 1H), 3.83 (s, 3H), 3.32 - 3.24 (m, 4H), 3.12 - 2.97 (m, 4H), 2.13 (s, 3H), 2.10 (s, 3H), 2.03 (s, 3H). LCMS (ESI) calcd. for C₂₆H₃₁BrN₈O₂P⁺ [M+H]⁺: 597.2; found, 597.1.

### Intermediate Example 53: preparation of (6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51)

(6-((5-bromo-2-((2-methoxy-5-methyl-4-(4-(methylamino)piperidin-1-yl)phenyl)amino)pyrimidin-4-yl)amino)quinoxalin-5-yl)dimethylphosphine oxide (GT-S-51) was prepared by referring to Scheme M-8 (yellow solid, 1.1 g). ¹H NMR (400 MHz, CD₃OD): δ ppm 8.94 - 8.90 (br, 3H), 8.30 (s, 1H), 8.10 - 8.07 (br, 1H) , 7.35 (s, 1H), 6.89 (s, 1H), 3.91 (s, 3H), 3.39 - 3.24 (m, 3H), 2.91 (t, *J =* 15.6 Hz, 2H), 2.82 (s, 3H), 2.30 - 2.19 (m, 11H), 1.98 - 1.84 (m, 11H), 1.98 - 1.84 (m, 2H). LCMS (ESI) calcd. for C₂₈H₃₅BrN₈O₂P⁺ [M+H]⁺: 625.2; found, 625.1.

### Intermediate Example 54: preparation of (6-((5-bromo-2-((2-methoxy-5-methyl-4-(4-(piperazin-1-yl)piperidin-1-yl)phenyl)amino)pyrimidin-4-yl)amino)quinoxalin-5-yl)dimethylphosphine oxide (GT-S-52)

(6-((5-bromo-2-((2-methoxy-5-methyl-4-(4-(piperazin-1-yl)piperidin-1-yl)phenyl)amino)pyrimidin-4-yl)amino)quinoxalin-5-yl)dimethylphosphine oxide (GT-S-52) was prepared by referring to Scheme M-8 (yellow solid, 150 mg, yield 108.88%). ¹H NMR (400 MHz, DMSO-d₆) δ ppm 9.10 - 8.80 (m, 2H), 8.41 (s, 1H), 8.05 - 7.95 (m, 1H), 7.60 - 7.50 (m, 2H), 7.48 - 7.36 (m, 3H), 7.20 - 7.10 (m, 2H), 6.85 - 6.80 (m, 1H), 3.80 - 3.62 (m, 6H), 3.60 - 3.40 (m, 6H), 3.35 - 3.30 (m, 2H), 2.85 - 2.60 (m, 2H), 2.38 - 2.25 (m, 2H), 2.20 - 1.90 (m, 11H). LCMS (ESI) calcd. for C₃₁H₄₀BrN₉O₂P⁺ [M+H]⁺: 680.2; found, 680.1.

### Intermediate Example 55: preparation of 1-isopropyl-N-((6-methyl-2-oxo-4-propyl-1,2-dihydropyridin-3-yl)methyl)-6-(6-(piperazin-1-yl)pyridin-3-yl)-1H-indazole-4-carboxamide (GT-S-7)

1-isopropyl-N-((6-methyl-2-oxo-4-propyl-1,2-dihydropyridin-3-yl)methyl)-6-(6-(piperazin-1-yl)pyridin-3-yl)-1H-indazole-4-carboxamide (GT-S-7) was prepared by referring to Scheme M-29 (yellow solid, 300 mg, yield 21%). ¹H NMR (400 MHz, DMSO-d₆) δ ppm 8.67 - 8.61 (m, 2H), 8.38 (s, 1H), 8.08 - 8.05 (br, 2H), 7.85 (s, 1H), 6.96 (d, *J =* 11.6 Hz, 1H), 5.94 (s, 1H), 5.20 - 5.11 (m, 1H), 4.44 (d, *J =* 6.4 Hz, 2H), 3.52 - 3.49 (m, 4H), 2.86 - 2.78 (m, 4H), 2.57 - 2.53 (m, 2H), 2.11 (s, 3H), 1.58 - 1.51 (m, 8H), 0.90 (t, *J* = 9.6 Hz, 3 H). LCMS (ESI) calcd. for C₃₀H₃₈N₇O₂⁺ [M+H]⁺: 528.3; found, 528.5.

### Intermediate Example 56: preparation of 1-isopropyl-N-((6-methyl-2-oxo-4-propyl-1,2-dihydropyridin-3-yl)methyl)-6-(6-(4-(methylamino)piperidin-1-yl)pyridin-3-yl)-1H-indazole-4-carboxamide (GT-S-8)

1-isopropyl-N-((6-methyl-2-oxo-4-propyl-1,2-dihydropyridin-3-yl)methyl)-6-(6-(4-(methylamino)piperidin-1-yl)pyridin-3-yl)-1H-indazole-4-carboxamide (GT-S-8) was prepared by referring to Scheme M-27 (light yellow solid, 1.8 g). ¹H NMR (400 MHz, DMSO-d₆): δ ppm 8.69 - 8.57 (m, 4H), 8.63 (s, 1H), 8.15 - 8.08 (m, 4H), 7.82 (br, 1H), 7.11 (d, *J =* 8.8 Hz, 1H), 5.91 (s, 1H), 5.17 - 5.10 (m, 1H), 4.48 - 4.41 (m, 4H), 3.30 - 3.28 (br, 1H), 2.96 (t, *J =* 12.6 Hz, 2H), 2.59 (t, *J =* 5.2 Hz, 3H), 2.54 - 2.49 (m, 4H), 2.14 (s, 3H), 2.09 - 2.07 (m, 2H), 1.57 - 1.49 (m, 8H), 0.88 (t, *J =* 7.4 Hz, 3H). LCMS (ESI) calcd. for C₃₂H₄₂N₇O₂⁺ [M+H]⁺: 556.3; found, 556.5.

### Intermediate Example 57: preparation of 1-isopropyl-N-((6-methyl-2-oxo-4-propyl-1,2-dihydropyridin-3-yl)methyl)-6-(6-(4-(piperidin-4-yl)piperazin-1-yl)pyridin-3-yl)-1H-indazole-4-carboxamide (GT-S-9)

1-isopropyl-N-((6-methyl-2-oxo-4-propyl-1,2-dihydropyridin-3-yl)methyl)-6-(6-(4-(piperidin-4-yl)piperazin-1-yl)pyridin-3-yl)-1H-indazole-4-carboxamide (GT-S-9) was prepared by referring to Scheme M-28 (light yellow solid, 2.0 g). ¹H NMR (400 MHz, DMSO-d₆): δ ppm 11.99 (br, 1H), 9.40 - 9.37 (br, 1H), 8.78 (br, 1H), 8.67 (s, 1H), 8.45 - 8.40 (m, 2H), 8.24 (s, 1H), 7.88 (s, 1H), 7.40 (d, *J =* 12 Hz, 1H), 5.99 (s, 1H), 5.24 - 5.15 (m, 1H), 4.66 - 4.62 (m, 2H), 4.45 - 4.44 (br, 2H), 3.77 - 3.18 (m, 7H), 3.25 - 3.18 (m, 2H), 2.97 - 2.90 (m, 2H), 2.58 - 2.52 (m, 4H), 2.37 - 2.33 (br, 2H), 2.17 (s, 3H), 2.10 - 2.02 (m, 2H), 1.57 - 1.51 (m, 8H), 0.89 (t, *J =* 9.6 Hz, 3H). LCMS (ESI) calcd. for C₃₅H₄₇N₈O₂⁺ [M+H]⁺: 611.4; found, 611.7.

### Intermediate Example 58: preparation of N-((4,6-dimethyl-2-oxo-1,2-dihydropyridin-3-yl)methyl)-5-(ethyl(tetrahydro-2H-pyran-4-yl)amino)-4-methyl-4'-(piperazin-1-ylmethyl)-[1,1'-biphenyl]-3-carboxamide (GT-S-10)

N-((4,6-dimethyl-2-oxo-1,2-dihydropyridin-3-yl)methyl)-5-(ethyl(tetrahydro-2H-pyran-4-yl)amino)-4-methyl-4'-(piperazin-1-ylmethyl)-[1,1'-biphenyl]-3-carboxamide (GT-S-10) was prepared by referring to Scheme M-31 (yellow solid, 1.4 g). ¹H NMR (400 MHz, DMSO-d₆): δ ppm 11.99 (br, 1H), 9.40 - 9.37 (br, 1H), 8.78 (br, 1H), 8.67 (s, 1H), 8.45 - 8.40 (m, 2H), 8.24 (s, 1H), 7.88 (s, 1H), 7.40 (d, *J=* 12 Hz, 1H), 5.99 (s, 1H), 5.24 - 5.15 (m, 1H), 4.66 - 4.62 (m, 2H), 4.45 - 4.44 (br, 2H), 3.77 - 3.18 (m, 7H), 3.25 - 3.18 (m, 2H), 2.97 - 2.90 (m, 2H), 2.58 - 2.52 (m, 4H), 2.37 - 2.33 (br, 2H), 2.17 (s, 3H), 2.10 - 2.02 (m, 2H), 1.57 - 1.51 (m, 8H), 0.89 (t, *J =* 9.6 Hz, 3H). LCMS (ESI) calcd. for C₃₄H₄₆N₅O₃⁺ [M+H]⁺: 611.4; found, 611.7.

### Intermediate Example 59: preparation of N-((4,6-dimethyl-2-oxo-1,2-dihydropyridin-3-yl)methyl)-5-(ethyl(tetrahydro-2H-pyran-4-yl)amino)-4-methyl-4'-(piperazin-1-yl)-[1,1'-biphenyl]-3-carboxamide (GT-S-11)

N-((4,6-dimethyl-2-oxo-1,2-dihydropyridin-3-yl)methyl)-5-(ethyl(tetrahydro-2H-pyran-4-yl)amino)-4-methyl-4'-(piperazin-1-yl)-[1,1'-biphenyl]-3-carboxamide (GT-S-11) was prepared by referring to Scheme M-32 (white solid, 2.6 g). ¹H NMR (400 MHz, DMSO-d₆) δ 8.21 (t, J = 6.0 Hz, 1H), 7.54 (d, J = 11.6 Hz, 2H), 7.3 (s, 1H), 7.20 (s, 1H), 7.07 (d, J = 11.2 Hz, 2H), 4.31 (d, J =6 Hz, 2H), 3.86 (d, J = 13.6 Hz, 2H), 3.35 - 3.12 (m, 15 H), 2.24 (d, J = 6.4 Hz, 6H), 2.14 (s, 2H), 1.71 - 1.48 (m, 4H), 0.86 (t, J = 9.2 Hz, 3H). LCMS (ESI) calcd. for C₃₃H₄₄N₅O₃⁺ [M+H]⁺: 558.3; found, 558.5.

### Intermediate Example 60: preparation of N-((4,6-dimethyl-2-oxo-1,2-dihydropyridin-3-yl)methyl)-5-(ethyl(tetrahydro-2H-pyran-4-yl)amino)-4-methyl-4'-(4-(methylamino)piperidin-1-yl)-[1,1'-biphenyl]-3-carboxamide (GT-S-12)

N-((4,6-dimethyl-2-oxo-1,2-dihydropyridin-3-yl)methyl)-5-(ethyl(tetrahydro-2H-pyran-4-yl)amino)-4-methyl-4'-(4-(methylamino)piperidin-1-yl)-[1,1'-biphenyl]-3-carboxamide (GT-S-12) was prepared by referring to Scheme M-33 (white solid, 1.8 g). ¹H NMR (400 MHz, DMSO-d₆) δ 8.16 (t, J = 4.8 Hz, 1H), 7.45 (d, J = 8.8 Hz, 2H), 7.33 (s, 1H), 7.16 (s, 1H), 6.98 (d, J = 8.8 Hz, 2H), 5.86 (s, 1H), 4.29 (d, J = 4.8 Hz, 2H), 3.82 (d, J = 10.4 Hz, 2H), 3.65 (d, J = 12.4 Hz, 2H), 3.25 (t, J = 11.2 Hz, 3H),3.10-3.01 (m, 4H), 2.76 (t, J = 11.6 Hz, 2H), 2.45-2.38 (m, 1H), 2.29 (s, 3H),2.21( d, J = 6.8 Hz, 6H), 2.11 (s, 3H), 1.89 - 1.85 (m, 2H), 1.66 - 1.64 (m, 2H), 1.55 - 1.49 (m, 2H), 1.32 - 1.28 (m, 2H), 0.83 (t, J = 6.8 Hz, 3H). LCMS (ESI) calcd. for C₃₅H₄₈N₅O₃⁺ [M+H]⁺: 586.4; found, 586.2.

### Intermediate Example 61: preparation of N-((4,6-dimethyl-2-oxo-1,2-dihydropyridin-3-yl)methyl)-5-(ethyl(tetrahydro-2H-pyran-4-yl)amino)-4-methyl-4'-(4-(piperidin-4-yl)piperazin-1-yl)-[1,1'-biphenyl]-3-carboxamide (GT-S-13)

N-((4,6-dimethyl-2-oxo-1,2-dihydropyridin-3-yl)methyl)-5-(ethyl(tetrahydro-2H-pyran-4-yl)amino)-4-methyl-4'-(4-(piperidin-4-yl)piperazin-1-yl)-[1,1'-biphenyl]-3-carboxamide (GT-S-13) was prepared by referring to Scheme M-34 (white solid, 1.6 g). ¹H NMR (400 MHz, DMSO-d₆) δ 8.18 (t, J = 2.0 Hz, 1H), 7.47 (d, J = 8.4 Hz, 2H), 7.33 (s, 1H), 7.16 (s, 1H), 6.98 (d, J = 8.0 Hz, 2H), 5.86 (s, 1H), 4.29 (d, J = 4.0 Hz, 2H), 4.14 - 4.06 (m, 1H), 3.82 (d, J = 9.2 Hz, 2H), 3.41 - 3.36 (m, 2H), 3.29 (d, J = 12.0 Hz, 3H), 3.14 - 2.98 (m, 9H), 3.62 (s, 4H), 2.28 - 2.21 (m, 7H), 2.11 ( s, 3H), 1.68 - 1.64 (m, 4H), 1.53 - 1.50 (m, 2H), 1.26 - 1.24 (m, 2H), 0.83 (t, J = 6.8 Hz, 3H). LCMS (ESI) calcd. for C₃₈H₅₃N₆O₃⁺ [M+H]⁺: 641.4; found, 642.0.

### Intermediate Example 62: preparation of the following intermediate compounds

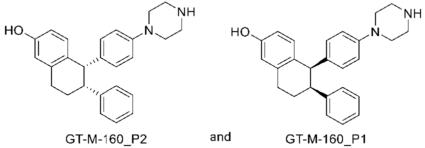

Following the method of Scheme M-36, the enantiomeric mixture product obtained according to Step 8 of Scheme 49 was separated by supercritical fluid chromatography (SFC) to obtain the two intermediate compounds as described above (their retention times were 8.232 min and 9.400 min, respectively, in terms of analytical methods). The analytical method, preparation method, and conditions for SFC were shown below.

The analysis method and conditions for SFC are as follows:

| | |
|---|---|
| System: | Shimadzu LC-20AP |
| Column name: | Daicel CHIRALPAK^{®}IE |
| Column size: | 250*4.6mm 5µm |
| Mobile Phase A: | n-Hexane |
| Mobile Phase B: | EtOH (0.2% Diethylamine) |
| Mobile Phase A: Mobile Phase B | 70:30 |
| Wavelength: | 254 nm |
| Flow | 1mL/min |
| Column temp: | 25°C |
| Injection: | 5µL |
| Solvent: | EtOH: HPLC grade |
| | n-Hexane: HPLC grade |

Preparation method for SFC is as follows:

| | |
|---|---|
| System: | Shimadzu |
| Column name: | DAICELCHIRALPAK^{®}IE |
| Column size: | 250*25 mm 10 µm |
| Mobile Phase A: | n-Hexane |
| Mobile Phase B: | ETOH (+0.1% 7.0mol/1 Ammonia in MeOH) |
| A:B: | 70:30 |
| Wavelength: | 214 nm |
| Flow: | 30ml/min |
| Column temp: | rt |
| Injection: | 1mL |
| Cycle time: | 12min |
| Solvent: | n-Hexane: redistilled grade |
| | ETOH: redistilled grade |
| Preparation of sample solution: | The sample was dissolved in approximately 30 mL of ethanol. |

One of the obtained intermediates (GT-M-160_P1)(white solid, 565 mg): ¹H NMR (400 MHz, DMSO) δ 9.11 (s, 1H), 7.23 - 7.06 (m, 3H), 6.84 (d, *J =* 6.7 Hz, 2H), 6.62 (dd, *J =* 15.5, 5.3 Hz, 2H), 6.55 - 6.43 (m, 3H), 6.21 (d, *J =* 8.6 Hz, 2H), 4.13 (d, *J =* 4.9 Hz, 1H), 3.28 (s, 2H), 3.04 - 2.91 (m, 2H), 2.91 - 2.83 (m, 4H), 2.80 - 2.70 (m, 4H), 2.11 (dd, *J* = 12.3, 6.4 Hz, 1H), 1.71 (d, *J* = 7.5 Hz, 1H). LCMS (ESI) calcd. for C₂₆H₂₉N₂O⁺ [M+H]⁺: 385.23, found, 385.30. (retention time was 8.232 min in terms of analytical method).

Another intermediate (GT-M-160_P2)(white solid, 595 mg): ¹H NMR (400 MHz, DMSO) δ 9.10 (s, 1H), 7.14 (dd, *J =* 15.2, 7.8 Hz, 3H), 6.84 (d, *J =* 6.9 Hz, 2H), 6.66 - 6.59 (m, 2H), 6.50 (dd, *J =* 14.1, 8.3 Hz, 3H), 6.22 (d, *J =* 8.4 Hz, 2H), 4.14 (d, *J =* 4.6 Hz, 1H), 3.30 - 3.23 (m, 2H), 2.97 (dd, *J =* 15.6, 9.6 Hz, 2H), 2.90 (d, *J* = 5.0 Hz, 4H), 2.80 (d, *J* = 4.5 Hz, 4H), 2.11 (dd, *J* = 12.2, 6.8 Hz, 1H), 1.72 (s, 1H). LCMS (ESI) calcd. for C₂₆H₂₉N₂O⁺ [M+H]⁺: 385.23, found, 385.30. (retention time was 9.400 min in terms of analytical method).

### Intermediate Example 63: preparation of the following intermediate compounds

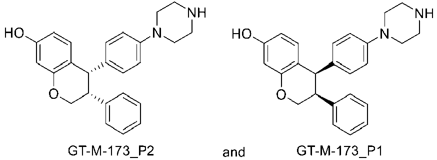

Following the method of Scheme M-37, the enantiomeric mixture product obtained according to Step 6 of Scheme M-37 was separated by supercritical fluid chromatography (SFC) to obtain the two intermediate compounds as described above (their retention times were 1.763 min and 3.506 min, respectively, in terms of analytical methods). The analytical method, preparation method, and conditions for SFC were shown below.

The analysis method and conditions for SFC are as follows:

| | | | |
|---|---|---|---|
| System: | Waters Ultra Performance Convergence Chromatography (UPCC) (CA-185) | | |
| Column name: | DAICELCHIRALPAK^{®}OD | | |
| Column size: | 100*3.0 mm *3.0 µm | | |
| Mobile Phase A: | Supercritical CO₂ | | |
| Mobile Phase B: | Isopropanol (0.1% Diethylamine) | | |
| Wavelength: | 214nm | | |
| Flow | 1.5 mL/min | | |
| Column temp: | 35°C | | |
| Back Pressure(psi): | 1800 psi | | |
| Injection: | 0.3 µL | | |
| Run time: | 8 min | | |
| Gradient | Time (min) | A(%V/V) | B(%V/V) |
| | 0.00 | 60 | 40 |
| | 8.00 | 60 | 40 |
| Solvent: | Isopropanol: HPLC grade | | |
| | Supercritical CO₂ : Food grade | | |
| Preparation of mobile phases: | Mobile Phase B: Add 1 mL Diethylamine in 1000 mL Isopropanol, then ultrasonic degassing for 15min. | | |

Preparation method for SFC is as follows:

| | |
|---|---|
| System: | Waters SFC 150 |
| Column name: | DAICELCHIRALCEL^{®}OD |
| Column size: | 250*25 mm 10 µm |
| Mobile Phase A: | Supercritical CO₂, |
| Mobile Phase B: | Isopropanol (+0.1% 7.0mol/l Ammonia in MEOH) |
| A:B: | 45:55 |
| Wavelength: | 214 nm |
| Flow: | 80ml/min |
| Column temp: | RT |
| Back Pressure: | 100 bar |
| Injection: | 1.5mL |
| Cycle time: | 6min |
| Solvent: | Isopropanol: redistilled grade |
| | Supercritical CO₂: Food grade |

One of the obtained intermediates (GT-M-173_P1)(white solid, 555 mg): ¹H NMR (400 MHz, DMSO) δ 9.33 (s, 1H), 7.22 (dd, *J =* 20.1, 17.2 Hz, 3H), 6.88 (d, *J =* 45.0 Hz, 2H), 6.71 (d, *J =* 8.3 Hz, 1H), 6.64 (d, *J =* 8.6 Hz, 2H), 6.44 (d, *J =* 8.5 Hz, 2H), 6.39 - 6.22 (m, 2H), 4.39 (t, *J =* 11.1 Hz, 1H), 4.30 - 4.06 (m, 2H), 3.55 (dt, *J* = 39.1, 19.2 Hz, 1H), 2.95 (d, *J* = 4.8 Hz, 4H), 2.83 (d, *J* = 4.5 Hz, 4H). LCMS (ESI) calcd. for C₂₅H₂₇N₂O₂⁺ [M+H]⁺: 387.21, found, 387.40.(retention time was 1.763 min in terms of analytical method).

Another intermediate (GT-M-173_P2)(white solid, 495 mg): ¹H NMR (400 MHz, DMSO) δ 9.33 (s, 1H), 7.22 (dd, *J =* 20.1, 17.2 Hz, 3H), 6.88 (d, *J =* 45.0 Hz, 2H), 6.71 (d, *J =* 8.3 Hz, 1H), 6.64 (d, *J =* 8.6 Hz, 2H), 6.44 (d, *J =* 8.5 Hz, 2H), 6.39 - 6.22 (m, 2H), 4.39 (t, *J =* 11.1 Hz, 1H), 4.30 - 4.06 (m, 2H), 3.55 (dt, *J =* 39.1, 19.2 Hz, 1H), 2.95 (d, *J =* 4.8 Hz, 4H), 2.83 (d, *J =* 4.5 Hz, 4H). LCMS (ESI) calcd. for C₂₅H₂₇N₂O₂⁺ [M+H]⁺: 387.21, found, 387.40.( retention time was 3.506 min in terms of analytical method).

### Intermediate Example 64: preparation of N-(3-(difluoromethyl)-1-(1-methylpiperidin-4-yl)-1H-pyrazol-4-yl)-5-morpholinopyrazolo[1,5-a]pyrimidine-3-carboxamide (GT-04349)

The target compound (GT-04349) was prepared by referring to Scheme M-35 (white solid, 15 mg, yield 67.08%). ¹H NMR (400 MHz, MeOD) δ 8.45 (d, *J=* 8.0 Hz, 1H), 8.33 (s, 1H), 8.22 (s, 1H), 6.97 - 6.59 (m, 2H), 4.50 (s, 2H), 3.69 (dd, *J=* 35.3, 32.6 Hz, 10H), 3.16 (s, 1H), 2.85 (s, 3H), 2.29 (s, 4H). LCMS (ESI) calcd. for C₂₁H₂₇F₂N₈O₂⁺ [M+H]⁺: 461.22, found, 461.2.

### Intermediate Example 65: preparation of 2-(6,7-dihydro-5H-pyrrolo[1,2-c]imidazol-1-yl)-2-(4-fluoro-1-oxo-6-(4-(4-(piperazin-1-yl)piperidin-1-yl)phenyl)isoindolin-2-yl)-N-(thiazol-2-yl)acetamide (GT-D-219)

The target compound GT-D-219 was prepared by referring to Scheme M-38 (yellow solid, 1.1 g, yield 113.53%). ¹H NMR (400 MHz, MeOD) δ 8.92 (s, 1H), 8.03 - 7.82 (m, 5H), 7.77 (dd, J = 10.0, 1.2 Hz, 1H), 7.58 (d, J = 4.0 Hz, 1H), 7.34 (d, J = 4.0 Hz, 1H), 4.95 (d, J = 17.2 Hz, 2H), 4.65 (d, J = 17.2 Hz, 1H), 4.44 - 4.29 (m, 2H), 4.00 (d, J = 12.0 Hz, 3H), 3.92 - 3.65 (m, 10H), 3.10 - 2.97 (m, 1H), 2.94 - 2.82 (m, 1H), 2.78 - 2.48 (m, 6H). LCMS (ESI) calcd. for C₃₄H₃₈FN₈O₂S⁺ [M+H]⁺: 641.28, found, 641.4.

### Intermediate Example 66: preparation of 2-(6,7-dihydro-5H-pyrrolo[1,2-c]imidazol-1-yl)-2-(4-fluoro-1-oxo-6-(4-(4-(piperidin-4-yl)piperazin-1-yl)phenyl)isoindolin-2-yl)-N-(thiazol-2-yl)acetamide (GT-D-217)

The target compound GT-D-217 was prepared by referring to Scheme M-39 (yellow solid, 800 mg, yield 99.45%). ¹H NMR (400 MHz, MeOD) δ 8.90 (s, 1H), 7.88 (s, 1H), 7.66 (d, J = 8.6 Hz, 3H), 7.57 - 7.51 (m, 1H), 7.32 - 7.26 (m, 1H), 7.18-6.89 (m, 3H), 4.91 (d, J = 16.0 Hz, 1H), 4.82-4.75 (m, 1H), 4.58 (d, J = 17.0 Hz, 1H), 4.38-4.33 (m, 2H), 4.01 (s, 2H), 3.78 (s, 2H), 3.64 (d, J = 12.0 Hz, 2H), 3.38 (s, 2H), 3.26 (s, 2H), 3.18-3.12 (m, 2H), 3.07-2.99 (m, 1H), 2.91 - 2.84 (m, 1H), 2.76 - 2.63 (m, 2H), 2.52 (d, J = 12.0 Hz, 2H), 2.18 - 2.05 (m, 2H). LCMS (ESI) calcd. for C₃₄H₃₈FN₈O₂S⁺ [M+H]⁺: 641.28, found, 641.4.

### Intermediate Example 67: preparation of 2-(6-(4-((1S,4S)-2,5-diazabicyclo[2.2.1]heptan-2-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-222)

Following steps 2-5 in Scheme M-38, the reaction of the starting materials ethyl 2-(6-bromo-4-fluoro-1-oxoisoindolin-2-yl)-2-(6,7-dihydro-5H-pyrrolo[1,2-c]imidazol-1-yl)acetate (CAS NO.: 2407965-04-0) and (1S,4S)-tert-butyl 5-(4-(4,4,5,5-tetramethyl-1,3,2-dioxaborolan-2-yl)phenyl)-2,5-diazabicyclo[2.2.1]heptane-2-carboxylate (CAS NO.: 942189-80-2) afforded the target compound GT-D-222 (off-white solid, 0.56 g, yield 109.7%). ¹H NMR (400 MHz, MeOD) δ 8.90 (s, 1H), 7.85 (d,J = 1.2 Hz, 1H), 7.69 - 7.59 (m, 3H), 7.54 (d, J =4.0 Hz, 1H), 7.29 (d, J = 4.0 Hz, 1H), 6.81 (d,J = 8.0 Hz, 2H), 4.91 (d, J = 10.0 Hz, 1H), 4.87 - 4.81 (m,1H), 4.74 (s, 1H), 4.62 - 4.50 (m, 2H), 4.35 (dd, *J =* 12.0, 7.2 Hz, 2H), 3.79 (dd, J = 10.0, 2.4 Hz, 1H),3.40 (s, 2H), 3.06-2.81 (m, 2H), 2.77 - 2.61 (m, 2H), 2.33 (d, J = 10.0 Hz, 1H), 2.09 (d,J = 12.0 Hz, 1H), 2.00 (d, J = 8.8 Hz, 1H), 1.60 (s, 1H). LCMS (ESI) calcd. for C₃₀H₂₉FN₇O₂S⁺ [M+H]⁺: 570.21, found, 570.2.

### Intermediate Example 68: preparation of 2-(6,7-dihydro-5H-pyrrolo[1,2-c]imidazol-1-yl)-2-(4-fluoro-6-(4-(4-(methylamino)piperidin-1-yl)phenyl)-1-oxoisoindolin-2-yl)-N-(thiazol-2-yl)acetamide (GT-D-221)

Following steps 2-5 in Scheme M-38, the reaction of the starting materials ethyl 2-(6-bromo-4-fluoro-1-oxoisoindolin-2-yl)-2-(6,7-dihydro-5H-pyrrolo[1,2-c]imidazol-1-yl)acetate and tert-butyl (1-(4-(4,4,5,5-tetramethyl-1,3,2-dioxaborolan-2-yl)phenyl)piperidin-4-yl)carbamate (CAS No.: 2235416-80-3) afforded the target compound GT-D-221 (yellow solid, 0.72 g, yield 105.4%). ¹H NMR (400 MHz, MeOD) δ 8.91 (s, 1H), 7.96 (d, *J* = 1.2 Hz, 1H), 7.92 (d, *J =* 3.2 Hz, 3H), 7.77 (dd, *J =* 10.0, 1.2 Hz, 1H), 7.55 (d, *J =* 3.6 Hz, 1H), 7.30 (d, *J* = 3.6 Hz, 1H), 6.51 (s, 1H), 4.96 (d, *J =* 17.2 Hz, 1H), 4.88 (s, 1H), 4.83 (s, 1H), 4.63 (d, *J =* 17.2 Hz, 1H), 4.34 (dt, *J =* 12.0, 7.2 Hz, 2H), 3.93 (d, *J =* 12.0 Hz, 2H), 3.85 (t, *J =* 11.2 Hz, 2H), 3.63 (dd, *J =* 20.0, 9.6 Hz, 1H), 3.08 - 2.98 (m, 1H), 2.91 - 2.83 (m, 1H), 2.81 (s, 3H), 2.74 - 2.63 (m, 2H), 2.51 (d, *J =* 12.0 Hz, 2H), 2.44 - 2.31 (m, 2H). LCMS (ESI) calcd. for C₃₁H₃₃FN₇O₂S⁺ [M+H]⁺: 586.24, found, 586.3.

### Intermediate Example 69: preparation of 2-(6-(4-(3,8-diazabicyclo[3.2.1]octan-3-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-224)

With reference to Scheme M-38, replacement of the Step 1 compound (CAS No.: 2762613-62-5) with the starting compound 3-(4-bromophenyl)-3,8-diazabicyclo[3.2.1]octane-8-carboxylate (CAS No.: 1146427-86-2) afforded the target compound GT-D-224 as an off-white solid (619 mg, yield 105.0%). ¹H NMR (400 MHz, MeOD) δ 8.90 (s, 1H), 7.88 (d, J = 1.0 Hz, 1H), 7.65 (dd, J = 10.0, 7.2 Hz, 3H), 7.50 (d, J = 4.0 Hz, 1H), 7.24 (d, J = 4.0 Hz, 1H), 7.07 (d, J = 8.0 Hz, 2H), 4.91 (d, J = 16.0 Hz, 1H), 4.55 (d, J = 16.0 Hz, 1H), 4.35 (dd, J = 12.0, 8.0 Hz, 2H), 4.22 (s, 2H), 4.10 (q, J = 7.2 Hz, 1H), 3.80 (d, J = 10.0 Hz, 2H), 3.16 (t, J = 12.0 Hz, 2H), 3.09 - 2.94 (m, 1H), 2.92 - 2.77 (m, 1H), 2.76 - 2.57 (m, 2H), 2.16 (s, 4H). LCMS (ESI) calcd. for C₃₁H₃₁FN₇O₂S⁺ [M+H]⁺: 584.22, found, 584.2.

### Intermediate Example 70: preparation of 2-(6-(4-((1R,4R)-2,5-diazabicyclo[2.2.1]heptan-2-yl)phenyl)-4-fluoro-1-oxoisoindolin-2-yl)-2-(6,7-dihydro-SH-pyrrolo[1,2-c]imidazol-1-yl)-N-(thiazol-2-yl)acetamide (GT-D-223)

With reference to Steps 2-5 of Scheme M-38, the reaction of the starting compounds ethyl 2-(6-bromo-4-fluoro-1-oxoisoindolin-2-yl)-2-(6,7-dihydro-5H-pyrrolo[1,2-c]imidazol-1-yl)acetate (CAS No.: 2407965-04-0) and (1R,4R)-tert-butyl 5-(4-(4,4,5,5-tetramethyl-1,3,2-dioxaborolan-2-yl)phenyl)-2,5-diazabicyclo[2.2.1]heptane-2-carboxylate (CAS No.: 2654825-27-9) afforded the target compound GT-D-223 as an off-white solid (1.3 g, 101.7% yield). ¹H NMR (400 MHz, MeOD) δ 8.90 (s, 1H), 7.84 (d, J = 4.0 Hz, 1H), 7.64-7.58 (m, 4H), 7.35(d, J = 4.0 Hz, 1H), 6.81 (d, J = 8.7 Hz, 2H), 4.92 (s, 1H), 4.87 - 4.84 (m, 1H), 4.74 (s, 1H), 4.67 - 4.48(m, 2H), 4.39-4.32 (m, 2H), 3.80-3.77 (m, 1H), 3.40 (s, 3H), 3.11 - 2.96 (m,1H), 2.95 - 2.82 (m, 1H), 2.72-2.66 (m, 2H), 2.32 (d, J = 8.0 Hz, 1H), 2.10 (d, J = 12.0 Hz,1H). LCMS (ESI) calcd. for C₃₀H₂₉FN₇O₂S⁺ [M+H]⁺: 570.21, found, 570.2.

### Intermediate Example 71: preparation of 2-(6-(4-(4-(3,3-difluoropiperidin-4-yl)piperazin-1-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-218)

With reference to Steps 3-6 of Scheme M-39, the reaction of the starting compounds ethyl 2-(6,7-dihydro-5H-pyrrolo[1,2-c]imidazol-1-yl)-2-(4-fluoro-1-oxo-6-(4-(piperazin-1-yl)phenyl)isoindolin-2-yl)acetate (Compound 2) and tert-butyl 3,3-difluoro-4-oxopiperidine-1-carboxylate afforded the target compound GT-D-218 (off-white solid, 645 mg, yield 103.32%). ¹H NMR (400 MHz, MeOD) δ 8.92 (s, 1H), 7.93 (d,J = 1.2 Hz, 1H), 7.84 (d,J = 8.8 Hz, 2H), 7.74 (dd,J = 10.0, 1.2 Hz, 1H), 7.69 - 7.55 (m, 3H), 7.42 (d, J = 4.0 Hz, 1H), 4.95 (s, 2H), 4.68 (d, *J =* 16.0 Hz,1H), 4.45 - 4.24 (m, 2H), 3.88 (t, J = 8.0 Hz, 2H), 3.76 (s, 4H), 3.67 - 3.49 (m, 6H), 3.33 (d, J = 12.0 Hz,1H), 3.10 - 2.96 (m, 1H), 2.96 - 2.81 (m, 1H), 2.77 - 2.59 (m, 2H), 2.49 (d, J = 12.0 Hz, 1H), 2.37 - 2.18(m, 1H). LCMS (ESI) calcd. for C₃₄H₃₆F₃N₈O₂S⁺ [M+H]⁺: 677.26, found, 677.3.

### Intermediate Example 72: preparation of N-(5-(2-hydroxypropan-2-yl)-2-(piperidin-4-yl)benzo[d]oxazol-6-yl)-6-(trifluoromethyl)picolinamide (GT-D-208)

With reference to Scheme M-40, the target compound GT-D-208 was obtained as a yellow solid (1.7 g, 109% yield). ¹H NMR (400 MHz, MeOD) δ 8.79 (s, 1H), 8.48 (d, *J =* 8.0 Hz, 1H), 8.30 (t, *J =* 8.0 Hz, 1H), 8.04 (d, *J =* 7.6 Hz, 1H), 7.71 (s, 1H), 3.56 - 3.48 (m, 3H), 3.47 - 3.41 (m, 1H), 3.28 - 3.20 (m, 2H), 2.45 (dd, *J* = 14.4, 3.6 Hz, 2H), 2.23 - 2.11 (m, 2H), 1.71 (s, 6H). LCMS (ESI) calcd. for C₂₀H₂₄F₃N₄O₃⁺ [M+H]⁺: 449.18, found, 449.2.

### Intermediate Example 73: preparation of N-(6-(2-hydroxypropan-2-yl)-2-(piperidin-4-yl)-2H-indazol-5-yl)-6-(trifluoromethyl)picolinamide (GT-D-210)

With reference to Scheme M-41, the target compound GT-D-210 was obtained as a yellow solid (1.52 g, yield 109.4%). ¹H NMR (400 MHz, D₂O) δ 8.20 (s, 1H), 8.10 (s, 1H), 8.04 (d, *J =* 4.0 Hz, 2H), 7.85 - 7.79 (m, 1H), 7.49 (s, 1H), 4.67 - 4.62 (m, 1H), 3.57 (d, *J =* 13.2 Hz, 2H), 3.20 (dd, *J =* 12.8, 10.0 Hz, 2H), 2.34 - 2.14 (m, 4H), 1.54 (s, 6H). LCMS (ESI) calcd. for C₂₂H₂₅F₃N₅O₂⁺ [M+H]⁺: 448.20, found, 448.2.

### Intermediate Example 74: preparation of 2-(6-(4-(3,3-difluoro-4-(piperazin-1-yl)piperidin-1-yl)phenyl)-4-fluoro-1-oxoisoindolin-2-yl)-2-(6,7-dihydro-5H-pyrrolo[1,2-c]imidazol-1-yl)-N-(thiazol-2-yl)acetamide (GT-D-220)

With reference to Scheme M-42, the target compound GT-D-220 was obtained as a brown solid (0.5 g, yield 66.6%). ¹H NMR (400 MHz, MeOD) δ 7.83 (s, 1H), 7.65 (s, 1H), 7.60-7.58 (m, 3H), 7.44 (d, J = 3.6 Hz, 1H), 7.15 (d, J = 4.0 Hz, 1H), 7.06 (d, J = 8.0 Hz, 2H), 4.85 (s, 1H), 4.81 (s, 1H), 4.25 (d, J = 16.0 Hz, 1H), 4.12-4.04 (m, 2H), 3.98-3.91 (m, 2H), 3.19 (d, J = 4.0 Hz, 4H), 3.16 - 2.76 (m, 8H), 2.65-2.56 (m, 3H), 2.11-2.06 (m, 1H), 2.00 - 1.86 (m, 1H). LCMS (ESI) calcd. for C₃₄H₃₆F₃N₈O₂S⁺ [M+H]⁺: 677.26, found, 677.4.

### Intermediate Example 75: preparation of N-(6-methoxy-2-(piperidin-4-yl)-2H-indazol-5-yl)-6-(trifluoromethyl)picolinamide (GT-D-209)

With reference to Scheme M-43, the target compound GT-D-209 was obtained as a yellow solid (4.2 g, yield 89.70%). ¹H NMR (400 MHz, MeOD) δ 8.82 (s, 1H), 8.78 (s, 1H), 8.40 (d, J = 7.8 Hz, 1H), 8.29 (t, J = 7.8 Hz, 1H), 8.04 (d, J = 7.8 Hz, 1H), 7.11 (s, 1H), 5.19 - 5.05 (m, 1H), 4.09 (s, 3H), 3.71 (d, J = 13.2 Hz, 2H), 3.45 - 3.34 (m, 2H), 2.71 - 2.44 (m, 4H). LCMS (ESI) calcd. for C₂₀H₂₁F₃N₅O₂⁺ [M+H]⁺: 420.16, found, 420.2.

### Intermediate Example 76: N-(6-methoxy-2-(piperidin-4-yl)-2H-indazol-5-yl)-3-(trifluoromethyl)benzamide (GT-D-225)

With reference to Scheme M-43, the target compound GT-D-225 was obtained as a white solid (1.8 g, yield 92.95 %). ¹H NMR (400 MHz, MeOD) δ 8.58 (s, 1H), 8.53 (s, 1H), 8.27 (s, 1H), 8.23 (d, J *=* 8.0 Hz,1H), 7.94 (d, J = 7.6 Hz, 1H), 7.78 (t, J = 7.6 Hz, 1H), 7.17 (s, 1H), 5.03 - 4.94 (m, 1H),4.07 (s, 3H), 3.68 (d, J = 13.2 Hz, 2H), 3.39 - 3.34 (m, 2H), 2.71 - 2.30 (m, 4H). LCMS (ESI) calcd. for C₂₁H₂₂F₃N₄O₂⁺ [M+H]⁺: 419.17, found, 419.0.

### Intermediate Example 77: N-(1-((1s,4s)-4-(hydroxymethyl)cyclohexyl)-5-(4-(piperidin-4-yl)piperazin-1-yl)-1H-benzo[d]imidazol-2-yl)-3-(trifluoromethyl)benzamide (GT-D-131)

With reference to Scheme M-44, the target compound GT-D-131 was obtained as a brown solid (410 mg, yield 42.96%). ¹H NMR (400 MHz, DMSO) δ 8.49 (d, *J =* 7.6 Hz, 1H), 8.44 (s, 1H), 7.88 (d, *J =* 7.8 Hz, 1H), 7.73 (t, *J =* 7.6 Hz, 1H), 7.43 (d, *J =* 8.8 Hz, 1H), 7.15 (d, *J =* 2.2 Hz, 1H), 6.94 (dd, *J =* 8.8, 2.4 Hz, 1H), 4.69 (s, 1H), 4.56 (s, 1H), 4.43 (s, 1H), 3.68 (s, 2H), 3.39 (s, 2H), 3.07 (d, *J =* 5.2 Hz, 4H), 3.03 (d, *J =* 12.8 Hz, 2H), 2.66 (d, *J =* 4.4 Hz, 4H), 2.46 (d, *J =* 11.6 Hz, 2H), 2.32 (d, *J =* 3.6 Hz, 1H), 1.94 (d, *J =* 13.2 Hz, 2H), 1.85 (s, 1H), 1.76 (d, *J =* 11.2 Hz, 2H), 1.68 (d, *J =* 9.6 Hz, 1H), 1.61 (d, *J =* 10.4 Hz, 2H), 1.33 (dt, *J =* 11.2, 9.6 Hz, 2H). LCMS (ESI) calcd. for C₃₁H₄₀F₃N₆O₂⁺ [M+H]⁺: 585.32, found, 585.2.

### Intermediate Example 78: N-(1-((1s,4s)-4-(hydroxymethyl)cyclohexyl)-5-(piperazin-1-yl)-1H-benzo[d]imidazol-2-yl)-3-(trifluoromethyl)benzamide (GT-D-129)

With reference to Steps 1-6 and Step 9 of Scheme M-44, the target compound GT-D-129 was obtained as a yellow solid (1 g, yield 42.23%). ¹H NMR (400 MHz, DMSO) δ 8.61 - 8.42 (m, 2H), 7.94 (s, 1H), 7.80 (s, 1H), 7.51 (d, J = 8.0 Hz, 1H), 7.22 (s, 1H), 7.01 (d, J = 7.6 Hz, 1H), 4.76-4.64 (m, 2H), 3.74 (s, 2H), 3.11 (s, 4H), 2.99 (s, 4H), 2.05-1.91 (m, 4H), 1.69 (s, 4H). LCMS (ESI) calcd. for C₂₆H₃₁F₃N₅O₂⁺ [M+H]⁺: 502.24, found, 502.3.

### Example 1 of compound of Formula (II): Preparation of 3-(5-(hydroxymethyl)-1-thioxoisoindolin-2-yl)piperidine-2,6-dione (GT-06977)

The target compound (GT-06977) was prepared by referring to the method of Scheme II-1.

Step 1: To a solution of 3-(5-bromo-1-oxoisoindolin-2-yl)piperidine-2,6-dione (20 g, 61.891 mmol) in 1,4-dioxane (200 mL) was added Lawesson's reagent (11.26 g, 27.851 mmol). The reaction mixture was stirred at 110°C for 12 hours. Reaction completion was monitored by TLC. The reaction mixture was concentrated, and the residue was purified by column chromatography to afford 3-(5-bromo-1-thioisoindolin-2-yl)piperidine-2,6-dione as an off-white solid (10 g, 29.480 mmol, yield 47.64%).

Step 2: To a solution of 3-(5-bromo-1-thioisoindolin-2-yl)piperidine-2,6-dione (6 g, 17.688 mmol) and (tributyl-λ4-stannanyl)methanol (8.52 g, 26.532 mmol) in 1,4-dioxane (60 mL) was added XPhos Pd G3 (0.1 g, 0.147 mmol). The reaction mixture was stirred at 100°C under an argon atmosphere for 3 hours. The reaction mixture was concentrated, and the residue was purified by column chromatography to afford the target compound (GT-06977) as a gray solid (1 g, yield 19.46%). ¹H NMR (400 MHz, DMSO) δ 11.17 (s, 1H), 7.91 (d, J = 7.9 Hz, 1H), 7.66 (s, 1H), 7.55 (d, J = 8.0 Hz, 1H), 6.09 - 5.91 (m, 1H), 5.49 (t, J = 5.7 Hz, 1H), 4.91 - 4.67 (m, 4H), 3.13 - 2.93 (m, 1H), 2.79 - 2.58 (m, 2H), 2.16 (dd, J = 8.9, 3.6 Hz, 1H). LCMS (ESI) calcd. for C₁₄H₁₅N₂O₃S⁺ [M+H]+: 291.08, found, 291.0.

### Example 2 of compound of Formula (II): Preparation of 3-(4-(hydroxymethyl)-1-thioxoisoindolin-2-yl)piperidine-2,6-dione (GT-06981)

With reference to the method of Scheme II-1 or Example 1 of compound of Formula (II), the target compound (GT-06981) was obtained as a gray solid (0.8 g, yield 33.32%). ¹HNMR (400 MHz, DMSO) δ 11.12 (s, 1H), 7.80 (d, J = 7.4 Hz, 1H), 7.61 (d, J = 7.4 Hz, 1H), 7.53 (t, J = 7.6 Hz, 1H), 5.98 (d, J = 8.3 Hz, 1H), 5.37 (d, J = 5.4 Hz, 1H), 4.79 (dd, J = 49.7, 20.2 Hz, 2H), 4.65 (d, J = 4.1 Hz, 2H), 2.96 (s, 1H), 2.64 (d, J = 18.4 Hz, 2H), 2.09 (dd, J = 8.9, 3.5 Hz, 1H). ¹³C NMR (101 MHz, DMSO-d6) δ 194.06, 173.08, 170.28, 139.30, 138.79, 137.62, 130.10, 128.82, 124.03, 61.22, 56.07, 31.53, 22.58. LCMS (ESI) calcd. for C₁₄H₁₅N₂O₃S⁺ [M+H]+: 291.08, found, 291.0.

### Example 3 of compound of Formula (II): Preparation of 3-(6-(hydroxymethyl)-1-thioxoisoindolin-2-yl)piperidine-2,6-dione (GT-05566)

With reference to the method of Scheme II-1 or Example 1 of compound of Formula (II), the target compound (GT-05566) was obtained as a gray solid (1 g, yield 38.91%). ¹HNMR (400 MHz, DMSO) δ 11.06 (d, J = 31.4 Hz, 1H), 7.86 (d, J = 19.3 Hz, 1H), 7.60 (d, J = 7.8 Hz, 2H), 5.95 (d, J = 8.9 Hz, 1H), 5.41 (t, J = 5.7 Hz, 1H), 4.82 - 4.60 (m, 4H), 3.00 - 2.90 (m, 1H), 2.64 (d, J = 17.9 Hz, 1H), 2.54 (s, 1H), 2.16 - 2.05 (m, 1H). LCMS (ESI) calcd. for C₁₄H₁₅N₂O₃S⁺ [M+H]+: 291.08, found, 291.0.

### Example 4 of compound of Formula (II): Preparation of 3-(5-bromo-1-thioxoisoindolin-2-yl)piperidine-2,6-dione (GT-09531)

The target compound (GT-09531) was prepared by referring to the method of step 1 of Scheme II-1.

To a solution of 3-(5-bromo-1-oxoisoindolin-2-yl)piperidine-2,6-dione (1.0 eq.) in 1,4-dioxane (20 mL) was added Lawesson's reagent (0.4 eq.). The reaction mixture was stirred at 110°C for 12 hours. Reaction completion was monitored by TLC. The reaction mixture was concentrated, and the residue was purified by column chromatography to afford the target compound (GT-09531) as an off-white solid (yield 48%). ¹H NMR (400 MHz, DMSO) δ 11.11 (s, 1H), 7.94 (s, 1H), 7.79 (dd, J = 23.5, 8.2 Hz, 2H), 5.91 (dd, J = 12.8, 4.5 Hz, 1H), 4.77 (dd, J = 52.4, 20.3 Hz, 2H), 3.03 - 2.86 (m, 1H), 2.59 (dd, J = 39.1, 11.0 Hz, 2H), 2.15 - 2.03 (m, 1H). LCMS (ESI): calcd. for [M+H]⁺: 338.97, found, 339.0.

### Example 5 of compound of Formula (II): Preparation of 3-(1-thioxoisoindolin-2-yl)piperidine-2,6-dione (GT-05563)

To a solution of 3-(5-bromo-1-thioisoindolin-2-yl)piperidine-2,6-dione (1.0 eq.) in 1,4-dioxane (10 mL) was added XPhos Pd G3 (0.02 eq.). The reaction mixture was stirred at 100°C under an argon atmosphere for 3 hours. The reaction mixture was concentrated, and the residue was purified by column chromatography to afford the target compound (GT-05563) as a gray solid (yield 9.5%). ¹H NMR (400 MHz, DMSO-d6) δ 11.13 (s, 1H), 7.92 (d, *J* = 7.6 Hz, 1H), 7.68 (d, *J =* 3.3 Hz, 2H), 7.61 - 7.53 (m, 1H), 6.14 - 5.80 (m, 1H), 4.77 (dd, *J =* 56.0, 20.0 Hz, 2H), 3.04 - 2.90 (m, 1H), 2.78 - 2.54 (m, 2H), 2.17 - 2.05 (m, 1H). LCMS (ESI) calcd. for C₁₃H₁₃N₂O₂S⁺ [M+H]⁺: 261.07, found, 261.1.

### Example 6 of compound of Formula (II): Preparation of (2-(2,6-dioxopiperidin-3-yl)-1-thioxoisoindolin-5-yl)methyl methanesulfonate (GT-09596)

With reference to the method of Scheme II-2, the target compound GT-09596 was obtained (343 mg, crude, yield 112%). LCMS (ESI) calcd. for C₁₅H₁₇N₂O₅S₂⁺ [M+H]⁺: 369.06, found, 369.1.

### Example 7 of compound of Formula (II): Preparation of ((2-(2,6-dioxopiperidin-3-yl)-1-thioxoisoindolin-4-yl)methyl methanesulfonate (GT-09597)

With reference to the method of Scheme II-2, the target compound GT-09597 was obtained (500 mg, crude, yield 120%). LCMS (ESI) calcd. for C₁₅H₁₇N₂O₅S₂⁺ [M+H]⁺: 369.06, found, 369.1.

### Example 8 of compound of Formula (II): Preparation of (2-(2,6-dioxopiperidin-3-yl)-3-thioxoisoindolin-5-yl)methyl methanesulfonate (GT-09598)

With reference to the method of Scheme II-2, the target compound GT-09598 was obtained (190 mg, crude, yield 115%). LCMS (ESI) calcd. for C₁₅H₁₇N₂O₅S₂⁺ [M+H]⁺: 369.06, found, 369.1.

### Example 1: Preparation of 2-(2-((cyclopropylmethyl)amino)pyridin-4-yl)-N-(3-(difluoromethyl)-1-(1-((2-(2,6-dioxopiperidin-3-yl)-1-thioxoisoindolin-5-yl)methyl)piperidin-4-yl)-1H-pyrazol-4-yl)oxazole-4-carboxamide (GT-05286)

With reference to the method of Scheme T-1, the target compound (GT-05286) was obtained (white solid, 12 mg, yield 9.01%). ¹H NMR (400 MHz, DMSO) δ 11.15 (s, 1H), 10.27 (s, 1H), 9.86 (s, 1H), 9.01 (s, 1H), 8.22 (s, 1H), 8.12 (s, 1H), 8.01 (d, J = 8.0 Hz, 1H), 7.88 (s, 1H), 7.76 (d, J = 7.8 Hz, 1H), 7.33 (s, 1H), 7.20 (s, 1H), 7.14 (s, 1H), 5.95 (s, 1H), 4.82 (dd, J = 52.8, 20.0 Hz, 2H), 4.51 (s, 2H), 3.53 (s, 2H), 3.23 - 3.11 (m, 6H), 2.98 (s, 1H), 2.27 (s, 4H), 2.09 (s, 1H), 1.10 (s, 1H), 0.52 (s, 2H), 0.27 (s, 2H). LCMS (ESI) calcd. for C₃₆H₃₈F₂N₉O₄S⁺ [M+H]⁺: 730.27, found, 730.3.

### Example 2: Preparation of N-(3-(difluoromethyl)-1-(1-((2-(2,6-dioxopiperidin-3-yl)-1-thioxoisoindolin-5-yl)methyl)piperidin-4-yl)-1H-pyrazol-4-yl)-2-(2-((2,2,2-trifluoroethyl)amino)pyridin-4-yl)oxazole-4-carboxamide (GT-05292)

With reference to the method of Scheme T-1, the target compound (GT-05292) was obtained (white solid, 8 mg, yield 7.68%). ¹H NMR (400 MHz, DMSO) δ 11.14 (s, 1H), 10.80 (s, 1H), 9.83 (s, 1H), 8.99 (d, J = 9.3 Hz, 1H), 8.27 - 8.19 (m, 2H), 7.97 (dd, J = 18.9, 9.9 Hz, 2H), 7.82 - 7.70 (m, 2H), 7.38 - 7.09 (m, 3H), 5.97 (d, J = 9.0 Hz, 1H), 4.82 (dd, J = 54.6, 20.4 Hz, 2H), 4.54 (d, J = 32.7 Hz, 3H), 4.35 - 4.21 (m, 2H), 3.53 (s, 2H), 3.39 - 3.32 (m, 1H), 3.14 (d, J = 11.7 Hz, 2H), 2.98 (t, J = 12.9 Hz, 1H), 2.65 (d, J = 15.1 Hz, 1H), 2.39 - 2.22 (m, 4H), 2.16 - 2.06 (m, 1H). LCMS (ESI) calcd. for C₃₄H₃₃F₅N₉O₄S⁺ [M+H]⁺: 758.23, found, 758.3.

### Example 3: Preparation of N-(5-(4-((2-(2,6-dioxopiperidin-3-yl)-1-thioxoisoindolin-5-yl)methyl)piperazin-1-yl)-1-((1s,4s)-4-(hydroxymethyl)cyclohexyl)-1H-benzo[d]imidazol-2-yl)-3-(trifluoromethyl)benzamide (GT-05293)

With reference to the method of Scheme T-1, the target compound (GT-05293) was obtained (white solid, 14 mg, yield 12.96%). ¹H NMR (400 MHz, DMSO) δ 12.76 (s, 1H), 11.14 (s, 1H), 10.93 (s, 1H), 8.49 (d, J = 7.7 Hz, 1H), 8.44 (s, 1H), 8.00 (d, J = 7.9 Hz, 1H), 7.94 (s, 1H), 7.89 (d, J = 7.7 Hz, 1H), 7.81 (t, J = 8.0 Hz, 1H), 7.74 (t, J = 7.9 Hz, 1H), 7.51 (d, J = 8.9 Hz, 1H), 7.19 (s, 1H), 7.00 (d, J = 9.0 Hz, 1H), 5.96 (s, 1H), 4.77 (dd, J = 44.7, 24.3 Hz, 2H), 4.55 (s, 2H), 3.68 (d, J = 7.4 Hz, 4H), 3.24 (s, 4H), 3.15 (d, J = 13.1 Hz, 2H), 2.96 (d, J = 12.3 Hz, 1H), 2.67 (s, 1H), 2.46 - 2.38 (m, 2H), 2.15 (d, J = 32.4 Hz, 1H), 1.94 (d, J = 13.7 Hz, 2H), 1.85 (s, 1H), 1.76 - 1.55 (m, 5H), 1.36 - 1.26 (m, 3H). LCMS (ESI) calcd. for C₄₀H₄₃F₃N₇O₄S⁺ [M+H]⁺: 774.30, found, 774.4.

### Example 4: Preparation of 6-(5-cyano-1H-pyrrolo[2,3-b]pyridin-1-yl)-N-(1-((2-(2,6-dioxopiperidin-3-yl)-1-thioxoisoindolin-5-yl)methyl)piperidin-4-yl)-4-(isopropylamino)nicotinamide (GT-05294)

With reference to the method of Scheme T-1, the target compound (GT-05294) was obtained (white solid, 16 mg, yield 13.65%). ¹H NMR (400 MHz, DMSO) δ 11.14 (s, 1H), 10.64 (s, 1H), 8.81 (d, J = 2.0 Hz, 1H), 8.65 (dd, J = 25.7, 7.2 Hz, 3H), 8.51 (d, J = 3.8 Hz, 1H), 8.06 (s, 1H), 7.95 - 7.76 (m, 2H), 6.90 (d, J = 3.8 Hz, 1H), 4.78 (s, 1H), 4.44 (d, J = 3.7 Hz, 1H), 4.17 - 3.76 (m, 2H), 3.43 (s, 4H), 3.13 (dd, J = 7.4, 4.2 Hz, 3H), 2.02 (t, J = 23.5 Hz, 5H), 1.31 - 1.23 (m, 9H).. LCMS (ESI) calcd. for C₃₆H₃₈N₉O₃S⁺ [M+H]⁺: 676.28, found, 676.3.

### Example 5: Preparation of N-(2-(1-((2-(2,6-dioxopiperidin-3-yl)-1-thioxoisoindolin-5-yl)methyl)piperidin-4-yl)-6-methoxy-2H-indazol-5-yl)-3-(trifluoromethyl)benzamide (GT-05295)

With reference to the method of Scheme T-1, the target compound (GT-05295) was obtained (white solid, 32 mg, yield 27.69%). ¹H NMR (400 MHz, DMSO) δ 11.13 (d, J = 14.9 Hz, 1H), 10.94 (s, 1H), 9.81 (d, J = 17.2 Hz, 1H), 8.38 - 8.22 (m, 3H), 8.05 - 7.95 (m, 3H), 7.81 (dt, J = 16.2, 7.9 Hz, 2H), 7.09 (d, J = 4.2 Hz, 1H), 5.96 (s, 1H), 4.95 - 4.72 (m, 2H), 4.57 (d, J = 48.0 Hz, 2H), 3.88 (d, J = 7.3 Hz, 3H), 3.65 - 3.54 (m, 2H), 3.37 - 3.31 (m, 1H), 3.28 - 3.11 (m, 3H), 3.06 - 2.93 (m, 1H), 2.65 (d, J = 15.4 Hz, 1H), 2.53 (s, 1H), 2.47 - 2.43 (m, 1H), 2.34 (d, J = 9.4 Hz, 2H), 2.15 - 2.07 (m, 1H). LCMS (ESI) calcd. for C₃₅H₃₄F₃N₆O₄S⁺ [M+H]⁺: 691.23, found, 691.3.

### Example 6: Preparation of N-(2-(1-((2-(2,6-dioxopiperidin-3-yl)-1-thioxoisoindolin-5-yl)methyl)piperidin-4-yl)-6-methoxy-2H-indazol-5-yl)-6-(trifluoromethyl)picolinamide (GT-05296)

With reference to the method of Scheme T-1, the target compound (GT-05296) was obtained (white solid, 40 mg, yield 34.65%). ¹H NMR (400 MHz, DMSO) δ 11.15 (s, 1H), 10.85 (s, 1H), 10.51 (s, 1H), 8.71 (s, 1H), 8.51 - 8.30 (m, 3H), 8.22 (d, J = 7.2 Hz, 1H), 8.00 (d, J = 7.9 Hz, 1H), 7.85 - 7.75 (m, 1H), 7.18 (s, 1H), 5.96 (s, 1H), 4.91 - 4.71 (m, 2H), 4.58 (d, J = 46.8 Hz, 2H), 3.99 (d, J = 8.4 Hz, 3H), 3.57 (d, J = 11.1 Hz, 2H), 3.28 - 3.14 (m, 3H), 2.96 (d, J = 12.1 Hz, 1H), 2.66 (d, J = 17.5 Hz, 2H), 2.47 (s, 1H), 2.34 (d, J = 7.9 Hz, 2H), 2.20 - 2.00 (m, 2H). LCMS (ESI) calcd. for [M+H]⁺: 692.23, found, 692.3.

### Example 7: Preparation of N-(2-(1-((2-(2,6-dioxopiperidin-3-yl)-1-thioxoisoindolin-5-yl)methyl)piperidin-4-yl)-6-(2-hydroxypropan-2-yl)-2H-indazol-5-yl)-6-(trifluoromethyl)picolinamide (GT-05297)

With reference to the method of Scheme T-1, the target compound (GT-05297) was obtained (white solid, 33 mg, yield 29.31%). ¹H NMR 1H NMR (400 MHz, DMSO) δ 12.38 (s, 1H), 11.15 (s, 3H), 8.74 (s, 1H), 8.47 - 8.33 (m, 3H), 8.16 (d, J = 7.7 Hz, 1H), 8.04 - 7.94 (m, 2H), 7.83 (d, J = 3.4 Hz, 1H), 7.60 (d, J = 3.4 Hz, 1H), 5.97 (s, 2H), 4.79 (s, 2H), 4.63 (s, 1H), 4.53 (s, 2H), 3.61 - 3.56 (m, 2H), 3.23 (s, 2H), 3.01 - 2.94 (m, 1H), 2.65 (d, J = 15.9 Hz, 2H), 2.34 (d, J = 11.7 Hz, 2H), 2.19 - 2.06 (m, 2H), 1.63 (d, J = 9.0 Hz, 6H). LCMS (ESI) calcd. for C₃₆H₃₇F₃N₇O₄S⁺ [M+H]⁺: 720.26, found, 720.3.

### Example 8: Preparation of N-(2-(1-((2-(2,6-dioxopiperidin-3-yl)-1-thioxoisoindolin-5-yl)methyl)piperidin-4-yl)-5-(2-hydroxypropan-2-yl)benzo[d]thiazol-6-yl)-6-(trifluoromethyl)picolinamide (GT-05298)

With reference to the method of Scheme T-1, the target compound (GT-05298) was obtained (white solid, 13 mg, yield 11.71%). ¹H NMR (400 MHz, DMSO) δ 12.58 (d, J = 8.8 Hz, 1H), 11.13 (d, J = 15.4 Hz, 1H), 10.46 (s, 1H), 9.11 (d, J = 11.1 Hz, 1H), 8.51 - 8.34 (m, 2H), 8.19 (d, J = 7.7 Hz, 1H), 8.06 - 7.76 (m, 4H), 6.03 (d, J = 57.7 Hz, 2H), 4.87 - 4.49 (m, 4H), 3.65 - 3.51 (m, 2H), 3.16 - 3.09 (m, 2H), 2.95 (s, 1H), 2.67 (s, 1H), 2.33 (s, 2H), 2.23 - 2.02 (m, 4H), 1.64 (d, J = 9.9 Hz, 6H). LCMS (ESI) calcd. for C₃₆H₃₆F₃N₆O₄S₂⁺ [M+H]⁺: 737.22, found, 737.3.

### Example 9: Preparation of N-(2-(1-((2-(2,6-dioxopiperidin-3-yl)-1-thioxoisoindolin-5-yl)methyl)piperidin-4-yl)-5-(2-hydroxypropan-2-yl)benzo[d]oxazol-6-yl)-6-(trifluoromethyl)picolinamide (GT-05320)

With reference to the method of Scheme T-1, the target compound (GT-05320) was obtained (white solid, 35 mg, yield 31.11%). ¹H NMR (400 MHz, DMSO) δ 12.63 (d, *J =* 9.5 Hz, 1H), 11.13 (d, J = 13.6 Hz, 1H), 10.90 (s, 1H), 8.84 - 8.72 (m, 1H), 8.48 - 8.37 (m, 2H), 8.19 (d, J = 7.8 Hz, 1H), 8.04 - 7.91 (m, 2H), 7.83 (dd, J = 16.3, 8.2 Hz, 1H), 7.68 (d, J = 11.7 Hz, 1H), 6.01 (d, J = 48.2 Hz, 2H), 4.91 - 4.41 (m, 4H), 3.63 - 3.50 (m, 2H), 3.17 - 3.08 (m, 2H), 3.04 - 2.92 (m, 2H), 2.66 (d, J = 17.1 Hz, 1H), 2.34 (d, J = 14.3 Hz, 1H), 2.26 - 2.01 (m, 4H), 1.65 - 1.59 (m, 6H).. LCMS (ESI) calcd. for C₃₆H₃₆F₃N₆O₅S⁺ [M+H]⁺: 721.24, found, 721.3.

### Example 10: Preparation of N-(3-(difluoromethyl)-1-(1-((2-(2,6-dioxopiperidin-3-yl)-1-thioxoisoindolin-5-yl)methyl)piperidin-4-yl)-1H-pyrazol-4-yl)-5-morpholinopyrazolo[1,5-a]pyrimidine-3-carboxamide (GT-05098)

With reference to the method of Scheme T-1, the target compound (GT-05098) was obtained (white solid, 11 mg, yield 9.76%). ¹H NMR (400 MHz, DMSO) δ 11.15 (s, 1H), 10.59 (s, 1H), 9.42 (s, 1H), 8.83 (d, J = 7.9 Hz, 1H), 8.42 (s, 1H), 8.29 (s, 1H), 7.95 (d, J = 32.0 Hz, 3H), 7.13 (s, 1H), 6.91 (d, J = 8.0 Hz, 1H), 5.96 (s, 1H), 4.82 (d, J = 33.8 Hz, 2H), 4.50 (s, 3H), 3.83 - 3.70 (m, 9H), 3.52 (s, 2H), 3.15 (s, 2H), 2.98 (t, J = 12.9 Hz, 1H), 2.67 (s, 2H), 2.29 (d, J = 28.5 Hz, 3H), 2.15 - 2.07 (m, 1H). LCMS (ESI) calcd. for C₃₄H₃₇F₂N₁₀O₄S⁺ [M+H]⁺: 719.27, found, 719.3.

### Example 11: Preparation of 5-((1R,4R)-2-oxa-5-azabicyclo[2.2.1]heptan-5-yl)-N-(3-(difluoromethyl)-1-(1-((2-(2,6-dioxopiperidin-3-yl)-1-thioxoisoindolin-5-yl)methyl)piperidin-4-yl)-1H-pyrazol-4-yl)pyrazolo[1,5-a]pyrimidine-3-carboxamide (GT-05321)

With reference to the method of Scheme T-1, the target compound (GT-05321) was obtained (white solid, 12 mg, yield 10.75%). ¹H NMR (400 MHz, DMSO) δ 11.15 (s, 1H), 10.70 (s, 1H), 9.52 (d, J = 6.4 Hz, 1H), 8.78 (d, J = 7.6 Hz, 1H), 8.42 (d, J = 3.2 Hz, 1H), 8.27 (t, J = 7.3 Hz, 1H), 8.07 - 7.77 (m, 3H), 7.14 (dd, J = 55.3, 51.9 Hz, 1H), 6.66 (dd, J = 165.1, 7.7 Hz, 1H), 5.97 (d, J = 10.1 Hz, 1H), 4.91 (dd, J = 69.7, 49.4 Hz, 3H), 4.52 (d, J = 23.3 Hz, 2H), 3.84 - 3.41 (m, 8H), 3.20 - 2.95 (m, 4H), 2.65 (d, J = 15.8 Hz, 2H), 2.35 - 2.22 (m, 4H), 2.03 (ddd, J = 43.6, 18.3, 7.6 Hz, 4H). LCMS (ESI) calcd. for C₃₅H₃₇F₂N₁₀O₄S⁺ [M+H]⁺: 731.27, found, 731.3.

### Example 12: Preparation of N-(2-(4-((2-(2,6-dioxopiperidin-3-yl)-1-thioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide (GT-05322)

With reference to the method of Scheme T-1, the target compound (GT-05322) was obtained (white solid, 24 mg, yield 22.05%). ¹H NMR (400 MHz, DMSO) δ 11.98 (s, 1H), 11.26 - 10.85 (m, 1H), 10.01 (s, 1H), 9.38 (s, 2H), 9.14 (s, 1H), 8.88 (d, J = 5.9 Hz, 1H), 8.28 (s, 1H), 8.17 (d, J = 5.9 Hz, 1H), 7.97 (d, J = 7.9 Hz, 1H), 7.94 - 7.88 (m, 1H), 7.83 (t, J = 8.8 Hz, 1H), 7.70 (s, 1H), 4.82 (dd, J = 57.2, 20.4 Hz, 1H), 4.52 (s, 2H), 4.28 (s, 4H), 3.60 (ddd, J = 13.1, 10.5, 6.6 Hz, 16H), 3.45 (s, 4H), 3.26 (d, J = 9.8 Hz, 3H), 2.78 (s, 3H), 2.65 (d, J = 16.8 Hz, 1H), 2.09 (dd, J = 11.9, 6.7 Hz, 1H), 1.94 - 1.85 (m, 1H), 1.77 (d, J = 8.5 Hz, 1H). LCMS (ESI) calcd. for C₃₈H₃₉N₁₀O₆S⁺ [M+H]⁺: 763.28, found, 763.4.

### Example 13: Preparation of N-(2-(1-((2-(2,6-dioxopiperidin-3-yl)-1-thioxoisoindolin-4-yl)methyl)piperidin-4-yl)-6-methoxy-2H-indazol-5-yl)-6-(trifluoromethyl)picolinamide (GT-04950)

With reference to the method of Scheme T-1, the target compound (GT-04950) was obtained (white solid, 9 mg, yield 13.70%). ¹H NMR (400 MHz, DMSO) δ 11.20 (d, J = 12.6 Hz, 1H), 10.67 (s, 1H), 10.52 (d, J = 9.9 Hz, 1H), 8.73 (d, J = 15.0 Hz, 1H), 8.49 - 8.35 (m, 3H), 8.22 (d, J = 7.7 Hz, 1H), 8.02 (d, J = 7.7 Hz, 1H), 7.97 (d, J = 7.4 Hz, 1H), 7.71 (t, J = 7.6 Hz, 1H), 7.18 (d, J = 10.5 Hz, 1H), 6.02 (s, 1H), 5.08 (dd, J = 115.7, 20.4 Hz, 2H), 4.73 (s, 1H), 4.48 (s, 2H), 4.00 (d, J = 11.2 Hz, 3H), 3.59 (d, J = 18.0 Hz, 2H), 3.33 - 3.23 (m, 4H), 3.00 (d, J = 12.0 Hz, 1H), 2.70 (d, J = 24.5 Hz, 2H), 2.35 (d, J = 13.6 Hz, 2H), 2.15 (s, 1H). LCMS (ESI) calcd. for C₃₄H₃₃F₃N₇O₄S⁺ [M+H]⁺: 692.23, found, 692.3.

### Example 14: Preparation of N-(3-(difluoromethyl)-1-(1-((2-(2,6-dioxopiperidin-3-yl)-1-thioxoisoindolin-4-yl)methyl)piperidin-4-yl)-1H-pyrazol-4-yl)-5-morpholinopyrazolo[1,5-a]pyrimidine-3-carboxamide (GT-05287)

With reference to the method of Scheme T-1, the target compound (GT-05287) was obtained (white solid, 8 mg, yield 8.28%). ¹H NMR (400 MHz, DMSO) δ 11.18 (d, J = 31.4 Hz, 1H), 10.54 (s, 1H), 9.42 (s, 1H), 8.83 (d, J = 7.9 Hz, 1H), 8.44 (s, 1H), 8.29 (s, 1H), 8.04 - 7.95 (m, 1H), 7.70 (t, J = 7.8 Hz, 1H), 7.13 (t, J = 53.7 Hz, 1H), 6.91 (d, J = 8.0 Hz, 1H), 6.01 (s, 1H), 5.05 (dd, J = 111.6, 20.7 Hz, 1H), 4.51 (d, J = 39.6 Hz, 2H), 3.67 (dd, J = 67.0, 20.3 Hz, 10H), 3.30 - 3.20 (m, 4H), 3.16 (d, J = 20.5 Hz, 2H), 3.05 - 2.91 (m, 2H), 2.67 (s, 1H), 2.30 (d, J = 23.7 Hz, 2H), 2.13 (s, 1H).. LCMS (ESI) calcd. for C₃₄H₃₇F₂N₁₀O₄S⁺ [M+H]⁺: 719.27, found, 719.3.

### Example 15: Preparation of 5-((1R,4R)-2-oxa-5-azabicyclo[2.2.1]heptan-5-yl)-N-(3-(difluoromethyl)-1-(1-((2-(2,6-dioxopiperidin-3-yl)-1-thioxoisoindolin-4-yl)methyl)piperidin-4-yl)-1H-pyrazol-4-yl)pyrazolo[1,5-a]pyrimidine-3-carboxamide (GT-05288)

With reference to the method of Scheme T-1, the target compound (GT-05288) was obtained (white solid, 7 mg, yield 6.76%). ¹H NMR,1H NMR (400 MHz, DMSO-d6) δ 11.22 (s, 1H), 9.52 (s, 1H), 8.79 (d, J = 7.7 Hz, 1H), 8.46 (s, 1H), 8.26 (d, J = 5.2 Hz, 1H), 7.95 (d, J = 60.9 Hz, 2H), 7.20 (d, J = 57.0 Hz, 1H), 6.67 (dd, J = 164.6, 7.8 Hz, 1H), 5.17 (d, J = 76.5 Hz, 1H), 4.86 (d, J = 54.4 Hz, 1H), 4.61 (d, J = 101.0 Hz, 2H), 3.76 (d, J = 31.3 Hz, 2H), 3.59 (s, 2H), 3.46 (s, 3H), 3.26 - 3.12 (m, 6H), 2.93 (s, 2H), 2.26 (s, 2H), 2.18 - 2.10 (m, 2H), 1.98 (s, 3H). LCMS (ESI) calcd. for C₃₅H₃₇F₂N₁₀O₄S⁺ [M+H]⁺: 731.27, found, 731.3.

### Example 16: Preparation of N-(2-(4-((2-(2,6-dioxopiperidin-3-yl)-1-thioxoisoindolin-4-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide (GT-05289)

With reference to the method of Scheme T-1, the target compound (GT-05289) was obtained (white solid, 5 mg, yield 4.59%). ¹H NMR (400 MHz, DMSO) δ 12.38 (s, 1H), 10.84 (s, 1H), 10.66 - 10.36 (m, 2H), 8.75 (s, 1H), 8.55 - 8.33 (m, 3H), 8.17 (d, J = 7.8 Hz, 1H), 7.76 (d, J = 8.5 Hz, 1H), 7.60 (s, 1H), 7.47 (s, 1H), 7.36 (d, J = 8.6 Hz, 1H), 4.81 (s, 1H), 4.28 (d, J = 12.7 Hz, 1H), 3.84 - 3.69 (m, 4H), 3.49 (s, 4H), 3.19 (s, 3H), 3.08 - 2.95 (m, 4H), 2.84 (t, J = 6.9 Hz, 2H), 2.61 (d, J = 12.1 Hz, 1H), 2.36 (d, J = 15.5 Hz, 2H), 2.19 (s, 2H), 1.74 (s, 3H). LCMS (ESI) calcd. for C₃₈H₃₉N₁₀O₆S⁺ [M+H]⁺: 763.28, found, 763.3.

### Example 17: Preparation of 3-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione (GT-05058)

With reference to the method of Scheme T-1, the target compound (GT-05058) was obtained (white solid, 13 mg, yield 18.57%). ¹H NMR (400 MHz, DMSO) δ 11.12 (s, 1H), 9.59 (s, 1H), 9.22 (s, 1H), 8.66 (s, 1H), 8.31 (d, J = 9.0 Hz, 2H), 8.19 (d, J = 9.2 Hz, 1H), 8.02 (d, J = 9.2 Hz, 1H), 7.87 (d, J = 7.8 Hz, 1H), 7.74 (s, 1H), 7.56 (d, J = 7.2 Hz, 1H), 7.35 (s, 1H), 6.65 (s, 1H), 5.93 (s, 1H), 4.77 (d, J = 19.0 Hz, 1H), 3.74 (s, 3H), 3.44 (s, 8H), 2.96 (dd, J = 34.4, 21.5 Hz, 6H), 2.64 (s, 2H), 2.09 (s, 3H), 1.76 (d, J = 13.7 Hz, 9H), 1.67 (s, 3H). LCMS (ESI) calcd. for C₄₅H₅₂BrN₁₁O₄PS⁺ [M+H]⁺: 952.28, found, 952.3.

### Example 18: Preparation of 8-(4-(4-((2-(2,6-dioxopiperidin-3-yl)-1-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile (GT-05097)

With reference to the method of Scheme T-1, the target compound (GT-05097) was obtained (white solid, 12 mg, yield 15.33%). ¹H NMR (400 MHz, DMSO) δ 12.84 (s, 1H), 11.14 (s, 1H), 8.32 (d, J = 8.1 Hz, 1H), 8.06 (s, 1H), 8.01 (s, 1H), 7.95 (d, J = 7.9 Hz, 1H), 7.78 (d, J = 47.1 Hz, 2H), 7.62 (s, 1H), 7.36 (s, 1H), 5.95 (s, 1H), 4.90 - 4.63 (m, 1H), 4.07 (s, 1H), 3.67 (s, 4H), 3.31 - 3.08 (m, 11H), 2.86 - 2.70 (m, 4H), 2.16 (d, J = 48.7 Hz, 3H), 1.96 - 1.74 (m, 9H), 1.28 (dd, J = 8.6, 6.3 Hz, 3H). LCMS (ESI) calcd. for C₄₄H₄₈N₇O₃S⁺ [M+H]⁺: 754.35, found, 754.4.

### Example 19: Preparation of 8-(4-(4-((2-(2,6-dioxopiperidin-3-yl)-3-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile (GT-05030)

With reference to the method of Scheme T-1, the target compound (GT-05030) was obtained (white solid, 23 mg, yield 32.80%). ¹H NMR (400 MHz, DMSO) δ 12.88 (s, 1H), 11.14 (s, 1H), 8.32 (d, J = 8.2 Hz, 1H), 8.05 (t, J *=* 17.1 Hz, 3H), 7.87 (s, 1H), 7.75 (d, J = 7.3 Hz, 1H), 7.63 - 7.56 (m, 1H), 7.36 (s, 1H), 5.95 (s, 1H), 4.80 (d, J = 38.0 Hz, 2H), 4.35 (s, 2H), 3.68 (s, 2H), 3.32 - 3.15 (m, 6H), 2.96 (dd, J = 21.6, 9.3 Hz, 2H), 2.82 (t, J = 11.9 Hz, 2H), 2.71 (dd, J = 15.0, 7.5 Hz, 3H), 2.52 (s, 2H), 2.20 (s, 2H), 2.15 - 2.03 (m, 1H), 1.90 (d, J = 10.4 Hz, 2H), 1.76 (s, 6H), 1.28 (dd, J = 12.9, 5.5 Hz, 3H). LCMS (ESI) calcd. for C₄₄H₄₈N₇O₃S⁺ [M+H]⁺: 754.35, found, 754.3.

### Example 20: Preparation of 3-(4-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione (GT-05074)

With reference to the method of Scheme T-1, the target compound (GT-05074) was obtained (white solid, 25 mg, yield 24.44%). ¹H NMR (400 MHz, DMSO) δ 11.10 (d, J = 31.9 Hz, 1H), 10.73 (s, 1H), 8.48 (s, 1H), 8.16 (d, J = 8.5 Hz, 1H), 7.96 (d, J = 7.8 Hz, 1H), 7.79 (d, J = 7.8 Hz, 1H), 7.65 (d, J = 8.7 Hz, 2H), 7.52 - 7.40 (m, 2H), 7.17 (ddd, J = 11.8, 11.0, 7.5 Hz, 5H), 5.96 (d, J = 9.0 Hz, 1H), 5.08 (t, J = 11.7 Hz, 1H), 4.94 - 4.73 (m, 1H), 4.53 (s, 2H), 3.30 (dd, J = 40.8, 31.3 Hz, 6H), 3.02 - 2.92 (m, 1H), 2.64 (dd, J = 31.5, 15.1 Hz, 3H), 2.24 - 2.05 (m, 3H). LCMS (ESI) calcd. for C₃₆H₃₅N₈O₃S⁺ [M+H]⁺: 659.26, found, 659.3.

### Example 21: Preparation of 3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-thioxoisoindolin-2-yl)piperidine-2,6-dione (GT-08754)

With reference to the method of Scheme T-1, the target compound (GT-08754) was obtained (yellow solid, 5 mg, yield 8 %). ¹H NMR (400 MHz, DMSO) δ 11.15 (s, 1H), 10.86 (s, 1H), 9.69 (s, 1H), 9.30 (s, 1H), 8.74 (s, 1H), 8.38 (d, *J =* 18.3 Hz, 2H), 8.26 (d, *J =* 9.3 Hz, 1H), 8.09 (d, *J =* 9.1 Hz, 1H), 8.02 - 7.97 (m, 2H), 7.93 - 7.80 (m, 2H), 7.41 (s, 1H), 6.73 (s, 1H), 5.96 (s, 1H), 4.89 - 4.71 (m, 2H), 4.45 - 4.37 (m, 1H), 3.80 (s, 3H), 3.36 - 3.29 (m, 3H), 3.10 (s, 3H), 2.68 (s, 3H), 2.29 - 2.18 (m, 3H), 2.12 (s, 2H), 1.99 (s, 2H), 1.84 (d, *J =* 13.7 Hz, 6H), 1.74 (s, 3H), 1.33 - 1.24 (m, 1H). LCMS (ESI) calcd. for C₄₂H₄₇BrN₁₀O₄PS ⁺ [M+H]⁺: 897.24, found, 897.0.

### Example 22: Preparation of 3-(5-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione (GT-08755)

With reference to the method of Scheme T-1, the target compound (GT-08755) was obtained (yellow solid, 17 mg, yield 21 %). ¹H NMR (400 MHz, DMSO) δ 12.11 (s, 1H), 11.13 (d, *J =* 12.5 Hz, 1H), 9.83 (s, 1H), 9.31 (s, 1H), 8.98 (s, 1H), 8.44 (s, 1H), 8.36 (s, 1H), 8.24 (d, *J =* 8.6 Hz, 1H), 8.10 (d, *J* = 9.1 Hz, 1H), 7.96 (d, *J =* 7.8 Hz, 2H), 7.86 - 7.77 (m, 1H), 7.39 (s, 1H), 6.74 (s, 1H), 5.98 (s, 1H), 4.88 (d, *J =* 20.5 Hz, 1H), 4.75 (d, *J =* 15.0 Hz, 1H), 4.49 (s, 2H), 3.81 (s, 3H), 3.74 - 3.68 (m, 6H), 3.46 - 3.34 (m, 5H), 3.13 (s, 2H), 3.02 - 2.94 (m, 1H), 2.74 - 2.63 (m, 3H), 2.21 - 2.14 (m, 2H), 2.13 - 2.08 (m, 1H), 1.87 (s, 2H), 1.84 (d, *J =* 13.7 Hz, 6H), 1.72 (s, 2H). LCMS (ESI) calcd. for C₄₅H₅₂BrN₁₁O₄PS ⁺ [M+H]⁺: 952.28, found, 952.1.

### Example 23: Preparation of 3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione (GT-08756)

With reference to the method of Scheme T-1, the target compound (GT-08756) was obtained (yellow solid, 6 mg, yield 10 %). ¹H NMR (400 MHz, DMSO) δ 13.27 (s, 1H), 11.88 (s, 1H), 11.31 (s, 1H), 11.15 (s, 1H), 9.10 - 8.97 (m, 1H), 8.11 (s, 1H), 8.03 (s, 1H), 7.99 - 7.95 (m, 2H), 7.86 (d, *J =* 8.1 Hz, 1H), 6.61 (s, 1H), 5.96 (s, 1H), 4.89 (d, *J =* 20.4 Hz, 1H), 4.75 (d, *J =* 20.3 Hz, 1H), 4.68 - 4.62 (m, 1H), 4.58 (s, 2H), 4.10 - 4.03 (m, 4H), 3.57 - 3.53 (m, 2H), 3.45 (d, *J =* 12.4 Hz, 4H), 3.30 (s, 6H), 3.04 - 2.91 (m, 1H), 2.65 (d, *J =* 16.5 Hz, 1H), 2.13 - 2.05 (m, 1H), 1.96 (s, 2H), 1.61 (d, *J =* 7.9 Hz, 2H), 1.44 (d, *J* = 6.6 Hz, 6H). LCMS (ESI) calcd. for C₃₇H₄₆N₁₁O₃S ⁺ [M+H]⁺: 724.35, found, 724.2.

### Example 24: Preparation of 2-(6,7-dihydro-5H-pyrrolo[1,2-c]imidazol-1-yl)-2-(6-(4-((1R,4R)-5-((2-(2,6-dioxopiperidin-3-yl)-1-thioxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide (GT-08757)

With reference to the method of Scheme T-1, the target compound (GT-08757) was obtained (white solid, 5 mg, yield 7 %). ¹H NMR (400 MHz, DMSO) δ 11.14 (s, 1H), 8.98 - 8.82 (m, 1H), 8.01 (s, 1H), 7.98 - 7.91 (m, 1H), 7.90 - 7.83 (m, 1H), 7.82 - 7.75 (m, 2H), 7.71 - 7.62 (m, 2H), 7.52 (d, *J =* 3.5 Hz, 1H), 7.32 (d, *J =* 3.4 Hz, 1H), 6.89 - 6.66 (m, 2H), 6.34 (s, 1H), 5.91 (d, *J =* 18.9 Hz, 1H), 4.82 - 4.70 (m, 3H), 4.45 (s, 2H), 4.22 (s, 2H), 3.67 - 3.60 (m, 2H), 3.18 - 3.09 (m, 3H), 2.94 - 2.81 (m, 2H), 2.63 - 2.55 (m, 4H), 2.31 (s, 3H), 1.29 - 1.26 (m, 4H). LCMS (ESI) calcd. for C₄₄H₄₁FN₉O₄S₂ ⁺ [M+H]⁺: 842.27, found, 842.1.

### Example 25: Preparation of 3-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-thioxoisoindolin-2-yl)piperidine-2,6-dione (GT-08758)

With reference to the method of Scheme T-1, the target compound (GT-08758) was obtained (yellow solid, 5 mg, yield 7 %). ¹H NMR (400 MHz, DMSO) δ 11.21 (s, 1H), 10.82 (s, 1H), 9.72 (s, 1H), 9.30 (s, 1H), 8.79 (s, 1H), 8.39 (d, *J =* 22.3 Hz, 2H), 8.26 (d, *J =* 9.2 Hz, 1H), 8.15 - 7.92 (m, 3H), 7.76 - 7.65 (m, 1H), 7.42 (s, 1H), 6.74 (s, 1H), 6.03 (s, 1H), 4.96 (t, *J =* 19.0 Hz, 1H), 4.80 - 4.61 (m, 1H), 4.37 - 4.18 (m, 1H), 3.82 (s, 3H), 3.42 - 3.33 (m, 3H), 3.13 (s, 2H), 3.04 - 2.96 (m, 1H), 2.70 (dd, *J=* 18.1, 4.2 Hz, 4H), 2.33 - 2.24 (m, 2H), 2.05 (d, *J* = 47.9 Hz, 4H), 1.84 (d, *J =* 13.7 Hz, 6H), 1.75 (s, 2H), 1.32 - 1.23 (m, 1H). LCMS (ESI) calcd. for C₄₂H₄₇BrN₁₀O₄PS ⁺ [M+H]⁺: 897.24, found, 897.1.

### Example 26: Preparation of 3-(4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione (GT-08759)

With reference to the method of Scheme T-1, the target compound (GT-08759) was obtained (yellow solid, 14 mg, yield 23 %). ¹H NMR (400 MHz, DMSO) δ 11.91 (s, 1H), 11.35 (s, 1H), 11.21 (s, 1H), 9.05 (s, 1H), 8.11 (s, 1H), 8.07 (d, *J =* 7.6 Hz, 1H), 7.99 (dd, *J =* 17.1, 7.4 Hz, 2H), 7.69 (t, *J =* 7.7 Hz, 1H), 6.62 (s, 1H), 6.03 (d, *J =* 9.1 Hz, 1H), 5.53 (d, *J =* 20.2 Hz, 1H), 4.96 (d, *J =* 20.5 Hz, 1H), 4.72 - 4.62 (m, 1H), 4.54 (dd, *J =* 32.3, 11.2 Hz, 2H), 4.13 - 4.03 (m, 4H), 3.77 (s, 2H), 3.58 - 3.51 (m, 4H), 3.40 (s, 3H), 3.30 (s, 3H), 3.04 - 2.94 (m, 1H), 2.70 (d, *J =* 16.6 Hz, 1H), 2.17 - 2.08 (m, 1H), 1.96 (s, 2H), 1.61 (d, *J =* 8.5 Hz, 2H), 1.44 (d, *J =* 6.6 Hz, 6H). LCMS (ESI) calcd. for C₃₇H₄₆N₁₁O₃S ⁺ [M+H]⁺: 724.35, found, 724.2.

### Example 27: Preparation of 2-(6,7-dihydro-5H-pyrrolo[1,2-c]imidazol-1-yl)-2-(6-(4-((1R,4R)-5-((2-(2,6-dioxopiperidin-3-yl)-1-thioxoisoindolin-4-yl)methyl)-2,5-diazabicyclo[2.2.1]heptan-2-yl)phenyl)-4-fluoro-1-oxoisoindolin-2-yl)-N-(thiazol-2-yl)acetamide (GT-08760)

With reference to the method of Scheme T-1, the target compound (GT-08760) was obtained (white solid, 7 mg, yield 10 %). ¹H NMR (400 MHz, DMSO) δ 11.20 (s, 1H), 10.86 (s, 1H), 9.00 (s, 1H), 8.11 - 8.03 (m, 1H), 7.97 (d, *J =* 7.9 Hz, 1H), 7.77 (d, *J =* 8.1 Hz, 3H), 7.66 (d, *J =* 8.1 Hz, 2H), 7.52 (d, *J =* 3.6 Hz, 1H), 7.32 (d, *J =* 3.5 Hz, 1H), 6.75 (s, 1H), 6.37 (s, 1H), 6.00 (s, 1H), 4.88 - 4.66 (m, 3H), 4.53 - 4.46 (m, 2H), 4.25 (d, *J =* 6.8 Hz, 2H), 3.89 - 3.78 (m, 1H), 3.64 (d, *J =* 10.8 Hz, 1H), 3.50 - 3.43 (m, 6H), 2.98 - 2.84 (m, 2H), 2.69 - 2.55 (m, 5H), 2.17 (d, *J* = 40.7 Hz, 2H), 1.33 - 1.21 (m, 1H). LCMS (ESI) calcd. for C₄₄H₄₁FN₉O₄S₂ ⁺ [M+H]⁺: 842.27, found, 842.1.

### Example 28: Preparation of 3-(6-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-thioxoisoindolin-2-yl)piperidine-2,6-dione (GT-08761)

With reference to the method of Scheme T-1, the target compound (GT-08761) was obtained (yellow solid, 24 mg, yield 32 %). ¹H NMR (400 MHz, DMSO) δ 11.15 (s, 1H), 11.03 (s, 1H), 10.09 (s, 1H), 9.34 (s, 2H), 8.52 (s, 1H), 8.31 (s, 1H), 8.24 - 8.18 (m, 2H), 8.13 (d, *J =* 9.1 Hz, 1H), 8.08 (t, *J =* 6.7 Hz, 1H), 7.78 (d, *J =* 7.8 Hz, 1H), 7.37 (s, 1H), 6.78 (s, 1H), 5.97 (d, *J =* 8.9 Hz, 1H), 4.90 (d, *J =* 20.5 Hz, 1H), 4.76 (d, *J =* 20.6 Hz, 1H), 4.68 (d, *J =* 12.5 Hz, 1H), 4.44 (s, 1H), 3.82 (s, 3H), 3.40 (s, 1H), 3.18 (s, 3H), 3.00 - 2.92 (m, 1H), 2.75 (s, 1H), 2.68 - 2.63 (m, 4H), 2.28 (s, 2H), 2.15 - 2.00 (m, 3H), 1.85 (d, *J=* 13.7 Hz, 6H), 1.71 (s, 2H), 1.35 - 1.26 (m, 2H). LCMS (ESI) calcd. for C₄₂H₄₇BrN₁₀O₄P ⁺ [M+H]⁺: 897.24, found, 897.1.

### Example 29: Preparation of 3-(6-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione (GT-08762)

With reference to the method of Scheme T-1, the target compound (GT-08762) was obtained (yellow solid, 38 mg, yield 46 %). ¹H NMR (400 MHz, DMSO) δ 12.05 (s, 1H), 11.14 (s, 1H), 9.87 (s, 1H), 9.31 (s, 1H), 9.03 (s, 1H), 8.45 (s, 1H), 8.36 (s, 1H), 8.24 (d, *J =* 8.8 Hz, 1H), 8.18 (s, 1H), 8.10 (d, *J* = 9.1 Hz, 1H), 8.00 (d, *J* = 7.1 Hz, 1H), 7.78 (d, *J =* 7.9 Hz, 1H), 7.39 (s, 1H), 6.74 (s, 1H), 5.95 (s, 1H), 4.89 (d, *J =* 20.8 Hz, 1H), 4.75 (d, *J =* 20.9 Hz, 1H), 4.57 (s, 1H), 3.81 (s, 3H), 3.52 - 3.43 (m, 6H), 3.21 (s, 1H), 3.12 (s, 2H), 3.01 - 2.95 (m, 1H), 2.77 - 2.57 (m, 5H), 2.19 - 2.07 (m, 3H), 1.88 (s, 1H), 1.84 (d, *J* = 13.7 Hz, 6H), 1.72 (s, 3H), 1.36 - 1.30 (m, 1H). LCMS (ESI) calcd. for C₄₅H₅₂BrN₁₁O₄PS ⁺ [M+H]⁺: 952.28, found, 952.1.

### Example 30: Preparation of 3-(6-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione (GT-08763)

With reference to the method of Scheme T-1, the target compound (GT-08763) was obtained (white solid, 18 mg, yield 30 %). ¹H NMR (400 MHz, DMSO) δ 11.65 (s, 1H), 11.36 (s, 1H), 11.15 (s, 1H), 9.05 (s, 1H), 8.26 - 8.11 (m, 2H), 8.08 (d, *J =* 7.8 Hz, 1H), 7.97 (d, *J =* 7.1 Hz, 1H), 7.80 (d, *J*= 7.9 Hz, 1H), 6.63 (s, 1H), 5.96 (d, *J =* 8.5 Hz, 1H), 4.90 (d, *J =* 20.5 Hz, 1H), 4.76 (d, *J =* 20.6 Hz, 1H), 4.69 - 4.64 (m, 1H), 4.60 (s, 2H), 4.13 - 4.04 (m, 4H), 3.58 - 3.53 (m, 2H), 3.45 (d, *J =* 12.0 Hz, 4H), 3.30 (s, 6H), 3.02 - 2.93 (m, 1H), 2.70 - 2.58 (m, 2H), 2.14 - 2.07 (m, 1H), 1.97 (d, *J =* 7.0 Hz, 2H), 1.61 (d, *J =* 8.2 Hz, 2H), 1.44 (d, *J =* 6.6 Hz, 6H). LCMS (ESI) calcd. for C₃₇H₄₆N₁₁O₃S ⁺ [M+H]⁺: 724.35, found, 724.2.

### Example 31: Preparation of 2-(6,7-dihydro-5H-pyrrolo[1,2-c]imidazol-1-yl)-2-(6-(4-((1R,4R)-5-((2-(2,6-dioxopiperidin-3-yl)-3-thioxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide (GT-08764)

With reference to the method of Scheme T-1, the target compound (GT-08764) was obtained (white solid, 14 mg, yield 20 %). ¹H NMR (400 MHz, DMSO) δ 11.13 (s, 1H), 10.60 (s, 1H), 9.03 (s, 1H), 8.22 (s, 1H), 8.07 (d, *J =* 8.2 Hz, 1H), 7.79 - 7.73 (m, 3H), 7.65 (d, *J =* 8.6 Hz, 2H), 7.52 (d, *J =* 3.5 Hz, 1H), 7.32 (d, *J =* 3.5 Hz, 1H), 6.74 (d, *J =* 8.4 Hz, 2H), 6.38 (s, 1H), 5.96 (d, 1H), 4.94 - 4.78 (m, 2H), 4.76 - 4.70 (m, 3H), 4.53 - 4.42 (m, 3H), 4.25 (t, *J =* 6.6 Hz, 2H), 3.73 - 3.64 (m, 2H), 3.28 - 3.21 (m, 3H), 2.95 - 2.83 (m, 2H), 2.68 - 2.58 (m, 4H), 2.20 (d, *J =* 10.8 Hz, 1H), 2.09 (s, 1H), 1.36 - 1.28 (m, 2H). LCMS (ESI) calcd. for C₄₄H₄₁FN₉O₄S₂ ⁺ [M+H]⁺: 842.27, found, 842.1.

### Example 32: Preparation of 7-cyclopentyl-2-((5-(4-((2-(2,6-dioxopiperidin-3-yl)-1-thioxoisoindolin-5-yl)methyl)piperazin-1-yl)pyridin-2-yl)amino)-N,N-dimethyl-7H-pyrrolo[2,3-d]pyrimidine-6-carboxamide (GT-08765)

With reference to the method of Scheme T-1, the target compound (GT-08765) was obtained (yellow solid, 18 mg, yield 30 %). ¹H NMR (400 MHz, DMSO) δ 12.00 (s, 1H), 11.52 (s, 1H), 11.13 (d, *J= 13.2* Hz, 1H), 9.01 (s, 1H), 8.08 - 8.02 (m, 2H), 8.00 - 7.95 (m, 2H), 7.86 (d, 1H), 7.65 (d, *J =* 9.4 Hz, 1H), 6.84 (s, 1H), 5.97 (d, *J =* 8.8 Hz, 1H), 4.90 - 4.70 (m, 3H), 4.56 (s, 2H), 3.81 (d, *J =* 10.9 Hz, 2H), 3.35 - 3.25 (m, 6H), 3.06 (s, 6H), 2.69 - 2.58 (m, 2H), 2.35 - 2.26 (m, 2H), 2.14 - 2.08 (m, 1H), 2.06 - 1.94 (m, 4H), 1.68 - 1.58 (m, 2H), 1.37 - 1.24 (m, 1H). LCMS (ESI) calcd. for C₃₇H₄₃N₁₀O₃S ⁺ [M+H]⁺: 707.32, found, 707.2.

### Example 33: Preparation of 3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione (GT-08766)

With reference to the method of Scheme T-1, the target compound (GT-08766) was obtained (yellow solid, 18 mg, yield 30 %). ¹H NMR (400 MHz, DMSO) δ 11.63 (s, 1H), 11.15 (s, 1H), 10.90 (s, 1H), 9.00 (s, 1H), 8.08 (s, 1H), 7.98 (d, *J =* 8.2 Hz, 2H), 7.90 - 7.75 (m, 3H), 6.06 - 5.89 (m, 1H), 5.88 - 5.75 (m, 1H), 4.89 (d, *J* = 20.4 Hz, 1H), 4.75 (d, *J =* 20.4 Hz, 1H), 4.55 (s, 2H), 3.85 (d, *J=* 11.0 Hz, 2H), 3.44 (d, 2H), 3.33 - 3.16 (m, 5H), 3.01 - 2.92 (m, 1H), 2.69 - 2.60 (m, 1H), 2.44 (s, 3H), 2.34 (s, 3H), 2.27 - 2.20 (m, 2H), 2.14 - 2.06 (m, 1H), 1.96 - 1.87 (m, 2H), 1.83 - 1.75 (m, 2H), 1.64 - 1.54 (m, 2H). LCMS (ESI) calcd. for C₃₈H₄₂N₉O₄S ⁺ [M+H]⁺: 720.31, found, 720.2.

### Example 34: Preparation of 7-cyclopentyl-2-((5-(4-((2-(2,6-dioxopiperidin-3-yl)-1-thioxoisoindolin-4-yl)methyl)piperazin-1-yl)pyridin-2-yl)amino)-N,N-dimethyl-7H-pyrrolo[2,3-d]pyrimidine-6-carboxamide (GT-08771)

With reference to the method of Scheme T-1, the target compound (GT-08771) was obtained (yellow solid, 18 mg, yield 30 %). ¹H NMR (400 MHz, DMSO) δ 11.93 (s, 1H), 11.44 (s, 1H), 11.22 (s, 1H), 9.00 (s, 1H), 8.12 - 7.96 (m, 4H), 7.69 (t, *J =* 7.9 Hz, 2H), 6.83 (s, 1H), 6.02 (d, *J =* 8.6 Hz, 1H), 5.51 (d, *J =* 20.2 Hz, 1H), 4.94 (d, *J =* 20.4 Hz, 1H), 4.84 - 4.72 (m, 1H), 4.52 (d, *J =* 22.5 Hz, 2H), 3.81 (s, 2H), 3.63 - 3.52 (m, 4H), 3.34 - 3.25 (m, 5H), 3.14 - 3.09 (m, 1H), 3.02 - 2.97 (m, 1H), 2.70 (d, *J =* 17.2 Hz, 1H), 2.37 - 2.28 (m, 2H), 2.16 - 2.09 (m, 1H), 2.06 - 1.94 (m, 4H), 1.65 (d, *J =* 5.7 Hz, 2H), 1.34 - 1.26 (m, 3H). LCMS (ESI) calcd. for C₃₇H₄₃N₁₀O₃S ⁺ [M+H]⁺: 707.32, found, 707.8.

### Example 35: Preparation of 3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione (GT-08772)

With reference to the method of Scheme T-1, the target compound (GT-08772) was obtained (yellow solid, 16 mg, yield 27 %). ¹H NMR (400 MHz, DMSO) δ 11.49 (s, 1H), 11.21 (s, 1H), 10.71 (s, 1H), 8.99 (s, 1H), 8.09 (d, *J =* 2.7 Hz, 1H), 8.02 (t, *J =* 6.8 Hz, 2H), 7.87 (d, *J* = 9.2 Hz, 1H), 7.79 - 7.73 (m, 1H), 7.70 (t, *J* = 7.7 Hz, 1H), 6.03 (d, *J =* 8.2 Hz, 1H), 5.89 - 5.73 (m, 1H), 5.39 (d, *J =* 20.6 Hz, 1H), 4.93 (d, *J =* 20.3 Hz, 1H), 4.51 (d, *J =* 13.7 Hz, 2H), 3.85 (s, 2H), 3.43 (s, 2H), 3.37 - 3.28 (m, 5H), 3.04 - 2.96 (m, 1H), 2.70 (d, *J =* 18.7 Hz, 1H), 2.43 (s, 3H), 2.33 (s, 3H), 2.23 (s, 2H), 2.17 - 2.10 (m, 1H), 1.96 - 1.87 (m, 2H), 1.83 - 1.74 (m, 2H), 1.63 - 1.54 (m, 2H). LCMS (ESI) calcd. for C₃₈H₄₂N₉O₄S ⁺ [M+H]⁺: 720.31, found, 720.2.

### Example 36: Preparation of 2-((5-bromo-2-((4-((4-((2-(2,6-dioxopiperidin-3-yl)-1-thioxoisoindolin-5-yl)methyl)piperazin-1-yl)sulfonyl)-2-methylphenyl)amino)pyrimidin-4-yl)amino)-6-fluorobenzamide (GT-08714)

With reference to the method of Scheme T-1, the target compound (GT-08714) was obtained (white solid, 24 mg, yield 32.38%). ¹H NMR (400 MHz, DMSO) δ 11.13 (s, 1H), 10.95 (s, 1H), 10.26 (s, 1H), 9.18 (s, 1H), 8.35 (s, 1H), 8.16 (s, 1H), 8.10 (d, J = 8.7 Hz, 2H), 7.93 (dd, J = 10.1, 8.3 Hz, 2H), 7.85 (s, 1H), 7.71 (d, J = 7.8 Hz, 1H), 7.59 (d, J = 1.9 Hz, 1H), 7.51 (dd, J = 8.5, 2.0 Hz, 1H), 7.34 (d, J = 6.5 Hz, 1H), 7.07 - 6.93 (m, 1H), 5.95 (d, J = 9.6 Hz, 1H), 4.78 (dd, J = 54.0, 20.4 Hz, 2H), 4.48 (s, 2H), 3.75 (s, 2H), 3.40 (d, J = 8.5 Hz, 2H), 3.22 (s, 2H), 2.96 (s, 1H), 2.67 (dd, J = 22.6, 20.8 Hz, 4H), 2.34 (s, 3H), 2.09 (d, J = 6.5 Hz, 1H). LCMS (ESI) calcd. for C₃₆H₃₆BrFN₉O₅S₂ [M+H]⁺: 836.14, found, 836.0.

### Example 37: Preparation of 2-((5-bromo-2-((4-((4-((2-(2,6-dioxopiperidin-3-yl)-1-thioxoisoindolin-4-yl)methyl)piperazin-1-yl)sulfonyl)-2-methylphenyl)amino)pyrimidin-4-yl)amino)-6-fluorobenzamide (GT-08793)

With reference to the method of Scheme T-1, the target compound (GT-08793) was obtained (white solid, 30 mg, yield 33.73%). ¹H NMR (400 MHz, DMSO) δ 11.20 (s, 2H), 10.31 (s, 1H), 9.29 (s, 1H), 8.37 (s, 1H), 8.16 (s, 1H), 8.11 - 8.04 (m, 2H), 7.99 - 7.88 (m, 3H), 7.65 (t, J = 7.6 Hz, 1H), 7.60 (d, J = 1.8 Hz, 1H), 7.51 (dd, J = 8.5, 1.9 Hz, 1H), 7.08 - 6.97 (m, 1H), 6.00 (d, J = 8.4 Hz, 1H), 5.41 - 4.80 (m, 2H), 4.44 (s, 2H), 3.36 (d, J = 29.0 Hz, 4H), 3.14 (d, J = 13.9 Hz, 1H), 3.07 - 2.93 (m, 1H), 2.86 (d, J = 9.7 Hz, 2H), 2.68 (d, J = 16.1 Hz, 1H), 2.51 (s, 1H), 2.43 (d, J = 13.1 Hz, 1H), 2.34 (s, 3H), 2.20 - 2.06 (m, 1H). LCMS (ESI) calcd. for C₃₆H₃₆BrFN₉O₅S₂ [M+H]⁺: 836.14, found, 836.0.

### Example 38: Preparation of 2-((5-bromo-2-((4-((4-((2-(2,6-dioxopiperidin-3-yl)-3-thioxoisoindolin-5-yl)methyl)piperazin-1-yl)sulfonyl)-2-methylphenyl)amino)pyrimidin-4-yl)amino)-6-fluorobenzamide (GT-08794)

With reference to the method of Scheme T-1, the target compound (GT-08794) was obtained (white solid, 45 mg, yield 55.19%). ¹H NMR (400 MHz, DMSO) δ 11.14 (s, 1H), 10.99 (s, 1H), 10.35 (s, 1H), 9.38 (s, 1H), 8.39 (s, 1H), 8.17 (s, 1H), 8.07 (dd, J = 11.6, 5.9 Hz, 3H), 7.95 - 7.88 (m, 2H), 7.74 (dd, J = 8.4, 3.7 Hz, 1H), 7.60 (d, J = 1.8 Hz, 1H), 7.51 (dd, J = 8.5, 2.0 Hz, 1H), 7.35 (td, J = 8.4, 6.6 Hz, 1H), 7.07 - 6.98 (m, 1H), 5.94 (d, J = 9.2 Hz, 1H), 4.80 (dd, J = 57.6, 20.6 Hz, 2H), 4.52 (s, 2H), 3.44 - 3.33 (m, 2H), 3.18 (d, J = 38.8 Hz, 3H), 3.03 - 2.90 (m, 1H), 2.65 (dd, J = 46.3, 30.7 Hz, 5H), 2.35 (s, 3H), 2.15 - 2.01 (m, 1H). LCMS (ESI) calcd. for C₃₆H₃₆BrFN₉O₅S₂ [M+H]⁺: 836.14, found, 836.0.

### Example 39: Preparation of 3-(4-((4-((4-((5-amino-1-(2,6-difluorobenzoyl)-1H-1,2,4-triazol-3-yl)amino)phenyl)sulfonyl)piperazin-1-yl)methyl)-1-thioxoisoindolin-2-yl)piperidine-2,6-dione (GT-08795)

With reference to the method of Scheme T-1, the target compound (GT-08795) was obtained (white solid, 21 mg, yield 26.46%). ¹H NMR (400 MHz, DMSO) δ 11.16 (d, J = 24.4 Hz, 1H), 11.00 (s, 1H), 10.07 (d, J = 5.6 Hz, 1H), 8.12 - 7.93 (m, 2H), 7.88 (d, J = 6.6 Hz, 1H), 7.80 - 7.69 (m, 1H), 7.58 (dddd, J = 25.9, 21.9, 14.1, 8.4 Hz, 5H), 7.39 - 7.21 (m, 2H), 5.99 (d, J = 8.2 Hz, 1H), 5.19 (s, 1H), 4.85 (dd, J = 20.3, 8.1 Hz, 1H), 4.53 (d, J = 99.8 Hz, 2H), 3.64 (s, 2H), 3.27 - 3.21 (m, 1H), 3.14 (s, 1H), 3.06 (d, J = 3.8 Hz, 1H), 3.00 - 2.92 (m, 1H), 2.75 - 2.62 (m, 2H), 2.17 - 2.02 (m, 1H). LCMS (ESI) calcd. for C₃₃H₃₂F₂N₉O₅S₂ [M+H]⁺: 736.19, found, 736.0.

### Example 40: Preparation of 3-(6-((4-((4-((5-amino-1-(2,6-difluorobenzoyl)-1H-1,2,4-triazol-3-yl)amino)phenyl)sulfonyl)piperazin-1-yl)methyl)-1-thioxoisoindolin-2-yl)piperidine-2,6-dione (GT-08796)

With reference to the method of Scheme T-1, the target compound (GT-08796) was obtained (white solid, 25 mg, yield 34.99%). ¹H NMR (400 MHz, DMSO) δ 11.13 (s, 1H), 10.54 (s, 1H), 10.07 (s, 1H), 8.04 (s, 2H), 7.83 (d, J = 7.7 Hz, 1H), 7.77 - 7.68 (m, 2H), 7.60 - 7.48 (m, 4H), 7.34 (t, J = 8.3 Hz, 2H), 5.94 (d, J = 8.6 Hz, 1H), 4.79 (dd, J = 58.3, 20.6 Hz, 2H), 4.48 (s, 2H), 3.66 (s, 2H), 3.17 (s, 4H), 2.95 (d, J = 12.5 Hz, 1H), 2.71 - 2.55 (m, 4H), 2.18 - 2.01 (m, 1H). LCMS (ESI) calcd. for C₃₃H₃₂F₂N₉O₅S₂ [M+H]⁺: 736.19, found, 736.0.

### Example 41: Preparation of 3-(5-((4-((4-((5-amino-1-(3-methylthiophene-2-carbonyl)-1H-1,2,4-triazol-3-yl)amino)phenyl)sulfonyl)piperazin-1-yl)methyl)-1-thioxoisoindolin-2-yl)piperidine-2,6-dione (GT-08792)

With reference to the method of Scheme T-1, the target compound (GT-08792) was obtained (white solid, 8 mg, yield 9.95%). ¹H NMR (400 MHz, DMSO) δ 11.16 (d, J = 29.8 Hz, 1H), 10.95 - 10.41 (m, 1H), 10.11 (s, 1H), 8.04 (t, J = 5.8 Hz, 1H), 7.97 - 7.82 (m, 5H), 7.69 (t, J = 11.9 Hz, 3H), 7.17 (t, J = 4.8 Hz, 1H), 5.93 (s, 1H), 4.92 - 4.63 (m, 2H), 4.46 (s, 1H), 3.69 (s, 2H), 3.49 - 3.42 (m, 3H), 3.14 (d, J = 29.0 Hz, 3H), 3.02 - 2.88 (m, 1H), 2.65 (d, J = 13.0 Hz, 5H), 2.52 (s, 1H), 2.12 - 1.98 (m, 1H). LCMS (ESI) calcd. for C₃₂H₃₄N₉O₅S₃ [M+H]⁺: 720.18, found, 720.1.

### Example 42: Preparation of 3-(4-((4-((4-((5-amino-1-(3-methylthiophene-2-carbonyl)-1H-1,2,4-triazol-3-yl)amino)phenyl)sulfonyl)piperazin-1-yl)methyl)-1-thioxoisoindolin-2-yl)piperidine-2,6-dione (GT-08797)

With reference to the method of Scheme T-1, the target compound (GT-08797) was obtained (white solid, 20 mg, yield 24.87%). ¹H NMR (400 MHz, DMSO) δ 11.18 (s, 1H), 10.97 (s, 1H), 10.10 (s, 1H), 8.03 (d, J = 5.0 Hz, 1H), 7.93 (dd, J = 24.0, 8.2 Hz, 6H), 7.70 (d, J = 8.9 Hz, 2H), 7.62 (dd, J = 14.9, 7.4 Hz, 1H), 7.16 (d, J = 5.1 Hz, 1H), 5.98 (d, J = 8.3 Hz, 1H), 5.21 (d, J = 19.8 Hz, 1H), 4.84 (d, J = 20.6 Hz, 1H), 4.41 (s, 2H), 3.70 (s, 2H), 3.30 (s, 4H), 3.04 - 2.92 (m, 1H), 2.83 - 2.61 (m, 6H), 2.47 - 2.34 (m, 1H), 2.14 - 2.00 (m, 1H). LCMS (ESI) calcd. for C₃₂H₃₄N₉O₅S₃ [M+H]⁺: 720.18, found, 720.1.

### Example 43: Preparation of 3-(6-((4-((4-((5-amino-1-(3-methylthiophene-2-carbonyl)-1H-1,2,4-triazol-3-yl)amino)phenyl)sulfonyl)piperazin-1-yl)methyl)-1-thioxoisoindolin-2-yl)piperidine-2,6-dione (GT-08798)

With reference to the method of Scheme T-1, the target compound (GT-08798) was obtained (white solid, 29 mg, yield 40.07%). ¹H NMR (400 MHz, DMSO) δ 11.13 (s, 1H), 10.69 (s, 1H), 10.11 (s, 1H), 8.04 (d, J = 5.1 Hz, 2H), 7.88 (dd, J = 19.1, 8.5 Hz, 5H), 7.72 (t, J = 9.7 Hz, 3H), 7.16 (d, J = 5.0 Hz, 1H), 5.93 (d, J = 8.1 Hz, 1H), 4.79 (dd, J = 58.2, 20.5 Hz, 2H), 4.49 (s, 2H), 3.71 (s, 2H), 3.21 (s, 4H), 2.95 (s, 1H), 2.65 (d, J = 15.7 Hz, 6H), 2.55 (s, 1H), 2.09 (d, J = 5.4 Hz, 1H). LCMS (ESI) calcd. for C₃₂H₃₄N₉O₅S₃ [M+H]⁺: 720.18, found, 720.0.

### Example 44: Preparation of 3-(5-((4-(3-amino-6-(2-hydroxyphenyl)pyridazin-4-yl)piperazin-1-yl)methyl)-1-thioxoisoindolin-2-yl)piperidine-2,6-dione (GT-08785)

With reference to the method of Scheme T-1, the target compound (GT-08785) was obtained (white solid, 14 mg, yield 15.53%). ¹H NMR (400 MHz, DMSO) δ 12.06 (s, 1H), 11.14 (s, 1H), 8.05 - 7.94 (m, 2H), 7.85 (t, J = 10.4 Hz, 1H), 7.70 - 7.54 (m, 3H), 7.42 - 7.33 (m, 1H), 7.09 (d, J = 8.1 Hz, 1H), 6.97 (t, J = 7.5 Hz, 1H), 5.96 (d, J = 8.4 Hz, 1H), 4.81 (dd, J = 57.6, 20.4 Hz, 2H), 4.53 (s, 2H), 3.70 (d, J = 21.3 Hz, 6H), 3.24 (s, 2H), 3.03 - 2.90 (m, 1H), 2.61 (dd, J = 28.6, 15.3 Hz, 2H), 2.18 - 2.06 (m, 1H). LCMS (ESI) calcd. for C₂₈H₃₀N₇O₃S [M+H]⁺: 544.21, found, 544.1.

### Example 45: Preparation of 3-(4-((4-(3-amino-6-(2-hydroxyphenyl)pyridazin-4-yl)piperazin-1-yl)methyl)-1-thioxoisoindolin-2-yl)piperidine-2,6-dione (GT-08786)

With reference to the method of Scheme T-1, the target compound (GT-08786) was obtained (white solid, 30 mg, yield 33.27%). ¹H NMR (400 MHz, DMSO) δ 12.06 (s, 1H), 11.20 (s, 1H), 8.01 (dd, J = 7.6, 4.2 Hz, 2H), 7.67 (ddd, J = 23.6, 13.2, 7.9 Hz, 4H), 7.42 - 7.32 (m, 1H), 7.10 (d, J = 8.1 Hz, 1H), 6.97 (t, J = 7.5 Hz, 1H), 6.01 (d, J = 9.3 Hz, 1H), 5.20 (dd, J = 217.7, 20.3 Hz, 2H), 4.49 (dd, J = 33.1, 13.3 Hz, 2H), 3.76 (s, 6H), 3.28 (s, 2H), 3.03 - 2.93 (m, 1H), 2.69 (d, J = 16.8 Hz, 1H), 2.48 (d, J = 12.4 Hz, 1H), 2.17 - 2.07 (m, 1H). LCMS (ESI) calcd. for C₂₈H₃₀N₇O₃S [M+H]⁺: 544.21, found, 544.1.

### Example 46: Preparation of 3-(6-((4-(3-amino-6-(2-hydroxyphenyl)pyridazin-4-yl)piperazin-1-yl)methyl)-1-thioxoisoindolin-2-yl)piperidine-2,6-dione (GT-08787)

With reference to the method of Scheme T-1, the target compound (GT-08787) was obtained (white solid, 35 mg, yield 38.82%). ¹H NMR (400 MHz, DMSO) δ 11.93 (s, 1H), 11.14 (s, 1H), 8.14 (s, 1H), 8.10 - 8.02 (m, 1H), 7.78 (d, J = 7.9 Hz, 1H), 7.64 - 7.56 (m, 2H), 7.38 (s, 1H), 7.11 (d, J = 8.1 Hz, 1H), 6.98 (d, J = 7.4 Hz, 1H), 5.95 (d, J = 8.4 Hz, 1H), 5.00 - 4.66 (m, 2H), 4.56 (s, 2H), 3.62 (s, 6H), 3.24 (d, J = 26.5 Hz, 2H), 3.02 - 2.88 (m, 1H), 2.74 - 2.52 (m, 2H), 2.08 (dd, J = 15.0, 9.8 Hz, 1H). LCMS (ESI) calcd. for C₂₈H₃₀N₇O₃S [M+H]⁺: 544.21, found, 544.1.

### Example 47: Preparation of 3-(5-((4-(3-amino-6-(5-fluoro-2-hydroxyphenyl)pyridazin-4-yl)piperazin-1-yl)methyl)-1-thioxoisoindolin-2-yl)piperidine-2,6-dione (GT-08712)

With reference to the method of Scheme T-1, the target compound (GT-08712) was obtained (white solid, 21 mg, yield 24.04%). ¹H NMR (400 MHz, DMSO) δ 12.11 (s, 1H), 11.15 (s, 1H), 8.06 - 7.94 (m, 2H), 7.85 (d, J = 8.2 Hz, 2H), 7.66 (s, 1H), 7.53 (dd, J = 9.4, 3.1 Hz, 1H), 7.24 (td, J = 8.6, 3.1 Hz, 1H), 7.10 (dd, J = 9.0, 4.8 Hz, 1H), 5.96 (d, J = 8.8 Hz, 1H), 4.82 (dd, J = 58.0, 20.4 Hz, 2H), 4.54 (s, 2H), 3.71 (d, J = 27.6 Hz, 6H), 3.20 (s, 2H), 2.98 (s, 1H), 2.65 (d, J = 17.1 Hz, 2H), 2.10 (s, 1H). LCMS (ESI) calcd. for C₂₈H₂₉FN₇O₃S [M+H]⁺: 562.20, found, 562.1.

### Example 48: Preparation of 3-(4-((4-(3-amino-6-(5-fluoro-2-hydroxyphenyl)pyridazin-4-yl)piperazin-1-yl)methyl)-1-thioxoisoindolin-2-yl)piperidine-2,6-dione (GT-08788)

With reference to the method of Scheme T-1, the target compound (GT-08788) was obtained (white solid, 21 mg, yield 24.04%). ¹H NMR (400 MHz, DMSO) δ 12.01 (s, 1H), 11.20 (s, 1H), 8.07 - 7.96 (m, 2H), 7.82 - 7.60 (m, 4H), 7.55 (dt, J = 10.9, 5.4 Hz, 1H), 7.23 (td, J = 8.6, 3.1 Hz, 1H), 7.08 (dd, J = 9.0, 4.8 Hz, 1H), 6.01 (d, J = 8.4 Hz, 1H), 5.46 (d, J = 20.4 Hz, 1H), 4.93 (d, J = 20.3 Hz, 1H), 4.59 - 4.39 (m, 2H), 3.74 (s, 6H), 3.28 (s, 3H), 3.02 - 2.94 (m, 1H), 2.69 (d, J = 16.8 Hz, 1H), 2.19 - 2.06 (m, 1H). LCMS (ESI) calcd. for C₂₈H₂₉FN₇O₃S [M+H]⁺: 562.20, found, 562.1.

### Example 49: Preparation of 3-(6-((4-(3-amino-6-(5-fluoro-2-hydroxyphenyl)pyridazin-4-yl)piperazin-1-yl)methyl)-1-thioxoisoindolin-2-yl)piperidine-2,6-dione (GT-08789)

With reference to the method of Scheme T-1, the target compound (GT-08789) was obtained (white solid, 41 mg, yield 46.93%). ¹H NMR (400 MHz, DMSO) δ 11.91 (s, 1H), 11.14 (s, 1H), 8.14 (s, 1H), 8.05 (d, J = 7.9 Hz, 1H), 7.80 (t, J = 12.4 Hz, 2H), 7.66 (s, 1H), 7.51 (dd, J = 9.4, 3.0 Hz, 1H), 7.23 (td, J = 8.5, 3.1 Hz, 1H), 7.11 (dd, J = 9.0, 4.8 Hz, 1H), 5.95 (d, J = 9.0 Hz, 1H), 4.79 (dt, J = 36.3, 18.2 Hz, 2H), 4.59 (d, J = 19.7 Hz, 2H), 3.67 (d, J = 39.5 Hz, 6H), 3.28 - 3.11 (m, 2H), 3.02 - 2.91 (m, 1H), 2.72 - 2.53 (m, 2H), 2.08 (dd, J = 15.1, 9.9 Hz, 1H). LCMS (ESI) calcd. for C₂₈H₂₉FN₇O₃S [M+H]⁺: 562.20, found, 562.1.

### Example 50: Preparation of 3-(5-((3-(3-amino-6-(5-fluoro-2-hydroxyphenyl)pyridazin-4-yl)-3,8-diazabicyclo[3.2.1]octan-8-yl)methyl)-1-thioxoisoindolin-2-yl)piperidine-2,6-dione (GT-08713)

With reference to the method of Scheme T-1, the target compound (GT-08713) was obtained (white solid, 19 mg, yield 22.66%). ¹H NMR (400 MHz, DMSO) δ 11.92 (s, 1H), 11.14 (s, 1H), 8.16 (s, 1H), 8.04 - 7.95 (m, 2H), 7.63 (s, 1H), 7.53 - 7.46 (m, 1H), 7.24 (td, J = 8.4, 3.2 Hz, 3H), 7.11 - 7.05 (m, 1H), 5.96 (d, J = 9.1 Hz, 1H), 4.82 (dd, J = 58.9, 20.4 Hz, 2H), 4.42 (s, 2H), 4.01 (s, 2H), 3.76 (d, J = 19.1 Hz, 4H), 3.01 - 2.89 (m, 1H), 2.67 - 2.56 (m, 2H), 2.33 (t, J = 10.8 Hz, 4H), 2.17 - 2.07 (m, 1H). LCMS (ESI) calcd. for C₃₀H₃₁FN₇O₃S [M+H]⁺: 588.22, found, 588.1.

### Example 51: Preparation of 3-(4-((3-(3-amino-6-(5-fluoro-2-hydroxyphenyl)pyridazin-4-yl)-3,8-diazabicyclo[3.2.1]octan-8-yl)methyl)-1-thioxoisoindolin-2-yl)piperidine-2,6-dione (GT-08790)

With reference to the method of Scheme T-1, the target compound (GT-08790) was obtained (white solid, 18 mg, yield 19.32%). ¹H NMR (400 MHz, DMSO) δ 11.64 (s, 1H), 11.20 (s, 1H), 8.09 (d, J = 7.3 Hz, 1H), 8.00 (d, J = 7.6 Hz, 1H), 7.74 - 7.57 (m, 2H), 7.52 (dd, J = 11.8, 8.3 Hz, 1H), 7.32 - 7.17 (m, 2H), 7.08 (dd, J = 9.0, 4.8 Hz, 1H), 6.01 (d, J = 8.9 Hz, 1H), 5.48 (d, J = 20.2 Hz, 1H), 5.05 - 4.88 (m, 1H), 4.51 - 4.11 (m, 4H), 3.96 - 3.84 (m, 2H), 3.65 (s, 4H), 2.97 (dt, J = 23.0, 7.1 Hz, 1H), 2.68 (d, J = 16.0 Hz, 1H), 2.36 (s, 3H), 2.18 - 2.03 (m, 1H). LCMS (ESI) calcd. for C₃₀H₃₁FN₇O₃S [M+H]⁺: 588.22, found, 588.1.

### Example 52: Preparation of 3-(6-((3-(3-amino-6-(5-fluoro-2-hydroxyphenyl)pyridazin-4-yl)-3,8-diazabicyclo[3.2.1]octan-8-yl)methyl)-1-thioxoisoindolin-2-yl)piperidine-2,6-dione (GT-08791)

With reference to the method of Scheme T-1, the target compound (GT-08791) was obtained (white solid, 40 mg, yield 42.93%). ¹H NMR (400 MHz, DMSO) δ 11.72 (s, 1H), 11.15 (s, 1H), 8.32 - 8.19 (m, 2H), 7.79 (d, J = 7.8 Hz, 1H), 7.65 (s, 1H), 7.50 (dd, J = 9.3, 2.7 Hz, 1H), 7.23 (td, J = 8.5, 3.1 Hz, 3H), 7.12 (dd, J = 8.9, 4.8 Hz, 1H), 5.96 (d, J = 8.6 Hz, 1H), 4.82 (dd, J = 58.5, 20.5 Hz, 2H), 4.46 (s, 2H), 3.99 (s, 2H), 3.71 (d, J = 15.7 Hz, 4H), 3.03 - 2.92 (m, 1H), 2.62 (dd, J = 33.8, 15.6 Hz, 2H), 2.33 (t, J = 10.3 Hz, 4H), 2.15 - 2.08 (m, 1H). LCMS (ESI) calcd. for C₃₀H₃₁FN₇O₃S [M+H]⁺: 588.22, found, 588.1.

### Example 53: Preparation of 3-(5-((4-(5-(5-methyl-5H-pyrido[4,3-b]indol-7-yl)pyridin-2-yl)piperazin-1-yl)methyl)-1-thioxoisoindolin-2-yl)piperidine-2,6-dione (GT-08982)

With reference to the method of Scheme T-1, the target compound (GT-08982) was obtained (white solid, 14 mg, yield 17.35%). ¹H NMR (400 MHz, DMSO) δ 15.42 (s, 1H), 11.86 (s, 1H), 11.13 (d, J = 14.4 Hz, 1H), 9.78 (s, 1H), 8.74 (t, J = 5.0 Hz, 2H), 8.54 (d, J = 8.2 Hz, 1H), 8.35 - 8.13 (m, 3H), 8.06 - 7.81 (m, 4H), 7.16 (d, J = 9.0 Hz, 1H), 4.82 (dd, J = 57.7, 20.3 Hz, 1H), 4.55 (s, 3H), 4.14 (s, 3H), 3.56 (s, 4H), 3.42 - 3.38 (m, 2H), 3.25 - 3.07 (m, 3H), 3.04 - 2.92 (m, 1H), 2.70 - 2.54 (m, 2H), 2.18 - 2.05 (m, 1H). LCMS (ESI) calcd. for C₃₅H₃₄N₇O₂S [M+H]⁺: 616.25, found, 616.3.

### Example 54: Preparation of 3-(5-((4-(5-(5-(difluoromethyl)-5H-pyrido[4,3-b]indol-7-yl)pyridin-2-yl)piperazin-1-yl)methyl)-1-thioxoisoindolin-2-yl)piperidine-2,6-dione (GT-08979)

With reference to the method of Scheme T-1, the target compound (GT-08979) was obtained (white solid, 27 mg, yield 31.44%). ¹H NMR (400 MHz, DMSO) δ 11.89 (s, 1H), 11.15 (s, 1H), 10.00 (d, J = 13.2 Hz, 1H), 8.92 (t, J = 7.6 Hz, 1H), 8.79 - 8.60 (m, 2H), 8.41 (d, J = 6.6 Hz, 1H), 8.32 (s, 1H), 8.19 (dd, J = 9.0, 2.2 Hz, 1H), 7.97 (dd, J = 17.8, 10.1 Hz, 2H), 7.90 - 7.80 (m, 1H), 7.16 (d, J = 9.1 Hz, 1H), 5.96 (d, J = 9.2 Hz, 1H), 4.82 (dd, J = 58.0, 20.4 Hz, 1H), 4.54 (s, 3H), 3.91 (s, 2H), 3.47 - 3.30 (m, 4H), 3.24 - 3.08 (m, 3H), 2.98 (dd, J = 22.6, 9.1 Hz, 1H), 2.61 (dd, J = 28.4, 14.8 Hz, 2H), 2.18 - 2.02 (m, 1H). LCMS (ESI) calcd. for C₃₅H₃₂F₂N₇O₂S [M+H]⁺: 652.23, found, 652.3.

### Example 55: Preparation of 3-(5-((4-(3-methyl-5-(5-methyl-5H-pyrido[4,3-b]indol-7-yl)pyridin-2-yl)piperazin-1-yl)methyl)-1-thioxoisoindolin-2-yl)piperidine-2,6-dione (GT-08980)

With reference to the method of Scheme T-1, the target compound (GT-08980) was obtained (white solid, 17 mg, yield 21.44%). ¹H NMR (400 MHz, DMSO) δ 15.49 (s, 1H), 11.79 (s, 1H), 11.43 - 10.93 (m, 1H), 9.81 (s, 1H), 8.76 (d, J = 6.9 Hz, 1H), 8.68 (s, 1H), 8.57 (d, J = 8.2 Hz, 1H), 8.28 - 8.14 (m, 3H), 8.07 - 7.97 (m, 1H), 7.90 (dd, J = 13.4, 5.5 Hz, 2H), 5.97 (d, J = 8.7 Hz, 1H), 4.83 (dd, J = 58.8, 20.5 Hz, 1H), 4.60 (d, J = 22.9 Hz, 1H), 4.16 (d, J = 12.1 Hz, 3H), 3.67 (s, 4H), 3.43 (d, J = 9.7 Hz, 4H), 3.25 (d, J = 23.1 Hz, 3H), 2.98 (dd, J = 14.9, 10.9 Hz, 1H), 2.62 (dd, J = 27.2, 14.5 Hz, 1H), 2.37 (d, J = 9.1 Hz, 3H), 2.16 - 2.08 (m, 1H). LCMS (ESI) calcd. for C₃₆H₃₆N₇O₂S [M+H]⁺: 630.27, found, 630.3.

### Example 56: Preparation of 3-(5-((4-(3-chloro-5-(5-methyl-5H-pyrido[4,3-b]indol-7-yl)pyridin-2-yl)piperazin-1-yl)methyl)-1-thioxoisoindolin-2-yl)piperidine-2,6-dione (GT-08978)

With reference to the method of Scheme T-1, the target compound (GT-08978) was obtained (white solid, 17 mg, yield 20.58%). ¹H NMR (400 MHz, DMSO) δ 15.40 (s, 1H), 11.74 (s, 1H), 11.15 (s, 1H), 9.81 (s, 1H), 8.84 (d, J = 2.0 Hz, 1H), 8.77 (d, J = 6.9 Hz, 1H), 8.58 (d, J = 8.2 Hz, 1H), 8.46 (d, J = 2.0 Hz, 1H), 8.31 (s, 1H), 8.21 (d, J = 6.9 Hz, 1H), 7.94 (ddd, J = 28.2, 19.4, 10.4 Hz, 4H), 5.96 (s, 1H), 5.02 - 4.74 (m, 1H), 4.57 (s, 1H), 4.14 (d, J = 7.4 Hz, 3H), 3.96 (d, J = 11.3 Hz, 2H), 3.59 (s, 4H), 3.22 (s, 4H), 2.97 (d, J = 12.6 Hz, 1H), 2.72 - 2.55 (m, 2H), 2.17 - 2.04 (m, 1H). LCMS (ESI) calcd. for C₃₅H₃₃ClN₇O₂S [M+H]⁺: 650.21, found, 650.3.

### Example 57: Preparation of 3-(5-((4-(benzo[4,5]imidazo[1,2-a]pyrimidin-2-yl)piperazin-1-yl)methyl)-1-thioxoisoindolin-2-yl)piperidine-2,6-dione (GT-08981)

With reference to the method of Scheme T-1, the target compound (GT-08981) was obtained (white solid, 15 mg, yield 20.65%). ¹H NMR (400 MHz, DMSO) δ 14.19 (s, 1H), 12.40 (s, 1H), 11.15 (s, 1H), 9.43 (d, J = 7.8 Hz, 1H), 8.27 (d, J = 8.0 Hz, 1H), 8.02 - 7.93 (m, 2H), 7.82 (d, J = 7.9 Hz, 1H), 7.67 (d, J = 7.9 Hz, 1H), 7.59 (t, J = 7.4 Hz, 1H), 7.52 (t, J = 7.6 Hz, 1H), 7.41 (d, J = 7.9 Hz, 1H), 5.96 (d, J = 9.1 Hz, 1H), 4.97 - 4.74 (m, 2H), 4.49 (s, 2H), 4.08 (d, J = 86.4 Hz, 2H), 3.59 (d, J = 3.3 Hz, 1H), 3.43 (s, 2H), 3.31 - 3.18 (m, 3H), 3.05 - 2.92 (m, 1H), 2.62 (dd, J = 29.0, 15.8 Hz, 2H), 2.21 - 2.03 (m, 1H). LCMS (ESI) calcd. for C₂₈H₂₈N₇O₂S [M+H]⁺: 526.20, found, 526.3.

### Example 58: Preparation of N-(4-((4-((2-(2,6-dioxopiperidin-3-yl)-1-thioxoisoindolin-4-yl)methyl)piperazin-1-yl)methyl)-3-(trifluoromethyl)phenyl)-3-(imidazo[1,2-b]pyridazin-3-ylethynyl)-4-methylbenzamide (GT-08773)

With reference to the method of Scheme T-1, the target compound (GT-08773) was obtained (white solid, 16 mg, yield 23 %). ¹H NMR (400 MHz, DMSO) δ 11.20 (s, 1H), 10.73 (s, 1H), 8.76 (d, *J =* 4.4, 1.3 Hz, 1H), 8.34 - 8.26 (m, 3H), 8.23 (d, *J=* 1.5 Hz, 1H), 8.18 (d, *J =* 8.2 Hz, 1H), 8.04 - 7.86 (m, 4H), 7.65 (t, *J =* 7.6 Hz, 1H), 7.57 (d, *J=* 8.2 Hz, 1H), 7.44 (dd, *J =* 9.2, 4.4 Hz, 1H), 6.01 (d, *J =* 8.7 Hz, 1H), 5.31 (d, *J =* 20.4 Hz, 1H), 4.89 (d, *J =* 20.1 Hz, 1H), 4.41 (s, 2H), 4.02 (s, 2H), 3.48 - 3.45 (m, 1H), 3.41 - 3.27 (m, 4H), 3.14 (s, 2H), 3.05 - 2.94 (m, 2H), 2.67 (d, 1H), 2.62 (s, 3H), 2.17 - 2.08 (m, 1H), 1.35 - 1.18 (m, 1H). LCMS (ESI) calcd. for C₄₂H₃₈F₃N₈O₃S ⁺ [M+H]⁺: 791.27, found, 791.2.

### Example 59: Preparation of N-(4-(4-((2-(2,6-dioxopiperidin-3-yl)-1-thioxoisoindolin-4-yl)methyl)piperazin-1-yl)-3-(trifluoromethyl)phenyl)-3-(imidazo[1,2-b]pyridazin-3-ylethynyl)-4-methylbenzamide (GT-08774)

With reference to the method of Scheme T-1, the target compound (GT-08774) was obtained (white solid, 16 mg, yield 25 %). ¹H NMR (400 MHz, DMSO) δ 11.23 (s, 1H), 11.10 (s, 1H), 10.61 (s, 1H), 8.74 (dd, *J* = 4.4, 1.5 Hz, 1H), 8.28 (dd, *J* = 9.2, 1.5 Hz, 1H), 8.25 (s, 1H), 8.23 (dd, *J* = 9.4, 2.1 Hz, 2H), 8.16 - 8.10 (m, 1H), 8.06 (d, *J* = 7.5 Hz, 1H), 8.01 (d, *J* = 7.7 Hz, 1H), 7.96 (dd, *J =* 8.0, 1.8 Hz, 1H), 7.69 (t, *J =* 7.7 Hz, 1H), 7.56 (d, *J =* 8.2 Hz, 2H), 7.41 (dd, *J =* 9.2*,* 4.5 Hz, 1H), 6.03 (d, *J =* 8.9 Hz, 1H), 5.33 (d, *J =* 20.6 Hz, 1H), 4.95 (d, *J =* 20.3 Hz, 1H), 4.55 (s, 2H), 3.53 (d, 1H), 3.47 - 3.44 (m, 2H), 3.37 - 3.24 (m, 4H), 3.05 (d, *J =* 9.5 Hz, 2H), 2.75 - 2.67 (m, 1H), 2.61 (s, 3H), 2.21 - 2.08 (m, 1H), 1.35 - 1.19 (m, 1H). LCMS (ESI) calcd. for C₄₁H₃₆F₃N₈O₃S ⁺ [M+H]⁺: 777.26, found, 777.1.

### Example 60: Preparation of N-(4-(chlorodifluoromethoxy)phenyl)-6-(4-((2-(2,6-dioxopiperidin-3-yl)-1-thioxoisoindolin-4-yl)methyl)piperazin-1-yl)-5-(1H-pyrazol-3-yl)nicotinamide (GT-08775)

With reference to the method of Scheme T-1, the target compound (GT-08775) was obtained (white solid, 15 mg, yield 25 %). ¹H NMR (400 MHz, DMSO) δ 11.22 (s, 1H), 11.15 (s, 1H), 10.52 (s, 1H), 8.81 (d, *J* = 2.4 Hz, 1H), 8.46 (d, *J =* 2.4 Hz, 1H), 7.97 (t, *J* = 18.4, 9.9 Hz, 2H), 7.93 - 7.84 (m, 3H), 7.67 (t, *J =* 7.7 Hz, 1H), 7.36 (d, *J =* 9.0 Hz, 2H), 6.82 (d, *J =* 2.2 Hz, 1H), 6.02 (d, *J* = 9.0 Hz, 1H), 5.33 (d, *J =* 20.3 Hz, 1H), 4.91 (d, *J =* 20.3 Hz, 1H), 4.49 (d, 2H), 3.73 - 3.69 (m, 2H), 3.46 - 3.43 (m, 2H), 3.35 - 3.28 (m, 4H), 3.04 - 2.95 (m, 1H), 2.70 (d, *J =* 17.8 Hz, 1H), 2.47 - 2.38 (m, 1H), 2.20 - 2.04 (m, 1H). LCMS (ESI) calcd. for C₃₄H₃₂ClF₂N₈O₄S ⁺ [M+H]⁺: 721.19, found, 721.1.

### Example 61: Preparation of N-(4-(chlorodifluoromethoxy)phenyl)-6-(4-(4-((2-(2,6-dioxopiperidin-3-yl)-1-thioxoisoindolin-4-yl)methyl)piperazin-1-yl)piperidin-1-yl)-5-(1H-pyrazol-3-yl)nicotinamide (GT-08776)

With reference to the method of Scheme T-1, the target compound (GT-08776) was obtained (white solid, 11 mg, yield 16 %). ¹H NMR (400 MHz, DMSO) δ 11.19 (s, 1H), 10.47 (d, *J =* 14.9 Hz, 1H), 8.76 (d, *J =* 12.7, 2.4 Hz, 1H), 8.35 (d, *J =* 19.0, 2.4 Hz, 1H), 7.99 - 7.92 (m, 1H), 7.92 - 7.72 (m, 4H), 7.63 (t, *J =* 7.5 Hz, 1H), 7.35 (d, *J* = 9.0 Hz, 2H), 6.67 (d, *J =* 6.6 Hz, 1H), 5.99 (s, 1H), 5.21 (s, 1H), 4.85 (d, *J =* 20.2 Hz, 1H), 4.22 (s, 2H), 3.77 (d, *J =* 11.9 Hz, 4H), 3.47 - 3.40 (m, 4H), 3.38 - 3.24 (m, 4H), 3.04 - 2.96 (m, 1H), 2.77 (t, *J =* 12.1 Hz, 2H), 2.69 (d, *J =* 14.6 Hz, 1H), 2.15 - 2.02 (m, 3H), 1.84 - 1.71 (m, 2H). LCMS (ESI) calcd. for C₃₉H₄₁ClF₂N₉O₄S ⁺ [M+H]⁺: 804.27, found, 804.1.

### Example 62: Preparation of N-(4-((4-((2-(2,6-dioxopiperidin-3-yl)-1-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide (GT-08767)

With reference to the method of Scheme T-1, the target compound (GT-08767) was obtained (white solid, 12 mg, yield 18 %). ¹H NMR (400 MHz, DMSO) δ 11.14 (s, 1H), 10.72 (s, 1H), 8.76 (dd, *J* = 4.4, 1.5 Hz, 1H), 8.35 - 8.26 (m, 3H), 8.23 (d, *J = 1.7* Hz, 1H), 8.17 (d, *J =* 8.4 Hz, 1H), 8.06 - 7.85 (m, 4H), 7.80 (d, *J =* 8.1 Hz, 1H), 7.57 (d, *J =* 8.3 Hz, 1H), 7.44 (dd, *J =* 9.2, 4.5 Hz, 1H), 5.96 (d, *J =* 8.3 Hz, 1H), 4.88 (d, 1H), 4.73 (d, *J =* 20.4 Hz, 1H), 4.48 (s, 2H), 4.01 (s, 2H), 3.75 (s, 2H), 3.44 - 3.36 (m, 2H), 3.27 - 3.12 (m, 3H), 3.03 - 2.88 (m, 2H), 2.70 - 2.64 (m, 1H), 2.62 (s, 3H), 2.59 - 2.54 (m, 1H), 2.14 - 2.03 (m, 1H). LCMS (ESI) calcd. for C₄₂H₃₈F₃N₈O₃S ⁺ [M+H]⁺: 791.27, found, 791.1.

### Example 63: Preparation of N-(4-(4-((2-(2,6-dioxopiperidin-3-yl)-1-thioxoisoindolin-5-yl)methyl)piperazin-1-yl)-3-(trifluoromethyl)phenyl)-3-(imidazo[1,2-b]pyridazin-3-ylethynyl)-4-methylbenzamide (GT-08768)

With reference to the method of Scheme T-1, the target compound (GT-08768) was obtained (white solid, 12 mg, yield 19 %). ¹H NMR (400 MHz, DMSO) δ 11.18 (s, 1H), 11.15 (s, 1H), 10.62 (s, 1H), 8.74 (dd, *J* = 4.4, 1.4 Hz, 1H), 8.28 (dd, *J* = 9.2, 1.5 Hz, 1H), 8.26 (s, 1H), 8.25 - 8.20 (m, 2H), 8.13 (d, *J =* 8.7 Hz, 1H), 8.02 - 7.92 (m, 3H), 7.86 (d, *J =* 8.2 Hz, 1H), 7.56 (d, *J =* 8.2 Hz, 2H), 7.42 (dd, *J =* 9.2, 4.5 Hz, 1H), 5.97 (d, *J =* 8.9 Hz, 1H), 4.84 (dd, 2H), 4.58 (s, 2H), 3.45 - 3.44 (m, 4H), 3.34 - 3.26 (m, 2H), 3.25 - 3.16 (m, 2H), 3.05 (d, *J =* 11.2 Hz, 2H), 2.66 (d, *J =* 14.5 Hz, 1H), 2.61 (s, 3H), 2.19 - 2.06 (m, 1H). LCMS (ESI) calcd. for C₄₁H₃₆F₃N₈O₃S ⁺ [M+H]⁺: 777.26, found, 777.1.

### Example 64: Preparation of N-(4-(chlorodifluoromethoxy)phenyl)-6-(4-((2-(2,6-dioxopiperidin-3-yl)-1-thioxoisoindolin-5-yl)methyl)piperazin-1-yl)-5-(1H-pyrazol-3-yl)nicotinamide (GT-08769)

With reference to the method of Scheme T-1, the target compound (GT-08769) was obtained (white solid, 7 mg, yield 14 %). ¹H NMR (400 MHz, DMSO) δ 11.14 (s, 1H), 11.05 (s, 1H), 10.53 (s, 1H), 8.81 (d, *J =* 2.3 Hz, 1H), 8.45 (d, *J =* 2.3 Hz, 1H), 7.97 (d, *J =* 8.0 Hz, 1H), 7.94 - 7.88 (m, 3H), 7.86 (s, 1H), 7.78 (d, *J =* 7.8 Hz, 1H), 7.36 (d, *J =* 9.0 Hz, 2H), 6.80 (d, *J =* 2.1 Hz, 1H), 5.95 (s, 1H), 4.88 (d, *J =* 20.5 Hz, 1H), 4.74 (d, *J =* 20.3 Hz, 1H), 4.51 (s, 2H), 3.68 (s, 3H), 3.25 (d, *J =* 10.8 Hz, 5H), 2.99 - 2.92 (m, 1H), 2.65 (d, *J* = 16.3 Hz, 2H), 2.09 (d, *J* = 6.1 Hz, 1H). LCMS (ESI) calcd. for C₃₄H₃₂ClF₂N₈O₄S ⁺ [M+H]⁺: 721.19, found, 721.1.

### Example 65: Preparation of N-(4-(chlorodifluoromethoxy)phenyl)-6-(4-(4-((2-(2,6-dioxopiperidin-3-yl)-1-thioxoisoindolin-5-yl)methyl)piperazin-1-yl)piperidin-1-yl)-5-(1H-pyrazol-3-yl)nicotinamide (GT-08770)

With reference to the method of Scheme T-1, the target compound (GT-08770) was obtained (white solid, 16 mg, yield 27 %). ¹H NMR (400 MHz, DMSO) δ 11.14 (s, 1H), 10.48 (s, 1H), 8.78 (d, *J* = 2.3 Hz, 1H), 8.37 (d, *J =* 2.3 Hz, 1H), 7.98 - 7.85 (m, 4H), 7.84 - 7.71 (m, 2H), 7.35 (d, *J =* 8.9 Hz, 2H), 6.68 (d, *J* = 2.1 Hz, 1H), 5.95 (d, 1H), 4.86 (d, *J =* 20.6 Hz, 1H), 4.72 (d, *J =* 20.2 Hz, 1H), 4.39 (s, 2H), 3.77 (d, *J =* 11.6 Hz, 3H), 3.62 (s, 2H), 3.46 - 3.38 (m, 6H), 3.00 - 2.93 (m, 1H), 2.77 (t, *J=* 12.4 Hz, 2H), 2.69 - 2.59 (m, 2H), 2.14 - 2.03 (m, 3H), 1.76 (d, *J* = 8.7 Hz, 2H). LCMS (ESI) calcd. for C₃₉H₄₁ClF₂N₉O₄S ⁺ [M+H]⁺: 804.27, found, 804.0.

### Example 66: Preparation of N-(3-(((2-((4-(4-((2-(2,6-dioxopiperidin-3-yl)-1-thioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide (GT-08779)

With reference to the method of Scheme T-1, the target compound (GT-08779) was obtained (white solid, 12 mg, yield 18 %). ¹H NMR (400 MHz, DMSO) δ 11.50 (s, 1H), 11.15 (s, 1H), 10.18 (s, 1H), 8.69 (d, *J =* 2.5 Hz, 1H), 8.60 (d, *J =* 2.4 Hz, 1H), 8.36 (s, 1H), 8.13 - 7.88 (m, 3H), 7.84 (d, *J =* 8.2 Hz, 1H), 7.27 (s, 2H), 6.83 (d, *J* = 7.7 Hz, 2H), 5.97 (d, *J =* 8.6 Hz, 1H), 4.95 (d, *J =* 4.9 Hz, 2H), 4.89 (d, *J =* 20.5 Hz, 1H), 4.75 (d, *J =* 20.3 Hz, 1H), 4.53 (s, 2H), 3.40 (s, 2H), 3.20 - 3.16 (m, 6H), 3.16 - 3.11 (m, 4H), 2.96 (d, *J* = 12.5 Hz, 1H), 2.69 - 2.52 (m, 3H), 2.15 - 2.07 (m, 1H), 1.37 - 1.27 (m, 1H). LCMS (ESI) calcd. for C₃₆H₃₉F₃N₁₁O₄S₂ ⁺ [M+H]⁺: 810.26, found, 810.0.

### Example 67: Preparation of 3-(5-((4-(4-((4-((3-(methylsulfonyl)benzyl)amino)-5-(trifluoromethyl)pyrimidin-2-yl)amino)phenyl)piperazin-1-yl)methyl)-1-thioxoisoindolin-2-yl)piperidine-2,6-dione (GT-08780)

With reference to the method of Scheme T-1, the target compound (GT-08780) was obtained (white solid, 15 mg, yield 23 %). ¹H NMR (400 MHz, DMSO) δ 11.50 (s, 1H), 11.13 (s, 1H), 10.45 - 10.09 (m, 1H), 8.67 (s, 1H), 8.37 (s, 1H), 8.03 - 7.95 (m, 2H), 7.91 (s, 1H), 7.82 (dd, *J =* 13.8, 7.7 Hz, 2H), 7.61 (t, *J =* 7.6 Hz, 1H), 7.55 (s, 1H), 7.29 (d, *J =* 6.4 Hz, 2H), 6.89 (d, *J =* 8.7 Hz, 2H), 5.97 (d, *J =* 9.1 Hz, 1H), 4.90 (d, *J =* 20.4 Hz, 1H), 4.76 (d, *J =* 16.9 Hz, 1H), 4.72 (d, *J =* 5.7 Hz, 2H), 4.54 (s, 2H), 3.63 - 3.56 (m, 2H), 3.43 - 3.37 (m, 2H), 3.22 - 3.16 (m, 4H), 3.15 (s, 3H), 3.02 - 2.93 (m, 1H), 2.70 - 2.54 (m, 2H), 2.13 - 2.06 (m, 1H). LCMS (ESI) calcd. for C₃₇H₃₈F₃N₈O₄S₂ ⁺ [M+H]⁺: 779.24, found, 779.1.

### Example 68: Preparation of N-(3-(((2-((4-(4-((2-(2,6-dioxopiperidin-3-yl)-1-thioxoisoindolin-5-yl)methyl)piperazin-1-yl)phenyl)amino)-5-(trifluoromethyl)pyrimidin-4-yl)amino)methyl)phenyl)-N-methylmethanesulfonamide (GT-08781)

With reference to the method of Scheme T-1, the target compound (GT-08781) was obtained (white solid, 7 mg, yield 11 %). ¹H NMR (400 MHz, DMSO) δ 11.32 (s, 1H), 11.15 (s, 1H), 10.11 (s, 1H), 8.38 (s, 1H), 8.30 (s, 1H), 8.02 - 7.93 (m, 2H), 7.82 (d, *J =* 8.4 Hz, 1H), 7.40 - 7.34 (m, 4H), 7.27 (d, *J =* 7.2 Hz, 1H), 7.20 (s, 1H), 6.90 (d, *J =* 8.8 Hz, 2H), 5.96 (d, 1H), 4.89 (d, *J* = 20.4 Hz, 1H), 4.75 (d, *J =* 20.4 Hz, 1H), 4.63 (d, *J =* 5.0 Hz, 2H), 4.54 (s, 2H), 3.71 (d, 2H), 3.46 - 3.40 (m, 3H), 3.18 - 3.12 (m, 6H), 2.87 (s, 3H), 2.70 - 2.53 (m, 3H), 2.13 - 2.07 (m, 1H). LCMS (ESI) calcd. for C₃₈H₄₁F₃N₉O₄S₂ ⁺ [M+H]⁺: 808.27, found, 808.1.

### Example 69: Preparation of 2-((2-((4-(1-((2-(2,6-dioxopiperidin-3-yl)-1-thioxoisoindolin-5-yl)methyl)piperidin-4-yl)-2-isopropoxy-5-methylphenyl)amino)-5-(trifluoromethyl)pyridin-4-yl)amino)-N-methylbenzamide (GT-08782)

With reference to the method of Scheme T-1, the target compound (GT-08782) was obtained (white solid, 8 mg, yield 12 %). ¹H NMR (400 MHz, DMSO) δ 11.15 (s, 1H), 11.04 (s, 1H), 10.50 (s, 1H), 9.29 (s, 1H), 8.73 (d, *J* = 4.6 Hz, 1H), 8.22 (s, 1H), 7.98 (d, *J* = 7.6 Hz, 1H), 7.82 (d, *J* = 8.4 Hz, 1H), 7.74 (d, *J =* 6.8 Hz, 1H), 7.59 (d, *J =* 7.9 Hz, 1H), 7.51 (t, *J* = 7.5 Hz, 1H), 7.32 - 7.15 (m, 2H), 6.87 (s, 1H), 6.65 (s, 1H), 5.96 (d, 1H), 4.89 (d, *J =* 20.5 Hz, 1H), 4.75 (d, *J =* 20.4 Hz, 1H), 4.57 - 4.35 (m, 3H), 3.13 - 3.06 (m, 2H), 3.01 - 2.93 (m, 2H), 2.75 (d, *J=* 4.5 Hz, 3H), 2.69 - 2.57 (m, 2H), 2.24 (s, 3H), 2.19 - 2.06 (m, 3H), 1.83 (d, *J =* 13.0 Hz, 2H), 1.34 - 1.22 (m, 2H), 1.20 (d, *J =* 5.1 Hz, 6H). LCMS (ESI) calcd. for C₄₃H₄₇F₃N₇O₄S ⁺ [M+H]⁺: 814.34, found, 814.2.

### Example 70: Preparation of N-(3-(((2-((4-(4-((2-(2,6-dioxopiperidin-3-yl)-1-thioxoisoindolin-4-yl)methyl)piperazin-1-yl)phenyl)amino)-5-(trifluoromethyl)pyrimidin-4-yl)amino)methyl)pyrazin-2-yl)-N-methylmethanesulfonamide (GT-08783)

With reference to the method of Scheme T-1, the target compound (GT-08783) was obtained (white solid, 9 mg, yield 14 %). ¹H NMR (400 MHz, DMSO) δ 11.37 (s, 1H), 11.21 (s, 1H), 10.02 (s, 1H), 8.69 (d, *J =* 2.5 Hz, 1H), 8.59 (d, *J =* 2.5 Hz, 1H), 8.32 (s, 1H), 8.02 (t, *J =* 8.2 Hz, 2H), 7.94 - 7.76 (m, 1H), 7.69 (t, *J =* 7.7 Hz, 1H), 7.30 (s, 2H), 6.84 (d, *J =* 8.0 Hz, 2H), 6.02 (d, *J =* 9.2 Hz, 1H), 5.39 (d, *J =* 20.4 Hz, 1H), 5.01 - 4.86 (m, 3H), 4.57 - 4.39 (m, 2H), 3.68 (d, 4H), 3.47 - 3.36 (m, 3H), 3.32 - 3.26 (m, 2H), 3.18 (s, 3H), 3.13 (s, 2H), 3.04 - 2.95 (m, 1H), 2.70 (d, *J=* 18.4 Hz, 1H), 2.48 - 2.40 (m, 1H), 2.18 - 2.07 (m, 1H). LCMS (ESI) calcd. for C₃₆H₃₉F₃N₁₁O₄S₂ ⁺ [M+H]⁺: 810.26, found, 810.1.

### Example 71: Preparation of 3-(4-((4-(4-((4-((3-(methylsulfonyl)benzyl)amino)-5-(trifluoromethyl)pyrimidin-2-yl)amino)phenyl)piperazin-1-yl)methyl)-1-thioxoisoindolin-2-yl)piperidine-2,6-dione (GT-08784)

With reference to the method of Scheme T-1, the target compound (GT-08784) was obtained (white solid, 15 mg, yield 23 %). ¹H NMR (400 MHz, DMSO) δ 11.31 (s, 1H), 11.21 (s, 1H), 10.05 (s, 1H), 8.30 (s, 1H), 8.06 - 7.96 (m, 2H), 7.91 (s, 1H), 7.80 (d, *J =* 7.3 Hz, 1H), 7.70 (t, *J =* 7.7 Hz, 1H), 7.64 - 7.50 (m, 3H), 7.32 (s, 2H), 6.88 (d, *J =* 8.5 Hz, 2H), 6.05 - 5.94 (m, 1H), 5.38 (d, *J =* 20.5 Hz, 1H), 4.96 - 4.87 (m, 1H), 4.85 - 4.73 (m, 1H), 4.73 - 4.65 (m, 2H), 4.49 (dd, *J =* 25.2, 13.5 Hz, 2H), 3.75 (d, *J* = 11.8 Hz, 2H), 3.46 - 3.40 (m, 4H), 3.23 - 3.17 (m, 2H), 3.15 (s, 3H), 3.05 - 2.91 (m, 2H), 2.76 - 2.63 (m, 2H), 2.23 - 1.99 (m, 2H). LCMS (ESI) calcd. for C₃₇H₃₈F₃N₈O₄S₂ ⁺ [M+H]⁺: 779.24, found, 779.1.

### Example 72: Preparation of N-(3-(((2-((4-(4-((2-(2,6-dioxopiperidin-3-yl)-1-thioxoisoindolin-4-yl)methyl)piperazin-1-yl)phenyl)amino)-5-(trifluoromethyl)pyrimidin-4-yl)amino)methyl)phenyl)-N-methylmethanesulfonamide (GT-08883)

With reference to the method of Scheme T-1, the target compound (GT-08883) was obtained (white solid, 9 mg, yield 14 %). ¹H NMR (400 MHz, DMSO) δ 11.36 (s, 1H), 11.21 (s, 1H), 10.10 (s, 1H), 8.43 (s, 1H), 8.29 (s, 1H), 8.02 (t, *J =* 6.9 Hz, 2H), 7.69 (t, *J =* 7.6 Hz, 1H), 7.40 - 7.34 (m, 4H), 7.27 (d, *J* = 8.1 Hz, 1H), 7.21 (s, 1H), 6.91 (d, *J =* 8.9 Hz, 2H), 6.01 (s, 1H), 5.39 (d, *J=* 20.4 Hz, 1H), 4.93 (d, *J=* 20.5 Hz, 1H), 4.63 (d, *J =* 5.5 Hz, 2H), 4.57 - 4.41 (m, 2H), 3.72 (d, 2H), 3.46 - 3.43 (m, 3H), 3.31 - 3.26 (m, 2H), 3.15 (s, 3H), 3.02 - 2.97 (m, 1H), 2.87 (s, 3H), 2.70 (d, *J =* 17.9 Hz, 1H), 2.16 - 2.10 (m, 1H), 1.33 - 1.20 (m, 2H). LCMS (ESI) calcd. for C₃₈H₄₁F₃N₉O₄S₂ ⁺ [M+H]⁺: 808.27, found, 808.1.

### Example 73: Preparation of 2-((2-((4-(1-((2-(2,6-dioxopiperidin-3-yl)-1-thioxoisoindolin-4-yl)methyl)piperidin-4-yl)-2-isopropoxy-5-methylphenyl)amino)-5-(trifluoromethyl)pyridin-4-yl)amino)-N-methylbenzamide (GT-08884)

With reference to the method of Scheme T-1, the target compound (GT-08884) was obtained (white solid, 8 mg, yield 12 %). ¹H NMR (400 MHz, DMSO) δ 11.22 (s, 1H), 11.13 (s, 1H), 10.49 (s, 1H), 9.29 (s, 1H), 8.73 (d, *J =* 4.5 Hz, 1H), 8.22 (s, 1H), 8.06 - 7.95 (m, 2H), 7.74 (d, *J =* 6.8 Hz, 1H), 7.69 (t, *J* = 7.7 Hz, 1H), 7.59 (d, *J =* 8.0 Hz, 1H), 7.51 (t, *J =* 7.8 Hz, 1H), 7.29 - 7.18 (m, 2H), 6.90 (s, 1H), 6.65 (s, 1H), 6.02 (s, 1H), 5.44 (d, *J =* 20.5 Hz, 1H), 4.93 (d, *J =* 20.4 Hz, 1H), 4.55 - 4.30 (m, 3H), 3.50 (s, 3H), 3.25 - 3.17 (m, 3H), 3.02 - 2.93 (m, 2H), 2.75 (d, *J =* 4.6 Hz, 3H), 2.73 - 2.67 (m, 1H), 2.25 (s, 3H), 2.21 - 2.09 (m, 2H), 1.83 (d, *J =* 12.5 Hz, 2H), 1.21 - 1.17 (m, 6H). LCMS (ESI) calcd. for C₄₃H₄₇F₃N₇O₄S ⁺ [M+H]⁺: 814.34, found, 814.2.

### Example 74: Preparation of 3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-1-thioxoisoindolin-2-yl)piperidine-2,6-dione (GT-08674)

With reference to the method of Scheme T-1, the target compound (GT-08674) was obtained (white solid, 8 mg, yield 12 %). ¹H NMR (400 MHz, DMSO) δ 11.15 (s, 1H), 11.00 (s, 1H), 9.62 (s, 1H), 8.49 - 8.33 (m, 2H), 8.31 (s, 1H), 7.98 (d, *J =* 7.9 Hz, 2H), 7.88 - 7.79 (m, 2H), 7.63 (t, *J =* 7.6 Hz, 1H), 7.45 (s, 1H), 7.39 (t, *J =* 7.8 Hz, 1H), 6.81 (s, 1H), 6.06 - 5.87 (m, 1H), 4.82 (dd, *J =* 55.9, 20.4 Hz, 2H), 4.49 (s, 3H), 3.15 - 3.06 (m, 2H), 3.02 - 2.90 (m, 2H), 2.73 - 2.54 (m, 3H), 2.13 (s, 6H), 1.85 (d, *J =* 12.5 Hz, 2H), 1.23 (d, *J =* 6.1 Hz, 6H), 1.15 (d, *J* = 6.8 Hz, 6H). LCMS (ESI) calcd. for C₄₂H₄₉ClN₇O₅S₂ ⁺ [M+H]⁺: 830.29, found, 830.1.

### Example 75: Preparation of 3-(5-((4-(4-((5-chloro-4-((2-(dimethylphosphoryl)phenyl)amino)pyrimidin-2-yl)amino)-3-methoxyphenyl)piperazin-1-yl)methyl)-1-thioxoisoindolin-2-yl)piperidine-2,6-dione (GT-08675)

With reference to the method of Scheme T-1, the target compound (GT-08675) was obtained (brown solid, 19 mg, yield 30 %). ¹H NMR (400 MHz, DMSO) δ 12.04 (s, 1H), 11.80 (s, 1H), 11.15 (s, 1H), 9.51 (s, 1H), 8.33 (s, 1H), 8.28 (s, 1H), 8.03 (s, 1H), 7.98 (d, *J =* 7.9 Hz, 1H), 7.86 (d, *J =* 7.7 Hz, 1H), 7.66 - 7.60 (m, 1H), 7.43 - 7.33 (m, 2H), 7.25 (t, *J =* 7.2 Hz, 1H), 6.74 (s, 1H), 6.57 (d, *J =* 8.8 Hz, 1H), 5.96 (s, 1H), 4.94 - 4.77 (m, 2H), 4.63 (s, 1H), 4.56 (s, 2H), 3.88 (d, *J =* 10.9 Hz, 2H), 3.79 (s, 3H), 3.01 - 2.89 (m, 2H), 2.70 - 2.56 (m, 3H), 2.15 - 2.02 (m, 2H), 1.80 (d, *J =* 13.7 Hz, 6H), 1.36 - 1.32 (m, 2H). LCMS (ESI) calcd. for C₃₇H₄₁ClN₈O₄PS ⁺ [M+H]⁺: 759.24, found, 759.1.

### Example 76: Preparation of 3-(4-((4-(4-((S-chloro-4-((2-(isopropylsulfonyl)phenyl)amino)pyrimidin-2-yl)amino)-5-isopropoxy-2-methylphenyl)piperidin-1-yl)methyl)-1-thioxoisoindolin-2-yl)piperidine-2,6-dione (GT-08676)

With reference to the method of Scheme T-1, the target compound (GT-08676) was obtained (white solid, 7 mg, yield 9 %). ¹H NMR (400 MHz, DMSO) δ 11.22 (s, 1H), 11.06 (s, 1H), 9.63 (s, 1H), 8.46 - 8.33 (m, 2H), 8.31 (s, 1H), 8.01 (dd, *J =* 7.6, 4.4 Hz, 2H), 7.85 (d, *J =* 8.0, 1.4 Hz, 1H), 7.71 - 7.61 (m, 2H), 7.44 (s, 1H), 7.39 (t, *J =* 7.6 Hz, 1H), 6.85 (s, 1H), 6.13 - 5.91 (m, 1H), 5.42 (d, *J =* 20.2 Hz, 1H), 4.93 (d, *J =* 20.3 Hz, 1H), 4.57 - 4.32 (m, 3H), 3.47 - 3.40 (m, 5H), 3.27 - 3.19 (m, 2H), 3.03 - 2.92 (m, 2H), 2.70 (d, *J =* 19.6 Hz, 1H), 2.30 - 2.18 (m, 2H), 2.12 (s, 3H), 1.84 (d, *J =* 12.1 Hz, 2H), 1.21 (dd, *J* = 6.0, 3.3 Hz, 6H), 1.15 (d, *J* = 6.8 Hz, 6H). LCMS (ESI) calcd. for C₄₂H₄₉ClN₇O₅S₂ ⁺ [M+H]⁺: 830.29, found, 830.1.

### Example 77: Preparation of 3-(4-((4-(4-((5-chloro-4-((2-(dimethylphosphoryl)phenyl)amino)pyrimidin-2-yl)amino)-3-methoxyphenyl)piperazin-1-yl)methyl)-1-thioxoisoindolin-2-yl)piperidine-2,6-dione (GT-08677)

With reference to the method of Scheme T-1, the target compound (GT-08677) was obtained (brown solid, 9 mg, yield 12 %). ¹H NMR (400 MHz, DMSO) δ 11.88 (s, 1H), 11.59 (s, 1H), 11.22 (s, 1H), 9.22 (s, 1H), 8.38 (s, 1H), 8.23 (s, 1H), 8.09 - 7.98 (m, 2H), 7.70 (t, *J =* 7.6 Hz, 1H), 7.65 - 7.58 (m, 1H), 7.37 (d, *J =* 8.6 Hz, 2H), 7.21 (t, *J =* 6.9 Hz, 1H), 6.73 (d, *J =* 1.9 Hz, 1H), 6.57 (d, *J =* 8.7 Hz, 1H), 6.13 - 5.84 (m, 1H), 5.46 (d, *J =* 20.4 Hz, 1H), 5.08 - 4.86 (m, 1H), 4.60 - 4.38 (m, 2H), 3.88 (d, *J* = 8.5 Hz, 2H), 3.79 (s, 3H), 3.34 - 3.25 (m, 5H), 3.07 - 2.90 (m, 2H), 2.72 (d, 1H), 2.16 - 2.09 (m, 1H), 1.79 (d, *J =* 13.6 Hz, 6H), 1.33 - 1.20 (m, 1H). LCMS (ESI) calcd. for C₃₇H₄₁ClN₈O₄PS ⁺ [M+H]⁺: 759.24 , found, 759.1.

### Example 78: Preparation of 3-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)-1-thioxoisoindolin-2-yl)piperidine-2,6-dione (GT-08678)

With reference to the method of Scheme T-1, the target compound (GT-08678) was obtained (white solid, 17 mg, yield 18 %). ¹H NMR (400 MHz, DMSO) δ 11.15 (s, 1H), 10.75 (s, 1H), 9.69 (s, 1H), 8.52 (s, 1H), 8.37 - 8.27 (m, 2H), 8.14 (s, 1H), 7.99 (d, *J =* 7.9 Hz, 1H), 7.86 (d, *J =* 7.9, 1.5 Hz, 1H), 7.79 (d, *J =* 7.8 Hz, 1H), 7.65 (t, *J =* 7.7 Hz, 1H), 7.47 - 7.39 (m, 2H), 6.80 (s, 1H), 5.97 (d, *J =* 8.5 Hz, 1H), 4.83 (dd, *J =* 57.4, 20.6 Hz, 2H), 4.52 - 4.47 (m, 2H), 3.49 - 3.39 (m, 5H), 3.17 - 3.06 (m, 2H), 3.01 - 2.90 (m, 2H), 2.66 (d, *J =* 16.6 Hz, 1H), 2.09 (s, 6H), 1.94 - 1.82 (m, 2H), 1.23 (d, *J =* 6.1 Hz, 6H), 1.14 (d, *J =* 6.8 Hz, 6H). LCMS (ESI) calcd. for C₄₂H₄₉ClN₇O₅S₂ ⁺ [M+H]⁺: 830.29, found, 830.1.

### Example 79: Preparation of 3-(6-((4-(4-((5-chloro-4-((2-(dimethylphosphoryl)phenyl)amino)pyrimidin-2-yl)amino)-3-methoxyphenyl)piperazin-1-yl)methyl)-1-thioxoisoindolin-2-yl)piperidine-2,6-dione (GT-08679)

With reference to the method of Scheme T-1, the target compound (GT-08679) was obtained (white solid, 13 mg, yield 15 %). ¹H NMR (400 MHz, DMSO) δ 11.92 (s, 1H), 11.34 (s, 1H), 11.13 (s, 1H), 9.35 (s, 1H), 8.38 (s, 1H), 8.25 (s, 1H), 8.17 (s, 1H), 8.04 (d, *J =* 7.7 Hz, 1H), 7.80 (d, *J =* 7.9 Hz, 1H), 7.64 - 7.58 (m, 1H), 7.37 (d, *J =* 8.7 Hz, 2H), 7.22 (t, *J =* 7.3 Hz, 1H), 6.74 (d, *J =* 2.1 Hz, 1H), 6.56 (d, *J =* 8.7 Hz, 1H), 5.95 (s, 1H), 4.81 (dd, *J =* 37.3, 18.7 Hz, 2H), 4.59 (s, 2H), 3.90 (d, *J =* 7.9 Hz, 2H), 3.79 (s, 3H), 3.44 - 3.40 (m, 3H), 3.23 - 3.18 (m, 4H), 2.71 - 2.59 (m, 2H), 2.12 - 2.05 (m, 1H), 1.79 (d, *J =* 13.6 Hz, 6H). LCMS (ESI) calcd. for C₃₇H₄₁ClN₈O₄PS ⁺ [M+H]⁺: 759.24, found, 759.0.

### Example 80: Preparation of 3-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-thioxoisoindolin-2-yl)piperidine-2,6-dione (GT-08680)

With reference to the method of Scheme T-1, the target compound (GT-08680) was obtained (pink solid, 14 mg, yield 16 %). ¹H NMR (400 MHz, DMSO) δ 12.03 (s, 1H), 11.15 (s, 2H), 9.54 (s, 1H), 8.37 (s, 1H), 8.32 (s, 1H), 8.20 (s, 1H), 8.09 (t, *J =* 6.8 Hz, 1H), 7.78 (d, *J =* 7.9 Hz, 1H), 7.65 (dd, *J =* 13.5, 7.4 Hz, 1H), 7.54 - 7.38 (m, 2H), 7.27 (t, *J =* 7.3 Hz, 1H), 7.02 (s, 1H), 6.85 (s, 1H), 5.97 (d, *J =* 8.2 Hz, 1H), 4.83 (d, *J =* 37.3 Hz, 2H), 4.68 (d, *J =* 12.7 Hz, 1H), 4.45 (s, 1H), 3.91 (s, 2H), 3.83 (s, 4H), 3.06 (s, 1H), 2.99 - 2.93 (m, 1H), 2.67 (s, 1H), 2.63 (d, *J =* 4.2 Hz, 3H), 2.42 - 2.33 (m, 2H), 2.17 - 2.07 (m, 2H), 1.80 (d, *J* = 13.7 Hz, 6H), 1.35 - 1.25 (m, 1H). LCMS (ESI) calcd. for C₃₉H₄₅ClN₈O₄PS ⁺ [M+H]⁺: 787.27, found, 787.1.

### Example 81: Preparation of (9R)-N-(1-(4-(4-((2-(2,6-dioxopiperidin-3-yl)-1-thioxoisoindolin-5-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-08777)

With reference to the method of Scheme T-1, the target compound (GT-08777) was obtained (white solid, 19 mg, yield 30 %). ¹H NMR (400 MHz, DMSO) δ 11.23 (s, 1H), 11.14 (s, 1H), 10.64 (s, 1H), 8.35 (d, *J =* 4.8 Hz, 1H), 8.30 (d, *J =* 8.2 Hz, 1H), 8.17 (s, 1H), 7.99 - 7.95 (m, 1H), 7.91 (d, *J =* 8.2 Hz, 1H), 7.80 (d, *J=* 8.2 Hz, 1H), 7.77 (s, 1H), 7.23 (d, *J=* 8.6 Hz, 2H), 7.13 (s, 1H), 6.97 (d, *J=* 8.7 Hz, 2H), 5.96 (d, *J =* 8.5 Hz, 1H), 5.32 - 5.25 (m, 1H), 5.23 (s, 2H), 4.88 (d, *J= 20.4* Hz, 1H), 4.74 (d, *J =* 20.5 Hz, 1H), 4.48 (s, 2H), 3.90 (dd, *J =* 12.9, 4.4 Hz, 1H), 3.81 (d, *J =* 8.3 Hz, 2H), 3.42 - 3.34 (m, 3H), 3.16 (d, *J =* 8.3 Hz, 4H), 3.03 - 2.88 (m, 1H), 2.69 - 2.55 (m, 2H), 2.16 - 2.05 (m, 1H), 1.42 (d, *J* = 6.5 Hz, 3H). LCMS (ESI) calcd. for C₄₀H₄₁N₁₀O₄S ⁺ [M+H]⁺: 757.30, found, 757.2.

### Example 82: Preparation of (9R)-N-(1-(4-(4-(4-((2-(2,6-dioxopiperidin-3-yl)-1-thioxoisoindolin-5-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide (GT-08778)

With reference to the method of Scheme T-1, the target compound (GT-08778) was obtained (white solid, 16 mg, yield 22 %). ¹H NMR (400 MHz, DMSO) δ 11.91 (s, 1H), 11.14 (s, 1H), 10.64 (s, 1H), 8.36 (d, *J =* 6.2 Hz, 1H), 8.30 (d, *J =* 8.2 Hz, 1H), 8.18 (s, 1H), 7.96 (s, 1H), 7.95 - 7.88 (m, 2H), 7.81 (d, *J =* 8.1 Hz, 1H), 7.77 (s, 1H), 7.23 (d, *J =* 8.4 Hz, 2H), 7.17 - 7.01 (m, 3H), 5.96 (d, *J =* 8.4 Hz, 1H), 5.32 - 5.27 (m, 1H), 5.24 (s, 2H), 4.87 (d, *J =* 20.6 Hz, 1H), 4.72 (d, *J =* 20.3 Hz, 1H), 4.51 (s, 2H), 3.91 (dd, *J =* 12.8, 4.2 Hz, 2H), 3.84 *(d, J=* 11.5 Hz, 2H), 3.51 - 3.44 (m, 6H), 3.28 - 3.17 (m, 1H), 3.01 - 2.93 (m, 1H), 2.83 (s, 2H), 2.68 - 2.53 (m, 3H), 2.18 (d, *J =* 10.1 Hz, 2H), 2.13 - 2.05 (m, 1H), 1.92 - 1.74 (m, 2H), 1.42 (d, *J =* 6.5 Hz, 3H), 1.35 - 1.28 (m, 1H). LCMS (ESI) calcd. for C₄₅H₅₀N₁₁O₄S ⁺ [M+H]⁺: 840.38, found, 840.2.

### Example 83: Preparation of 5-chloro-4-(4-chloro-1-methyl-1H-pyrazol-5-yl)-N-((2S)-1-(3,4-difluorophenyl)-3-(((2-(2,6-dioxopiperidin-3-yl)-1-thioxoisoindolin-5-yl)methyl)amino)propan-2-yl)furan-2-carboxamide (GT-08895)

With reference to the method of Scheme T-1, the target compound (GT-08895) was obtained (white solid, 3 mg, yield 6 %). ¹H NMR (400 MHz, DMSO) δ 11.14 (s, 1H), 9.27 - 9.20 (m, 1H), 8.76 (d, *J =* 8.9 Hz, 1H), 7.96 (d, *J =* 8.0 Hz, 1H), 7.83 (s, 1H), 7.74 - 7.67 (m, 2H), 7.56 (s, 1H), 7.39 - 7.31 (m, 2H), 7.09 (s, 1H), 5.94 (d, 1H), 4.91 - 4.68 (m, 2H), 4.54 (s, 1H), 4.34 (d, *J =* 19.3 Hz, 2H), 3.75 (d, *J =* 6.4 Hz, 3H), 3.25 - 3.22 (m, 1H), 3.16 (d, *J =* 6.0 Hz, 2H), 2.98 - 2.87 (m, 3H), 2.65 (d, *J =* 16.3 Hz, 2H), 2.12 - 2.04 (m, 1H). LCMS (ESI) calcd. for C₃₂H₂₉Cl₂F₂N₆O₄S ⁺ [M+H]⁺: 701.13, found, 701.2.

### Example 84: Preparation of 5-chloro-4-(4-chloro-1-methyl-1H-pyrazol-5-yl)-N-((2S)-1-(3,4-difluorophenyl)-3-(4-((2-(2,6-dioxopiperidin-3-yl)-1-thioxoisoindolin-5-yl)methyl)piperazin-1-yl)propan-2-yl)furan-2-carboxamide (GT-08896)

With reference to the method of Scheme T-1, the target compound (GT-08896) was obtained (white solid, 15 mg, yield 23 %). ¹H NMR (400 MHz, DMSO) δ 11.14 (s, 1H), 8.83 (s, 1H), 7.94 (d, *J =* 7.9 Hz, 1H), 7.88 (s, 1H), 7.74 (d, *J =* 16.8 Hz, 1H), 7.71 (s, 1H), 7.59 (s, 1H), 7.41 - 7.28 (m, 2H), 7.12 (s, 1H), 5.96 (d, *J =* 9.1 Hz, 1H), 4.86 (d, *J =* 20.4 Hz, 1H), 4.72 (d, *J =* 20.4 Hz, 1H), 4.54 (s, 1H), 4.39 (s, 2H), 3.76 (s, 3H), 3.49 (s, 6H), 3.32 - 3.14 (m, 4H), 3.04 - 2.92 (m, 3H), 2.91 - 2.84 (m, 1H), 2.68 - 2.55 (m, 2H), 2.12 - 2.05 (m, 1H). LCMS (ESI) calcd. for C₃₆H₃₆Cl₂F₂N₇O₄S ⁺ [M+H]⁺: 770.19, found, 770.1.

### Example 85: Preparation of 5-chloro-4-(4-chloro-1-methyl-1H-pyrazol-5-yl)-N-((2S)-1-(4-((2-(2,6-dioxopiperidin-3-yl)-1-thioxoisoindolin-5-yl)methyl)piperazin-1-yl)-3-(3-fluorophenyl)propan-2-yl)thiophene-2-carboxamide (GT-08897)

With reference to the method of Scheme T-1, the target compound (GT-08897) was obtained (white solid, 7 mg, yield 11 %). ¹H NMR (400 MHz, DMSO) δ 11.13 (s, 1H), 8.94 (s, 1H), 8.15 (s, 2H), 7.93 (d, *J* = 7.9 Hz, 1H), 7.83 (s, 1H), 7.71 (s, 1H), 7.28 (dd, *J =* 14.2, 7.8 Hz, 1H), 7.14 (t, *J =* 9.7 Hz, 2H), 7.06 - 6.91 (m, 1H), 5.95 (d, *J= 7.9* Hz, 1H), 4.85 (d, *J =* 20.4 Hz, 1H), 4.71 (d, *J =* 20.6 Hz, 1H), 4.50 (s, 1H), 4.44 - 4.21 (m, 1H), 3.85 (d, *J =* 5.8 Hz, 3H), 3.44 (s, 4H), 3.38 - 3.29 (m, 6H), 3.29 - 3.06 (m, 4H), 3.04 - 2.87 (m, 4H), 2.70 - 2.56 (m, 2H), 2.13 - 2.02 (m, 1H). LCMS (ESI) calcd. for C₃₆H₃₇Cl₂FN₇O₃S₂ ⁺ [M+H]⁺: 768.18 , found, 768.2.

### Example 86: Preparation of 6-(6-(4-((2-(2,6-dioxopiperidin-3-yl)-1-thioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide (GT-08885)

With reference to the method of Scheme T-1, the target compound (GT-08885) was obtained (white solid, 24 mg, yield 36 %). ¹H NMR (400 MHz, DMSO) δ 11.50 (d, *J =* 57.8 Hz, 2H), 11.15 (s, 1H), 8.69 (d, *J =* 2.3 Hz, 1H), 8.62 (t, *J =* 4.9 Hz, 1H), 8.36 (s, 1H), 8.20 (d, *J =* 7.0 Hz, 1H), 8.11 (s, 1H), 8.02 - 7.92 (m, 2H), 7.85 - 7.78 (m, 2H), 7.13 (d, *J =* 8.9 Hz, 1H), 5.97 (d, *J=* 10.9 Hz, 1H), 5.93 (s, 1H), 5.21 - 5.08 (m, 1H), 4.89 (d, *J =* 20.5 Hz, 1H), 4.75 (d, *J =* 20.6 Hz, 1H), 4.59 - 4.36 (m, 6H), 3.46 - 3.40 (m, 4H), 3.16 (s, 2H), 3.03 - 2.93 (m, 1H), 2.65 (d, *J =* 15.7 Hz, 1H), 2.54 (d, *J =* 7.1 Hz, 2H), 2.17 - 2.08 (m, 4H), 1.54 (d, *J* = 7.7 Hz, 1H), 1.50 (d, *J* = 6.6 Hz, 6H), 1.31 - 1.19 (m, 1H), 0.88 (t, *J* = 7.3 Hz, 3H). LCMS (ESI) calcd. for C₄₄H₅₀N₉O₄S ⁺ [M+H]⁺: 800.37, found, 800.2.

### Example 87: Preparation of N-((4,6-dimethyl-2-oxo-1,2-dihydropyridin-3-yl)methyl)-4'-((4-((2-(2,6-dioxopiperidin-3-yl)-1-thioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide (GT-08886)

With reference to the method of Scheme T-1, the target compound (GT-08886) was obtained (white solid, 15 mg, yield 21 %). ¹H NMR (400 MHz, DMSO) δ 11.53 (s, 1H), 11.14 (s, 1H), 8.40 (s, 1H), 7.94 (d, *J =* 7.8 Hz, 2H), 7.89 - 7.71 (m, 5H), 7.71 - 7.18 (m, 2H), 5.95 (d, *J =* 8.9 Hz, 1H), 5.89 (s, 1H), 4.86 (d, *J =* 20.4 Hz, 1H), 4.71 (d, *J =* 20.2 Hz, 1H), 4.42 (s, 3H), 4.30 (d, *J =* 4.7 Hz, 2H), 3.86 (s, 2H), 3.55 - 3.54 (m, 2H), 3.45 - 3.42 (m, 6H), 3.31 - 3.20 (m, 4H), 3.15 - 3.09 (m, 1H), 3.00 - 2.93 (m, 1H), 2.65 (d, *J* = 17.7 Hz, 1H), 2.43 - 2.34 (m, 2H), 2.23 (s, 3H), 2.12 (s, 3H), 2.10 - 2.05 (m, 1H), 1.64 (s, 2H), 1.37 - 1.31 (m, 1H), 1.28 (dd, *J* = 12.6, 6.7 Hz, 3H), 0.89 (s, 3H). LCMS (ESI) calcd. for C₄₈H₅₈N₇O₅S ⁺ [M+H]⁺: 844.42, found, 844.2.

### Example 88: Preparation of N-((4,6-dimethyl-2-oxo-1,2-dihydropyridin-3-yl)methyl)-4'-(4-((2-(2,6-dioxopiperidin-3-yl)-1-thioxoisoindolin-5-yl)methyl)piperazin-1-yl)-5-(ethyl(tetrahydro-2H-pyran-4-yl)amino)-4-methyl-[1,1'-biphenyl]-3-carboxamide (GT-08887)

With reference to the method of Scheme T-1, the target compound (GT-08887) was obtained (white solid, 15 mg, yield 22 %). ¹H NMR (400 MHz, DMSO) δ 11.54 (s, 1H), 11.13 (s, 1H), 10.37 (s, 1H), 8.55 - 8.26 (m, 1H), 8.08 - 7.95 (m, 2H), 7.84 (d, *J =* 7.9 Hz, 1H), 7.69 (s, 2H), 7.62 - 7.31 (m, 2H), 7.07 (d, *J =* 8.4 Hz, 2H), 5.95 (s, 1H), 5.89 (s, 1H), 4.89 (d, *J =* 20.5 Hz, 1H), 4.75 (d, *J =* 20.5 Hz, 1H), 4.54 (s, 1H), 4.30 (d, *J =* 4.5 Hz, 2H), 3.95 - 3.78 (m, 4H), 3.58 - 3.56 (m, 2H), 3.47 - 3.39 (m, 4H), 3.24 (d, *J =* 7.9 Hz, 6H), 3.02 - 2.94 (m, 1H), 2.70 - 2.57 (m, 2H), 2.47 - 2.31 (m, 3H), 2.22 (s, 3H), 2.12 (s, 3H), 1.60 (s, 2H), 1.35 - 1.26 (m, 2H), 0.90 (s, 3H). LCMS (ESI) calcd. for C₄₇H₅₆N₇O₅S ⁺ [M+H]⁺: 830.41, found, 830.3.

### Example 89: Preparation of 4-(((4-(5-chloro-2-((1-((2-(2,6-dioxopiperidin-3-yl)-1-thioxoisoindolin-5-yl)methyl)piperidin-4-yl)amino)pyridin-4-yl)thiazol-2-yl)amino)methyl)tetrahydro-2H-pyran-4-carbonitrile (GT-08681)

With reference to the method of Scheme T-1, the target compound (GT-08681) was obtained (white solid, 7 mg, yield 12 %). ¹H NMR (400 MHz, DMSO) δ 11.23 (s, 1H), 11.14 (s, 1H), 8.30 (s, 1H), 8.03 (s, 1H), 7.96 (d, *J =* 7.8 Hz, 2H), 7.82 (d, *J =* 7.9 Hz, 1H), 7.57 (d, *J =* 21.6 Hz, 1H), 7.41 (s, 1H), 5.96 (d, *J* = 9.2 Hz, 1H), 4.81 (dd, *J =* 57.6, 20.3 Hz, 2H), 4.45 (d, *J =* 4.2 Hz, 2H), 3.95 - 3.90 (m, 3H), 3.69 (d, *J =* 6.0 Hz, 3H), 3.50 - 3.40 (m, 4H), 3.08 - 2.96 (m, 2H), 2.65 (d, *J =* 17.9 Hz, 1H), 2.21 - 2.05 (m, 3H), 2.03 - 1.84 (m, 4H), 1.76 - 1.64 (m, 2H), 1.44 - 1.14 (m, 1H). LCMS (ESI) calcd. for C₃₄H₃₈ClN₈O₃S₂ ⁺ [M+H]⁺: 705.22, found, 705.1.

### Example 90: Preparation of 3-(5-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione (GT-08682)

With reference to the method of Scheme T-1, the target compound (GT-08682) was obtained (white solid, 5 mg, yield 10 %). ¹H NMR (400 MHz, DMSO) δ 11.14 (s, 1H), 10.74 (s, 1H), 8.34 (d, *J =* 16.2 Hz, 1H), 8.02 - 7.87 (m, 3H), 7.78 (d, *J =* 8.0 Hz, 1H), 7.57 (s, 1H), 5.95 (s, 1H), 4.81 (dd, *J =* 54.7, 20.4 Hz, 2H), 4.41 (s, 2H), 3.85 (s, 3H), 3.47 - 3.38 (m, 2H), 3.25 (s, 1H), 3.04 - 2.92 (m, 4H), 2.65 (d, *J* = 17.2 Hz, 1H), 2.15 - 2.03 (m, 3H), 1.97 - 1.77 (m, 2H), 0.92 (s, 1H), 0.40 - 0.29 (m, 2H), 0.17 - 0.08 (m, 1H). LCMS (ESI) calcd. for C₃₁H₃₆ClN₈O₂S ⁺ [M+H]⁺: 619.24, found, 619.1.

### Example 91: Preparation of 4-(((5'-chloro-2'-((1-((2-(2,6-dioxopiperidin-3-yl)-1-thioxoisoindolin-5-yl)methyl)piperidin-4-yl)amino)-[2,4'-bipyridinl-6-yl)amino)methyl)tetrahydro-2H-pyran-4-carbonitrile (GT-08683)

With reference to the method of Scheme T-1, the target compound (GT-08683) was obtained (white solid, 10 mg, yield 17 %). ¹H NMR (400 MHz, DMSO) δ 11.25 (s, 1H), 11.14 (s, 1H), 8.12 (d, *J =* 17.2 Hz, 1H), 7.96 (d, *J =* 8.0 Hz, 2H), 7.82 (d, *J* = 7.6 Hz, 1H), 7.65 (s, 2H), 7.03 - 6.65 (m, 3H), 5.96 (s, 1H), 4.81 (dd, *J =* 57.5, 20.5 Hz, 2H), 4.45 (s, 2H), 3.90 (d, *J =* 8.8 Hz, 4H), 3.70 (s, 3H), 3.49 - 3.40 (m, 4H), 3.13 - 2.91 (m, 3H), 2.65 (d, *J =* 18.0 Hz, 1H), 2.19 - 2.06 (m, 3H), 1.96 - 1.84 (m, 3H), 1.73 - 1.62 (m, 2H). LCMS (ESI) calcd. for C₃₆H₄₀ClN₈O₃S ⁺ [M+H]⁺: 699.26, found, 699.1.

### Example 92: Preparation of N-(5-cyclobutyl-1H-pyrazol-3-yl)-2-(4-(4-((2-(2,6-dioxopiperidin-3-yl)-1-thioxoisoindolin-5-yl)methyl)piperazin-1-yl)phenyl)acetamide (GT-08684)

With reference to the method of Scheme T-1, the target compound (GT-08684) was obtained (white solid, 12 mg, yield 23 %). ¹H NMR (400 MHz, DMSO) δ 11.25 (s, 1H), 11.14 (s, 1H), 10.56 (s, 1H), 7.99 - 7.91 (m, 2H), 7.81 (d, *J =* 8.0 Hz, 1H), 7.21 (d, *J =* 8.5 Hz, 2H), 6.92 (d, *J =* 8.6 Hz, 2H), 6.28 (s, 1H), 5.96 (d, *J =* 7.7 Hz, 1H), 4.91 - 4.70 (m, 2H), 4.51 (s, 2H), 3.77 - 3.75 (m, 2H), 3.52 - 3.46 (m, 3H), 3.44 - 3.36 (m, 3H), 3.21 - 3.12 (m, 4H), 3.03 - 2.94 (m, 1H), 2.65 (d, *J=* 18.0 Hz, 1H), 2.28 - 2.21 (m, 2H), 2.12 - 2.05 (m, 3H), 1.99 - 1.89 (m, 1H), 1.86 - 1.79 (m, 1H). LCMS (ESI) calcd. for C₃₃H₃₈N₇O₃S ⁺ [M+H]⁺: 612.28, found, 612.2.

### Example 93: Preparation of 4-(((4-(5-chloro-2-((1-((2-(2,6-dioxopiperidin-3-yl)-1-thioxoisoindolin-4-yl)methyl)piperidin-4-yl)amino)pyridin-4-yl)thiazol-2-yl)amino)methyl)tetrahydro-2H-pyran-4-carbonitrile (GT-08685)

With reference to the method of Scheme T-1, the target compound (GT-08685) was obtained (white solid, 13 mg, yield 17 %). ¹H NMR (400 MHz, DMSO) δ 11.32 (s, 1H), 11.20 (s, 1H), 8.33 (s, 1H), 8.06 - 7.94 (m, 3H), 7.67 (t, *J =* 7.6 Hz, 1H), 7.64 - 7.59 (m, 1H), 7.45 (s, 1H), 6.01 (d, *J =* 11.3 Hz, 1H), 5.44 (d, *J =* 20.4 Hz, 1H), 4.92 (d, *J =* 20.4 Hz, 1H), 4.48 - 4.38 (m, 2H), 3.94 - 3.89 (m, 5H), 3.69 (d, *J* = 5.9 Hz, 2H), 3.51 - 3.39 (m, 4H), 3.19 - 3.15 (m, 1H), 2.98 (d, *J =* 12.2 Hz, 1H), 2.70 (d, *J =* 17.7 Hz, 1H), 2.15 - 2.01 (m, 4H), 1.87 (d, *J =* 13.1 Hz, 2H), 1.75 - 1.64 (m, 2H), 1.33 - 1.15 (m, 1H). LCMS (ESI) calcd. for C₃₄H₃₈ClN₈O₃S₂ ⁺ [M+H]⁺: 705.22, found, 705.1.

### Example 94: Preparation of 3-(4-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione (GT-08686)

With reference to the method of Scheme T-1, the target compound (GT-08686) was obtained (white solid, 16 mg, yield 23 %). ¹H NMR (400 MHz, DMSO) δ 11.21 (s, 1H), 11.06 (s, 1H), 8.35 (d, *J =* 19.8 Hz, 1H), 7.98 (d, *J =* 12.0 Hz, 3H), 7.69 - 7.62 (m, 1H), 7.58 (s, 1H), 6.02 (d, *J =* 9.0 Hz, 1H), 5.39 - 5.24 (m, 1H), 4.91 (d, *J =* 20.2 Hz, 1H), 4.48 - 4.32 (m, 2H), 3.86 (s, 5H), 3.50 - 3.30 (m, 3H), 3.07 - 2.98 (m, 3H), 2.70 (d, *J =* 18.6 Hz, 1H), 2.19 - 2.03 (m, 3H), 2.00 - 1.88 (m, 2H), 1.34 - 1.19 (m, 1H), 0.93 (d, *J* = 6.4 Hz, 1H), 0.42 - 0.31 (m, 2H), 0.14 - 0.04 (m, 2H). LCMS (ESI) calcd. for C₃₁H₃₆ClN₈O₂S ⁺ [M+H]⁺: 619.24, found, 619.1.

### Example 95: Preparation of 4-(((5'-chloro-2'-((1-((2-(2,6-dioxopiperidin-3-yl)-1-thioxoisoindolin-4-yl)methyl)piperidin-4-yl)amino)-[2,4'-bipyridin]-6-yl)amino)methyl)tetrahydro-2H-pyran-4-carbonitrile (GT-08687)

With reference to the method of Scheme T-1, the target compound GT- was obtained (GT-08687) (white solid, 3 mg, yield 4 %). ¹H NMR (400 MHz, DMSO) δ 11.20 (s, 1H), 11.12 (s, 1H), 8.10 (s, 1H), 8.00 (d, *J =* 7.5 Hz, 2H), 7.82 - 7.39 (m, 4H), 6.84 (d, *J=* 7.4 Hz, 2H), 6.00 (s, 1H), 5.37 (d, *J* = 20.1 Hz, 1H), 4.91 (d, *J =* 21.0 Hz, 1H), 4.46 - 4.34 (m, 2H), 3.96 - 3.87 (m, 4H), 3.73 - 3.68 (m, 6H), 3.22 - 3.14 (m, 2H), 3.04 - 2.98 (m, 1H), 2.70 (d, *J =* 17.4 Hz, 2H), 2.13 (d, *J =* 10.8 Hz, 2H), 2.03 - 1.92 (m, 2H), 1.85 (d, *J* = 13.2 Hz, 2H), 1.72 - 1.62 (m, 2H). LCMS (ESI) calcd. for C₃₆H₄₀ClN₈O₃S ⁺ [M+H]⁺: 699.26, found, 699.1.

### Example 96: Preparation of N-(5-cyclobutyl-1H-pyrazol-3-yl)-2-(4-(4-((2-(2,6-dioxopiperidin-3-yl)-1-thioxoisoindolin-4-yl)methyl)piperazin-1-yl)phenyl)acetamide (GT-08688)

With reference to the method of Scheme T-1, the target compound (GT-08688) was obtained (white solid, 15 mg, yield 22 %). ¹H NMR (400 MHz, DMSO) δ 11.27 (s, 1H), 11.17 (s, 1H), 10.56 (s, 1H), 8.01 (d, *J* = 7.5, 4.8 Hz, 2H), 7.68 (t, *J =* 7.6 Hz, 1H), 7.21 (d, *J =* 8.5 Hz, 2H), 6.93 (d, *J =* 8.6 Hz, 2H), 6.28 (s, 1H), 6.00 (s, 1H), 5.35 (d, *J =* 20.4 Hz, 1H), 4.91 (d, *J =* 20.5 Hz, 1H), 4.52 - 4.40 (m, 2H), 3.77 (d, *J =* 10.8 Hz, 3H), 3.49 (s, 3H), 3.45 - 3.40 (m, 2H), 3.29 - 3.16 (m, 4H), 3.01 - 2.94 (m, 1H), 2.69 (d, *J =* 16.5 Hz, 1H), 2.28 - 2.20 (m, 2H), 2.13 - 2.04 (m, 3H), 1.97 - 1.90 (m, 1H), 1.83 - 1.76 (m, 1H). LCMS (ESI) calcd. for C₃₃H₃₈N₇O₃S ⁺ [M+H]⁺: 612.28, found, 612.2.

### Example 97: Preparation of 4-(((4-(5-chloro-2-((1-((2-(2,6-dioxopiperidin-3-yl)-3-thioxoisoindolin-5-yl)methyl)piperidin-4-yl)amino)pyridin-4-yl)thiazol-2-yl)amino)methyl)tetrahydro-2H-pyran-4-carbonitrile (GT-08750)

With reference to the method of Scheme T-1, the target compound (GT-08750) was obtained (white solid, 9 mg, yield 12 %). ¹H NMR (400 MHz, DMSO) δ 11.14 (s, 1H), 10.90 (s, 1H), 8.33 - 8.24 (m, 1H), 8.14 (s, 1H), 8.06 (d, *J =* 13.9 Hz, 1H), 7.97 (d, *J =* 8.8 Hz, 1H), 7.77 (d, *J =* 7.9 Hz, 1H), 7.59 (s, 1H), 7.41 (s, 1H), 5.97 (s, 1H), 4.89 (d, *J =* 20.6 Hz, 1H), 4.74 (d, *J =* 20.8 Hz, 1H), 4.49 (s, 2H), 3.92 (d, *J* = 11.4 Hz, 3H), 3.70 - 3.67 (m, 2H), 3.50 - 3.41 (m, 4H), 3.08 - 2.97 (m, 2H), 2.70 - 2.57 (m, 2H), 2.21 - 2.08 (m, 3H), 1.87 (d, *J =* 12.4 Hz, 4H), 1.74 - 1.65 (m, 2H), 1.33 (s, 1H). LCMS (ESI) calcd. for C₃₄H₃₈ClN₈O₃S₂ ⁺ [M+H]⁺: 705.22, found, 705.1.

### Example 98: Preparation of 3-(6-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione (GT-08751)

With reference to the method of Scheme T-1, the target compound (GT-08751) was obtained (white solid, 14 mg, yield 20 %). ¹H NMR (400 MHz, DMSO) δ 11.14 (s, 1H), 10.67 (d, *J =* 66.0 Hz, 1H), 8.34 (d, *J =* 15.7 Hz, 1H), 8.12 (s, 1H), 8.01 - 7.90 (m, 2H), 7.77 (d, *J =* 7.9 Hz, 1H), 7.63 - 7.50 (m, 1H), 5.96 (d, *J =* 8.8 Hz, 1H), 4.89 (d, *J =* 20.5 Hz, 1H), 4.74 (d, *J =* 20.4 Hz, 1H), 4.47 (d, *J =* 3.8 Hz, 2H), 3.86 (s, 3H), 3.48 - 3.36 (m, 2H), 3.24 (s, 1H), 3.06 - 2.96 (m, 4H), 2.69 - 2.57 (m, 2H), 2.15 - 1.96 (m, 4H), 1.89 - 1.81 (m, 1H), 1.40 - 1.09 (m, 1H), 0.92 (s, 1H), 0.41 - 0.30 (m, 2H), 0.09 (s, 2H). LCMS (ESI) calcd. for C₃₁H₃₆ClN₈O₂S ⁺ [M+H]⁺: 619.24, found, 619.1.

### Example 99: Preparation of 4-(((5'-chloro-2'-((1-((2-(2,6-dioxopiperidin-3-yl)-3-thioxoisoindolin-5-yl)methyl)piperidin-4-yl)amino)-[2,4'-bipyridinl-6-yl)amino)methyl)tetrahydro-2H-pyran-4-carbonitrile (GT-08752)

With reference to the method of Scheme T-1, the target compound (GT-08752) was obtained (white solid, 10 mg, yield 13 %). ¹H NMR (400 MHz, DMSO) δ 11.14 (s, 1H), 10.85 (d, *J =* 69.6 Hz, 1H), 8.12 (d, J = 10.7 Hz, 2H), 7.99 (d, *J =* 8.5 Hz, 1H), 7.77 (d, *J =* 7.8 Hz, 1H), 7.66 (s, 2H), 6.96 - 6.85 (m, 3H), 5.96 (d, *J =* 9.5 Hz, 1H), 4.88 (d, *J=* 20.5 Hz, 1H), 4.74 (d, *J =* 20.5 Hz, 1H), 4.48 (d, *J =* 4.1 Hz, 2H), 3.89 (d, 4H), 3.70 (s, 2H), 3.48 - 3.41 (m, 4H), 3.09 - 2.97 (m, 2H), 2.65 (d, *J =* 17.1 Hz, 1H), 2.20 - 2.09 (m, 3H), 1.93 - 1.83 (m, 4H), 1.73 - 1.65 (m, 2H), 1.38 - 1.21 (m, 1H). LCMS (ESI) calcd. for C₃₆H₄₀ClN₈O₃S ⁺ [M+H]⁺: 699.26, found, 699.2.

### Example 100: Preparation of N-(5-cyclobutyl-1H-pyrazol-3-yl)-2-(4-(4-((2-(2,6-dioxopiperidin-3-yl)-3-thioxoisoindolin-5-yl)methyl)piperazin-1-yl)phenyl)acetamide (GT-08753)

With reference to the method of Scheme T-1, the target compound (GT-08753) was obtained (white solid, 15 mg, yield 22 %). ¹H NMR (400 MHz, DMSO) δ 11.14 (s, 1H), 10.98 (s, 1H), 10.63 (s, 1H), 8.15 (s, 1H), 7.97 (d, *J =* 8.1 Hz, 1H), 7.78 (d, *J =* 7.8 Hz, 1H), 7.21 (d, *J =* 8.6 Hz, 2H), 6.92 (d, *J =* 8.7 Hz, 2H), 6.28 (s, 1H), 5.96 (d, *J =* 9.7 Hz, 1H), 4.89 (d, *J =* 20.6 Hz, 1H), 4.72 (d, 1H), 4.55 (s, 2H), 3.80 (s, 3H), 3.50 (s, 2H), 3.44 (t, *J =* 7.1 Hz, 1H), 3.37 (d, *J =* 10.6 Hz, 2H), 3.19 (d, *J =* 8.5 Hz, 2H), 3.11 - 3.07 (m, 1H), 3.01 - 2.92 (m, 1H), 2.65 (d, *J =* 16.6 Hz, 1H), 2.29 - 2.20 (m, 2H), 2.13 - 2.02 (m, 3H), 1.98 - 1.89 (m, 1H), 1.86 - 1.77 (m, 1H), 1.38 - 1.20 (m, 1H). LCMS (ESI) calcd. for C₃₃H₃₈N₇O₃S ⁺ [M+H]⁺: 612.28, found, 612.2.

### Example 101: Preparation of 2-(6,7-dihydro-5H-pyrrolo[1,2-c]imidazol-1-yl)-2-(6-(4-(4-((2-(2,6-dioxopiperidin-3-yl)-1-thioxoisoindolin-5-yl)methyl)piperazin-1-yl)phenyl)-4-fluoro-1-oxoisoindolin-2-yl)-N-(thiazol-2-yl)acetamide (GT-09423)

With reference to the method of Scheme T-1, the target compound (GT-09423) was obtained (white solid, 12 mg, yield 17 %). ¹H NMR (400 MHz, DMSO) δ 11.69 (s, 1H), 11.15 (s, 1H), 9.08 (s, 1H), 8.08 - 7.92 (m, 2H), 7.87 - 7.78 (m, 3H), 7.73 (d, *J =* 8.8 Hz, 2H), 7.53 (d, *J =* 3.6 Hz, 1H), 7.33 (d, *J =* 3.6 Hz, 1H), 7.09 (d, *J =* 8.9 Hz, 2H), 6.40 (s, 1H), 5.97 (d, *J =* 8.8 Hz, 1H), 4.94 - 4.69 (m, 3H), 4.58 - 4.45 (m, 3H), 4.26 (t, *J =* 7.0 Hz, 2H), 3.92 (d, *J =* 12.0 Hz, 2H), 3.41 - 3.39 (m, 2H), 3.34 - 3.26 (m, 3H), 3.21 (s, 2H), 3.03 - 2.94 (m, 1H), 2.91 - 2.84 (m, 1H), 2.69 - 2.61 (m, 2H), 2.61 - 2.54 (m, 3H), 2.18 - 2.06 (m, 1H). LCMS (ESI) calcd. for C₄₃H₄₁FN₉O₄S₂ ⁺ [M+H]⁺: 830.27, found, 830.3.

### Example 102: Preparation of 8-(4-((2-(2,6-dioxopiperidin-3-yl)-1-thioxoisoindolin-5-yl)methyl)piperazin-1-yl)-9-ethyl-6,6-dimethyl-11-oxo-6,11-dihydro-5H-benzo[b]carbazole-3-carbonitrile (GT-09424)

With reference to the method of Scheme T-1, the target compound (GT-09424) was obtained (white solid, 11 mg, yield 19 %). ¹H NMR (400 MHz, DMSO) δ 12.85 (d, *J =* 32.2 Hz, 1H), 11.15 (s, 2H), 8.31 (d, *J =* 8.2 Hz, 1H), 8.09 (s, 1H), 8.02 - 7.98 (m, 2H), 7.86 (d, *J =* 8.0 Hz, 1H), 7.68 - 7.53 (m, 1H), 7.37 (s, 1H), 5.97 (d, *J* = 9.0 Hz, 1H), 4.92 - 4.74 (m, 1H), 4.60 (s, 2H), 3.43 (s, 2H), 3.33 - 3.21 (m, 6H), 3.04 - 2.91 (m, 1H), 2.85 - 2.56 (m, 5H), 2.17 - 2.06 (m, 1H), 1.77 (s, 6H), 1.29 (t, *J =* 7.3 Hz, 3H). LCMS (ESI) calcd. for C₃₉H₃₉N₆O₃S ⁺ [M+H]⁺: 671.28, found, 671.3.

### Example 103: Preparation of 3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-1-thioxoisoindolin-2-yl)piperidine-2,6-dione (GT-09425)

With reference to the method of Scheme T-1, the target compound (GT-09425) was obtained (white solid, 20 mg, yield 27 %). ¹H NMR (400 MHz, DMSO) δ 12.02 (d, 2H), 11.09 (s, 1H), 9.48 (s, 1H), 8.51 - 8.19 (m, 2H), 8.00 - 7.90 (m, 1H), 7.85 (d, *J =* 7.8 Hz, 1H), 7.68 - 7.61 (m, 1H), 7.47 (s, 2H), 7.27 (t, *J =* 7.0 Hz, 1H), 6.90 (s, 1H), 6.74 (s, 1H), 5.94 (d, 1H), 4.87 - 4.61 (m, 2H), 4.53 (s, 2H), 3.90 (d, 3H), 3.81 (s, 5H), 3.51 - 3.47 (m, 3H), 3.25 (s, 2H), 2.94 (s, 4H), 2.69 - 2.57 (m, 2H), 2.24 (s, 2H), 2.10 (s, 1H), 1.97 (s, 2H), 1.80 (d, *J* = 13.6 Hz, 6H). LCMS (ESI) calcd. for C₄₂H₅₀ClN₉O₄PS ⁺ [M+H]⁺: 842.31, found, 842.3.

### Example 104: Preparation of 2-(6,7-dihydro-5H-pyrrolo[1,2-c]imidazol-1-yl)-2-(6-(4-(4-((2-(2,6-dioxopiperidin-3-yl)-3-thioxoisoindolin-5-yl)methyl)piperazin-1-yl)phenyl)-4-fluoro-1-oxoisoindolin-2-yl)-N-(thiazol-2-yl)acetamide (GT-09426)

With reference to the method of Scheme T-1, the target compound (GT-09426) was obtained (white solid, 22 mg, yield 32 %). ¹H NMR (400 MHz, DMSO) δ 11.37 (s, 1H), 11.14 (s, 1H), 9.06 (d, *J =* 16.9 Hz, 1H), 8.16 (s, 1H), 8.04 (d, *J =* 8.1 Hz, 1H), 7.84 - 7.77 (m, 3H), 7.73 (d, *J =* 8.7 Hz, 2H), 7.53 (d, *J =* 3.6 Hz, 1H), 7.33 (d, *J =* 3.6 Hz, 1H), 7.09 (d, *J =* 8.8 Hz, 2H), 6.40 (s, 1H), 5.96 (d, *J =* 8.3 Hz, 1H), 4.97 - 4.72 (m, 3H), 4.60 - 4.48 (m, 3H), 4.26 (t, *J=* 7.1 Hz, 2H), 3.95 (d, *J =* 9.8 Hz, 2H), 3.42 - 3.37 (m, 4H), 3.27 - 3.19 (m, 4H), 2.98 - 2.84 (m, 2H), 2.68 - 2.56 (m, 4H), 2.15 - 2.05 (m, 1H). LCMS (ESI) calcd. for C₄₃H₄₁FN₉O₄S₂ ⁺ [M+H]⁺: 830.27, found, 830.3.

### Example 105: Preparation of 7-cyclopentyl-2-((5-(4-((2-(2,6-dioxopiperidin-3-yl)-3-thioxoisoindolin-5-yl)methyl)piperazin-1-yl)pyridin-2-yl)amino)-N,N-dimethyl-7H-pyrrolo[2,3-d]pyrimidine-6-carboxamide (GT-09427)

With reference to the method of Scheme T-1, the target compound (GT-09427) was obtained (white solid, 22 mg, yield 37 %). ¹H NMR (400 MHz, DMSO) δ 11.51 (s, 1H), 11.13 *(d, J =* 12.0 Hz, 1H), 8.97 (s, 1H), 8.16 (s, 1H), 8.04 (d, *J =* 7.6 Hz, 1H), 7.98 - 7.89 (m, 2H), 7.79 (d, *J =* 7.8 Hz, 1H), 7.72 (d, *J =* 8.4 Hz, 1H), 7.60 (s, 1H), 6.80 (s, 1H), 5.96 (d, *J =* 8.7 Hz, 1H), 4.90 - 4.73 (m, 3H), 4.60 (d, *J =* 18.0 Hz, 3H), 3.81 (s, 2H), 3.26 (s, 4H), 3.06 (s, 6H), 2.98 - 2.92 (m, 1H), 2.71 - 2.55 (m, 3H), 2.33 (s, 2H), 2.13 - 2.08 (m, 1H), 2.04 - 1.93 (m, 4H), 1.70 - 1.61 (m, 2H). LCMS (ESI) calcd. for C₃₇H₄₃N₁₀O₃S ⁺ [M+H]⁺: 707.32, found, 707.3.

### Example 106: Preparation of 3-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione (GT-09428)

With reference to the method of Scheme T-1, the target compound (GT-09428) was obtained (white solid, 22 mg, yield 36 %). ¹H NMR (400 MHz, DMSO) δ 11.37 (s, 1H), 11.15 (s, 1H), 10.85 (s, 1H), 9.00 (s, 1H), 8.16 (s, 1H), 8.08 (d, *J =* 2.4 Hz, 1H), 8.03 (d, *J =* 7.8 Hz, 1H), 7.86 (d, 1H), 7.84 - 7.77 (m, 2H), 5.96 (d, *J =* 9.3 Hz, 1H), 5.86 - 5.73 (m, 1H), 4.83 (dd, *J =* 57.4, 20.5 Hz, 2H), 4.58 (s, 2H), 3.86 (s, 2H), 3.44 (s, 3H), 3.25 (d, *J =* 7.1 Hz, 4H), 3.01 - 2.94 (m, 1H), 2.66 (d, *J =* 16.7 Hz, 1H), 2.44 (s, 3H), 2.34 (s, 3H), 2.27 - 2.20 (m, 2H), 2.13 - 2.07 (m, 1H), 1.93 (s, 2H), 1.82 - 1.74 (m, 2H), 1.62 - 1.55 (m, 2H). LCMS (ESI) calcd. for C₃₈H₄₂N₉O₄S ⁺ [M+H]⁺: 720.31, found, 720.3.

### Example 107: Preparation of 2-(6,7-dihydro-5H-pyrrolo[1,2-c]imidazol-1-yl)-2-(6-(4-(4-((2-(2,6-dioxopiperidin-3-yl)-1-thioxoisoindolin-4-yl)methyl)piperazin-1-yl)phenyl)-4-fluoro-1-oxoisoindolin-2-yl)-N-(thiazol-2-yl)acetamide (GT-09429)

With reference to the method of Scheme T-1, the target compound (GT-09429) was obtained (white solid, 6 mg, yield 9 %). ¹H NMR (400 MHz, DMSO) δ 11.54 (s, 1H), 11.17 (d, *J =* 34.1 Hz, 1H), 9.02 (s, 1H), 8.06 - 7.99 (m, 2H), 7.82 (d, *J* = 7.8 Hz, 2H), 7.75 - 7.68 (m, 3H), 7.53 (d, *J =* 3.6 Hz, 1H), 7.33 (d, *J* = 3.6 Hz, 1H), 7.09 (d, *J =* 8.8 Hz, 2H), 6.38 (s, 1H), 6.01 (d, 1H), 5.42 (d, *J =* 20.8 Hz, 1H), 4.93 (d, *J =* 20.7 Hz, 1H), 4.80 (d, *J =* 17.1 Hz, 1H), 4.50 (d, *J =* 17.2 Hz, 3H), 4.25 (t, *J =* 7.1 Hz, 2H), 3.93 (s, 2H), 3.48 (s, 2H), 3.33 - 3.26 (m, 6H), 3.03 - 2.95 (m, 1H), 2.91 - 2.84 (m, 1H), 2.76 - 2.63 (m, 2H), 2.59 - 2.54 (m, 2H), 2.16 - 2.09 (m, 1H). LCMS (ESI) calcd. for C₄₃H₄₁FN₉O₄S₂ ⁺ [M+H]⁺: 830.27, found, 830.3.

### Example 108: Preparation of 4-(2,6-dichlorobenzamido)-N-(1-((2-(2,6-dioxopiperidin-3-yl)-1-thioxoisoindolin-5-yl)methyl)piperidin-4-yl)-1H-pyrazole-3-carboxamide (GT-09434)

With reference to the method of Scheme T-1, the target compound (GT-09434) was obtained (white solid, 8 mg, yield 15 %). ¹H NMR (400 MHz, DMSO) δ 13.52 (d, *J =* 25.2 Hz, 1H), 11.14 (s, 1H), 10.38 (s, 1H), 10.11 (s, 1H), 8.68 (d, *J =* 7.8 Hz, 1H), 8.38 (d, *J =* 16.0 Hz, 1H), 7.97 (d, *J =* 7.9 Hz, 1H), 7.93 - 7.86 (m, 1H), 7.80 - 7.70 (m, 1H), 7.62 - 7.52 (m, 3H), 5.96 *(d, J=* 9.4 Hz, 1H), 4.81 (dd, *J* = 54.3, 20.5 Hz, 2H), 4.50 - 4.36 (m, 2H), 4.02 - 3.84 (m, 1H), 3.37 (s, 2H), 3.24 (s, 1H), 3.13 - 2.91 (m, 3H), 2.65 (d, *J =* 18.0 Hz, 1H), 2.59 - 2.54 (m, 1H), 2.12 - 2.06 (m, 1H), 1.99 - 1.89 (m, 3H). LCMS (ESI) calcd. for C₃₀H₃₀Cl₂N₇O₄S ⁺ [M+H]⁺: 654.15, found, 654.2.

### Example 109: Preparation of N-(5-(((5-(tert-butyl)oxazol-2-yl)methyl)thio)thiazol-2-yl)-1-((2-(2,6-dioxopiperidin-3-yl)-1-thioxoisoindolin-5-yl)methyl)piperidine-4-carboxamide (GT-09435)

With reference to the method of Scheme T-1, the target compound (GT-09435) was obtained (white solid, 7 mg, yield 13 %). ¹H NMR (400 MHz, DMSO) δ 12.44 (s, 1H), 11.13 (d, *J =* 12.0 Hz, 1H), 10.78 (s, 1H), 7.96 (t, 2H), 7.78 (d, *J =* 8.3 Hz, 1H), 7.40 (d, *J =* 2.8 Hz, 1H), 6.72 (d, *J =* 3.4 Hz, 1H), 5.96 (d, *J= 7.9* Hz, 1H), 4.81 (dd, *J =* 55.9, 20.4 Hz, 2H), 4.51 - 4.37 (m, 2H), 4.06 (s, 2H), 3.41 (d, *J =* 11.6 Hz, 2H), 3.04 - 2.90 (m, 3H), 2.78 - 2.56 (m, 3H), 2.18 - 2.04 (m, 2H), 2.03 - 1.95 (m, 3H), 1.20 - 1.15 (m, 9H). LCMS (ESI) calcd. for C₃₁H₃₇N₆O₄S₃ ⁺ [M+H]⁺: 653.20, found, 653.3.

### Example 110: Preparation of 3-(5-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione (GT-09436)

With reference to the method of Scheme T-1, the target compound (GT-09436) was obtained (white solid, 10 mg, yield 18 %). ¹H NMR (400 MHz, DMSO) δ 11.07 (d, *J =* 57.3 Hz, 2H), 8.46 (s, 1H), 7.98 (d, *J =* 8.3 Hz, 2H), 7.82 (d, *J =* 8.3 Hz, 1H), 7.66 (d, *J =* 8.9 Hz, 2H), 7.44 (t, *J =* 7.9 Hz, 2H), 7.26 - 7.08 (m, 6H), 5.95 (s, 1H), 5.14 - 4.98 (m, 1H), 4.82 (dd, *J =* 55.4, 20.4 Hz, 2H), 4.49 (s, 2H), 3.34 - 3.27 (m, 3H), 2.99 - 2.92 (m, 1H), 2.68 - 2.53 (m, 5H), 2.17 (d, *J =* 11.8 Hz, 2H), 2.12 - 2.07 (m, 1H). LCMS (ESI) calcd. for C₃₆H₃₅N₈O₃S ⁺ [M+H]⁺: 659.25, found, 659.3.

### Example 111: Preparation of N-(3-(((2-((4-(4-((2-(2,6-dioxopiperidin-3-yl)-3-thioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide (GT-09437)

With reference to the method of Scheme T-1, the target compound (GT-09437) was obtained (white solid, 20 mg, yield 18 %). ¹H NMR (400 MHz, DMSO) δ 11.28 (s, 1H), 11.15 (s, 1H), 10.31 (s, 1H), 8.69 (d, *J =* 2.5 Hz, 1H), 8.60 (d, *J =* 2.5 Hz, 1H), 8.38 (s, 1H), 8.16 (s, 1H), 8.04 (d, *J* = 7.8 Hz, 1H), 7.77 (d, *J =* 12.1 Hz, 1H), 7.27 (s, 2H), 6.83 (d, *J =* 7.7 Hz, 2H), 5.96 (d, *J =* 10.2 Hz, 1H), 4.96 (d, *J=* 5.0 Hz, 2H), 4.93 - 4.70 (m, 2H), 4.56 (s, 2H), 3.68 - 3.65 (m, 2H), 3.39 (d, *J =* 10.4 Hz, 2H), 3.24 - 3.19 (m, 2H), 3.18 (s, 3H), 3.16 - 3.08 (m, 5H), 3.01 - 2.91 (m, 1H), 2.71 - 2.55 (m, 2H), 2.14 - 2.05 (m, 1H). LCMS (ESI) calcd. for C₃₆H₃₉F₃N₁₁O₄S₂ ⁺ [M+H]⁺: 810.26, found, 810.3.

### Example 112: Preparation of N-(3-(((2-((4-(4-((2-(2,6-dioxopiperidin-3-yl)-3-thioxoisoindolin-5-yl)methyl)piperazin-1-yl)phenyl)amino)-5-(trifluoromethyl)pyrimidin-4-yl)amino)methyl)phenyl)-N-methylmethanesulfonamide (GT-09438)

With reference to the method of Scheme T-1, the target compound (GT-09438) was obtained (white solid, 27 mg, yield 24 %). ¹H NMR (400 MHz, DMSO) δ 11.14 (s, 2H), 10.28 (s, 1H), 8.57 (s, 1H), 8.33 (s, 1H), 8.16 (s, 1H), 8.01 (d, *J* = 7.9 Hz, 1H), 7.79 (d, *J =* 7.8 Hz, 1H), 7.40 - 7.33 (m, 4H), 7.27 (d, *J= 7.8* Hz, 1H), 7.20 (s, 1H), 6.91 (d, *J =* 8.8 Hz, 2H), 5.96 (d, *J =* 8.4 Hz, 1H), 4.82 (dd, *J =* 57.7, 20.6 Hz, 2H), 4.67 - 4.56 (m, 4H), 3.76 (d, *J =* 11.5 Hz, 4H), 3.39 (d, *J =* 9.9 Hz, 3H), 3.26 - 3.16 (m, 4H), 3.15 (s, 3H), 3.12 - 3.09 (m, 1H), 3.00 - 2.93 (m, 1H), 2.87 (s, 3H), 2.71 - 2.53 (m, 3H), 2.14 - 2.07 (m, 1H). LCMS (ESI) calcd. for C₃₈H₄₁F₃N₉O₄S₂ ⁺ [M+H]⁺: 808.27, found, 808.3.

### Example 113: Preparation of N-(4-((4-((2-(2,6-dioxopiperidin-3-yl)-3-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide (GT-09439)

With reference to the method of Scheme T-1, the target compound (GT-09439) was obtained (white solid, 26 mg, yield 22 %). ¹H NMR (400 MHz, DMSO) δ 11.14 (s, 1H), 10.69 (s, 1H), 8.75 (dd, J = 4.4, 1.5 Hz, 1H), 8.32 - 8.25 (m, 3H), 8.23 (d, *J =* 1.7 Hz, 1H), 8.20 - 8.11 (m, 2H), 8.01 - 7.83 (m, 3H), 7.76 (d, *J= 7.9* Hz, 1H), 7.57 (d, *J =* 8.3 Hz, 1H), 7.43 (dd, *J* = 9.2, 4.5 Hz, 1H), 5.94 (d, 1H), 4.81 (dd, *J =* 58.1, 20.5 Hz, 2H), 4.51 (s, 2H), 3.92 (s, 2H), 3.42 - 3.06 (m, 8H), 3.00 - 2.93 (m, 1H), 2.67 (s, 1H), 2.62 (s, 4H), 2.14 - 2.06 (m, 1H). LCMS (ESI) calcd. for C₄₂H₃₈F₃N₈O₃S ⁺ [M+H]⁺: 791.27, found, 791.3.

### Example 114: Preparation of N-(4-(4-((2-(2,6-dioxopiperidin-3-yl)-3-thioxoisoindolin-5-yl)methyl)piperazin-1-yl)-3-(trifluoromethyl)phenyl)-3-(imidazo[1,2-b]pyridazin-3-ylethynyl)-4-methylbenzamide (GT-09440)

With reference to the method of Scheme T-1, the target compound (GT-09440) was obtained (white solid, 28 mg, yield 25 %). ¹H NMR (400 MHz, DMSO) δ 11.14 (s, 1H), 10.85 (s, 1H), 10.62 (s, 1H), 8.74 (d, *J =* 4.3 Hz, 1H), 8.31 - 8.18 (m, 5H), 8.14 (d, *J=* 8.6 Hz, 1H), 8.02 - 7.94 (m, 2H), 7.79 (d, *J =* 7.8 Hz, 1H), 7.59 - 7.51 (m, 2H), 7.42 (dd, *J=* 9.2, 4.4 Hz, 1H), 5.95 (s, 1H), 4.82 (dd, *J =* 58.0, 20.5 Hz, 2H), 4.62 (s, 2H), 3.42 - 3.39 (m, 2H), 3.27 - 3.15 (m, 4H), 3.07 (d, *J =* 7.9 Hz, 2H), 3.02 - 2.93 (m, 1H), 2.67 (d, *J =* 9.7 Hz, 1H), 2.61 (s, 3H), 2.15 - 2.07 (m, 1H). LCMS (ESI) calcd. for C₄₁H₃₆F₃N₈O₃S ⁺ [M+H]⁺: 777.26, found, 777.3.

### Example 115: Preparation of 3-(6-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione (GT-09441)

With reference to the method of Scheme T-1, the target compound (GT-09441) was obtained (white solid, 37 mg, yield 39 %). ¹H NMR (400 MHz, DMSO) δ 11.14 (s, 1H), 10.81 (s, 1H), 8.49 (s, 1H), 8.15 (s, 1H), 7.97 (d, *J =* 7.8 Hz, 1H), 7.79 (d, *J= 7.8* Hz, 1H), 7.69 - 7.55 (m, 3H), 7.47 - 7.42 (m, 2H), 7.24 - 7.11 (m, 6H), 5.95 (s, 1H), 5.12 - 5.02 (m, 1H), 4.82 (dd, 2H), 4.53 (s, 2H), 3.50 (d, 3H), 3.34 - 3.29 (m, 2H), 2.98 - 2.91 (m, 1H), 2.68 - 2.55 (m, 3H), 2.19 (d, *J=* 12.8 Hz, 2H), 2.13 - 2.08 (m, 1H). LCMS (ESI) calcd. for C₃₆H₃₅N₈O₃S ⁺ [M+H]⁺: 659.25, found, 659.3.

### Example 116: Preparation of 3-(5-((4-(4-(4-acetyl-1,4-diazepan-1-yl)-6-((2-(thiophen-2-yl)ethyl)amino)-1,3,5-triazin-2-yl)piperazin-1-yl)methyl)-1-thioxoisoindolin-2-yl)piperidine-2,6-dione (GT-08867)

With reference to the method of Scheme T-1, the target compound (GT-08867) was obtained (white solid, 12 mg, yield 14.70%). ¹H NMR (400 MHz, DMSO) δ 11.61 (s, 1H), 11.14 (s, 1H), 8.01 - 7.89 (m, 2H), 7.79 (s, 1H), 7.34 (d, J = 4.7 Hz, 1H), 6.95 (d, J = 4.5 Hz, 2H), 5.96 (d, J = 8.6 Hz, 1H), 4.81 (dd, J = 55.3, 20.3 Hz, 3H), 4.48 (s, 2H), 3.81 (d, J = 56.3 Hz, 8H), 3.46 - 3.27 (m, 8H), 3.03 (dd, J = 21.3, 14.3 Hz, 5H), 2.65 (d, J = 17.8 Hz, 1H), 2.11 - 1.90 (m, 4H), 1.70 (s, 2H). LCMS (ESI) calcd. for C₃₄H₄₃N₁₀O₃S₂⁺ [M+H]⁺: 703.30, found, 702.7.

### Example 117: Preparation of N-(2-(1-((2-(2,6-dioxopiperidin-3-yl)-1-thioxoisoindolin-5-yl)methyl)piperidin-4-yl)-6-methoxy-2H-indazol-7-yl)-3-(trifluoromethyl)benzamide (GT-09413)

With reference to the method of Scheme T-1, the target compound (GT-09413) was obtained (white solid, 12 mg, yield 16.50%). ¹H NMR (400 MHz, DMSO) δ 11.14 (s, 1H), 10.68 (s, 1H), 10.10 (s, 1H), 8.40 (s, 3H), 7.97 (d, J = 7.9 Hz, 2H), 7.90 (s, 1H), 7.77 (d, J = 8.0 Hz, 2H), 7.70 (d, J = 9.1 Hz, 1H), 7.11 (d, J = 9.2 Hz, 1H), 5.94 (s, 1H), 4.81 (dd, J = 54.0, 20.4 Hz, 3H), 4.49 (s, 2H), 3.84 (d, J = 16.3 Hz, 3H), 3.53 (d, J = 11.5 Hz, 2H), 3.33 - 3.27 (m, 2H), 3.19 (d, J = 11.6 Hz, 2H), 2.97 (s, 1H), 2.65 (d, J = 17.7 Hz, 1H), 2.40 (s, 1H), 2.30 (d, J = 11.7 Hz, 2H), 2.10 (d, J = 5.4 Hz, 1H). LCMS (ESI) calcd. for C₃₅H₃₄F₃N₆O₄S⁺ [M+H]⁺: 691.23, found, 691.3.

### Example 118: Preparation of N-(2-(1-((2-(2,6-dioxopiperidin-3-yl)-1-thioxoisoindolin-4-yl)methyl)piperidin-4-yl)-6-methoxy-2H-indazol-5-yl)-3-(trifluoromethyl)benzamide (GT-09414)

With reference to the method of Scheme T-1, the target compound (GT-09414) was obtained (yellow solid, 9 mg, yield 12.89%). ¹H NMR (400 MHz, DMSO) δ 11.17 (s, 1H), 8.30 (t, J = 12.8 Hz, 3H), 8.07 - 7.93 (m, 2H), 7.80 (dd, J = 27.3, 19.6 Hz, 2H), 7.63 (d, J = 31.4 Hz, 1H), 7.09 (s, 1H), 5.91 (d, J = 53.1 Hz, 1H), 4.67 (d, J = 158.7 Hz, 2H), 3.86 (s, 3H), 3.43 (s, 3H), 3.34 - 3.19 (m, 4H), 3.03 - 2.91 (m, 2H), 2.67 (d, J = 17.5 Hz, 2H), 2.11 (dd, J = 15.5, 10.5 Hz, 4H). LCMS (ESI) calcd. for C₃₅H₃₄F₃N₆O₄S⁺ [M+H]⁺: 691.23, found, 691.3.

### Example 119: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)piperazin-1-yl)-1-(phenylthio)butan-2-yl)amino)-3-((trifluoromethyl)sulfonyl)phenyl)sulfonyl)benzamide (GT-09415)

With reference to the method of Scheme T-1, the target compound (GT-09415) was obtained (white solid, 57 mg, yield 67.83%). ¹H NMR (400 MHz, DMSO) δ 12.17 (s, 1H), 11.14 (s, 1H), 10.46 (s, 1H), 8.18 (d, J = 2.0 Hz, 1H), 7.99 (d, J = 8.8 Hz, 1H), 7.92 (d, J = 7.3 Hz, 1H), 7.77 (d, J = 8.6 Hz, 4H), 7.41 (d, J = 8.3 Hz, 2H), 7.29 (d, J = 7.3 Hz, 2H), 7.26 - 7.04 (m, 6H), 6.97 (t, J = 11.5 Hz, 2H), 5.94 (s, 1H), 4.96 - 4.64 (m, 2H), 4.20 (s, 1H), 3.88 (d, J = 12.3 Hz, 2H), 3.57 (s, 2H), 3.39 (s, 8H), 3.34 - 2.94 (m, 11H), 2.69 (dd, J = 35.0, 26.1 Hz, 4H), 2.28 (s, 2H), 2.14 (d, J = 32.3 Hz, 5H), 1.47 (t, J = 6.1 Hz, 2H), 1.00 (s, 6H). LCMS (ESI) calcd. for C₆₁H₆₉ClF₃N₈O₇S₄⁺ [M+H]⁺: 1245.38, found, 1245.3.

### Example 120: Preparation of 4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1-thioxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide (GT-09416)

With reference to the method of Scheme T-1, the target compound (GT-09416) was obtained (white solid, 24 mg, yield 28.96%). ¹H NMR (400 MHz, DMSO) δ 12.16 (s, 1H), 11.13 (d, J = 5.7 Hz, 2H), 10.21 (s, 1H), 8.17 (s, 1H), 7.97 (d, J = 9.3 Hz, 2H), 7.90 (d, J = 8.0 Hz, 1H), 7.79 - 7.72 (m, 3H), 7.62 (d, J = 7.9 Hz, 1H), 7.41 (d, J = 8.4 Hz, 2H), 7.30 (d, J = 7.1 Hz, 2H), 7.24 (d, J = 7.3 Hz, 2H), 7.17 (d, J = 8.3 Hz, 2H), 6.96 (d, J = 8.8 Hz, 2H), 5.93 (s, 1H), 4.97 - 4.69 (m, 6H), 4.30 (s, 2H), 4.10 (s, 2H), 3.89 (d, J = 12.1 Hz, 4H), 3.58 (s, 10H), 3.07 - 2.84 (m, 3H), 2.76 (s, 3H), 2.65 (d, J = 18.0 Hz, 4H), 2.28 (s, 2H), 2.11 (dd, J = 16.4, 11.2 Hz, 4H), 1.79 (s, 1H), 1.48 (d, J = 6.1 Hz, 2H), 1.01 (s, 6H). LCMS (ESI) calcd. for C₆₃H₇₁ClF₃N₈O₇S₄⁺ [M+H]⁺: 1271.40, found, 1271.4.

### Biological activity assay

### Reagents and Materials

| Reagents and materials | Suppliers or Source |
|---|---|
| Multiple myeloma cell line: MM.1S | ATCC (American Type Culture Collection) |
| Human large cell immunoblastic lymphoma cell line: SR | ATCC |
| Human non-small cell lung cancer cell line: HCC827 | ATCC |
| Human myeloid monocytic leukemia cell line: MV-4-11 | ATCC |
| Human breast cancer cell line: MCF-7 | ATCC |
| Human pancreatic cancer cell line: CFPAC-1 | Dalian Meilun Biotech Co., Ltd. |
| Human malignant melanoma cell line: A375 | Dalian Meilun Biotech Co., Ltd. |
| Human non-small cell lung cancer cell line: NCI-H1975 | ATCC |
| Human multiple myeloma peripheral blood B lymphocyte cell line: RPMI-8226 | ATCC |
| Human colon cancer cell line: SW620 | ATCC |
| Human diffuse large B-cell lymphoma cell line: TMD8 | Kyinno Biotechnology Co., Ltd |
| Human T lymphocytic leukemia cell line: Jurkat | ATCC |
| Human Malignant Meningioma Cell Line: JEKO-1 | ATCC |
| RPMI-1640 Medium | ATCC |
| IMDM Medium | Gibco |
| EMEM Medium | ATCC |
| DMEM high-glucose medium | Dalian Meilun Biotech Co., Ltd. |
| L-15 Medium | ATCC |
| Meilun Cell Counting Kit-8 | Dalian Meilun Biotech Co., Ltd. |
| Fetal bovine serum (FBS) | Sunrise Science Products company |
| Penicillin-Streptomycin | Beijing TransGen Biotech Co., Ltd. |
| CellTiter-Meiluncell Luminescent Cell Viability Assay Kit | Dalian Meilun Biotech Co., Ltd. |
| Mycoplasma detection kit | Beijing TransGen Biotech Co., Ltd. |
| STR genotype detection | Shanghai Biowing Applied Biotechnology Co. Ltd. |

### Methods:

### Cell cultures

The tumor cells used herein were cultured in a cell culture medium listed in table 3 at 37°C in an incubator with 5% CO₂. Prior to experimentation, all cell lines used were correctly identified by STR profiling, and tested negative for mycoplasma contamination using a mycoplasma detection kit through routine screenings.

**Table 3. Tumor cells and cell culture medium used in the present disclosure**

| Cell line | Cell culture medium used |
|---|---|
| MM.1S | RPMI 1640 culture medium supplemented with 10% FBS, as well as penicillin at a final concentration of 100 U/mL and streptomycin at a final concentration of 100 |
| MM.1R | |
| JEKO-1 | µg/mL |
| NCI-H1975 | |
| Jurkat | |
| RPMI-8226 | |
| HCC827 | |
| SR | |
| TMD8 | |
| MCF-7 | EMEM culture medium supplemented with 10% FBS, 10 µg/mL insulin, and penicillin at a final concentration of 100 U/mL and streptomycin at a final concentration of 100 µg/mL |
| MV-4-11 | IMDM culture medium supplemented with 10% FBS, 10 µg/mL insulin, and penicillin at a final concentration of 100 U/mL and streptomycin at a final concentration of 100 µg/mL |
| CFPAC-1 | |
| SW620 | L-15 culture medium supplemented with 10% FBS, as well as penicillin at a final concentration of 100 U/mL and streptomycin at a final concentration of 100 µg/mL |
| A375 | DMEM high-glucose medium supplemented with 10% FBS, as well as penicillin at a final concentration of 100 U/mL and streptomycin at a final concentration of 100 µg/mL |

### I. Determination of half inhibitory concentration (IC₅₀) of the compounds against tumor cells:

The IC₅₀ values of the compounds of the present disclosure (including the compounds in Table 1, Table 2, and Examples) were determined using the CellTiter-Meiluncell Luminescent Cell Viability Assay Kit from Dalian Meilun Biotech Co., Ltd. The detailed procedure is as follows: Tumor cells were seeded in 96-well cell culture plates at the densities specified in Table 4. On the following day, the test compounds of the present disclosure were subjected to serial dilution. The dilution can be performed according to one of the following two methods:
(1) The first dilution method: The test compounds of the present disclosure (including the compounds in Table 1, Table 2, and Examples) were subjected to 5-fold serial dilutions starting from a highest concentration of 2000 µM, resulting in a total of 9 concentrations from high to low (2000, 400, 80, 16, 3.2, 0.64, 0.128, 0.0256, 0.00512 µM). Subsequently, 0.5 µL of the test compound dilution was transferred into the 96-well plate containing the cells, with each well containing 100 µL of cell culture medium. This resulted in final test compound concentrations of 10, 2, 0.4, 0.08, 0.016, 0.0032, 0.00064, 0.000128, and 0.0000256 µM, respectively, or
(2) As an alternative, a second dilution method can be used: The test compounds of the present disclosure (including the compounds in Table 1, Table 2, and Examples) were subjected to a 50-fold gradient dilution starting from a highest concentration of 100 µM, resulting in 2 concentrations (100 µM, 2 µM). Into the 96-well plate, the diluted test compounds and corresponding detection reagents were added, resulting in final test compound concentrations of 500 nM and 10 nM, respectively. The experimental procedure is described below using the diluted 2 µM test compound as an example.

0.5 µL of the aforementioned diluted compound of the present disclosure (including the compounds in Table 1, Table 2, and Examples) was added to the seeded cells in 100 µL of medium, yielding a final test compound concentration of 10 nM. After the cells were treated with the compounds of the present disclosure for a specified period, the cell viability assay reagent was added to the culture medium according to the kit's instructions to determine cell viability. The negative control was DMSO, and the reference controls were corresponding commercial inhibitors (including Orelabrutinib (CAS No.: 1655504-04-3); Ibrutinib (CAS No.: 936563-96-1); Compound GT-03308 (CAS No.: 1679327-21-9); Compound GT-03335 (CAS No.: 1801765-04-7); Compound GT-03336 (CAS No.: 2160546-07-4); Compound GT-03334 (CAS No.: 1801747-42-1); Compound TQB3804 (CAS No.: 2267329-76-8); Erlotinib (CAS No.: 183321-74-6); Brigatinib (CAS No.: 1197953-54-0); Alectinib (CAS No.: 1256580-46-7); Abemaciclib (CAS No.: 1231929-97-7); Ribociclib (CAS No.: 1211443-58-1); Palbociclib (CAS No.: 571190-30-2); Defactinib (CAS No.: 1073154-85-4); PND-1186 (CAS No.: 1061353-68-1); Dasatinib (CAS No.: 302962-49-8); Emavusertib (CAS No.: 1801344-14-8); AT7519 (CAS No.: 844442-38-2); Venetoclax (ABT-199, CAS No.: 1257044-40-8); PF-06650833 (CAS No.: 1817626-54-2); HJM-561 (CAS No.: 2570251-68-0); Osimertinib (CAS No.: 1421373-65-0); FN-1501 (CAS No.: 1429515-59-2); and ABT-263 (Navitoclax, CAS No.: 923564-51-6); Afatinib (CAS No.: 850140-72-6); Enzalutamide (CAS No.: 915087-33-1); Tazemetostat (CAS No.: 1403254-99-8); UNC1999 (CAS No.: 1431612-23-5); AT7519 (CAS No.: 844442-38-2); lorlatinib (also known as lorbrena; CAS No.: 1454846-35-5); and BI-D1870 (CAS No.: 501437-28-1)). All controls were treated using the same procedure as the compounds of the present disclosure. The growth inhibition curves for the compounds of the present disclosure were plotted using Prism GraphPad software, and the IC₅₀ values were calculated therefrom.

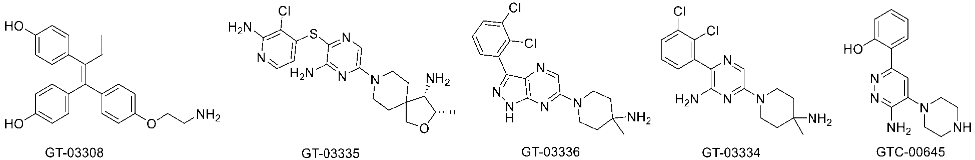

**Table 4. Seeding protocol and treatment time for tumor cells**

| Cells | Cells seeding protocol | The times for treating cells with the compounds of the present disclosure (hours) |
|---|---|---|
| MM.1S | cells were seeded at a density of 10,000 cells per well in 100 µL of serum-containing RPMI-1640 medium per well | 96 hours |
| Jurkat | | |
| SR | | |
| RPMI-8226 | | |
| TMD8 | | |
| JEKO-1 | cells were seeded at a density of 4,000 cells per well in 100 µL of serum-containing RPMI-1640 medium per well | 96 hours |
| WSU-DLCL2 | | |
| H358 | | |
| NCI-H1975 | | |
| H2228 | | |
| CCRF-CEM | | |
| H292 | | |
| HCC827 | | |
| MGC803 | cells were seeded at a density of 2,000 cells per well in 100 µL of serum-containing RPMI-1640 medium per well | 96 hours |
| MV-4-11 | cells were seeded at a density of 10,000 cells per well in 100 µL of serum-containing IMDM medium per well | 96 hours |
| CFPAC-1 | cells were seeded at a density of 3,000 cells per well in 100 µL of serum-containing IMDM medium per well | 96 hours |
| MDA-MB-231 | cells were seeded at a density of 4,000 cells per well in 100 µL of serum-containing DMEM medium per well | 96 hours |
| A375 | | |
| SK-N-AS | cells were seeded at a density of 3,000 cells per well in 100 µL of serum-containing DMEM medium per well | 96 hours |
| MCF-7 | cells were seeded at a density of 4,000 cells per well in 100 µL of serum-containing EMEM medium per well | 96 hours |
| SW620 | cells were seeded at a density of 4,000 cells per well in 100 µL of serum-containing L-15 medium per well | 96 hours |

The results of tumor cell proliferation inhibition are presented in Tables I-1 through I-16 below

**Table I-1: Inhibitory activity (IC₅₀, nM) of the compounds of the present disclosure, designed based on the ALK inhibitors, on tumor cell proliferation**

| Comp. No. /names | HCC827 | SR |
|---|---|---|
| lorlatinib | G | G |
| GT-05030 | D | A++ |
| GT-05097 | - | A+ |

| | | |
|---|---|---|
| Note: in table I-1, the symbols A+++ , A++, A+, A, B, C, D, E, F, and G are defined as follows: 0 nM < A+++ ≤ 10 nM; 10 nM < A++ ≤ 20 nM; 20 nM < A+ ≤ 30 nM; 30 nM < A ≤ 50 nM; 50 nM < B ≤ 100 nM; 100 nM < C ≤ 500 nM; 500 nM < D ≤ 1000 nM; 1000 nM < E ≤ 5000 nM; 5000 nM < F ≤ 10000 nM; 10000 nM < G. The symbol "-" indicates "not detected". | | |

**Table I-2: Inhibitory activity (Inhibition rate %) of the compounds of the present disclosure, designed based on ALK inhibitors, against tumor cell proliferation**

| Comp. No. /names | MV-4-11 | | MM.1S | | MCF-7 | | CFPAC-1 | | A375 | |
|---|---|---|---|---|---|---|---|---|---|---|
| | Inhibiti on Rate at 500 nM (%) | Inhibiti on Rate at 10 nM (%) | Inhibiti on Rate at 500 nM (%) | Inhibiti on Rate at 10 nM (%) | Inhibiti on Rate at 500 nM (%) | Inhibiti on Rate at 10 nM (%) | Inhibiti on Rate at 500 nM (%) | Inhibiti on Rate at 10 nM (%) | Inhibiti on Rate at 500 nM (%) | Inhibiti on Rate at 10 nM (%) |
| lorlatin ib | 1a3 | 1a1 | 1a1 | 1a3 | 1a6 | 1a5 | 1a6 | 1a5 | 1a5 | 1a5 |
| GT-08674 | 1a7 | 1a4 | 1a12 | 1a7 | 1a10 | 1a7 | - | - | - | - |
| GT-08675 | 1a11 | 1a5 | 1a7 | 1a5 | 1a8 | 1a6 | - | - | - | - |
| GT-08676 | 1a10 | 1a8 | 1a7 | 1a5 | 1a10 | 1a8 | - | - | - | - |
| GT-08677 | 1a10 | 1a4 | 1a6 | 1a5 | 1a7 | 1a3 | - | - | - | - |
| GT-08678 | - | - | 1a9 | 1a4 | 1a8 | 1a1 | - | - | - | - |
| GT-08679 | 1a12 | 1a9 | 1a6 | 1a5 | 1a8 | 1a6 | - | - | - | - |
| GT-08680 | 1a10 | 1a5 | 1a9 | 1a4 | - | - | - | - | - | - |
| GT-09425 | - | - | - | - | - | - | 1a10 | 1a5 | 1a9 | 1a6 |

| | | | | | | | | | | |
|---|---|---|---|---|---|---|---|---|---|---|
| Note: in table I-2, the symbols 1a1, 1a2, 1a3, 1a4, 1a5, 1a6, 1a7, 1a8, 1a9, 1a10, 1a11, 1a12, and 1a13 are defined as follows: -20% < 1a1 ≤ -10%, -10% < 1a2 < -5%, -5% ≤ 1a3 < 0%, 0% ≤ 1a4 < 10%, 10% ≤ 1a5 < 20%, 20% ≤ 1a6 < 30%, 30% ≤ 1a7 < 40%, 40% ≤ 1a8 < 50%, 50% ≤ 1a9 < 60%, 60% ≤ 1a10 < 70%, 70% ≤ 1a11 < 80%, 80% ≤ 1a12 ≤ 100%, 100% < 1a13 ≤ 110%. The symbol "-" indicates "not detected". | | | | | | | | | | |

**Table I-3: Inhibitory activity (Inhibition rate %) of the compounds of the present disclosure, designed based on EGFR inhibitors, against tumor cell proliferation**

| Comp. No. /names | MV-4-11 | | H1975 | | RPMI-8226 | | SW620 | |
|---|---|---|---|---|---|---|---|---|
| | Inhibitio n Rate at 500 nM (%) | Inhibitio n Rate at 10 nM (%) | Inhibitio n Rate at 500 nM (%) | Inhibitio n Rate at 10 nM (%) | Inhibition Rate at 500 nM (%) | Inhibitio n Rate at 10 nM (%) | Inhibition Rate at 500 nM (%) | Inhibitio n Rate at 10 nM (%) |
| erlotinib | 1b3 | 1b1 | 1b1 | 1b1 | 1b4 | 1b4 | 1b3 | 1b3 |
| GT-08754 | 1b9 | 1b3 | - | - | - | - | - | - |
| GT-08755 | 1b9 | 1b4 | - | - | - | - | - | - |
| GT-08756 | 1b6 | 1b2 | - | - | - | - | - | - |
| GT-08757 | - | - | 1b7 | 1b4 | - | - | - | - |
| GT-08758 | 1b8 | 1b3 | - | - | - | - | - | - |
| GT-08760 | - | - | 1b7 | 1b4 | - | - | - | - |
| GT-08761 | 1b8 | 1b2 | - | - | 1b6 | 1b4 | - | - |
| GT-08762 | 1b10 | 1b5 | - | - | 1b6 | 1b5 | - | - |
| GT-08763 | 1b7 | 1b5 | - | - | 1b7 | 1b5 | - | - |
| GT-08764 | - | - | 1b7 | 1b4 | - | - | - | - |
| GT-09429 | - | - | - | - | - | - | 1b9 | 1b3 |

| | | | | | | | | |
|---|---|---|---|---|---|---|---|---|
| Note: in table I-3, the symbols 1b1, 1b2, 1b3, 1b4, 1b5, 1b6, 1b7, 1b8, 1b9, and 1b10 are defined as follows: 0 < 1b1 < 5%, 5% ≤ 1b2 < 10%, 10% ≤ 1b3 < 20%, 20% ≤ 1b4 < 30%, 30% ≤ 1b5 < 40%, 40% ≤ 1b6 < 50%, 50% ≤ 1b7 < 60%, 60% ≤ 1b8 < 70%, 70% ≤ 1b9 < 80%, 80% ≤ 1b10 ≤ 100%. The symbol "-" indicates "not detected". | | | | | | | | |

**Table I-4: Inhibitory activity (Inhibition rate %) of the compounds of the present disclosure, designed based on BTK inhibitors, against tumor cell proliferation**

| Comp. No. /names | TMD8 | | SW620 | |
|---|---|---|---|---|
| | Inhibition Rate at 500 nM (%) | Inhibition Rate at 10 nM (%) | Inhibition Rate at 500 nM (%) | Inhibition Rate at 10 nM (%) |
| ibrutinib | 1c7 | 1c1 | 1c2 | 1c1 |
| GT-09436 | 1c12 | 1c5 | 1c12 | 1c11 |

| | | | | |
|---|---|---|---|---|
| Note: in table I-4, the symbols 1c1, 1c2, 1c3, 1c4, 1c5, 1c6, 1c7, 1c8, 1c9, 1c10, 1c11, and 1c12 are defined as follows: -5% < 1c1 ≤ 0%, 0% < 1c2 < 5%, 5% ≤ 1c3 < 10%, 10% ≤ 1c4 < 15%, 15% ≤ 1c5 < 20%, 20% ≤ 1c6 < 30%, 30% ≤ 1c7 < 40%, 40% ≤ 1c8 < 50%, 50% ≤ 1c9 < 60%, 60% ≤ 1c10 < 70%, 70% ≤ 1c11 < 80%, 80% ≤ 1c12 ≤ 100%. The symbol "-" indicates "not detected". | | | | |

**Table I-5: Inhibitory activity (IC₅₀, nM) of the compounds of the present disclosure, designed based on the IRAK4 inhibitors, on tumor cell proliferation**

| Comp. No. | Jurkat | MM.1S |
|---|---|---|
| GT-04950 | D | C |

| | | |
|---|---|---|
| Note: in table I-5, the symbols A+++ , A++, A+, A, B, C, D, E, F, and G are defined as follows: 0 nM < A+++ ≤ 10 nM; 10 nM < A++ ≤ 20 nM; 20 nM < A+ ≤ 30 nM; 30 nM < A ≤ 50 nM; 50 nM < B ≤ 100 nM; 100 nM < C ≤ 500 nM; 500 nM < D ≤ 1000 nM; 1000 nM < E ≤ 5000 nM; 5000 nM < F ≤ 10000 nM; 10000 nM < G. The symbol "-" indicates "not detected". | | |

**Table I-6: Inhibitory activity (Inhibition rate %) of the compounds of the present disclosure, designed based on IRAK4 inhibitors, against tumor cell proliferation**

| Comp. No. /names | MV-4-11 | | SW620 | | MM.1S | | JEKO-1 | |
|---|---|---|---|---|---|---|---|---|
| | Inhibitio n Rate at 500 nM (%) | Inhibitio n Rate at 10 nM (%) | Inhibitio n Rate at 500 nM (%) | Inhibitio n Rate at 10 nM (%) | Inhibitio n Rate at 500 nM (%) | Inhibitio n Rate at 10 nM (%) | Inhibitio n Rate at 500 nM (%) | Inhibitio n Rate at 10 nM (%) |
| PF0665083 3 | 1d4 | 1d4 | 1d3 | 1d3 | 1d1 | 1d1 | 1d4 | 1d3 |
| GT-05098 | 1d10 | 1d4 | 1d6 | 1d3 | 1d10 | 1d4 | 1d4 | 1d2 |
| GT-05321 | 1d10 | 1d6 | 1d9 | 1d5 | 1d10 | 1d5 | 1d6 | 1d2 |
| GT-05322 | 1d10 | 1d3 | - | - | - | - | - | - |
| GT-05286 | 1d10 | 1d10 | 1d9 | 1d8 | 1d10 | 1d7 | 1d10 | 1d3 |
| GT-05287 | 1d9 | 1d3 | - | - | - | - | - | - |
| GT-05288 | 1d6 | 1d1 | - | - | - | - | - | - |
| GT-05289 | 1d9 | 1d2 | - | - | - | - | - | - |
| GT-05292 | 1d10 | 1d10 | 1d10 | 1d9 | 1d10 | 1d7 | 1d10 | 1d5 |
| GT-05293 | 1d8 | 1d3 | - | - | - | - | - | - |
| GT-05296 | 1d7 | 1d3 | - | - | - | - | - | - |

| | | | | | | | | |
|---|---|---|---|---|---|---|---|---|
| Note: in table I-6, the symbols 1d1, 1d2, 1d3, 1d4, 1d5, 1d6, 1d7, 1d8, 1d9, and 1d10 are defined as follows: 0 < 1d1 < 5%, 5% ≤ 1d2 < 10%, 10% ≤ 1d3 < 20%, 20% ≤ 1d4 < 30%, 30% ≤ 1d5 < 40%, 40% ≤ 1d6 < 50%, 50% ≤ 1d7 < 60%, 60% ≤ 1d8 < 70%, 70% ≤ 1d9 < 80%, 80% ≤ 1d10 ≤ 100%. | | | | | | | | |

**Table I-7: Inhibitory activity (Inhibition rate %) of the compounds of the present disclosure, designed based on CDK9 inhibitors, against tumor cell proliferation**

| Comp. No. /names | MV-4-11 | | MM.1S | | TMD8 | |
|---|---|---|---|---|---|---|
| | Inhibition Rate at 500 nM (%) | Inhibition Rate at 10 nM (%) | Inhibition Rate at 500 nM (%) | Inhibition Rate at 10 nM (%) | Inhibition Rate at 500 nM (%) | Inhibition Rate at 10 nM (%) |
| GT-08682 | 1e8 | 1e5 | 1e7 | 1e3 | - | - |
| GT-08683 | 1e7 | 1e7 | - | - | - | - |
| GT-08686 | 1e10 | 1e7 | 1e10 | 1e1 | - | - |
| GT-08688 | 1e7 | 1e6 | - | - | - | - |
| GT-08751 | 1e8 | 1e3 | - | - | - | - |
| GT-08752 | 1e9 | 1e3 | - | - | - | - |
| GT-09435 | - | - | - | - | 1e9 | 1e1 |

| | | | | | | |
|---|---|---|---|---|---|---|
| Note: in table I-7, the symbols 1e1, 1e2, 1e3, 1e4, 1e5, 1e6, 1e7, 1e8, 1e9, and 1e10 are defined as follows: 0 < 1e1 < 5%, 5% ≤ 1e2 < 10%, 10% ≤ 1e3 < 20%, 20% ≤ 1e4 < 30%, 30% ≤ 1e5 < 40%, 40% ≤ 1e6 < 50%, 50% ≤ 1e7 < 60%, 60% ≤ 1e8 < 70%, 70% ≤ 1e9 < 80%, 80% ≤ 1e10 ≤ 100%. | | | | | | |

**Table I-8: Inhibitory activity (Inhibition rate %) of the compounds of the present disclosure, designed based on SMARCA2/4 inhibitors, against tumor cell proliferation**

| Comp. No. /names | MV-4-11 | | RPMI-8226 | | MM.1S | |
|---|---|---|---|---|---|---|
| | Inhibition Rate at 500 nM (%) | Inhibition Rate at 10 nM (%) | Inhibition Rate at 500 nM (%) | Inhibition Rate at 10 nM (%) | Inhibition Rate at 500 nM (%) | Inhibition Rate at 10 nM (%) |
| GTC00645 (Positive Control) | 1f4 | 1f4 | 1f1 | 1f1 | 1f5 | 1f5 |
| GT-08714 | - | - | 1f8 | 1f7 | 1f9 | 1f7 |
| GT-08786 | 1f8 | 1f4 | - | - | - | - |
| GT-08787 | - | - | - | - | 1f8 | 1f7 |

| | | | | | | |
|---|---|---|---|---|---|---|
| Note: in table I-8, the symbols 1f1, 1f2, 1f3, 1f4, 1f5, 1f6, 1f7, 1f8, 1f9, 1f10, 1f11, and 1f12 are defined as follows: -5% < 1f1 ≤ 0%, 0% < 1f2 < 5%, 5% ≤ 1f3 < 10%, 10% ≤ 1f4 < 15%, 15% ≤ 1f5 < 20%, 20% ≤ 1f6 < 30%, 30% ≤ 1f7 < 40%, 40% ≤ 1f8 < 50%, 50% ≤ 1f9 < 60%, 60% ≤ 1f10 < 70%, 70% ≤ 1f11 < 80%, 80% ≤ 1f12 ≤ 100%. The symbol "-" indicates "not detected". | | | | | | |

**Table I-9: Inhibitory activity (Inhibition rate %) of the compounds of the present disclosure, designed based on CDK4/6 inhibitors, against tumor cell proliferation**

| Comp. No. /names | MV-4-11 | | MM.1S | |
|---|---|---|---|---|
| | Inhibition Rate at 500 nM (%) | Inhibition Rate at 10 nM (%) | Inhibition Rate at 500 nM (%) | Inhibition Rate at 10 nM (%) |
| Ribociclib | 1g4 | 1g2 | 1g3 | 1g3 |
| GT-08765 | - | - | 1g6 | 1g5 |
| GT-08772 | 1g6 | 1g5 | - | - |

| | | | | |
|---|---|---|---|---|
| Note: in table I-9, the symbols 1g1, 1g2, 1g3, 1g4, 1g5, 1g6, 1g7, 1g8, 1g9, and 1g10 are defined as follows: 0 < 1g1 < 5%, 5% ≤ 1g2 < 10%, 10% ≤ 1g3 < 20%, 20% ≤ 1g4 < 30%, 30% ≤ 1g5 < 40%, 40% ≤ 1g6 < 50%, 50% ≤ 1g7 < 60%, 60% ≤ 1g8 < 70%, 70% ≤ 1g9 < 80%, 80% ≤ 1g10 ≤ 100%. The symbol "-" indicates "not detected". | | | | |

**Table I-10: Inhibitory activity (Inhibition rate %) of the compounds of the present disclosure, designed based on BCR-ABL inhibitors, against tumor cell proliferation**

| Comp. No. /name s | MV-4-11 | | RPMI-8226 | | SW620 | | MM.1S | | CFPAC-1 | |
|---|---|---|---|---|---|---|---|---|---|---|
| | Inhibiti on Rate at 500 nM (%) | Inhibiti on Rate at 10 nM (%) | Inhibiti on Rate at 500 nM (%) | Inhibiti on Rate at 10 nM (%) | Inhibiti on Rate at 500 nM (%) | Inhibiti on Rate at 10 nM (%) | Inhibiti on Rate at 500 nM (%) | Inhibiti on Rate at 10 nM (%) | Inhibiti on Rate at 500 nM (%) | Inhibiti on Rate at 10 nM (%) |
| imatin ib | 1h1 | 1h1 | 1h3 | 1h2 | 1h3 | 1h2 | - | - | - | - |
| GT-08767 | 1h10 | 1h10 | 1h10 | 1h4 | 1h10 | 1h6 | 1h10 | 1h9 | - | - |
| GT-08770 | - | - | 1h6 | 1h3 | - | - | 1h5 | 1h2 | - | - |
| GT-08773 | 1h10 | 1h5 | 1h8 | 1h2 | 1h9 | 1h1 | 1h10 | 1h3 | - | - |
| GT-08774 | 1h9 | 1h4 | - | - | - | - | - | - | - | - |
| GT-09439 | - | - | - | - | - | - | - | - | 1h8 | 1h2 |

| | | | | | | | | | | |
|---|---|---|---|---|---|---|---|---|---|---|
| Note: in table I-10, the symbols 1h1, 1h2, 1h3, 1h4, 1h5, 1h6, 1h7, 1h8, 1h9, and 1h10 are defined as follows: 0 < 1h1 < 5%, 5% ≤ 1h2 < 10%, 10% ≤ 1h3 < 20%, 20% ≤ 1h4 < 30%, 30% ≤ 1h5 < 40%, 40% ≤ 1h6 < 50%, 50% ≤ 1h7 < 60%, 60% ≤ 1h8 < 70%, 70% ≤ 1h9 < 80%, 80% ≤ 1h10 ≤ 100%. The symbol "-" indicates "not detected". | | | | | | | | | | |

**Table I-11: Inhibitory activity (Inhibition rate %) of the compounds of the present disclosure, designed based on RSK inhibitors, against tumor cell proliferation**

| Comp. No. /names | MV-4-11 | | RPMI-8226 | | MM.1S | |
|---|---|---|---|---|---|---|
| | Inhibition Rate at 500 nM (%) | Inhibition Rate at 10 nM (%) | Inhibition Rate at 500 nM (%) | Inhibition Rate at 10 nM (%) | Inhibition Rate at 500 nM (%) | Inhibition Rate at 10 nM (%) |
| BI-D1870 | 1i4 | 1i1 | 1i3 | 1i3 | 1i4 | 114 |
| GT-08777 | 1i8 | 1i4 | 1i6 | 1i1 | 1i7 | 1i4 |
| GT-08778 | 1i10 | 1i7 | 1i7 | 1i3 | 1i10 | 1i6 |

| | | | | | | |
|---|---|---|---|---|---|---|
| Note: in table I-11, the symbols 1i1, 112, 1i3, 1i4, 1i5, 1i6, 1i7, 1i8, 1i9, and 1i10 are defined as follows: 0 < 1i1 < 5%, 5% ≤ 1i2 < 10%, 10% ≤ 1i3 < 20%, 20% ≤ 1i4 < 30%, 30% ≤ 1i5 < 40%, 40% ≤ 1i6 < 50%, 50% ≤ 1i7 < 60%, 60% ≤ 1i8 < 70%, 70% ≤ 1i9 < 80%, 80% ≤ 1i10 ≤ 100%. The symbol "-" indicates "not detected". | | | | | | |

**Table I-12: Inhibitory activity (Inhibition rate %) of the compounds of the present disclosure, designed based on FAK inhibitors, against tumor cell proliferation**

| Comp. No. /names | MV-4-11 | | SW620 | | MM.1S | |
|---|---|---|---|---|---|---|
| | Inhibition Rate at 500 nM (%) | Inhibition Rate at 10 nM (%) | Inhibition Rate at 500 nM (%) | Inhibition Rate at 10 nM (%) | Inhibition Rate at 500 nM (%) | Inhibition Rate at 10 nM (%) |
| Defactinib | 1j7 | 1j1 | 1j2 | 1j1 | 1j2 | 1j3 |
| GT-08779 | 1j9 | 1j3 | - | - | - | - |
| GT-08780 | 1j9 | 1j4 | 1j6 | 1j3 | 1j6 | 1j4 |
| GT-08781 | 1j8 | 1j2 | - | - | - | - |
| GT-08783 | 1j9 | 1j4 | - | - | - | - |
| GT-08883 | 1j9 | 1j4 | 1j6 | 1j5 | 1j6 | 1j3 |

| | | | | | | |
|---|---|---|---|---|---|---|
| Note: in table I-12, the symbols 1j1, 1j2, 1j3, 1j4, 1j5, 1j6, 1j7, 1j8, 1j9, and 1j10 are defined as follows: 0 < 1j1 < 5%, 5% ≤ 1j2 < 10%, 10% ≤ 1j3 < 20%, 20% ≤ 1j4 < 30%, 30% ≤ 1j5 < 40%, 40% ≤ 1j6 < 50%, 50% ≤ 1j7 < 60%, 60% ≤ 1j8 < 70%, 70% ≤ 1j9 < 80%, 80% ≤ 1j10 ≤ 100%. The symbol "-" indicates "not detected". | | | | | | |

**Table I-13: Inhibitory activity (Inhibition rate %) of the compounds of the present disclosure, designed based on CDK2 inhibitors, against tumor cell proliferation**

| Comp. No. /names | MV-4-11 | | MM.1S | | JEKO-1 | |
|---|---|---|---|---|---|---|
| | Inhibition Rate at 500 nM (%) | Inhibition Rate at 10 nM (%) | Inhibition Rate at 500 nM (%) | Inhibition Rate at 10 nM (%) | Inhibition Rate at 500 nM (%) | Inhibition Rate at 10 nM (%) |
| GT-08792 | 1k7 | 1k1 | 1k8 | 1k3 | - | - |
| GT-08793 | 1k8 | 1k5 | - | - | - | - |
| GT-08797 | 1k8 | 1k2 | - | - | - | - |
| GT-08798 | 1k8 | 1k4 | 1k7 | 1k3 | 1k8 | 1k3 |

| | | | | | | |
|---|---|---|---|---|---|---|
| Note: in table I-13, the symbols 1k1, 1k2, 1k3, 1k4, 1k5, 1k6, 1k7, 1k8, 1k9, and 1k10 are defined as follows: 0 < 1k1 < 5%, 5% ≤ 1k2 < 10%, 10% ≤ 1k3 < 20%, 20% ≤ 1k4 < 30%, 30% ≤ 1k5 < 40%, 40% ≤ 1k6 < 50%, 50% ≤ 1k7 < 60%, 60% ≤ 1k8 < 70%, 70% ≤ 1k9 < 80%, 80% ≤ 1k10 ≤ 100%. The symbol "-" indicates "not detected". | | | | | | |

**Table I-14: Inhibitory activity (Inhibition rate %) of the compounds of the present disclosure, designed based on AKT inhibitors, against tumor cell proliferation**

| Comp. No. /names | MM.1S | |
|---|---|---|
| | Inhibition Rate at 500 nM (%) | Inhibition Rate at 10 nM (%) |
| GT-08895 | 1L7 | 1L5 |

| | | |
|---|---|---|
| Note: in table I-14, the symbols 1L1, 1L2, 1L3, 1L4, 1L5, 1L6, 1L7, 1L8, 1L9, and 1L10 are defined as follows: 0 < 1L1 < 5%, 5% ≤ 1L2 < 10%, 10% ≤ 1L3 < 20%, 20% ≤ 1L4 < 30%, 30% ≤ 1L5 < 40%, 40% ≤ 1L6 < 50%, 50% ≤ 1L7 < 60%, 60% ≤ 1L8 < 70%, 70% ≤ 1L9 < 80%, 80% ≤ 1L10 ≤ 100%. | | |

**Table I-15: Inhibitory activity (Inhibition rate %) of the compounds of the present disclosure, designed based on BCL-2 inhibitors, against tumor cell proliferation**

| Comp. No. /names | SW620 | |
|---|---|---|
| | Inhibition Rate at 500 nM (%) | Inhibition Rate at 10 nM (%) |
| ABT-199 | 1m2 | 1m2 |
| GT-09415 | 1m10 | 1m5 |

| | | |
|---|---|---|
| Note: in table I-15, the symbols 1m1, 1m2, 1m3, 1m4, 1m5, 1m6, 1m7, 1m8, 1m9, and 1m10 are defined as follows: 0 < 1m1 < 5%, 5% ≤ 1m2 < 10%, 10% ≤ 1m3 < 20%, 20% ≤ 1m4 < 30%, 30% ≤ 1m5 < 40%, 40% ≤ 1m6 < 50%, 50% ≤ 1m7 < 60%, 60% ≤ 1m8 < 70%, 70% ≤ 1m9 < 80%, 80% ≤ 1m10 ≤ 100%. | | |

**Table I-16: Inhibitory activity (Inhibition rate %) of the compounds of Formula (II) of the present disclosure against tumor cell proliferation**

| Comp. No. /names | MM.1S | |
|---|---|---|
| | Inhibition Rate at 500 nM (%) | Inhibition Rate at 10 nM (%) |
| Lenalidomide | 1n4 | ln2 |
| GT-06977 | 1n5 | ln6 |
| GT-06981 | 1n5 | ln3 |
| GT-05566 | 1n3 | ln3 |

| | | |
|---|---|---|
| Note: in table I-16, the symbols 1n1, 1n2, ln3, 1n4, 1n5, ln6, ln7, 1n8, ln9, and 1n10 are defined as follows: 0 < 1n1 < 10%, 10% ≤ 1n2 < 20%, 20% ≤ ln3 < 30%, 30% ≤ 1n4 < 35%, 35% ≤ 1n5 < 40%, 40% ≤ 1n6 < 50%, 50% ≤ ln7 < 60%, 60% ≤ 1n8 < 70%, 70% ≤ ln9 < 80%, 80% ≤ 1n10 ≤ 100%. | | |

The experimental results demonstrated that the compounds of the present disclosure (including those listed in Table 1 and in Examples 1-467) can effectively inhibit the proliferation of tumor cells proliferation (as shown in Tables I-1 through I-16), which were superior to those of the corresponding positive control drugs.

### II. Determining the Effect of Compounds of the present disclosure on Target Protein Expression by Western Blot

Conventional Western blot assay was performed to evaluate the effects of the compounds of the present disclosure on the expression of target proteins in cells. Cell lines selected for WB detection based on the target of interest were summarized in Table II-1.

**Table II-1**

| Cell Type | Cell Line | Corresponding Target(s) |
|---|---|---|
| hPBMC Cell | hPBMC Cell | IKZF1/2/3, Wee1, CKlα, GSPT1, IRAK4, CDK2, RSK1, RSK2, AKT, EZH2 |
| Adherent Cells | MDA-MB-231 Cell | CDK4/6, CDK9 |
| | LnCap Cell | AR |
| | H1975 Cell | EGFR |
| | H2228 Cell | ALK |
| | MCF-7 Cell | ER |
| | A549 Cell | FAK |
| | T47D Cell | PARP |
| | H4 Cell | tau |
| Suspension Cells | Molt-4 Cell | BCL-2, BRM, BRG1 |
| | K562 Cell | BCR-ABL |
| | Daudi Cell | BTK |

(1) Cell Seeding and Protein Harvesting: The selected cells were processed according to the cell seeding procedure and protein harvesting procedure in Table II-2.

**Table II-2 Cell Seeding and Protein Harvesting Procedures**

| Cell Type | Cell Line | Cell Plating Procedures for Various Cell Types |
|---|---|---|
| hPBMC Cell | hPBMC Cell | Human peripheral blood mononuclear cells (hPBMCs) at a density of 1×10⁶ cells/mL were thawed according to the PBMC Recovery Protocol (Milecell Biotechnologies Inc, Shanghai). The cells were then treated with the compounds of the present disclosure (including the compounds listed in Tables 1 and 2, and the Examples compounds) at concentrations of 50 nM and 500 nM. Controls included a negative control (DMSO) and a positive control (the commercial immunomodulatory drug lenalidomide), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA (Bicinchoninic Acid) assay. |
| Adherent Cells | MDA-MB-231 Cell, LnCap Cell, H1975 Cell, H2228 Cell, MCF-7 Cell, A549 Cell, T47D Cell, H4 Cell, | Adherent cells (e.g., MDA-MB-231 cells) were added to 6-well plates at a seeding density of 2.5×10⁵/mL, with a total volume of 2 mL of cell suspension per well, cultured for 12 hours, and then treated with the compounds of the present disclosure (including the compounds listed in Tables 1 and 2, and the Examples compounds) at concentrations of 500 nM and 50 nM. Controls included a negative control (DMSO) and a positive control (corresponding commercial inhibitors), both processed following the identical experimental protocol as the compounds of the present disclosure. After 24 hours of compounds treatment, the culture medium was removed. The cells were washed once with PBS and then lysed with RIPA protein lysate containing protease inhibitors. The resulting lysed proteins were subsequently scraped with a cell scraper, placed on ice for 30 minutes, and centrifuged. The supernatant was aspirated as the extracted total cell protein. The protein concentration of the supernatant was determined using the conventional BCA assay. |
| Suspension Cells | Molt-4 Cell, K562 Cell, Daudi Cell | Suspension cells (e.g., Molt-4 cells) were added to 12-well plates at a seeding density of 5×10⁵/mL, with a total volume of 1 mL of cell suspension per well, cultured for 12 hours, and then treated with the compounds of the present disclosure (including the compounds listed in Tables 1 and 2, and the Examples compounds) at concentrations of 500 nM and 50 nM. Controls included a negative control (DMSO) and a positive control (corresponding commercial inhibitors), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA assay. |

(2) To the collected supernatant, 5X SDS sample loading buffer was added, followed by denaturation at 95°C for 5 minutes. The denatured samples were then separated by SDS-PAGE electrophoresis on a polyacrylamide gel. Proteins were transferred to a nitrocellulose membrane (0.45 µm NC membrane), blocked with blocking buffer at room temperature for 1 hour, and subjected to antibody incubation and development procedures according to the antibody manufacturer's instructions (Cell Signaling Technology).

Half-Maximal Degradation Concentration value (the drug concentration required for degrading proteins by 50%, abbreviated as DC₅₀) reads method: comparing the grayscale values of Western blot bands from drug-treated samples with those from blank DMSO-treated controls, and identifying the drug concentration range at which the grayscale value is half that of the DMSO-treated control bands.

DC₅₀ values can be calculated using ImageJ software to measure grayscale values of Western blot bands from drug-treated samples. The concentration-response curve is fitted to estimate the drug concentration corresponding to half the grayscale value.

Target protein remaining (%) refers to the percentage of target protein remaining in cells after treatment with protein degrader compounds.

Calculation method for target protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment. $Target protein degradation rate \left(%\right) = 1 - target protein remaining \left(%\right) .$

The effects of the compounds of the present disclosure (including the compounds listed in Tables 1 and 2, and the Examples compounds) on substrate proteins expression in cells were shown in Tables IIA1-IIA12 below.

**Table IIA1 Degradation of substrate protein ALK by the Example compounds of the present disclosure designed based on ALK inhibitors**

| Comp. No. | ALK | Comp. No. | ALK |
|---|---|---|---|
| Alectinib | No degradation | GT-08676 | A |
| GT-05030 | A | GT-08678 | A |
| GT-05097 | A | GT-08679 | A |
| GT-08675 | A | GT-08680 | A |

**Table IIA2 Degradation of substrate protein CDK9 by the Example compounds of the present disclosure designed based on CDK9 inhibitors**

| Comp. No. | CDK9 | Comp. No. | CDK9 |
|---|---|---|---|
| GT-08681 | A | GT-08686 | A |
| GT-08682 | A | GT-08687 | A |
| GT-08683 | A | GT-08688 | A |
| GT-08684 | A | GT-08752 | A |
| GT-08685 | A | GT-08753 | A |

**Table IIA3 Degradation of substrate protein EGFR by the Example compounds of the present disclosure designed based on EGFR inhibitors**

| Comp. No. | EGFR | Comp. No. | EGFR |
|---|---|---|---|
| Osimertinib | No degradation | GT-08763 | A |
| GT-08761 | A | GT-08764 | A |
| GT-08762 | A | | |

**Table IIA4 Degradation of substrate protein IRAK4 by the Example compounds of the present disclosure designed based on IRAK4 inhibitors**

| Comp. No. | IRAK4 |
|---|---|
| emavusertib | No degradation |
| GT-05292 | A |
| GT-05297 | A |

**Table IIA5 Degradation of substrate proteins CDK4, CDK6 by the Example compounds of the present disclosure designed based on CDK4/6 inhibitors**

| Comp. No. | CDK4 | CDK6 | Comp. No. | CDK4 | CDK6 |
|---|---|---|---|---|---|
| GT-08765 | A | A | GT-08771 | A | A |
| GT-08766 | A | A | GT-08772 | A | A |

**Table IIA6 Degradation of substrate protein C-ABL by the Example compounds of the present disclosure designed based on BCR-ABL inhibitors**

| Comp. No. | BCR-ABL | Comp. No. | BCR-ABL |
|---|---|---|---|
| GT-08767 | A | GT-08774 | A |
| GT-08768 | A | GT-08776 | A |
| GT-08770 | A | | |

**Table IIA7 Degradation of substrate proteins BRM, BRG1 by the Example compounds of the present disclosure designed based on SMARCA2/4 inhibitors**

| Comp. No. | BRM | BRG1 | Comp. No. | BRM | BRG1 |
|---|---|---|---|---|---|
| GT-08787 | A | A | GT-09066 | A | B |
| GT-09064 | A | B | GT-09067 | A | B |
| GT-09065 | A | B | | | |

**Table IIA8 Degradation of substrate protein FAK by the Example compounds of the present disclosure designed based on FAK inhibitors**

| Comp. No. | FAK | Comp. No. | FAK |
|---|---|---|---|
| GT-08779 | A | GT-08782 | A |
| GT-08780 | A | GT-08783 | A |
| GT-08781 | A | GT-08784 | A |
| GT-08883 | A | | |

**Table IIA9 Degradation of substrate protein CDK2 by the Example compounds of the present disclosure designed based on CDK2 inhibitors**

| Comp. No. | CDK2 |
|---|---|
| GT-08792 | Yes |
| GT-08798 | Yes |

**Table IIA10 Degradation of substrate protein AKT by the Example compounds of the present disclosure designed based on AKT inhibitors**

| Comp. No. | AKT |
|---|---|
| GT-08895 | A |
| GT-08896 | A |
| GT-08897 | A |

**Table IIA11 Degradation of substrate protein Tau by the Example compounds of the present disclosure designed based on Tau inhibitors**

| Comp. No. | Tau |
|---|---|
| GT-08867 | A |
| GT-08979 | A |
| GT-08981 | A |

**Table IIA12 Degradation of substrate proteins WEE1, IKZF1, IKZF2, IKZF3, CK1α, and GSPT1 by the compounds of Formula (II) of the present disclosure**

| Comp. No. | Weel | IKZF 1 | IKZF2 | IKZF3 | GSPT1 | CK1α |
|---|---|---|---|---|---|---|
| Lenalidomide | B | A | B | A | B | B |
| GT-06977 | B | B | B | A | B | B |
| GT-06981 | B | B | B | A | B | B |
| GT-05566 | A | A | A | A | A | B |
| GT-05563 | A | A | A | A | A | B |

| | | | | | | |
|---|---|---|---|---|---|---|
| Note: In Tables IIA1 to IIA12, "A" is defined as 0% ≤ substrate protein remaining % ≤ 80%, and "B" is defined as 80%< substrate protein remaining %≤ 100%. | | | | | | |

The degradation effects of the designed and synthesized compounds of Formula (I) and Formula (II) of the present invention on the target substrate proteins are shown in Tables IIA1-IIA12 and Figure 1. The experimental results from Tables IIA1 through IIA12 and Figure 1 demonstrated that the compounds of formula (I) and formula (II) of the present invention can significantly degrade the target substrate proteins. The commercially available inhibitor cannot degrade the target protein (with 100% target protein remaining), whereas the compounds of formula (I) of the present invention achieve significant degradation of the target substrate proteins. Compared to lenalidomide, the compounds of formula (II) of the present invention show a significant improvement in the efficiency of inducing substrate protein degradation and can also selectively induce the degradation of substrate proteins. In summary, the structural diversity of the compounds of the present invention enables the effective degradation of diverse substrate proteins, thereby potentially enabling their use in treating indications associated with these substrate proteins.

### III. Determination of CRBN-binding affinity of compounds

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:
1. According to the instructions of the CEREBLON BINDING KITS, serial dilutions were performed for the test compounds of the present disclosure (including the compounds in Table 1, Table 2, and Examples) and for lenalidomide. Specifically, diluent #9 (1X) solution was used as the diluent, and multiple concentration gradients were set. The dilution operation can be performed according to one of the following two methods:
   (1) The first dilution method: The test compounds of the present disclosure (including the compounds in Table 1, Table 2, and Examples) were subjected to 5-fold serial dilutions starting from a highest concentration of 40 µM, resulting in a total of 7 concentrations from high to low (40, 8, 1.6, 0.32, 0.064, 0.0128, 0.00256 µM). Subsequently, the diluted test compounds and corresponding detection reagents were added to each well of a 96-well plate, resulting in final test compound concentrations of 10, 2, 0.4, 0.08, 0.016, 0.0032, and 0.00064 µM, respectively.
   (2) As an alternative, a second dilution method can be used: The test compounds of the present disclosure (including the compounds in Table 1, Table 2, and Examples) were subjected to 4-fold gradient dilutions starting from a highest concentration of 8 µM, resulting in 2 concentrations (8 µM, 2 µM). Into the 96-well plate, the diluted test compounds and corresponding detection reagents were added, resulting in final test compound concentrations of 2 µM and 0.5 µM, respectively. The experimental procedure is described below using the diluted 8 µM test compound and lenalidomide solutions as examples.
2. 2.5 µL of the above 8 µM of the tested compounds and lenalidomide solution, as well as the same volume of diluent #9 (1X) solution (solvent control group, Std0) were added to each well of a 96-well plate, respectively. Then, 2.5 µL of human Cereblon WT GST-tagged protein solution was added to each well. Finally, 5 µL of the thoroughly mixed Thalidomide-Re reagent and GST Eu antibody working solution were added to each of the aforementioned wells. The final concentration of the tested compounds and lenalidomide in each well was 2 µM.
3. The blank control wells were sequentially added with 2.5 µL of diluent #9 (1X) solution, 2.5 µL of PROTAC binding buffer, and 5 µL of thoroughly mixed Thalidomide-Re reagent and GST Eu antibody working solution.
**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).
The corresponding CRBN binding inhibition rate was calculated using the following method: ${R}_{compound} = OD 665 {nm}_{compound} / OD 620 {nm}_{compound} - OD 665 {nm}_{control} / OD 620 {nm}_{control}$ ${R}_{Std 0} = OD 665 {nm}_{Std 0} / OD 620 {nm}_{Std 0} - OD 665 {nm}_{control} / OD 620 {nm}_{control}$ $inhibition rate \left(%\right) = \left(1-{R}_{compound}/{R}_{Std 0}\right) * 100$
Note: OD 665 nm _{compound} is the absorbance value of each well of the compounds to be tested of the present disclosure at an emission wavelength of 665 nm.
OD 620 nm _{compound} is the absorbance value of each well of the compounds to be tested of the present disclosure at an emission wavelength of 620 nm.
OD 665 nm _{control} is the absorbance value of the blank control well at an emission wavelength of 665 nm.
OD 620 nm _{control} is the absorbance value of the blank control well at an emission wavelength of 620 nm.
OD 665 nm _{Std0} is the absorbance value of the solvent control well at an emission wavelength of 665 nm.
OD 620 nm _{Std0} is the absorbance value of the solvent control well at an emission wavelength of 620 nm.

The test results were shown in Tables III-1 and III-2 below.

**Table III-1. HTRF inhibitory activity (IC₅₀, µM) of the compounds of the present disclosure (including but not limited to the compounds in Tables 1, 2, and the Examples compounds)**

| Comp. No. | HTRF IC₅₀ (µM) | Comp. No. | HTRF IC₅₀ (µM) |
|---|---|---|---|
| Lenalidomide | e | GT-05566 | a |
| GT-06977 | a | GT-05563 | a |
| GT-06981 | a | | |

| | | | |
|---|---|---|---|
| Note: in Table III-1, the symbols a, b, c, d, and e are defined as follows: 0<a≤0.5µM, 0.5µM<b≤1µM, 1µM<c≤1.5µM, 1.5µM<d<1.9µM, 1.9µM ≤ e ≤10µM, 10µM < f. | | | |

**Table III-2: Screening of CRBN Binding Affinity of the Compounds of the Present Invention (including but not limited to the compounds in Tables 1 and 2, and Examples Compounds) at Concentrations of 2 µM and 0.5 µM**

| Comp. No. | Inhibitio n Rate at 2 µM (%) | Inhibition Rate at 0.5 µM (%) | Comp. No. | Inhibitio n Rate at 2 µM (%) | Inhibitio n Rate at 0.5 µM (%) | Comp. No. | Inhibitio n Rate at 2 µM (%) | Inhibitio n Rate at 0.5 µM (%) |
|---|---|---|---|---|---|---|---|---|
| Lenalidomid e | a4 | a2 | GT-08682 | a7 | a6 | GT-08777 | a6 | a7 |
| alectinib | a | a | GT-08683 | a7 | a4 | GT-08778 | a7 | a7 |
| Brigatinib | a1 | a | GT-08684 | a7 | a6 | GT-08779 | a7 | a3 |
| ibrutinib | a1 | a | GT-08685 | a7 | a5 | GT-08781 | a6 | a5 |
| emavusertib | a | a | GT-08686 | a7 | a5 | GT-08783 | a6 | a4 |
| GT-05030 | a6 | a6 | GT-08687 | a6 | a3 | GT-08784 | a7 | a5 |
| GT-05097 | a7 | a4 | GT-08688 | a7 | a6 | GT-08785 | a7 | a6 |
| GT-05058 | a7 | a4 | GT-08750 | a7 | a6 | GT-08786 | a7 | a5 |
| GT-05074 | a6 | a4 | GT-08751 | a7 | a4 | GT-08787 | a7 | a6 |
| GT-05320 | a7 | a7 | GT-08752 | a7 | a5 | GT-08788 | a7 | a4 |
| GT-05098 | a7 | a7 | GT-08753 | a7 | a6 | GT-08789 | a7 | a6 |
| GT-05321 | a7 | a6 | GT-08754 | a7 | a7 | GT-08790 | a7 | a6 |
| GT-05322 | a7 | a6 | GT-08755 | a7 | a7 | GT-08791 | a7 | a4 |
| GT-05286 | a6 | a3 | GT-08756 | a7 | a3 | GT-08792 | a6 | a6 |
| GT-05287 | a7 | a5 | GT-08757 | a6 | a5 | GT-08794 | a6 | a5 |
| GT-05288 | a7 | a5 | GT-08758 | a6 | a5 | GT-08795 | a6 | a5 |
| GT-05289 | a6 | a4 | GT-08759 | a6 | a3 | GT-08796 | a7 | a7 |
| GT-05292 | a6 | a2 | GT-08760 | a5 | a3 | GT-08797 | a5 | a4 |
| GT-05293 | a6 | a4 | GT-08761 | a7 | a6 | GT-08798 | a6 | a5 |
| GT-05294 | a7 | a6 | GT-08762 | a7 | a6 | GT-08883 | a5 | a4 |
| GT-05295 | a7 | a6 | GT-08763 | a7 | a3 | GT-08885 | a6 | a4 |
| GT-05296 | a7 | a6 | GT-08764 | a6 | a6 | GT-08886 | a7 | a5 |
| GT-05297 | a7 | a6 | GT-08712 | a7 | a6 | GT-08887 | a7 | a6 |
| GT-05298 | a7 | a7 | GT-08713 | a7 | a4 | GT-08895 | a6 | a3 |
| GT-04950 | a6 | a4 | GT-08714 | a6 | a5 | GT-08896 | a7 | a5 |
| GT-08674 | a5 | a4 | GT-08765 | a7 | a4 | GT-08897 | a6 | a6 |
| GT-08675 | a7 | a7 | GT-08766 | a7 | a5 | GT-08867 | a7 | a5 |
| GT-08676 | a6 | a4 | GT-08771 | a7 | a6 | GT-08978 | a6 | a6 |
| GT-08677 | a7 | a5 | GT-08769 | a7 | a5 | GT-08979 | a6 | a5 |
| GT-08678 | a6 | a4 | GT-08770 | a6 | a2 | GT-08980 | a6 | a4 |
| GT-08679 | a7 | a6 | GT-08773 | a6 | a3 | GT-08981 | a7 | a6 |
| GT-08680 | a7 | a6 | GT-08775 | a6 | a3 | GT-08982 | a7 | a6 |
| GT-08681 | a7 | a5 | GT-08776 | a6 | a4 | GT-04950 | a6 | a4 |
| GT-09423 | a6 | a5 | GT-09424 | a6 | a4 | GT-09425 | a7 | a6 |
| GT-09427 | a7 | a6 | GT-09428 | a6 | a4 | GT-09434 | a7 | a5 |
| GT-09435 | a7 | a4 | GT-09441 | a7 | a4 | GT-09436 | a7 | a4 |
| GT-09437 | a6 | a4 | GT-09438 | a6 | a5 | GT-09413 | a8 | a6 |
| GT-09414 | a6 | a2 | GT-09415 | a7 | a5 | GT-09416 | a7 | a7 |

| | | | | | | | | |
|---|---|---|---|---|---|---|---|---|
| Note: in table III-2, the symbols a1, a2, a3, a4, a5, a6, a7, and a8 are defined as follows: 0% < a ≤ 10%, 10% < a1 ≤ 20%, 20% < a2 ≤ 30%, 30% < a3 ≤ 40%, 40% < a4 ≤ 53%, 53% < a5 ≤ 60%, 60% < a6 ≤ 80%, 80% < a7 ≤100%, 100% < a8 ≤110%. | | | | | | | | |

The results in Table III-1 and Table III-2 demonstrate that the compounds of the present invention (including but not limited to the compounds in Tables 1 and 2, and Examples) exhibit lower or comparable IC₅₀ values, or higher inhibition rates compared to lenalidomide, indicating stronger or comparable binding affinity to CRBN.

### IV. TNF-α Activity Inhibition Assay

PBMCs cells were cultured at 37°C with 5% CO₂ and seeded in 96-well plates at 1×10⁷ cells/well. The compounds to be tested (including those listed in Tables 1 and 2, and Examples compounds) were dissolved in DMSO and diluted to appropriate concentrations, ensuring that the final DMSO concentration in cell culture did not exceed 0.5%. After 1-hour incubation in medium with or without the compounds, the cells were stimulated with Lipopolysaccharide (LPS, 1 ng/ml) and continued to be cultured for 18-20 hours. Culture supernatants were then collected, diluted with serum-free medium, and analyzed for TNF-α levels using an ELISA kit. IC₅₀ values were calculated using GraphPad Prism 7.0.

TNF-α downregulation is a key mechanism for the anti-tumor action of immunomodulators. The compounds of the present disclosure demonstrated dose-dependent inhibition of TNF-α levels.

### V. Pharmacokinetic Study of the compounds of the present disclosure

The pharmacokinetic properties of the compounds of the present disclosure were evaluated using conventional pharmacokinetic experimental methods.

The test compounds of the present disclosure (including the compounds in Tables 1 and 2, and the Examples compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). The experimental animals used in this study were mice.

The experimental animals were divided into two groups: a control group (for collecting blank plasma) and an oral administration group (administered at a dose of 10 mg/kg). A test solution of the compound of the present disclosure (concentration: 1 mg/mL) was prepared by adding an appropriate amount of the test compound to a conventional vehicle (e.g., 5% DMSO + 10% Solutol + 85% saline). The solution was orally administered to the experimental animals at a dose of 10 mg/kg. Blood samples were collected at predetermined time points after administration, such as 0.25 h, 0.5 h, 1 h, 2 h, 4 h, 8 h, and 24 h post-dose. Blank plasma was collected from the control group.

Prior to analysis, plasma samples were processed as follows: To 10 µL of plasma sample were added 10 µL of 50% acetonitrile and 200 µL of internal standard solution (containing 50 ng/mL dexamethasone in acetonitrile). For blank control plasma samples, no internal standard was added, instead an equivalent volume of acetonitrile was supplemented. The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.

The results demonstrated that the compounds of the present disclosure administered via oral route exhibited favorable drug metabolism and pharmacokinetic (DMPK) properties, indicating their potential as therapeutic agents for cancer patients.

### VI. Effects of the compounds of the present disclosure on TNF-α, IL-10, IL-6, IL-1β, IFN-γ, GM-CSF, IL-2, and IL-12p40 release from PBMCs induced by Lipopolysaccharide (LPS) or Phytohemagglutinin (PHA)

The following materials were used in this study:

### VI.1 Reagents and Consumable materials

| Name | Supplier or Source |
|---|---|
| Human PBMC cell | Shanghai Sai Li |
| Lipopolysaccharide (LPS) | Sigma |
| Phytohemagglutinin (PHA) | MCE |
| RPMI 1640 Medium | Gibco |
| Human TNF-alpha DuoSet ELISA | R&D Systems |
| Human IL-6 DuoSet ELISA | R&D Systems |
| Human IL-10 DuoSet ELISA | R&D Systems |
| Human IFN-gamma DuoSet ELISA | R&D Systems |
| Human IL-2 DuoSet ELISA | R&D Systems |
| Human IL-1 beta ELISA Kit | Abclonal |
| Human GM-CSF ELISA Kit | Abclonal |
| Human IL-12/IL-23 p40 ELISA Kit | Abclonal |

### VI.2 Methods

### VI.2.1 Cell Thawing and Plating

A complete medium was prepared and pre-warmed to 37°C. Cryovials containing the cells were retrieved from liquid nitrogen, and the area immediately below the cap-notch interface was firmly gripped with forceps. The cryovials were immersed in a 37°C water bath, and shook occasionally to facilitate thawing. The cell suspension was aspirated and transferred to a centrifuge tube containing 10 mL complete medium, followed by thorough mixing and centrifugation at 400 × g for 10 min at room temperature. The supernatant was discarded, and the pellet was resuspended in fresh complete medium. After cell counting and the cell density adjustment, the cell suspension was seeded in 96-well plates at a density of 1×10⁵ cells/well (75 µL/well) and incubated in an incubator for 2 h at 37°C with 5% CO₂ under high humidity conditions.

### VI.2.2 Compound Preparation and Addition to Cell Plates

1) The test compounds were prepared as 10 mM stock solutions in DMSO.
2) The test compounds were serially diluted 5-fold in DMSO starting from 1 mM (highest concentration) to generate 9 concentration gradients.
3) The test compounds were further diluted in culture medium to five times their final working concentration intended for use.
4) The diluted compounds were added to designated wells according to the plate layout at 25 µL/well, achieving final concentrations starting from 1 µM with 5-fold serial dilution (9 concentration points in total).
5) After 1 h incubation, LPS or PHA (25 µL/well) was added to the cell wells to reach final concentrations of: 1 µg/mL LPS (for TNF-α, IL-10, IL-6, GM-CSF and IL-12p40), 5 µg/mL LPS (IL-1β), 50 µg/mL LPS (IFN-γ), or 1 µg/mL PHA (IL-2).
6) The plates were incubated in an incubator for 24 h at 37°C with 5% CO₂ under high humidity. Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | H₂O | H₂O | H₂O | H₂O | H₂O | H₂O | H₂O | H₂O | H₂O | H₂O | H₂O | H₂O |
| B | H₂O | Cmpd 1# (1 µM Starting, 5-fold dilution, 9 points) | | | | | | | | | LPS/PHA | H₂O |
| C | H₂O | | | | | | | | | | | H₂O |
| D | H₂O | Cmpd 2# (1 µM Starting, 5-fold dilution, 9 points) | | | | | | | | | | H₂O |
| E | H₂O | | | | | | | | | | | H₂O |
| F | H₂O | Cmpd 3# (1 µM Starting, 5-fold dilution, 9 points) | | | | | | | | | Blank | H₂O |
| G | H₂O | | | | | | | | | | | H₂O |
| H | H₂O | H₂O | H₂O | H₂O | H₂O | H₂O | H₂O | H₂O | H₂O | H₂O | H₂O | H₂O |

### VI.2.3 Detection

(1) The cell plate was centrifuged and the culture supernatant was aspirated. TNF-α, IL-10, IL-6, IL-1β, IFN-y, GM-CSF, IL-2, and IL-12p40 cytokines in the culture supernatant were detected according to the ELISA kit instructions.
   The concentrations of TNF-α, IL-10, IL-6, IL-1β, IFN-γ, GM-CSF, IL-2, and IL-12p40 in the samples were calculated based on the standard curve. Inhibition rate % = [(Ac-As)/(Ac-Ab)]× 100%
   As: OA of the samples (cells + LPS/PHA + test compound)
   Ac: OA of positive cell control (cells + LPS/PHA + DMSO)
   Ab: OA of blank control (cells + culture medium + DMSO)
(2) IC₅₀ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform IC₅₀ curve fitting and determine the IC₅₀ values.

The experimental results demonstrated that the compounds of the present disclosure can modulate the production levels of cytokines associated with autoimmune diseases and can be used for the treatment of autoimmune diseases.

### VII. Efficacy experiment of the compounds of the present disclosure on Psoriasis-like skin lesions induced by Imiquimod in Mice

The compounds of the present disclosure (test compounds, including the compounds in Tables 1, 2, and Examples) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |

| | | | | | | |
|---|---|---|---|---|---|---|
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a vehicle control, a positive control drug, or the test compounds, according to the above table, once daily for 7 consecutive days.

All clinical symptoms of each animal were observed at the beginning and throughout the experiment. The animals' weights were measured and recorded every 2 days.

### Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by CO₂ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-α were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean ± SD. Statistical analysis between two groups was performed using SPSS, with p < 0.05 considered statistically significant.

The experimental results demonstrated that the compounds of the present disclosure exhibited ameliorative effects on psoriasis-like skin lesions on the back of mice induced by imiquimod and can be used for the treatment of psoriasis.

### VIII. Efficacy Experiment of the compounds of the present disclosure on the CIA Model in Rats Induced by Type II Collagen

The compounds of the present disclosure (test compounds, including the compounds in Tables 1, 2, and Examples) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

### Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |

| | | | | | | |
|---|---|---|---|---|---|---|
| Note: ig. = oral gavage; sc. = subcutaneous injection | | | | | | |

Administration commenced between 3 and 7 days after the second booster immunization, once the AI score of the affected paws reached 1-2. According to the table above, animals in each group received daily oral gavage (or subcutaneous injection) of either the vehicle control, positive control drug, or the test compounds for 21 consecutive days.

Beginning on day 3 after the second booster immunization, the volume of both hind paws (or toes) was measured and recorded twice weekly.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

### Scoring

Arthritis Index (AI): The foot volume was measured twice a week, and the swelling of the four toes was scored simultaneously. Each foot was scored on a scale from 0 to 4, with a maximum score of 16 for each rat.

### Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-α, IL-1β, and IL-6 in serum were measured by ELISA.

After the experiment, the animals were euthanized by CO₂ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

### Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.
Scoring and Counting Method: 0, 1, 2, 3, and 4 levels, where "0" indicates no observation; "1" is mild; "2" is moderate; "3" is severe; and "4" is extremely severe.

Experimental data were presented as Mean ± SEM. Data between two groups were analyzed using Excel-T test, with p<0.05 considered statistically significant. Scoring data were analyzed using the non-parametric Mann-Whitney U test in SPSS, with p<0.05 considered statistically significant.

The experimental results demonstrated that the compounds of the present disclosure regulated the serum inflammatory cytokines levels in the bovine type II collagen-induced rat model of collagen-induced arthritis (CIA), significantly ameliorated limb swelling in the rats, and thus can be applicable for the treatment of arthritis.

The above descriptions represent only preferred embodiments of the present invention, but the scope of protection is not limited thereto. Any person skilled in the art, within the technical scope disclosed by the invention, may make equivalent replacements or modifications based on the technical solutions and inventive concepts of the present invention, and such changes shall fall within the protection scope of the invention.

## Claims

1. A compound of Formula (I)
PBM-LIN-ULM (I)
or salts, enantiomers, stereoisomers, solvates, isotopically enriched analogs, prodrugs, or polymorphs thereof,
wherein PBM represents a protein-binding moiety capable of binding to a target protein, and is covalently linked to ULM through LIN;
ULM represents a ligand moiety capable of binding to an E3 ubiquitin ligase, and represents the structure of the following Formula (ULM): 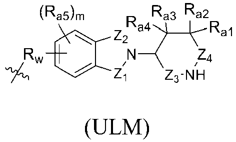
wherein Z₁ represents C(O), C(S), CH₂ or CD₂; Z₂, Z₃, and Z₄ each independently represent C(O) or C(S), wherein at least one of Z₁, Z₂, Z₃, and Z₄ represents C(S);
Rₐ₁, Rₐ₂, Rₐ₃, and Rₐ₄ are identical or different and each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy or halogenated C₁₋₆ alkoxy;
(Rₐ₅)ₘ indicates that the isoindoline ring to which it is attached is optionally substituted with m Rₐ₅,
with each Rₐ₅ being identical or different and each independently representing halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2 or 3;
R_{w} represents O, S, S(O), S(O)₂, alkenylene, alkynylene or N(Rₐ₆), wherein Rₐ₆ represents H or C₁₋₃ alkyl; or R_{w} represents a bond; or
R_{w} represents: 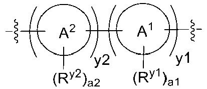
wherein each ring A¹ is identical or different and each independently represents nitrogen-containing heterocyclylene, arylene or heteroarylene, y1 represents an integer of 1 or 2, and (R^{y1})ₐ₁ indicates that each ring A¹ is independently optionally substituted with a1 R^{y1} groups, with each R^{y1} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a1 represents an integer of 0-20;
each ring A² is identical or different and each independently represents heterocyclylene, cycloalkylene, arylene or heteroarylene, y2 represents an integer of 0 or 1, and (R^{y2})ₐ₂ indicates that each ring A² is independently optionally substituted with a2 R^{y2} groups, with each R^{y2} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a2 represents an integer of 0-20; and
LIN represents: 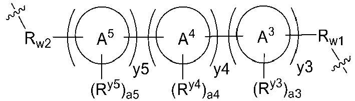
wherein R_{w2} is linked to PBM, and R_{w1} is linked to R_{w};
R_{w2} represents a bond, C(O), C(O)NH or NHC(O);
each ring A³ is identical or different and each independently represents heterocyclylene, cycloalkylene, arylene, or heteroarylene, y3 represents an integer of 0, 1, 2 or 3, and (R^{y3})ₐ₃ indicates that each ring A³ is independently optionally substituted with a3 R^{y3} substituents, wherein each R^{y3} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a3 represents an integer of 0-10;
each ring A⁴ is identical or different and each independently represents heterocyclylene, cycloalkylene, arylene, or heteroarylene, y4 represents an integer of 0, 1, 2 or 3, and (R^{y4})ₐ₄ indicates that each ring A⁴ is independently optionally substituted with a4 R^{y4} substituents, wherein each R^{y4} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a4 represents an integer of 0-10;
each ring A⁵ is identical or different and each independently represents heterocyclylene, cycloalkylene, arylene, or heteroarylene, y5 represents an integer of 0, 1, 2 or 3, and (R^{y5})ₐ₅ indicates that each ring A⁵ is independently optionally substituted with a5 R^{y5} substituents, wherein each R^{y5} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a5 represents an integer of 0-10;
R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof.

2. The compound of Formula (I) or salts, enantiomers, stereoisomers, solvates, isotopically enriched analogs, prodrugs, or polymorphs thereof as claimed in claim 1, wherein the PBM represents a small molecule ligand binding to the following protein: epidermal growth factor receptor (EGFR), Cyclin-dependent kinase 4/6 (CDK4/6), anaplastic lymphoma kinase (ALK), Focal adhesion kinase (FAK), breakpoint cluster region (BCR)-Abelson leukemia virus (ABL) (BCR-ABL) tyrosine kinase, Bruton tyrosine kinase (BTK), Androgen receptor (AR), Estrogen receptor (ER), Bromodomain and extra-terminal domain protein (BET), Interleukin-1 receptor-associated kinase 4 (IRAK4), Cyclin-dependent kinase 9 (CDK9), Histone-lysine N-methyltransferase EZH2 (EZH2), B cell lymphoma-2 (BCL-2) family proteins, Cyclin-dependent kinase 2 (CDK2), serine/threonine protein kinase (AKT), microtubule-associated protein tau, SWI/SNF related, matrix associated, actin dependent regulator of chromatin, subfamily a, member 2/4 (SMARCA2/4), or ribosomal protein S6 kinase (RSK).

3. The compound of Formula (I) or salts, enantiomers, stereoisomers, solvates, isotopically enriched analogs, prodrugs, or polymorphs thereof as claimed in claim 1, wherein said PBM comprises a structure selected from the following formula: 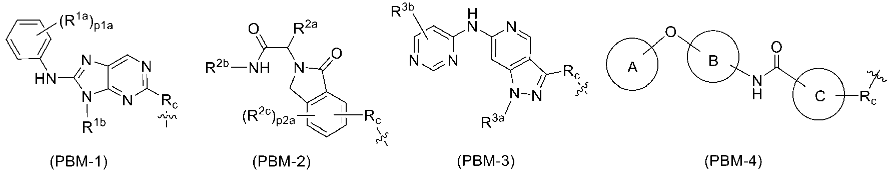 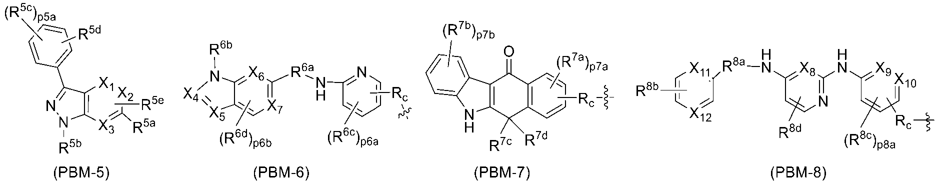 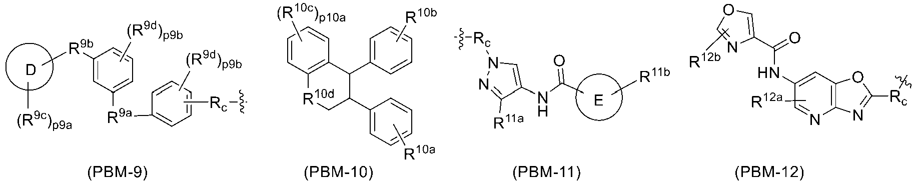 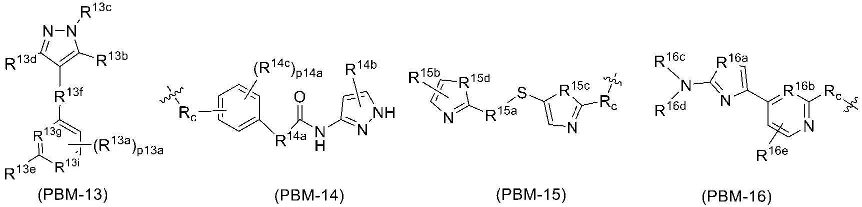 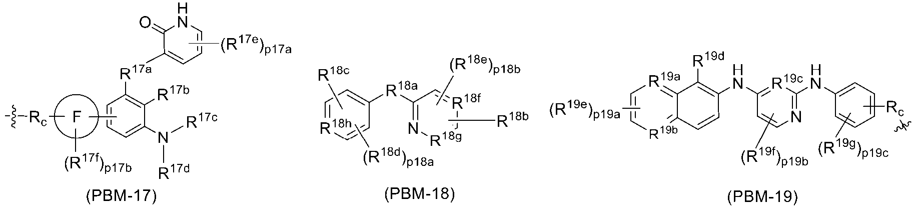 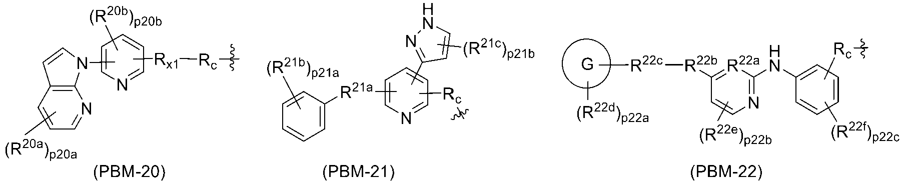 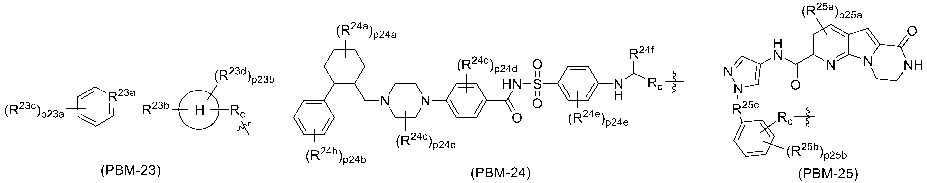 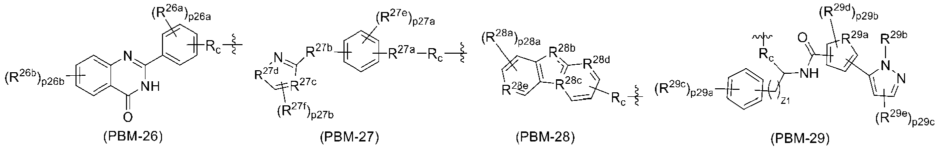 or 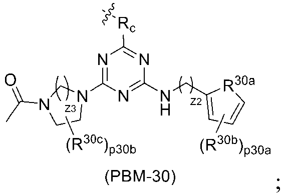
wherein (R^{1a})ₚ₁ₐ in Formula (PBM-1) indicates that the benzene ring to which it is attached is substituted with p1a R^{1a} groups, with each R^{1a} being identical or different and each independently representing deuterium, halogen, hydroxy, C₁₋₆ alkyl, deuterated C₁₋₆ alkyl, halogenated C₁₋₆ alkyl, C₁₋₆ alkoxy, halogenated C₁₋₆ alkoxy, NO₂, NH₂, or cyano;
p1a represents an integer of 0, 1, 2, 3, 4 or 5;
R^{1b} represents substituted or unsubstituted C₃₋₁₅ cycloalkyl, such as cyclopentyl;
R^{2a} in Formula (PBM-2) represents C₆₋₁₀ aryl or 5- to 20-membered heteroaryl, wherein the C₆₋₁₀ aryl and 5- to 20-membered heteroaryl are each independently optionally substituted with one or more substituents independently selected from the group consisting of deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, and any combination thereof;
R^{2b} represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), wherein the C₆₋₁₀ aryl and 5- to 20-membered heteroaryl are each independently optionally substituted with one or more R^{2d}, with each R^{2d} being identical or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
(R^{2c})ₚ₂ₐ indicates that the isoindoline ring in Formula (PBM-2) is optionally substituted with p2a R^{2c}, with each R^{2c} being identical or different and each independently representing deuterium, halogen, hydroxy, C₁₋₆ alkyl, halogenated C₁₋₆ alkyl, deuterated C₁₋₆ alkyl, NO₂, NH₂, C₁₋₆ alkoxy, halogenated C₁₋₆ alkoxy, or cyano;
p2a represents an integer of 0, 1, 2 or 3;
R^{3a} in Formula (PBM-3) represents hydrogen, C₁₋₁₀ alkyl (e.g., 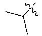 ), deuterated C₁₋₁₀ alkyl or halogenated C₁₋₁₀ alkyl;
R^{3b} represents 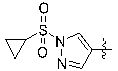
or 4- to 12-membered nitrogen-containing heterocyclyl, wherein the 4-to 12-membered nitrogen-containing heterocyclyl is optionally substituted with one or more substituents independently selected from the group consisting of deuterium, halogen, hydroxy, C₁₋₆ alkyl, halogenated C₁₋₆ alkyl, deuterated C₁₋₆ alkyl, C₁₋₆ alkoxy, halogenated C₁₋₆ alkoxy, deuterated C₁₋₆ alkoxy, NO₂, NH₂, cyano, and any combination thereof;
ring A in Formula (PBM-4) represents C₆₋₁₀ aryl or 5- to 20-membered heteroaryl (e.g., 5- to 20-membered heteroaryl containing one or two 5- to 7-membered rings and 1 to 4 heteroatoms selected from N, O, and S), and the ring A is optionally substituted with one or more R^{4a}, with each R^{4a} being identical or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, or C₂₋₆ alkynyl;
ring B in Formula (PBM-4) represents C₃₋₁₅ cycloalkyl or 4- to 20-membered heterocyclyl (e.g., 4- to 20-membered heterocyclyl containing 1 to 4 heteroatoms selected from N, O and S), wherein the ring B is optionally substituted with one or more R^{4b}, with each R^{4b} being identical or different and each independently representing deuterium, halogen, C₁₋₆ alkyl, halogenated C₁₋₆ alkyl, deuterated C₁₋₆ alkyl, C₁₋₆ alkoxy, or halogenated C₁₋₆ alkoxy;
ring C in Formula (PBM-4) represents 5- to 20-membered heteroaryl (e.g., 5- to 20-membered monocyclic or bicyclic heteroaryl containing 1 to 4 heteroatoms selected from N, O and S), wherein the ring C is optionally substituted with one or more R^{4c}, with each R^{4c} being identical or different and each independently representing deuterium, halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
X₁ in Formula (PBM-5) represents N or CR^{5f}, where R^{5f} represents hydrogen, deuterium, or amino, and X₂ represents N or CR^{5g}, where R^{5g} represents hydrogen or R_{c}, and X₃ represents CH or N;
R^{5a} and R^{5b} each independently represent hydrogen or R_{c};
(R^{5c})ₚ₅ₐ in Formula (PBM-5) indicates that the benzene ring to which it is attached is substituted with p5a R^{5c}, with each R^{5c} being identical or different and each independently representing -O-aryl, -O-heteroaryl, 4- to 20-membered heterocyclyl, -C₁₋₃ alkylene-NHC(O)-heteroaryl, -C₁₋₃ alkylene-C(O)NH-heteroaryl, C₁₋₆ alkyl, deuterium, deuterated C₁₋₆ alkyl, halogen, or halogenated C₁₋₆ alkyl, wherein the 4-to 20-membered heterocyclyl, aryl, and heteroaryl are each independently optionally substituted with one or more R^{5h}, with each R^{5h} being identical or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p5a represents an integer of 1, 2, 3 or 4;
R^{5d} represents R_{c}, hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
R^{5e} represents hydrogen, halogen, cyano, C₁₋₆ alkyl, halogenated C₁₋₆ alkyl, or amino;
wherein R^{5g}, R^{5a}, R^{5b}, and R^{5d} are not simultaneously hydrogen, and only one of R^{5g}, R^{5a}, R^{5b}, and R^{5a} represents R_{c}, wherein
when R^{5a} represents R_{c}, X₂ represents CH or N, and R^{5b} is hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
when X₂ represents CR^{5g}, where R^{5g} represents R_{c}, R^{5a} and R^{5b} are hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy;
when R^{5b} represents R_{c}, X₂ represents N or CH, and R^{5a} is hydrogen, and R^{5d} represents hydrogen, halogen, C₁₋₆ alkyl, halogenated C₁₋₆ alkyl, deuterated C₁₋₆ alkyl, halogenated C₁₋₆ alkoxy, or C₁₋₆ alkoxy; and
when R^{5d} represents R_{c}, X₂ represents N or CH, and R^{5a} and R^{5b} are hydrogen;
X₄ in Formula (PBM-6) represents CR^{6e} or a fragment 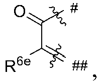
wherein symbol # indicates the point of attachment to N atom adjacent to X₄, symbol ## indicates the point of attachment to X₅, and each R^{6e} independently represents deuterium, hydrogen, halogen, C₁₋₆ alkyl, halogenated C₁₋₆ alkyl, deuterated C₁₋₆ alkyl, -C(O)-C₁₋₆ alkyl, -C(O)-deuterated C₁₋₆ alkyl or -C(O)NR^{6f}R^{6g}, where R^{6f} and R^{6g} are identical or different and each independently represent hydrogen, C₁₋₆ alkyl or deuterated C₁₋₆ alkyl;
X₅ in Formula (PBM-6) represents N or CR^{6h}, where R^{6h} represents hydrogen, deuterium, halogen, C₁₋₆ alkyl, deuterated C₁₋₆ alkyl or halogenated C₁₋₆ alkyl;
X₆ and X₇ each independently represent CH or N;
R^{6a} represents a bond or optionally substituted heteroarylene (e.g., heteroarylene substituted with halogen or deuterium);
R^{6b} represents hydrogen, C₁₋₆ alkyl, halogenated C₁₋₆ alkyl, deuterated C₁₋₆ alkyl, or optionally substituted C₃₋₁₂ cycloalkyl;
(R^{6c})ₚ₆ₐ in Formula (PBM-6) indicates that the pyridine ring to which it is attached is optionally substituted with p6a R^{6c}, with each R^{6c} being identical or different and each independently representing deuterium, halogen, C₁₋₆ alkyl, C₁₋₆ alkoxy, halogenated C₁₋₆ alkoxy, deuterated C₁₋₆ alkyl or halogenated C₁₋₆ alkyl, and p6a represents an integer of 0, 1, 2 or 3;
(R^{6d})_{p6b} in Formula (PBM-6) indicates that the nitrogen-containing bicyclic heteroaryl ring to which it is attached is optionally substituted with p6b R^{6d}, with each R^{6d} being identical or different and each independently representing deuterium, halogen, C₁₋₆ alkyl, C₁₋₆ alkoxy, halogenated C₁₋₆ alkoxy, deuterated C₁₋₆ alkyl or halogenated C₁₋₆ alkyl, and p6b represents an integer of 0 or 1;
(R^{7a})ₚ₇ₐ in Formula (PBM-7) indicates that the benzene ring to which it is attached is optionally substituted with p7a R^{7a}, with each R^{7a} being identical or different and each independently representing deuterium, cyano, halogen, C₁₋₆ alkyl, C₁₋₆ alkoxy, halogenated C₁₋₆ alkoxy, halogenated C₁₋₆ alkyl, or deuterated C₁₋₆ alkyl, and p7a represents an integer of 0, 1, 2 or 3;
(R^{7b})_{p7b} indicates that the benzene ring to which it is attached is optionally substituted with p7b R^{7b}, with each R^{7b} being identical or different and each independently representing deuterium, cyano, halogen, C₁₋₆ alkyl, C₁₋₆ alkoxy, halogenated C₁₋₆ alkoxy, halogenated C₁₋₆ alkyl, or deuterated C₁₋₆ alkyl, and p7b represents an integer of 0, 1, 2, 3 or 4;
R^{7c} and R^{7d} are identical or different and each independently represent deuterium, C₁₋₆ alkyl, halogen, halogenated C₁₋₆ alkyl, deuterated C₁₋₆ alkyl, C₁₋₆ alkoxy, or halogenated C₁₋₆ alkoxy;
X₈, X₉, X₁₀, X₁₁, and X₁₂ in Formula (PBM-8) are identical or different and each independently represent N or CH;
R^{8a} represents a bond or optionally substituted methylene (e.g., halogenated methylene);
R^{8b} represents -C(O)N(R^{8f})R^{8e}, -N(R^{8f})C(O)R^{8e}, -S(O)₂N(R^{8f})R^{8e}, -N(R^{8f})S(O)₂R^{8e}, -S(O)₂R^{8e} or - P(O)(R^{8e})₂, wherein each R^{8e} is identical or different and each independently represents C₁₋₆ alkyl or deuterated C₁₋₆ alkyl, and R^{8f} represents hydrogen or C₁₋₆ alkyl;
(R^{8c})ₚ₈ₐ in Formula (PBM-8) indicates that the 6-membered ring containing X₉ and X₁₀ to which it is attached is optionally substituted with p8a R^{8c}, with each R^{8c} being identical or different and each independently representing deuterium, halogen, hydroxy, amino, cyano, C₁₋₆ alkoxy, halogenated C₁₋₆ alkoxy, C₁₋₆ alkyl, deuterated C₁₋₆ alkyl or halogenated C₁₋₆ alkyl;
p8a represents an integer of 0, 1, 2, 3 or 4;
R^{8d} represents deuterium, halogen, hydroxy, amino, cyano, C₁₋₆ alkoxy, halogenated C₁₋₆ alkoxy, C₁₋₆ alkyl, deuterated C₁₋₆ alkyl or halogenated C₁₋₆ alkyl;
R^{9a} in Formula (PBM-9) represents -NHC(O)-, or -C(O)NH-;
R^{9b} represents -NH-, -N(C₁₋₆ alkyl)- or ethynylene; ring D in Formula (PBM-9) represents a 5- to 20-membered monocyclic, bicyclic, or polycyclic heteroaryl group containing 1 to 4 heteroatoms selected from N, O, and S, and (R^{9c})ₚ₉ₐ indicates that the ring D is optionally substituted with p9a R^{9c}, with each R^{9c} being identical or different and each independently representing optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p9a represents an integer of 0, 1, 2, or 3;
each (R^{9d})_{p9b} in Formula (PBM-9) independently indicates that the benzene ring to which it is attached is independently optionally substituted with p9b R^{9d}, with each R^{9d} being identical or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl or halogenated C₁₋₆ alkyl;
each p9b is identical or different and each independently represents an integer of 0, 1, 2, 3 or 4;
R^{10d} in Formula (PBM-10) represents O, S or CH₂;
(R^{10c})ₚ₁₀ₐ indicates that the benzo-fused six-membered ring containing R^{10d} in Formula (PBM-10) is optionally substituted with p10a R^{10c}, with each R^{10c} being identical or different and each independently representing deuterium, halogen, hydroxy, cyano, amino, C₁₋₆ alkoxy, halogenated C₁₋₆ alkoxy, C₁₋₆ alkyl, deuterated C₁₋₆ alkyl, or halogenated C₁₋₆ alkyl;
p10a represents an integer of 0, 1, 2, 3 or 4;
only one of R^{10a} and R^{10b} represents R_{c}, and the other represents hydrogen, halogen, hydroxy, cyano, amino, C₁₋₆ alkoxy, halogenated C₁₋₆ alkoxy, C₁₋₆ alkyl, deuterated C₁₋₆ alkyl or halogenated C₁₋₆ alkyl;
ring E in Formula (PBM-11) represents a 5- to 20-membered monocyclic, bicyclic, or polycyclic heteroarylene group containing 1 to 4 heteroatoms selected from N, O, and S;
R^{11b} represents optionally substituted 5- to 15-membered heteroaryl, optionally substituted 5- to 15-membered heterocyclyl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{11a} represents hydrogen, halogen, C₁₋₆ alkyl, halogenated C₁₋₆ alkyl, deuterated C₁₋₆ alkyl, C₁₋₆ alkoxy, or halogenated C₁₋₆ alkoxy;
R^{12a} in Formula (PBM-12) represents optionally substituted 5- to 15-membered heterocyclyl, optionally substituted 5- to 15-membered heteroaryl, deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
R^{12b} represents a 5- to 20-membered monocyclic, bicyclic, or polycyclic heteroaryl group containing 1 to 4 heteroatoms selected from N, O, and S, and the heteroaryl is optionally substituted with one or more substituents selected from the group consisting of deuterium, halogen, hydroxy, cyano, amino, C₁₋₆ alkyl, halogenated C₁₋₆ alkyl, deuterated C₁₋₆ alkyl, C₁₋₆ alkoxy, halogenated C₁₋₆ alkoxy, and any combination thereof;
R^{13g} in Formula (PBM-13) represents N or CR^{13h}, where R^{13h} represents hydrogen or halogen; R¹³ⁱ represents N or CR^{13j}, where R^{13j} represents hydrogen or halogen;
R^{13f} represents a bond, -C(O)NH-, -NHC(O)-, -S(O)₂NH-, or -NHS(O)₂-;
(R^{13a})ₚ₁₃ₐ indicates that the 6-membered ring containing R^{13g} and R¹³ⁱ in Formula (PBM-13) is optionally substituted with p13a R^{13a}, with each R^{13a} being identical or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl or halogenated C₁₋₆ alkyl;
p13a represents an integer of 0, 1 or 2;
R^{13b} represents hydrogen, halogen, C₁₋₆ alkyl, deuterated C₁₋₆ alkyl, halogenated C₁₋₆ alkyl or -C₁₋₃alkylene-C₃₋₆ cycloalkyl;
R^{13e} represents hydrogen, C₁₋₆ alkyl, deuterated C₁₋₆ alkyl or halogenated C₁₋₆ alkyl; or
R^{13b} and R^{13c} together with their respective attached nitrogen and carbon atoms form an optionally substituted 5- to 7-membered nitrogen-containing heterocycle;
R^{13d} represents hydrogen or R_{c}, and R^{13e} represents hydrogen or R_{c}, wherein R^{13d} and R^{13e} are not simultaneously hydrogen, and one and only one of R^{13d} and R^{13e} represents R_{c};
R^{14a} in Formula (PBM-14) represents a bond, C₁₋₃ alkylene, or halogenated C₁₋₃ alkylene;
R^{14b} represents halogen, optionally substituted C₁₋₆ alkyl, C₁₋₆ alkoxy, halogenated C₁₋₆ alkoxy, or optionally substituted C₃₋₁₅cycloalkyl;
(R^{14c})ₚ₁₄ₐ indicates that the benzene ring in Formula (PBM-14) is optionally substituted with p14a R^{14c}, with each R^{14c} being identical or different and each independently representing deuterium, halogen, hydroxy, cyano, amino, C₁₋₆ alkyl, C₁₋₆ alkoxy, halogenated C₁₋₆ alkoxy, deuterated C₁₋₆ alkyl or halogenated C₁₋₆ alkyl;
p14a represents an integer of 0, 1, 2, 3 or 4;
R^{15a} in Formula (PBM-15) represents C₁₋₃ alkylene or halogenated C₁₋₃ alkylene;
R^{15b} represents deuterium, C₁₋₆ alkyl, deuterated C₁₋₆ alkyl, halogenated C₁₋₆ alkyl, or optionally substituted C₃₋₆ cycloalkyl;
R^{15c} and R^{15d} each independently represent NH, S or O;
R^{16a} in Formula (PBM-16) represents -S- or a fragment 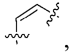
wherein when R^{16a} represents -S-, the heteroaryl containing R^{16a} and nitrogen atom in Formula (PBM-16) is thiazolyl, and when R^{16a} represents the fragment 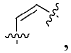 the heteroaryl containing R^{16a} and nitrogen atom in Formula (PBM-16) is pyridyl;
R^{16b} represents N or CH;
R^{16c} represents hydrogen or optionally substituted C₁₋₁₀ alkyl;
R^{16d} represents hydrogen, -C₁₋₃alkylene-optionally substituted 4- to 10-membered heterocyclyl, or optionally substituted 4- to 10-membered heterocyclyl;
R^{16e} represents hydroxy, halogen, C₁₋₆ alkyl, C₁₋₆ alkoxy, halogenated C₁₋₆ alkoxy, deuterated C₁₋₆ alkyl or halogenated C₁₋₆ alkyl;
R^{17a} in Formula (PBM-17) represents C(O)NH, NHC(O), (CH₂)₁₋₂C(O)NH, (CH₂)₁₋₂NHC(O), C(O)NH(CH₂)₁₋₂, NHC(O)(CH₂)₁₋₂, S(O)₂NH or NHS(O)₂;
R^{17b} represents hydrogen, hydroxy, halogen, cyano, C₁₋₆ alkoxy, halogenated C₁₋₆ alkoxy, C₁₋₆ alkyl, deuterated C₁₋₆ alkyl or halogenated C₁₋₆ alkyl;
R^{17c} represents hydrogen, C₁₋₆ alkyl, deuterated C₁₋₆ alkyl or halogenated C₁₋₆ alkyl; or
R^{17b} and R^{17c}, together with their respective attached carbon and nitrogen atoms and the carbon atom on the benzene ring attached to the nitrogen atom, form an optionally substituted 4- to 6-membered heteroaromatic ring or an optionally substituted 4- to 6-membered heterocycle ring;
R^{17d} represents optionally substituted C₃₋₆ cycloalkyl, C₁₋₆ alkyl, deuterated C₁₋₆ alkyl, halogenated C₁₋₆ alkyl or optionally substituted 4- to 20-membered heterocyclyl;
(R^{17e})ₚ₁₇ₐ indicates that pyridin-2(1H)-one ring to which it is attached is substituted with p17a R^{17e}, with each R^{17e} being identical or different and each independently representing hydroxy, halogen, cyano, C₁₋₆ alkyl, C₁₋₆ alkoxy, halogenated C₁₋₆ alkoxy, deuterated C₁₋₆ alkyl or halogenated C₁₋₆ alkyl;
p17a represents an integer of 0, 1, 2 or 3; ring F in Formula (PBM-17) represents arylene or 5- to 20-membered monocyclic or bicyclic heteroarylene containing from 1 to 4 heteroatoms selected from the group consisting of oxygen, nitrogen, and sulfur; and (R^{17f})_{p17b} indicates that the ring F is optionally substituted with p17b R^{17f}, with each R^{17f} being identical or different and each independently representing deuterium, halogen, hydroxy, cyano, amino, C₁₋₆ alkyl, halogenated C₁₋₆ alkyl, deuterated C₁₋₆ alkyl, C₁₋₆ alkoxy, or halogenated C₁₋₆ alkoxy;
p17b represents an integer of 0, 1, 2, 3 or 4;
R^{18f}, R^{18g}, and R^{18h} in Formula (PBM-18) each independently represent CH or N;
R^{18a} represents a bond, S or NH;
R^{18b} represents R_{c}, optionally substituted cycloalkyl or optionally substituted 4- to 15-membered nitrogen-containing heterocyclyl;
R^{18c} represents R_{c}, halogen, amino, hydroxy, cyano, C₁₋₆ alkyl, C₁₋₆ alkoxy, halogenated C₁₋₆ alkoxy, deuterated C₁₋₆ alkyl, halogenated C₁₋₆ alkyl, optionally substituted cycloalkyl or optionally substituted 4-to 15-membered nitrogen-containing heterocyclyl;
wherein R^{18b} and R^{18c} do not simultaneously represent R_{c}, and one and only one of R^{18b} and R^{18c} represents represents R_{c};
(R^{18d})ₚ₁₈ₐ indicates that the 6-membered ring to which it is attached is optionally substituted with p18a R^{18d}, with each R^{18d} being identical or different and each independently representing hydroxy, amino, cyano, halogen, C₁₋₆ alkyl, C₁₋₆ alkoxy, halogenated C₁₋₆ alkoxy, deuterated C₁₋₆ alkyl or halogenated C₁₋₆ alkyl;
p18a represents an integer of 0, 1, 2, 3 or 4;
(R^{18e})_{p18b} indicates that the 6-membered nitrogen-containing heteroaryl ring to which it is attached is optionally substituted with p18b R^{18e}, with each R^{18c} being identical or different and each independently representing hydroxy, amino, cyano, halogen, C₁₋₆ alkyl, C₁₋₆ alkoxy, halogenated C₁₋₆ alkoxy, deuterated C₁₋₆ alkyl or halogenated C₁₋₆ alkyl;
p18b represents an integer of 0, 1 or 2;
R^{19a}, R^{19b}, and R^{19c} in Formula (PBM-19) each independently represent CH or N;
R^{19d} represents -C(O)NHR^{19h} -NHC(O)R^{19h}, -S(O)₂NHR^{19h}, -NHS(O)₂R^{19h}, -S(O)₂R^{19h} or - P(O)(R^{19h})₂, wherein each R^{19h} is identical or different and each independently represents C₁₋₆ alkyl, deuterated C₁₋₆ alkyl or halogenated C₁₋₆ alkyl;
(R^{19e})ₚ₁₉ₐ represents p19a R^{19e} groups, wherein each R^{19e} is identical or different and each independently represents halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p19a represents an integer of 0, 1, 2, 3 or 4;
(R^{19f})_{p19b} represents p19b R^{19f} groups, wherein each R^{19f} is identical or different and each independently represents halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p19b represents an integer of 0, 1 or 2;
(R^{19g})_{p19c} indicates that the benzene ring to which it is attached is optionally substituted with p19c R^{19g}, with each R^{19g} each independently representing halogen, hydroxy, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy;
p19c represents an integer of 0, 1, 2, 3 or 4;
Rₓ₁ in Formula (PBM-20) represents a bond or C(O), or Rₓ₁ represents -C(O)NH-Rₓ₂-C(O)-***, wherein symbol *** indicates the point of attachment to R_{c}, and Rₓ₂ represents optionally substituted C₃₋₁₅ cycloalkyl;
(R^{20a})ₚ₂₀ₐ indicates that the 1H-pyrrolo[2,3-b]pyridine ring to which it is attached is optionally substituted with p20a R^{20a}, wherein each R^{20a} is independently cyano, halogen, hydroxy, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p20a represents an integer of 0, 1, 2, 3, 4 or 5;
(R^{20b})_{p20b} indicates that the pyridine ring to which it is attached is optionally substituted with p20b R^{20b}, wherein each R^{20b} is independently cyano, halogen, hydroxy, nitro, C₁₋₆ alkyl, halogenated C₁₋₆ alkyl, deuterated C₁₋₆ alkyl, C₁₋₆ alkoxy, or halogenated C₁₋₆ alkoxy or NR^{20c}R^{20d}, where R^{20c} and R^{20d} are identical or different and each independently represent hydrogen, C₁₋₆ alkyl, deuterated C₁₋₆ alkyl, halogenated C₁₋₆ alkyl, optionally substituted C₃₋₁₅cycloalkyl or optionally substituted 4- to 15-membered heterocyclyl, and p20b represents an integer of 0, 1, 2 or 3;
R^{21a} in Formula (PBM-21) represents NHC(O), C(O)NH, NHS(O)₂ or S(O)₂NH;
(R^{21b})ₚ₂₁ₐ indicates that the benzene ring to which it is attached is optionally substituted with p21a R^{21b} , wherein each R^{21b} is independently halogen, hydroxy, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p21a represents an integer of 0, 1, 2, 3, 4 or 5;
(R^{21c})_{p21b} indicates that the 1H-pyrazole ring to which it is attached is optionally substituted with p21b R^{21c} , wherein each R^{21c} is independently halogen, hydroxy, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p21b represents an integer of 0, 1 or 2;
R^{22a} in Formula (PBM-22) represents N or CH, and R^{22b} represents O, S, NH, CH₂, CH(F) or C(F)₂; R^{22c} represents a bond or optionally substituted methylene (e.g., halogenated methylene); ring G represents C₆₋₁₅ aryl, 4- to 15-membered heterocyclyl, or 5- to 15-membered heteroaryl, and (R^{22d})ₚ₂₂ₐ indicates that the ring G to which it is attached is optionally substituted with p22a R^{22d} , wherein each R^{22d} is identical or different and each independently represents deuterium, halogen, hydroxy, cyano, amino, nitro, oxo, C₁₋₆ alkyl, halogenated C₁₋₆ alkyl, deuterated C₁₋₆ alkyl, C₁₋₆ alkoxy, or halogenated C₁₋₆ alkoxy, and p22a represents an integer of 0, 1, 2, 3 or 4;
(R^{22e})_{p22b} indicates that the 6-membered nitrogen-containing heteroaromatic ring containing R^{22a} to which it is attached is optionally substituted with p22b R^{22e} , wherein each R^{22e} independently represents deuterium, halogen, hydroxy, cyano, amino, nitro, C₁₋₆ alkoxy, halogenated C₁₋₆ alkoxy, C₁₋₆ alkyl, deuterated C₁₋₆ alkyl or halogenated C₁₋₆ alkyl, and p22b represents an integer of 0, 1 or 2;
(R^{22f})_{p22c} indicates that the benzene ring to which it is attached is optionally substituted with p22c R^{22f} , wherein each R^{22f} is identical or different and each independently represents deuterium, halogen, hydroxy, cyano, amino, nitro, C₁₋₆ alkoxy, halogenated C₁₋₆ alkoxy, C₁₋₆ alkyl, deuterated C₁₋₆ alkyl or halogenated C₁₋₆ alkyl, and p22c represents an integer of 0, 1, 2, 3 or 4;
R^{23a} in Formula (PBM-23) represents N or CH;
R^{23b} represents NHC(O), C(O)NH, NHS(O)₂ or S(O)₂NH;
(R^{23c})ₚ₂₃ₐ indicates that the 6-membered ring containing R^{23a} is substituted with p23a R^{23c} , wherein each R^{23c} is identical or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p23a represents an integer of 0, 1, 2, 3 or 4;
ring H represents 5- to 15-membered heteroarylene, and (R^{23d})_{p23b} indicates that the ring H to which it is attached is optionally substituted with p23b R^{23d} , wherein each R^{23d} is identical or different and each independently represents halogen, hydroxy, mercapto, cyano, amino, nitro, C₁₋₆ alkyl, halogenated C₁₋₆ alkyl, deuterated C₁₋₆ alkyl, hydroxy-substituted C₁₋₆ alkyl, amino-substituted C₁₋₆ alkyl, C₁₋₆ alkoxy, halogenated C₁₋₆ alkoxy or optionally substituted C₃₋₁₅cycloalkyl, and p23b represents an integer of 0, 1, 2 or 3;
the dashed line --- in Formula (PBM-24) indicates the optional presence of a double bond;
(R^{24a})ₚ₂₄ₐ indicates that the 6-membered cycloalkyl ring to which it is attached is optionally substituted with p24a R^{24a} , wherein each R^{24a} is identical or different and each independently represents halogen, hydroxy, mercapto, cyano, amino, nitro, oxo, C₁₋₆ alkyl, halogenated C₁₋₆ alkyl, deuterated C₁₋₆ alkyl, C₁₋₆ alkoxy or halogenated C₁₋₆ alkoxy, and p24a represents an integer of 0, 1, 2, 3, 4, 5, 6, 7 or 8;
(R^{24b})_{p24b} indicates that the benzene ring to which it is attached is optionally substituted with p24b R^{24b} , wherein each R^{24b} is identical or different and each independently represents deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p24b represents an integer of 0, 1, 2, 3, 4 or 5;
(R^{24c})_{p24c} indicates that the 6-membered cycloalkyl to which it is attached is optionally substituted with p24c R^{24c} , wherein each R^{24c} is identical or different and each independently represents halogen, cyano, oxo, C₁₋₆ alkyl, halogenated C₁₋₆ alkyl, deuterated C₁₋₆ alkyl, C₁₋₆ alkoxy or halogenated C₁₋₆ alkoxy, and p24c represents an integer of 0, 1, 2, 3, 4, 5, 6, 7 or 8;
(R^{24d})_{p24d} indicates that the benzene ring to which it is attached is optionally substituted with p24d R^{24d} , wherein each R^{24d} is identical or different and each independently represents halogen, hydroxy, mercapto, C₁₋₆ alkyl, halogenated C₁₋₆ alkyl, deuterated C₁₋₆ alkyl, C₁₋₆ alkoxy, halogenated C₁₋₆ alkoxy or 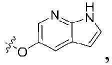 and p24d represents an integer of 0, 1 or 2;
(R^{24e})ₚ₂₄ₑ indicates that the benzene ring to which it is attached is optionally substituted with p24e R^{24e} , wherein each R^{24e} is identical or different and each independently represents deuterium, halogen, hydroxy, mercapto, cyano, amino, nitro, -SO₂CF₃, C₁₋₆ alkyl, halogenated C₁₋₆ alkyl, deuterated C₁₋₆ alkyl, C₁₋₆ alkoxy or halogenated C₁₋₆ alkoxy, and p24e represents an integer of 0, 1, 2, 3 or 4;
R^{24f} represents hydrogen, deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, halogenated C₁₋₆ alkoxy or 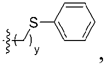 wherein y represents an integer of 1, 2 or 3;
(R^{25a})ₚ₂₅ₐ in Formula (PBM-25) represents p25a R^{25a} groups, wherein each R^{25a} is identical or different and each independently represents halogen, hydroxy, mercapto, cyano, amino, nitro, C₁₋₆ alkyl, halogenated C₁₋₆ alkyl, deuterated C₁₋₆ alkyl, C₁₋₆ alkoxy, or halogenated C₁₋₆ alkoxy, and p25a represents an integer of 0, 1, 2, 3, 4 or 5;
(R^{25b})_{p25b} indicates that the benzene ring to which it is attached is optionally substituted with p25b R^{25h}, wherein each R^{25b} is identical or different and each independently represents deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy or halogenated C₁₋₆ alkoxy, and p25b represents an integer of 0, 1, 2, 3 or 4; and
R^{25c} represents C₁₋₆ alkylene or halogenated C₁₋₆ alkylene;
(R^{26a})ₚ₂₆ₐ in Formula (PBM-26) indicates that the benzene ring to which it is attached is optionally substituted with p26a R^{26a} , wherein each R^{26a} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p26a represents an integer of 0, 1, 2, 3 or 4;
(R^{26b})_{p26b} indicates that the 4-oxo-3,4-dihydroquinazoline ring to which it is attached is substituted with p26b R^{26b} , wherein each R^{26b} is identical or different and each independently represents halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p26b represents an integer of 1, 2, 3 or 4;
R^{27a} represents S(O)₂ or a bond;
R^{27b} represents NH, NHC(O) or C(O)NH;
R^{27c} represents CH or N;
R^{27d} represents N(R^{27h}), wherein R^{27h} represents H, -C(O)-optionally substituted aryl or -C(O)-optionally substituted heteroaryl; or
R^{27d} represents a fragment 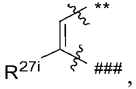
wherein symbol ** indicates the point of attachment to the adjacent nitrogen atom, symbol ### indicates the point of attachment to the adjacent carbon atom, and R²⁷ⁱ represents deuterium, hydrogen, halogen, C₁₋₆ alkyl, halogenated C₁₋₆ alkyl or deuterated C₁₋₆ alkyl;
(R^{27e})ₚ₂₇ₐ indicates that the benzene ring to which it is attached is optionally substituted with p27a R^{27e}, wherein each R^{27e} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p27a represents an integer of 0, 1, 2, 3 or 4;
(R^{27f})_{p27b} indicates that the nitrogen-containing ring to which it is attached is substituted by p27b R^{27f}, wherein each R^{27f} is identical or different and each independently represents deuterium, amino, halogen, hydroxy, cyano, C₁₋₆ alkoxy, halogenated C₁₋₆ alkoxy, C₁₋₆ alkyl, deuterated C₁₋₆ alkyl, halogenated C₁₋₆ alkyl, -NH-optionally substituted aryl, or -NH-optionally substituted heteroaryl;
p27b represents an integer of 1 or 2;
(R^{28a})ₚ₂₈ₐ in Formula (PBM-28) indicates that the fused tricyclic ring to which it is attached is optionally substituted by p28a R^{28a} substituents, wherein each R^{28a} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl; and p28a represents an integer of 0, 1, 2, 3, 4, 5 or 6;
-̅-̅-̅ in Formula (PBM-28) represents a single bond or a double bond;
a fragment 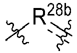 of the fused tricyclic ring in Formula (PBM-28) represents 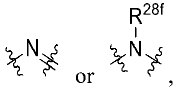 wherein R^{28f} represents hydrogen, C₁₋₆ alkyl, deuterated C₁₋₆ alkyl, or halogenated C₁₋₆ alkyl;
wherein when the fragment 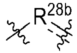 represents 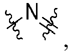 the fragment 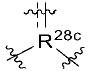 represents 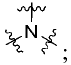
when the fragment 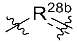 represents 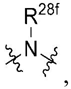 the fragment 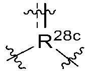 represents 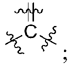
R^{28d} and R^{28e} are identical or different and each independently represent N or CH;
R^{29a} in Formula (PBM-29) represents O, S or NH;
R^{29b} represents hydrogen, C₁₋₆ alkyl, deuterated C₁₋₆ alkyl, or halogenated C₁₋₆ alkyl;
Z1 represents an integer of 1, 2 or 3;
(R^{29c})ₚ₂₉ₐ indicates that the benzene ring to which it is attached is optionally substituted with p29a R^{29c} substituents, wherein each R^{29c} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p29a represents an integer of 0, 1, 2, 3, 4 or 5;
(R^{29d})_{p29b} indicates that the 5-membered ring containing R^{29a} to which it is attached is optionally substituted with p29b R^{29d} , wherein each R^{29d} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p29b represents an integer of 0, 1 or 2;
(R^{29e})_{p29c} indicates that the pyrazole ring to which it is attached is optionally substituted with p29c R^{29e} , wherein each R^{29e} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p29c represents an integer of 0, 1 or 2;
R^{30a} in Formula (PBM-30) represents O, S or NH;
(R^{30b})ₚ₃₀ₐ indicates that the 5-membered ring containing R^{30a} to which it is attached is optionally substituted with p30a R^{30b} substituents, wherein each R^{30b} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p30a represents an integer of 0, 1, 2 or 3;
(R^{30c})_{p30b} indicates that the diazaheterocyclyl to which it is attached is optionally substituted by p30b R^{30c} substituents, wherein each R^{30c} is identical or different and each independently represents deuterium, halogen, hydroxy, amino, cyano, C₁₋₆ alkoxy, halogenated C₁₋₆ alkoxy, C₁₋₆ alkyl, deuterated C₁₋₆ alkyl, or halogenated C₁₋₆ alkyl;
p30b represents an integer of 0, 1, 2, 3, 4, 5, 6, 7, 8, 9 or 10;
Z2 represents an integer of 1, 2 or 3;
Z3 represents an integer of 1, 2, 3, 4, 5 or 6; and
R_{c} in each general formula independently represents a bond, -O-, -N(R_{d})-, -NHC(O)-*, -C(O)NH-*, - N(R_{d})-R_{f}-N(Rₑ)-*, -R_{f}-C(O)NH-*, -R_{f}-NHC(O)-*, -R_{f}-C(O)O-*, -R_{f}-OC(O)-*, -C(O)O-*, -OC(O)-*, -R_{f}-, -R_{f}-N(R_{d})-*, -O-R_{f}-N(R_{d})-*, 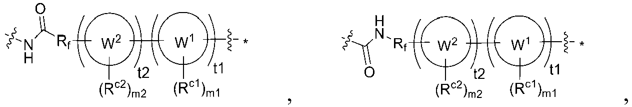 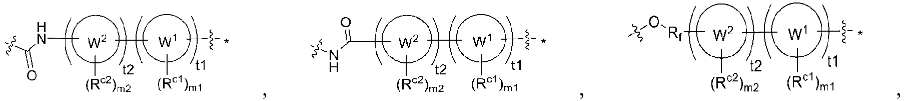 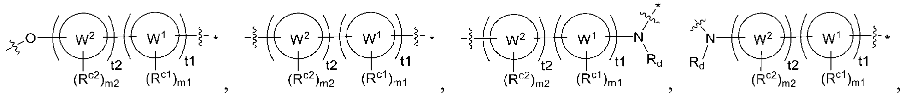 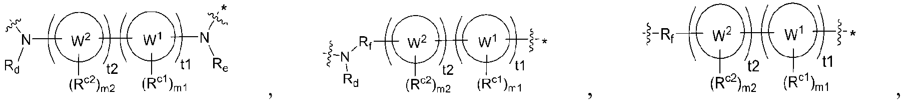 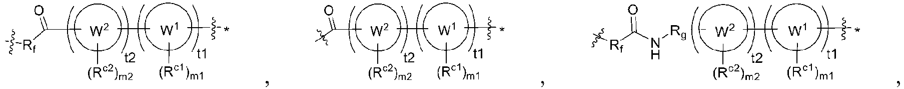 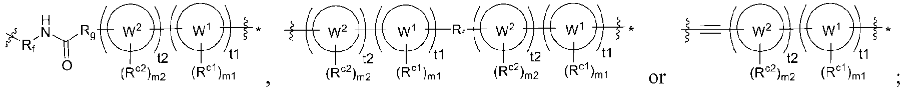
wherein each ring W¹ is identical or different and each independently represents nitrogen-containing heterocyclylene, t1 represents an integer of 1 or 2, and (R^{c1})ₘ₁ indicates that each ring W¹ is independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆ cycloalkyl, or R^{c4}-R^{c3}-, wherein R^{c3} represents optionally substituted C₁₋₆ alkylene or optionally substituted C₂₋₆ alkenylene, and R^{c4} represents halogen, R^{c5}R^{c6}N-, hydroxy, carboxyl, ethynyl, or vinyl, wherein R^{c5} and R^{c6} are identical or different and each independently represent hydrogen or C₁₋₃ alkyl, and m1 represents an integer of 0-20;
each ring W² is identical or different and each independently represents heterocyclylene, cycloalkylene, arylene, or heteroarylene, t2 represents an integer of 0 or 1, and (R^{c2})ₘ₂ indicates that each ring W² is independently optionally substituted with m2 R^{e2} groups, wherein each R^{c2} is independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, or R^{c8}-R^{c7}-, wherein R^{c7} represents optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are identical or different and each independently represent hydrogen or C₁₋₃ alkyl, and m2 represents an integer of 0-20; and
R_{d} and Rₑ each independently represent hydrogen or C₁₋₆ alkyl; R_{f} and R_{g} each independently represent optionally substituted C₁₋₆ alkylene or optionally substituted C₂₋₆ alkenylene; and symbol * indicates the point of attachment to LIN.

4. The compound of Formula (I) or salts, enantiomers, stereoisomers, solvates, isotopically enriched analogs, prodrugs, or polymorphs thereof as claimed in claim 3, wherein the PBM represents a structure of the following formulae: 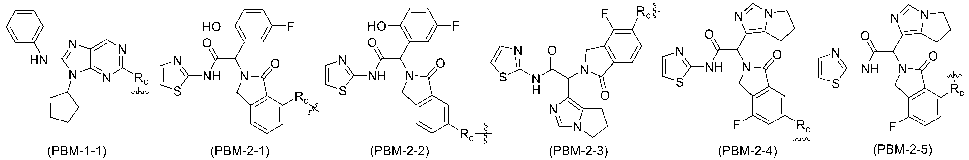 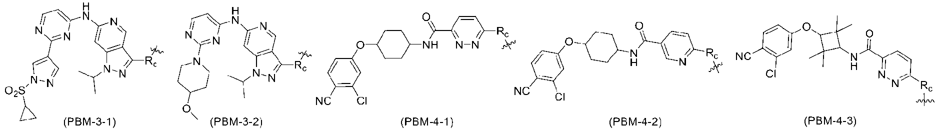 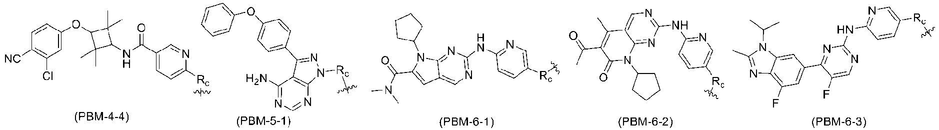 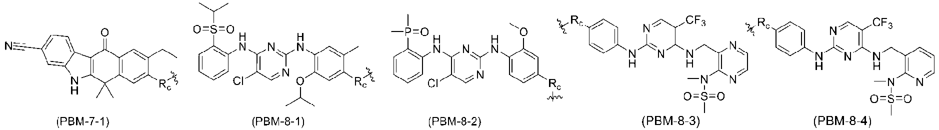 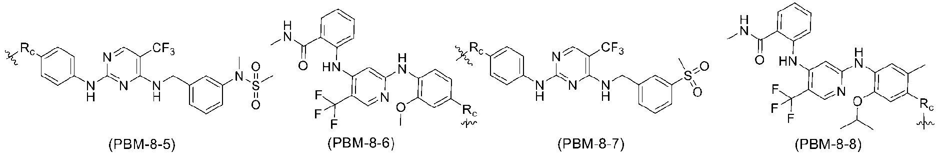 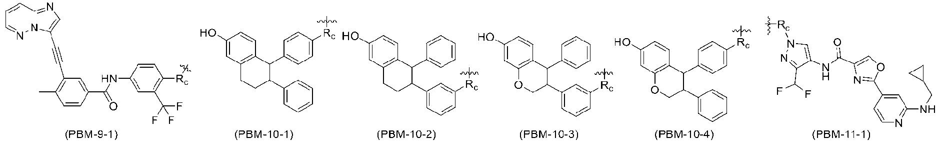 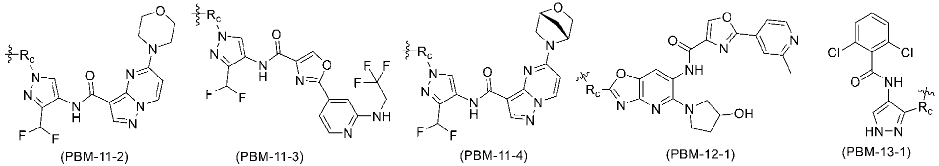 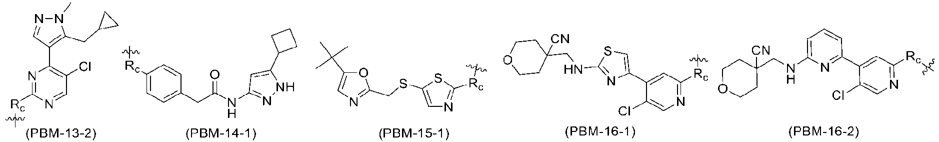 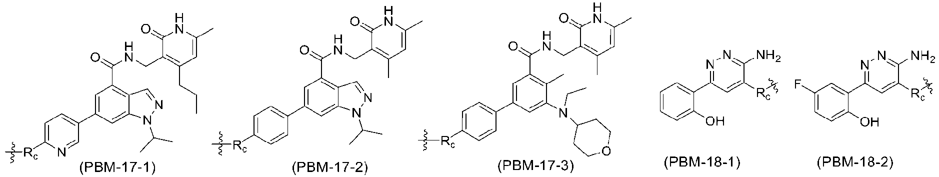 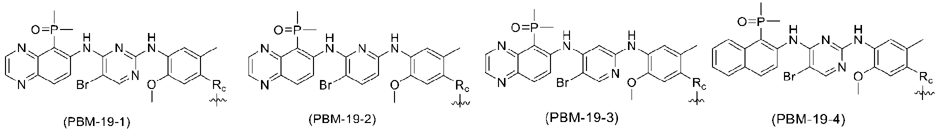 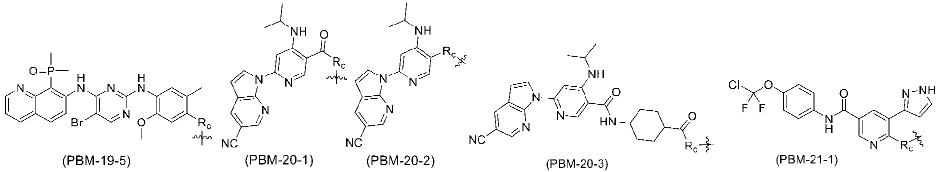 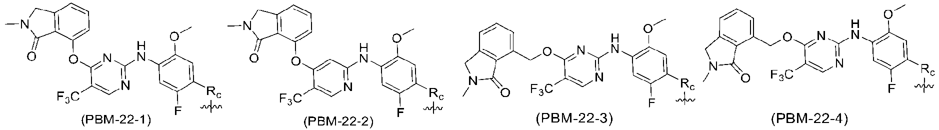 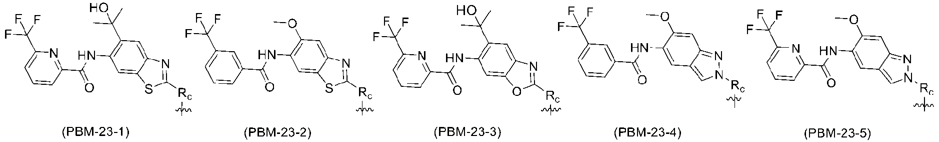 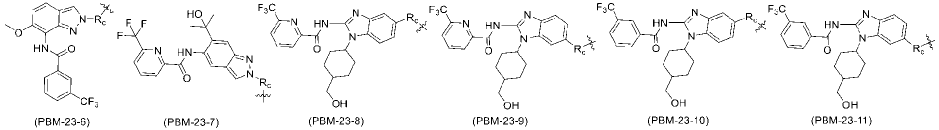 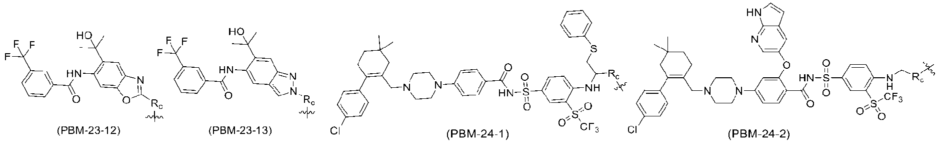 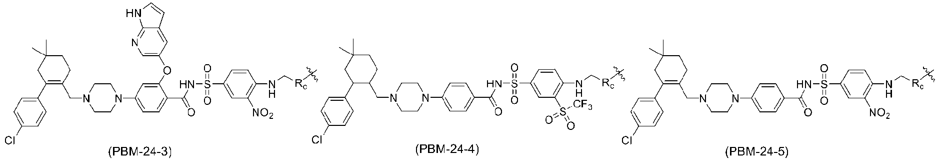 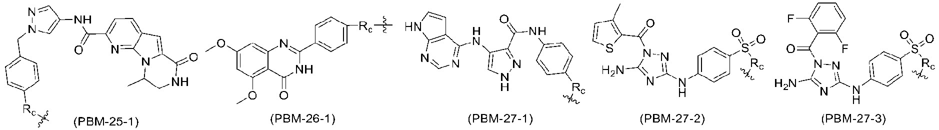 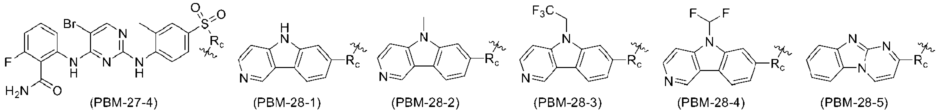 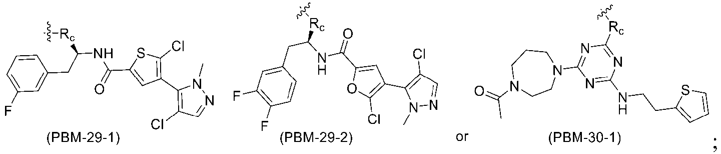 wherein R_{c} is as defined in claim 3.

5. The compound of Formula (I) or salts, enantiomers, stereoisomers, solvates, isotopically enriched analogs, prodrugs, or polymorphs thereof as claimed in claim 3, wherein
the R_{c} represents a bond, -O-, -NHC(O)-*, -C(O)NH-*, -C(O)O-*, -OC(O)-*, or -NH-, or
R_{c} represents a structure of the following formulae:
-N(R_{d})-, -N(R_{d})-R_{f}-N(Rₑ)-*, -R_{f}-C(O)NH-*, -R_{f}-NHC(O)-*, -R_{f}-C(O)O-*, -R_{f}-OC(O)-*, -R_{f}-, -R_{f}-N(R_{d})-*, -O-R_{f}-N(R_{d})-*, 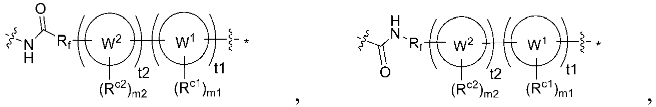 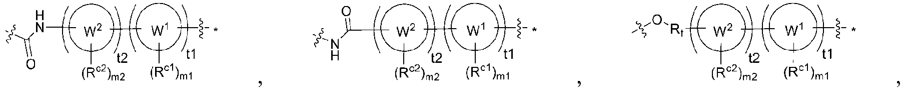 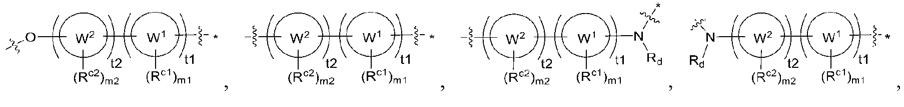 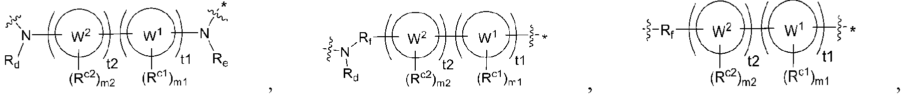 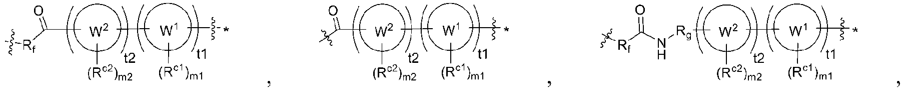 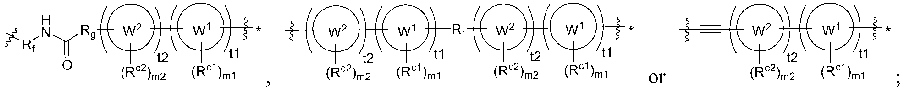
wherein each ring W¹ is identical or different and each independently represents 4- to 20-membered nitrogen-containing heterocyclylene, t1 represents an integer of 1 or 2, and (R^{c1})ₘ₁ indicates that each ring W¹ is independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, or R^{c4}-R^{c3}-, wherein R^{c3} represents optionally substituted C₁₋₆ alkylene or optionally substituted C₂₋₆ alkenylene, and R^{c4} represents halogen, R^{c5}R^{c6}N-, hydroxy, carboxyl, ethynyl, or vinyl, wherein R^{c5} and R^{c6} are identical or different and each independently represent hydrogen or C₁₋₃ alkyl, and m1 represents an integer of 0-20;
each ring W² is identical or different and each independently represents 4- to 20-membered heterocyclylene, C₃₋₂₀ cycloalkylene, C₅₋₂₀ arylene, or 5- to 20-membered heteroarylene, t2 represents an integer of 0 or 1, and (R^{c2})ₘ₂ indicates that each ring W² is independently optionally substituted with m2 R^{c2} groups, wherein each R^{c2} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, or R^{c8}-R^{c7}-, wherein R^{c7} represents optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are identical or different and each independently represent hydrogen or C₁₋₃ alkyl, and m2 represents an integer of 0-20; and
R_{d} and Rₑ each independently represent hydrogen or C₁₋₆ alkyl; R_{f} and R_{g} each independently represent optionally substituted C₁₋₆ alkylene or optionally substituted C₂₋₆ alkenylene;
the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy; and
symbol * indicates the point of attachment to LIN.

6. The compound of Formula (I) or salts, enantiomers, stereoisomers, solvates, isotopically enriched analogs, prodrugs, or polymorphs thereof as claimed in any one of claims 3-5, wherein each ring W¹ is identical or different and each independently represents 4- to 15-membered nitrogen-containing heterocyclylene, e.g.,
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, or 3-methyl-2-oxa-8-azaspiro[4.5]decanylene, or 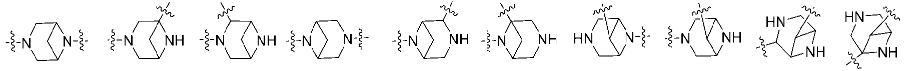 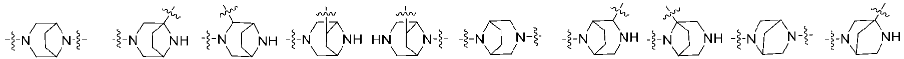 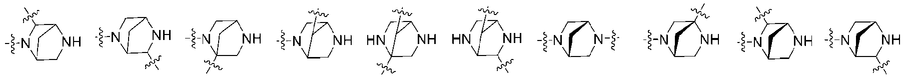 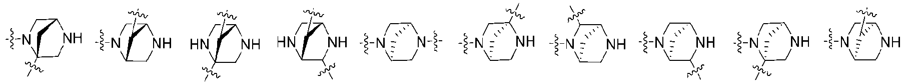 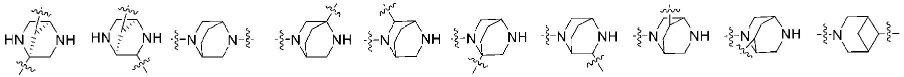 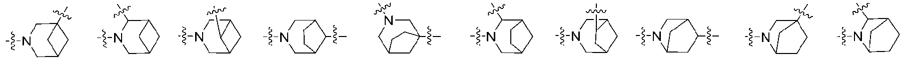 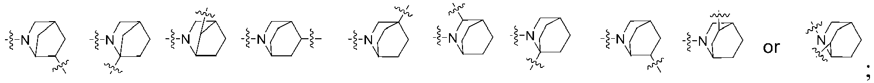
wherein each ring W¹ is each independently optionally substituted with m1 R^{c1} groups, wherein each R^{c1} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, or R^{c4}-R^{c3}-, wherein R^{c3} represents optionally substituted C₁₋₆alkylene or optionally substituted C₂₋₆alkenylene, and R^{c4} represents halogen, R^{c5}R^{c6}N-, hydroxy, carboxyl, ethynyl, or vinyl, wherein R^{c5} and R^{c6} are identical or different and each independently represent hydrogen or C₁₋₃ alkyl, and the C₁₋₆ alkylene and the C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy, and m1 represents an integer of 0-20; and/or
each ring W² is identical or different and each independently represents 4- to 15-membered nitrogen-containing heterocyclylene, C₃₋₁₅cycloalkylene, 6- to 15-membered arylene, or 5- to 15-membered heteroarylene, e.g.,
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, 3-methyl-2-oxa-8-azaspiro[4.5]decanylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 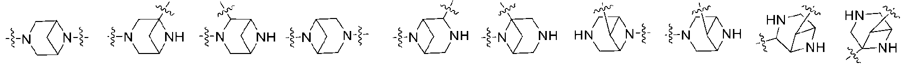 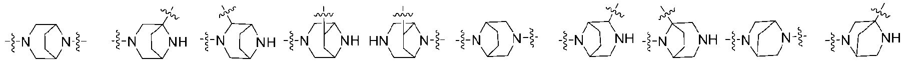 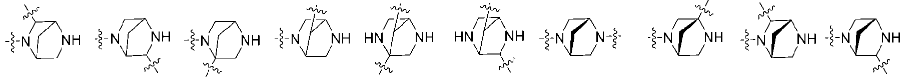 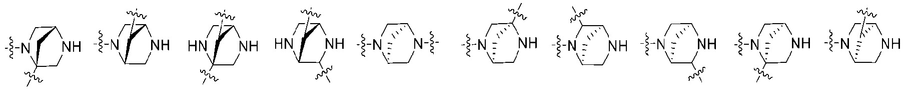 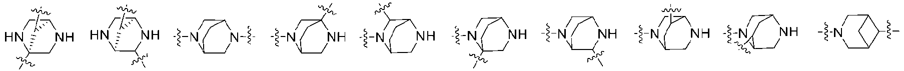 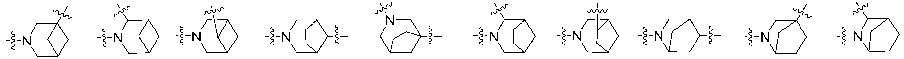 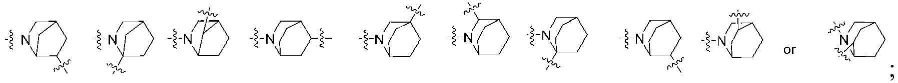
wherein each ring W² is each independently optionally substituted with m2 R^{c2} groups, wherein each R^{c2} is independently deuterium, C₁₋₆ alkyl, deuterated C₁₋₆ alkyl, halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, or R^{c8}-R^{c7}-, wherein R^{c7} represents optionally substituted C₁₋₆ alkylene or optionally substituted C₂₋₆ alkenylene, and R^{c8} represents halogen, R^{c9}R^{c10}N-, hydroxy, carboxyl, ethynyl, or vinyl, wherein R^{c9} and R^{c10} are identical or different and each independently represent hydrogen or C₁₋₃ alkyl, and the C₁₋₆ alkylene and the C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy, and m2 represents an integer of 0-20.

7. The compound of Formula (I) or salts, enantiomers, stereoisomers, solvates, isotopically enriched analogs, prodrugs, or polymorphs thereof as claimed in any one of claims 3-6, wherein the 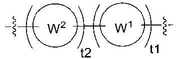 moiety of R_{c} in each general formula represents the following groups: 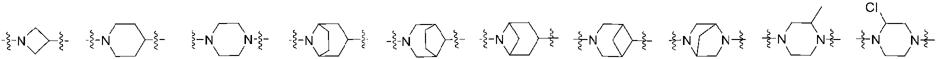 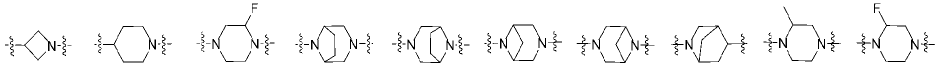 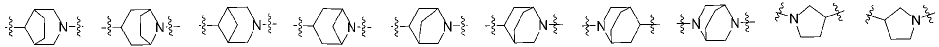 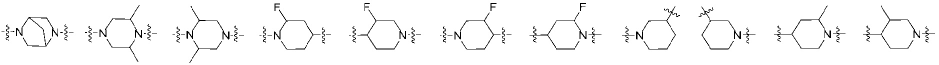 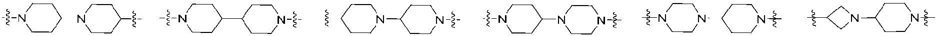 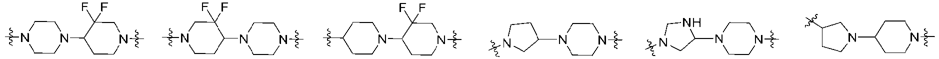 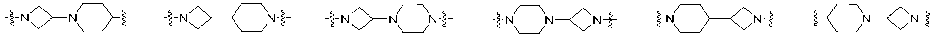 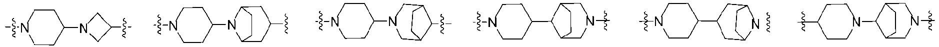 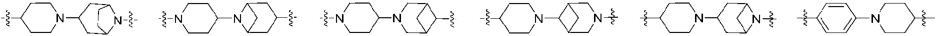 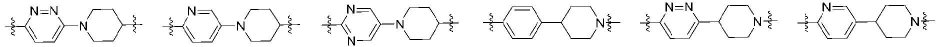 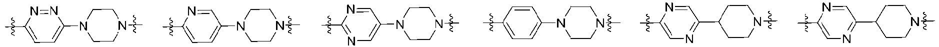 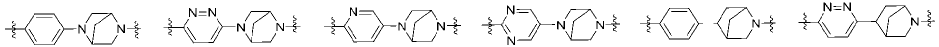 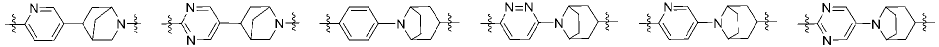 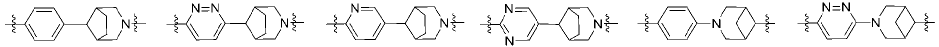 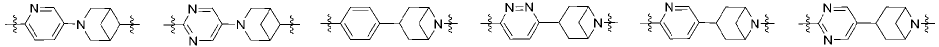 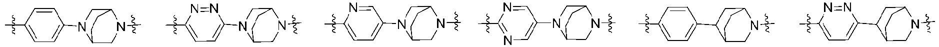 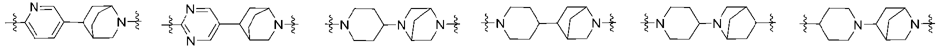 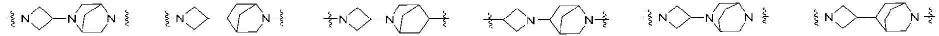 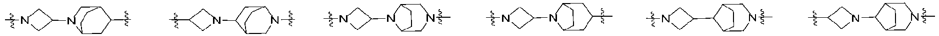 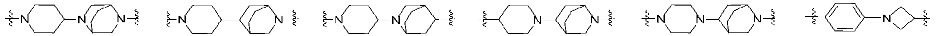 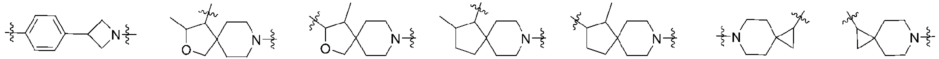 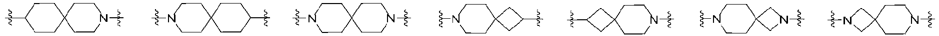 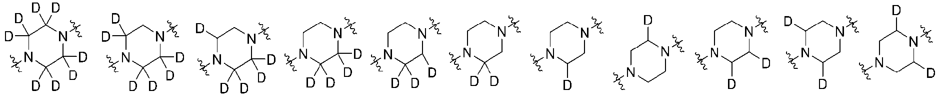 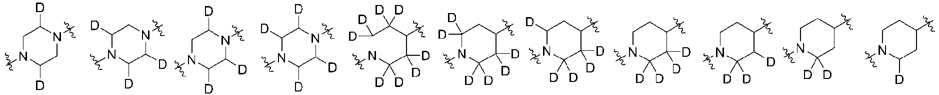 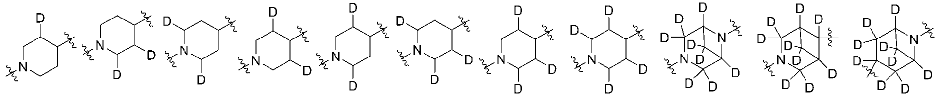 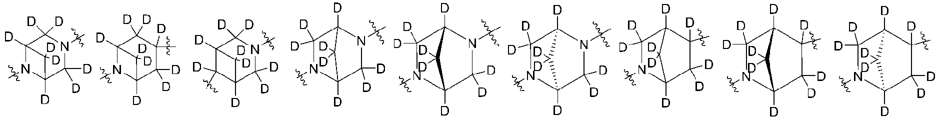 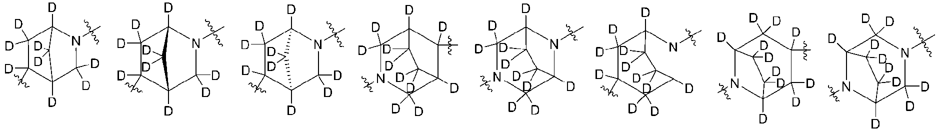 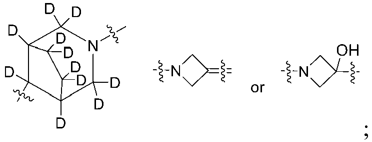 wherein the groups are each independently optionally substituted with one or more substituents selected from the group consisting of deuterium, optionally deuterated C₁₋₆ alkyl, optionally halogenated C₁₋₆ alkyl, C₁₋₆ alkoxy, halogenated C₁₋₆ alkoxy, deuterated C₁₋₆ alkoxy, halogen, amino, hydroxy, mercapto, cyano, carboxyl, C₂₋₆ alkynyl, C₂₋₆ alkenyl, optionally deuterated C₃₋₆cycloalkyl, R^{c12}-R^{c11}, and any combination thereof, wherein R^{c11} represents optionally substituted C₁₋₆ alkylene or optionally substituted C₂₋₆ alkenylene, and R^{c12} represents halogen, R^{c13}R^{c14}N-, hydroxy, carboxyl, ethynyl, or vinyl, wherein R^{c13} and R^{c14} are identical or different and each independently represent hydrogen or C₁₋₃ alkyl, and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy.

8. The compound of Formula (I) or salts, enantiomers, stereoisomers, solvates, isotopically enriched analogs, prodrugs, or polymorphs thereof as claimed in any one of claims 3-6, wherein the R_{c} represents the following groups:
a bond, -O-, -NHC(O)-*, -C(O)NH-*, -C(O)O-*, -OC(O)-*, -CH₂C(O)O-*, -CH₂OC(O)-*, -NH-, - N(CH₃)-, -N(CH₃)-(CH₂)₂-N(CH₃)-*, -N(CH₃)-(CH₂)₃-N(CH₃)-*, -NH-(CH₂)₂-N(CH₃)-*, -N(CH₃)-(CH₂)₃-NH-*, -CH₂-NHC(O)-*, -CH₂-C(O)NH-*, -CH₂-NH-*, -O-(CH₂)₂-N(CH₃)-*, -O-(CH₂)₂-NH-*, or -CH₂-N(CH₃)-*, or 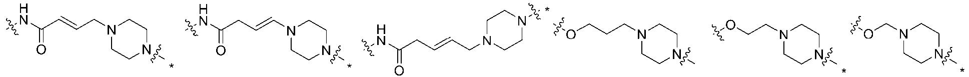 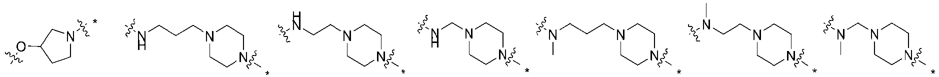 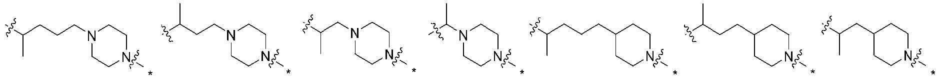 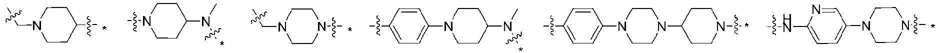 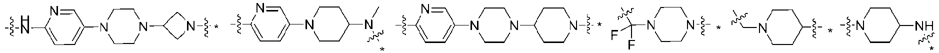 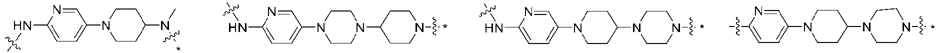 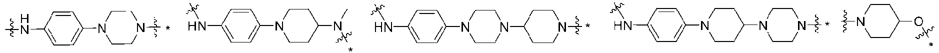 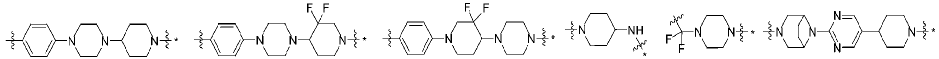 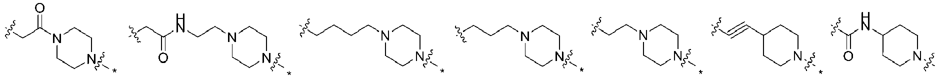 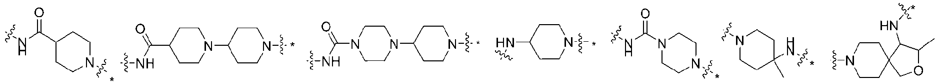 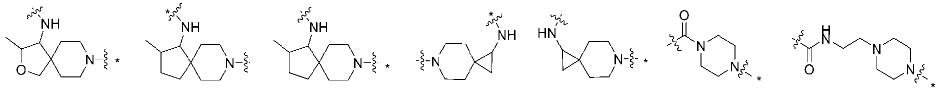 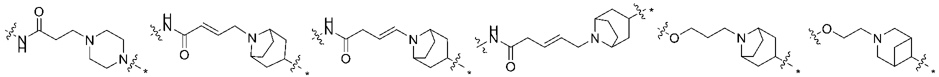 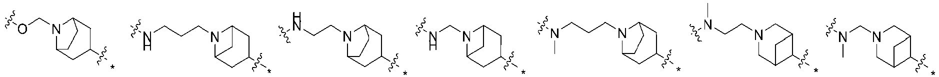 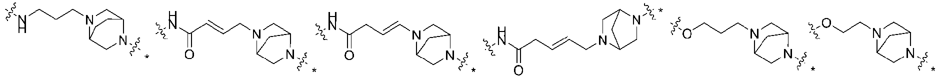 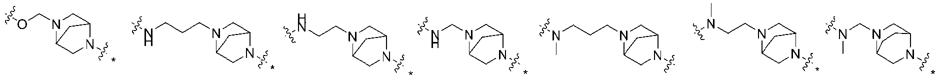 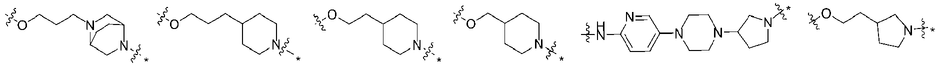 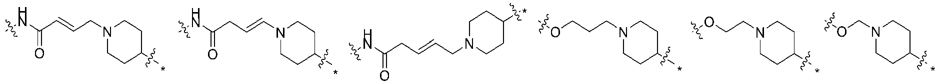 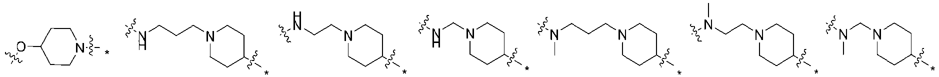 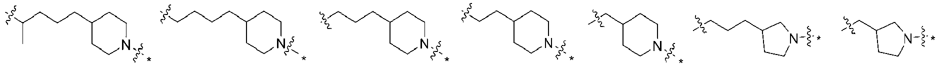 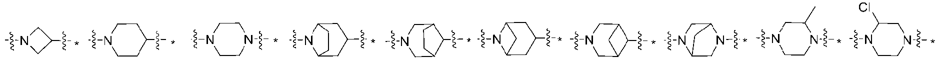 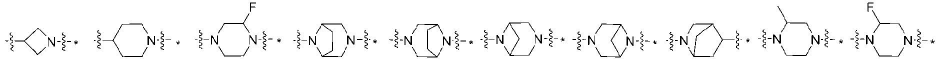 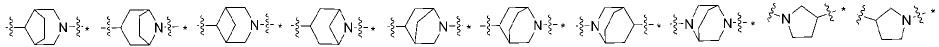 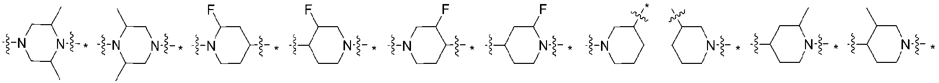 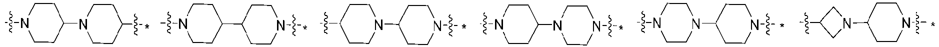 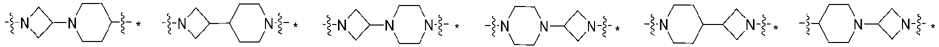 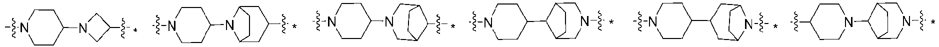 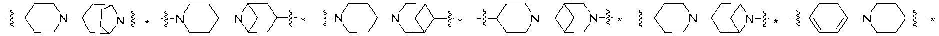 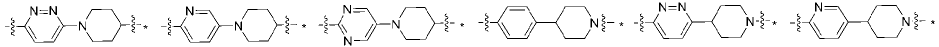 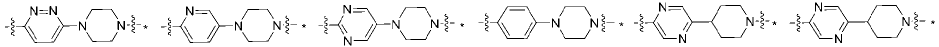 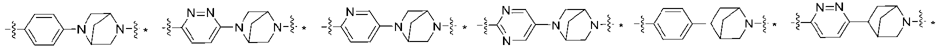 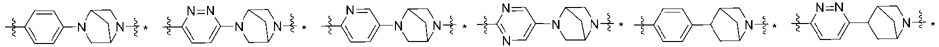 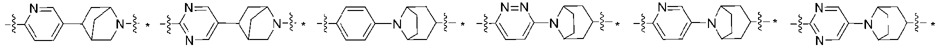 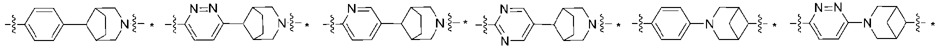 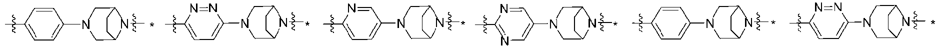 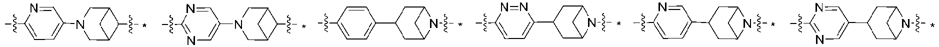 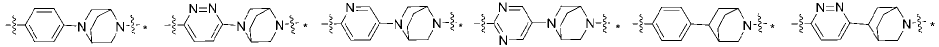 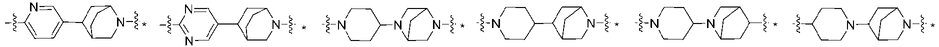 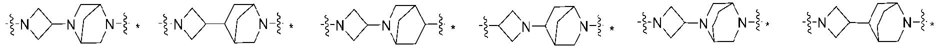 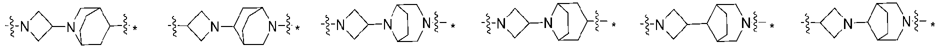 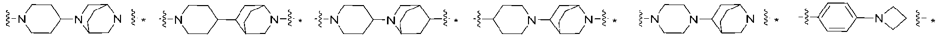 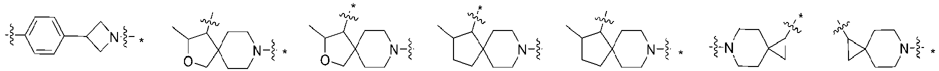 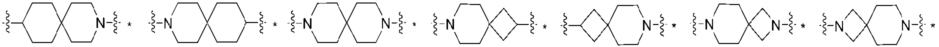 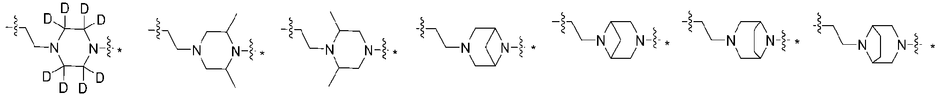 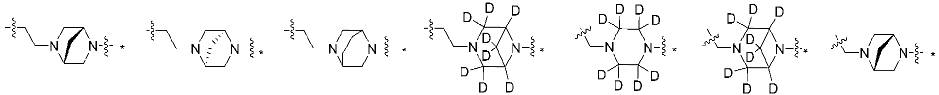 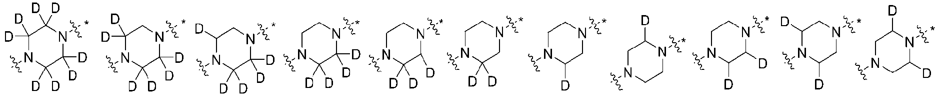 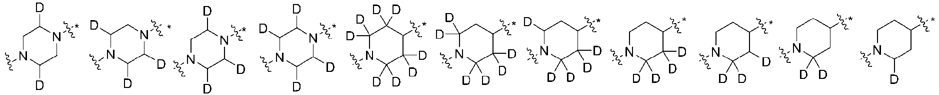 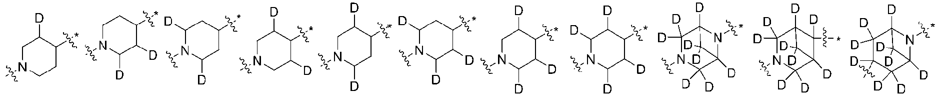 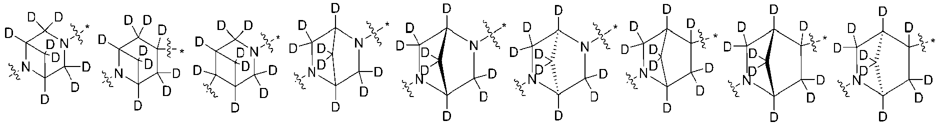 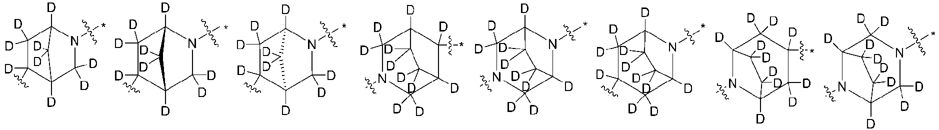 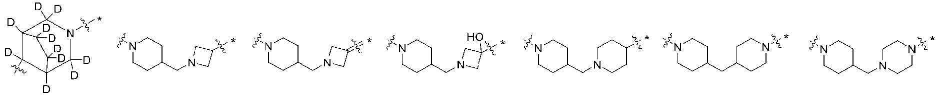 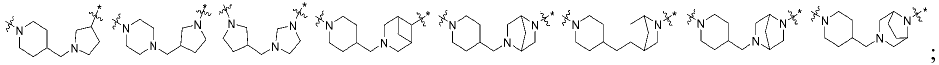 wherein the groups are each independently optionally substituted with one or more substituents selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxy, mercapto, cyano, C₂₋₆ alkynyl, C₂₋₆ alkenyl, optionally deuterated C₃₋₆ cycloalkyl, R^{c12}-R^{c11}-, and any combination thereof, wherein R^{c11} represents optionally substituted C₁₋₆ alkylene or optionally substituted C₂₋₆ alkenylene, and R^{c12} represents halogen, R^{c13}R^{c14}N-, hydroxy, carboxyl, ethynyl, or vinyl, wherein R^{c13} and R^{c14} are identical or different and each independently represent hydrogen or C₁₋₃ alkyl, and the C₁₋₆ alkylene and C₂₋₆ alkenylene are each independently optionally substituted with one or more substituents selected from the group consisting of deuterium, halogen, hydroxy, mercapto, cyano, amino, nitro, C₁₋₆ alkyl, halogenated C₁₋₆ alkyl, deuterated C₁₋₆ alkyl, C₁₋₆ alkoxy, and halogenated C₁₋₆ alkoxy; and symbol * indicates the point of attachment to LIN.

9. The compound of Formula (I) or salts, enantiomers, stereoisomers, solvates, isotopically enriched analogs, prodrugs, or polymorphs thereof as claimed in claim 1, wherein the ULM represents a structure of the following Formula (ULM-1-1), Formula (ULM-1-2), Formula (ULM-1-3), or Formula (ULM-1-4): 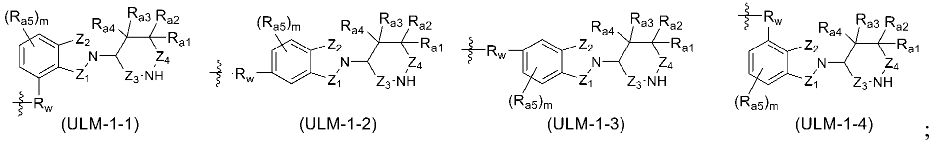
wherein Z₁ represents C(O), C(S), CH₂ or CD₂; Z₂, Z₃, and Z₄ each independently represent C(O) or C(S), wherein at least one of Z₁, Z₂, Z₃, and Z₄ represents C(S);
Rₐ₁, Rₐ₂, Rₐ₃, and Rₐ₄ are identical or different and each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy or halogenated C₁₋₆ alkoxy;
(Rₐ₅)ₘ indicates that the isoindoline ring to which it is attached is optionally substituted with m Rₐ₅,
with each Rₐ₅ being identical or different and each independently representing halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2 or 3;
R_{w} represents O, S, S(O), S(O)₂, alkenylene, alkynylene or N(Rₐ₆), wherein Rₐ₆ represents H or C₁₋₃ alkyl, or R_{w} represents a bond; or
R_{w} represents: 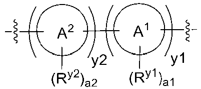
wherein each ring A¹ is identical or different and each independently represents nitrogen-containing heterocyclylene, arylene or heteroarylene, y1 represents an integer of 1 or 2, and (R^{y1})ₐ₁ indicates that each ring A¹ is independently optionally substituted with a1 R^{y1} groups, with each R^{y1} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a1 represents an integer of 0-20;
each ring A² is identical or different and each independently represent heterocyclylene, cycloalkylene, arylene or heteroarylene, y2 represents an integer of 0 or 1, and (R^{y2})ₐ₂ indicates that each ring A² is independently optionally substituted with a2 R^{y2} groups, with each R^{y2} being deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a2 represents an integer of 0-20.

10. The compound of Formula (I) or salts, enantiomers, stereoisomers, solvates, isotopically enriched analogs, prodrugs, or polymorphs thereof as claimed in claim 1 or 9, wherein the ULM represents a structure of the following Formula (ULM-2-1), Formula (ULM-2-2), Formula (ULM-2-3), or Formula (ULM-2-4): 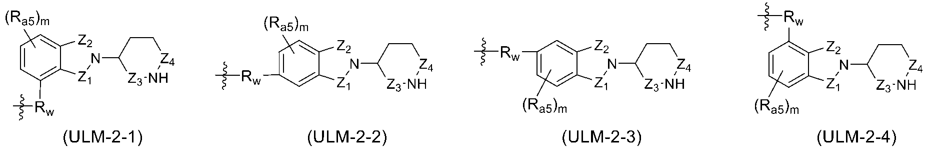 wherein Z₁, Z₂, Z₃, Z₄, (Rₐ₅)ₘ, and R_{w} are as defined in claim 1 or 9.

11. The compound of Formula (I) or salts, enantiomers, stereoisomers, solvates, isotopically enriched analogs, prodrugs, or polymorphs thereof as claimed in claim 1, wherein
(i) each ring A¹ independently represents 4- to 20-membered nitrogen-containing heterocyclylene, C₅₋₂₀ arylene or 5- to 20-membered heteroarylene; preferably each ring A¹ independently represents:
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, 3-methyl-2-oxa-8-azaspiro[4.5]decanylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 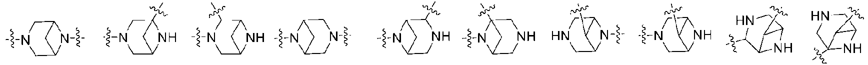 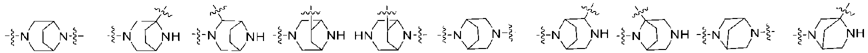 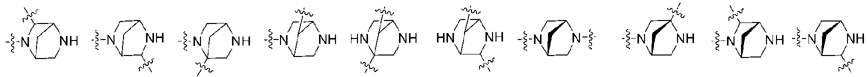 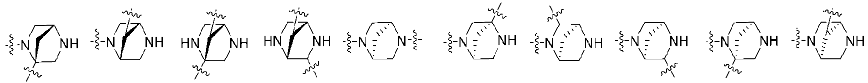 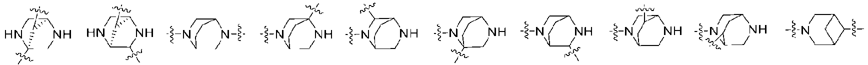 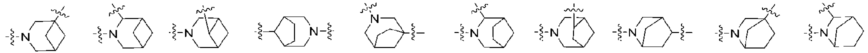 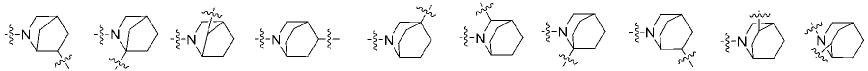 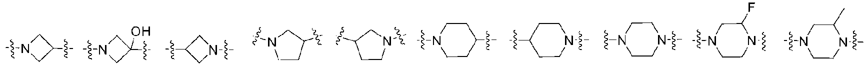 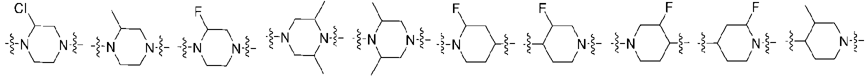 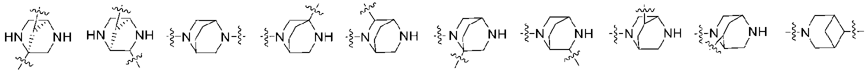 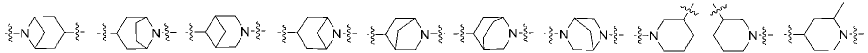 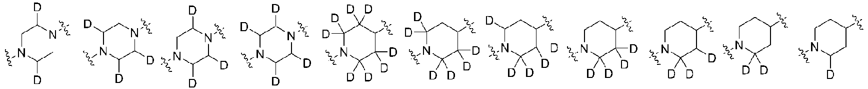 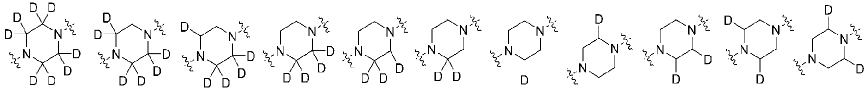 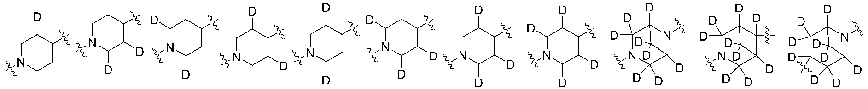 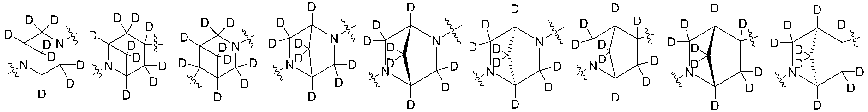 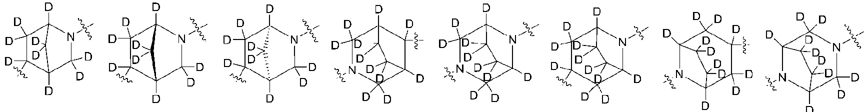 or 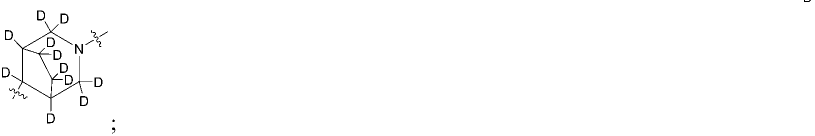
each ring A¹ is independently optionally substituted with a1 R^{y1} substituents, and each R^{y1} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl;
a1 represents an integer of 0-20; and/or
(ii) each ring A² independently represents 4- to 20-membered heterocyclylene, C₃₋₂₀ cycloalkylene, C₅₋₂₀ arylene or 5- to 20-membered heteroarylene; preferably each ring A² independently represents:
azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 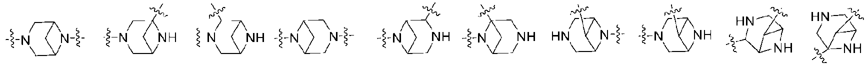 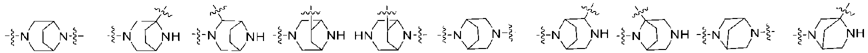 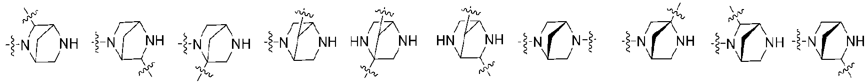 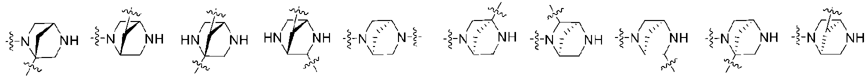 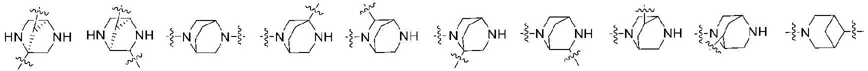 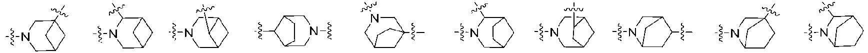 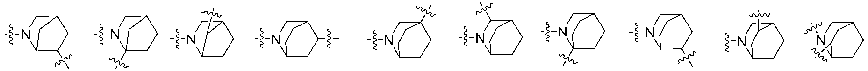 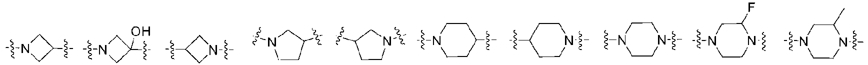 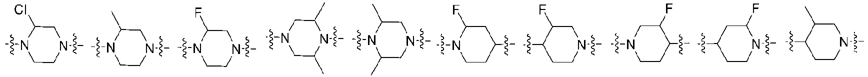 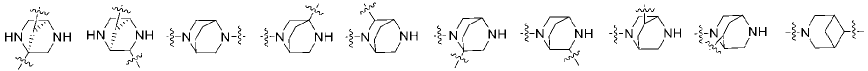 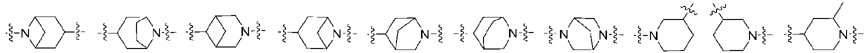 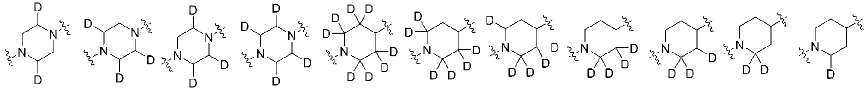 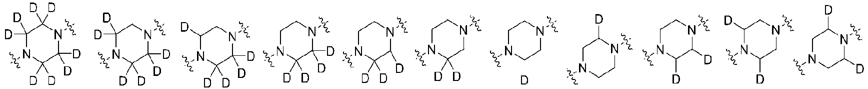 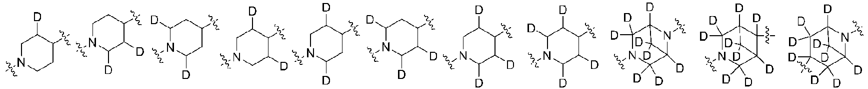 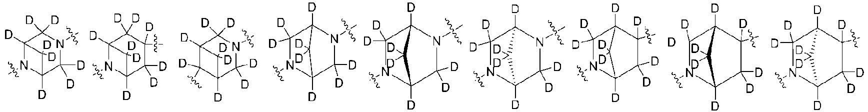 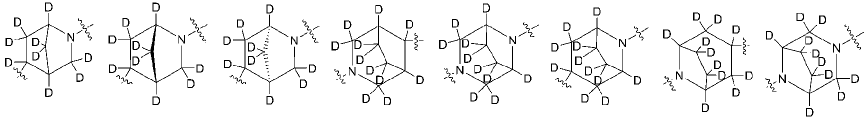 or 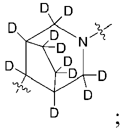
each ring A² is independently optionally substituted with a2 R^{y2} groups, wherein each R^{y2} is independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl; and
a2 represents an integer of 0-20.

12. The compound of Formula (I) or salts, enantiomers, stereoisomers, solvates, isotopically enriched analogs, prodrugs, or polymorphs thereof as claimed in claim 1 or 9, wherein R_{w} represents: 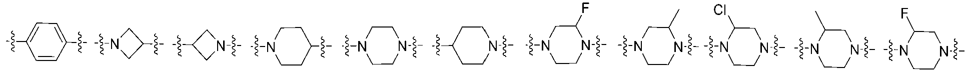 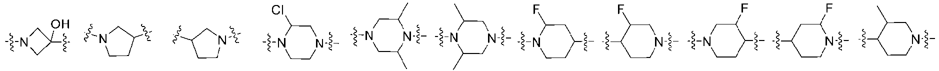 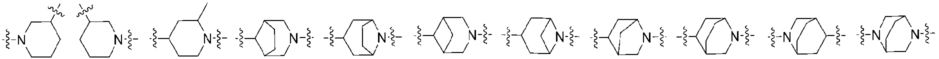 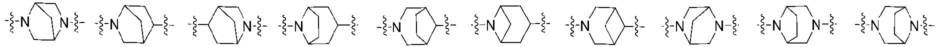 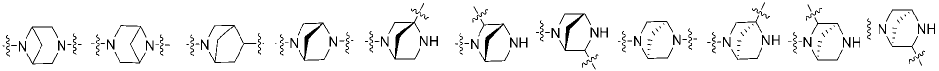 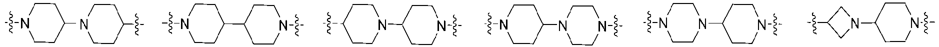 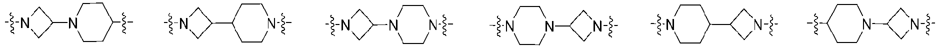 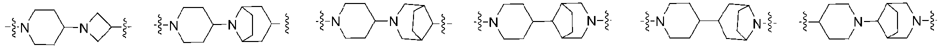 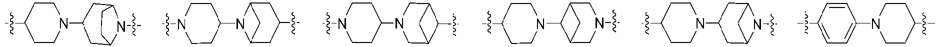 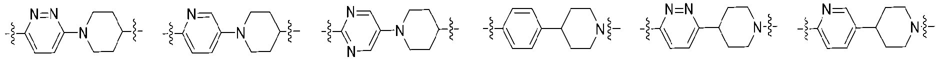 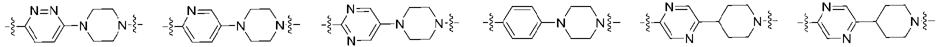 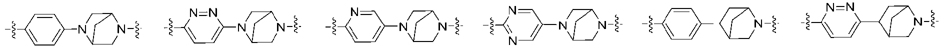 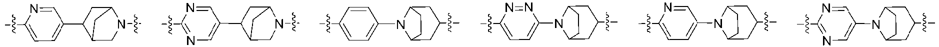 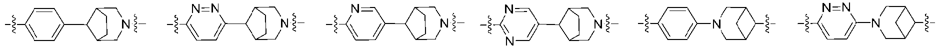 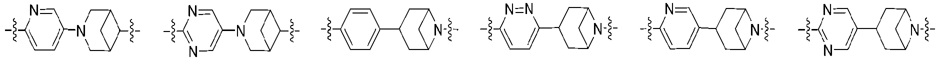 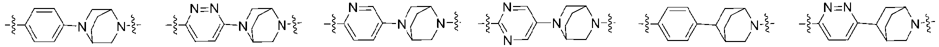 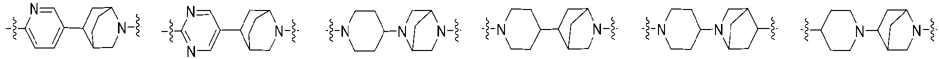 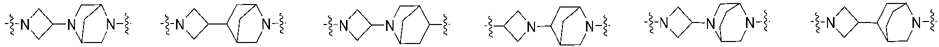 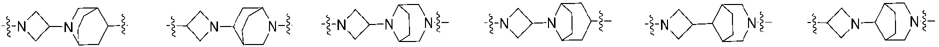 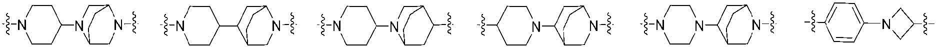 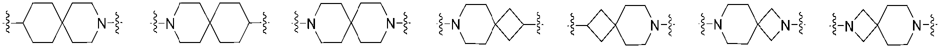 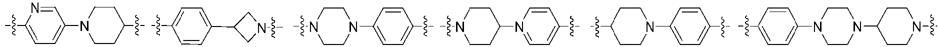 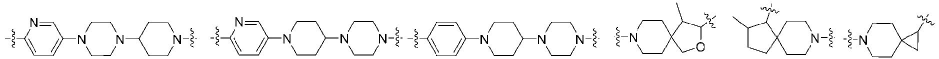 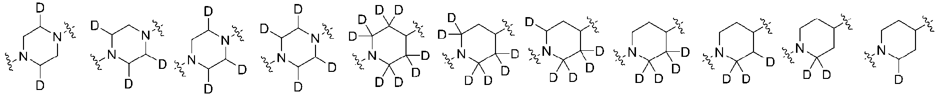 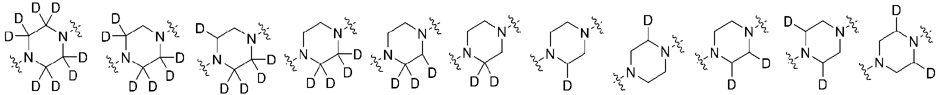 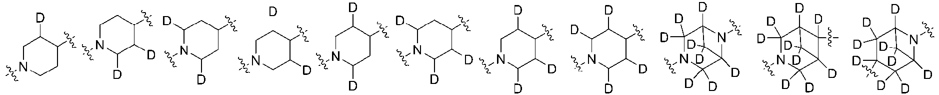 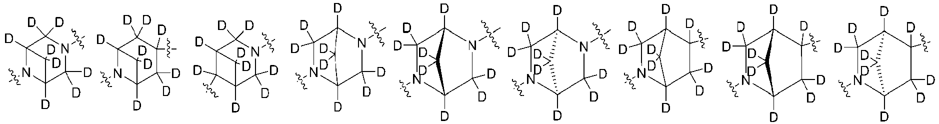 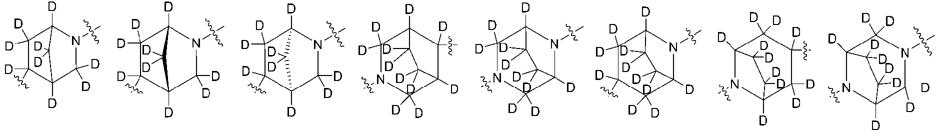 or 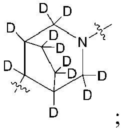 wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, and C₂₋₆ alkenyl.

13. The compound of Formula (I) or salts, enantiomers, stereoisomers, solvates, isotopically enriched analogs, prodrugs, or polymorphs thereof as claimed in any one of claims 1-12, wherein
each ring A³ is identical or different and each independently represents 4- to 20-membered heterocyclylene, C₃₋₂₀ cycloalkylene, C₅₋₂₀ arylene, or 5- to 20-membered heteroarylene; preferably each ring A³ independently represents:
azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 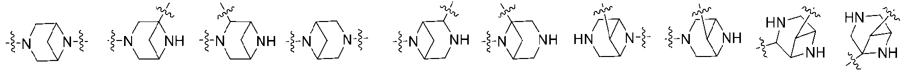 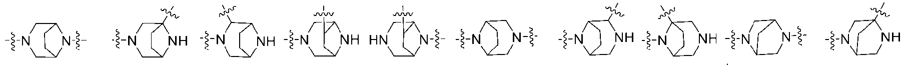 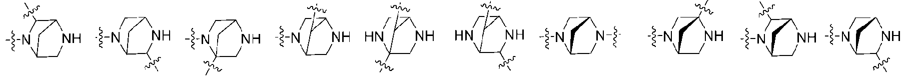 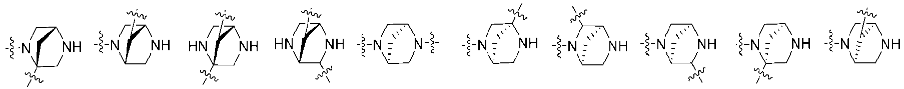 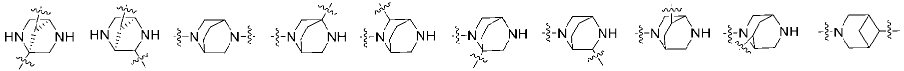 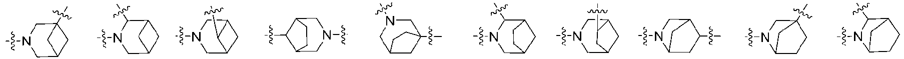 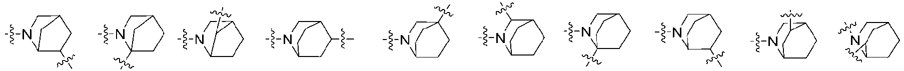 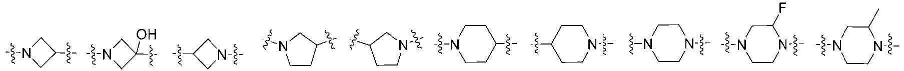 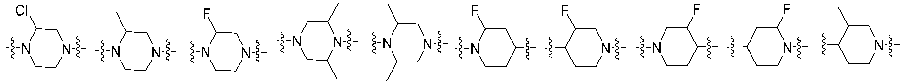 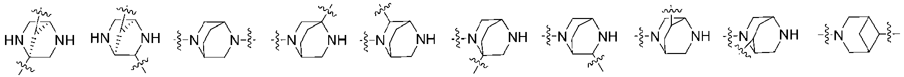 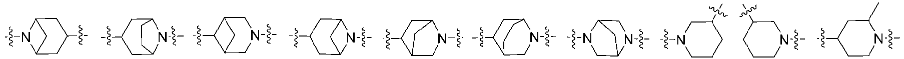 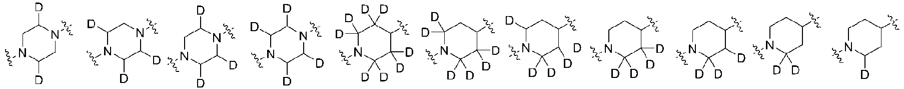 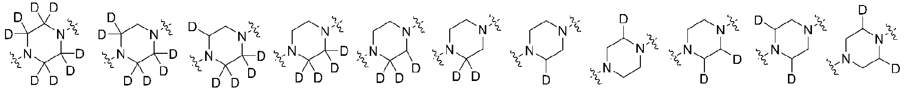 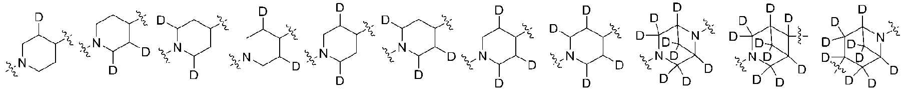 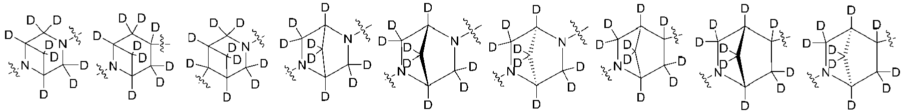 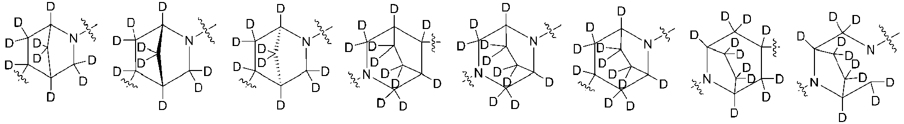 or 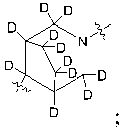
y3 represents an integer of 0, 1, 2 or 3, and (R^{y3})ₐ₃ indicates that each ring A³ is independently optionally substituted with a3 R^{y3} substituents, wherein each R^{y3} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a3 represents an integer of 0-10; and/or
each ring A⁴ is identical or different and each independently represents 4- to 20-membered heterocyclylene, C₃₋₂₀ cycloalkylene, C₅₋₂₀ arylene, or 5- to 20-membered heteroarylene; preferably each ring A⁴ independently represents:
azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 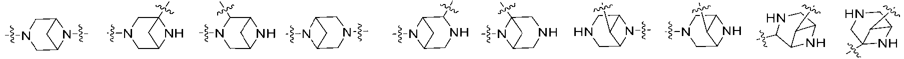 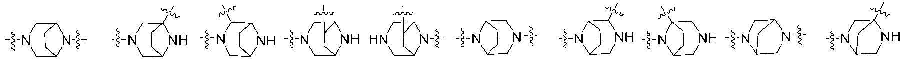 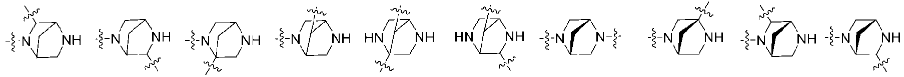 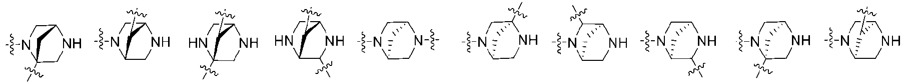 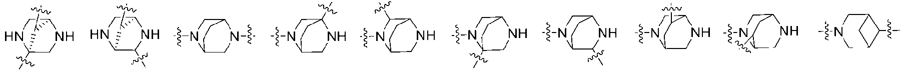 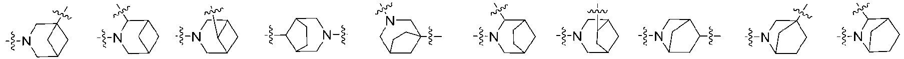 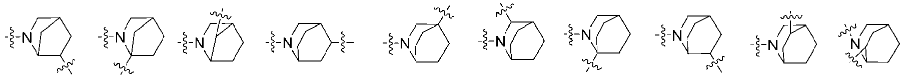 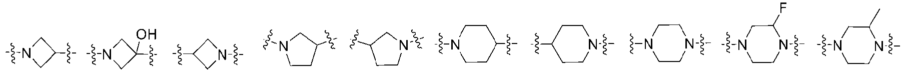 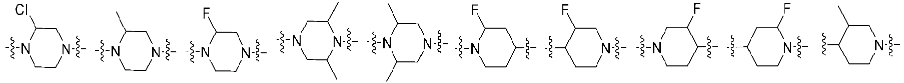 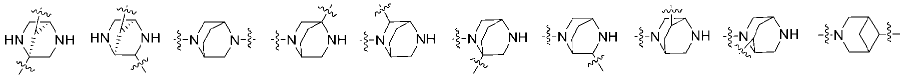 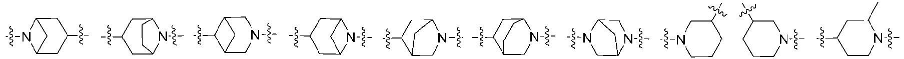 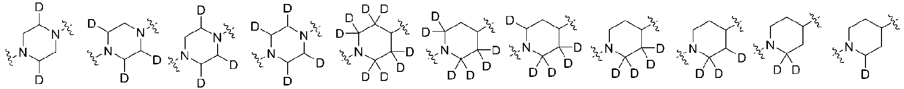 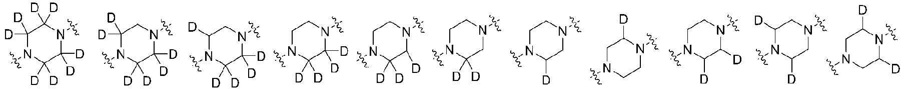 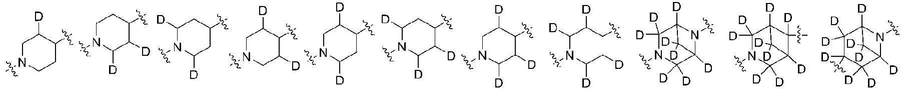 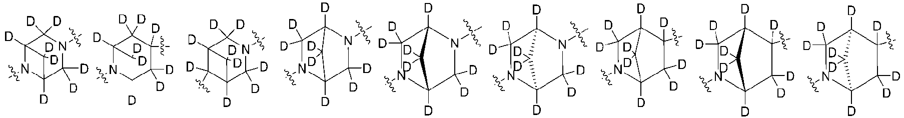 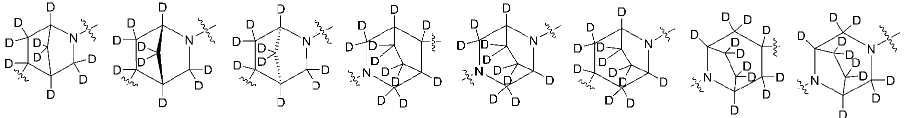 or 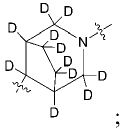
y4 represents an integer of 0, 1, 2 or 3, and (R^{y4})ₐ₄ indicates that each ring A⁴ is independently optionally substituted with a4 R^{y4} substituents, wherein each R^{y4} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a4 represents an integer of 0-10; and/or
each ring A⁵ is identical or different and each independently represents 4- to 20-membered heterocyclylene, C₃₋₂₀ cycloalkylene, C₅₋₂₀ arylene, or 5- to 20-membered heteroarylene; preferably each ring A⁵ independently represents:
azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 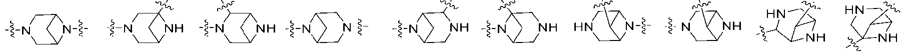 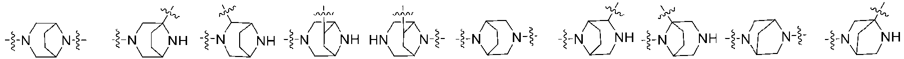 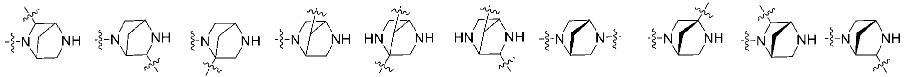 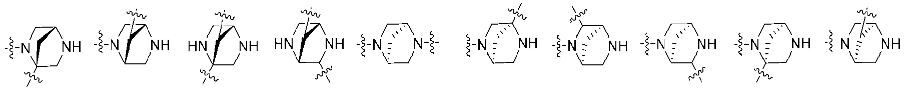 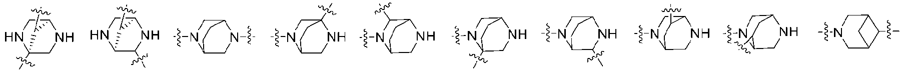 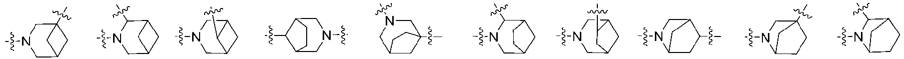 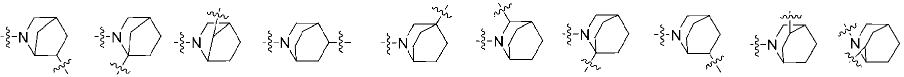 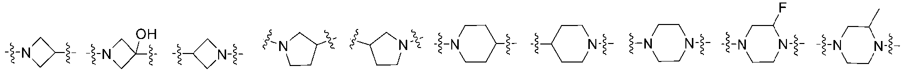 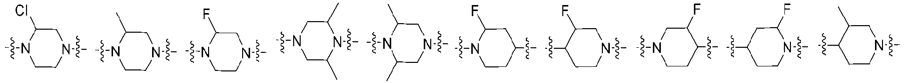 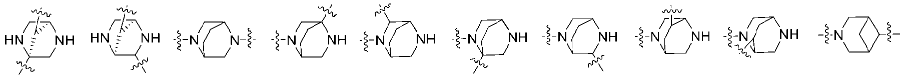 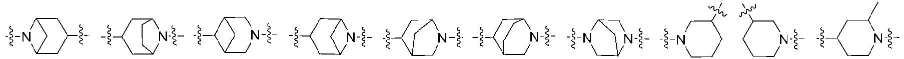 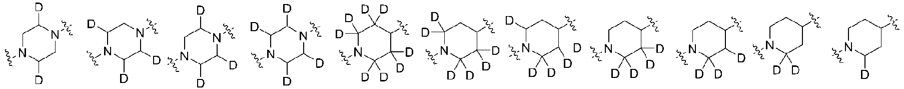 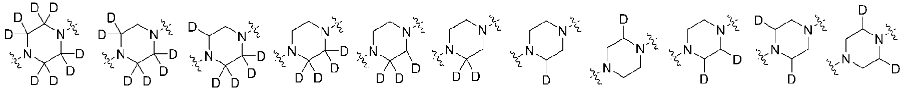 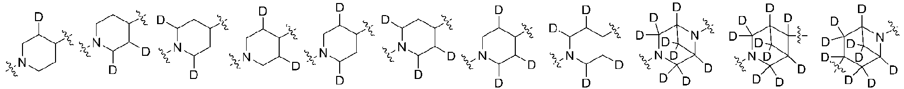 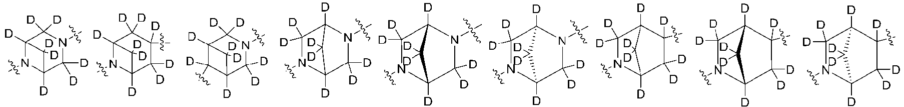 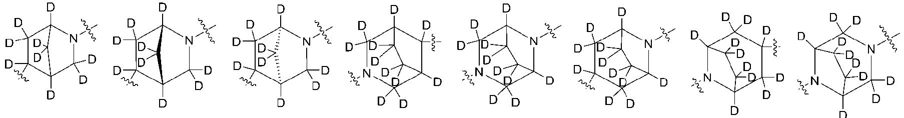 or 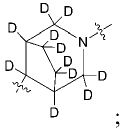
y5 represents an integer of 0, 1, 2 or 3, and (R^{y5})ₐ₅ indicates that each ring A⁵ is independently optionally substituted with a5 R^{y5} substituents, wherein each R^{y5} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a5 represents an integer of 0-10; and/or
R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from: Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆ alkynylene), alkenylene (e.g., C₂₋₆ alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and wherein one or more hydrogens of said linear or branched C₁₋₂₀ alkylene are optionally replaced by a substituent selected from the group consisting of deuterium, hydroxy, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

14. The compound of Formula (I) or salts, enantiomers, stereoisomers, solvates, isotopically enriched analogs, prodrugs, or polymorphs thereof as claimed in any one of claims 1-13, wherein the 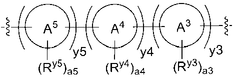 moiety in the general formula of LIN represents: 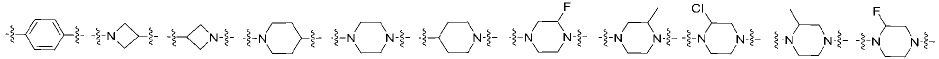 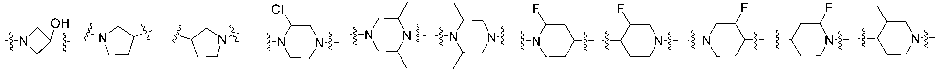 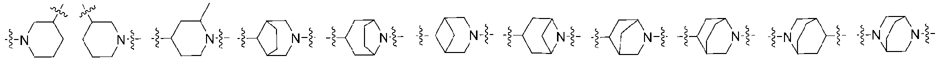 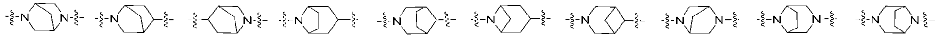 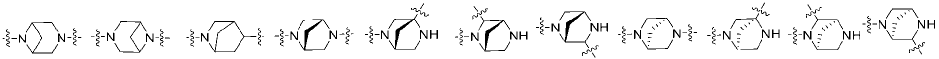 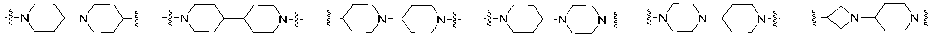 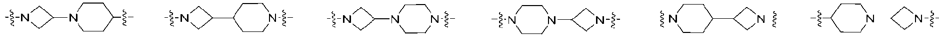 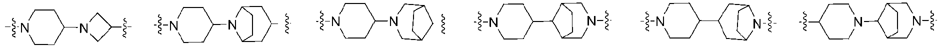 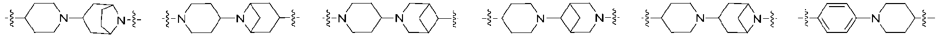 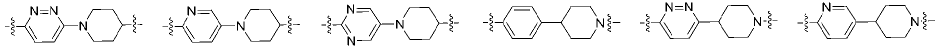 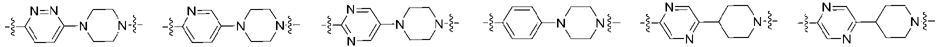 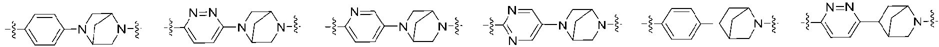 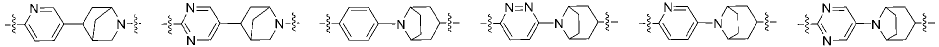 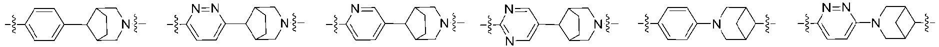 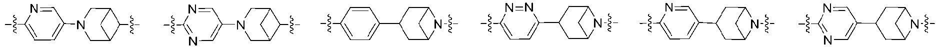 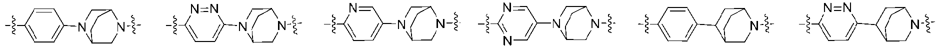 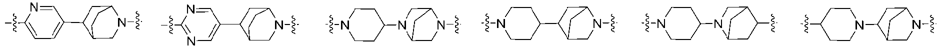 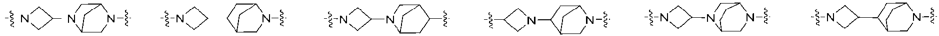 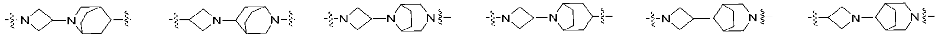 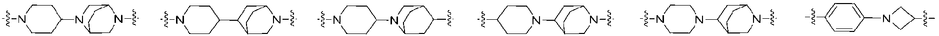 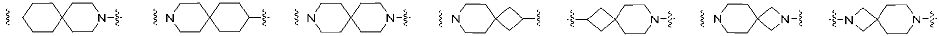 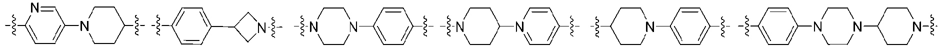 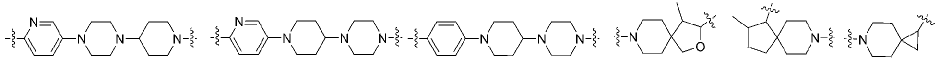 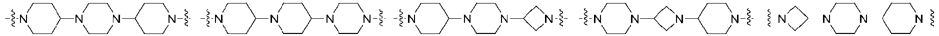 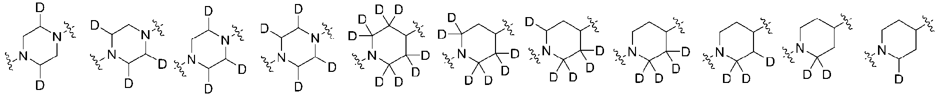 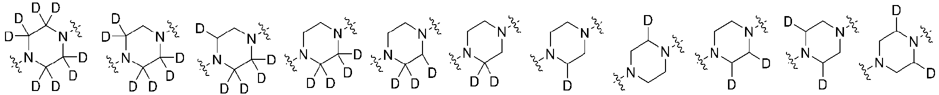 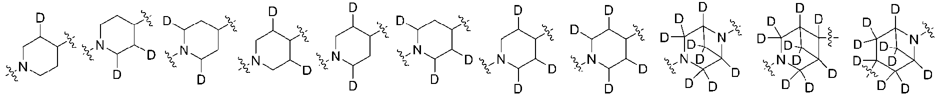 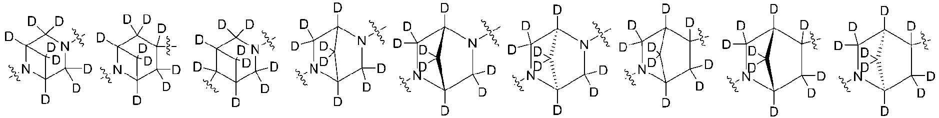 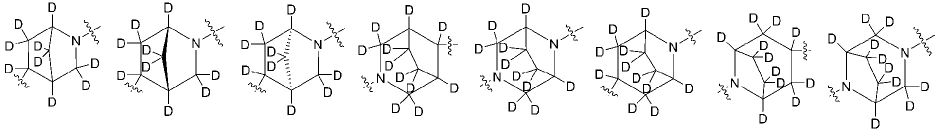 or 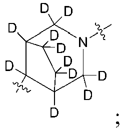 wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, and C₂₋₆ alkenyl; and/or
R_{w1} represents a bond, or
R_{w1} represents:
-C₁₋₁₅alkylene-;
-(C(Rₐ₈)(Rₐ₉))ₙ₁-(Rₐ(C(Rₐ₁₀)(Rₐ₁₁))ₙ₂)ₘ₃-;
-(C(Rₐ₈)(Rₐ₉))ₙ₁-(Rₐ(C(Rₐ₁₀)(Rₐ₁₁))ₙ₂)ₘ₃-(Rₐ(C(Rₐ₁₂)(Rₐ₁₃))ₙ₃)ₘ₄-;
-(C(Rₐ₈)(Rₐ₉))ₙ₁-(Rₐ(C(Rₐ₁₀)(Rₐ₁₁))ₙ₂)ₘ₃-(Rₐ(C(Rₐ₁₂)(Rₐ₁₃))ₙ₃)ₘ₄-(Rₐ(C(Rₐ₁₄)(Rₐ₁₅))ₙ₄)ₘ₅-;
-(C(Rₐ₈)(Rₐ₉))ₙ₁-((C(Rₐ₁₀)(Rₐ₁₁))ₙ₂Rₐ)ₘ₃-;
-(C(Rₐ₈)(Rₐ₉))ₙ₁-((C(Rₐ₁₀)(Rₐ₁₁))ₙ₂Rₐ)ₘ₃-((C(Rₐ₁₂)(Rₐ₁₃))ₙ₃Rₐ)ₘ₄-;
-(C(Rₐ₈)(Rₐ₉))ₙ₁-((C(Rₐ₁₀)(Rₐ₁₁))ₙ₂Rₐ)ₘ₃-((C(Rₐ₁₂)(Rₐ₁₃))ₙ₃Rₐ)ₘ₄-((C(Rₐ₁₄)(Rₐ₁₅))ₙ₄Rₐ)ₘ₅-;
-(C(Rₐ₈)(Rₐ₉))ₙ₁-(R_{b}(C(Rₐ₁₀)(Rₐ₁₁))ₙ₂)ₘ₃-;
-(C(Rₐ₈)(Rₐ₉))ₙ₁-(R_{b}(C(Rₐ₁₀)(Rₐ₁₁))ₙ₂)ₘ₃-(R_{b}(C(Rₐ₁₂)(Rₐ₁₃))ₙ₃)ₘ₄-;
-(C(Rₐ₈)(Rₐ₉))ₙ₁-(R_{b}(C(Rₐ₁₀)(Rₐ₁₁))ₙ₂)ₘ₃-(R_{b}(C(Rₐ₁₂)(Rₐ₁₃))ₙ₃)ₘ₄-(R_{b}(C(Rₐ₁₄)(Rₐ₁₅))ₙ₄)ₘ₅-;
-(C(Rₐ₈)(Rₐ₉))ₙ₁-((C(Rₐ₁₀)(Rₐ₁₁)ₙ₂R_{b})ₘ₃-;
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃R_{b})ₘ₄-;
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃R_{b})ₘ₄-((C(Rₐ₁₄)(Rₐ₁₅))ₙ₄R_{b})ₘ₅-;
-(C(Rₐ₈)(Rₐ₉))ₙ₁-(Rₐ-R_{b}-(C(Rₐ₁₀)(Rₐ₁₁))ₙ₂R_{b})ₘ₃-;
-(C(Rₐ₈)(Rₐ₉))ₙ₁-(Rₐ(C(Rₐ₁₀)(Rₐ₁₁))ₙ₂)ₘ₃-(R_{b}(C(Rₐ₁₂)(Rₐ₁₃))ₙ₃)ₘ₄-;
-(C(Rₐ₈)(Rₐ₉))ₙ₁-(R_{b}-Rₐ-(C(Rₐ₁₀)(Rₐ₁₁))ₙ₂)ₘ₃-;
-(C(Rₐ₈)(Rₐ₉))ₙ₁-(R_{b}(C(Rₐ₁₀)(Rₐ₁₁))ₙ₂)ₘ₃-(RₐC(Rₐ₁₂)(Rₐ₁₃)ₙ₃)ₘ₄-;
-(C(Rₐ₈)(Rₐ₉))ₙ₁-((C(Rₐ₁₀)(Rₐ₁₁))ₙ₂-Rₐ-R_{b})ₘ₃-;
-(C(Rₐ₈)(Rₐ₉))ₙ₁-((C(Rₐ₁₀)(Rₐ₁₁))ₙ₂Rₐ)ₘ₃-((C(Rₐ₁₂)(Rₐ₁₃))ₙ₃R_{b})ₘ₄-;
-(C(Rₐ₈)(Rₐ₉))ₙ1-((C(Rₐ₁₀)(Rₐ₁₁))ₙ₂-R_{b}-Rₐ)ₘ₃-; or
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃Rₐ)ₘ₄-;
wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl; and each R_{b} is independently selected from the group consisting of optionally substituted cycloalkylene (e.g., optionally substituted C₃₋₂₀ cycloalkylene), optionally substituted arylene (e.g., optionally substituted C₅₋₂₀ arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆ alkynylene), alkenylene (e.g., C₂₋₆alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof;
Rₐ₈, Rₐ₉, Ra₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
n1, n2, n3, n4, m3, m4, m5 each independently represent an integer of 1, 2, 3, 4, 5, 6, 7, 8, 9 or 10; and
any one or more hydrogens of said C₁₋₁₅ alkylene are optionally replaced by a substituent selected from: deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

15. The compound of Formula (I) or salts, enantiomers, stereoisomers, solvates, isotopically enriched analogs, prodrugs, or polymorphs thereof as claimed in any one of claims 1-13, wherein the R_{w1} represents a structure of the following Formulae:
-(C(Rₐ₈)(Rₐ₉))ₙ₁-(O(C(Rₐ₁₀)(Rₐ₁₁))ₙ₂)ₘ₃-;
-(C(Rₐ₈)(Rₐ₉))ₙ₁-(O(C(Rₐ₁₀)(Rₐ₁₁))ₙ₂)ₘ₃-(O(C(Rₐ₁₂)(Rₐ₁₃))ₙ₃)ₘ₄-;
-(C(Rₐ₈)(Rₐ₉))ₙ₁-(O(C(Rₐ₁₀)(Rₐ₁₁))ₙ₂)ₘ₃-(O(C(Rₐ₁₂)(Ra₁₃))ₙ₃)ₘ₄- (O(C(Rₐ₁₄)(Rₐ₁₅))ₙ₄)ₘ₅-;
-(C(Rₐ₈)(Rₐ₉))ₙ₁-((C(Rₐ₁₀)(Rₐ₁₁)ₙ₂O)ₘ₃-;
-(C(Rₐ₈)(Rₐ₉))ₙ₁-((C(Rₐ₁₀)(Rₐ₁₁))ₙ₂O)ₘ₃₋((C(Rₐ₁₂)(Rₐ₁₃))ₙ₃O)ₘ₄-;
-(C(Rₐ₈)(Rₐ₉))ₙ₁-((C(Rₐ₁₀)(Rₐ₁₁))ₙ₂O)ₘ₃-((C(Rₐ₁₂)(Rₐ₁₃))ₙ₃O)ₘ₄- ((C(Rₐ₁₄)(Rₐ₁₅))ₙ₄O)ₘ₅-;
-(C(Rₐ₈)(Rₐ₉))ₙ₁-(N(Rₐ₇)(C(Rₐ₁₀)(Rₐ₁₁))ₙ₂)ₘ₃-;
-(C(Rₐ₈)(Rₐ₉))ₙ₁-(N(Rₐ₇)(C(Rₐ₁₀)(Rₐ₁₁))ₙ₂)ₘ₃-(N(Rₐ₇)(C(Rₐ₁₂)(Rₐ₁₃))ₙ₃)ₘ₄-;
-(C(Rₐ₈)(Rₐ₉))ₙ₁-(N(Rₐ₇)(C(Rₐ₁₀)(Rₐ₁₁))ₙ₂)ₘ₃-(N(Rₐ₇)(C(Rₐ₁₂)(Rₐ₁₃))ₙ₃)ₘ₄-(N(Rₐ₇)(C(Rₐ₁₄)(Rₐ₁₅))ₙ₄)ₘ₅-;
-(C(Rₐ₈)(Rₐ₉))ₙ₁-((C(Rₐ₁₀)(Rₐ₁₁))ₙ₂N(Rₐ₇))ₘ₃-;
-(C(Rₐ₈)(Rₐ₉))ₙ₁-((C(Rₐ₁₀)(Ra₁₁))ₙ₂N(Rₐ₇))ₘ₃-((C(Rₐ₁₂)(Rₐ₁₃))ₙ₃N(Rₐ₇))ₘ₄-;
-(C(Rₐ₈)(Rₐ₉))ₙ₁-((C(Rₐ₁₀)(Rₐ₁₁))ₙ₂N(Rₐ₇))ₘ₃-((C(Rₐ₁₂)(Rₐ₁₃))ₙ₃N(Rₐ₇))ₘ₄-((C(Rₐ₁₄)(Rₐ₁₅))ₙ₄N(Rₐ₇))ₘ₅-;
-(C(Rₐ₈)(Rₐ₉))ₙ₁-(C(O)N(Rₐ₇)-(C(Rₐ₁₀)(Rₐ₁₁))ₙ₂)ₘ₃-;
-(C(Rₐ₈)(Rₐ₉))ₙ₁-(C(O)N(Rₐ₇)-(C(Rₐ₁₀)(Rₐ₁₁))ₙ₂)ₘ₃-(C(O)N(Rₐ₇)-(C(Rₐ₁₂)(Rₐ₁₃))ₙ₃)ₘ₄-;
-(C(Rₐ₈)(Rₐ₉))ₙ₁-(C(O)N(Rₐ₇)-(C(Rₐ₁₀)(Rₐ₁₁))ₙ₂)ₘ₃-(C(O)N(Rₐ₇)-(C(Rₐ₁₂)(Ra₁₃))ₙ₃)ₘ₄-(C(O)N(Rₐ₇)-(C(Rₐ₁₄)(Rₐ₁₅))ₙ₄)ₘ₅-;
-(C(Rₐ₅)(Rₐ₉))ₙ₁-((C(Rₐ₁₀)(Rₐ₁₁))ₙ₂-C(O)N(Rₐ₇))ₘ₃-;
-(C(Rₐ₈)(Rₐ₉))ₙ₁-((C(Rₐ₁₀)(Rₐ₁₁))ₙ₂-C(O)N(Rₐ₇))ₘ₃-((C(Rₐ₁₂)(Rₐ₁₃))ₙ₃-C(O)N(Rₐ₇))ₘ₄-;
-(C(Rₐ₈)(Rₐ₉))ₙ₁₋((C(Rₐ₁₀)(Rₐ₁₁))ₙ₂₋C(O)N(Rₐ₇))ₘ₃-((C(Rₐ₁₂)(Rₐ₁₃))ₙ₃-C(O)N(Rₐ₇)ₘ₄-((C(Rₐ₁₄)(Rₐ₁₅)ₙ₄-C(O)N(Rₐ₇))ₘ₅-;
-(C(Rₐ₈)(Rₐ₉))ₙ₁-(C(O)N(Rₐ₇)-(C(Rₐ₁₀)(Rₐ₁₁))ₙ₂)ₘ₃-(O-(C(Rₐ₁₂)(Rₐ₁₃))ₙ₃)ₘ₄-;
-(C(Rₐ₈)(Rₐ₉))ₙ₁-C(O)N(Rₐ₇)-(C(Rₐ₁₀)(Rₐ₁₁))ₙ₂-(O(C(Rₐ₁₂)(Rₐ₁₃))ₙ₃)ₘ₃-;
-(C(Rₐ₉)(Rₐ₉))ₙ₁-((C(Rₐ₁₀)(Rₐ₁₁))ₙ₂-C(O)N(Rₐ₇))ₘ₃-((C(Rₐ₁₂)(Rₐ₁₃))ₙ₃O)ₘ₄-;
-(C(Rₐ₈)(Rₐ₉))ₙ₁-(C(Rₐ₁₀)(Rₐ₁₁)ₙ₂-C(O)N(Rₐ₇)-((C(Rₐ₁₂)(Rₐ₁₃))ₙ₃O)ₘ₃-;
-(C(Rₐ₈)(Rₐ₉))ₙ₁-(N(Rₐ₇)C(O)-(C(Rₐ₁₀)(Rₐ₁₁))ₙ₂)ₘ₃-;
-(C(Rₐ₈)(Rₐ₉))ₙ₁-(N(Rₐ₇)C(O)-(C(Rₐ₁₀)(Rₐ₁₁))ₙ₂)ₘ₃-(O-(C(Rₐ₁₂)(Rₐ₁₃)))ₙ₃)ₘ₄-;
-(C(Rₐ₈)(Rₐ₉))ₙ₁-(N(Rₐ₇)C(O)-(C(Rₐ₁₀)(Rₐ₁₁))ₙ₂)ₘ₃-(O-(C(Rₐ₁₂)(Rₐ₁₃))ₙ₃)ₘ₄-(O-(C(Rₐ₁₄)(Rₐ₁₅))ₙ₄)ₘ₅-;
-(C(Rₐ₈)(Rₐ₉))ₙ₁-N(Rₐ₇)C(O)-(C(Rₐ₁₀)(Rₐ₁₁))ₙ₂-(O(C(Rₐ₁₂)(Rₐ₁₃))ₙ₃)ₘ₃-;
-(C(Rₐ₈)(Rₐ₉))ₙ₁-N(Rₐ₇)C(O)N(Rₐ₇)-(C(Rₐ₁₀)(Rₐ₁₁))ₙ₂-;
-(C(Ra₈)(Ra₉))ₙ₁-((C(Rₐ₁₀)(Rₐ₁₁))ₙ₂-N(Rₐ₇)C(O))ₘ₃-;
-(C(Rₐ₈)(Rₐ₉))ₙ₁-((C(Rₐ₁₀)(Rₐ₁₁))ₙ₂-N(Rₐ₇)C(O))ₘ₃-((C(Rₐ₁₂)(Rₐ₁₃)))ₙ₃₋O)ₘ₄-;
-(C(Rₐ₈)(Rₐ₉))ₙ₁-((C(Rₐ₁₀)(Rₐ₁₁))ₙ₂-N(Rₐ₇)C(O))ₘ₃-((C(Rₐ₁₂)(Rₐ₁₃))ₙ₃O)ₘ₄-((C(Rₐ₁₄)(Rₐ₁₅))ₙ₄O)ₘ₅-;
-(C(Rₐ₈)(Rₐ₉))ₙ₁- (C(Rₐ₁₀)(Ra₁₁))ₙ₂-N(Rₐ₇)C(O)-((C(Rₐ₁₂)(Rₐ₁₃))ₙ₃O)ₘ₃-;
-(C(Rₐ₈)(Rₐ₉))ₙ₁-(arylene-(C(Rₐ₁₀)(Rₐ₁₁))ₙ₂)ₘ₃-;
-(C(Rₐ₈)(Rₐ₉))ₙ₁-(arylene-(C(Rₐ₁₀)(Rₐ₁₁))ₙ₂)ₘ₃-arylene-(C(Rₐ₁₂)(Rₐ₁₃))ₙ₃-;
-(C(Rₐ₈)(Rₐ₉))ₙ₁-((C(Rₐ₁₀)(Ra₁₁))ₙ₂-arylene)ₘ₃-;
-(C(Rₐ₈)(Rₐ₉))ₙ₁-((C(Rₐ₁₀)(Rₐ₁₁))ₙ₂-arylene)ₘ₃-(C(Rₐ₁₂)(Rₐ₁₃))ₙ₃-arylene-;
-(C(Rₐ₈)(Rₐ₉))ₙ₁-(heterocyclylene-(C(Rₐ₁₀)(Rₐ₁₁))ₙ₂)ₘ₃-;
-(C(Rₐ₈)(Rₐ₉))ₙ₁-(heterocyclylene-(C(Rₐ₁₀)(Rₐ₁₁))ₙ₂)ₘ₃-(heterocyclylene-(C(Rₐ₁₂)(Rₐ₁₃))ₙ₃)ₘ₄-;
-(C(Rₐ₈)(Rₐ₉))ₙ₁-((C(Rₐ₁₀)(Rₐ₁₁))ₙ₂-heterocyclylene)ₘ₃-;
-(C(Rₐ₈)(Rₐ₉))ₙ₁-((C(Rₐ₁₀)(Rₐ₁₁))ₙ₂-heterocyclylene)ₘ₃-((C(Rₐ₁₂)(Rₐ₁₃))ₙ₃-heterocyclylene)ₘ₄-;
-(C(Rₐ₈)(Rₐ₉))ₙ₁-(heteroarylene-(C(Rₐ₁₀)(Rₐ₁₁))ₙ₂)ₘ₃-;
-(C(Rₐ₈)(Rₐ₉))ₙ₁-(heteroarylene-(C(Rₐ₁₀)(Rₐ₁₁))ₙ₂)ₘ₃-(heteroarylene-(C(Rₐ₁₂)(Rₐ₁₃))ₙ₃)ₘ₄-;
-(C(Rₐ₈)(Rₐ₉))ₙ₁-((C(Rₐ₁₀)(Rₐ₁₁))ₙ₂-heteroarylene)ₘ₃-;
-(C(Rₐ₈)(Rₐ₉))ₙ₁-((C(Rₐ₁₀)(Rₐ₁₁))ₙ₂-heteroarylene)ₘ₃-((C(Rₐ₁₂)(Ra₁₃))ₙ₃-heteroarylene)ₘ₄-;
-(C(Rₐ₈)(Rₐ₉))ₙ₁-(cycloalkylene-(C(Rₐ₁₀)(Rₐ₁₁))ₙ₂)ₘ₃-;
-(C(Rₐ₈)(Rₐ₉))ₙ₁-(cycloalkylene-(C(Rₐ₁₀)(Rₐ₁₁))ₙ₂)ₘ₃-(cycloalkylene-(C(Rₐ₁₂)(Rₐ₁₃))ₙ₃)ₘ₄-;
-(C(Rₐ₈)(Rₐ₉))ₙ₁-((C(Rₐ₁₀)(Rₐ₁₁))ₙ₂-cycloalkylene)ₘ₃-; or
-(C(Rₐ₈)(Rₐ₉))ₙ₁-((C(Rₐ₁₀)(Rₐ₁₁))ₙ₂-cycloalkylene)ₘ₃-((C(Rₐ₁₂)(Rₐ₁₃))ₙ₃-cycloalkylene)ₘ₄-;
wherein each Rₐ₇ independently represents H or C₁₋₃ alkyl;
the cycloalkylene (e.g., C₃₋₂₀ cycloalkylene), arylene (e.g., C₅₋₂₀ arylene), heterocyclylene (e.g., 4- to 20-membered heterocyclylene), and heteroarylene (e.g., 5- to 20-membered heteroarylene) are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof;
Rₐ₈, Rₐ₉, Ra₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof; and
n1, n2, n3, n4, m3, m4, m5 each independently represent an integer of 1, 2, 3, 4, 5, 6, 7, 8, 9 or 10.

16. The compound of Formula (I) or salts, enantiomers, stereoisomers, solvates, isotopically enriched analogs, prodrugs, or polymorphs thereof as claimed in claim 1, wherein R_{w1} represents:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, - (CH₂)₁₁-, -(CH₂)₁₂-, -(CH₂)₁₃-, -(CH₂)₁₄-, -(CH₂)₁₅-; -O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, -(CH₂)₁-O-, -(CH₂)₂-O-, -(CH₂)₃-O-, -(CH₂)₄-O-, -(CH₂)₅-O-, -(CH₂)₆-O-, -(CH₂)₇-O-, -(CH₂)₈-O-, -(CH₂)₉-O-, -(CH₂)₁₀-O-, -CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, -(CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, - CH(CH₃)-O-(CH₂)₅-, -CH(CH₃)-O-(CH₂)₆-, -CH(CH₃)-O-(CH₂)₇-, -CH(CH₃)-O-(CH₂)₉-, -CH(CH₃)-O-(CH₂)₉-, -CH(CH₃)-O-(CH₂)₁₀-, -(O(CH₂)₂)₂-, -(O(CH₂)₂)₃-, -(O(CH₂)₂)₄-, -(O(CH₂)₂)₅-, -(O(CH₂)₂)₆-, - (O(CH₂)₂)₇-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -(CH₂)₂-(O(CH₂)₂)₂-, -(CH₂)₂-(O(CH₂)₂)₃-, - (CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, - (CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, -CH₂-O-(CH₂)₂-O-CH₂-, -(CH₂)₂-O-(CH₂)₂-O-CH₂-, -(CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, -(CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)1-N(Rₐ₇)-(CH₂)₉-, -(CH₂)₁-N(Rₐ₇)-(CH₂)₁₀-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, -N(Rₐ₇)-(CH2)6-, -N(Rₐ₇)-(CH₂)₇-, -N(Rₐ₇)-(CH₂)₈-, -N(Rₐ₇)-(CH₂)₉-, -N(Rₐ₇)-(CH₂)₁₀-, -(CH₂)₂-N(Rₐ₇)-(CH₂)₁-, - (CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, -(CH₂)₂-N(Rₐ₇)-(CH₂)₆-, -(CH₂)₂-N(Rₐ₇)-(CH₂)₇-, -(CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, - (CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, - CH(CH₃)-N(Rₐ₇)-(CH₂)₈-, -CH(CH₃)-N(Rₐ₇)-(CH₂)₉-, -CH(CH₃)-N(Rₐ₇)-(CH₂)₁₀-, -C(O)NHCH₂-, - C(O)NH(CH₂)₂-, -C(O)NH(CH₂)₃-, -C(O)NH(CH₂)₄-, -C(O)NH(CH₂)₅-, -CH₂C(O)NHCH₂-, - (CH₂)₂C(O)NH(CH₂)₂-, -(CH₂)₂C(O)NH(CH₂)₃-, -(CH₂)₂C(O)NH(CH₂)₄-, -(CH₂)₂C(O)NH(CH₂)₅-, - (CH₂)₃C(O)NH(CH₂)₃-, -(CH₂)₃C(O)NH(CH₂)₄-, -(CH₂)₄C(O)NH(CH₂)₄-, -(CH₂)₅C(O)NH(CH₂)₅-, - (CH₂)₆C(O)NH(CH₂)₇-, -(CH₂)₆C(O)NH(CH₂)₆-, -(CH₂)₇C(O)NH(CH₂)₇-, -(CH₂)₂C(O)NH(CH₂)₂-O-(CH₂)₂-, -NHC(O)CH₂-, -NHC(O)(CH₂)₂-, -NHC(O)(CH₂)₃-, -NHC(O)(CH₂)₄-, -NHC(O)(CH₂)₅-, - CH₂NHC(O)CH₂-, -(CH₂)₂NHC(O)(CH₂)₂-, -(CH₂)₂NHC(O)(CH₂)₃-, -(CH₂)₂NHC(O)(CH₂)₄-, - (CH₂)₂NHC(O)(CH₂)₅-, -(CH₂)₃NHC(O)(CH₂)₃-, -(CH₂)₃NHC(O)(CH₂)₄-, -(CH₂)₄NHC(O)(CH₂)₄-, - (CH₂)₅NHC(O)(CH₂)₅-, -(CH₂)₆NHC(O)(CH₂)₇-, -(CH₂)₆NHC(O)(CH₂)₆-, -(CH₂)₇NHC(O)(CH₂)₇-, - (CH₂)₄NHC(O)(CH₂)₈-, -(CH₂)₂NHC(O)(CH₂)₂-O-(CH₂)₂-, -(CH₂)₄NHC(O)CH₂-, -phenylene-CH₂-, - phenylene-(CH₂)₂-, -phenylene-(CH₂)₃-, -phenylene-(CH₂)₄-, -phenylene-(CH₂)₅-, -phenylene-(CH₂)₆-, - phenylene-(CH₂)₇-, -phenylene-(CH₂)₈-, -CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₉-, -(CH₂)₂-phenylene-(CH₂)₁-, -(CH₂)₂-phenylene-(CH₂)₂-, - (CH₂)₂-phenylene-(CH₂)₃-, -(CH₂)₂-phenylene-(CH₂)₄-, -(CH₂)₂-phenylene-(CH₂)₅-, -(CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, - (CH₂)₃-phenylene-(CH₂)₆-, -(CH₂)₃-phenylene-(CH₂)₇-, -(CH₂)₃-phenylene-(CH₂)₈-, -(CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, - (CH₂)₅-phenylene-(CH₂)₁-, -(CH₂)₅-phenylene-(CH₂)₂-, -(CH₂)₅-phenylene-(CH₂)₃-, -(CH₂)₅-phenylene-(CH₂)₄-, -(CH₂)₅-phenylene-(CH₂)₅-, -(CH₂)₅-phenylene-(CH₂)₆-, -(CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, - (CH₂)₆-phenylene-(CH₂)₄-, -(CH₂)₆-phenylene-(CH₂)₅-, -(CH₂)₆-phenylene-(CH₂)₆-, -(CH₂)₆-phenylene-(CH₂)₇-, -(CH₂)₆-phenylene-(CH₂)₈-, -(CH₂)₇-phenylene-(CH₂)₁-, -(CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₈-phenylene-CH₂-, - (CH₂)₈-phenylene-(CH₂)₂-, -(CH₂)₈-phenylene-(CH₂)₃-, -(CH₂)₈-phenylene-(CH₂)₄-, -(CH₂)₈-phenylene-(CH₂)₅-, -(CH₂)₈-phenylene-(CH₂)₆-, -(CH₂)₈-phenylene-(CH₂)₇-, -N(Rₐ₇)-CH₂-phenylene-CH₂-, -N(Rₐ₇)-CH₂-phenylene-(CH₂)₂-, -N(Rₐ₇)-CH₂-phenylene-(CH₂)₃-, -N(Rₐ₇)-CH₂-phenylene-(CH₂)₄-, -N(Rₐ₇)-CH₂-phenylene-(CH₂)₅-, -N(Rₐ₇)-CH₂-phenylene-(CH₂)₆-, -N(Rₐ₇)-CH₂-phenylene-(CH₂)₇-, -N(Rₐ₇)-CH₂-phenylene-(CH₂)₈-, -CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, -CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, - CH₂-N(Rₐ₇)-(CH₂)₂-phenylene-(CH₂)₈-, -(CH₂)₂-N(Rₐ₇)-CH₂-phenylene-CH₂-, -(CH₂)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, - (CH₂)₂-N(Rₐ₇)-CH₂-phenylene-(CH₂)₅-, -(CH₂)₂-N(Rₐ₇)-CH₂-phenylene-(CH₂)₆-, -(CH₂)₂-N(Rₐ₇)-CH₂-phenylene-(CH₂)₇-, -(CH₂)₂-N(Rₐ₇)-CH₂-phenylene-(CH₂)₈-, -(CH₂)₃-N(Rₐ₇)-CH₂-phenylene-CH₂-, - (CH₂)₃-N(Rₐ₇)-CH₂-phenylene-(CH₂)₂-, -(CH₂)₃-N(Rₐ₇)-CH₂-phenylene-(CH₂)₃-, -(CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, - (CH₂)₄-N(Rₐ₇)-CH₂-phenylene-(CH₂)₃-, -(CH₂)₄-N(Rₐ₇)-CH₂-phenylene-(CH₂)₈-, -(CH₂)₅-N(Rₐ₇)-CH₂-phenylene-(CH₂)₃-, -(CH₂)₅-N(Rₐ₇)-CH₂-phenylene-(CH₂)₈-, -(CH₂)₆-N(Rₐ₇)-CH₂-phenylene-(CH₂)₃-, - (CH₂)₆-N(Rₐ₇)-CH₂-phenylene-(CH₂)₈-, -(CH₂)₇-N(Rₐ₇)-CH₂-phenylene-(CH₂)₃-, -(CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, - (CH₂)₈-N(Rₐ₇)-CH₂-phenylene-(CH₂)₄-, -(CH₂)₈-N(Rₐ₇)-CH₂-phenylene-(CH₂)₅-, -(CH₂)₈-N(Rₐ₇)-CH₂-phenylene-(CH₂)₆-, -piperidinylene-CH₂-, -piperidinylene-(CH₂)₂-, -piperidinylene-(CH₂)₃-, - piperidinylene-(CH₂)₄-, -piperidinylene-(CH₂)₅-, -piperidinylene-(CH₂)₆-, -piperidinylene-(CH₂)₇-, - piperidinylene-(CH₂)₈-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperidinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₈-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperldinylene-(CH₂)₆-, -(CH₂)₈-piperidinylene-(CH₂)₇-, -N(Rₐ₇)-CH₂-piperidinylene-CH₂-, -N(Rₐ₇)-CH₂-piperidinylene-(CH₂)₂-, -N(Rₐ₇)-CH₂-piperidinylene-(CH₂)₃-, -N(Rₐ₇)-CH₂-piperidinylene-(CH₂)₄-, -N(Rₐ₇)-CH₂-piperidinylene-(CH₂)₅-, -N(Rₐ₇)-CH₂-piperidinylene-(CH₂)₆-, - N(Rₐ₇)-CH₂-piperidinylene-(CH₂)₇-, -N(Rₐ₇)-CH₂-piperidinylene-(CH₂)₈-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, -CH₂-N(Rₐ₇)-CH₂-piperidinylene-(CH₂)₄-, -CH₂-N(Rₐ₇)-CH₂-piperidinylene-(CH₂)₅-, -CH₂-N(Ra₇)-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇)-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇)-CH₂-piperidinylene-(CH₂)₆-, -(CH₂)₂-N(Rₐ₇)-CH₂-piperidinylene-(CH₂)₇-, -(CH₂)₂-N(Ra₇)-CH₂-piperidinylene-(CH₂)₈-, - (CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, - (CH₂)₅-N(Rₐ₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperidinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, - (CH₂)₈-N(Rₐ₇)-CH₂-piperidinylene-(CH₂)₆-, -piperazinylene-CH₂-, -piperazinylene-(CH₂)₂-, - piperazinylene-(CH₂)₃-, -piperazinylene-(CH₂)₄-, -piperazinylene-(CH₂)₅-, -piperazinylene-(CH₂)₆-, - piperazinylene-(CH₂)₇-, -piperazinylene-(CH₂)₈-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₈, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene and piperidinylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

17. The compound of Formula (I) or salts, enantiomers, stereoisomers, solvates, isotopically enriched analogs, prodrugs, or polymorphs thereof as claimed in claim 1, wherein
LIN represents: a bond, C(O), C(O)NH, NHC(O), -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄-, -(CH₂)₁₅-; -O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, (CH₂)₁-O-, -(CH₂)₂-O-, -(CH₂)₃-O-, -(CH₂)₄-O-, -(CH₂)₅-O-, -(CH₂)₆-O-, -(CH₂)₇-O-, -(CH₂)₈-O-, -(CH₂)₉-O-, -(CH₂)₁₀-O-, -CH₂-O-CH₂-, -CH₂-O-(CH₂)₂-, -(CH₂)₁-O-(CH₂)₃-, -(CH₂)₁-O-(CH₂)₄-, - (CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₂-O-(CH₂)₁-, - (CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, - (CH₂)₂-O-(CH₂)₇-, -(CH₂)₂-O-(CH₂)₈-, -(CH₂)₃-O-(CH₂)₁-, -(CH₂)₃-O-(CH₂)₂-, -(CH₂)₃-O-(CH₂)₃-, - (CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, - (CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, - (CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, - (CH₂)-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, - (CH₂)₇-O-(CH₂)₂-, -(CH₂)₇-O-(CH₂)₃-, -(CH₂)₈-O-(CH₂)₁-, -(CH₂)₈-O-(CH₂)₂-, -CH(CH₃)-O-(CH₂)₁-, - CH(CH₃)-O-(CH₂)₂-, -CH(CH₃)-O-(CH₂)₃-, -CH(CH₃)-O-(CH₂)₄-, -CH(CH₃)-O-(CH₂)₅-, -CH(CH₃)-O-(CH₂)₆-, -CH(CH₃)-O-(CH₂)₇-, -CH(CH₃)-O-(CH₂)₈-, -(O(CH₂)₂)₂-, -(O(CH₂)₂)₃-, -(O(CH₂)₂)₄-, - (O(CH₂)₂)₅-, -(O(CH₂)₂)₆-, -CH₂-(O(CH₂)₂)₂-, -CH₂-(O(CH₂)₂)₃-, -CH₂-(O(CH₂)₂)₄-, -CH₂-(O(CH₂)₂)₅-, - CH₂-(O(CH₂)₂)₆-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -CH₂-(O(CH₂)₂)₆-OCH₂-, -(CH₂)₂-(O(CH₂)₂)₂-, -(CH₂)₂-(O(CH₂)₂)₃-, -(CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, - (CH₂)₃-(O(CH₂)₂)₃-, -(CH₂)₃-(O(CH₂)₂)₄-, -(CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -CH₂-(O(CH₂)₃)₂-, -CH₂-(O(CH₂)₃)₃-, -CH₂-(O(CH₂)₃)₄-, - (CH₂)₂-(O(CH₂)₃)₂-, -(CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -CH₂-O-(CH₂)₂-O-(CH₂)₃-, -CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, (CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, -(CH₂)₁-N(Rₐ₇)-(CH₂)₅-, -(CH₂)₁-N(Rₐ₇)-(CH₂)₆-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, -N(Rₐ₇)-(CH₂)₆-, -(CH₂)₂-N(Rₐ₇)-(CH₂)₁-, -(CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, -(CH₂)₂-N(Rₐ₇)-(CH₂)₆-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, - (CH₂)₄-N(Rₐ₇)-(CH₂)₁-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, -(CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, - (CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -C(O)NHCH₂-, - C(O)NH(CH₂)₂-, -C(O)NH(CH₂)₃-, -C(O)NH(CH₂)₄-, -C(O)NH(CH₂)₅-, -CH₂C(O)NHCH₂-, - (CH₂)₂C(O)NH(CH₂)₂-, -(CH₂)₂C(O)NH(CH₂)₃-, -(CH₂)₂C(O)NH(CH₂)₄-, -(CH₂)₂C(O)NH(CH₂)₅-, - (CH₂)₃C(O)NH(CH₂)₃-, -(CH₂)₃C(O)NH(CH₂)₄-, -(CH₂)₄C(O)NH(CH₂)₄-, -(CH₂)₅C(O)NH(CH₂)₅-, - (CH₂)₂C(O)NH(CH₂)₂-O-(CH₂)₂-, -NHC(O)CH₂-, -NHC(O)(CH₂)₂-, -NHC(O)(CH₂)₃-, -NHC(O)(CH₂)₄-, -NHC(O)(CH₂)₅-, -CH₂NHC(O)CH₂-, -(CH₂)₂NHC(O)(CH₂)₂-, -(CH₂)₂NHC(O)(CH₂)₃-, - (CH₂)₂NHC(O)(CH₂)₄-, -(CH₂)₂NHC(O)(CH₂)₅-, -(CH₂)₃NHC(O)(CH₂)₃-, -(CH₂)₃NHC(O)(CH₂)₄-, - (CH₂)₄NHC(O)(CH₂)₄-, -(CH₂)₅NHC(O)(CH₂)₅-, -(CH₂)₂NHC(O)(CH₂)₂-O-(CH₂)₂-, -(CH₂)₄NHC(O)CH₂-, -CH₂-phenylene-CH₂-, -CH₂-phenylene-(CH₂)₂-, -CH₂-phenylene-(CH₂)₃-, -CH₂-phenylene-(CH₂)₄-, - CH₂-phenylene-(CH₂)₅-, -CH₂-phenylene-(CH₂)₆-, -(CH₂)₂-phenylene-(CH₂)₁-, -(CH₂)₂-phenylene-(CH₂)₂-, -(CH₂)₂-phenylene-(CH₂)₃-, -(CH₂)₂-phenylene-(CH₂)₄-, -(CH₂)₂-phenylene-(CH₂)₅-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, -(CH₂)₄-phenylene-CH₂-, -(CH₂)₄-phenylene-(CH₂)₂-, -(CH₂)₄-phenylene-(CH₂)₃-, - (CH₂)₄-phenylene-(CH₂)₄-, -(CH₂)₄-phenylene-(CH₂)₅-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -(CH₂)₂-piperidinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, - (CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, or -(CH₂)₈-piperazinylene-(CH₂)₅-; or
LIN represents: 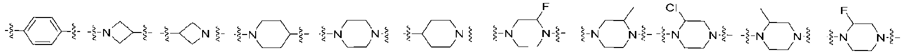 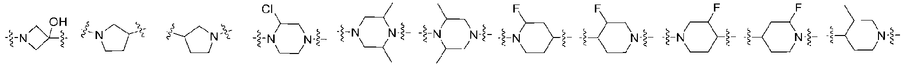 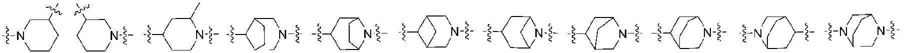 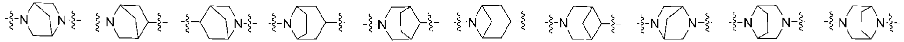 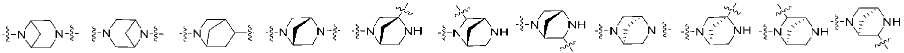 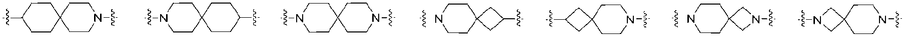 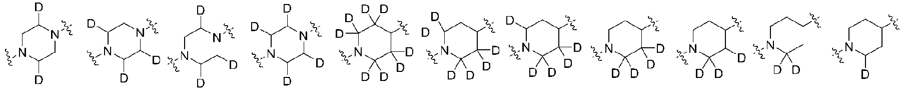 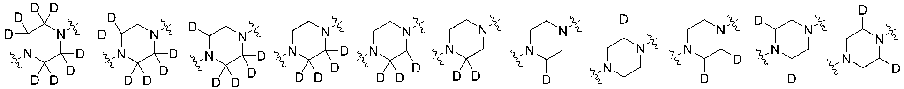 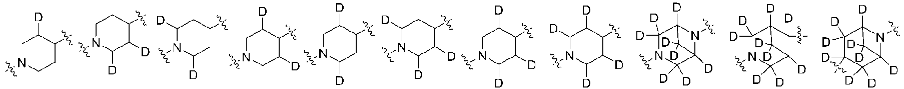 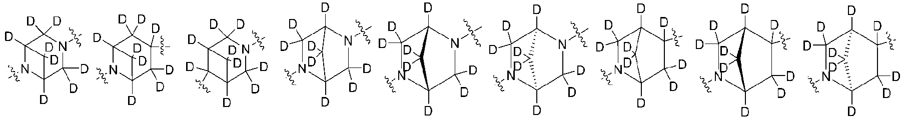 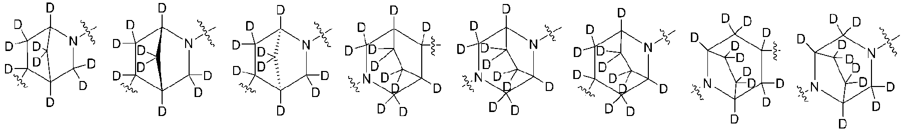 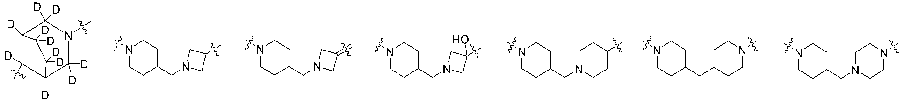 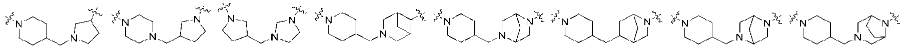 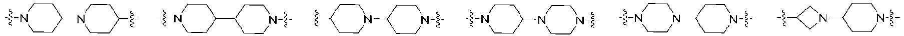 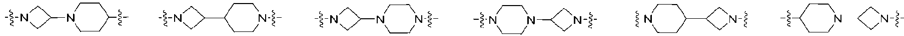 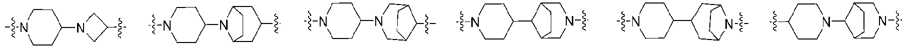 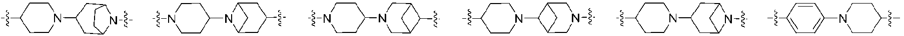 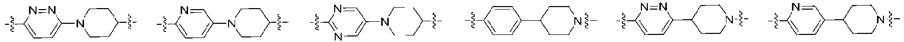 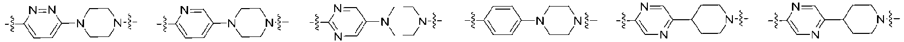 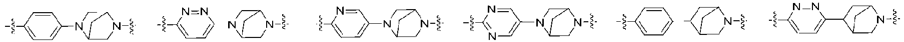 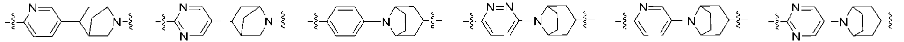 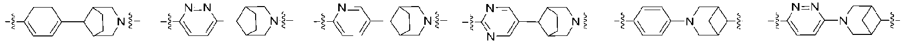 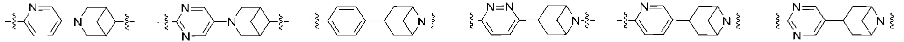 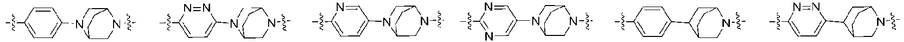 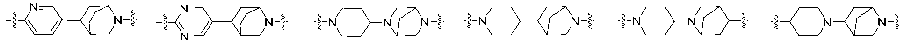 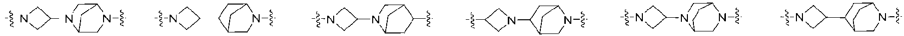 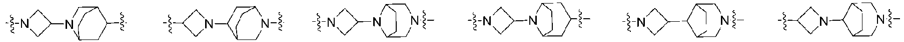 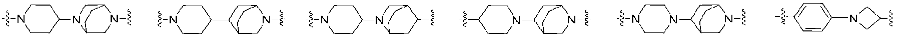 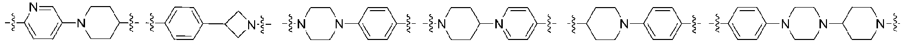 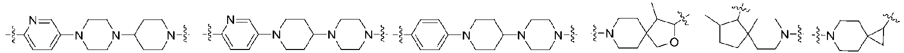 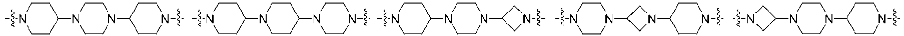 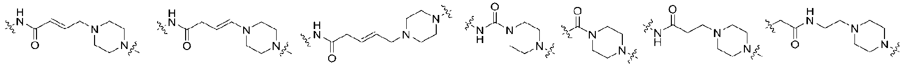 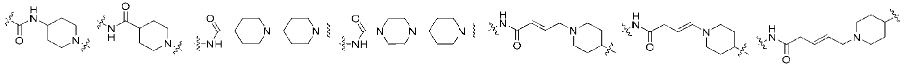 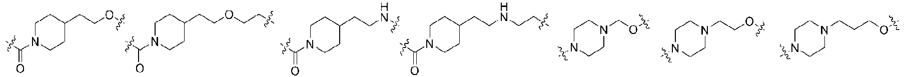 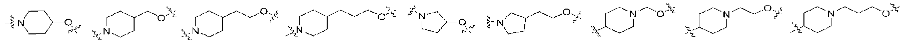 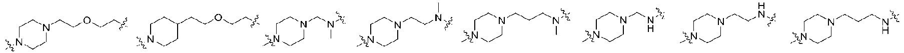 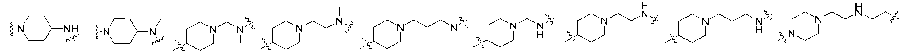 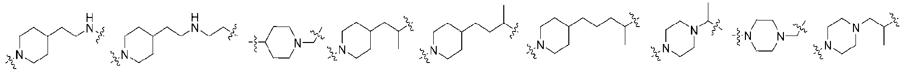 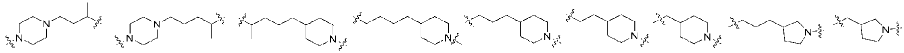 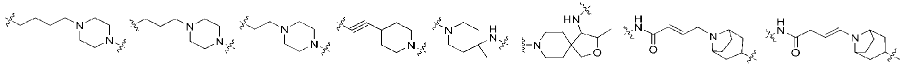 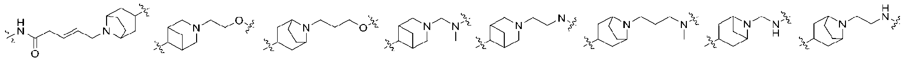 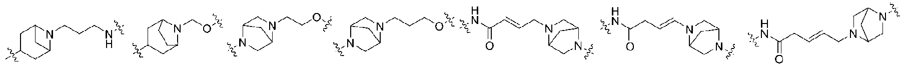 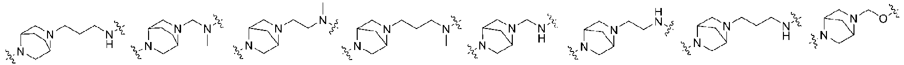 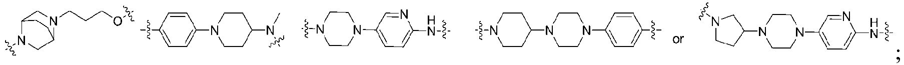 wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, and C₂₋₆ alkenyl.

18. The compound of Formula (I) or salts, enantiomers, stereoisomers, solvates, isotopically enriched analogs, prodrugs, or polymorphs thereof as claimed in claim 1, which is also represented by Formula (I-1), Formula (I-2), Formula (I-3), Formula (I-4), Formula (I-5-1), Formula (I-5-2), Formula (I-5-3), Formula (I-5-4), Formula (I-6), Formula (I-7), Formula (I-8), Formula (I-9), Formula (1-10-1), Formula (I-10-2), Formula (I-11), Formula (I-12), Formula (I-13-1), Formula (I-13-2), Formula (I-14), Formula (I-15), Formula (I-16), Formula (I-17), Formula (1-18-1), Formula (I-18-2), Formula (I-19), Formula (I-20), Formula (I-21), Formula (I-22), Formula (I-23), Formula (I-24), Formula (I-25), Formula (I-26), Formula (I-27), Formula (I-28), Formula (I-29) or Formula (I-30): 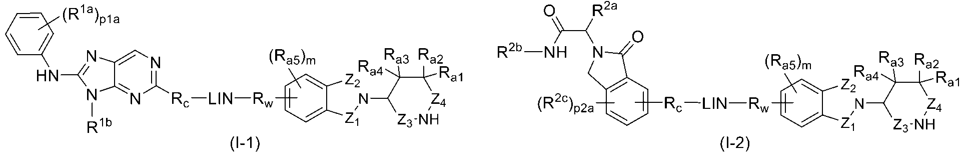 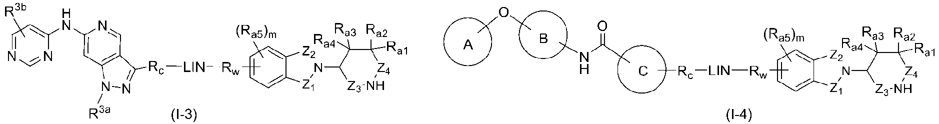 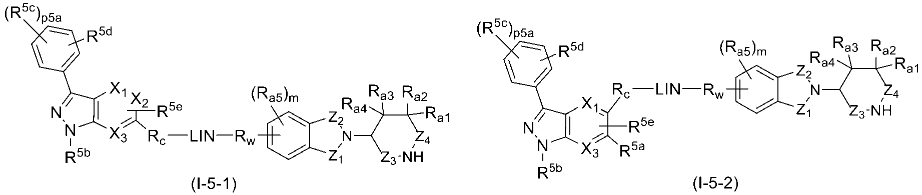 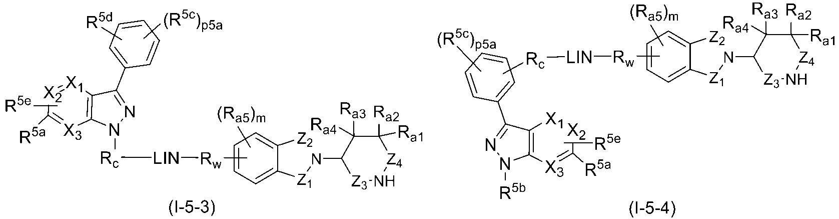 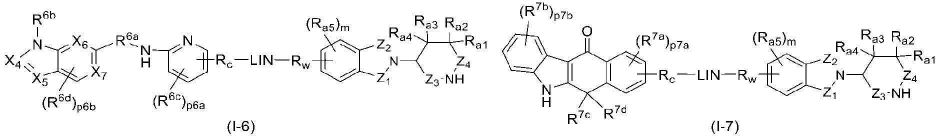 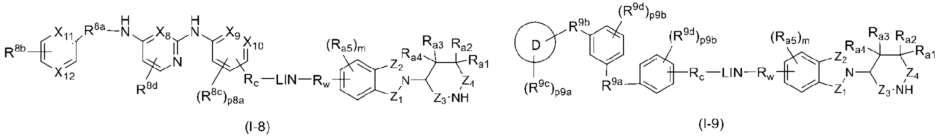 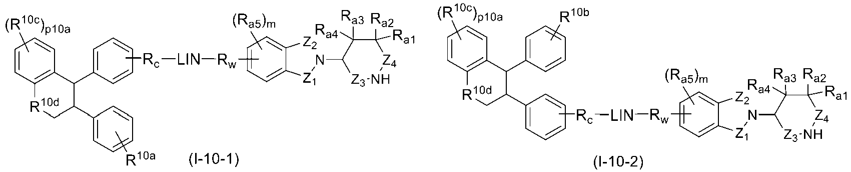 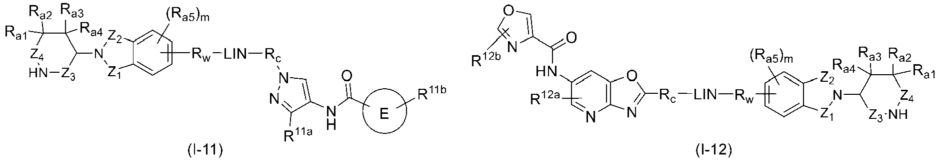 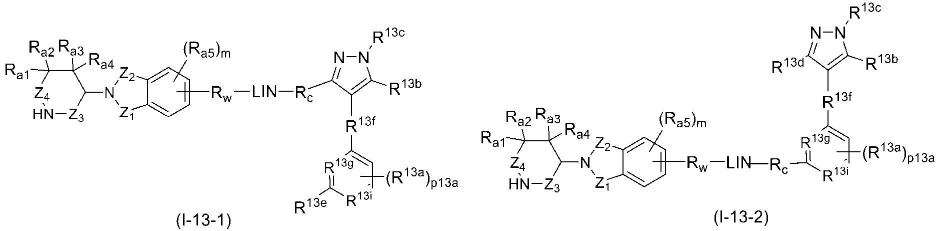 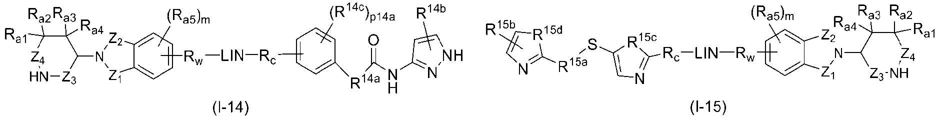 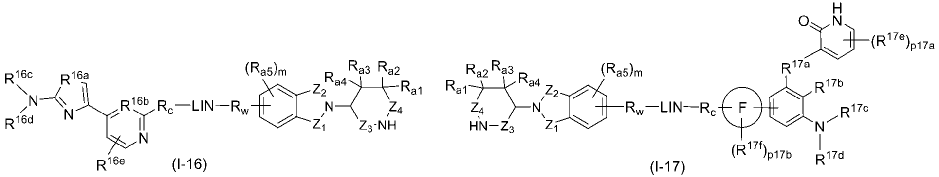 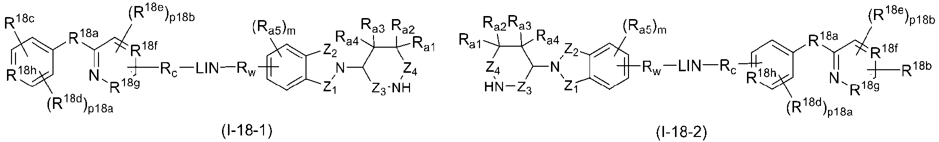 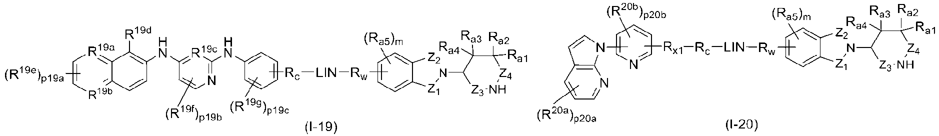 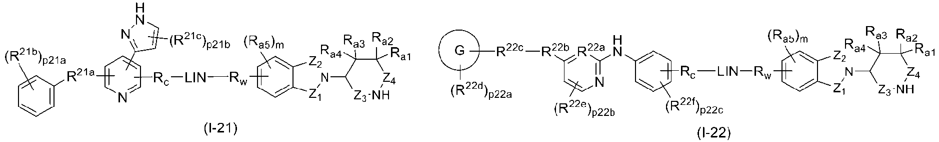 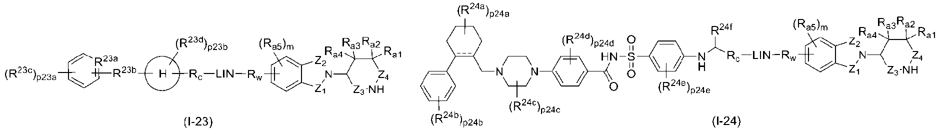 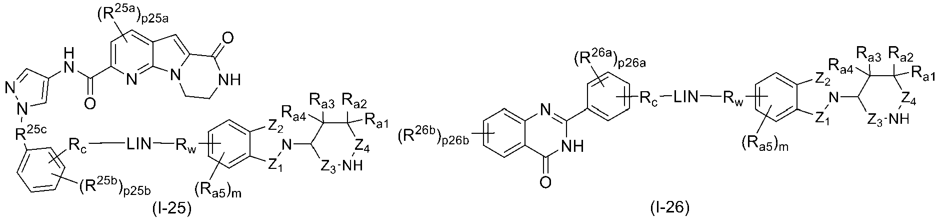 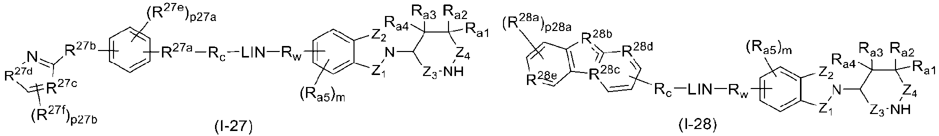 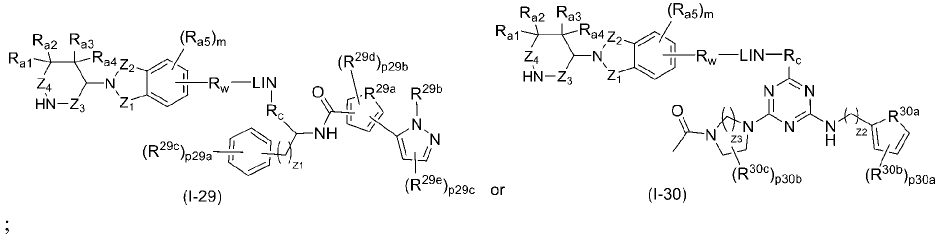 wherein Z₁, Z₂, Z₃, Z₄, Rₐ₁, Rₐ₂, Rₐ₃, Rₐ₄, (Rₐ₅)ₘ, R_{w}, LIN, R_{c}, (R^{1a})ₚ₁ₐ, R^{1b}, R^{2a}, R^{2b}, (R^{2c})ₚ₂ₐ, R^{3a}, R^{3b}, ring A, ring B, ring C, R^{5a}, R^{5b}, (R^{5c})ₚ₅ₐ, R^{5d}, R^{5e}, X₁, X₂, X₃, R^{6a}, R^{6b}, (R^{6c})ₚ₆ₐ, (R^{6d})_{p6b}, X₄, X₅, X₆, X₇, (R^{7a})ₚ₇ₐ, (R^{7b})_{p7b}, R^{7c}, R^{7d}, R^{8a}, R^{8b}, (R^{8c})ₚ₈ₐ, R^{8d}, X₈, X₉, X₁₀, X₁₁, X₁₂, R^{9a}, R^{9b}, (R^{9c})ₚ₉ₐ, (R^{9d})_{p9b}, R^{10a}, R^{10b}, (R^{10c})ₚ₁₀ₐ, R^{10d}, R^{11a}, R¹¹ⁿ, ring E, R^{12a}, R^{12b}, (R^{13b})ₚ₁₃ₐ, R^{13b}, R^{13c}, R^{13d}, R^{13e}, R^{13f}, R^{13g}, R¹³ⁱ, R^{14a}, R^{14b}, (R^{14c})ₚ₁₄ₐ, R^{15a}, R^{15b}, R^{15c}, R^{15d}, R^{16a}, R^{16b}, R^{16c}, R^{16d}, R^{16e}, R^{17a}, R^{17b}, R^{17c}, R^{17d}, (R^{17e})ₚ₁₇ₐ, (R^{17f})_{p17b}, ring F, R^{18a}, R^{18c}, (R^{18d})ₚ₁₈ₐ, (R^{18e})_{p18b}, R^{18f}, R^{18g}, R^{18h} , R^{19a}, R^{19b}, R^{19c}, R^{19d}, (R^{19e})ₚ₁₉ₐ, (R^{19f})_{p19b}, (R^{19g})_{p19c}, (R^{20a})ₚ₂₀ₐ, (R^{20b})_{p20b}, Rₓ₁, R^{21a}, (R^{21b})ₚ₂₁ₐ, (R^{21c})_{p21b}, R^{22a}, R^{22b}, R^{22c}, (R^{22d})ₚ₂₂ₐ, (R^{22e})_{p22b}, (R^{22f})_{p22c}, ring G, R^{23a}, R^{23b}, (R^{23c})ₚ₂₃ₐ, (R^{23d})_{p23b}, ring H, (R^{24a})ₚ₂₄ₐ, (R^{24b})_{p24b}, (R^{24c})_{p24c}, (R^{24d})_{p24d}, (R^{24e})ₚ₂₄ₑ, R^{24f}, (R^{25a})ₚ₂₅ₐ, (R^{25b})_{p25b}, R^{25c}, (R^{26a})ₚ₂₆ₐ, (R^{26b})_{p26b}, R^{27a}, R^{27b}, R^{27c}, R^{27d}, (R^{27e})ₚ₂₇ₐ, (R^{27f})_{p27b}, (R^{28a})ₚ₂₈ₐ, R^{28b}, R^{28c}, R^{28d}, R^{28e}, R^{29a}, R^{29b}, Z1, (R^{29c})ₚ₂₉ₐ, (R^{29d})_{p29b}, (R²⁷)_{p29c}, R^{30a}, (R^{30b})ₚ₃₀ₐ, (R^{30c})_{p30b}, Z2, and Z3 are as defined in claim 3.

19. The compound of Formula (I) or salts, enantiomers, stereoisomers, solvates, isotopically enriched analogs, prodrugs, or polymorphs thereof as claimed in claim 1, which is selected from:
3-(4-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione;
3-(6-((4-(4-amino-3-(4-phenoxyphenyl)-1H-pyrazolo[3,4-d]pyrimidin-1-yl)piperidin-1-yl)methyl)-1-thioxoisoindolin-2-yl)piperidine-2,6-dione;
3-(5-((4-(4-((9-cyclopentyl-8-(phenylamino)-9H-purin-2-yl)amino)phenyl)piperazin-1-yl)methyl)-1-thioxoisoindolin-2-yl)piperidine-2,6-dione;
3-(4-((4-(4-((9-cyclopentyl-8-(phenylamino)-9H-purin-2-yl)amino)phenyl)piperazin-1-yl)methyl)-1-thioxoisoindolin-2-yl)piperidine-2,6-dione;
3-(6-((4-(4-((9-cyclopentyl-8-(phenylamino)-9H-purin-2-yl)amino)phenyl)piperazin-1-yl)methyl)-1-thioxoisoindolin-2-yl)piperidine-2,6-dione;
3-(5-(((1-(4-((9-cyclopentyl-8-(phenylamino)-9H-purin-2-yl)amino)phenyl)piperidin-4-yl)(methyl)amino)methyl)-1-thioxoisoindolin-2-yl)piperidine-2,6-dione;
3-(5-((4-(1-(4-((9-cyclopentyl-8-(phenylamino)-9H-purin-2-yl)amino)phenyl)piperidin-4-yl)piperazin-1-yl)methyl)-1-thioxoisoindolin-2-yl)piperidine-2,6-dione;
3-(6-((4-(1-(4-((9-cyclopentyl-8-(phenylamino)-9H-purin-2-yl)amino)phenyl)piperidin-4-yl)piperazin-1-yl)methyl)-1-thioxoisoindolin-2-yl)piperidine-2,6-dione;
3-(4-((4-(1-(4-((9-cyclopentyl-8-(phenylamino)-9H-purin-2-yl)amino)phenyl)piperidin-4-yl)piperazin-1-yl)methyl)-1-thioxoisoindolin-2-yl)piperidine-2,6-dione;
3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-1-thioxoisoindolin-2-yl)piperidine-2,6-dione;
3-(4-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-1-thioxoisoindolin-2-yl)piperidine-2,6-dione;
3-(6-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-1-thioxoisoindolin-2-yl)piperidine-2,6-dione;
3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-thioxoisoindolin-2-yl)piperidine-2,6-dione;
3-(5-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione;
3-(4-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione;
2-(7-(((2-(2,6-dioxopiperidin-3-yl)-1-thioxoisoindolin-5-yl)methyl)amino)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-1-thioxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-1-thioxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-1-thioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-1-thioxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-1-thioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6,7-dihydro-5H-pyrrolo[1,2-c]imidazol-1-yl)-2-(6-(4-(4-((2-(2,6-dioxopiperidin-3-yl)-1-thioxoisoindolin-5-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-(2,6-dioxopiperidin-3-yl)-1-thioxoisoindolin-4-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-((2-(2,6-dioxopiperidin-3-yl)-3-thioxoisoindolin-5-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-((2-(2,6-dioxopiperidin-3-yl)-1-thioxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((2-(2,6-dioxopiperidin-3-yl)-1-thioxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-((2-(2,6-dioxopiperidin-3-yl)-1-thioxoisoindolin-5-yl)methyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((2-(2,6-dioxopiperidin-3-yl)-1-thioxoisoindolin-5-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((2-(2,6-dioxopiperidin-3-yl)-1-thioxoisoindolin-5-yl)methyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((2-(2,6-dioxopiperidin-3-yl)-1-thioxoisoindolin-5-yl)methyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((2-(2,6-dioxopiperidin-3-yl)-1-thioxoisoindolin-5-yl)methyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((2-(2,6-dioxopiperidin-3-yl)-1-thioxoisoindolin-5-yl)methyl)-3,3-difluoropiperidin-4-yl)piperazin-1-yl)phenyl)-4-fluoro-1-oxoisoindolin-2-yl)-N-(thiazol-2-yl)acetamide;
3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-1-thioxoisoindolin-2-yl)piperidine-2,6-dione;
3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-1-thioxoisoindolin-2-yl)piperidine-2,6-dione;
3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-1-thioxoisoindolin-2-yl)piperidine-2,6-dione;
3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione;
3-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)-1-thioxoisoindolin-2-yl)piperidine-2,6-dione;
3-(5-((4-(1-(1-isopropyl-6-((2-(4-methoxypiperidin-l-yl)pyrimidin-4-yl)amino)-1H-pyrazolo[4,3-c]pyridin-3-yl)piperidin-4-yl)piperazin-1-yl)methyl)-1-thioxoisoindolin-2-yl)piperidine-2,6-dione;
N-(5-(((5-(tert-butyl)oxazol-2-yl)methyl)thio)thiazol-2-yl)-1-((2-(2,6-dioxopiperidin-3-yl)-1-thioxoisoindolin-5-yl)methyl)piperidine-4-carboxamide;
N-(5-(((5-(tert-butyl)oxazol-2-yl)methyl)thio)thiazol-2-yl)-1'-((2-(2,6-dioxopiperidin-3-yl)-1-thioxoisoindolin-5-yl)methyl)-[1,4'-bipiperidine]-4-carboxamide;
4-(2,6-dichlorobenzamido)-N-(1-((2-(2,6-dioxopiperidin- 3-yl)-1-thioxoisoindolin-5-yl)methyl)piperidin-4-yl)-1H-pyrazole-3-carboxamide;
N-(5-cyclobutyl-1H-pyrazol-3-yl)-2-(4-((2-(2,6-dioxopiperidin-3-yl)-1-thioxoisoindolin-5-yl)methoxy)phenyl)acetamide;
3-(5-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione;
4-(((4-(5-chloro-2-((1-((2-(2,6-dioxopiperidin-3-yl)-1-thioxoisoindolin-5-yl)methyl)piperidin-4-yl)amino)pyridin-4-yl)thiazol-2-yl)amino)methyl)tetrahydro-2H-pyran-4-carbonitrile;
4-(((5'-chloro-2'-((1-((2-(2,6-dioxopiperidin-3-yl)-1-thioxoisoindolin-5-yl)methyl)piperidin-4-yl)amino)-[2,4'-bipyridin]-6-yl)amino)methyl)tetrahydro-2H-pyran-4-carbonitrile;
4-(((5'-chloro-2' -((1-((2-(2,6-dioxopiperidin-3-yl)-1-thioxoisoindolin-4-yl)methyl)piperidin-4-yl)amino)-[2,4'-bipyridin]-6-yl)amino)methyl)tetrahydro-2H-pyran-4-carbonitrile;
N-(4-(chlorodifluoromethoxy)phenyl)-6-(4-((2-(2,6-dioxopiperidin-3-yl)-1-thioxoisoindolin-5-yl)methyl)piperazin-1-yl)-5-(1H-pyrazol-3-yl)nicotinamide;
N-(4-(chlorodifluoromethoxy)phenyl)-6-(4-(((2-(2,6-dioxopiperidin-3-yl)-1-thioxoisoindolin-5-yl)methyl)(methyl)amino)piperidin-1-yl)-5-(1H-pyrazol-3-yl)nicotinamide;
N-(4-(chlorodifluoromethoxy)phenyl)-6-(4-(1-((2-(2,6-dioxopiperidin-3-yl)-1-thioxoisoindolin-5-yl)methyl)piperidin-4-yl)piperazin-1-yl)-5-(1H-pyrazol-3-yl)nicotinamide;
N-(4-(chlorodifluoromethoxy)phenyl)-6-(4-(4-((2-(2,6-dioxopiperidin-3-yl)-1-thioxoisoindolin-5-yl)methyl)piperazin-1-yl)piperidin-1-yl)-5-(1H-pyrazol-3-yl)nicotinamide;
N-(4-((4-((2-(2,6-dioxopiperidin-3-yl)-1-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((2-(2,6-dioxopiperidin-3-yl)-1-thioxoisoindolin-4-yl)methyl)piperazin-1-yl)methyl)-3-(trifluoromethyl)phenyl)-3-(imidazo[1,2-b]pyridazin-3-ylethynyl)-4-methylbenzamide;
N-(4-((4-((2-(2,6-dioxopiperidin-3-yl)-3-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-1-thioxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-1-thioxoisoindolin-4-yl)methyl)piperazin-1-yl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-3-thioxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-1-thioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-1-thioxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
7-cyclopentyl-2-((5-(4-((2-(2,6-dioxopiperidin-3-yl)-1-thioxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-1-thioxoisoindolin-4-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((2-(2,6-dioxopiperidin-3-yl)-3-thioxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-1-thioxoisoindolin-5-yl)piperazin-1-yl)methyl)piperidin-l-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-1-thioxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(4-((2-(2,6-dioxopiperidin-3-yl)-1-thioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide;
3-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione;
3-(4-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione;
3-(6-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-thioxoisoindolin-2-yl)piperidine-2,6-dione;
3-(5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-thioxoisoindolin-2-yl)piperidine-2,6-dione;
3-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)-1-thioxoisoindolin-2-yl)piperidine-2,6-dione;
3-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-thioxoisoindolin-2-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione;
3-(4-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione;
3-(6-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-thioxoisoindolin-2-yl)piperidine-2,6-dione;
3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-1-thioxoisoindolin-2-yl)piperidine-2,6-dione;
3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-1-thioxoisoindolin-2-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-thioxoisoindolin-2-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)amino)methyl)-1-thioxoisoindolin-2-yl)piperidine-2,6-dione;
3-(5-((4-(4-((5-chloro-4-((2-(dimethylphosphoryl)phenyl)amino)pyrimidin-2-yl)amino)-3-methoxyphenyl)piperazin-1-yl)methyl)-1-thioxoisoindolin-2-yl)piperidine-2,6-dione;
3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-1-thioxoisoindolin-2-yl)piperidine-2,6-dione;
8-(4-(4-((2-(2,6-dioxopiperidin-3-yl)-1-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-(4-((2-(2,6-dioxopiperidin-3-yl)-3-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-((2-(2,6-dioxopiperidin-3-yl)-1-thioxoisoindolin-5-yl)methyl)piperazin-1-yl)-9-ethyl-6,6-dimethyl-11-oxo-6,11-dihydro-5H-benzo[b]carbazole-3-carbonitrile;
8-(4-(((2-(2,6-dioxopiperidin-3-yl)-1-thioxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
2-((2-((4-(4-(4-((2-(2,6-dioxopiperidin-3-yl)-1-thioxoisoindolin-5-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-1-thioxoisoindolin-5-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-1-thioxoisoindolin-4-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-3-thioxoisoindolin-5-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-1-thioxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-1-thioxoisoindolin-5-yl)methyl)piperazin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-1-thioxoisoindolin-4-yl)methyl)piperazin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-3-thioxoisoindolin-5-yl)methyl)piperazin-1-yl)-2-methoxyphenyl)amino)-5-(trifluoromethyl)pyridin-4-yl)amino)-N-methylbenzamide;
2-((2-((4-(1-((2-(2,6-dioxopiperidin-3-yl)-1-thioxoisoindolin-5-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,6-dioxopiperidin-3-yl)-1-thioxoisoindolin-4-yl)methyl)piperidin-4-yl)-2-isopropoxy-5-methylphenyl)amino)-5-(trifluoromethyl)pyridin-4-yl)amino)-N-methylbenzamide;
2-((2-((4-(1-((2-(2,6-dioxopiperidin-3-yl)-3-thioxoisoindolin-5-yl)methyl)piperidin-4-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-1-thioxoisoindolin-5-yl)methyl)piperazin-1-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-1-thioxoisoindolin-4-yl)methyl)piperazin-1-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-3-thioxoisoindolin-5-yl)methyl)piperazin-1-yl)-2-isopropoxy-5-methylphenyl)amino)-5-(trifluoromethyl)pyridin-4-yl)amino)-N-methylbenzamide;
2-((2-((4-(4-(((2-(2,6-dioxopiperidin-3-yl)-1-thioxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(1-((2-(2,6-dioxopiperidin-3-yl)-1-thioxoisoindolin-5-yl)methyl)piperidin-4-yl)piperazin-1-yl)-2-isopropoxy-5-methylphenyl)amino)-5-(trifluoromethyl)pyridin-4-yl)amino)-N-methylbenzamide;
N-(1-((2-(2,6-dioxopiperidin-3-yl)-1-thioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-((2-(2,6-dioxopiperidin-3-yl)-1-thioxoisoindolin-4-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(1-((2-(2,6-dioxopiperidin-3-yl)-3-thioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(3-(((2-((4-(4-((2-(2,6-dioxopiperidin-3-yl)-1-thioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-1-thioxoisoindolin-4-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-3-thioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-3-thioxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-1-thioxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-1-thioxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-1-thioxoisoindolin-4-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(4-((2-(2,6-dioxopiperidin-3-yl)-3-thioxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(1-((2-(2,6-dioxopiperidin-3-yl)-1-thioxoisoindolin-5-yl)methyl)piperidin-4-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-1-thioxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-1-thioxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
3-(5-((4-(4-((4-((3-(methylsulfonyl)benzyl)amino)-5-(trifluoromethyl)pyrimidin-2-yl)amino)phenyl)piperazin-1-yl)methyl)-1-thioxoisoindolin-2-yl)piperidine-2,6-dione;
2-(2-((cyclopropylmethyl)amino)pyridin-4-yl)-N-(3-(difluoromethyl)-1-(1-((2-(2,6-dioxopiperidin-3-yl)-1-thioxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((1-(2-(2,6-dioxopiperidin-3-yl)-1-thioxoisoindolin-4-yl)piperidin-4-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((1'-(2-(2,6-dioxopiperidin-3-yl)-1-thioxoisoindolin-5-yl)-[1,4'-bipiperidin]-4-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(4-(4-((2-(2,6-dioxopiperidin-3-yl)-1-thioxoisoindolin-5-yl)methyl)piperazin-1-yl)phenyl)-1H-pyrazol-4-yl)oxazole-4-carboxamide;
N-(2-(1-((2-(2,6-dioxopiperidin-3-yl)-1-thioxoisoindolin-5-yl)methyl)piperidin-4-yl)-6-methoxy-2H-indazol-5-yl)-3-(trifluoromethyl)benzamide;
N-(2-(1-((2-(2,6-dioxopiperidin-3-yl)-1-thioxoisoindolin-4-yl)methyl)piperidin-4-yl)-6-methoxy-2H-indazol-5-yl)-3-(trifluoromethyl)benzamide;
N-(2-(1-((2-(2,6-dioxopiperidin-3-yl)-3-thioxoisoindolin-5-yl)methyl)piperidin-4-yl)-6-methoxy-2H-indazol-5-yl)-3-(trifluoromethyl)benzamide;
N-(2-(1-((1-(2-(2,6-dioxopiperidin-3-yl)-1-thioxoisoindolin-5-yl)piperidin-4-yl)methyl)piperidin-4-yl)-6-methoxy-2H-indazol-5-yl)-3-(trifluoromethyl)benzamide;
N-(2-(1-((2-(2,6-dioxopiperidin-3-yl)-1-thioxoisoindolin-5-yl)methyl)piperidin-4-yl)-6-methoxy-2H-indazol-5-yl)-6-(trifluoromethyl)picolinamide;
N-(2-(1-((2-(2,6-dioxopiperidin-3-yl)-1-thioxoisoindolin-4-yl)methyl)piperidin-4-yl)-6-methoxy-2H-indazol-5-yl)-6-(trifluoromethyl)picolinamide;
N-(2-(1-((1-(2-(2,6-dioxopiperidin-3-yl)-1-thioxoisoindolin-5-yl)piperidin-4-yl)methyl)piperidin-4-yl)-6-methoxy-2H-indazol-5-yl)-6-(trifluoromethyl)picolinamide;
N-(2-(1-((2-(2,6-dioxopiperidin-3-yl)-1-thioxoisoindolin-5-yl)methyl)piperidin-4-yl)-6-methoxy-2H-indazol-7-yl)-3-(trifluoromethyl)benzamide;
N-(3-(difluoromethyl)-1-(1-((2-(2,6-dioxopiperidin-3-yl)-1-thioxoisoindolin-5-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((2-(2,6-dioxopiperidin-3-yl)-1-thioxoisoindolin-4-yl)methyl)piperidin-4-yl)-1H-pyrazol-4-yl)-5-morpholinopyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((2-(2,6-dioxopiperidin-3-yl)-1-thioxoisoindolin-5-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((2-(2,6-dioxopiperidin-3-yl)-1-thioxoisoindolin-4-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-1-thioxoisoindolin-5-yl)piperidin-4-yl)piperazin-1-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-1-thioxoisoindolin-5-yl)-3,3-difluoropiperidin-4-yl)piperazin-1-yl)methyl)cyclohexyl)-1H-pyrazol-4-yl)pyrazolo[1,5-a]pyrimidine-3-carboxamide;
N-(5-((4-((2-(2,6-dioxopiperidin-3-yl)-1-thioxoisoindolin-5-yl)methyl)piperazin- 1-yl)methyl)-1-((1s,4s)-4-(hydroxymethyl)cyclohexyl)-1H-benzo[d]imidazol-2-yl)-3-(trifluoromethyl)benzamide;
N-(5-(4-((2-(2,6-dioxopiperidin-3-yl)-1-thioxoisoindolin-5-yl)methyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-((1-(2-(2,6-dioxopiperidin-3-yl)-1-thioxoisoindolin-5-yl)piperidin-4-yl)methyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-((2-(2,6-dioxopiperidin-3-yl)-1-thioxoisoindolin-5-yl)methyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
6-(5-cyano-1H-pyrrolo[2,3-b]pyridin-1-yl)-N-(1-((2-(2,6-dioxopiperidin-3-yl)-1-thioxoisoindolin-5-yl)methyl)piperidin-4-yl)-4-(isopropylamino)nicotinamide;
N-(3-(difluoromethyl)-1-(1-((2-(2,6-dioxopiperidin-3-yl)-1-thioxoisoindolin-5-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((1-(2-(2,6-dioxopiperidin-3-yl)-1-thioxoisoindolin-5-yl)piperidin-4-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((1'-(2-(2,6-dioxopiperidin-3-yl)-1-thioxoisoindolin-5-yl)-[1,4'-bipiperidin]-4-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(4-(4-((2-(2,6-dioxopiperidin-3-yl)-1-thioxoisoindolin-5-yl)methyl)piperazin-1-yl)phenyl)-1H-pyrazol-4-yl)-2-(2-((2,2,2-trifluoroethyl)amino)pyridin-4-yl)oxazole-4-carboxamide;
N-(2-(4-((2-(2,6-dioxopiperidin-3-yl)-1-thioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(4-((2-(2,6-dioxopiperidin-3-yl)-1-thioxoisoindolin-4-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(1-((2-(2,6-dioxopiperidin-3-yl)-1-thioxoisoindolin-5-yl)methyl)piperidin-4-yl)-5-(2-hydroxypropan-2-yl)benzo[d]thiazol-6-yl)-6-(trifluoromethyl)picolinamide;
N-(2-(1-((2-(2,6-dioxopiperidin-3-yl)-1-thioxoisoindolin-5-yl)methyl)piperidin-4-yl)-5-(2-hydroxypropan-2-yl)benzo[d]oxazol-6-yl)-6-(trifluoromethyl)picolinamide;
N-(2-(1-((2-(2,6-dioxopiperidin-3-yl)-1-thioxoisoindolin-5-yl)methyl)piperidin-4-yl)-6-(2-hydroxypropan-2-yl)-2H-indazol-5-yl)-6-(trifluoromethyl)picolinamide;
6-(6-(4-((2-(2,6-dioxopiperidin-3-yl)-1-thioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-1-thioxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-1-thioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
N-((4,6-dimethyl-2-oxo-1,2-dihydropyridin-3-yl)methyl)-4'-((4-((2-(2,6-dioxopiperidin-3-yl)-1-thioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-1-thioxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(((2-(2,6-dioxopiperidin-3-yl)-1-thioxoisoindolin-5-yl)methyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-((2-(2,6-dioxopiperidin-3-yl)-1-thioxoisoindolin-5-yl)methyl)piperidin-4-yl)piperazin-1-yl)-5-(ethyl(tetrahydro-2H-pyran-4-yl)amino)-4-methyl-[1,1'-biphenyl]-3-carboxamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)piperazin-1-yl-2,2,3,3,5,5,6,6-d8)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-1-thioxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-thioxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1-thioxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-thioxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-1-thioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-1-thioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-1-thioxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-thioxoisoindolin-5-yl)methyl)piperazin-1-yl)-1-(phenylthio)butan-2-yl)amino)-3-((trifluoromethyl)sulfonyl)phenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-(((1-((2-(2,6-dioxopiperidin-3-yl)-1-thioxoisoindolin-5-yl)methyl)piperidin-4-yl)methyl)amino)-3-nitrophenyl)sulfonyl)benzamide;
(9R)-N-(1-(4-(((2-(2,6-dioxopiperidin-3-yl)-1-thioxoisoindolin-5-yl)methyl)amino)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((2-(2,6-dioxopiperidin-3-yl)-1-thioxoisoindolin-5-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(((2-(2,6-dioxopiperidin-3-yl)-1-thioxoisoindolin-5-yl)methyl)(methyl)amino)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(((2-(2,6-dioxopiperidin-3-yl)-4-fluoro-1-thioxoisoindolin-5-yl)methyl)(methyl)amino)piperidin-1-yl)benzyl)-1H -pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(4-((2-(2,6-dioxopiperidin-3-yl)-1-thioxoisoindolin-5-yl)methyl)piperazin-1-yl)piperidin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
3-(5-((4-(3-amino-6-(2-hydroxyphenyl)pyridazin-4-yl)piperazin-1-yl)methyl)-1-thioxoisoindolin-2-yl)piperidine-2,6-dione;
3-(5-((4-(3-amino-6-(5-fluoro-2-hydroxyphenyl)pyridazin-4-yl)piperazin-1-yl)methyl)-1-thioxoisoindolin-2-yl)piperidine-2,6-dione;
3-(5-((4-(4-amino-3-(4-phenoxyphenyl)-1H-pyrazolo[3,4-d]pyrimidin-1-yl)piperidin-1-yl)methyl)-3-oxo-1-thioxoisoindolin-2-yl)piperidine-2,6-dione;
3-(5-(4-((4-(4-amino-3-(4-phenoxyphenyl)-1H-pyrazolo[3,4-d]pyrimidin-1-yl)piperidin-1-yl)methyl)piperidin-1-yl)-3-oxo-1-thioxoisoindolin-2-yl)piperidine-2,6-dione;
3-(4-(4-((4-(4-amino-3-(4-phenoxyphenyl)-1H-pyrazolo [3,4-d]pyrimidin-1-yl)piperidin-1-yl)methyl)piperidin-1-yl)-3-oxo-1-thioxoisoindolin-2-yl)piperidine-2,6-dione;
3-(5-(4-((4-(4-amino-3-(4-phenoxyphenyl)-1H-pyrazolo[3,4-d]pyrimidin-1-yl)piperidin-1-yl)methyl)-[1,4'-bipiperidin]-1'-yl)-3-oxo-1-thioxoisoindolin-2-yl)piperidine-2,6-dione;
3-(5-((4-(4-((1R,2S)-6-hydroxy-2-phenyl-1,2,3,4-tetrahydronaphthalen-1-yl)phenyl)piperazin-1-yl)methyl)-1-oxo-3-thioxoisoindolin-2-yl)piperidine-2,6-dione;
3-(5-((4-(4-((3S,4R)-7-hydroxy-3-phenylchroman-4-yl)phenyl)piperazin-1-yl)methyl)-1-oxo-3-thioxoisoindolin-2-yl)piperidine-2,6-dione;
N-((1r,3r)-3-(3-chloro-4-cyanophenoxy)-2,2,4,4-tetramethylcyclobutyl)-6-(4-((4-(2-(2,6-dioxopiperidin-3-yl)-3-oxo-1-thioxoisoindolin-5-yl)piperazin-1-yl)methyl)piperidin-1-yl)nicotinamide;
N-((1r,3S)-3-(3-chloro-4-cyanophenoxy)-2,2,4,4-tetramethylcyclobutyl)-6-(4-(((1S,4S)-5-(2-(2,6-dioxopiperidin-3-yl)-3-oxo-1-thioxoisoindolin-5-yl)-2,5-diazabicyclo[2.2.1]heptan-2-yl)methyl)piperidin-1-yl)nicotinamide;
N-((1r,3R)-3-(3-chloro-4-cyanophenoxy)-2,2,4,4-tetramethylcyclobutyl)-6-(4-(((1R,4R)-5-(2-(2,6-dioxopiperidin-3-yl)-3-oxo-1-thioxoisoindolin-5-yl)-2,5-diazabicyclo[2.2.1]heptan-2-yl)methyl)piperidin-1-yl)nicotinamide;
N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-3-oxo-1-thioxoisoindolin-5-yl)piperazin-1-yl)methyl)piperidin-1-yl)nicotinamide;
N-((1r,3r)-3-(3-chloro-4-cyanophenoxy)-2,2,4,4-tetramethylcyclobutyl)-6-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)nicotinamide;
N-((1r,3r)-3-(3-chloro-4-cyanophenoxy)-2,2,4,4-tetramethylcyclobutyl)-6-(4-((6-(2-(2,6-dioxopiperidin-3-yl)-3-oxo-1-thioxoisoindolin-5-yl)-3,6-diazabicyclo[3.1.1]heptan-3-yl)methyl)piperidin-1-yl)nicotinamide;
N-((1r,3r)-3-(3-chloro-4-cyanophenoxy)-2,2,4,4-tetramethylcyclobutyl)-6-(4-((4-(2-(2,6-dioxopiperidin-3-yl)-3-oxo-1-thioxoisoindolin-4-yl)piperazin-1-yl)methyl)piperidin-1-yl)nicotinamide;
N-((1r,3r)-3-(3-chloro-4-cyanophenoxy)-2,2,4,4-tetramethylcyclobutyl)-6-(4-((4-(2-(2,6-dioxopiperidin-3-yl)-7-fluoro-3-oxo-1-thioxoisoindolin-5-yl)piperazin-1-yl)methyl)piperidin-1-yl)nicotinamide;
N-((1r,3r)-3-(3-chloro-4-cyanophenoxy)-2,2,4,4-tetramethylcyclobutyl)-6-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)nicotinamide;
N-((1r,4r)-4-(3-chloro-4-cyanophenoxy)cyclohexyl)-6-(4-((4-(2-(2,6-dioxopiperidin-3-yl)-3-oxo-1-thioxoisoindolin-5-yl)piperazin-1-yl)methyl)piperidin-1-yl)pyridazine-3-carboxamide;
N-((1r,4r)-4-(3-chloro-4-cyanophenoxy)cyclohexyl)-6-(4-(((1S,4S)-5-(2-(2,6-dioxopiperidin-3-yl)-3-oxo-1-thioxoisoindolin-5-yl)-2,5-diazabicyclo[2.2.1]heptan-2-yl)methyl)piperidin-1-yl)pyridazine-3-carboxamide;
N-((1r,4r)-4-(3-chloro-4-cyanophenoxy)cyclohexyl)-6-(4-(((1R,4R)-5-(2-(2,6-dioxopiperidin-3-yl)-3-oxo-1-thioxoisoindolin-5-yl)-2,5-diazabicyclo[2.2.1]heptan-2-yl)methyl)piperidin-1-yl)pyridazine-3-carboxamide;
N-((1r,4r)-4-(3-chloro-4-cyanophenoxy)cyclohexyl)-6-(4-((4-(2-(2,6-dioxopiperidin-3-yl)-6-fluoro-3-oxo-1-thioxoisoindolin-5-yl)piperazin-1-yl)methyl)piperidin-1-yl)pyridazine-3-carboxamide;
N-((1r,4r)-4-(3-chloro-4-cyanophenoxy)cyclohexyl)-6-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)pyridazine-3-carboxamide;
N-((1r,4r)-4-(3-chloro-4-cyanophenoxy)cyclohexyl)-6-(4-((4-(2-(2,6-dioxopiperidin-3-yl)-3-oxo-1-thioxoisoindolin-4-yl)piperazin-1-yl)methyl)piperidin-1-yl)pyridazine-3-carboxamide;
N-((1r,4r)-4-(3-chloro-4-cyanophenoxy)cyclohexyl)-6-(4-((6-(2-(2,6-dioxopiperidin-3-yl)-3-oxo-1-thioxoisoindolin-5-yl)-3,6-diazabicyclo[3.1.1]heptan-3-yl)methyl)piperidin-1-yl)pyridazine-3-carboxamide;
N-((1r,4r)-4-(3-chloro-4-cyanophenoxy)cyclohexyl)-6-(4-((4-(2-(2,6-dioxopiperidin-3-yl)-4-fluoro-3-oxo-1-thioxoisoindolin-5-yl)piperazin-1-yl)methyl)piperidin-1-yl)pyridazine-3-carboxamide;
N-((1r,4r)-4-(3-chloro-4-cyanophenoxy)cyclohexyl)-6-(4-((4-(2-(2,6-dioxopiperidin-3-yl)-7-fluoro-3-oxo-1-thioxoisoindolin-5-yl)piperazin-1-yl)methyl)piperidin-1-yl)pyridazine-3-carboxamide;
N-((1r,4r)-4-(3-chloro-4-cyanophenoxy)cyclohexyl)-6-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)pyridazine-3-carboxamide;
3-(5-((4-(4-((9-cyclopentyl-8-(phenylamino)-9H-purin-2-yl)amino)phenyl)piperazin-1-yl)methyl)-1-oxo-3-thioxoisoindolin-2-yl)piperidine-2,6-dione;
3-(5-(((1-(4-((9-cyclopentyl-8-(phenylamino)-9H-purin-2-yl)amino)phenyl)piperidin-4-yl)(methyl)amino)methyl)-1-oxo-3-thioxoisoindolin-2-yl)piperidine-2,6-dione;
3-(5-((4-(1-(4-((9-cyclopentyl-8-(phenylamino)-9H-purin-2-yl)amino)phenyl)piperidin-4-yl)piperazin-1-yl)methyl)-3-oxo-1-thioxoisoindolin-2-yl)piperidine-2,6-dione;
3-(5-((4-(4-((5-bromo-4-((5-(dimethylphosphoryl)quinoxalin-6-yl)amino)pyrimidin-2-yl)amino)-5-methoxy-2-methylphenyl)piperazin-1-yl)methyl)-3-oxo-1-thioxoisoindolin-2-yl)piperidine-2,6-dione;
3-(5-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-3-oxo-1-thioxoisoindolin-2-yl)piperidine-2,6-dione;
3-(5-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-3-oxo-1-thioxoisoindolin-2-yl)piperidine-2,6-dione;
2-(7-(((2-(2,6-dioxopiperidin-3-yl)-3-oxo-1-thioxoisoindolin-5-yl)methyl)amino)-1-oxoisoindolin-2-yl)-2-(5-fluoro-2-hydroxyphenyl)-N-(thiazol-2-yl)acetamide;
2-(6-(4-((2-(2,6-dioxopiperidin-3-yl)-3-oxo-1-thioxoisoindolin-5-yl)methyl)piperazin-1-yl)-1-oxoisoindolin-2-yl)-2-(5-fluoro-2-hydroxyphenyl)-N-(thiazol-2-yl)acetamide;
2-(6-(4-(((2-(2,6-dioxopiperidin-3-yl)-3-oxo-1-thioxoisoindolin-5-yl)methyl)(methyl)amino)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-3-oxo-1-thioxoisoindolin-5-yl)methyl)piperazin-1-yl)piperidin-1-yl)-1-oxoisoindolin-2-yl)-2-(5-fluoro-2-hydroxyphenyl)-N-(thiazol-2-yl)acetamide;
2-(6-(4-(4-((2-(2,6-dioxopiperidin-3-yl)-3-oxo-1-thioxoisoindolin-5-yl)methyl)piperazin-1-yl)phenyl)-1-oxoisoindolin-2-yl)-2-(5-fluoro-2-hydroxyphenyl)-N-(thiazol-2-yl)acetamide;
2-(6-(6-(4-((2-(2,6-dioxopiperidin-3-yl)-3-oxo-1-thioxoisoindolin-5-yl)methyl)piperazin-1-yl)pyridazin-3-yl)-1-oxoisoindolin-2-yl)-2-(5-fluoro-2-hydroxyphenyl)-N-(thiazol-2-yl)acetamide;
2-(6,7-dihydro-5H-pyrrolo[1,2-c]imidazol-1-yl)-2-(6-(4-(4-((2-(2,6-dioxopiperidin-3-yl)-3-oxo-1-thioxoisoindolin-5-yl)methyl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1S,4S)-5-((2-(2,6-dioxopiperidin-3-yl)-3-oxo-1-thioxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((2-(2,6-dioxopiperidin-3-yl)-3-oxo-1-thioxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(8-((2-(2,6-dioxopiperidin-3-yl)-3-oxo-1-thioxoisoindolin-5-yl)methyl)-3,8-diazabicyclo[3.2.1]octan-3-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(((2-(2,6-dioxopiperidin-3-yl)-3-oxo-1-thioxoisoindolin-5-yl)methyl)(methyl)amino)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((2-(2,6-dioxopiperidin-3-yl)-3-oxo-1-thioxoisoindolin-5-yl)methyl)piperazin-1-yl)piperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(4-((2-(2,6-dioxopiperidin-3-yl)-3-oxo-1-thioxoisoindolin-5-yl)methyl)piperazin-1-yl)-3,3-difluoropiperidin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((2-(2,6-dioxopiperidin-3-yl)-3-oxo-1-thioxoisoindolin-5-yl)methyl)piperidin-4-yl)piperazin-1-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-(4-(1-((2-(2,6-dioxopiperidin-3-yl)-3-oxo-1-thioxoisoindolin-5-yl)methyl)-3,3-difluoropiperidin-4-yl)piperazin-1-yl)phenyl)-4-fluoro-1-oxoisoindolin-2-yl)-N-(thiazol-2-yl)acetamide;
3-(5-((4-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperazin-1-yl)methyl)-3-oxo-1-thioxoisoindolin-2-yl)piperidine-2,6-dione;
3-(5-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)(methyl)amino)methyl)-3-oxo-1-thioxoisoindolin-2-yl)piperidine-2,6-dione;
3-(5-((4-(1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)piperazin-1-yl)methyl)-3-oxo-1-thioxoisoindolin-2-yl)piperidine-2,6-dione;
3-(5-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-3-oxo-1-thioxoisoindolin-2-yl)piperidine-2,6-dione;
3-(5-(((1-(1-isopropyl-6-((2-(4-methoxypiperidin-1-yl)pyrimidin-4-yl)amino)-1H-pyrazolo[4,3-c]pyridin-3-yl)piperidin-4-yl)(methyl)amino)methyl)-3-oxo-1-thioxoisoindolin-2-yl)piperidine-2,6-dione;
3-(5-((4-(1-(1-isopropyl-6-((2-(4-methoxypiperidin-1-yl)pyrimidin-4-yl)amino)-1H-pyrazolo[4,3-c]pyridin-3-yl)piperidin-4-yl)piperazin-1-yl)methyl)-3-oxo-1-thioxoisoindolin-2-yl)piperidine-2,6-dione;
N-(5-(((5-(tert-butyl)oxazol-2-yl)methyl)thio)thiazol-2-yl)-1-((2-(2,6-dioxopiperidin-3-yl)-3-oxo-1-thioxoisoindolin-5-yl)methyl)piperidine-4-carboxamide;
N-(5-(((5-(tert-butyl)oxazol-2-yl)methyl)thio)thiazol-2-yl)-1'-((2-(2,6-dioxopiperidin-3-yl)-3-oxo-1-thioxoisoindolin-5-yl)methyl)-[1,4'-bipiperidine]-4-carboxamide;
4-(2,6-dichlorobenzamido)-N-(1-((2-(2,6-dioxopiperidin-3-yl)-3-oxo-1-thioxoisoindolin-5-yl)methyl)piperidin-4-yl)-1H-pyrazole-3-carboxamide;
4-(2,6-dichlorobenzamido)-N-(1-((4-(2-(2,6-dioxopiperidin-3-yl)-3-oxo-1-thioxoisoindolin-5-yl)piperazin-1-yl)methyl)piperidin-4-yl)-1H-pyrazole-3-carboxamide;
N-(5-cyclobutyl-1H-pyrazol-3-yl)-2-(4-((2-(2,6-dioxopiperidin-3-yl)-3-oxo-1-thioxoisoindolin-5-yl)methoxy)phenyl)acetamide;
3-(5-((4-((5-chloro-4-(5-(cyclopropylmethyl)-1-methyl-1H-pyrazol-4-yl)pyrimidin-2-yl)amino)piperidin-1-yl)methyl)-3-oxo-1-thioxoisoindolin-2-yl)piperidine-2,6-dione;
4-(((4-(5-chloro-2-((1-((2-(2,6-dioxopiperidin-3-yl)-3-oxo-1-thioxoisoindolin-5-yl)methyl)piperidin-4-yl)amino)pyridin-4-yl)thiazol-2-yl)amino)methyl)tetrahydro-2H-pyran-4-carbonitrile;
4-(((5'-chloro-2'-((1-((2-(2,6-dioxopiperidin-3-yl)-3-oxo-1-thioxoisoindolin-5-yl)methyl)piperidin-4-yl)amino)-[2,4'-bipyridin]-6-yl)amino)methyl)tetrahydro-2H-pyran-4-carbonitrile;
N-(4-(chlorodifluoromethoxy)phenyl)-6-(4-((2-(2,6-dioxopiperidin-3-yl)-1-oxo-3-thioxoisoindolin-5-yl)methyl)piperazin-1-yl)-5-(1H-pyrazol-3-yl)nicotinamide;
N-(4-(chlorodifluoromethoxy)phenyl)-6-(4-(((2-(2,6-dioxopiperidin-3-yl)-3-oxo-1-thioxoisoindolin-5-yl)methyl)(methyl)amino)piperidin-1-yl)-5-(1H-pyrazol-3-yl)nicotinamide;
N-(4-(chlorodifluoromethoxy)phenyl)-6-(4-(1-((2-(2,6-dioxopiperidin-3-yl)-3-oxo-1-thioxoisoindolin-5-yl)methyl)piperidin-4-yl)piperazin-1-yl)-5-(1H-pyrazol-3-yl)nicotinamide;
N-(4-(chlorodifluoromethoxy)phenyl)-6-(4-(4-((2-(2,6-dioxopiperidin-3-yl)-3-oxo-1-thioxoisoindolin-5-yl)methyl)piperazin-1-yl)piperidin-1-yl)-5-(1H-pyrazol-3-yl)nicotinamide;
N-(4-((4-((2-(2,6-dioxopiperidin-3-yl)-3-oxo-1-thioxoisoindolin-5-yl)methyl)piperazin-1-yl)methyl)-3-(trifluoromethyl)phenyl)-3-(imidazo[1,2-b]pyridazin-3-ylethynyl)-4-methylbenzamide;
N-(4-(4-((2-(2,6-dioxopiperidin-3-yl)-1-oxo-3-thioxoisoindolin-5-yl)methyl)piperazin-1-yl)-3-(trifluoromethyl)phenyl)-3-(imidazo[1,2-b]pyridazin-3-ylethynyl)-4-methylbenzamide;
N-(4-(4-(1-((2-(2,6-dioxopiperidin-3-yl)-1-oxo-3-thioxoisoindolin-5-yl)methyl)piperidin-4-yl)piperazin-1-yl)-3-(trifluoromethyl)phenyl)-3-(imidazo[1,2-b]pyridazin-3-ylethynyl)-4-methylbenzamide;
N-(4-(4-(((2-(2,6-dioxopiperidin-3-yl)-3-oxo-1-thioxoisoindolin-5-yl)methyl)(methyl)amino)piperidin-1-yl)-3-(trifluoromethyl)phenyl)-3-(imidazo[1,2-b]pyridazin-3-ylethynyl)-4-methylbenzamide;
7-cyclopentyl-2-((5-(4-((2-(2,6-dioxopiperidin-3-yl)-1-oxo-3-thioxoisoindolin-5-yl)methyl)piperazin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-(((2-(2,6-dioxopiperidin-3-yl)-3-oxo-1-thioxoisoindolin-5-yl)methyl)(methyl)amino)piperidin-1-yl)pyridin-2-yl)amino)-N,N-dimethyl-7H-pyrrolo[2,3-d]pyrimidine-6-carboxamide;
7-cyclopentyl-2-((5-(4-(4-((2-(2,6-dioxopiperidin-3-yl)-3-oxo-1-thioxoisoindolin-5-yl)methyl)piperazin-1-yl)piperidin-1-yl)pyridin-2-yl)amino)-N,N-dimethyl-7H-pyrrolo[2,3-d]pyrimidine-6-carboxamide;
7-cyclopentyl-2-((5-(4-(((1S,4S)-5-(2-(2,6-dioxopiperidin-3-yl)-3-oxo-1-thioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-(((1R,4R)-5-(2-(2,6-dioxopiperidin-3-yl)-3-oxo-1-thioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-6-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((8-(2-(2,6-dioxopiperidin-3-yl)-3-oxo-1-thioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((6-(2-(2,6-dioxopiperidin-3-yl)-3-oxo-1-thioxoisoindolin-5-yl)-3,6-diazabicyclo[3.1.1]heptan-3-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-3-oxo-1-thioxoisoindolin-4-yl)piperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-4-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-7-fluoro-3-oxo-1-thioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(1-(2-(2,6-dioxopiperidin-3-yl)-3-oxo-1-thioxoisoindolin-5-yl)piperidin-4-yl)piperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(1-(2-(2,6-dioxopiperidin-3-yl)-3-oxo-1-thioxoisoindolin-5-yl)-3,3-difluoropiperidin-4-yl)piperazin-1-yl)methyl)piperidin-1-yl)pyridin-2-yl)amino)-N,N-dimethyl-7H-pyrrolo[2,3-d]pyrimidine-6-carboxamide;
3-(5-((4-((6-((5-fluoro-4-(4-fluoro-1-isopropyl-2-methyl-1H-benzo[d]imidazol-6-yl)pyrimidin-2-yl)amino)pyridin-3-yl)methyl)piperazin-1-yl)methyl)-1-oxo-3-thioxoisoindolin-2-yl)piperidine-2,6-dione;
3-(5-((4-(6-((5-fluoro-4-(4-fluoro-1-isopropyl-2-methyl-1H-benzo[d]imidazol-6-yl)pyrimidin-2-yl)amino)pyridin-3-yl)piperazin-1-yl)methyl)-1-oxo-3-thioxoisoindolin-2-yl)piperidine-2,6-dione;
3-(5-(((1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)(methyl)amino)methyl)-1-oxo-3-thioxoisoindolin-2-yl)piperidine-2,6-dione;
3-(5-((4-(1-(6-((5-fluoro-4-(4-fluoro-1-isopropyl-2-methyl-1H-benzo[d]imidazol-6-yl)pyrimidin-2-yl)amino)pyridin-3-yl)piperidin-4-yl)piperazin-1-yl)methyl)-1-oxo-3-thioxoisoindolin-2-yl)piperidine-2,6-dione;
3-(5-((4-(6-((6-acetyl-8-cyclopentyl-5-methyl-7-oxo-7,8-dihydropyrido[2,3-d]pyrimidin-2-yl)amino)pyridin-3-yl)piperazin-1-yl)methyl)-1-oxo-3-thioxoisoindolin-2-yl)piperidine-2,6-dione;
3-(5-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)(methyl)amino)methyl)-3-oxo-1-thioxoisoindolin-2-yl)piperidine-2,6-dione;
3-(5-((4-(1-(6-((6-acetyl-8-cyclopentyl-5-methyl-7-oxo-7,8-dihydropyrido[2,3-d]pyrimidin-2-yl)amino)pyridin-3-yl)piperidin-4-yl)piperazin-1-yl)methyl)-3-oxo-1-thioxoisoindolin-2-yl)piperidine-2,6-dione;
3-(5-((4-(4-(6-((6-acetyl-8-cyclopentyl-5-methyl-7-oxo-7,8-dihydropyrido[2,3-d]pyrimidin-2-yl)amino)pyridin-3-yl)piperazin-1-yl)piperidin-1-yl)methyl)-3-oxo-1-thioxoisoindolin-2-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-3-oxo-1-thioxoisoindolin-2-yl)piperidine-2,6-dione;
3-(5-(((1-(4-((5-chloro-4-((2-(dimethylphosphoryl)phenyl)amino)pyrimidin-2-yl)amino)-3-methoxyphenyl)piperidin-4-yl)amino)methyl)-3-oxo-1-thioxoisoindolin-2-yl)piperidine-2,6-dione;
3-(5-((4-(4-((5-chloro-4-((2-(dimethylphosphoryl)phenyl)amino)pyrimidin-2-yl)amino)-3-methoxyphenyl)piperazin-1-yl)methyl)-3-oxo-1-thioxoisoindolin-2-yl)piperidine-2,6-dione;
3-(5-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)-3-oxo-1-thioxoisoindolin-2-yl)piperidine-2,6-dione;
8-(4-(4-((2-(2,6-dioxopiperidin-3-yl)-3-oxo-1-thioxoisoindolin-5-yl)methyl)piperazin-1-yl)piperidin-1-yl)-9-ethyl-6,6-dimethyl-11-oxo-6,11-dihydro-5H-benzo[b]carbazole-3-carbonitrile;
8-(4-((2-(2,6-dioxopiperidin-3-yl)-3-oxo-1-thioxoisoindolin-5-yl)methyl)piperazin-1-yl)-9-ethyl-6,6-dimethyl-11-oxo-6,11-dihydro-5H-benzo[b]carbazole-3-carbonitrile;
8-(4-(((2-(2,6-dioxopiperidin-3-yl)-3-oxo-1-thioxoisoindolin-5-yl)methyl)(methyl)amino)piperidin-1-yl)-9-ethyl-6,6-dimethyl-11-oxo-6,11-dihydro-5H-benzo[b]carbazole-3-carbonitrile;
3-(5-((4-(1-(4-(5,7-dimethoxy-4-oxo-3,4-dihydroquinazolin-2-yl)phenyl)piperidin-4-yl)piperazin-1-yl)methyl)-3-oxo-1-thioxoisoindolin-2-yl)piperidine-2,6-dione;
2-((2-((4-(4-(4-((2-(2,6-dioxopiperidin-3-yl)-3-oxo-1-thioxoisoindolin-5-yl)methyl)piperazin-1-yl)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-3-oxo-1-thioxoisoindolin-5-yl)methyl)amino)piperidin-1-yl)-2-methoxyphenyl)amino)-5-(trifluoromethyl)pyridin-4-yl)amino)-N-methylbenzamide;
2-((2-((4-(4-(((2-(2,6-dioxopiperidin-3-yl)-3-oxo-1-thioxoisoindolin-5-yl)methyl)(methyl)amino)piperidin-1-yl)-2-methoxyphenyl)amino)-5-(trifluoromethyl)pyridin-4-yl)amino)-N-methylbenzamide;
2-((2-((4-(4-((2-(2,6-dioxopiperidin-3-yl)-3-oxo-1-thioxoisoindolin-5-yl)methyl)piperazin-1-yl)-2-methoxyphenyl)amino)-5-(trifluoromethyl)pyridin-4-yl)amino)-N-methylbenzamide;
2-((2-((4-(1-((2-(2,6-dioxopiperidin-3-yl)-3-oxo-1-thioxoisoindolin-5-yl)methyl)piperidin-4-yl)-2-isopropoxy-5-methylphenyl)amino)-5-(trifluoromethyl)pyridin-4-yl)amino)-N-methylbenzamide;
2-((2-((4-(4-((2-(2,6-dioxopiperidin-3-yl)-3-oxo-1-thioxoisoindolin-5-yl)methyl)piperazin-1-yl)-2-isopropoxy-5-methylphenyl)amino)-5-(trifluoromethyl)pyridin-4-yl)amino)-N-methylbenzamide;
2-((2-((4-(4-(((2-(2,6-dioxopiperidin-3-yl)-3-oxo-1-thioxoisoindolin-5-yl)methyl)(methyl)amino)piperidin-1-yl)-2-isopropoxy-5-methylphenyl)amino)-5-(trifluoromethyl)pyridin-4-yl)amino)-N-methylbenzamide;
2-((2-((4-(4-(1-((2-(2,6-dioxopiperidin-3-yl)-3-oxo-1-thioxoisoindolin-5-yl)methyl)piperidin-4-yl)piperazin-1-yl)-2-isopropoxy-5-methylphenyl)amino)-5-(trifluoromethyl)pyridin-4-yl)amino)-N-methylbenzamide;
N-(1-((2-(2,6-dioxopiperidin-3-yl)-3-oxo-1-thioxoisoindolin-5-yl)methyl)piperidin-4-yl)-2-fluoro-5-methoxy-4-((4-((2-methyl-3-oxoisoindolin-4-yl)oxy)-5-(trifluoromethyl)pyrimidin-2-yl)amino)benzamide;
N-(3-(((2-((4-(4-((2-(2,6-dioxopiperidin-3-yl)-3-oxo-1-thioxoisoindolin-5-yl)methyl)piperazin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(((2-(2,6-dioxopiperidin-3-yl)-1-oxo-3-thioxoisoindolin-5-yl)methyl)(methyl)amino)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-(4-((2-(2,6-dioxopiperidin-3-yl)-1-oxo-3-thioxoisoindolin-5-yl)methyl)piperazin-1-yl)piperidin-1-yl)phenyl)amino)-5-(trifluoromethyl)pyrimidin-4-yl)amino)methyl)pyrazin-2-yl)-N-methylmethanesulfonamide;
N-(3-(((2-((4-(4-((2-(2,6-dioxopiperidin-3-yl)-3-oxo-1-thioxoisoindolin-5-yl)methyl)piperazin-1-yl)phenyl)amino)-5-(trifluoromethyl)pyrimidin-4-yl)amino)methyl)phenyl)-N-methylmethanesulfonamide;
N-(3-(((2-((4-(1-((2-(2,6-dioxopiperidin-3-yl)-3-oxo-1-thioxoisoindolin-5-yl)methyl)piperidin-4-yl)phenyl)amino)-5-(trifluoromethyl)pyrimidin-4-yl)amino)methyl)phenyl)-N-methylmethanesulfonamide;
N-(3-(((2-((4-(4-(((2-(2,6-dioxopiperidin-3-yl)-1-oxo-3-thioxoisoindolin-5-yl)methyl)(methyl)amino)piperidin-1-yl)phenyl)amino)-5-(trifluoromethyl)pyrimidin-4-yl)amino)methyl)phenyl)-N-methylmethanesulfonamide;
N-(3-(((2-((4-(4-(4-((2-(2,6-dioxopiperidin-3-yl)-3-oxo-1-thioxoisoindolin-5-yl)methyl)piperazin-1-yl)piperidin-1-yl)phenyl)amino)-5-(trifluoromethyl)pyrimidin-4-yl)amino)methyl)phenyl)-N-methylmethanesulfonamide;
3-(5-((4-(4-((4-((3-(methylsulfonyl)benzyl)amino)-5-(trifluoromethyl)pyrimidin-2-yl)amino)phenyl)piperazin-1-yl)methyl)-3-oxo-1-thioxoisoindolin-2-yl)piperidine-2,6-dione;
2-(2-((cyclopropylmethyl)amino)pyridin-4-yl)-N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((1-(2-(2,6-dioxopiperidin-3-yl)-1-oxo-3-thioxoisoindolin-4-yl)piperidin-4-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(1-((1'-(2-(2,6-dioxopiperidin-3-yl)-3-oxo-1-thioxoisoindolin-5-yl)-[1,4'-bipiperidin]-4-yl)methyl)piperidin-4-yl)-1H-pyrazol-4-yl)oxazole-4-carboxamide;
2-(2-((cyclopropylmethyl)amino)pyridin-4-yl)-N-(3-(difluoromethyl)-1-(4-(4-((2-(2,6-dioxopiperidin-3-yl)-3-oxo-1-thioxoisoindolin-5-yl)methyl)piperazin-1-yl)phenyl)-1H-pyrazol-4-yl)oxazole-4-carboxamide;
N-(2-(1-((2-(2,6-dioxopiperidin-3-yl)-3-oxo-1-thioxoisoindolin-5-yl)methyl)piperidin-4-yl)-6-methoxy-2H-indazol-5-yl)-3-(trifluoromethyl)benzamide;
N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-5-yl)-3-(trifluoromethyl)benzamide;
N-(2-(1-((2-(2,6-dioxopiperidin-3-yl)-3-oxo-1-thioxoisoindolin-5-yl)methyl)piperidin-4-yl)-6-methoxy-2H-indazol-5-yl)-6-(trifluoromethyl)picolinamide;
N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-5-yl)-6-(trifluoromethyl)picolinamide;
N-(2-(1-((1-(2-(2,6-dioxopiperidin-3-yl)-3-oxo-1-thioxoisoindolin-5-yl)piperidin-4-yl)methyl)piperidin-4-yl)-6-methoxy-2H-indazol-7-yl)-3-(trifluoromethyl)benzamide;
N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((1-(2-(2,6-dioxopiperidin-3-yl)-3-oxo-1-thioxoisoindolin-4-yl)piperidin-4-yl)methyl)piperidin-4-yl)-1H-pyrazol-4-yl)-5-morpholinopyrazolo[1,5-a]pyrimidine-3-carboxamide;
N-(3-(difluoromethyl)-1-(1-((1-(2-(2,6-dioxopiperidin-3-yl)-3-oxo-1-thioxoisoindolin-5-yl)piperidin-4-yl)methyl)piperidin-4-yl)-1H-pyrazol-4-yl)-5-morpholinopyrazolo[1,5-a]pyrimidine-3-carboxamide;
5-((1S,4S)-2-oxa-5-azabicyclo[2.2.1]heptan-5-yl)-N-(3-(difluoromethyl)-1-(1-((2-(2,6-dioxopiperidin-3-yl)-3-oxo-1-thioxoisoindolin-5-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((1-(2-(2,6-dioxopiperidin-3-yl)-3-oxo-1-thioxoisoindolin-5-yl)piperidin-4-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-(1-((1-(2-(2,6-dioxopiperidin-3-yl)-3-oxo-1-thioxoisoindolin-4-yl)piperidin-4-yl)methyl)piperidin-4-yl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-3-oxo-1-thioxoisoindolin-5-yl)piperazin-1-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1S,4R)-4-(((1S,4S)-5-(2-(2,6-dioxopiperidin-3-yl)-3-oxo-1-thioxoisoindolin-5-yl)-2,5-diazabicyclo[2.2.1]heptan-2-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1R,4R)-4-(((1R,4R)-5-(2-(2,6-dioxopiperidin-3-yl)-3-oxo-1-thioxoisoindolin-5-yl)-2,5-diazabicyclo[2.2.1]heptan-2-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-3-oxo-1-thioxoisoindolin-5-yl)piperidin-4-yl)piperazin-1-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-6-fluoro-3-oxo-1-thioxoisoindolin-5-yl)piperazin-1-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((8-(2-(2,6-dioxopiperidin-3-yl)-3-oxo-1-thioxolsoindolin-5-yl)-3,8-diazabicyclo[3.2.1]octan-3-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-3-oxo-1-thioxoisoindolin-4-yl)piperazin-1-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((6-(2-(2,6-dioxopiperidin-3-yl)-3-oxo-1-thioxoisoindolin-5-yl)-3,6-diazabicyclo[3.1.1]heptan-3-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((4-(2-(2,6-dioxopiperidin-3-yl)-7-fluoro-3-oxo-1-thioxoisoindolin-5-yl)piperazin-1-yl)methyl)cyclohexyl)-1H-pyrazol-4-yl)pyrazolo[1,5-a]pyrimidine-3-carboxamide;
5-((1R,4R)-2-oxa-5-azabicyclo[2.2.1]heptan-5-yl)-N-(3-(difluoromethyl)-1-((1r,4R)-4-((4-(1-(2-(2,6-dioxopiperidin-3-yl)-3-oxo-1-thioxoisoindolin-5-yl)-3,3-difluoropiperidin-4-yl)piperazin-1-yl)methyl)cyclohexyl)-1H-pyrazol-4-yl)pyrazolo[1,5-a]pyrimidine-3-carboxamide;
N-(5-((4-((2-(2,6-dioxopiperidin-3-yl)-3-oxo-1-thioxoisoindolin-5-yl)methyl)piperazin-1-yl)methyl)-1-((1s,4s)-4-(hydroxymethyl)cyclohexyl)-1H-benzo[d] midazole-2-yl)-3-(trifluoromethyl)benzamide;
N-(5-(4-((2-(2,6-dioxopiperidin-3-yl)-3-oxo-1-thioxoisoindolin-5-yl)methyl)piperazin-1-yl)-1-((1s,4s)-4-(hydroxymethyl)cyclohexyl)-1H-benzo[d] midazole-2-yl)-3-(trifluoromethyl)benzamide;
N-(5-(4-((1-(2-(2,6-dioxopiperidin-3-yl)-3-oxo-1-thioxoisoindolin-5-yl)piperidin-4-yl)methyl)piperazin-1-yl)-1-((1s,4s)-4-(hydroxymethyl)cyclohexyl)-1H-benzo[d] midazole-2-yl)-3-(trifluoromethyl)benzamide;
N-(5-(4-((1-(2-(2,6-dioxopiperidin-3-yl)-3-oxo-1-thioxoisoindolin-4-yl)piperidin-4-yl)methyl)piperazin-1-yl)-1-((1s,4s)-4-(hydroxymethyl)cyclohexyl)-1H-benzo[d]imidazol-2-yl)-3-(trifluoromethyl)benzamide;
N-(5-(4-(1-((2-(2,6-dioxopiperidin-3-yl)-1-oxo-3-thioxoisoindolin-5-yl)methyl)piperidin-4-yl)piperazin-1-yl)-1-((1s,4s)-4-(hydroxymethyl)cyclohexyl)-1H-benzo[d]imidazol-2-yl)-3-(trifluoromethyl)benzamide;
6-(5-cyano-1H-pyrrolo[2,3-b]pyridin-1-yl)-N-(1-((2-(2,6-dioxopiperidin-3-yl)-3-oxo-1-thioxoisoindolin-5-yl)methyl)piperidin-4-yl)-4-(isopropylamino)nicotinamide;
N-(3-(difluoromethyl)-1-(1-((2-(2,6-dioxopiperidin-3-yl)-1-oxo-3-thioxoisoindolin-5-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((1-(2-(2,6-dioxopiperidin-3-yl)-1-oxo-3-thioxoisoindolin-5-yl)piperidin-4-yl)methyl)piperidin-4-yl)-1H-pyrazol-4-yl)-2-(2-((2,2,2-trifluoroethyl)amino)pyridin-4-yl)oxazole-4-carboxamide;
N-(3-(difluoromethyl)-1-(1-((1'-(2-(2,6-dioxopiperidin-3-yl)-3-oxo-1-thioxoisoindolin-5-yl)-[1,4'-bipiperidin]-4-yl)methyl)piperidin-4-yl)-1H-pyrazol-4-yl)-2-(2-((2,2,2-trifluoroethyl)amino)pyridin-4-yl)oxazole-4-carboxamide;
N-(2-(4-((2-(2,6-dioxopiperidin-3-yl)-1-oxo-3-thioxoisoindolin-5-yl)methyl)piperazin-1-yl)-5-((R)-3-hydroxypyrrolidin-1-yl)oxazolo[4,5-b]pyridin-6-yl)-2-(2-methylpyridin-4-yl)oxazole-4-carboxamide;
N-(2-(1-((2-(2,6-dioxopiperidin-3-yl)-3-oxo-1-thioxoisoindolin-5-yl)methyl)piperidin-4-yl)-5-(2-hydroxypropan-2-yl)benzo[d]thiazol-6-yl)-6-(trifluoromethyl)picolinamide;
N-(2-(1-((2-(2,6-dioxopiperidin-3-yl)-3-oxo-1-thioxoisoindolin-5-yl)methyl)piperidin-4-yl)-5-(2-hydroxypropan-2-yl)benzo[d]oxazol-6-yl)-6-(trifluoromethyl)picolinamide;
N-(2-(1-((1-(2-(2,6-dioxopiperidin-3-yl)-3-oxo-1-thioxoisoindolin-5-yl)piperidin-4-yl)methyl)piperidin-4-yl)-5-(2-hydroxypropan-2-yl)benzo[d]oxazol-6-yl)-6-(trifluoromethyl)picolinamide;
N-(2-(1-((1-(2-(2,6-dioxopiperidin-3-yl)-3-oxo-1-thioxoisoindolin-4-yl)piperidin-4-yl)methyl)piperidin-4-yl)-5-(2-hydroxypropan-2-yl)benzo[d]oxazol-6-yl)-6-(trifluoromethyl)picolinamide;
N-(2-(1-((2-(2,6-dioxopiperidin-3-yl)-3-oxo-1-thioxoisoindolin-5-yl)methyl)piperidin-4-yl)-6-(2-hydroxypropan-2-yl)-2H-indazol-5-yl)-6-(trifluoromethyl)picolinamide;
N-(2-(1-((1-(2-(2,6-dioxopiperidin-3-yl)-3-oxo-1-thioxoisoindolin-5-yl)piperidin-4-yl)methyl)piperidin-4-yl)-6-(2-hydroxypropan-2-yl)-2H-indazol-5-yl)-6-(trifluoromethyl)picolinamide;
N-(2-(1-((1-(2-(2,6-dioxopiperidin-3-yl)-3-oxo-1-thioxoisoindolin-4-yl)piperidin-4-yl)methyl)piperidin-4-yl)-6-(2-hydroxypropan-2-yl)-2H-indazol-5-yl)-6-(trifluoromethyl)picolinamide;
N-(2-(1-((1'-(2-(2,6-dioxopiperidin-3-yl)-3-oxo-1-thioxoisoindolin-5-yl)-[1,4'-bipiperidin]-4-yl)methyl)piperidin-4-yl)-6-(2-hydroxypropan-2-yl)-2H-indazol-5-yl)-6-(trifluoromethyl)picolinamide;
6-(6-(4-((2-(2,6-dioxopiperidin-3-yl)-1-oxo-3-thioxoisoindolin-5-yl)methyl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(((2-(2,6-dioxopiperidin-3-yl)-3-oxo-1-thioxoisoindolin-5-yl)methyl)(methyl)amino)piperidin-1-yl)pyridin-3-yl)-1-isopropyl-N-((6-methyl-2-oxo-4-propyl-1,2-dihydropyridin-3-yl)methyl)-1H-indazole-4-carboxamide;
6-(6-(4-(1-((2-(2,6-dioxopiperidin-3-yl)-3-oxo-1-thioxoisoindolin-5-yl)methyl)piperidin-4-yl)piperazin-1-yl)pyridin-3-yl)-1-isopropyl-N-((6-methyl-2-oxo-4-propyl-1,2-dihydropyridin-3-yl)methyl)-1H-indazole-4-carboxamide;
N-((4,6-dimethyl-2-oxo-1,2-dihydropyridin-3-yl)methyl)-4'-((4-((2-(2,6-dioxopiperidin-3-yl)-1-oxo-3-thioxoisoindolin-5-yl)methyl)piperazin-1-yl)methyl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-((2-(2,6-dioxopiperidin-3-yl)-3-oxo-1-thioxoisoindolin-5-yl)methyl)piperazin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(((2-(2,6-dioxopiperidin-3-yl)-3-oxo-1-thioxoisoindolin-5-yl)methyl)(methyl)amino)piperidin-1-yl)-5-(ethyl(tetrahydro-2H-pyran-4-yl)amino)-4-methyl-[1,1'-biphenyl]-3-carboxamide;
N-((4,6-dimethyl-2-oxo-1,2-dihydropyridin-3-yl)methyl)-4'-(4-(1-((2-(2,6-dioxopiperidin-3-yl)-3-oxo-1-thioxoisoindolin-5-yl)methyl)piperidin-4-yl)piperazin-1-yl)-5-(ethyl(tetrahydro-2H-pyran-4-yl)amino)-4-methyl-[1,1'-biphenyl]-3-carboxamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)piperazin-1-yl-2,2,3,3,5,5,6,6-d8)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)-3,5-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)-2,6-dimethylpiperazin-1-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(6-((2-(2,6-dioxopiperidin-3-yl)-1-oxo-3-thioxoisoindolin-5-yl)methyl)-3,6-diazabicyclo[3.1.1]heptan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-oxo-3-thioxoisoindolin-5-yl)methyl)-3,6-diazabicyclo[3.1.1]heptan-6-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(8-((2-(2,6-dioxopiperidin-3-yl)-1-oxo-3-thioxoisoindolin-5-yl)methyl)-3,8-diazabicyclo[3.2.1]octan-3-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(3-((2-(2,6-dioxopiperidin-3-yl)-1-oxo-3-thioxoisoindolin-5-yl)methyl)-3,8-diazabicyclo[3.2.1]octan-8-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1R,4R)-5-((2-(2,6-dioxopiperidin-3-yl)-1-oxo-3-thioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-((1S,4S)-5-((2-(2,6-dioxopiperidin-3-yl)-1-oxo-3-thioxoisoindolin-5-yl)methyl)-2,5-diazabicyclo[2.2.1]heptan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(5-((2-(2,6-dioxopiperidin-3-yl)-1-oxo-3-thioxoisoindolin-5-yl)methyl)-2,5-diazabicyclo[2.2.2]octan-2-yl)-1-(phenylthio)butan-2-yl)amino)-3-((trifluoromethyl)sulfonyl)phenyl)sulfonyl)benzamide;
4-(4-((4'-chloro-4,4-dimethyl-3,4,5,6-tetrahydro-[1,1'-biphenyl]-2-yl)methyl)piperazin-1-yl)-N-((4-(((2R)-4-(4-((2-(2,6-dioxopiperidin-3-yl)-1-oxo-3-thioxoisoindolin-5-yl)methyl)piperazin-1-yl)-1-(phenylthio)butan-2-yl)amino)-3-((trifluoromethyl)sulfonyl)phenyl)sulfonyl)benzamide;
2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((4-(((1-((2-(2,6-dioxopiperidin-3-yl)-1-oxo-3-thioxoisoindolin-5-yl)methyl)piperidin-4-yl)methyl)amino)-3-nitrophenyl)sulfonyl)benzamide;
(9R)-N-(1-(4-(((2-(2,6-dioxopiperidin-3-yl)-3-oxo-1-thioxoisoindolin-5-yl)methyl)amino)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-((2-(2,6-dioxopiperidin-3-yl)-3-oxo-1-thioxoisoindolin-5-yl)methyl)piperazin-1-yl)benzyl)-1H-pyrazol-4-yl)-9-methyl-6-oxo-6,7,8,9-tetrahydropyrido[3',2':4,5]pyrrolo[1,2-a]pyrazine-2-carboxamide;
(9R)-N-(1-(4-(4-(((2-(2,6-dioxopiperidin-3-yl)-3-oxo-1-thioxoisoindolin-5-yl)methyl)(methyl)amino)piperidin-1-yl)benzyl)-1H-pyrazol-4-yl)-9-methyl-6-oxo-6,7,8,9-tetrahydropyrido [3',2':4,5]pyrrolo[1,2-a]pyrazine-2-carboxamide;
(9R)-N-(1-(4-(4-(4-((2-(2,6-dioxopiperidin-3-yl)-3-oxo-1-thioxoisoindolin-5-yl)methyl)piperazin-1-yl)piperidin-1-yl)benzyl)-1H-pyrazol-4-yl)-9-methyl-6-oxo-6,7,8,9-tetrahydropyrido [3',2':4,5]pyrrolo[1,2-a]pyrazine-2-carboxamide;
3-(5-((4-(3-amino-6-(2-hydroxyphenyl)pyridazin-4-yl)piperazin-1-yl)methyl)-3-oxo-1-thioxoisoindolin-2-yl)piperidine-2,6-dione;
3-(5-(4-((4-(3-amino-6-(2-hydroxyphenyl)pyridazin-4-yl)piperazin-1-yl)methyl)piperidin-1-yl)-3-oxo-1-thioxoisoindolin-2-yl)piperidine-2,6-dione;
3-(4-(4-((4-(3-amino-6-(2-hydroxyphenyl)pyridazin-4-yl)piperazin-1-yl)methyl)piperidin-1-yl)-3-oxo-1-thioxoisoindolin-2-yl)piperidine-2,6-dione;
3-(5-(4-((4-(3-amino-6-(2-hydroxyphenyl)pyridazin-4-yl)piperazin-1-yl)methyl)-[1,4'-bipiperidin]-1'-yl)-3-oxo-1-thioxoisoindolin-2-yl)piperidine-2,6-dione;
3-(5-((4-(3-amino-6-(5-fluoro-2-hydroxyphenyl)pyridazin-4-yl)piperazin-1-yl)methyl)-3-oxo-1-thioxoisoindolin-2-yl)piperidine-2,6-dione;
3-(5-(4-((4-(3-amino-6-(5-fluoro-2-hydroxyphenyl)pyridazin-4-yl)piperazin-1-yl)methyl)piperidin-1-yl)-3-oxo-1-thioxoisoindolin-2-yl)piperidine-2,6-dione;
3-(4-(4-((4-(3-amino-6-(5-fluoro-2-hydroxyphenyl)pyridazin-4-yl)piperazin-1-yl)methyl)piperidin-1-yl)-3-oxo-1-thioxoisoindolin-2-yl)piperidine-2,6-dione;
3-(5-(4-((4-(3-amino-6-(5-fluoro-2-hydroxyphenyl)pyridazin-4-yl)piperazin-1-yl)methyl)-[1,4'-bipiperidin]-1'-yl)-3-oxo-1-thioxoisoindolin-2-yl)piperidine-2,6-dione;
3-(4-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-thioxoisoindolin-2-yl)piperidine-2,6-dione;
3-(6-(((1-(4-((5-bromo-4-((5-(dimethylphosphoryl)quinoxalin-6-yl)amino)pyrimidin-2-yl)amino)-5-methoxy-2-methylphenyl)piperidin-4-yl)(methyl)amino)methyl)-1-thioxoisoindolin-2-yl)piperidine-2,6-dione;
3-(6-((4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)methyl)-1-thioxoisoindolin-2-yl)piperidine-2,6-dione;
2-(6,7-dihydro-5H-pyrrolo[1,2-c]imidazol-1-yl)-2-(6-(4-((1R,4R)-5-((2-(2,6-dioxopiperidin-3-yl)-1-thioxoisoindolin-4-yl)methyl)-2,5-diazabicyclo[2.2.1]heptan-2-yl)phenyl)-4-fluoro-1-oxoisoindolin-2-yl)-N-(thiazol-2-yl)acetamide;
2-(6,7-dihydro-5H-pyrrolo[1,2-c]imidazol-1-yl)-2-(6-(4-((1R,4R)-5-((2-(2,6-dioxopiperidin-3-yl)-3-thioxoisoindolin-5-yl)methyl)-2,5-diazabicyclo[2.2.1]heptan-2-yl)phenyl)-4-fluoro-1-oxoisoindolin-2-yl)-N-(thiazol-2-yl)acetamide;
3-(4-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione;
3-(6-((4-(1-isopropyl-6-((2-(4-methoxypiperidin-1-yl)pyrimidin-4-yl)amino)-1H-pyrazolo[4,3-c]pyridin-3-yl)piperazin-1-yl)methyl)-1-thioxoisoindolin-2-yl)piperidine-2,6-dione;
2-((5-bromo-2-((4-((4-((2-(2,6-dioxopiperidin-3-yl)-1-thioxoisoindolin-5-yl)methyl)piperazin-1-yl)sulfonyl)-2-methylphenyl)amino)pyrimidin-4-yl)amino)-6-fluorobenzamide;
2-((5-bromo-2-((4-((4-((2-(2,6-dioxopiperidin-3-yl)-1-thioxoisoindolin-4-yl)methyl)piperazin-1-yl)sulfonyl)-2-methylphenyl)amino)pyrimidin-4-yl)amino)-6-fluorobenzamide ;
2-((5-bromo-2-((4-((4-((2-(2,6-dioxopiperidin-3-yl)-3-thioxoisoindolin-5-yl)methyl)piperazin-1-yl)sulfonyl)-2-methylphenyl)amino)pyrimidin-4-yl)amino)-6-fluorobenzamide ;
3-(4-((4-((4-((5-amino-1-(2,6-difluorobenzoyl)-1H-1,2,4-triazol-3-yl)amino)phenyl)sulfonyl)piperazin-1-yl)methyl)-1-thioxoisoindolin-2-yl)piperidine-2,6-dione ;
3-(6-((4-((4-((5-amino-1-(2,6-difluorobenzoyl)-1H-1,2,4-triazol-3-yl)amino)phenyl)sulfonyl)piperazin-1-yl)methyl)-1-thioxoisoindolin-2-yl)piperidine-2,6-dione ;
3-(5-((4-((4-((5-amino-1-(3-methylthiophene-2-carbonyl)-1H-1,2,4-triazol-3-yl)amino)phenyl)sulfonyl)piperazin-1-yl)methyl)-1-thioxoisoindolin-2-yl)piperidine-2,6-dione ;
3-(4-((4-((4-((5-amino-1-(3-methylthiophene-2-carbonyl)-1H-1,2,4-triazol-3-yl)amino)phenyl)sulfonyl)piperazin-1-yl)methyl)-1-thioxoisoindolin-2-yl)piperidine-2,6-dione ;
3-(6-((4-((4-((5-amino-1-(3-methylthiophene-2-carbonyl)-1H-1,2,4-triazol-3-yl)amino)phenyl)sulfonyl)piperazin-1-yl)methyl)-1-thioxoisoindolin-2-yl)piperidine-2,6-dione ;
N-(4-(chlorodifluoromethoxy)phenyl)-6-(4-((2-(2,6-dioxopiperidin-3-yl)-1-thioxoisoindolin-4-yl)methyl)piperazin-1-yl)-5-(1H-pyrazol-3-yl)nicotinamide;
N-(4-(chlorodifluoromethoxy)phenyl)-6-(4-(4-((2-(2,6-dioxopiperidin-3-yl)-1-thioxoisoindolin-4-yl)methyl)piperazin-1-yl)piperidin-1-yl)-5-(1H-pyrazol-3-yl)nicotinamide;
3-(4-((4-(4-((4-((3-(methylsulfonyl)benzyl)amino)-5-(trifluoromethyl)pyrimidin-2-yl)amino)phenyl)piperazin-1-yl)methyl)-1-thioxoisoindolin-2-yl)piperidine-2,6-dione ;
3-(4-((4-(3-amino-6-(2-hydroxyphenyl)pyridazin-4-yl)piperazin-1-yl)methyl)-1-thioxoisoindolin-2-yl)piperidine-2,6-dione ;
3-(6-((4-(3-amino-6-(2-hydroxyphenyl)pyridazin-4-yl)piperazin-1-yl)methyl)-1-thioxoisoindolin-2-yl)piperidine-2,6-dione ;
3-(4-((4-(3-amino-6-(5-fluoro-2-hydroxyphenyl)pyridazin-4-yl)piperazin-1-yl)methyl)-1-thioxoisoindolin-2-yl)piperidine-2,6-dione ;
3-(6-((4-(3-amino-6-(5-fluoro-2-hydroxyphenyl)pyridazin-4-yl)piperazin-1-yl)methyl)-1-thioxoisoindolin-2-yl)piperidine-2,6-dione ;
3-(5-((3-(3-amino-6-(5-fluoro-2-hydroxyphenyl)pyridazin-4-yl)-3,8-diazabicyclo[3.2.1]octan-8-yl)methyl)-1-thioxoisoindolin-2-yl)piperidine-2,6-dione ;
3-(4-((3-(3-amino-6-(5-fluoro-2-hydroxyphenyl)pyridazin-4-yl)-3,8-diazabicyclo[3.2.1]octan-8-yl)methyl)-1-thioxoisoindolin-2-yl)piperidine-2,6-dione;
3-(6-((3-(3-amino-6-(5-fluoro-2-hydroxyphenyl)pyridazin-4-yl)-3,8-diazabicyclo[3.2.1]octan-8-yl)methyl)-1-thioxoisoindolin-2-yl)piperidine-2,6-dione ;
3-(5-((4-(4-((5-chloro-4-((2-(isopropylsulfonyl)phenyl)amino)pyrimidin-2-yl)amino)-5-isopropoxy-2-methylphenyl)piperidin-1-yl)methyl)-1-thioxoisoindolin-2-yl)piperidine-2,6-dione ;
3-(4-((4-(4-((5-chloro-4-((2-(isopropylsulfonyl)phenyl)amino)pyrimidin-2-yl)amino)-5-isopropoxy-2-methylphenyl)piperidin-1-yl)methyl)-1-thioxoisoindolin-2-yl)piperidine-2,6-dione;
3-(6-((4-(4-((5-chloro-4-((2-(isopropylsulfonyl)phenyl)amino)pyrimidin-2-yl)amino)-5-isopropoxy-2-methylphenyl)piperidin-1-yl)methyl)-1-thioxoisoindolin-2-yl)piperidine-2,6-dione;
3-(4-((4-(4-((5-chloro-4-((2-(dimethylphosphoryl)phenyl)amino)pyrimidin-2-yl)amino)-3-methoxyphenyl)piperazin-1-yl)methyl)-1-thioxoisoindolin-2-yl)piperidine-2,6-dione;
3-(6-((4-(4-((5-chloro-4-((2-(dimethylphosphoryl)phenyl)amino)pyrimidin-2-yl)amino)-3-methoxyphenyl)piperazin-1-yl)methyl)-1-thioxoisoindolin-2-yl)piperidine-2,6-dione;
3-(6-(((1-(4-((5-chloro-4-((2-(dimethylphosphoryl)phenyl)amino)pyrimidin-2-yl)amino)-3-methoxyphenyl)piperidin-4-yl)(methyl)amino)methyl)-1-thioxoisoindolin-2-yl)piperidine-2,6-dione;
3-(5-((4-(5-(5-methyl-5H-pyrido[4,3-b]indol-7-yl)pyridin-2-yl)piperazin-1-yl)methyl)-1-thioxoisoindolin-2-yl)piperidine-2,6-dione ;
3-(5-((4-(5-(5-(difluoromethyl)-5H-pyrido[4,3-b]indol-7-yl)pyridin-2-yl)piperazin-1-yl)methyl)-1-thioxoisoindolin-2-yl)piperidine-2,6-dione;
3-(5-((4-(3-methyl-5-(5-methyl-5H-pyrido[4,3-b]indol-7-yl)pyridin-2-yl)piperazin-1-yl)methyl)-1-thioxoisoindolin-2-yl)piperidine-2,6-dione ;
3-(5-((4-(3-chloro-5-(5-methyl-5H-pyrido[4,3-b]indol-7-yl)pyridin-2-yl)piperazin-1-yl)methyl)-1-thioxoisoindolin-2-yl)piperidine-2,6-dione ;
3-(5-((4-(benzo[4,5]imidazo[1,2-a]pyrimidin-2-yl)piperazin-1-yl)methyl)-1-thioxoisoindolin-2-yl)piperidine-2,6-dione ;
5-chloro-4-(4-chloro-1-methyl-1H-pyrazol-5-yl)-N-((2S)-1-(3,4-difluorophenyl)-3-(((2-(2,6-dioxopiperidin-3-yl)-1-thioxoisoindolin-5-yl)methyl)amino)propan-2-yl)furan-2-carboxamide;
5-chloro-4-(4-chloro-1-methyl-1H-pyrazol-5-yl)-N-((2S)-1-(3,4-difluorophenyl)-3-(4-((2-(2,6-dioxopiperidin-3-yl)-1-thioxoisoindolin-5-yl)methyl)piperazin-1-yl)propan-2-yl)furan-2-carboxamide;
5-chloro-4-(4-chloro-1-methyl-1H-pyrazol-5-yl)-N-((2S)-1-(4-((2-(2,6-dioxopiperidin-3-yl)-1-thioxoisoindolin-5-yl)methyl)piperazin-1-yl)-3-(3-fluorophenyl)propan-2-yl)thiophene-2-carboxamide;
4-(((4-(5-chloro-2-((1-((2-(2,6-dioxopiperidin-3-yl)-1-thioxoisoindolin-4-yl)methyl)piperidin-4-yl)amino)pyridin-4-yl)thiazol-2-yl)amino)methyl)tetrahydro-2H-pyran-4-carbonitrile;
4-(((4-(5-chloro-2-((1-((2-(2,6-dioxopiperidin-3-yl)-3-thioxoisoindolin-5-yl)methyl)piperidin-4-yl)amino)pyridin-4-yl)thiazol-2-yl)amino)methyl)tetrahydro-2H-pyran-4-carbonitrile;
3-(4-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione;
3-(6-((4-((5-chloro-4-(5-(cyclopropylmethyl)-1-methyl-1H-pyrazol-4-yl)pyrimidin-2-yl)amino)piperidin-1-yl)methyl)-1-thioxoisoindolin-2-yl)piperidine-2,6-dione;
4-(((5'-chloro-2'-((1-((2-(2,6-dioxopiperidin-3-yl)-3-thioxoisoindolin-5-yl)methyl)piperidin-4-yl)amino)-[2,4'-bipyridin]-6-yl)amino)methyl)tetrahydro-2H-pyran-4-carbonitrile;
N-(5-cyclobutyl-1H-pyrazol-3-yl)-2-(4-(4-((2-(2,6-dioxopiperidin-3-yl)-1-thioxoisoindolin-5-yl)methyl)piperazin-1-yl)phenyl)acetamide ;
N-(5-cyclobutyl-1H-pyrazol-3-yl)-2-(4-(4-((2-(2,6-dioxopiperidin-3-yl)-1-thioxoisoindolin-4-yl)methyl)piperazin-1-yl)phenyl)acetamide ;
N-(5-cyclobutyl-1H-pyrazol-3-yl)-2-(4-(4-((2-(2,6-dioxopiperidin-3-yl)-3-thioxoisoindolin-5-yl)methyl)piperazin-1-yl)phenyl)acetamide; and
3-(5-((4-(4-(4-acetyl-1,4-diazepan-1-yl)-6-((2-(thiophen-2-yl)ethyl)amino)-1,3,5-triazin-2-yl)piperazin-1-yl)methyl)-1-thioxoisoindolin-2-yl)piperidine-2,6-dione.

20. The compound of Formula (I) or salts, enantiomers, stereoisomers, solvates, or polymorphs thereof as claimed in claims 1-19, which is a hydrohalic acid salt (including hydrochloride, hydrobromide), sulfate, citrate, maleate, mesylate, citrate, lactate, L-tartrate, fumarate, L-malate, phosphate, dihydrogen phosphate, pyrophosphate, metaphosphate, oxalate, malonate, benzoate, mandelate, succinate, trifluoroacetate, methanesulfonate, hippurate, glycolate, or 4-methylbenzenesulfonate of the compound of Formula (I).

21. A compound of Formula (II) 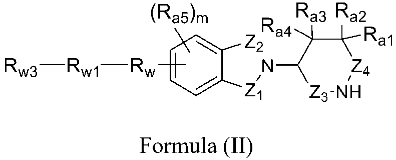 or salts, enantiomers, stereoisomers, solvates, isotopically enriched analogs, prodrugs, or polymorphs thereof,
wherein Z₁ represents C(O), C(S), CH₂ or CD₂; Z₂, Z₃, and Z₄ each independently represent C(O) or C(S), wherein at least one of Z₁, Z₂, Z₃, and Z₄ represents C(S);
Rₐ₁, Rₐ₂, Rₐ₃, and Rₐ₄ are identical or different and each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, and halogenated C₁₋₆ alkoxy;
(Rₐ₅)ₘ indicates that the isoindoline ring to which it is attached is optionally substituted with m Rₐ₅, with each Rₐ₅ being identical or different and each independently representing halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2 or 3;
R_{w} represents O, S, S(O), S(O)₂, alkenylene, alkynylene or N(Rₐ₆), wherein Rₐ₆ represents H or C₁₋₃ alkyl, or R_{w} represents a bond; or
R_{w} represents: 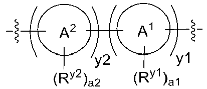
wherein each ring A¹ is identical or different and each independently represents nitrogen-containing heterocyclylene, arylene or heteroarylene, y1 represents an integer of 1 or 2, and (R^{y1})ₐ₁ indicates that each ring A¹ is independently optionally substituted with a1 R^{y1} groups, with each R^{y1} being independently deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a1 represents an integer of 0-20;
each ring A² is identical or different and each independently represents heterocyclylene, cycloalkylene, arylene or heteroarylene, y2 represents an integer of 0 or 1, and (R^{y2})ₐ₂ indicates that each ring A² is independently optionally substituted with a2 R^{y2} groups, with each R^{y2} being deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl, and a2 represents an integer of 0-20; and
R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from Rₐ, R_{b}, and any combination thereof; wherein each Rₐ is independently selected from the group consisting of: O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when one or more adjacent methylene in the main carbon chain skeleton of the linear or branched C₁₋₂₀ alkylene are replaced by one or more Rₐ groups, the Rₐ groups are not directly connected to each other; and wherein each R_{b} is independently selected from the group consisting of: optionally substituted cycloalkylene, optionally substituted arylene, optionally substituted heterocyclylene, optionally substituted heteroarylene, alkynylene, alkenylene, and any combination thereof; and
R_{w3} represents hydrogen, deuterium, leaving group, hydroxy, halogen, COOH, NH₂, N₃, CHO, methanesulfonyloxy, trifluoromethanesulfonyloxy or p-toluenesulfonyloxy.

22. The compound of Formula (II) or salts, enantiomers, stereoisomers, solvates, isotopically enriched analogs, prodrugs, or polymorphs thereof as claimed in claim 21, wherein the compound of Formula (II) is also represented by Formula (II-1) or Formula (II-2): 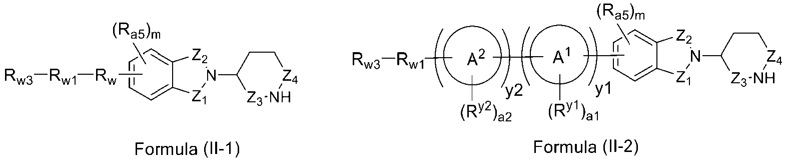 wherein Z₁, Z₂, Z₃, Z₄, (Rₐ₅)ₘ, R_{w}, ring A¹, ring A², y1, (R^{y1})ₐ₁, y2, (R^{y2})ₐ₂, R_{w1}, and R_{w3} are as defined in claim 20.

23. The compound of Formula (II) or salts, enantiomers, stereoisomers, solvates, isotopically enriched analogs, prodrugs, or polymorphs thereof as claimed in claim 21 or 22, wherein
(i) each ring A¹ independently represents 4- to 20-membered nitrogen-containing heterocyclylene, C₅₋₂₀ arylene or 5- to 20-membered heteroarylene; preferably each ring A¹ independently represents:
piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, 3-methyl-2-oxa-8-azaspiro[4.5]decanylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 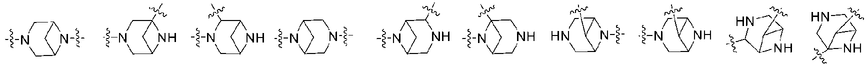 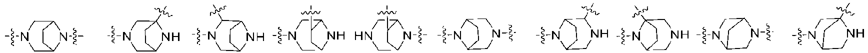 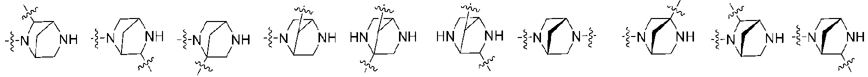 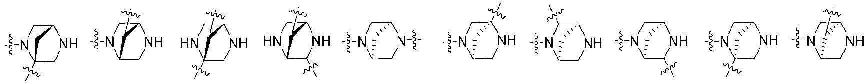 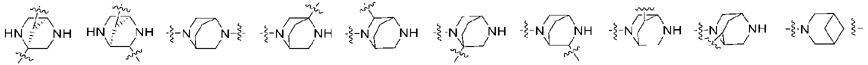 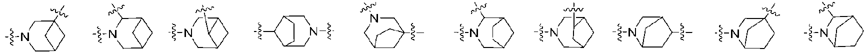 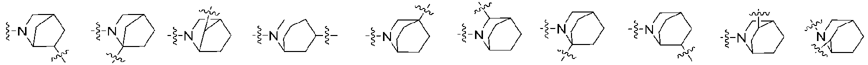 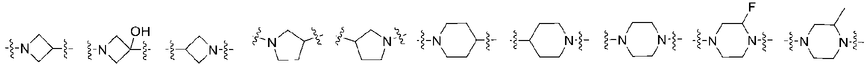 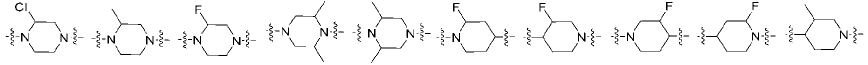 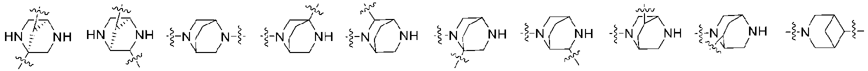 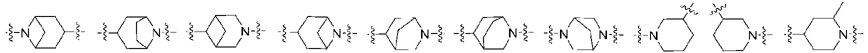 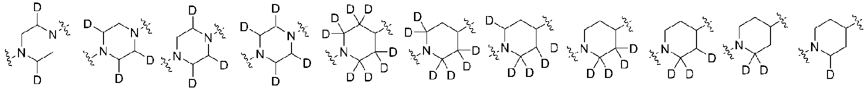 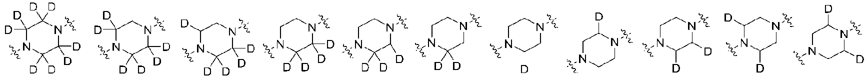 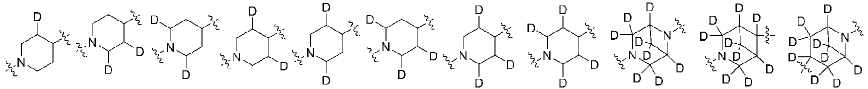 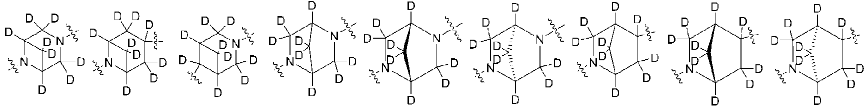 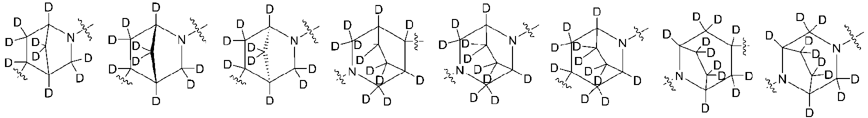 or 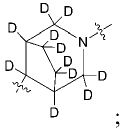
each ring A¹ is independently optionally substituted with a1 R^{y1} substituents, and each R^{y1} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl; and
a1 represents an integer of 0-20;
and/or
(ii) each ring A² independently represents 4- to 20-membered heterocyclylene, C₃₋₂₀cycloalkylene, C₅₋₂₀arylene or 5- to 20-membered heteroarylene; preferably each ring A² independently represents:
azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, C₅₋₁₅ spiro-cycloalkylene, adamantanylene, noradamantanylene, norbomylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene,pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene, or 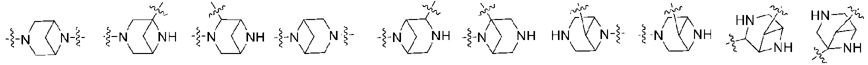 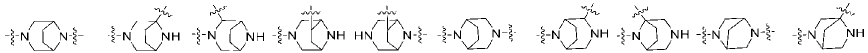 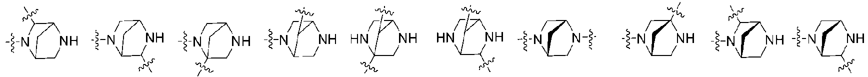 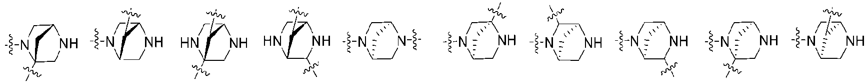 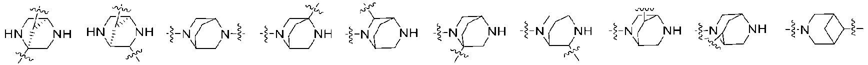 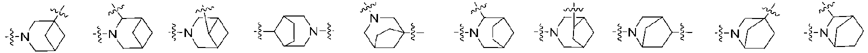 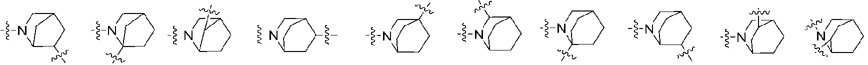 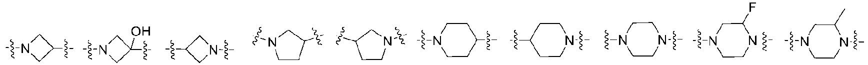 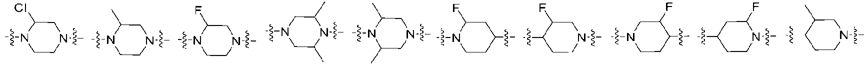 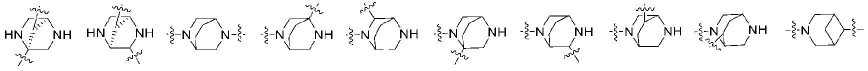 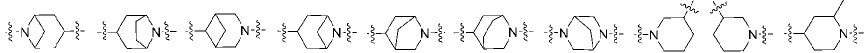 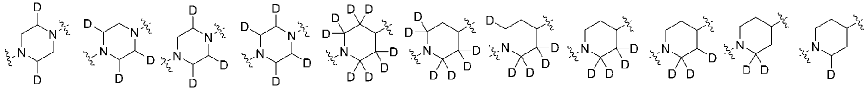 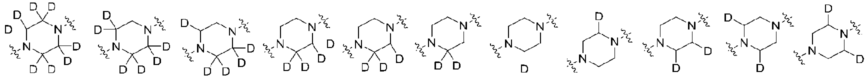 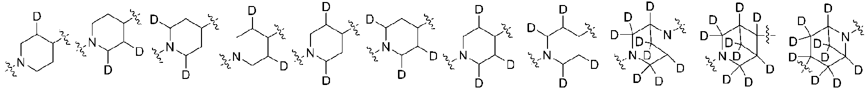 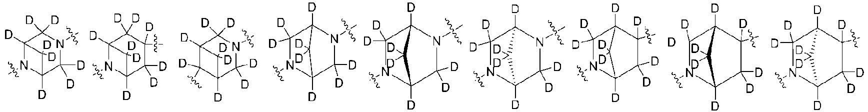 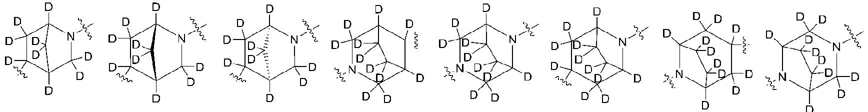 or 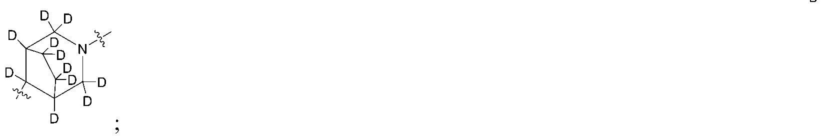
each ring A² is independently optionally substituted with a2 R^{y2} substituents, and each R^{y2} independently represents deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl or C₂₋₆ alkenyl; and
a2 represents an integer of 0-20.

24. The compound of Formula (II) or salts, enantiomers, stereoisomers, solvates, isotopically enriched analogs, prodrugs, or polymorphs thereof as claimed in claim 21, wherein R_{w} represents: 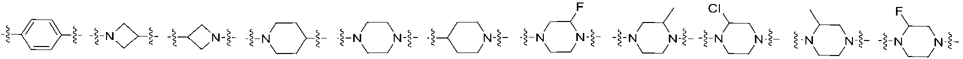 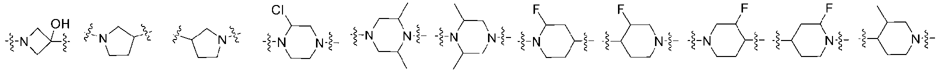 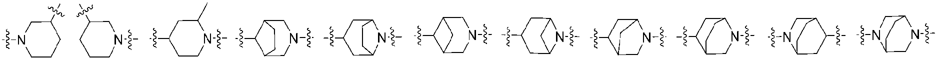 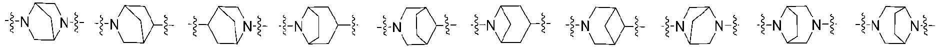 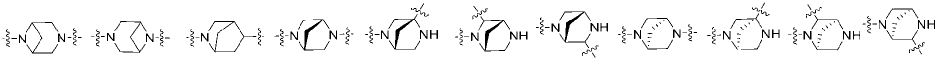 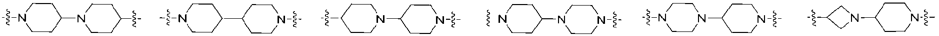 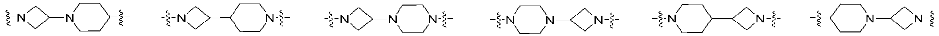 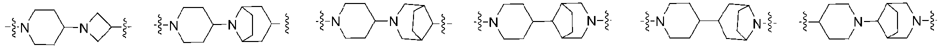 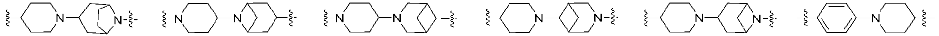 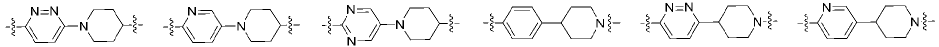 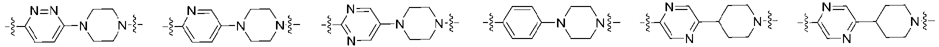 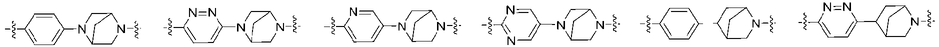 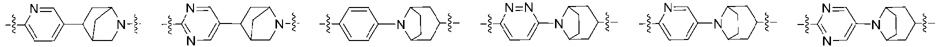 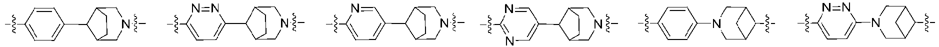 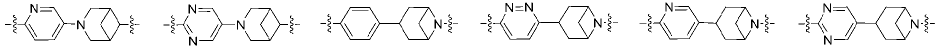 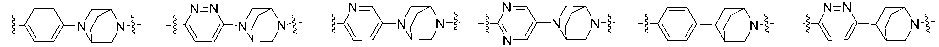 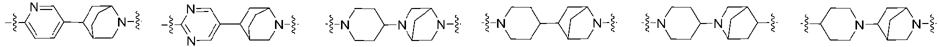 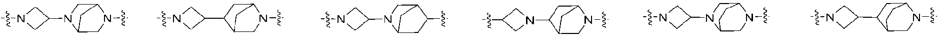 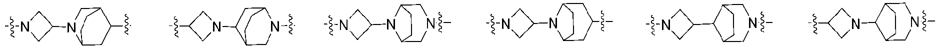 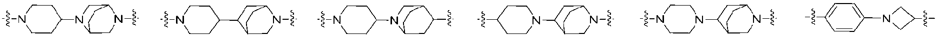 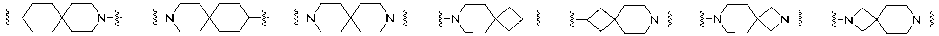 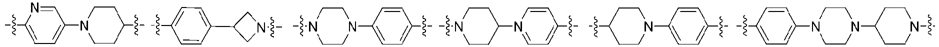 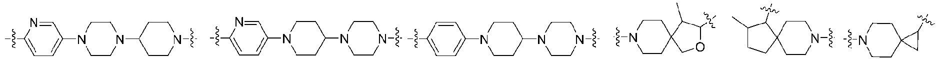 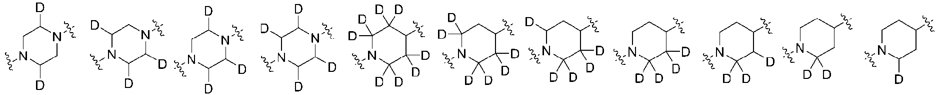 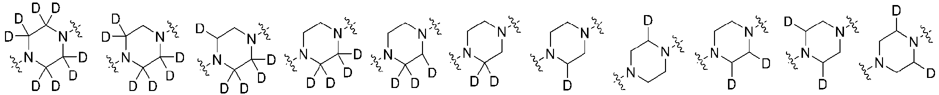 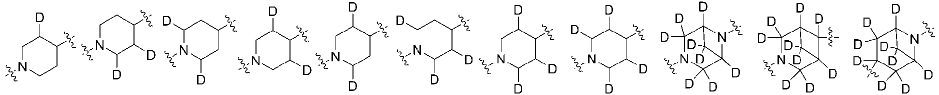 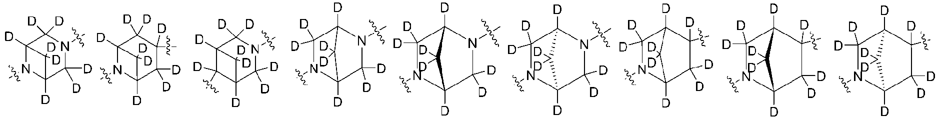 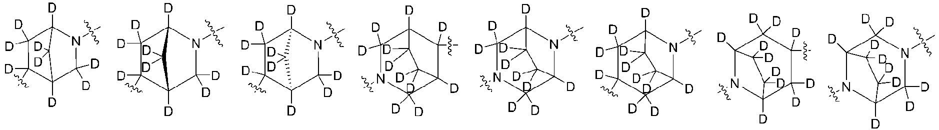 or 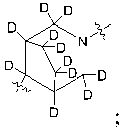 wherein the groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogen, amino, hydroxy, mercapto, cyano, carboxyl, C₂₋₆ alkynyl, and C₂₋₆ alkenyl.

25. The compound of Formula (II) or salts, enantiomers, stereoisomers, solvates, isotopically enriched analogs, prodrugs, or polymorphs thereof as claimed in claim 21 or 22, wherein the R_{w1} represents a bond or an optionally substituted linear or branched C₁₋₂₀ alkylene, wherein any one or more methylene groups in the main carbon chain skeleton of the linear or branched C₁₋₂₀ alkylene are optionally replaced by one or more groups selected from: Rₐ, R_{b}, and any combination thereof, wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl, and when any two or more methylene in the main carbon chain skeleton of the linear or branched C₁₋₂₀ alkylene are replaced by two or more Rₐ groups, the Rₐ groups are not directly connected to each other; and wherein each R_{b} is independently selected from the group consisting of: optionally substituted cycloalkylene (e.g., optionally substituted C₃₋₂₀cycloalkylene), optionally substituted arylene (e.g., optionally substituted C₅₋₂₀arylene), optionally substituted heterocyclylene (e.g., optionally substituted 4- to 20-membered heterocyclylene), optionally substituted heteroarylene (e.g., optionally substituted 5- to 20-membered heteroarylene), alkynylene (e.g., C₂₋₆alkynylene), alkenylene (e.g., C₂₋₆alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof; and wherein one or more hydrogens of said linear or branched C₁₋₂₀ alkylene are optionally replaced by a substituent selected from the group consisting of deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof.

26. The compound of Formula (II) or salts, enantiomers, stereoisomers, solvates, isotopically enriched analogs, prodrugs, or polymorphs thereof as claimed in claim 21 or 22, wherein R_{w1} represents a bond, or R_{w1} represents:
-C₁₋₁₅ alkylene-;
-(C(Rₐ₈)(Rₐ₉))ₙ₁-(Rₐ(C(Rₐ₁₀)(Rₐ₁₁))ₙ₂)ₘ₃-;
-(C(Rₐ₈)(Rₐ₉))ₙ₁-(Rₐ(C(Rₐ₁₀)(Rₐ₁₁))ₙ₂)ₘ₃-(Rₐ(C(Rₐ₁₂)(Rₐ₁₃))ₙ₃)ₘ₄-;
-(C(Rₐ₈)(Rₐ₉))ₙ₁-(Rₐ(C(Rₐ₁₀)(Rₐ₁₁))ₙ₂)ₘ₃-(Rₐ(C(Rₐ₁₂)(Rₐ₁₃))ₙ₃)ₘ₄-(Rₐ(C(Rₐ₁₄)(Rₐ₁₅))ₙ₄)ₘ₅-;
-(C(Rₐ₈)(Rₐ₉)ₙ₁-((C(Rₐ₁₀)(Rₐ₁₁))ₙ₂Rₐ)ₘ₃-;
-(C(Rₐ₈)(Rₐ₉)ₙ₁-((C(Rₐ₁₀)(Rₐ₁₁))ₙ₂Rₐ)ₘ₃-((C(Rₐ₁₂)(Rₐ₁₃))ₙ₃Rₐ)ₘ₄-;
-(C(Rₐ₈)(Rₐ₉))ₙ₁-((C(Rₐ₁₀)(Rₐ₁₁))ₙ₂Rₐ)ₘ₃-((C(Rₐ₁₂)(Rₐ₁₃))ₙ₃Rₐ)ₘ₄-((C(Rₐ₁₄)(Rₐ₁₅))ₙ₄Rₐ)ₘ₅-;
-(C(Rₐ₈)(Rₐ₉))ₙ₁-(R_{b}(C(Rₐ₁₀)(Rₐ₁₁))ₙ₂)ₘ₃-;
-(C(Rₐ₈)(Rₐ₉))ₙ₁-(R_{b}(C(Rₐ₁₀)(Rₐ₁₁))ₙ₂)ₘ₃-(R_{b}(C(Rₐ₁₂)(Rₐ₁₃))ₙ₃)ₘ₄-;
-(C(Rₐ₈)(Rₐ₉))ₙ₁-(R_{b}(C(Rₐ₁₀)(Rₐ₁₁))ₙ₂)ₘ₃-(R_{b}(C(Rₐ₁₂)(Rₐ₁₃))ₙ₃)ₘ₄-(R_{b}(C(Rₐ₁₄)(Rₐ₁₅))ₙ₄)ₘ₅-;
-(C(Rₐ₈)(Rₐ₉))ₙ₁-((C(Rₐ₁₀)(Rₐ₁₁))ₙ₂R_{b})ₘ₃-;
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃R_{b})ₘ₄-;
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃R_{b})ₘ₄-((C(Rₐ₁₄)(Rₐ₁₅))ₙ₄R_{b})ₘ₅-;
-(C(Rₐ₈)(Rₐ₉))ₙ₁-(Rₐ-R_{b}-(C(Rₐ₁₀)(Rₐ₁₁))ₙ₂)ₘ₃-;
~(C(Rₐ₈)(Rₐ₉))ₙ₁-(Rₐ(C(Rₐ₁₀)(Rₐ₁₁))ₙ₂)ₘ₃-(R_{b}(C(Rₐ₁₂)(Rₐ₁₃))ₙ₃)ₘ₄-;
-(C(Rₐ₈)(Rₐ₉))ₙ₁-(R_{b}-Rₐ-(C(Rₐ₁₀)(Rₐ₁₁))ₙ₂)ₘ₃-;
-(C(Rₐ₈)(Rₐ₉))ₙ₁-(Rb(C(Rₐ₁₀)(Rₐ₁₁))ₙ₂)ₘ₃-(Rₐ(C(Rₐ₁₂)(Rₐ₁₃))ₙ₃)ₘ₄-;
-(C(Rₐ₈)(Rₐ₉))ₙ₁-((C(Rₐ₁₀)(Rₐ₁₁))ₙ₂-Rₐ-R_{b})ₘ₃-;
-(C(Rₐ₈)(Rₐ₉))ₙ₁-((C(Rₐ₁₀)(Rₐ₁₁))ₙ₂Rₐ)ₘ₃-((C(Rₐ₁₂)(Rₐ₁₃))ₙ₃R_{b})ₘ₄-;
-(C(Rₐ₈)(Rₐ₉))ₙ₁((C(Rₐ₁₀)(Rₐ₁₁))ₙ₂-R_{b}-Rₐ)ₘ₃-; or
-(C(Rₐ₈)(Rₐ₉))ₙ₁-((C(Rₐ₁₀)(Rₐ₁₁))ₙ₂R_{b})ₘ₃-((C(Rₐ₁₂)(Rₐ₁₃))ₙ₃Rₐ)ₘ₄-;
wherein each Rₐ is independently selected from the group consisting of O, C(O), OC(O), S, S(O), S(O)₂, S(O)₂N(Rₐ₇), N(Rₐ₇)S(O)₂, C(O)N(Rₐ₇), N(Rₐ₇)C(O), N(Rₐ₇), and N(Rₐ₇)C(O)N(Rₐ₇), where each Rₐ₇ independently represents H or C₁₋₃ alkyl; and each R_{b} is independently selected from the group consisting of optionally substituted cycloalkylene (e.g., optionally substituted C₃₋₂₀cycloalkylene), optionally substituted arylene (e.g., optionally substituted C₅₋₂₀arylene), optionally substituted heterocyclylene (e.g., optionally substituted 4- to 20-membered heterocyclylene), optionally substituted heteroarylene (e.g., 5- to 20-membered heteroarylene), alkynylene (e.g., C₂₋₆alkynylene), alkenylene (e.g., C₂₋₆alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof;
Rₐ₈, Rₐ₉, Ra₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
n1, n2, n3, n4, m3, m4, m5 each independently represent an integer of 1, 2, 3, 4, 5, 6, 7, 8, 9 or 10; and
any one or more hydrogens of said C₁₋₁₅ alkylene are optionally replaced by a substituent selected from: deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

27. The compound of Formula (I) or salts, enantiomers, stereoisomers, solvates, isotopically enriched analogs, prodrugs, or polymorphs thereof as claimed in claim 21 or 22, wherein the R_{w1} represents a structure of the following Formulae:
-(C(Rₐ₈)(Rₐ₉))ₙ₁-(O(C(Rₐ₁₀)(Rₐ₁₁)ₙ₂)ₘ₃-;
-(C(Rₐ₈)(Rₐ₉))ₙ₁-(O(C(Rₐ₁₀)(Rₐ₁₁))ₙ₂)ₘ₃-(O(C(Rₐ₁₂)(Rₐ₁₃))ₙ₃)ₘ₄-;
-(C(Rₐ₈)(Rₐ₉))ₙ₁-(O(C(Rₐ₁₀)(Rₐ₁₁))ₙ₂)ₘ₃-(O(C(Rₐ₁₂)(Rₐ₁₃))ₙ₃)ₘ₄- (O(C(Rₐ₁₄)(Rₐ₁₅))ₙ₄)ₘ₅-;
-(C(Rₐ₈)(Rₐ₉))ₙ₁-((C(Rₐ₁₀)(Rₐ₁₁))ₙ₂O)ₘ₃-;
-(C(Rₐ₈)(Rₐ₉))ₙ₁₋((C(Rₐ₁₀)(Rₐ₁₁))ₙ₂O)ₘ₃₋((C(Rₐ₁₂)(Rₐ₁₃))ₙ₃O)ₘ₄-;
-(C(Rₐ₈)(Rₐ₉))ₙ₁-((C(Rₐ₁₀)(Ra₁₁))ₙ₂O)ₘ₃-((C(Rₐ₁₂)(Rₐ₁₃))ₙ₃O)ₘ₄-((C(Rₐ₁₄)(Rₐ₁₅))ₙ₄O)ₘ₅-;
-(C(Rₐ₈)(Rₐ₉))ₙ₁-(N(Rₐ₇)(C(Rₐ₁₀)(Rₐ₁₁))ₙ₂)ₘ₃-;
-(C(Rₐ₈)(Rₐ₉))ₙ₁-(N(Rₐ₇)(C(Rₐ₁₀)(Rₐ₁₁))ₙ₂)ₘ₃-(N(Rₐ₇)(C(Rₐ₁₂)(Rₐ₁₃))ₙ₃)ₘ₄-;
-(C(Rₐ₈)(Rₐ₉))ₙ₁-(N(Rₐ₇)(C(Rₐ₁₀)(Rₐ₁₁))ₙ₂)ₘ₃-(N(Rₐ₇)(C(Rₐ₁₂)(Rₐ₁₃))ₙ₃)ₘ₄-(N(Rₐ₇)(C(Rₐ₁₄)(Rₐ₁₅))ₙ₄)ₘ₅-;
-(C(Rₐ₈)(Rₐ₉))ₙ₁-((C(Rₐ₁₀)(Ra₁₁))ₙ₂N(Rₐ₇))ₘ₃-;
-(C(Rₐ₈)(Rₐ₉))ₙ₁-((C(Rₐ₁₀)(Rₐ₁₁))ₙ₂N(Rₐ₇))ₘ₃-((C(Rₐ₁₂)(Rₐ₁₃))ₙ₃N(Rₐ₇))ₘ₄-;
-(C(Rₐ₈)(Rₐ₉))ₙ₁₋((C(Rₐ₁₀₎(Rₐ₁₁))ₙ₂N(Rₐ₇)ₘ₃-((C(Rₐ₁₂)(Rₐ₁₃))ₙ₃N(Rₐ₇))ₘ₄-((C(Rₐ₁₄)(Rₐ₁₅))ₙ₄N(Rₐ₇))ₘ₅-;
-(C(Rₐ₈)(Rₐ₉))ₙ₁-(C(O)N(Rₐ₇)-(C(Rₐ₁₀)(Rₐ₁₁))ₙ₂)ₘ₃-;
-(C(Rₐ₈)(Rₐ₉))ₙ₁-(C(O)N(Rₐ₇)-(C(Rₐ₁₀)(Rₐ₁₁))ₙ₂)ₘ₃-(C(O)N(Rₐ₇)-(C(Rₐ₁₂)(Rₐ₁₃))ₙ₃)ₘ₄-;
-(C(Rₐ₈)(Rₐ₉))ₙ₁-(C(O)N(Rₐ₇)-(C(Rₐ₁₀)(Rₐ₁₁))ₙ₂)ₘ₃-(C(O)N(Rₐ₇)-(C(Rₐ₁₂)(Rₐ₁₃))ₙ₃)ₘ₄-(C(O)N(Rₐ₇)-(C(Rₐ₁₄)(Rₐ₁₅))ₙ₄)ₘ₅-;
-(C(Rₐ₈)(Rₐ₉))ₙ₁-((C(Rₐ₁₀)(Ra₁₁))ₙ₂-C(O)N(Rₐ₇))ₘ₃-;
-(C(Rₐ₈)(Rₐ₉))ₙ₁-((C(Rₐ₁₀)(Rₐ₁₁))ₙ₂-C(O)N(Rₐ₇))ₘ₃-((C(Rₐ₁₂)(Rₐ₁₃))ₙ₃-C(O)N(Rₐ₇))ₘ₄-;
-(C(Rₐ₈)(Rₐ₉))ₙ₁-((C(Rₐ₁₀₎(Rₐ₁₁))ₙ₂-C(O)N(Rₐ₇))ₘ₃-((C(Rₐ₁₂)(Rₐ₁₃))ₙ₃-C(O)N(Rₐ₇))ₘ₄-((C(Rₐ₁₄)(Rₐ₁₅))ₙ₄-C(O)N(Rₐ₇))ₘ₅-;
-(C(Rₐ₈)(Rₐ₉))ₙ₁-(C(O)N(Rₐ₇)-(C(Rₐ₁₀)(Rₐ₁₁))ₙ₂)ₘ₃-(O-(C(Rₐ₁₂)(Rₐ₁₃))ₙ₃)ₘ₄-;
-(C(Rₐ₈)(Rₐ₉))ₙ₁-C(O)N(Rₐ₇)-(C(Rₐ₁₀)(Rₐ₁₁))ₙ₂-(O(C(Rₐ₁₂)(Rₐ₁₃))ₙ₃)ₘ₃-;
-(C(Rₐ₈)(Rₐ₉))ₙ₁-((C(Rₐ₁₀)(Rₐ₁₁))ₙ₂-C(O)N(Rₐ₇))ₘ₃-((C(Rₐ₁₂)(Rₐ₁₃))ₙ₃O)ₘ₄-;
-(C(Rₐ₈)(Rₐ₉))ₙ₁-(C(Rₐ₁₀)(Rₐ₁₁))ₙ₂-C(O)N(Rₐ₇)-((C(Rₐ₁₂)(Rₐ₁₃))ₙ₃O)ₘ₃-;
-(C(Rₐ₈)(Rₐ₉))ₙ₁-(N(Rₐ₇)C(O)-(C(Rₐ₁₀)(Rₐ₁₁))ₙ₂)ₘ₃-;
-(C(Rₐ₈)(Rₐ₉))ₙ₁-(N(Rₐ₇)C(O)-(C(Rₐ₁₀)(Rₐ₁₁))ₙ₂)ₘ₃-(O-(C(Rₐ₁₂)(Rₐ₁₃)))ₙ₃)ₘ₄-;
-(C(Rₐ₈)(Rₐ₉))ₙ₁-(N(Rₐ₇)C(O)-(C(Rₐ₁₀)(Rₐ₁₁))ₙ₂)ₘ₃-(O-(C(Rₐ₁₂)(Rₐ₁₃))ₙ₃)ₘ₄-(O-(C(Rₐ₁₄)(Rₐ₁₅))ₙ₄)ₘ₅-;
-(C(Rₐ₈)(Rₐ₉))ₙ₁-N(Rₐ₇)C(O)-(C(Rₐ₁₀)(Rₐ₁₁))ₙ₂-(O(C(Rₐ₁₂)(Rₐ₁₃))ₙ₃)ₘ₃-;
-(C(Rₐ₈)(Rₐ₉))ₙ₁-N(Rₐ₇)C(O)N(Rₐ₇)-(C(Rₐ₁₀)(Rₐ₁₁))ₙ₂-;
-(C(Rₐ₈)(Rₐ₉))ₙ₁-((C(Rₐ₁₀)(Rₐ₁₁))ₙ₂-N(Rₐ₇)C(O))ₘ₃-;
-(C(Rₐ₈)(Rₐ₉))ₙ₁-((C(Rₐ₁₀)(Rₐ₁₁))ₙ₂-N(Rₐ₇)C(O))ₘ₃-((C(Rₐ₁₂)(Rₐ₁₃)))ₙ₃-O)ₘ₄-;
-(C(Rₐ₈)(Rₐ₉))ₙ₁-((C(Rₐ₁₀₎(Rₐ₁₁))ₙ₂-N(Rₐ₇)C(O))ₘ₃-((C(Rₐ₁₂)(Rₐ₁₃))ₙ₃O)ₘ₄-((C(Rₐ₁₄)(Rₐ₁₅))ₙ₄O)ₘ₅-;
-(C(Rₐ₈)(Rₐ₉))ₙ₁- (C(Rₐ₁₀)(Ra₁₁))ₙ₂-N(Rₐ₇)C(O)-((C(Rₐ₁₂)(Ra₁₃))ₙ₃O)ₘ₃-;
-(C(Rₐ₈)(Rₐ₉))ₙ₁-(arylene-(C(Rₐ₁₀)(Rₐ₁₁))ₙ₂)ₘ₃-;
-(C(Rₐ₈)(Rₐ₉))ₙ₁-(arylene-(C(Rₐ₁₀)(Rₐ₁₁₎)ₙ₂)ₘ₃-arylene-(C(Rₐ₁₂)(Rₐ₁₃₎)ₙ₃-;
-(C(Rₐ₈)(Rₐ₉))ₙ₁-((C(Rₐ₁₀)(Rₐ₁₁))ₙ₂-arylene)ₘ₃-;
-(C(Rₐ₈)(Rₐ₉))ₙ₁-((C(Rₐ₁₀)(Rₐ₁₁))ₙ₂-arylene)ₘ₃-(C(Rₐ₁₂)(Rₐ₁₃))ₙ₃-arylene-;
-(C(Rₐ₈)(Rₐ₉))ₙ₁-(heterocyclylene-(C(Rₐ₁₀)(Rₐ₁₁))ₙ₂)ₘ₃-;
-(C(Rₐ₈)(Rₐ₉))ₙ₁-(heterocyclylene-(C(Rₐ₁₀)(Rₐ₁₁))ₙ₂)ₘ₃-(heterocyclylene-(C(Rₐ₁₂)(Rₐ₁₃))ₙ₃)ₘ₄-;
-(C(Rₐ₈)(Rₐ₉))ₙ₁-((C(Rₐ₁₀)(Rₐ₁₁))ₙ₂-heterocyclylene)ₘ₃-;
-(C(Rₐ₈)(Rₐ₉))ₙ₁-((C(Rₐ₁₀)(Rₐ₁₁))ₙ₂-heterocyclylene)ₘ₃-((C(Rₐ₁₂)(Rₐ₁₃))ₙ₃-heterocyclylene)ₘ₄-;
-(C(Rₐ₈)(Rₐ₉))ₙ₁-(heteroarylene-(C(Rₐ₁₀)(Rₐ₁₁))ₙ₂)ₘ₃-;
-(C(Rₐ₈)(Rₐ₉))ₙ₁-(heteroarylene-(C(Rₐ₁₀)(Rₐ₁₁))ₙ₂)ₘ₃-(heteroarylene-(C(Rₐ₁₂)(Rₐ₁₃))ₙ₃)ₘ₄-;
-(C(Rₐ₈)(Rₐ₉))ₙ₁-((C(Rₐ₁₀)(Rₐ₁₁))ₙ₂-heteroarylene)ₘ₃-;
-(C(Rₐ₈)(Rₐ₉))ₙ₁-((C(Rₐ₁₀)(Ra₁₁))ₙ₂-heteroarylene)ₘ₃-((C(Rₐ₁₂)(Ra₁₃))ₙ₃-heteroarylene)ₘ₄-;
-(C(Rₐ₈)(Rₐ₉))ₙ₁-(cycloalkylene-(C(Rₐ₁₀(Rₐ₁₁))ₙ₂)ₘ₃-;
-(C(Rₐ₈)(Rₐ₉))ₙ₁-(cycloalkylene-(C(Rₐ₁₀)(Rₐ₁₁))ₙ₂)ₘ₃-(cycloalkylene-(C(Rₐ₁₂)(Rₐ₁₃))ₙ₃)ₘ₄-;
-(C(Rₐ₈)(Rₐ₉))ₙ₁-((C(Rₐ₁₀)(Rₐ₁₁))ₙ₂-cycloalkylene)ₘ₃-; or
-(C(Rₐ₈)(Rₐ₉))ₙ₁-((C(Rₐ₁₀)(Rₐ₁₁))ₙ₂-cycloalkylene)ₘ₃-((C(Rₐ₁₂)(Rₐ₁₃))ₙ₃-cycloalkylene)ₘ₄-;
wherein each Rₐ₇ independently represents H or C₁₋₃ alkyl;
the cycloalkylene (e.g., C₃₋₂₀ cycloalkylene), arylene (e.g., C₅₋₂₀ arylene), heterocyclylene (e.g., 4- to 20-membered heterocyclylene), and heteroarylene (e.g., 5- to 20-membered heteroarylene) are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof;
Rₐ₈, Rₐ₉, Rₐ₁₀, Rₐ₁₁, Rₐ₁₂, Rₐ₁₃, Rₐ₁₄, and Rₐ₁₅ each independently represent H, deuterium, hydroxy, amino, mercapto, nitro, halogen, cyano, oxo, halogenated C₁₋₆ alkyl, deuterated C₁₋₆ alkyl, C₁₋₆ alkoxy, halogenated C₁₋₆ alkoxy, C₃₋₆ cycloalkyl, halogenated C₃₋₆ cycloalkyl, deuterated C₃₋₆ cycloalkyl, 4- to 10-membered heterocyclyl, and any combination thereof; and
n1, n2, n3, n4, m3, m4, m5 each independently represent an integer of 1, 2, 3, 4, 5, 6, 7, 8, 9 or 10.

28. The compound of Formula (II) or salts, enantiomers, stereoisomers, solvates, isotopically enriched analogs, prodrugs, or polymorphs thereof as claimed in any one of claims 21-27, wherein R_{w1} represents:
-CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, - (CH₂)₁₁-, -(CH₂)₁₂-, -(CH₂)₁₃-, -(CH₂)₁₄-, -(CH₂)₁₅-; -O-CH₂-, -O-(CH₂)₂-, -O-(CH₂)₃-, -O-(CH₂)₄-, -O-(CH₂)₅-, -O-(CH₂)₆-, -O-(CH₂)₇-, -O-(CH₂)₈-, -O-(CH₂)₉-, -O-(CH₂)₁₀-, -(CH₂)₁-O-, -(CH₂)₂-O-, -(CH₂)₃-O-, -(CH₂)₄-O-, -(CH₂)₅-O-, -(CH₂)₆-O-, -(CH₂)₇-O-, -(CH₂)₈-O-, -(CH₂)₉-O-, -(CH₂)₁₀-O-, -CH₂-O-CH₂-, -CH₂-O-(CH₂)₂-, -(CH₂)₁-O-(CH₂)₃-, -(CH₂)₁-O-(CH₂)₄-, -(CH₂)₁-O-(CH₂)₅-, -(CH₂)₁-O-(CH₂)₆-, -(CH₂)₁-O-(CH₂)₇-, -(CH₂)₁-O-(CH₂)₈-, -(CH₂)₁-O-(CH₂)₉-, -(CH₂)₁-O-(CH₂)₁₀-, -(CH₂)₂-O-(CH₂)₁-, -(CH₂)₂-O-(CH₂)₂-, -(CH₂)₂-O-(CH₂)₃-, -(CH₂)₂-O-(CH₂)₄-, -(CH₂)₂-O-(CH₂)₅-, -(CH₂)₂-O-(CH₂)₆-, -(CH₂)₂-O-(CH₂)₇-, -(CH₂)₂-O-(CH₂)₈-, -(CH₂)₂-O-(CH₂)₉-, -(CH₂)₂-O-(CH₂)₁₀-, -(CH₂)₃-O-(CH₂)₁-, -(CH₂)₃-O-(CH₂)₂-, -(CH₂)₃-O-(CH₂)₃-, -(CH₂)₃-O-(CH₂)₄-, -(CH₂)₃-O-(CH₂)₅-, -(CH₂)₃-O-(CH₂)₆-, -(CH₂)₃-O-(CH₂)₇-, -(CH₂)₄-O-(CH₂)₁-, -(CH₂)₄-O-(CH₂)₂-, -(CH₂)₄-O-(CH₂)₃-, -(CH₂)₄-O-(CH₂)₄-, -(CH₂)₄-O-(CH₂)₅-, -(CH₂)₄-O-(CH₂)₆-, -(CH₂)₅-O-(CH₂)₁-, -(CH₂)₅-O-(CH₂)₂-, -(CH₂)₅-O-(CH₂)₃-, -(CH₂)₅-O-(CH₂)₄-, -(CH₂)₅-O-(CH₂)₅-, -(CH₂)₆-O-(CH₂)₁-, -(CH₂)₆-O-(CH₂)₂-, -(CH₂)₆-O-(CH₂)₃-, -(CH₂)₆-O-(CH₂)₄-, -(CH₂)₇-O-(CH₂)₁-, -(CH₂)₇-O-(CH₂)₂-, -(CH₂)₇-O-(CH₂)₃-, -(CH₂)₅-O-(CH₂)₁-, -(CH₂)₈-O-(CH₂)₂-, -CH(CH₃)-O-(CH₂)₁-, -CH(CH₃)-O-(CH₂)₂-, -CH(CH₃)-O-(CH₂)₃-, -CH(CH₃)-O-(CH₂)₄-, - CH(CH₃)-O-(CH₂)₅-, -CH(CH₃)-O-(CH₂)₆-, -CH(CH₃)-O-(CH₂)₇-, -CH(CH₃)-O-(CH₂)₈-, -CH(CH₃)-O-(CH₂)₉-, -CH(CH₃)-O-(CH₂)₁₀-, -(O(CH₂)₂)₂-, -(O(CH₂)₂)₃-, -(O(CH₂)₂)₄-, -(O(CH₂)₂)₅-, -(O(CH₂)₂)₆-, - (O(CH₂)₂)₇-, -CH₂-(O(CH₂)₂)₂-, -CH₂-(O(CH₂)₂)₃-, -CH₂-(O(CH₂)₂)₄-, -CH₂-(O(CH₂)₂)₅-, -CH₂-(O(CH₂)₂)₆-, -CH₂-(O(CH₂)₂)₇-, -CH₂-(O(CH₂)₂)₁-OCH₂-, -CH₂-(O(CH₂)₂)₂-OCH₂-, -CH₂-(O(CH₂)₂)₃-OCH₂-, -CH₂-(O(CH₂)₂)₄-OCH₂-, -CH₂-(O(CH₂)₂)₅-OCH₂-, -(CH₂)₂-(O(CH₂)₂)₂-, -(CH₂)₂-(O(CH₂)₂)₃-, - (CH₂)₂-(O(CH₂)₂)₄-, -(CH₂)₂-(O(CH₂)₂)₅-, -(CH₂)₂-(O(CH₂)₂)₆-, -(CH₂)₃-(O(CH₂)₂)₂-, -(CH₂)₃-(O(CH₂)₂)₃-, -(CH₂)₃-(O(CH₂)₂)₄-, -(CH₂)₃-(O(CH₂)₂)₅-, -(CH₂)₄-(O(CH₂)₂)₂-, -(CH₂)₄-(O(CH₂)₂)₃-, -(CH₂)₄-(O(CH₂)₂)₄-, -(CH₂)₄-(O(CH₂)₂)₅-, -CH₂-(O(CH₂)₃)₂-, -CH₂-(O(CH₂)₃)₃-, -CH₂-(O(CH₂)₃)₄-, -(CH₂)₂-(O(CH₂)₃)₂-, - (CH₂)₂-(O(CH₂)₃)₃-, -(CH₂)₂-(O(CH₂)₃)₄-, -(CH₂)₃-(O(CH₂)₃)₂-, -(CH₂)₃-(O(CH₂)₃)₃-, -(CH₂)₃-(O(CH₂)₃)₄-, -CH₂-O-(CH₂)₂-O-(CH₂)₃-, -CH₂-(O(CH₂)₂)₂-(O(CH₂)₃)₂-, (CH₂)₂-O-(CH₂)₂-O-(CH₂)₃-, -(CH₂)₂-(O(CH₂)₂)₂-(O(CH₂)₃)₂-, -(CH₂)₃-O-(CH₂)₂-O-(CH₂)₃-, -(CH₂)₃-(O(CH₂)₂)₂-(O(CH₂)₃)₂-, -CH₂-O-(CH₂)₃-O-(CH₂)₂-, -CH₂-(O(CH₂)₃)₂-(O(CH₂)₂)₂-, -(CH₂)₂-O-(CH₂)₃-O-(CH₂)₂-, -(CH₂)₂-(O(CH₂)₃)₂-(O(CH₂)₂)₂-, -(CH₂)₃-O-(CH₂)₃-O-(CH₂)₂-, -(CH₂)₃-(O(CH₂)₃)₂-(O(CH₂)₂)₂-, -CH₂-O-(CH₂)₂-O-CH₂-, -(CH₂)₂-O-(CH₂)₂-O-CH₂-, -(CH₂)₂-(O(CH₂)₂)₂-O-(CH₂)₃-, -(CH₂)₂-(O(CH₂)₂)₃-O-(CH₂)₃-, -(CH₂)₂-(O(CH₂)₂)₄-O-(CH₂)₃-, -(CH₂)₅-(O(CH₂)₂)₂-O-(CH₂)₅-, -(CH₂)₅-(O(CH₂)₂)₂-O-(CH₂)₆-, -(CH₂)₁-N(Rₐ₇)-(CH₂)₁-, -(CH₂)₁-N(Rₐ₇)-(CH₂)₂-, -(CH₂)₁-N(Rₐ₇)-(CH₂)₃-, -(CH₂)₁-N(Rₐ₇)-(CH₂)₄-, -(CH₂)₁-N(Rₐ₇)-(CH₂)₅-, -(CH₂)₁-N(Rₐ₇)-(CH₂)₆-, -(CH₂)₁-N(Rₐ₇)-(CH₂)₇-, -(CH₂)₁-N(Rₐ₇)-(CH₂)₈-, -(CH₂)₁-N(Rₐ₇)-(CH₂)₉-, -(CH₂)₁-N(Rₐ₇)-(CH₂)₁₀-, -N(Rₐ₇)-(CH₂)₁-, -N(Rₐ₇)-(CH₂)₂-, -N(Rₐ₇)-(CH₂)₃-, -N(Rₐ₇)-(CH₂)₄-, -N(Rₐ₇)-(CH₂)₅-, -N(Rₐ₇)-(CH2)6-, -N(Rₐ₇)-(CH₂)₇-, -N(Rₐ₇)-(CH₂)₈-, -N(Rₐ₇)-(CH₂)₉-, -N(Rₐ₇)-(CH₂)₁₀-, -(CH₂)₂-N(Rₐ₇)-(CH₂)₁-, - (CH₂)₂-N(Rₐ₇)-(CH₂)₂-, -(CH₂)₂-N(Rₐ₇)-(CH₂)₃-, -(CH₂)₂-N(Rₐ₇)-(CH₂)₄-, -(CH₂)₂-N(Rₐ₇)-(CH₂)₅-, -(CH₂)₂-N(Rₐ₇)-(CH₂)₆-, -(CH₂)₂-N(Rₐ₇)-(CH₂)₇-, -(CH₂)₂-N(Rₐ₇)-(CH₂)₈-, -(CH₂)₂-N(Rₐ₇)-(CH₂)₉-, -(CH₂)₂-N(Rₐ₇)-(CH₂)₁₀-, -(CH₂)₃-N(Rₐ₇)-(CH₂)₁-, -(CH₂)₃-N(Rₐ₇)-(CH₂)₂-, -(CH₂)₃-N(Rₐ₇)-(CH₂)₃-, -(CH₂)₄-N(Rₐ₇)-(CH2)1-, -(CH₂)₄-N(Rₐ₇)-(CH₂)₂-, -(CH₂)₄-N(Rₐ₇)-(CH₂)₃-, -(CH₂)₄-N(Rₐ₇)-(CH₂)₄-, -(CH₂)₅-N(Rₐ₇)-(CH₂)₁-, -(CH₂)₅-N(Rₐ₇)-(CH₂)₂-, -(CH₂)₅-N(Rₐ₇)-(CH₂)₃-, -(CH₂)₅-N(Rₐ₇)-(CH₂)₄-, -(CH₂)₅-N(Rₐ₇)-(CH₂)₅-, - (CH₂)₆-N(Rₐ₇)-(CH₂)₁-, -(CH₂)₆-N(Rₐ₇)-(CH₂)₂-, -(CH₂)₆-N(Rₐ₇)-(CH₂)₃-, -(CH₂)₇-N(Rₐ₇)-(CH₂)₁-, -(CH₂)₇-N(Rₐ₇)-(CH₂)₂-, -(CH₂)₇-N(Rₐ₇)-(CH₂)₃-, -(CH₂)₈-N(Rₐ₇)-(CH₂)₁-, -(CH₂)₈-N(Rₐ₇)-(CH₂)₂-, -(CH₂)₈-N(Rₐ₇)-(CH₂)₃-, -CH(CH₃)-N(Rₐ₇)-(CH₂)₁-, -CH(CH₃)-N(Rₐ₇)-(CH₂)₂-, -CH(CH₃)-N(Rₐ₇)-(CH₂)₃-, -CH(CH₃)-N(Rₐ₇)-(CH₂)₄-, -CH(CH₃)-N(Rₐ₇)-(CH₂)₅-, -CH(CH₃)-N(Rₐ₇)-(CH₂)₆-, -CH(CH₃)-N(Rₐ₇)-(CH₂)₇-, - CH(CH₃)-N(Rₐ₇)-(CH₂)₈-, -CH(CH₃)-N(Rₐ₇)-(CH₂)₉-, -CH(CH₃)-N(Rₐ₇)-(CH₂)₁₀-, -C(O)NHCH₂-, - C(O)NH(CH₂)₂-, -C(O)NH(CH₂)₃-, -C(O)NH(CH₂)₄-, -C(O)NH(CH₂)₅-, -CH₂C(O)NHCH₂-, - (CH₂)₂C(O)NH(CH₂)₂-, -(CH₂)₂C(O)NH(CH₂)₃-, -(CH₂)₂C(O)NH(CH₂)₄-, -(CH₂)₂C(O)NH(CH₂)₅-, - (CH₂)₃C(O)NH(CH₂)₃-, -(CH₂)₃C(O)NH(CH₂)₄-, -(CH₂)₄C(O)NH(CH₂)₄-, -(CH₂)₅C(O)NH(CH₂)₅-, - (CH₂)₆C(O)NH(CH₂)₇-, -(CH₂)₆C(O)NH(CH₂)₆-, -(CH₂)₇C(O)NH(CH₂)₇-, -(CH₂)₂C(O)NH(CH₂)₂-O-(CH₂)₂-, -NHC(O)CH₂-, -NHC(O)(CH₂)₂-, -NHC(O)(CH₂)₃-, -NHC(O)(CH₂)₄-, -NHC(O)(CH₂)₅-, - CH₂NHC(O)CH₂-, -(CH₂)₂NHC(O)(CH₂)₂-, -(CH₂)₂NHC(O)(CH₂)₃-, -(CH₂)₂NHC(O)(CH₂)₄-, - (CH₂)₂NHC(O)(CH₂)₅-, -(CH₂)₃NHC(O)(CH₂)₃-, -(CH₂)₃NHC(O)(CH₂)₄-, -(CH₂)₄NHC(O)(CH₂)₄-, - (CH₂)₅NHC(O)(CH₂)₅-, -(CH₂)₆NHC(O)(CH₂)₇-, -(CH₂)₆NHC(O)(CH₂)₆-, -(CH₂)₇NHC(O)(CH₂)₇-, - (CH₂)₄NHC(O)(CH₂)₈-, -(CH₂)₂NHC(O)(CH₂)₂-O-(CH₂)₂-, -(CH₂)₄NHC(O)CH₂-, -phenylene-CH₂-, - phenylene-(CH₂)₂-, -phenylene-(CH₂)₃-, -phenylene-(CH₂)₄-, -phenylene-(CH₂)₅-, -phenylene-(CH₂)₆-, - phenylene-(CH₂)₇-, -phenylene-(CH₂)₈-, -CH₂-phenylene-CH₂-, -CH₂-phenylene-(CH₂)₂-, -CH₂-phenylene-(CH₂)₃-, -CH₂-phenylene-(CH₂)₄-, -CH₂-phenylene-(CH₂)₅-, -CH₂-phenylene-(CH₂)₆-, -CH₂-phenylene-(CH₂)₇-, -CH₂-phenylene-(CH₂)₈-, -(CH₂)₂-phenylene-(CH₂)₁-, -(CH₂)₂-phenylene-(CH₂)₂-, - (CH₂)₂-phenylene-(CH₂)₃-, -(CH₂)₂-phenylene-(CH₂)₄-, -(CH₂)₂-phenylene-(CH₂)₅-, -(CH₂)₂-phenylene-(CH₂)₆-, -(CH₂)₂-phenylene-(CH₂)₇-, -(CH₂)₂-phenylene-(CH₂)₈-, -(CH₂)₃-phenylene-CH₂-, -(CH₂)₃-phenylene-(CH₂)₂-, -(CH₂)₃-phenylene-(CH₂)₃-, -(CH₂)₃-phenylene-(CH₂)₄-, -(CH₂)₃-phenylene-(CH₂)₅-, - (CH₂)₃-phenylene-(CH₂)₆-, -(CH₂)₃-phenylene-(CH₂)₇-, -(CH₂)₃-phenylene-(CH₂)₈-, -(CH₂)₄-phenylene-CH₂-, -(CH₂)₄-phenylene-(CH₂)₂-, -(CH₂)₄-phenylene-(CH₂)₃-, -(CH₂)₄-phenylene-(CH₂)₄-, -(CH₂)₄-phenylene-(CH₂)₅-, -(CH₂)₄-phenylene-(CH₂)₆-, -(CH₂)₄-phenylene-(CH₂)₇-, -(CH₂)₄-phenylene-(CH₂)₈-, - (CH₂)₅-phenylene-(CH₂)₁-, -(CH₂)₅-phenylene-(CH₂)₂-, -(CH₂)₅-phenylene-(CH₂)₃-, -(CH₂)₅-phenylene-(CH₂)₄-, -(CH₂)₅-phenylene-(CH₂)₅-, -(CH₂)₅-phenylene-(CH₂)₆-, -(CH₂)₅-phenylene-(CH₂)₇-, -(CH₂)₅-phenylene-(CH₂)₈-, -(CH₂)₆-phenylene-(CH₂)₁-, -(CH₂)₆-phenylene-(CH₂)₂-, -(CH₂)₆-phenylene-(CH₂)₃-, - (CH₂)₆-phenylene-(CH₂)₄-, -(CH₂)₆-phenylene-(CH₂)₅-, -(CH₂)₆-phenylene-(CH₂)₆-, -(CH₂)₆-phenylene-(CH₂)₇-, -(CH₂)₆-phenylene-(CH₂)₈-, -(CH₂)₇-phenylene-(CH₂)₁-, -(CH₂)₇-phenylene-(CH₂)₂-, -(CH₂)₇-phenylene-(CH₂)₃-, -(CH₂)₇-phenylene-(CH₂)₄-, -(CH₂)₇-phenylene-(CH₂)₈-, -(CH₂)₈-phenylene-CH₂-, - (CH₂)₈-phenylene-(CH₂)₂-, -(CH₂)₈-phenylene-(CH₂)₃-, -(CH₂)₈-phenylene-(CH₂)₄-, -(CH₂)₈-phenylene-(CH₂)₅-, -(CH₂)₈-phenylene-(CH₂)₆-, -(CH₂)₈-phenylene-(CH₂)₇-, -N(Rₐ₇)-CH₂-phenylene-CH₂-, -N(Rₐ₇)-CH₂-phenylene-(CH₂)₂-, -N(Rₐ₇)-CH₂-phenylene-(CH₂)₃-, -N(Rₐ₇)-CH₂-phenylene-(CH₂)₄-, -N(Rₐ₇)-CH₂-phenylene-(CH₂)₅-, -N(Rₐ₇)-CH₂-phenylene-(CH₂)₆-, -N(Rₐ₇)-CH₂-phenylene-(CH₂)₇-, -N(Rₐ₇)-CH₂-phenylene-(CH₂)₈-, -CH₂-N(Rₐ₇)-CH₂-phenylene-CH₂-, -CH₂-N(Rₐ₇)-CH₂-phenylene-(CH₂)₂-, -CH₂-N(Rₐ₇)-CH₂-phenylene-(CH₂)₃-, -CH₂-N(Rₐ₇)-CH₂-phenylene-(CH₂)₄-, -CH₂-N(Rₐ₇)-CH₂-phenylene-(CH₂)₅-, -CH₂-N(Rₐ₇)-CH₂-phenylene-(CH₂)₆-, -CH₂-N(Rₐ₇)-CH₂-phenylene-(CH₂)₇-, -CH₂-N(Rₐ₇)-CH₂-phenylene-(CH₂)₈-, -CH₂-N(Rₐ₇)-(CH₂)₂-phenylene-CH₂-, -CH₂-N(Rₐ₇)-(CH₂)₂-phenylene-(CH₂)₂-, -CH₂-N(Rₐ₇)-(CH₂)₂-phenylene-(CH₂)₃-, -CH₂-N(Rₐ₇)-(CH₂)₂-phenylene-(CH₂)₄-, -CH₂-N(Rₐ₇)-(CH₂)₂-phenylene-(CH₂)₅-, -CH₂-N(Rₐ₇)-(CH₂)₂-phenylene-(CH₂)₆-, -CH₂-N(Rₐ₇)-(CH₂)₂-phenylene-(CH₂)₇-, - CH₂-N(Rₐ₇)-(CH₂)₂-phenylene-(CH₂)₈-, -(CH₂)₂-N(Rₐ₇)-CH₂-phenylene-CH₂-, -(CH₂)₂-N(Rₐ₇)-CH₂-phenylene-(CH₂)₂-, -(CH₂)₂-N(Rₐ₇)-CH₂-phenylene-(CH₂)₃-, -(CH₂)₂-N(Rₐ₇)-CH₂-phenylene-(CH₂)₄-, - (CH₂)₂-N(Rₐ₇)-CH₂-phenylene-(CH₂)₅-, -(CH₂)₂-N(Rₐ₇)-CH₂-phenylene-(CH₂)₆-, -(CH₂)₂-N(Rₐ₇)-CH₂-phenylene-(CH₂)₇-, -(CH₂)₂-N(Rₐ₇)-CH₂-phenylene-(CH₂)₈-, -(CH₂)₃-N(Rₐ₇)-CH₂-phenylene-CH₂-, - (CH₂)₃-N(Rₐ₇)-CH₂-phenylene-(CH₂)₂-, -(CH₂)₃-N(Rₐ₇)-CH₂-phenylene-(CH₂)₃-, -(CH₂)₃-N(Rₐ₇)-CH₂-phenylene-(CH₂)₈-, -(CH₂)₄-N(Rₐ₇)-CH₂-phenylene-CH₂-, -(CH₂)₄-N(Rₐ₇)-CH₂-phenylene-(CH₂)₂-, - (CH₂)₄-N(Rₐ₇)-CH₂-phenylene-(CH₂)₃-, -(CH₂)₄-N(Rₐ₇)-CH₂-phenylene-(CH₂)₈-, -(CH₂)₅-N(Rₐ₇)-CH₂-phenylene-(CH₂)₃-, -(CH₂)₅-N(Rₐ₇)-CH₂-phenylene-(CH₂)₈-, -(CH₂)₆-N(Rₐ₇)-CH₂-phenylene-(CH₂)₃-, - (CH₂)₆-N(Rₐ₇)-CH₂-phenylene-(CH₂)₈-, -(CH₂)₇-N(Rₐ₇)-CH₂-phenylene-(CH₂)₃-, -(CH₂)₈-N(Rₐ₇)-CH₂-phenylene-CH₂-, -(CH₂)₈-N(Rₐ₇)-CH₂-phenylene-(CH₂)₂-, -(CH₂)₈-N(Rₐ₇)-CH₂-phenylene-(CH₂)₃-, - (CH₂)₈-N(Rₐ₇)-CH₂-phenylene-(CH₂)₄-, -(CH₂)₈-N(Rₐ₇)-CH₂-phenylene-(CH₂)₅-, -(CH₂)₈-N(Rₐ₇)-CH₂-phenylene-(CH₂)₆-, -piperidinylene-CH₂-, -piperidinylene-(CH₂)₂-, -piperidinylene-(CH₂)₃-, - piperidinylene-(CH₂)₄-, -piperidinylene-(CH₂)₅-, -piperidinylene-(CH₂)₆-, -piperidinylene-(CH₂)₇-, - piperidinylene-(CH₂)₈-, -CH₂-piperidinylene-CH₂-, -CH₂-piperidinylene-(CH₂)₂-, -CH₂-piperidinylene-(CH₂)₃-, -CH₂-piperidinylene-(CH₂)₄-, -CH₂-piperidinylene-(CH₂)₅-, -CH₂-piperidinylene-(CH₂)₆-, -CH₂-piperidinylene-(CH₂)₇-, -CH₂-piperidinylene-(CH₂)₈-, -(CH₂)₂-piperldinylene-(CH₂)₁-, -(CH₂)₂-piperidinylene-(CH₂)₂-, -(CH₂)₂-piperidinylene-(CH₂)₃-, -(CH₂)₂-piperidinylene-(CH₂)₄-, -(CH₂)₂-piperidinylene-(CH₂)₅-, -(CH₂)₂-piperidinylene-(CH₂)₆-, -(CH₂)₂-piperidinylene-(CH₂)₇-, -(CH₂)₂-piperidinylene-(CH₂)₈-, -(CH₂)₃-piperidinylene-CH₂-, -(CH₂)₃-piperidinylene-(CH₂)₂-, -(CH₂)₃-piperidinylene-(CH₂)₃-, -(CH₂)₃-piperidinylene-(CH₂)₄-, -(CH₂)₃-piperidinylene-(CH₂)₅-, -(CH₂)₃-piperidinylene-(CH₂)₆-, -(CH₂)₃-piperidinylene-(CH₂)₇-, -(CH₂)₃-piperidinylene-(CH₂)₈-, -(CH₂)₄-piperidinylene-CH₂-, -(CH₂)₄-piperidinylene-(CH₂)₂-, -(CH₂)₄-piperidinylene-(CH₂)₃-, -(CH₂)₄-piperidinylene-(CH₂)₄-, -(CH₂)₄-piperidinylene-(CH₂)₅-, -(CH₂)₄-piperidinylene-(CH₂)₆-, -(CH₂)₄-piperidinylene-(CH₂)₇-, -(CH₂)₄-piperidinylene-(CH₂)₈-, -(CH₂)₅-piperidinylene-(CH₂)₁-, -(CH₂)₅-piperidinylene-(CH₂)₂-, -(CH₂)₅-piperidinylene-(CH₂)₃-, -(CH₂)₅-piperidinylene-(CH₂)₄-, -(CH₂)₅-piperidinylene-(CH₂)₅-, -(CH₂)₅-piperidinylene-(CH₂)₆-, -(CH₂)₅-piperidinylene-(CH₂)₇-, -(CH₂)₅-piperidinylene-(CH₂)₈-, -(CH₂)₆-piperidinylene-(CH₂)₁-, -(CH₂)₆-piperidinylene-(CH₂)₂-, -(CH₂)₆-piperidinylene-(CH₂)₃-, -(CH₂)₆-piperidinylene-(CH₂)₄-, -(CH₂)₆-piperidinylene-(CH₂)₅-, -(CH₂)₆-piperidinylene-(CH₂)₆-, -(CH₂)₆-piperidinylene-(CH₂)₇-, -(CH₂)₆-piperidinylene-(CH₂)₈-, -(CH₂)₇-piperldinylene-(CH₂)₁-, -(CH₂)₇-piperidinylene-(CH₂)₂-, -(CH₂)₇-piperidinylene-(CH₂)₃-, -(CH₂)₇-piperidinylene-(CH₂)₄-, -(CH₂)₇-piperidinylene-(CH₂)₈-, -(CH₂)₈-piperidinylene-CH₂-, -(CH₂)₈-piperidinylene-(CH₂)₂-, -(CH₂)₈-piperidinylene-(CH₂)₃-, -(CH₂)₈-piperidinylene-(CH₂)₄-, -(CH₂)₈-piperidinylene-(CH₂)₅-, -(CH₂)₈-piperldinylene-(CH₂)₆-, -(CH₂)₈-piperidinylene-(CH₂)₇-, -N(Rₐ₇)-CH₂-piperidinylene-CH₂-, -N(Rₐ₇)-CH₂-piperidinylene-(CH₂)₂-, -N(Rₐ₇)-CH₂-piperidinylene-(CH₂)₃-, -N(Rₐ₇)-CH₂-piperidinylene-(CH₂)₄-, -N(Rₐ₇)-CH₂-piperidinylene-(CH₂)₅-, -N(Rₐ₇)-CH₂-piperidinylene-(CH₂)₆-, - N(Rₐ₇)-CH₂-piperidinylene-(CH₂)₇-, -N(Rₐ₇)-CH₂-piperidinylene-(CH₂)₈-, -CH₂-N(Rₐ₇)-CH₂-piperidinylene-CH₂-, -CH₂-N(Rₐ₇)-CH₂-piperidinylene-(CH₂)₂-, -CH₂-N(Rₐ₇)-CH₂-piperidinylene-(CH₂)₃-, -CH₂-N(Rₐ₇)-CH₂-piperidinylene-(CH₂)₄-, -CH₂-N(Rₐ₇)-CH₂-piperidinylene-(CH₂)₅-, -CH₂-N(Rₐ₇)-CH₂-piperidinylene-(CH₂)₆-, -CH₂-N(Rₐ₇)-CH₂-piperidinylene-(CH₂)₇-, -CH₂-N(Rₐ₇)-CH₂-piperidinylene-(CH₂)₈-, -CH₂-N(Rₐ₇)-(CH₂)₂-piperidinylene-CH₂-, -CH₂-N(Rₐ₇)-(CH₂)₂-piperidinylene-(CH₂)₂-, -CH₂-N(Rₐ₇)-(CH₂)₂-piperidinylene-(CH₂)₃-, -CH₂-N(Rₐ₇)-(CH₂)₂-piperidinylene-(CH₂)₄-, -CH₂-N(Rₐ₇)-(CH₂)₂-piperidinylene-(CH₂)₅-, -CH₂-N(Rₐ₇)-(CH₂)₂-piperidinylene-(CH₂)₆-, -CH₂-N(Rₐ₇)-(CH₂)₂-piperidinylene-(CH₂)₇-, -CH₂-N(Rₐ₇)-(CH₂)₂-piperidinylene-(CH₂)₈-, -(CH₂)₂-N(Rₐ₇)-CH₂-piperidinylene-CH₂-, -(CH₂)₂-N(Rₐ₇)-CH₂-piperidinylene-(CH₂)₂-, -(CH₂)₂-N(Rₐ₇)-CH₂-piperidinylene-(CH₂)₃-, -(CH₂)₂-N(Rₐ₇)-CH₂-piperidinylene-(CH₂)₄-, -(CH₂)₂-N(Rₐ₇)-CH₂-piperidinylene-(CH₂)₅-, -(CH₂)₂-N(Rₐ₇)-CH₂-piperidinylene-(CH₂)₆-, -(CH₂)₂-N(Rₐ₇)-CH₂-piperidinylene-(CH₂)₇-, -(CH₂)₂-N(Rₐ₇)-CH₂-piperidinylene-(CH₂)₈-,-(CH₂)₃-N(Rₐ₇)-CH₂-piperidinylene-CH₂-, -(CH₂)₃-N(Rₐ₇)-CH₂-piperidinylene-(CH₂)₂-, -(CH₂)₃-N(Rₐ₇)-CH₂-piperidinylene-(CH₂)₃-, -(CH₂)₃-N(Rₐ₇)-CH₂-piperidinylene-(CH₂)₈-, -(CH₂)₄-N(Rₐ₇)-CH₂-piperidinylene-CH₂-, -(CH₂)₄-N(Rₐ₇)-CH₂-piperidinylene-(CH₂)₂-, -(CH₂)₄-N(Rₐ₇)-CH₂-piperidinylene-(CH₂)₃-, -(CH₂)₄-N(Rₐ₇)-CH₂-piperidinylene-(CH₂)₈-, -(CH₂)₅-N(Rₐ₇)-CH₂-piperidinylene-(CH₂)₃-, - (CH₂)₅-N(Rₐ₇)-CH₂-piperidinylene-(CH₂)₈-, -(CH₂)₆-N(Rₐ₇)-CH₂-piperidinylene-(CH₂)₃-, -(CH₂)₆-N(Rₐ₇)-CH₂-piperidinylene-(CH₂)₈-, -(CH₂)₇-N(Rₐ₇)-CH₂-piperldinylene-(CH₂)₃-, -(CH₂)₈-N(Rₐ₇)-CH₂-piperidinylene-CH₂-, -(CH₂)₈-N(Rₐ₇)-CH₂-piperidinylene-(CH₂)₂-, -(CH₂)₈-N(Rₐ₇)-CH₂-piperidinylene-(CH₂)₃-, -(CH₂)₈-N(Rₐ₇)-CH₂-piperidinylene-(CH₂)₄-, -(CH₂)₈-N(Rₐ₇)-CH₂-piperidinylene-(CH₂)₅-, - (CH₂)₈-N(Rₐ₇)-CH₂-piperidinylene-(CH₂)₆-, -piperazinylene-CH₂-, -piperazinylene-(CH₂)₂-, - piperazinylene-(CH₂)₃-, -piperazinylene-(CH₂)₄-, -piperazinylene-(CH₂)₅-, -piperazinylene-(CH₂)₆-, - piperazinylene-(CH₂)₇-, -piperazinylene-(CH₂)₈-, -CH₂-piperazinylene-CH₂-, -CH₂-piperazinylene-(CH₂)₂-, -CH₂-piperazinylene-(CH₂)₃-, -CH₂-piperazinylene-(CH₂)₄-, -CH₂-piperazinylene-(CH₂)₅-, -CH₂-piperazinylene-(CH₂)₆-, -CH₂-piperazinylene-(CH₂)₇-, -CH₂-piperazinylene-(CH₂)₈-, -(CH₂)₂-piperazinylene-(CH₂)₁-, -(CH₂)₂-piperazinylene-(CH₂)₂-, -(CH₂)₂-piperazinylene-(CH₂)₃-, -(CH₂)₂-piperazinylene-(CH₂)₄-, -(CH₂)₂-piperazinylene-(CH₂)₅-, -(CH₂)₂-piperazinylene-(CH₂)₆-, -(CH₂)₂-piperazinylene-(CH₂)₇-, -(CH₂)₂-piperazinylene-(CH₂)₈-, -(CH₂)₃-piperazinylene-CH₂-, -(CH₂)₃-piperazinylene-(CH₂)₂-, -(CH₂)₃-piperazinylene-(CH₂)₃-, -(CH₂)₃-piperazinylene-(CH₂)₄-, -(CH₂)₃-piperazinylene-(CH₂)₅-, -(CH₂)₃-piperazinylene-(CH₂)₆-, -(CH₂)₃-piperazinylene-(CH₂)₇-, -(CH₂)₃-piperazinylene-(CH₂)₈-, -(CH₂)₄-piperazinylene-CH₂-, -(CH₂)₄-piperazinylene-(CH₂)₂-, -(CH₂)₄-piperazinylene-(CH₂)₃-, -(CH₂)₄-piperazinylene-(CH₂)₄-, -(CH₂)₄-piperazinylene-(CH₂)₅-, -(CH₂)₄-piperazinylene-(CH₂)₆-, -(CH₂)₄-piperazinylene-(CH₂)₇-, -(CH₂)₄-piperazinylene-(CH₂)₈-, -(CH₂)₅-piperazinylene-(CH₂)₁-, -(CH₂)₅-piperazinylene-(CH₂)₂-, -(CH₂)₅-piperazinylene-(CH₂)₃-, -(CH₂)₅-piperazinylene-(CH₂)₄-, -(CH₂)₅-piperazinylene-(CH₂)₅-, -(CH₂)₅-piperazinylene-(CH₂)₆-, -(CH₂)₅-piperazinylene-(CH₂)₇-, -(CH₂)₅-piperazinylene-(CH₂)₈-, -(CH₂)₆-piperazinylene-(CH₂)₁-, -(CH₂)₆-piperazinylene-(CH₂)₂-, -(CH₂)₆-piperazinylene-(CH₂)₃-, -(CH₂)₆-piperazinylene-(CH₂)₄-, -(CH₂)₆-piperazinylene-(CH₂)₅-, -(CH₂)₆-piperazinylene-(CH₂)₆-, -(CH₂)₆-piperazinylene-(CH₂)₇-, -(CH₂)₆-piperazinylene-(CH₂)₈-, -(CH₂)₇-piperazinylene-(CH₂)₁-, -(CH₂)₇-piperazinylene-(CH₂)₂-, -(CH₂)₇-piperazinylene-(CH₂)₃-, -(CH₂)₇-piperazinylene-(CH₂)₄-, -(CH₂)₈-piperazinylene-CH₂-, -(CH₂)₈-piperazinylene-(CH₂)₂-, -(CH₂)₈-piperazinylene-(CH₂)₃-, -(CH₂)₈-piperazinylene-(CH₂)₄-, -(CH₂)₈-piperazinylene-(CH₂)₅-, or -(CH₂)₈-piperazinylene-(CH₂)₆-;
wherein each Rₐ₇ independently represents H or C₁₋₃ alkyl;
the phenylene, piperazinylene and piperidinylene are each independently optionally substituted with a substituent selected from the group consisting of deuterium, hydroxy, amino, mercapto, nitro, halogen, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₃₋₆cycloalkyl, halogenated C₃₋₆cycloalkyl, deuterated C₃₋₆cycloalkyl, 4- to 10-membered heterocyclyl, and any combination thereof.

29. The compound of Formula (I) or salts, enantiomers, stereoisomers, solvates, isotopically enriched analogs, prodrugs, or polymorphs thereof as claimed in claim 21, which is selected from:
3-(5-fluoro-1-thioxoisoindolin-2-yl)piperidine-2,6-dione;
3-(4-fluoro-1-thioxoisoindolin-2-yl)piperidine-2,6-dione;
3-(6-fluoro-1-thioxoisoindolin-2-yl)piperidine-2,6-dione;
3-(7-fluoro-1-thioxoisoindolin-2-yl)piperidine-2,6-dione;
3-(4-hydroxy-1-thioxoisoindolin-2-yl)piperidine-2,6-dione;
3-(5-hydroxy-1-thioxoisoindolin-2-yl)piperidine-2,6-dione;
3-(6-hydroxy-1-thioxoisoindolin-2-yl)piperidine-2,6-dione;
3-(7-hydroxy-1-thioxoisoindolin-2-yl)piperidine-2,6-dione;
3-(5-bromo-1-thioxoisoindolin-2-yl)piperidine-2,6-dione;
3-(4-bromo-1-thioxoisoindolin-2-yl)piperidine-2,6-dione;
3-(5-bromo-1-thioxoisoindolin-2-yl)piperidine-2,6-dione;
3-(7-bromo-1-thioxoisoindolin-2-yl)piperidine-2,6-dione;
3-(5-(hydroxymethyl)-1-thioxoisoindolin-2-yl)piperidine-2,6-dione;
3-(4-(hydroxymethyl)-1-thioxoisoindolin-2-yl)piperidine-2,6-dione;
3-(6-(hydroxymethyl)-1-thioxoisoindolin-2-yl)piperidine-2,6-dione;
3-(5-(aminomethyl)-1-thioxoisoindolin-2-yl)piperidine-2,6-dione;
3-(5-(2-aminoethyl)-1-thioxoisoindolin-2-yl)piperidine-2,6-dione;
3-(5-(3-aminopropyl)-1-thioxoisoindolin-2-yl)piperidine-2,6-dione;
3-(4-(aminomethyl)-1-thioxoisoindolin-2-yl)piperidine-2,6-dione;
3-(4-(2-aminoethyl)-1-thioxoisoindolin-2-yl)piperidine-2,6-dione;
3-(4-(3-aminopropyl)-1-thioxoisoindolin-2-yl)piperidine-2,6-dione;
3-(5-(iodomethyl)-1-thioxoisoindolin-2-yl)piperidine-2,6-dione;
3-(5-(2-iodoethyl)-1-thioxoisoindolin-2-yl)piperidine-2,6-dione;
3-(5-(3-iodopropyl)-1-thioxoisoindolin-2-yl)piperidine-2,6-dione;
3-(4-(iodomethyl)-1-thioxoisoindolin-2-yl)piperidine-2,6-dione;
3-(4-(2-iodoethyl)-1-thioxoisoindolin-2-yl)piperidine-2,6-dione;
3-(4-(3-iodopropyl)-1-thioxoisoindolin-2-yl)piperidine-2,6-dione;
3-(5-(bromomethyl)-1-thioxoisoindolin-2-yl)piperidine-2,6-dione;
3-(5-(2-bromoethyl)-1-thioxoisoindolin-2-yl)piperidine-2,6-dione;
3-(5-(3-bromopropyl)-1-thioxoisoindolin-2-yl)piperidine-2,6-dione;
3-(4-(bromomethyl)-1-thioxoisoindolin-2-yl)piperidine-2,6-dione;
3-(4-(2-bromoethyl)-1-thioxoisoindolin-2-yl)piperidine-2,6-dione;
3-(4-(3-bromopropyl)-1-thioxoisoindolin-2-yl)piperidine-2,6-dione;
3-(5-(chloromethyl)-1-thioxoisoindolin-2-yl)piperidine-2,6-dione;
3-(5-(2-chloroethyl)-1-thioxoisoindolin-2-yl)piperidine-2,6-dione;
3-(5-(3-chloropropyl)-1-thioxoisoindolin-2-yl)piperidine-2,6-dione;
3-(4-(chloromethyl)-1-thioxoisoindolin-2-yl)piperidine-2,6-dione;
3-(4-(2-chloroethyl)-1-thioxoisoindolin-2-yl)piperidine-2,6-dione;
3-(4-(3-chloropropyl)-1-thioxoisoindolin-2-yl)piperidine-2,6-dione;
(2-(2,6-dioxopiperidin-3-yl)-1-thioxoisoindolin-5-yl)methyl methanesulfonate;
(2-(2,6-dioxopiperidin-3-yl)-1-thioxoisoindolin-5-yl)methyl 4-methylbenzenesulfonate;
2-(2-(2,6-dioxopiperidin-3-yl)-1-thioxoisoindolin-5-yl)ethyl 4-methylbenzenesulfonate;
3-(2-(2,6-dioxopiperidin-3-yl)-1-thioxoisoindolin-5-yl)propyl 4-methylbenzenesulfonate;
(2-(2,6-dioxopiperidin-3-yl)-1-thioxoisoindolin-4-yl)methyl 4-methylbenzenesulfonate;
2-(2-(2,6-dioxopiperidin-3-yl)-1-thioxoisoindolin-4-yl)ethyl 4-methylbenzenesulfonate;
3-(2-(2,6-dioxopiperidin-3-yl)-1-thioxoisoindolin-4-yl)propyl 4-methylbenzenesulfonate;
2-(2,6-dioxopiperidin-3-yl)-1-thioxoisoindoline-5-carboxylic acid;
2-(2,6-dioxopiperidin-3-yl)-1-thioxoisoindoline-4-carboxylic acid;
2-(2,6-dioxopiperidin-3-yl)-1-thioxoisoindoline-5-carboxamide;
2-(2,6-dioxopiperidin-3-yl)-1-thioxoisoindoline-4-carboxamide;
3-(5-(2-iodoethoxy)-1-thioxoisoindolin-2-yl)piperidine-2,6-dione;
3-(4-(2-iodoethoxy)-1-thioxoisoindolin-2-yl)piperidine-2,6-dione;
3-(5-(2-bromoethoxy)-1-thioxoisoindolin-2-yl)piperidine-2,6-dione;
3-(4-(2-bromoethoxy)-1-thioxoisoindolin-2-yl)piperidine-2,6-dione;
3-(5-(2-chloroethoxy)-1-thioxoisoindolin-2-yl)piperidine-2,6-dione;
3-(4-(2-chloroethoxy)-1-thioxoisoindolin-2-yl)piperidine-2,6-dione;
2-((2-(2,6-dioxopiperidin-3-yl)-1-thioxoisoindolin-5-yl)oxy)ethyl 4-methylbenzenesulfonate;
2-((2-(2,6-dioxopiperidin-3-yl)-1-thioxoisoindolin-4-yl)oxy)ethyl 4-methylbenzenesulfonate;
3-(5-((2-iodoethyl)amino)-1-thioxoisoindolin-2-yl)piperidine-2,6-dione;
3-(4-((2-iodoethyl)amino)-1-thioxoisoindolin-2-yl)piperidine-2,6-dione;
3-(5-((2-bromoethyl)amino)-1-thioxoisoindolin-2-yl)piperidine-2,6-dione;
3-(4-((2-bromoethyl)amino)-1-thioxoisoindolin-2-yl)piperidine-2,6-dione;
3-(5-((2-chloroethyl)amino)-1-thioxoisoindolin-2-yl)piperidine-2,6-dione;
3-(4-((2-chloroethyl)amino)-1-thioxoisoindolin-2-yl)piperidine-2,6-dione;
2-((2-(2,6-dioxopiperidin-3-yl)-1-thioxoisoindolin-5-yl)amino)ethyl 4-methylbenzenesulfonate;
2-((2-(2,6-dioxopiperidin-3-yl)-1-thioxoisoindolin-4-yl)amino)ethyl 4-methylbenzenesulfonate;
3-(5-((2-iodoethyl)thio)-1-thioxoisoindolin-2-yl)piperidine-2,6-dione;
3-(4-((2-iodoethyl)thio)-1-thioxoisoindolin-2-yl)piperidine-2,6-dione;
3-(5-((2-bromoethyl)thio)-1-thioxoisoindolin-2-yl)piperidine-2,6-dione;
3-(4-((2-bromoethyl)thio)-1-thioxoisoindolin-2-yl)piperidine-2,6-dione;
3-(5-((2-chloroethyl)thio)-1-thioxoisoindolin-2-yl)piperidine-2,6-dione;
3-(4-((2-chloroethyl)thio)-1-thioxoisoindolin-2-yl)piperidine-2,6-dione;
2-((2-(2,6-dioxopiperidin-3-yl)-1-thioxoisoindolin-5-yl)thio)ethyl 4-methylbenzenesulfonate;
2-((2-(2,6-dioxopiperidin-3-yl)-1-thioxoisoindolin-4-yl)thio)ethyl 4-methylbenzenesulfonate;
3-(4-(piperazin-1-yl)-1-thioxoisoindolin-2-yl)piperidine-2,6-dione;
3-(5-(piperazin-1-yl)-1-thioxoisoindolin-2-yl)piperidine-2,6-dione;
3-(4-fluoro-5-(piperazin-1-yl)-1-thioxoisoindolin-2-yl)piperidine-2,6-dione;
3-(6-fluoro-5-(piperazin-1-yl)-1-thioxoisoindolin-2-yl)piperidine-2,6-dione;
3-(7-fluoro-5-(piperazin-1-yl)-1-thioxoisoindolin-2-yl)piperidine-2,6-dione;
3-(5-(3,6-diazabicyclo[3.1.1]heptan-3-yl)-1-thioxoisoindolin-2-yl)piperidine-2,6-dione;
3-(5-(3,6-diazabicyclo[3.1.1]heptan-6-yl)-1-thioxoisoindolin-2-yl)piperidine-2,6-dione;
3-(5-((1R,4R)-2,5-diazabicyclo[2.2.1]heptan-2-yl)-1-thioxoisoindolin-2-yl)piperidine-2,6-dione;
3-(5-((1S,4S)-2,5-diazabicyclo[2.2.1]heptan-2-yl)-1-thioxoisoindolin-2-yl)piperidine-2,6-dione;
3-(5-(3,8-diazabicyclo[3.2.1]octan-3-yl)-1-thioxoisoindolin-2-yl)piperidine-2,6-dione;
3-(5-(3,8-diazabicyclo[3.2.1]octan-8-yl)-1-thioxoisoindolin-2-yl)piperidine-2,6-dione;
3-(5-(2,5-diazabicyclo[2.2.2]octan-2-yl)-1-thioxoisoindolin-2-yl)piperidine-2,6-dione;
3-(6-(4-(piperazin-1-yl)piperidin-1-yl)-1-thioxoisoindolin-2-yl)piperidine-2,6-dione;
3-(6-(3,3-difluoro-4-(piperazin-1-yl)piperidin-1-yl)-1-thioxoisoindolin-2-yl)piperidine-2,6-dione;
1-(2-(2,6-dioxopiperidin-3-yl)-1-thioxoisoindolin-4-yl)piperidine-4-carbaldehyde;
1-(2-(2,6-dioxopiperidin-3-yl)-1-thioxoisoindolin-5-yl)piperidine-4-carbaldehyde;
1'-(2-(2,6-dioxopiperidin-3-yl)-3-thioxoisoindolin-5-yl)-[1,4'-bipiperidine]-4-carbaldehyde;
3-(5-(piperidin-4-ylthio)-1-thioxoisoindolin-2-yl)piperidine-2,6-dione;
3-(5-(piperidin-4-ylmethyl)-1-thioxoisoindolin-2-yl)piperidine-2,6-dione;
3-(5-(piperidin-4-yloxy)-1-thioxoisoindolin-2-yl)piperidine-2,6-dione;
3-(5-(piperidin-4-ylamino)-1-thioxoisoindolin-2-yl)piperidine-2,6-dione;
3-(5-(azetidin-3-ylmethyl)-1-thioxoisoindolin-2-yl)piperidine-2,6-dione;
3-(5-(azetidin-3-ylidenemethyl)-1-thioxoisoindolin-2-yl)piperidine-2,6-dione;
3-(5-(azetidin-3-ylthio)-1-thioxoisoindolin-2-yl)piperidine-2,6-dione;
3-(5-(azetidin-3-yloxy)-1-thioxoisoindolin-2-yl)piperidine-2,6-dione;
3-(5-(piperidin-4-yl)-1-thioxoisoindolin-2-yl)piperidine-2,6-dione;
3-(5-(1,2,3,6-tetrahydropyridin-4-yl)-1-thioxoisoindolin-2-yl)piperidine-2,6-dione;
rel-3-(5-((3S,4S)-3,4-dihydroxypiperidin-4-yl)-1-thioxoisoindolin-2-yl)piperidine-2,6-dione;
3-(4-fluoro-5-(1,2,3,6-tetrahydropyridin-4-yl)-1-thioxoisoindolin-2-yl)piperidine-2,6-dione;
3-(4-fluoro-5-(piperidin-4-yl)-1-thioxoisoindolin-2-yl)piperidine-2,6-dione;
3-(7-fluoro-5-(piperidin-4-yl)-1-thioxoisoindolin-2-yl)piperidine-2,6-dione;
3-(5-((S)-hydroxy(3-hydroxyazetidin-3-yl)methyl)-1-thioxoisoindolin-2-yl)piperidine-2,6-dione;
3-(5-(3,8-diazabicyclo[3.2.1]octan-8-yl)-1-thioxoisoindolin-2-yl)piperidine-2,6-dione;
3-(5-(3,8-diazabicyclo[3.2.1]octan-3-yl)-1-thioxoisoindolin-2-yl)piperidine-2,6-dione;
3-(6-fluoro-5-(piperazin-1-yl)-1-thioxoisoindolin-2-yl)piperidine-2,6-dione;
3-(5-(3,8-diazabicyclo[3.2.1]octan-3-yl)-6-fluoro-1-thioxoisoindolin-2-yl)piperidine-2,6-dione;
3-(5-(3,8-diazabicyclo[3.2.1]octan-8-yl)-6-fluoro-1-thioxoisoindolin-2-yl)piperidine-2,6-dione;
3-(5-(piperazin-1-ylamino)-1-thioxoisoindolin-2-yl)piperidine-2,6-dione;
3-(5-(azetidin-3-ylamino)-6-fluoro-1-thioxoisoindolin-2-yl)piperidine-2,6-dione;
3-(5-(pyrrolidin-3-ylthio)-1-thioxoisoindolin-2-yl)piperidine-2,6-dione;
3-(5-(azetidin-3-ylthio)-1-thioxoisoindolin-2-yl)piperidine-2,6-dione;
3-(5-(azetidin-3-ylamino)-1-thioxoisoindolin-2-yl)piperidine-2,6-dione;
3-(5-(azetidin-3-ylmethyl)-6-fluoro-1-thioxoisoindolin-2-yl)piperidine-2,6-dione;
3-(1-thioxoisoindolin-2-yl)piperidine-2,6-dione;
(2-(2,6-dioxopiperidin-3-yl)-1-thioxoisoindolin-4-yl)methyl methanesulfonate; and
(2-(2,6-dioxopiperidin-3-yl)-3-thioxoisoindolin-5-yl)methyl methanesulfonate.

30. A pharmaceutical composition, comprising:
the compound of Formula (I) or pharmaceutically acceptable salts, enantiomers, stereoisomers, solvates, isotopically enriched analogs, prodrugs, or polymorphs thereof as claimed in any one of claims 1-20; or
the compound of Formula (II) or pharmaceutically acceptable salts, enantiomers, stereoisomers, solvates, isotopically enriched analogs, prodrugs, or polymorphs thereof as claimed in any one of claims 21-29; and
at least one pharmaceutically acceptable carrier.

31. The pharmaceutical composition as claimed in claim 30, further comprising at least one additional therapeutic agent, e.g., an anticancer agent.

32. A medicine kit or reagent kit comprising:
the compound of Formula (I) or a pharmaceutically acceptable salt thereof as claimed in any one of claims 1-20; or
the compound of Formula (II) or a pharmaceutically acceptable salt thereof as claimed in any one of claims 21-29; or
the pharmaceutical composition as claimed in claim 30 or 31.

33. The compound of Formula (I) or salts, enantiomers, stereoisomers, solvates, isotopically enriched analogs, prodrugs, or polymorphs thereof as claimed in any one of claims 1-20, for use in the prevention and/or treatment of a disease or disorder selected from the group consisting of: tumors, cancers, autoinflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

34. The compound of Formula (I) or salts, enantiomers, stereoisomers, solvates, isotopically enriched analogs, prodrugs, or polymorphs thereof as claimed in claim 33, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), monocytic leukemia, myelomonocytic leukemia, acute T-lymphocytic leukemia, T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

35. The compound of Formula (II) or salts, enantiomers, stereoisomers, solvates, isotopically enriched analogs, prodrugs, or polymorphs thereof as claimed in any one of claims 21-29 , for use in the prevention and/or treatment of a disease or disorder selected from the group consisting of: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

36. The compound of Formula (II) or salts, enantiomers, stereoisomers, solvates, isotopically enriched analogs, prodrugs, or polymorphs thereof as claimed in claim 35, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), monocytic leukemia, myelomonocytic leukemia, acute T-lymphocytic leukemia, T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

37. Use of the compound of Formula (I) or salts, enantiomers, stereoisomers, solvates, isotopically enriched analogs, prodrugs, or polymorphs thereof as claimed in any one of claims 1-20 for the manufacture of a medicament for the prevention and/or treatment of a disease or disorder selected from the group consisting of: tumors, cancers, autoinflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

38. A method for treating or preventing a disease or disorder in a subject, comprising administering to the subject a therapeutically effective amount of the compound of Formula (I) or salts, enantiomers, stereoisomers, solvates, isotopically enriched analogs, prodrugs, or polymorphs thereof as claimed in any one of claims 1-20, or the pharmaceutical composition as claimed in claim 30 or 31, wherein the disease or disorder comprises tumors, cancers, autoinflammatory diseases, inflammatory diseases, autoimmune diseases, neurological disorders, respiratory diseases, hemorrhagic shock, transplant rejection, multiple organ dysfunction syndrome (MODS), sarcoidosis, adult respiratory distress syndrome, cardiovascular diseases, Richter syndrome (RS), acute liver failure, diabetes, Kennedy disease, seborrheic alopecia, hirsutism, skin diseases, dysfunctional uterine bleeding, anemia, pediatric aplastic anemia, endometriosis, polycystic ovary syndrome, and thyroid diseases.

39. The use as claimed in claim 37, or the method as claimed in claim 38, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), monocytic leukemia, myelomonocytic leukemia, acute T-lymphocytic leukemia, T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

40. Use of the compound of Formula (II) or salts, enantiomers, stereoisomers, solvates, isotopically enriched analogs, prodrugs, or polymorphs thereof as claimed in any one of claims 21-29 for the manufacture of a medicament for the prevention and/or treatment of a disease or disorder selected from the group consisting of: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

41. A method for treating or preventing a disease or disorder in a subject, comprising administering to the subject a therapeutically effective amount of the compound of Formula (II) or salts, enantiomers, stereoisomers, solvates, isotopically enriched analogs, prodrugs, or polymorphs thereof as claimed in any one of claims 21-29, or the pharmaceutical composition as claimed in claim 30 or 31, wherein the disease or disorder comprises tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

42. The use as claimed in claim 40, or the method as claimed in claim 41, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, acute myeloid leukemia (AML), monocytic leukemia, myelomonocytic leukemia, acute T-lymphocytic leukemia, T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.
